# EUROPEAN PATENT APPLICATION

(11) **EP 2 119 702 A1**
(43) Date of publication of application: **18.11.2009**
(21) Application number: 08704181.0
(22) Date of filing: 30.01.2008
(51) Int. Cl.: C07D 207/14, A61K 31/40, A61K 31/439, A61K 31/445, A61K 31/4462, A61K 31/45, A61K 31/451, A61K 31/55, A61P 3/10, A61P 9/04, A61P 9/10, A61P 9/12, A61P 13/12, A61P 25/06, A61P 27/02, A61P 27/06, A61P 43/00, C07D 211/56, C07D 221/22, C07D 223/12

(54) **AMIDE DERIVATIVE**

(30) Priority: 31.01.2007 JP 2007022513; 28.02.2007 JP 2007050871; 27.12.2007 JP 2007338384
(71) Applicant: Dainippon Sumitomo Pharma Co., Ltd., Osaka 541-8524 (JP)
(72) Inventor: NAKAHIRA, Hiroyuki, Osaka-shi Osaka 554-0022 (JP); IKUMA, Yohei, Osaka-shi Osaka 554-0022 (JP); FUKUDA, Nobuhisa, Osaka-shi Osaka 554-0022 (JP); KOBAYASHI, Tomonori, Osaka-shi Osaka 554-0022 (JP); TOSAKI, Shinya, Osaka-shi Osaka 554-0022 (JP)
(74) Representative: Webster, Jeremy Mark
(86) International application number: PCT/JP2008/051418
(87) International publication number: WO 2008/093737

(57) **Abstract**

The present invention relates to a compound of the formula (I) being useful as a renin inhibitor, or a pharmaceutically acceptable salt thereof. 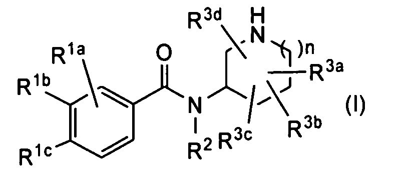 wherein R^{1a} is a hydrogen atom, an optionally substituted C₁₋₆ alkyl, etc.; R^{1b} is an optionally substituted C₁₋₆ alkoxy, etc.; R^{1c} is a hydrogen atom, an optionally substituted C₁₋₆ alkoxy, etc.; R² is a hydrogen atom, an optionally substituted C₁₋₆ alkyl, etc.; R^{3a}, R^{3b}, R^{3c} and R^{3d} are independently the same or different, and each is a group of the formula: -A-B (in which A is a single bond, -(CH₂)ₛO-, - (CH₂)ₛN(R⁴)CO-, etc., B is a hydrogen atom, an optionally substituted C₁₋₆ alkyl, etc.), etc.; R⁴ is a hydrogen atom, an optionally substituted C₁₋₆ alkyl, etc.; s is 0, etc.; and n is 1, etc.

SEQUENCE LISTING

<110>  Dainippon Sumitomo Pharma Co., Ltd.

<120>  Amido derivatives

<130>  668044

<150> JP 2007-022513
<151> 2007-01-31

<150> JP 2007-050871
<151> 2007-02-28

<150> JP 2007-338384
<151> 2007-12-27

<160>  2

<170>  PatentIn version 3.1

<210>  1
<211>  14
<212>  PRT
<213>  Artificial

<220>
<223>  2-Glu is designed by EDANS. 13-Lys is designed by DABCYL.

<400>  1

Arg Glu Ile His Pro Phe His Leu Val Ile His Thr Lys Arg
1               5                   10


<210>  2
<211>  10
<212>  PRT
<213>  Artificial

<220>
<223>  1-Ile is designed by (DABCYL-ƒÁ-Abu). 10-Thr is designed by EDANS
       .

<400>  2

Ile His Pro Phe His Leu Val Ile His Thr
1               5                   10

## Description

### TECHNICAL FIELD

The present invention relates to an amide derivative being useful as a medicament. More particularly, the present invention relates to an amide derivative being efficacious as a renin inhibitor. Further, the present invention relates to a therapeutic agent of hypertension, which comprises as the active ingredient an amide derivative being efficacious as a renin inhibitor.

### [BACKGROUND ART]

Renin-angiotensin (RA) system is a hormone system being important to the maintenance of blood pressure or electrolyte balance within the body, and plays an important role in the onset or development of circulatory system diseases such as hypertension, congestive heart failure, renal disease, etc.
Renin, which is an important component of RA system, is aspartic protease being mainly excreted from the kidney into the blood, and specifically decomposes angiotensinogen produced in the liver to produce angiotensin I. Angiotensin I is converted into angiotensin II by angiotensin-converting enzyme (ACE) being existing in the lung or vascular endothelial cells. Angiotensin II constricts the blood vessel as well as stimulates the adrenal gland to promote the secretion of aldosterone. Aldosterone acts on the kidney to store sodium and to excrete potassium. This cascade will lead to the increase in blood pressure (Non-patent document 1).

Recently, it has been indicated that there are components of RA system in the peripheral tissues or the central tissues such as heart, blood vessel, kidney, adrenal gland, adipose, etc. topically, and further that there is a possibility that a novel component, (pro)renin receptor, plays an important role in the activation of local RA system (Non-patent document 2), by which the importance of topical (tissue) RA system has become recognized. On the contrary to the circulatory RA system which participates in the short-term circulation control, the tissue RA system initiates the remodeling of various organs in the longer term such as heart, kidney, blood vessel, etc. Accordingly, it is suggested that there is a possibility that the tissue RA system may cause the organ damages such as cardiac enlargement, arteriosclerosis, renal disease, etc. (Non-patent document 3).

As a drug suppressing RA system, ACE inhibitors and angiotensin II receptor antagonists (ARB) can be exemplified, but these drugs (especially the former ones) have been proven to be useful as a therapeutic agent not only for hypertension but also for cardiovascular diseases such as heart failure or diabetic nephropathy, and hence, these drugs have widely been used (Non-patent document 4, Non-patent document 5).
There are multiple steps for suppressing RA system, and among them, renin is located at the most upper stream of RA system and controls the rate of this cascade, so that it is quite inviting approach on theory to inhibit renin (Non-patent document 6, Non-patent document 7). In fact, it is confirmed that aliskiren, which is a renin inhibitor and has been developed recently, significantly inhibits plasma renin activity and exhibits excellent hypotensive activity comparable to other RA system inhibitors in the clinical test in patients with hypertension (Non-patent document 8, Non-patent document 9, Non-patent document 10).

At the moment, various renin inhibitors have been reported, for example, Patent document 1 and Patent document 2 disclose that derivatives having a piperidine ring are useful as a renin inhibitor. Patent document 3 discloses that derivatives having a pyrrolidine ring are useful as a renin inhibitor. The compounds disclosed in these literatures are structurally characteristic in that the 3-position of piperidine ring or pyrrolidine ring is attached to an amino group via a carbonyl group or a methylene chain. However, it has not been known until now that compounds having a 3-((3-substituted phenyl)carbonylamino)piperidine as a basic skeleton are useful as a renin inhibitor.

[Non-patent document 1] Nat Rev Drug Discov. 1 (8): p.621-36 (2002)
[Non-patent document 2] Curr Hypertens Rep. 6(2): p.129-32 (2004)
[Non-patent document 3] Physiol. Rev. 86: p.747-803, (2006)
[Non-patent document 4] Curr Diab Rep. 6(1): p.8-16, (2006)
[Non-patent document 5] J Hypertens Suppl. 23(1): S9-17, (2005)
[Non-patent document 6] J Exp Med. 106(3): p.439-53, (1957)
[Non-patent document 7] J Am Soc Nephrol 16: p.592-599, (2005)
[Non-patent document 8] Hypertension 42(6): p.1137-43, (2003)
[Non-patent document 9] Circulation 111(8): p.1012-8, (2005)
[Non-patent document 10] J Hypertens. 24 (Suppl 4): S82. Abstract P4.269, (2006)
[Patent document 1] WO 06/069788 pamphlet
[Patent document 2] WO 06/094763 pamphlet
[Patent document 3] WO 06/066896 pamphlet

### [DISCLOSURE OF INVENTION]

### [PROBLEMS TO BE SOLVED BY INVENTION]

An object of the present invention is to provide a novel compound exhibiting an excellent renin inhibitory activity.

### [MEANS FOR SOLVING THE PROBLEMS]

The present inventors have intensively studied in order to achieve the above object, and have found that the following compounds or a pharmaceutically acceptable salt thereof (hereinafter, occasionally referred to as the present compound(s)) exhibit an excellent renin inhibitory activity, and have accomplished the present invention.

Namely, the present invention is as follows:
Item 1: A compound of the formula (I) or a pharmaceutically acceptable salt thereof.

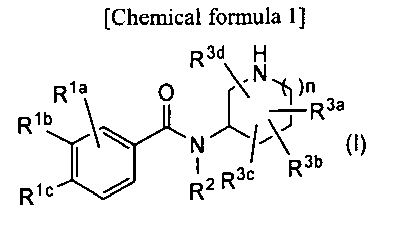

[wherein R^{1a} is a hydrogen atom, a halogen atom, a hydroxy group, a formyl group, a carboxyl group, a cyano group, an optionally substituted C₁₋₆ alkyl group, an optionally substituted C₂₋₆ alkenyl group, an optionally substituted C₂₋₆ alkynyl group, an optionally substituted C₃₋₆ cycloalkyl group, a C₁₋₆ alkylthio group, a C₁₋₆ alkylsulfonyl group, a C₁₋₆ alkoxy group optionally substituted by halogen, C₁₋₄ alkoxy or C₃₋₆ cycloalkyl, an optionally substituted C₃₋₆ cycloalkoxy group, an optionally substituted amino group, an aminocarbonyl group, a C₁₋₄ alkoxycarbonyl group, a C₁₋₄ alkylcarbonyl group, an optionally substituted C₆₋₁₀ aryl group, an optionally substituted C₆₋₁₀ aryloxy group, or an optionally substituted C₇₋₁₄ aralkyloxy group;
R^{1b} is a C₁₋₆ alkyl group substituted by mono-C₁₋₆ alkoxycarbonylamino, an optionally substituted C₁₋₆ alkylsulfinyl group, an optionally substituted C₁₋₆ alkylsulfonyl group, a substituted C₁₋₆ alkoxy group, an optionally substituted amino group, an optionally substituted aminocarbonyl group, an optionally substituted C₁₋₄ alkoxycarbonyl group, or an optionally substituted C₁₋₄ alkylcarbonyl group (where the substituted C₁₋₆ alkoxy group is substituted by one of the members selected from hydroxy, C₁₋₄ alkoxy, C₃₋₆ cycloalkoxy, trifluoromethyl, trifluoromethoxy, difluoromethoxy, carboxy, mono-C₁₋₆ alkylcarbonylamino and mono-C₁₋₆ alkoxycarbonylamino);
R^{1c} is a hydrogen atom, a halogen atom, a hydroxy group, a formyl group, a carboxyl group, a cyano group, an optionally substituted C₁₋₆ alkyl group, an optionally substituted C₃₋₆ cycloalkyl group, an optionally substituted C₅₋₆ cycloalkenyl group, an optionally substituted 5- or 6-membered saturated heterocyclic group, an optionally substituted C₁₋₆ alkylthio group, an optionally substituted C₁₋₆ alkylsulfinyl group, an optionally substituted C₁₋₆ alkylsulfonyl group, an optionally substituted C₆₋₁₀ arylthio group, an optionally substituted C₆₋₁₀ arylsulfinyl group, an optionally substituted C₆₋₁₀ arylsulfonyl group, an optionally substituted C₁₋₆ alkoxy group, an optionally substituted C₃₋₆ cycloalkyloxy group, an optionally substituted C₆₋₁₀ aryloxy group, an optionally substituted C₇₋₁₄ aralkyloxy group, an optionally substituted amino group, an optionally substituted aminocarbonyl group, an optionally substituted C₁₋₄ alkoxycarbonyl group, an optionally substituted C₃₋₆ cycloalkyloxycarbonyl group, an optionally substituted C₁₋₄ alkylcarbonyl group, an optionally substituted C₃₋₆ cycloalkylcarbonyl group, an optionally substituted C₆₋₁₀ arylcarbonyl group, an optionally substituted C₇₋₁₄ aralkyl group, or an optionally substituted 5- to 10-membered monocyclic or polycyclic heteroarylcarbonyl group; or
R^{1a} is a hydrogen atom; and R^{1b} and R^{1c} may combine each other to form a condensed ring with the benzene ring containing at least one heteroatom;
R² is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, or an optionally substituted 5- to 10-membered monocyclic or polycyclic heteroaryl group (provided that when R^{1a} is a C₁₋₆ alkoxy group having a halogen substituent, then R² is not a hydrogen atom);
R^{3a}, R^{3b}, R^{3c} and R^{3d} are independently the same or different and each is a halogen atom, a cyano group, or a group of the formula: -A-B (in which A is a single bond, -(CH₂)ₛO-, -(CH₂)ₛN(R⁴)-, - (CH₂)ₛSO₂-, -(CH₂)ₛCO-, -(CH₂)ₛCOO-, -(CH₂)ₛN(R⁴)CO-, -(CH₂)ₛN(R⁴)SO₂-, -(CH₂)ₛN(R⁴)COO-, -(CH₂)ₛOCON(R⁴)-, -(CH₂)ₛO-CO-, -(CH₂)ₛCON(R⁴)-, -(CH₂)ₛN(R⁴)CON(R⁴)-, or -(CH₂)ₛSO₂N(R⁴)-,
B is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted 5- to 10-membered monocyclic or polycyclic heteroaryl-C₁₋₄ alkyl group, or an optionally substituted saturated heterocyclic group (provided that when A is -(CH₂)ₛN(R⁴)-, -(CH₂)ₛOCON(R⁴)-, -(CH₂)ₛCON(R⁴)-, -(CH₂)ₛN(R⁴)CON(R⁴)-, or -(CH₂)ₛSO₂N(R⁴)-, then R⁴ and B may combine each other to form a ring), or two of R^{3a}, R^{3b}, R^{3c} and R^{3d} are a hydrogen atom, and the remaining two groups may combine to form a fused ring with a heterocyclic ring;
R⁴ is a hydrogen atom, an optionally substituted C₁₋₆ alkyl group, an optionally substituted C₃₋₁₀ cycloalkyl group, an optionally substituted C₆₋₁₀ aryl group, an optionally substituted C₇₋₁₄ aralkyl group, or an optionally substituted 5- to 10-membered monocyclic or polycyclic heteroaryl group;
s is 0, 1 or 2 (provided that when A is -(CH₂)ₛN(R⁴)-, then s is 0 or 2, and when A is - (CH₂)ₛCON(R⁴)-, then s is 1 or 2);
n is 0, 1, or 2]

Item 2: The compound of Item 1 or a pharmaceutically acceptable salt thereof, wherein R^{1a} is a hydrogen atom, a halogen atom, a hydroxy group, a cyano group, a C₃₋₆ cycloalkyl group, a C₁₋₆ alkyl group, or a C₃₋₆ cycloalkoxy group;
R^{1b} is a C₁₋₆ alkyl group substituted by mono-C₁₋₆ alkoxycarbonylamino, a substituted C₁₋₆ alkoxy group, or an optionally substituted amino group (in which the substituted C₁₋₆ alkoxy group is substituted by one group selected from hydroxy, C₁₋₄ alkoxy, trifluoromethyl, trifluoromethoxy, difluoromethoxy, carboxy, mono-C₁₋₆ alkylcarbonylamino and mono-C₁₋₆ alkoxycarbonylamino),
R^{1c} is a hydrogen atom; a halogen atom; a C₁₋₆ alkoxy group optionally substituted by 1 to 3 halogens or by one of the members selected from C₃₋₆ cycloalkyl, mono- or di-(C₁₋₆ alkyl)-substituted aminocarbonyl, 5- or 6-membered saturated heterocycle, and C₁₋₄ alkoxycarbonyl; a C₃₋₆ cycloalkyloxy group; or a 5- or 6-membered saturated heterocyclic group, or
R^{1a} is a hydrogen atom; and R^{1b} and R^{1c} may combine each other to form a condensed ring with the benzene ring containing at least one heteroatom.

Item 3: The compound of Item 1, or a pharmaceutically acceptable salt thereof, wherein R^{1a} is a hydrogen atom, a halogen atom, a hydroxy group, a cyano group, a C₃₋₆ cycloalkyl group, a C₁₋₆ alkyl group, or a C₃₋₆ cycloalkoxy group;
R^{1b} is a C₁₋₆ alkyl group substituted by mono-C₁₋₆ alkoxycarbonylamino, a substituted C₁₋₆ alkoxy group, or an optionally substituted amino group (in which the substituted C₁₋₆ alkoxy group is substituted by one group selected from hydroxy, C₁₋₄ alkoxy, trifluoromethyl, trifluoromethoxy, difluoromethoxy, carboxy, mono-C₁₋₆ alkylcarbonylamino and mono-C₁₋₆ alkoxycarbonylamino);
R^{1c} is a hydrogen atom; a halogen atom; a C₁₋₆ alkoxy group optionally substituted by 1 to 3 halogens, or by one of the members selected from C₃₋₆ cycloalkyl, mono- or di-(C₁₋₆ alkyl)-substituted aminocarbonyl, 5- or 6-membered saturated heterocycle, and C₁₋₄ alkoxycarbonyl; a C₃₋₆ cycloalkyloxy group; a C₇₋₁₄ aralkyloxy group having optionally a mono- or di-(C₁₋₆ alkyl)aminocarbonyl substituent; or a 5- or 6-membered saturated heterocyclic group.

Item 4: The compound of Item 1 or a pharmaceutically acceptable salt thereof, wherein R^{1a} is a hydrogen atom, a halogen atom, a cyano group, a C₁₋₆ alkyl group having optionally a halogen substituent, a C₇₋₆ alkenyl group, a C₃₋₆ cycloalkyl group, a C₁₋₆ alkoxy group having optionally a halogen substituent, or a C₃₋₆ cycloalkoxy group.

Item 5: The compound of any one of Items 1 to 4 or a pharmaceutically acceptable salt thereof, wherein R^{1a}, R^{1b} and R^{1c} attach to the benzene ring at the substitution positions as shown in the following formula:

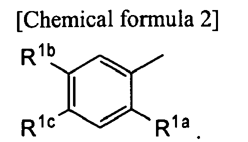

Item 6: The compound of Item 5 or a pharmaceutically acceptable salt thereof, wherein R^{1a} is a hydrogen atom, a halogen atom, a cyano group, a C₁₋₆ alkyl group, a C₃₋₆ cycloalkyl group, or a C₃₋₆ cycloalkoxy group.

Item 7: The compound of Item 6 or a pharmaceutically acceptable salt thereof, wherein R^{1a} is a hydrogen atom, a halogen atom, a C₁₋₆ alkyl group, a C₃₋₆ cycloalkyl group, or a C₃₋₆ cycloalkoxy group.

Item 8: The compound of Item 7 or a pharmaceutically acceptable salt thereof, wherein R^{1a} is a hydrogen atom, a C₁₋₆ alkyl group, a C₃₋₆ cycloalkyl group, or a C₃₋₆ cycloalkoxy group.

Item 9: The compound of Item 8 or a pharmaceutically acceptable salt thereof, wherein R^{1a} is a hydrogen atom.

Item 10: The compound of Item 6 or a pharmaceutically acceptable salt thereof, wherein R^{1a} is a halogen atom, a cyano group, or a C₁₋₆ alkyl group.

Item 11: The compound of Item 10 or a pharmaceutically acceptable salt thereof, wherein R^{1a} is a C₁₋₆ alkyl group.

Item 12: The compound of Item 10 or a pharmaceutically acceptable salt thereof, wherein R^{1a} is a halogen atom.

Item 13: The compound of Item 10 or a pharmaceutically acceptable salt thereof, wherein R^{1a} is a cyano group.

Item 14: The compound of any one of Items 1 to 4 or a pharmaceutically acceptable salt thereof, wherein R^{1a}, R^{1b} and R^{1c} attach to the benzene ring at the substitution positions as shown in the following formula:

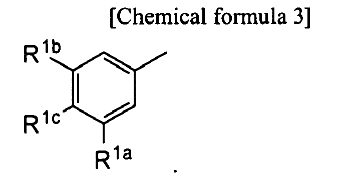

Item 15: The compound of Item 14 or a pharmaceutically acceptable salt thereof, wherein R^{1a} is a hydroxy group or a C₃₋₆ cycloalkoxy group.

Item 16: The compound of any one of Items 1 to 15 or a pharmaceutically acceptable salt thereof, wherein R^{1b} is a substituted C₁₋₆ alkoxy group (in which the substituted C₁₋₆ alkoxy group is substituted by C₁₋₄ alkoxy, carboxy, mono-C₁₋₆ alkylcarbonylamino or mono-C₁₋₆ alkoxycarbonylamino).

Item 17: The compound of Item 16 or a pharmaceutically acceptable salt thereof, wherein R^{1b} is a C₁₋₆ alkoxy group substituted by C₁₋₄ alkoxy.

Item 18: The compound of Item 17 or a pharmaceutically acceptable salt thereof, wherein R^{1b} is a 3-methoxypropyloxy group.

Item 19: The compound of any one of Items 1 to 18 or a pharmaceutically acceptable salt thereof, wherein R^{1c} is a hydrogen atom or a C₁₋₆ alkoxy group.

Item 20: The compound of Item 19 or a pharmaceutically acceptable salt thereof, wherein R^{1c} is a C₁₋₆ alkoxy group.

Item 21: The compound of Item 1 or Item 2 or a pharmaceutically acceptable salt thereof, wherein R^{1a} is a hydrogen atom; R^{1b} and R^{1c} combine each other to form a condensed ring with a benzene ring containing at least one heteroatom.

Item 22: The compound of Item 21 or a pharmaceutically acceptable salt thereof, wherein the condensed ring is a condensed ring of a 5- or 6-membered saturated heterocyclic ring and a benzene ring.

Item 23: The compound of Item 22 or a pharmaceutically acceptable salt thereof, wherein the condensed ring is a condensed ring with a ring having a partial structure of the following formula:

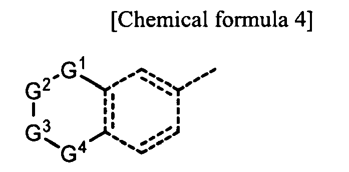

[wherein (i) G¹ is -N(R^{1d})-, G² is -CO-, G³ is -C(R^{1e})(R^{1f})-, and G⁴ is -CH₂-, an oxygen atom, a sulfur atom, or does not exist at all,
(ii) G¹ is -N(R^{1d})-, G² is -CO-, G³ is -N(R^{1d})-, and G⁴ does not exist at all (R^{1d} for G¹ and G³ is independent each other),
(iii) G¹ is an oxygen atom, G² is -CH₂-, G³ is an oxygen atom, and G⁴ does not exist at all, or
(iv) G¹ is an oxygen atom, G² is -CH₂-, G³ is -CH₂-, and G⁴ is an oxygen atom;
R^{1d} is a hydrogen atom, a C₆₋₁₀ aryl group, or a C₁₋₆ alkyl group optionally substituted by one group selected from hydroxy, C₃₋₆ cycloalkyl, C₁₋₆ alkoxy, C₃₋₆ cycloalkoxy, trifluoromethyl, trifluoromethoxy and mono-C₁₋₆ alkoxycarbonylamino;
R^{1e} and R^{1f} are independently the same or different and each is a hydrogen atom, a halogen atom, a hydroxy group, a carboxyl group, a cyano group, an optionally substituted C₁₋₆ alkyl group, an optionally substituted C₂₋₆ alkenyl group, an optionally substituted C₂₋₆ alkynyl group, an optionally substituted C₃₋₆ cycloalkyl group, an optionally substituted C₁₋₆ alkoxy group, an optionally substituted C₃₋₆ cycloalkoxy group, an optionally substituted amino group, an optionally substituted aminocarbonyl group, an optionally substituted C₁₋₄ alkoxycarbonyl group, an optionally substituted C₁₋₄ alkylcarbonyl group, an optionally substituted C₆₋₁₀ arylcarbonyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted C₆₋₁₀ aryloxy group, an optionally substituted 5- to 10-membered monocyclic or polycyclic heteroaryloxy group, or an optionally substituted C₇₋₁₄ aralkyloxy group, provided that the ring expressed with dashed line(s) in said chemical structure means a benzene ring].

Item 24: The compound of Item 23 or a pharmaceutically acceptable salt thereof, wherein R^{1d} is a C₁₋₆ alkyl group optionally substituted by C₁₋₆ alkoxy or mono-C₁₋₆ alkoxycarbonylamino.

Item 25: The compound of Item 24 or a pharmaceutically acceptable salt thereof, wherein R^{1d} is a C₁₋₆ alkyl group optionally substituted by C₁₋₆ alkoxy.

Item 26: The compound of Item 25 or a pharmaceutically acceptable salt thereof, wherein R^{1d} is a 4-methoxybutyl group.

Item 27: The compound of Item 24 or a pharmaceutically acceptable salt thereof, wherein R^{1d} is a C₁₋₆ alkyl group optionally substituted by mono-C₁₋₆ alkoxycarbonylamino.

Item 28: The compound of Item 27 or a pharmaceutically acceptable salt thereof, wherein R^{1d} is a methoxycarbonylaminoethyl group.

Item 29: The compound of any one of Items 23 to 28 or a pharmaceutically acceptable salt thereof, wherein R^{1e} and R^{1f} are independently the same or different and each is a hydrogen atom; a halogen atom; a hydroxy group; a cyano group; a C₃₋₆ cycloalkyl group; a C₁₋₆ alkyl group optionally substituted by C₁₋₄ alkoxy, C₃₋₆ cycloalkyl, C₃₋₆ cycloalkyloxy, C₇₋₁₀ aralkyloxy, or aminocarbonyl having optionally a mono- or di-(C₁₋₆ alkyl) substituent; a C₆₋₁₀ aryl group having optionally a halogen subsitutent; a C₇₋₁₄ aralkyl group having optionally a halogen substituent; an optionally substituted aminocarbonyl group; or a 5- to 10-membered monocyclic or polycyclic heteroaryl group optionally substituted by C₁₋₄ alkyl.

Item 30: The compound of Item 29 or a pharmaceutically acceptable salt thereof, wherein R^{1e} and R^{1f} are independently the same or different and each is a hydrogen atom; a C₁₋₆ alkyl group optionally substituted by C₁₋₆ alkoxy; a C₆₋₁₀ aryl group having optionally a halogen substituent; or an aminocarbonyl group optionally having a mono- or di-(C₁₋₆ alkyl) substituent.

Item 31: The compound of any one of Items 1 to 30 or a pharmaceutically acceptable salt thereof, wherein R² is a C₁₋₆ alkyl group having optionally a C₃₋₆ cycloalkyl substituent, a C₃₋₆ cycloalkyl group, a C₆₋₁₀ aryl group having optionally a halogen substituent, or a C₇₋₁₄ aralkyl group having optionally a halogen substituent.

Item 32: The compound of Item 31 or a pharmaceutically acceptable salt thereof, wherein R² is a C₁₋₆ alkyl group or a C₃₋₆ cycloalkyl group.

Item 33: The compound of Item 32 or a pharmaceutically acceptable salt thereof, wherein R² is isopropyl group.

Item 34: The compound of any one of Items 1 to 33 or a pharmaceutically acceptable salt thereof, wherein R^{3a}, R^{3b}, R^{3c}, and R^{3d} are independently a group of the formula: -A-B (in which A is a single bond, -(CH₂)ₛO-, -(CH₂)ₛN(R⁴)-, -(CH₂)ₛSO₂-, -(CH₂)ₛCO-, -(CH₂)ₛCOO-, -(CH₂)ₛN(R⁴)CO-, -(CH₂)ₛN(R⁴)SO₂-, -(CH₂)ₛN(R⁴)COO-, -(CH₂)ₛOCON(R⁴)-, -(CH₂)ₛO-CO-, -(CH₂)ₛCON(R⁴)-, -(CH₂)ₛN(R⁴)CON(R⁴)-, or -(CH₂)ₛSO₂N(R⁴)-,
B is a hydrogen atom; an optionally substituted C₁₋₆ alkyl group; a C₂₋₆ alkenyl group; a C₃₋₁₀ cycloalkyl group; a C₆₋₁₀ aryl group optionally substituted by the same or different 1 to 3 groups selected from halogen, cyano, C₁₋₄ alkyl, C₁₋₄ alkoxy, cyano, carboxy, C₁₋₄ alkyl having optionally carboxy substituent, or C₆ aryl having optionally a C₁₋₄ alkoxy substituent (said C₁₋₄ alkoxy being optionally substituted by fluorine, hydroxy, or carboxy), C₆ aryloxy, C₇₋₁₀ aralkyloxy having optionally a C₁₋₄ alkoxy substituent, aminocarbonyl having optionally a mono- or di-(C₁₋₆ alkyl) substituent, and C₁₋₄ alkylsulfonylamino; a C₇₋₁₄ aralkyl group optionaly substituted by the same or different 1 to 3 groups selected from halogen, cyano, C₁₋₄ alkyl having optionally a halogen substituent, hydroxy, C₁₋₄ alkoxy having optionally a halogen substituent, carboxy, C₁₋₄ alkoxycarbonyl, C₆ aryl having optionally a C₁₋₄ alkoxy substituent, C₆ aryloxy, C₇₋₁₀ aralkyloxy, aminocarbonyl having optionally a mono- or di-(C₁₋₆ alkyl) substituent, C₁₄ alkylsulfonylamino and C₁₋₄ alkylsulfonyl; a 5- or 6-membered monocyclic heteroaryl group optionally substituted by halogen or C₆ aryl; a 5- or 6-membered monocyclic heteroaryl-C₁₋₄ alkyl group having optionally a C₁₋₄ alkyl substituent; or a 5- or 6-membered saturated heterocyclic group (provided that when A is -(CH₂)ₛN(R⁴)-, -(CH₂)ₛOCON(R⁴)-, -(CH₂)ₛCON(R⁴)-, - (CH₂)ₛN(R⁴)CON(R⁴)-, or -(CH₂)ₛSO₂N(R⁴)-, then R⁴ and B combine each other to form a ring)).

Item 35: The compound of Item 34 or a pharmaceutically acceptable salt thereof, wherein B is a hydrogen atom; an optionally substituted C₁₋₆ alkyl group; a C₂₋₆ alkenyl group; a C₃₋₁₀ cycloalkyl group; a C₆₋₁₀ aryl group optionally substituted by the same or different 1 to 3 groups selected from halogen, C₆ aryl, and C₆ aryloxy; a C₇₋₁₄ aralkyl group optionally substituted by the same or different 1 to 3 groups selected from halogen, cyano, C₁₋₄ alkyl having optionally 1 to 3 halogen substituents, hydroxy, C₁₋₄ alkoxy, carboxy, C₁₋₄ alkoxycarbonyl, aminocarbonyl, and C₁₋₄ alkylsulfonylamino; a 5- or 6-membered monocyclic heteroaryl group optionally substituted by halogen or C₆ aryl; a 5- or 6-membered monocyclic heteroaryl-C₁₋₄ alkyl group having optionally a C₁₋₄ alkyl substituent; or a 5- or 6-membered saturated heterocyclic group.

Item 36: The compound of any one of Items 1 to 35 or a pharmaceutically acceptable salt thereof, wherein R^{3a}, R^{3b}, R^{3c} and R^{3d} attach to the piperidine ring at the substitution positions as shown in the following formula:

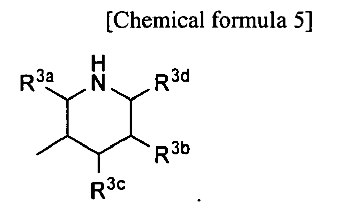

Item 37: The compound of Item 36 or a pharmaceutically acceptable salt thereof, wherein R^{3a} is a group of the formula: -A-B (in which A is a single bond, -(CH₂)ₛO-, -(CH₂)ₛN(R⁴)CO-, -(CH₂)ₛOCON(R⁴)-, or -(CH₂)ₛCON(R⁴)-, B is a hydrogen atom, an optionally substituted C₁₋₆ alkyl group, or an optionally substituted C₇₋₁₄ aralkyl group),
R^{3b} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, - (CH₂)ₛOCON(R⁴)-, -(CH₂)ₛCON(R⁴)-, or -(CH₂)ₛSO₂N(R⁴)-, B is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group),
R^{3c} is a group of the formula: -A-B (in which A is a single bond, -(CH₂)ₛO-, or -(CH₂)ₛCOO-, B is a hydrogen atom, an optionally substituted C₁₋₆ alkyl group, an optionally substituted C₆₋₁₀ aryl group, an optionally substituted C₇₋₁₄ aralkyl group, or an optionally substituted 5- to 10-membered monocyclic or polycyclic heteroaryl group),
R^{3d} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, - (CH₂)ₛOCON(R⁴)-, -(CH₂)ₛCON(R⁴)-, or -(CH₂)ₛ₂SO₂N(R⁴)-, B is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group), or
two groups of R^{3a}, R^{3b}, R^{3c} and R^{3d} are a hydrogen atom, and the remaining two groups combine each other to form a fused ring with a piperidine ring.

Item 38: The compound of Item 36 or a pharmaceutically acceptable salt thereof, wherein R^{3a} is a group of the formula: -A-B (in which A is a single bond, or -(CH₂)ₛCON(R⁴)-, B is a hydrogen atom, an optionally substituted C₁₋₆ alkyl group, or an optionally substituted C₇₋₁₄ aralkyl group);
R^{3b} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, - (CH₂)ₛOCON(R⁴)-, or -(CH₂)ₛCON(R⁴)-, B is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group);
R^{3c} is a group of the formula: -A-B (in which A is a single bond, -(CH₂)ₛO-, or -(CH₂)ₛCOO-, B is a hydrogen atom, an optionally substituted C₆₋₁₀ aryl group, an optionally substituted C₇₋₁₄ aralkyl group, or an optionally substituted 5- to 10-membered monocyclic or polycyclic heteroaryl group);
R^{3d} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, - (CH₂)ₛOCON(R⁴)-, or -(CH₂)ₛCON(R⁴)-, B is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group).

Item 39: The compound of any one of Items 36 to 38 or a pharmaceutically acceptable salt thereof, wherein R^{3a}, R^{3b} and R^{3d} are a group of the formula: -A-B (in which A is a single bond, B is a hydrogen atom);
R^{3c} is a group of the formula: -A-B (in which A is a single bond, -(CH₂)ₛO-, or -(CH₂)ₛCOO-, B is a hydrogen atom, an optionally substituted C₆₋₁₀ aryl group, an optionally substituted C₇₋₁₄ aralkyl group, or an optionally substituted 5- to 10-membered monocyclic or polycyclic heteroaryl group).

Item 40: The compound of Item 39 or a pharmaceutically acceptable salt thereof, wherein B for R^{3c} is an optionally substituted C₆₋₁₀ aryl group, an optionally substituted C₇₋₁₄ aralkyl group, or an optionally substituted 5- to 10-membered monocyclic or polycyclic heteroaryl group.

Item 41: The compound of Item 39 or a pharmaceutically acceptable salt thereof, wherein for R^{3c}, A is a single bond, and B is an optionally substituted C₆₋₁₀ aryl group.

Item 42: The compound of Item 36 or Item 38 or a pharmaceutically acceptable salt thereof, wherein R^{3a}, R^{3c} and R^{3d} are a group of the formula: -A-B (in which A is a single bond, and B is a hydrogen atom);
R^{3b} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, - (CH₂)ₛOCON(R⁴)-, or -(CH₂)ₛCON(R⁴)-, B is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group).

Item 43: The compound of Item 42 or a pharmaceutically acceptable salt thereof, wherein A for R^{3b} is -(CH₂)ₛO-, -(CH₂)ₛN(R⁴)CO-, -(CH₂)ₛN(R⁴)COO-, -(CH₂)ₛOCON(R⁴)-, or -(CH₂)ₛCON(R⁴)-.

Item 44: The compound of Item 42 or Item 43 or a pharmaceutically acceptable salt thereof, wherein B for R^{3b} is an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group.

Item 45: The compound of Item 36 or a pharmaceutically acceptable salt thereof, wherein R^{3a}, R^{3b} and R^{3c} are a group of the formula: -A-B (in which A is a single bond, B is a hydrogen atom);
R^{3d} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, -(CH₂)ₛOCON(R⁴)-, -(CH₂)ₛN(R⁴)CON(R⁴)-, or -(CH₂)ₛCON(R⁴)-, B is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- to 10-membered monocyclic or polycyclic heteroaryl-C₁₋₄ alkyl group, or an optionally substituted 5- or 6-membered saturated heterocyclic group).

Item 46: The compound of Item 45 or a pharmaceutically acceptable salt thereof, wherein A for R^{3d} is -(CH₂)ₛO-, -(CH₂)ₛCOO-, -(CH₂)ₛN(R⁴)CO-, -(CH₂)ₛN(R⁴)COO-, -(CH₂)ₛOCON(R⁴)-, or -(CH₂)ₛCON(R⁴)-.

Item 47: The compound of Item 45 or Item 46 or a pharmaceutically acceptable salt thereof, wherein A is -(CH₂)ₛN(R⁴)CO-.

Item 48: The compound of any one of Items 45 to 47 or a pharmaceutically acceptable salt thereof, wherein B for R^{3d} is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group.

Item 49: The compound of any one of Items 45 to 47 or a pharmaceutically acceptable salt thereof, wherein B for R^{3d} is an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- to 10-membered monocyclic or polycyclic heteroaryl-C₁₋₄ alkyl group, or an optionally substituted 5- or 6-membered saturated heterocyclic group.

Item 50: The compound of Item 45 or a pharmaceutically acceptable salt thereof, wherein for R^{3d}, A is -(CH₂)ₛN(R⁴)CO-, -(CH₂)ₛN(R⁴)SO₂-, or -(CH₂)ₛN(R⁴)COO-, and B is an optionally substituted C₁₋₆ alkyl group, an optionally substituted C₂₋₆ alkenyl group, an optionally substituted C₆₋₁₀ aryl group, an optionally substituted C₇₋₁₄ aralkyl group, or an optionally substituted 5- to 10-membered monocyclic or polycyclic heteroaryl-C₁₋₄ alkyl group; and R⁴ is a C₃₋₆ cycloalkyl group.

Item 51: The compound of Item 49 or Item 50 or a pharmaceutically acceptable salt thereof, wherein B is a C₁₋₆ alkyl group optionally substituted by one group selected from C₃₋₆ cycloalkyl, hydroxy, C₁₋₄ alkoxy, carboxy, C₁₋₄ alkoxycarbonyl, di-(C₁₋₆ alkyl)amino, and aminocarbonyl having optionally a mono- or di-(C₁₋₆ alkyl) substituent; a C₂₋₆ alkenyl group; a C₆₋₁₀ aryl group optionally substituted by the same or different 1 to 3 groups selected from halogen, C₆ aryl and C₆ aryloxy; a C₇₋₁₄ aralkyl group optionally substituted by the same or different 1 to 3 groups selected from halogen, cyano, C₁₋₄ alkyl having optionally 1 to 3 halogen substituents, hydroxy, C₁₋₄ alkoxy, carboxy, C₁₋₄ alkoxycarbonyl, aminocarbonyl, and C₁₋₄ alkylsulfonylamino; or a 5- to 10-membered monocyclic or polycyclic heteroaryl-C₁₋₄ alkyl group.

Item 52: The compound of any one of Items 1 to 51 or a pharmaceutically acceptable salt thereof, wherein s is 2.

Item 53: The compound of any one of Items 1 to 52 or a pharmaceutically acceptable salt thereof, wherein R⁴ is a hydrogen atom, an optionally substituted C₁₋₆ alkyl group, an optionally substituted C₃₋₁₀ cycloalkyl group, an optionally substituted C₆₋₁₀ aryl group, or an optionally substituted 5- to 10-membered monocyclic or polycyclic heteroaryl group.

Item 54: The compound of Item 53 or a pharmaceutically acceptable salt thereof, wherein R⁴ is a hydrogen atom, a C₁₋₆ alkyl group, a C₃₋₆ cycloalkyl group, a C₆₋₁₀ aryl group, a C₇₋₁₄ aralkyl group, or a 5- to 10-membered monocyclic or polycyclic heteroaryl group.

Item 55: The compound of Item 54 or a pharmaceutically acceptable salt thereof, wherein R⁴ is a C₃₋₆ cycloalkyl group.

Item 56: The compound of Item 36 or a pharmaceutically acceptable salt thereof, wherein R^{3a}, R^{3b}, R^{3c} and R^{3d} are independently a group of the formula: -A-B (in which A is a single bond, B is a hydrogen atom).

Item 57: The compound of any one of Items 1 to 35 or a pharmaceutically acceptable salt thereof, wherein n is 1.

Item 58: A compound of the formula (II) or a pharmaceutically acceptable salt thereof.

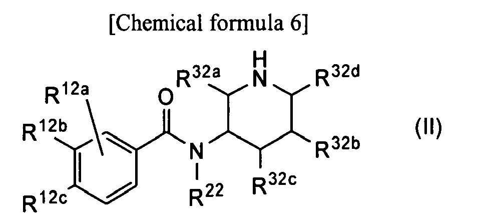

[wherein R^{12a} is a hydrogen atom, a halogen atom, a cyano group, an optionally substituted C₁₋₆ alkyl group, a C₂₋₆ alkenyl group, a C₂₋₆ alkynyl group, a C₃₋₆ cycloalkyl group, a C₁₋₆ alkylthio group, a C₁₋₆ alkylsulfonyl group, a C₁₋₆ alkoxy group optionally substituted by halogen, C₁₋₄ alkoxy or C₃₋₆ cycloalkyl, a C₃₋₆ cycloalkoxy group, an optionally substituted amino group, an aminocarbonyl group, a C₁₋₄ alkoxycarbonyl group, a C₁₋₄ alkylcarbonyl group, an optionally substituted C₆₋₁₀ aryl group, an optionally substituted C₆₋₁₀ aryloxy group, or an optionally substituted C₇₋₁₄ aralkyloxy group;
R^{12b} is a mono-C₁₋₆ alkoxycarbonylamino-substituted C₁₋₆ alkyl group, a substituted C₁₋₆ alkoxy group, or an optionally substituted amino group (in which the substituted C₁₋₆ alkoxy group is substituted by one group selected from hydroxy, C₁₋₄ alkoxy, C₃₋₆ cycloalkoxy, trifluoromethyl, trifluoromethoxy, difluoromethoxy, carboxy, mono-C₁₋₆ alkylcarbonylamino and mono-C₁₋₆ alkoxycarbonylamino);
R^{12c} is a hydrogen atom, a halogen atom, an optionally substituted C₁₋₆ alkoxy group, or an optionally substituted C₃₋₆ cycloalkyloxy group;
R²² is a hydrogen atom, an optionally substituted C₁₋₆ alkyl group, an optionally substituted C₃₋₆ cycloalkyl group, or an optionally substituted C₆₋₁₀ aryl group (when R^{12a} is a C₁₋₆ alkoxy group having a halogen substituent, then R²² is not a hydrogen atom);
R^{32a} is a group of the formula: - A²-B² (in which A² is a single bond, and B² is a hydrogen atom);
R^{32b}, R^{32c} and R^{32d} are independently the same or different and each is a group of the formula: - A²-B² (in which A² is a single bond, -(CH₂)ₛ₂O-, -(CH₂)ₛ₂N(R⁴²)-, -(CH₂)ₛ₂COO-, -(CH₂)ₛ₂N(R⁴²)CO-, -(CH₂)ₛ₂N(R⁴²)SO₂,- -(CH₂)ₛ₂N(R⁴²)COO-, -(CH₂)ₛ₂OCON(R⁴²)-, -(CH₂)ₛ₂N(R⁴²)CON(R⁴²)-, or -(CH₂)ₛ₂CON(R⁴²)-,
B² is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- or 6-membered monocyclic or polycyclic heteroaryl-C₁₋₄ alkyl group, or an optionally substituted 5- or 6-membered saturated heterocyclic group), or one of R^{32b}, R^{32c} and R^{32d} is a hydrogen atom, and the remaining two groups combine each other to form a fused ring with a piperidine ring;
R⁴² is a hydrogen atom, an optionally substituted C₁₋₆ alkyl group, an optionally substituted C₃₋₆ cycloalkyl group, or an optionally substituted C₇₋₁₄ aralkyl group;
s2 is 0, 1 or 2 (provided that when A² is -(CH₂)ₛ₂N(R⁴²)-, then s2 is 0 or 2, and when A² is - (CH₂)ₛ₂CON(R⁴²)-, then s2 is 1 or 2)].

Item 59: The compound of Item 58 or a pharmaceutically acceptable salt thereof, wherein R^{12a} is a hydrogen atom, a halogen atom, a cyano group, an optionally substituted C₁₋₆ alkyl group, a C₃₋₆ cycloalkyl group, or a C₃₋₆ cycloalkoxy group.

Item 60: The compound of Item 58 or Item 59 or a pharmaceutically acceptable salt thereon, wherein R^{12a}, R^{12b} and R^{12c} attach to the benzene ring at the substitution positions of the following formula:

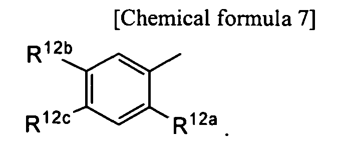

Item 61: The compound of Item 60 or a pharmaceutically acceptable salt thereof, wherein R^{12a} is a hydrogen atom, a halogen atom, a C₁₋₆ alkyl group, a C₃₋₆ cycloalkyl group, or a C₃₋₆ cycloalkoxy group.

Item 62: The compound of Item 61 or a pharmaceutically acceptable salt thereof, wherein R^{12a} is a hydrogen atom.

Item 63: The compound of Item 61 or a pharmaceutically acceptable salt thereof, wherein R^{12a} is a halogen atom or a C₁₋₆ alkyl group.

Item 64: The compound of Item 63 or a pharmaceutically acceptable salt thereof, wherein R^{12a} is a halogen atom.

Item 65: The compound of Item 63 or a pharmaceutically acceptable salt thereof, wherein R^{12a} is a C₁₋₆ alkyl group.

Item 66: The compound of Item 60 or a pharmaceutically acceptable salt thereof, wherein R^{12a} is a cyano group.

Item 67: The compound of Item 58 or Item 59 or a pharmaceutically acceptable salt thereof, wherein R^{12a}, R^{12b} and R^{12c} attach to the benzene ring at the substitution positions of the following formula:

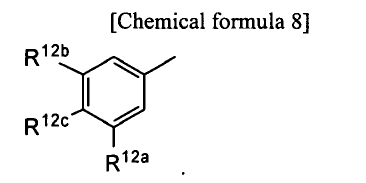

Item 68: The compound of Item 67 or a pharmaceutically acceptable salt thereof, wherein R^{12a} is a C₃₋₆ cycloalkoxy group.

Item 69: The compound of any one of Items 58 to 68 or a pharmaceutically acceptable salt thereof, wherein R^{12b} is a C₁₋₆ alkoxy group substituted by one group selected from C₁₋₄ alkoxy, carboxy, mono- C₁₋₆ alkylcarbonylamino and mono- C₁₋₆ alkoxycarbonylamino.

Item 70: The compound of Item 69 or a pharmaceutically acceptable salt thereof, wherein R^{12b} is a C₁₋₆ alkoxy group substituted by C₁₋₄ alkoxy.

Item 71: The compound of Item 70 or a pharmaceutically acceptable salt thereof, wherein R^{12b} is a 3-methoxypropyloxy group.

Item 72: The compound of any one of Items 58 to 71 or a pharmaceutically acceptable salt thereof, wherein R^{12c} is a C₁₋₆ alkoxy group.

Item 73: The compound of any one of Items 58 to 72 or a pharmaceutically acceptable salt thereof, wherein R²² is a C₁₋₆ alkyl group having optionally a C₃₋₆ cycloalkyl substituent, or a C₃₋₆ cycloalkyl group.

Item 74: The compound of Item 73 or a pharmaceutically acceptable salt thereof, wherein R²² is isopropyl group.

Item 75: The compound of any one of Items 58 to 74 or a pharmaceutically acceptable salt thereof, wherein R^{32a} is a group of the formula: -A²-B² (in which A² is a single bond, and B² is a hydrogen atom);
R^{32b} is a group of the formula: -A²-B² (in which A² is a single bond, -(CH₂)ₛ₂O-, -(CH₂)ₛ₂N(R⁴²)-, -(CH₂)ₛ₂COO-, -(CH₂)ₛ₂N(R⁴²)CO-, -(CH₂)ₛ₂N(R⁴²)SO₂, -(CH₂)ₛ₂N(R⁴²)COO-, -(CH₂)ₛ₂OCON(R⁴²)-, -(CH₂)ₛ₂N(R⁴²)CON(R⁴²)-, or -(CH₂)ₛ₂CON(R⁴²)-, and B² is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- or 6-membered monocyclic or polycyclic heteroaryl-C₁₋₄ alkyl group, or an optionally substituted 5- or 6-membered saturated heterocyclic group),
R^{32c} is a group of the formula: -A²-B² (in which A² is a single bond, -(CH₂)ₛ₂O-, or - (CH₂)ₛ₂COO-, and B² is a hydrogen atom, an optionally substituted C₁₋₆ alkyl group, an optionally substituted C₆₋₁₀ aryl group, an optionally substituted C₇₋₁₄ aralkyl group, or an optionally substituted 5-to 10-membered monocyclic or polycyclic heteroaryl group),
R^{32d} is a group of the formula: -A²-B² (in which A² is a single bond, -(CH₂)ₛ₂O-, - (CH₂)ₛ₂N(R⁴²)-, -(CH₂)ₛ₂COO-, -(CH₂)ₛ₂N(R⁴²)CO-, -(CH₂)ₛ₂N(R⁴²)SO₂-, -(CH₂)ₛ₂N(R⁴²)COO-, -(CH₂)ₛ₂OCON(R⁴²)-, -(CH₂)ₛ₂N(R⁴²)CON(R⁴²)-, or -(CH₂)ₛ₂CON(R⁴²)-, and B² is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- or 6-membered monocyclic or polycyclic heteroaryl-C₁₋₄ alkyl group, or an optionally substituted 5- or 6-membered saturated heterocyclic group), or one of R^{32b}, R^{32c} and R^{32d} is a hydrogen atom, and the remaining two groups combine each other to form a fused ring with a piperidine ring.

Item 76: The compound of any one of Items 58 to 74 or a pharmaceutically acceptable salt thereof, wherein R^{32a}, R^{32c} and R^{32d} are independently a group of the formula: -A²-B² (in which A² is a single bond, and B² is a hydrogen atom);
R^{32b} is a group of the formula: -A²-B² (in which A² is a single bond, -(CH₂)ₛ₂O-, -(CH₂)ₛ₂N(R⁴²)-, -(CH₂)ₛ₂COO-, -(CH₂)ₛ₂N(R⁴²)CO-, -(CH₂)ₛ₂N(R⁴²)SO₂-, -(CH₂)ₛ₂N(R⁴²)COO-, -(CH₂)ₛ₂OCON(R⁴²)-, -(CH₂)ₛ2N(R⁴²)CON(R⁴²)-, or -(CH₂)ₛ₂CON(R⁴²)-, and B² is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group).

Item 77: The compound of Item 76 or a pharmaceutically acceptable salt thereof, wherein B² for R^{32b} is an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group.

Item 78: The compound of any one of Items 58 to 74 or a pharmaceutically acceptable salt thereof, wherein R^{32a}, R^{32b} and R^{32d} are independently a group of the formula: -A²-B² (in which A² is a single bond, and B² is a hydrogen atom),
R^{32c} is a group of the formula: -A²-B² (in which when A² is a single bond, then B² is a hydrogen atom, an optionally substituted C₆₋₁₀ aryl group, or an optionally substituted 5- to 10-membered monocyclic or polycyclic heteroaryl group, and when A² is -(CH₂)ₛ₂O- or -(CH₂)ₛ₂COO-, then B² is a hydrogen atom, an optionally substituted C₁₋₆ alkyl group, an optionally substituted C₆₋₁₀ aryl group, or an optionally substituted C₇₋₁₄ aralkyl group).

Item 79: The compound of Item 78 or a pharmaceutically acceptable salt thereof, wherein when for R^{32c}, A² is a single bond, then B² is an optionally substituted C₆₋₁₀ aryl group, or an optionally substituted 5- to 10-membered monocyclic or polycyclic heteroaryl group.

Item 80: The compound of Item 78 or a pharmaceutically acceptable salt thereof, wherein R^{32c} is a group of the formula: -A²-B² (in which when A² is a single bond, then B² is a hydrogen atom, and when A² is -(CH₂)ₛ₂O- or -(CH₂)ₛ₂COO-, then B² is a hydrogen atom, an optionally substituted C₁₋₆ alkyl group, or an optionally substituted C₇₋₁₄ aralkyl group).

Item 81: The compound of Item 78 or a pharmaceutically acceptable salt thereof, wherein R^{32c} is a group of the formula: -A²-B² (in which A² is a single bond, and B² is an optionally substituted C₆₋₁₀ aryl group).

Item 82: The compound of any one of Items 58 to 74 or a pharmaceutically acceptable salt thereof, wherein R^{32a}, R^{32b} and R^{32c} are independently a group of the formula: -A²-B² (in which A² is a single bond, and B² is a hydrogen atom);
R^{32d} is a group of the formula: -A²-B² (in which A² is a single bond, -(CH₂)ₛ₂O-, - (CH₂)ₛ₂N(R⁴²)-, -(CH₂)ₛ₂COO-, -(CH₂)ₛ₂N(R⁴²)CO-, -(CH₂)ₛ₂N(R⁴²)SO₂-, -(CH₂)ₛ₂N(R⁴²)COO-, -(CH₂)ₛ₂OCON(R⁴²)-, -(CH₂)ₛ₂N(R⁴²)CON(R⁴²)-, or -(CH₂)ₛ₂CON(R⁴²)-, B² is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- to 10-membered monocyclic or polycyclic heteroaryl-C₁₋₄ alkyl group, or an optionally substituted 5- or 6-membered saturated heterocyclic group).

Item 83: The compound of Item 82 or a pharmaceutically acceptable salt thereof, wherein A² is -(CH₂)ₛ₂N(R⁴²)CO-.

Item 84: The compound of Item 82 or a pharmaceutically acceptable salt thereof, wherein for R^{32d}, A² is -(CH₂)ₛ₂N(R₄₂)CO-, -(CH₂)ₛ₂N(R⁴²)SO₂-, or -(CH₂)ₛ₂N(R⁴²)COO-; B² is an optionally substituted C₁₋₆ alkyl group, an optionally substituted C₂₋₆ alkenyl group, an optionally substituted C₆₋₁₀ aryl group, an optionally substituted C₇₋₁₄ aralkyl group, or an optionally substituted 5- to 10-membered monocyclic or polycyclic heteroaryl-C₁₋₄ alkyl group; and R⁴² is a C₃₋₆ cycloalkyl group.

Item 85: The compound of any one of Items 82 to 84 or a pharmaceutically acceptable salt thereof, wherein B² is a C₁₋₆ alkyl group optionally substituted by one of the members selected from C₃₋₆ cycloalkyl, hydroxy, C₁₋₄ alkoxy, carboxy, C₁₋₄ alkoxycarbonyl, di-(C₁₋₆ alkyl)amino, and aminocarbonyl having optionally a mono- or di-(C₁₋₆ alkyl) substituent; a C₂₋₆ alkenyl group; a C₆₋₁₀ aryl group optionally substituted by the same or different 1 to 3 groups selected from halogen, C₆ aryl and C₆ aryloxy; a C₇₋₁₄ aralkyl group optionally substituted by the same or different 1 to 3 groups selected from halogen, cyano, C₁₋₄ alkyl having optionally 1 to 3 halogen substituents, hydroxy, C₁₋₄ alkoxy, carboxy, C₁₋₄ alkoxycarbonyl, aminocarbonyl, and C₁₋₄ alkylsulfonylamino; or a 5- to 10-membered monocyclic or polycyclic heteroaryl-C₁₋₄ alkyl group.

Item 86: The compound of any of Items 58 to 83 or a pharmaceutically acceptable salt thereof, wherein R⁴² is a hydrogen atom, a C₁₋₆ alkyl group, a C₃₋₆ cycloalkyl group, or a C₇₋₁₄ aralkyl group.

Item 87: The compound of Item 86 or a pharmaceutically acceptable salt thereof, wherein R⁴² is a C₃₋₆ cycloalkyl group.

Item 88: The compound of any one of Items 58 to 74 or a pharmaceutically acceptable salt thereof, wherein R^{32a}, R^{32b}, R^{32c} and R^{32d} are independently a group of the formula: -A²-B² (in which A² is a single bond, and B² is a hydrogen atom).

Item 89: A compound of the following formula (III) or a pharmaceutically acceptable salt thereof.

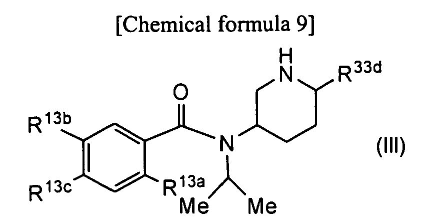

[wherein R^{13a} is a hydrogen atom, a halogen atom, a cyano group, a C₁₋₆ alkyl group having optionally a halogen substituent, a C₃₋₆ cycloalkyl group, or a C₃₋₆ cycloalkoxy group;
R^{13b} is a C₁₋₆ alkoxy group substituted by one of the members selected from C₁₋₄ alkoxy, carboxy, mono-C₁₋₆ alkylcarbonylamino and mono-C₁₋₆ alkoxycarbonylamino,
R^{13c} is a C₁₋₆ alkoxy group, or R^{13a} is a hydrogen atom; R^{13b} and R^{13c} combine each other to form a condensed ring with the benzene ring containing at least one heteroatom;
R^{33d} is a group of the formula: -A³-B³ (in which A³ is -(CH₂)ₛ₃N(R⁴³)-, -(CH₂)ₛ₃N(R⁴³)CO-, -(CH₂)ₛ₃N(R⁴³)SO₂-, -(CH₂)ₛ₃N(R⁴³)COO-, -(CH₂)ₛ₃OCON(R⁴³)-, -(CH₂)ₛ₃CON(R⁴³)-, -(CH₂)ₛ₃N(R⁴³)CON(H)-, or -(CH₂)ₛ₃SO₂N(R⁴³)-, B³ is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted 5- to 10-membered monocyclic or polycyclic heteroaryl-C₁₋₄ alkyl group, or an optionally substituted saturated heterocyclic group (provided that when A³ is -(CH₂)ₛ₃N(R⁴³)-, -(CH₂)ₛ₃OCON(R⁴³)-, -(CH₂)ₛ₃CON(R⁴³)-, or -(CH₂)ₛ₃SO₂N(R⁴³)-, then R⁴³ and B³ combine each other to form a ring)); R⁴³ is a C₃₋₆ cycloalkyl group; and s3 is 0, 1 or 2].

Item 90: The compound of Item 89 or a pharmaceutically acceptable salt thereof, wherein R^{13a} is a hydrogen atom.

Item 91: The compound of Item 89 or a pharmaceutically acceptable salt thereof, wherein R^{13a} is a halogen atom.

Item 92: The compound of Item 89 or a pharmaceutically acceptable salt thereof, wherein R^{13a} is a C₁₋₆ alkyl group having optionally a halogen substituent.

Item 93: The compound of Item 89 or a pharmaceutically acceptable salt thereof, wherein R^{13a} is a cyano group.

Item 94: The compound of any one of Items 89 to 93 or a pharmaceutically acceptable salt thereof, wherein R^{13b} is a C₁₋₆ alkoxy group having a C₁₋₄ alkoxy substituent.

Item 95: The compound of Item 94 or a pharmaceutically acceptable salt thereof, wherein R^{13b} is a 3-propylpropyloxy group.

Item 96: The compound of Item 89 or a pharmaceutically acceptable salt thereof, wherein the condensed ring is a condensed ring having a partial structure of the following formula:

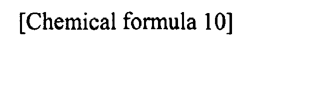

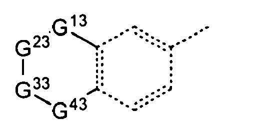

[wherein G¹³ is -N(R^{13d})-, G²³ is -CO-, G³³ is -C(R^{13e})(R^{13f})-, and G⁴³ is an oxygen atom or a sulfur atom; R^{13d} is a C₁₋₆ alkyl group optionally substituted by C₁₋₆ alkoxy or mono-C₁₋₆ alkoxycarbonylamino; R^{13e} and R^{13f} are independently the same or different and each is a hydrogen atom; a halogen atom; a hydroxy group; a cyano group; a C₃₋₆ cycloalkyl group; a C₁₋₆ alkyl group optionally substituted by C₁₋₄ alkoxy, C₃₋₆ cycloalkyl, or C₇₋₁₀ aralkyloxy; a C₆₋₁₀ aryl group having optionally a halogen substituent; a C₇₋₁₄ aralkyl group having optionally a halogen substituent; an aminocarbonyl group having optionally a mono- or di-(C₁₋₆ alkyl) substituent; or a 5- to 10-membered monocyclic or polycyclic heteroaryl group having optionally a C₁₋₄ alkyl substituent, provided that the ring expressed with dashed line(s) in said chemical structure means a benzene ring].

Item 97: The compound of Item 96 or a pharmaceutically acceptable salt thereof, wherein G⁴³ is an oxygen atom.

Item 98: The compound of Item 96 or a pharmaceutically acceptable salt thereof, wherein G⁴³ is a sulfur atom.

Item 99: The compound of any one of Items 96 to 98 or a pharmaceutically acceptable salt thereof, wherein R^{13d} is a 4-methoxybutyl group.

Item 100:The compound of any one of Items 96 to 98 or a pharmaceutically acceptable salt thereof, wherein R^{13d} is a methoxycarbonylaminoethyl group.

Item 101 :The compound of any one of Items 89 to 100 or a pharmaceutically acceptable salt thereof, wherein A³ is -(CH₂)ₛ₃N(R⁴³)CO-.

Item 102:The compound of any one of Items 89 to 101 or a pharmaceutically acceptable salt thereof, wherein S3 is 2.

Item 103:The compound of any one of Items 89 to 102 or a pharmaceutically acceptable salt thereof, wherein B³ is a C₁₋₆ alkyl group optionally substituted by one of the members selected from C₃₋₆ cycloalkyl, hydroxy, C₁₋₄ alkoxy, carboxy, C₁₋₄ alkoxycarbonyl, di-(C₁₋₆ alkyl)amino, and aminocarbonyl having optionally a mono- or di-(C₁₋₆ alkyl) substituent; a C₂₋₆ alkenyl group; a C₆₋₁₀ aryl group optionally substituted by the same or different 1 to 3 groups selected from halogen, C₆ aryl, and C₆ aryloxy; a C₇₋₁₄ aralkyl group optionally substituted by the same or different 1 to 3 groups selected from halogen, cyano, C₁₋₄ alkyl having optionally 1 to 3 halogen substituents, hydroxy, C₁₋₄ alkoxy, carboxy, C₁₋₄ alkoxycarbonyl, aminocarbonyl, and C₁₋₄ alkylsulfonylamino; or a 5- to 10-membered monocyclic or polycyclic heteroaryl-C₁₋₄ alkyl group.

Item 104:A medicament comprising as the active ingredient the compound as set forth in any one of Items 1 to 103 or a pharmaceutically acceptable salt thereof.

Item 105:A renin inhibitor comprising as the active ingredient the compound as set forth in any one of Items 1 to 103 or a pharmaceutically acceptable salt thereof.

Item 106:A therapeutic agent for hypertension comprising as the active ingredient the compound as set forth in any one of Items 1 to 103 or a pharmaceutically acceptable salt thereof.

Item 107:Use of the compound as set forth in any one of Items 1 to 103 or a pharmaceutically acceptable salt thereof in the preparation of a renin inhibitor.

Item 108:Use of the compound as set forth in any one of Items 1 to 103 or a pharmaceutically acceptable salt thereof in the preparation of a therapeutic agent for hypertension.

Item 109:A method for treatment of hypertension, which comprises administering an effect amount of the compound as set forth in any one of Items 1 to 103 or a pharmaceutically acceptable salt thereof to a patient in need.

Item 110:An intermediate compound of the following formula (VII) or a pharmaceutically acceptable salt thereof.

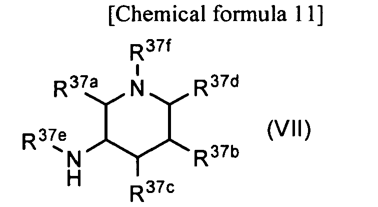

[wherein R^{37a}, R^{37b}, R^{37c} and R^{37d} are independently the same or different and each is a halogen atom, a cyano group, or a group of the formula: -A⁷-B⁷ (in which A⁷ is a single bond, -(CH₂)ₛ₇O-, -(CH₂)ₛ₇N(R⁴⁷)-, -(CH₂)ₛ₇SO₂-, -(CH₂)ₛ₇CO-, -(CH₂)ₛ₇COO-, -(CH₂)ₛ₇N(R⁴⁷)CO-, -(CH₂)ₛ₇N(R⁴⁷)SO₂-, -(CH₂)ₛ₇N(R⁴⁷)COO-, -(CH₂)ₛ₇OCON(R⁴⁷)-, -(CH₂)ₛ₇O-CO-, -(CH₂)ₛ₇CON(R⁴⁷)-, -(CH₂)ₛ₇N(R⁴⁷)CON(R⁴⁷)-, or -(CH₂)ₛ₇SO₂N(R⁴⁷)-, B⁷ is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted 5- to 10-membered monocyclic or polycyclic heteroaryl-C₁₋₄ alkyl group, or an optionally substituted saturated heterocyclic group (provided that when A⁷ is -(CH₂)ₛ₇N(R⁴⁷)-, - (CH₂)ₛ₇OCON(R⁴⁷)-, -(CH₂)ₛ₇CON(R⁴⁷)-, -(CH₂)ₛ₇N(R⁴⁷)CON(R⁴⁷)-, or -(CH₂)ₛ₇SO₂N(R⁴⁷)-, then R⁴⁷ and B⁷ combine each other to form a ring));
R⁴⁷ is a hydrogen atom, an optionally substituted C₁₋₆ alkyl group, an optionally substituted C₃₋₁₀ cycloalkyl group, an optionally substituted C₆₋₁₀ aryl group, an optionally substituted C₇₋₁₄ aralkyl group, or an optionally substituted 5- to 10-membered monocyclic or polycyclic heteroaryl group;
s7 is 0, 1 or 2 (provided that when A⁷ is -(CH₂)ₛ₇N(R⁴⁷)-, then s7 is 0 or 2, and when A⁷ is - (CH₂)ₛ₇CON(R⁴⁷)-, then s7 is 1 or 2);
R^{37e} is isopropyl group or cyclopropyl group;
R^{37f} is a hydrogen atom, a C₁₋₄ alkoxycarbonyl group, a phenyloxycarbonyl group, an allyloxycarbonyl group, a p-toluenesulfonyl group, or a nitrobenzenesulfonyl group (provided that when R^{37e} is a cyclopropyl group, and R^{37f} is a t-butoxycarbonyl group, then (i) the compound where R^{37a}, R^{37b} and R^{37d} are independently a group of the formula: - A⁷-B⁷ (in which A⁷ is a single bond, B⁷ is a hydrogen atom); R^{37c} is a group of the formula: -A⁷-B⁷ (in which A⁷ is a single bond, and B⁷ is a C₆ aryl group), and
   (ii) the compound where R^{37a}, R^{37b}, R^{37c}, and R^{37d} are independently a group of the formula: -A⁷-B⁷(in which A⁷ is a single bond, and B⁷ is a hydrogen atom) are excluded].

Item 111: The compound of Item 110 or a pharmaceutically acceptable salt thereof, wherein R^{37e} is an isopropyl group.

Item 112: The compound of Item 110 or a pharmaceutically acceptable salt thereof, wherein R^{37e} is a cyclopropyl group.

Item 113: The compound of any one of Items 110 to 112 or a pharmaceutically acceptable salt thereof, wherein R^{37f} is a hydrogen atom.

Item 114:The compound of any one of Items 100 to 112 or a pharmaceutically acceptable salt thereof, wherein R^{37f} is a C₁₋₄ alkoxycarbonyl group, or a phenyloxycarbonyl group.

Item 115: The compound of any one of Items 110 to 114 or a pharmaceutically acceptable salt thereof, wherein R^{37a}, R^{37b} and R^{37d} are independently a group of the formula: -A⁷-B⁷ (in which A⁷ is a single bond, and B⁷ is a hydrogen atom); R^{37e} is a group of the formula: -A⁷-B⁷(in which A⁷ is a single bond, -(CH₂)ₛ₇O-, or -(CH₂)ₛ₇COO-, B⁷ is a hydrogen atom, an optionally substituted C₆₋₁₀ aryl group, an optionally substituted C₇₋₁₄ aralkyl group, or an optionally substituted 5- or 6-membered monocyclic or polycyclic heteroaryl group).

Item 116: The compound of any one of Items 110 to 114, or a pharmaceutically acceptable salt thereof, wherein R^{37a}, R^{37c} and R^{37d} are independently a group of the formula: -A⁷-B⁷ (in which A⁷ is a single bond, and B⁷ is a hydrogen atom); R^{37b} is a group of the formula: -A⁷-B⁷(in which A⁷ is a single bond, -(CH₂)ₛ₇O-, -(CH₂)ₛ₇N(R⁴⁷)-, -(CH₂)ₛ₇COO-, -(CH₂)ₛ₇N(R⁴⁷)CO-, -(CH₂)ₛ₇N(R⁴⁷)SO₂-, -(CH₂)ₛ₇N(R⁴⁷)COO-, -(CH₂)ₛ₇OCON(R⁴⁷)-, or -(CH₂)ₛ₇CON(R⁴⁷)-, B⁷ is a hydrogen atom, an optionally substituted C₁₋₆ alkyl group, an optionally substituted C₆₋₁₀ aryl group, an optionally substituted C₇₋₁₄ aralkyl group, or an optionally substituted 5- or 6-membered saturated heterocyclic group).

Item 117: The compound of any one of Items 110 to 114, or a pharmaceutically acceptable salt thereof, wherein R^{37a}, R^{37b} and R^{37c} are independently a group of the formula: -A⁷-B⁷ (in which A⁷ is a single bond, and B⁷ is a hydrogen atom); R^{37d} is a group of the formula: -A⁷-B⁷(in which A⁷ is a single bond, -(CH₂)ₛ₇O-, -(CH₂)ₛ₇N(R⁴⁷)-, -(CH₂)ₛ₇COO-, -(CH₂)ₛ₇N(R⁴⁷)CO-, -(CH₂)ₛ₇N(R⁴⁷)SO₂-, -(CH₂)ₛ₇N(R⁴⁷)COO-, -(CH₂)ₛ₇OCON(R⁴⁷)-, or -(CH₂)ₛ₇CON(R⁴⁷)-, B⁷ is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- or 6-membered monocyclic or polycyclic heteroaryl group, an optionally substituted 5- or 6-membered monocyclic or polycyclic heteroaryl-C₁₋₄ alkyl group, or an optionally substituted 5- or 6-membered saturated heterocyclic group).

Item 118: The compound of Item 117 or a pharmaceutically acceptable salt thereof, wherein B⁷ for R^{37d} is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, or an optionally substituted 5- or 6-membered saturated heterocyclic group.

Item 119: The compound of any one of Items 110 to 114 or a pharmaceutically acceptable salt thereof, wherein R^{37a}, R^{37b}, R^{37c} and R^{37d} are independently a group of the formula: -A⁷-B⁷ (in which A⁷ is a single bond, and B⁷ is a hydrogen atom).

Hereinafter, the compound of the formula (I) or a pharmaceutically acceptable salt thereof are occasionally referred to as "the compound of the present invention".

### [EFFECTS OF INVENTION]

The compound of the present invention shows an excellent renin inhibitory activity and is useful as a therapeutic agent for hypertension.

### [BEST MODE FOR CARRYING OUT THE INVENTION]

Hereinafter, the present invention will be illustrated in more detail. In the present specification, the number of the carbon atom in definitions for substituents may be expressed, for example, as "C₁₋₆". For example, the expression of "C₁₋₆ alkyl" is the same as the expression of an alkyl group having 1 to 6 carbon atoms. In addition, in the present specification, any group without a term of "an optionally substituted" or "substituted" means a "unsubstituted" group. For example, "C₁₋₆ alkyl" means a "unsubstituted" one.

The term "group" in the present specification means a monovalent group unless specified otherwise. For example, the "alkyl group" means a monovalent saturated hydrocarbon group. In addition, in the explanation of each group in the present specification, the term "group" may be omitted. Further, the number of the substituents as defined with "an optionally substituted" or "substituted" is not necessarily limited, and it can be either one or more as long as the substitution can be possible. The definition for each group is also applied to cases where said group is a part of other groups, unless indicated specifically.

The "halogen atom" is, for example, fluorine atom, chlorine atom, bromine atom or iodine atom.

The "C₁₋₆ alkyl group" means a straight or branched chain saturated hydrocarbon group having 1 to 6 carbon atoms, and preferable one is a C₁₋₄ alkyl group. Examples of the C₁₋₆ alkyl group are methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, etc.

The "C₁₋₆ alkyl group" for "B" includes a group where a C₂₋₄ ring is formed on the 1-carbon atom of the saturated hydrocarbon group, for example, groups of the following formulae are exemplified:

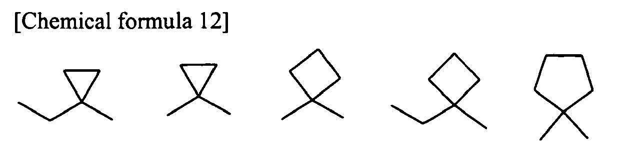

The "C₂₋₆ alkenyl group" means a straight or branched chain unsaturated hydrocarbon group having 2 to 6 carbon atoms and one double bond, and includes, for example, vinyl, propenyl, methylpropenyl, butenyl or methylbutenyl, etc.

The "C₂₋₆ alkynyl group" means a straight chain or branched chain unsaturated hydrocarbon group having 2 to 6 carbon atoms and one triple bond, and includes, for example, ethynyl, 1-propinyl, 2-propinyl, 2-butynyl, pentynyl or hexynyl, etc.

The "C₃₋₁₀ cycloalkyl group" means a saturated cyclic hydrocarbon group having 3 to 10 carbon atoms, and a preferable one is a C₃₋₆ cycloalkyl group. Examples of the "C₃₋₁₀ cycloalkyl group" are cyclopropyl, cyclobutyl, cyclopentyl, cyclohexyl, cycloheptyl, adamantyl or norbornyl, etc.

The "C₃₋₁₀ cycloalkyl group" for "B" also includes a saturated bicyclic group. Examples thereof are the groups of the following formulae:

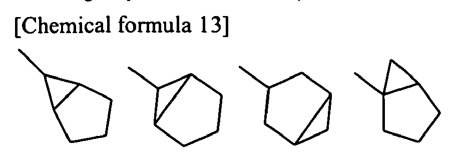

The "C₃₋₆ cycloalkyl-C₁₋₄ alkyl group" means a group where a C₃₋₆ cycloalkyl is attached to a C₁₋₄ alkyl. Examples thereof are cyclopropylmethyl, cyclobutylmethyl, cyclopentylmethyl, cyclohexylmethyl, etc.

The "C₅₋₆ cycloalkenyl group" means a cyclic unsaturated hydrocarbon group having a double bond, and includes, for example, 1-cyclopentenyl, 1-cyclohexenyl, etc.

The "C₆₋₁₀ aryl group" means an aromatic hydrocarbon group having 6 to 10 carbon atoms. Preferable one is a C₆ aryl group (phenyl). Examples of the C₆₋₁₀ aryl group are phenyl, 1-naphthyl or 2-naphthyl, etc.

The "C₇₋₁₄ aralkyl group" means a C₆₋₁₀ aryl-C₁₋₄ alkyl group, which is a group where the above-mentioned alkyl group is attached to the above-mentioned aryl group. Preferable one is a C₇₋₁₀ aralkyl group (a C₆ aryl-C₁₋₄ alkyl group). Examples of the C₇₋₁₄ aralkyl group are benzyl, 2-phenylethyl, 1-phenylpropyl or 1-naphthylmethyl, etc.

The "heteroaryl group" includes, for example, a 5- to 10-membered monocyclic or polycyclic group, etc., and said group contains the same or different one or more heteroatoms (e.g., 1 to 4 heteroatoms) selected from nitrogen atom, sulfur atom and oxygen atom. Preferable one is, for example, a 5- or 6-membered monocyclic group containing one heteroatom selected from nitrogen atom, sulfur atom and oxygen atom. Examples of the heteroaryl group are pyrrolyl, thienyl, benzothienyl, benzofuranyl, benzoxazolyl, benzothiazolyl, furyl, oxazolyl, thiazolyl, isoxazolyl, imidazolyl, pyrazolyl, pyridyl, pyrazyl, pyrimidyl, pyridazinyl, quinolyl, isoquinolyl, triazolyl, triazinyl, tetrazolyl, indolyl, imidazo[1,2-a]pyridyl, dibenzofuranyl, benzimidazolyl, quinoxalyl, cinnolyl, quinazolyl, indazolyl, naphthyridyl, quinolinoly, or isoquinolinolyl, etc.

The "heteroaryl-C₁₋₄ alkyl group" means a group where the above-mentioned alkyl group is substituted to the above-mentioned heteroaryl group. The heteroaryl moiety of said group includes the same ones as exemplified as the above-mentioned heteroaryl group, for example, a 5- or 6-membered monocyclic heteroaryl-C₁₋₄ alkyl, such as 2-pyridylmethyl, etc.

The "C₁₋₆ alkyl moiety" of the "C₁₋₆ alkoxy group" is the same as the above-mentioned C₁₋₆ alkyl. Preferable one is a C₁₋₄ alkoxy group. Examples of the C₁₋₆ alkoxy group are methoxy, ethoxy, propoxy, isopropoxy, butoxy, isobutoxy, sec-butoxy, tert-butoxy, etc.

The "C₁₋₆ alkyl moiety" of the "C₁₋₆ alkylthio group" is the same as the above-mentioned C₁₋₆ alkyl. Preferable one is a C₁₋₄ alkylthio group. Examples of the C₁₋₆ alkylthio group are methylthio, ethylthio, propylthio, isopropylthio, butylthio, sec-butylthio, tert-butylthio, pentylthio or hexylthio, etc.

The "C₁₋₆ alkyl moiety" of the "C₁₋₆ alkylsulfinyl group" is the same as the above-mentioned C₁₋₆ alkyl. Preferable one is a C₁₋₄ alkylsulfinyl group. Examples of the C₁₋₆ alkylsulfinyl group are methylsulfinyl, ethylsulfinyl, propylsulfinyl, isopropylsulfinyl, butylsulfinyl, pentylsulfinyl or hexylsulfinyl, etc.

The "C₁₋₆ alkyl moiety" of the "C₁₋₆ alkylsulfonyl group" is the same as the above-mentioned C₁₋₆ alkyl. Preferable one is a C₁₋₄ alkylsulfonyl group. Examples of the C₁₋₆ alkylsulfonyl group are methylsulfonyl, ethylsulfonyl, propylsulfonyl, isopropylsulfonyl, butylsulfonyl, pentylsulfonyl or hexylsulfonyl, etc.

The "C₆₋₁₀ aryl moiety" of the "C₆₋₁₀ arylthio group" is the same as the above-mentioned C₆₋₁₀ aryl. Examples of the C₆₋₁₀ arylthio group are phenylthio, 1-naphthylthio or 2-naphthylthio, etc.

The "C₆₋₁₀ aryl moiety" of the "C₆₋₁₀ arylsulfinyl group" is the same as the above-mentioned C₆₋₁₀ aryl. Examples of the C₆₋₁₀ arylsulfinyl group are phenylsulfinyl, 1-naphthylsulfinyl or 2-naphthylsulfinyl, etc.

The "C₆₋₁₀ aryl moiety" of the "C₆₋₁₀ arylsulfonyl group" is the same as the above-mentioned C₆₋₁₀ aryl. Examples of the C₆₋₁₀ arylsulfonyl group are phenylsulfonyl, tosyl, 1-naphthylsulfonyl or 2-naphthylsulfonyl.

The "C₃₋₁₀ cycloalkyl moiety" of the "C₃₋₁₀ cycloalkoxy group" is the same as the above-mentioned C₃₋₁₀ cycloalkyl. Preferable one is a C₃₋₆ cycloalkoxy group, etc. Examples of the C₃₋₁₀ cycloalkoxy group are cyclopropyloxy, cyclobutyloxy, cyclopentyloxy, cyclohexyloxy, cycloheptyloxy, adamantyloxy or norbomyloxy, etc.

The "C₆₋₁₀ aryl moiety" of the "C₆₋₁₀ aryloxy group" is the same as the above-mentioned C₆₋₁₀ aryl. Preferable one is a C₆ aryloxy (phenyloxy). Examples of the C₆₋₁₀ aryloxy group are phenoxyl, 1-naphthyloxy or 2-naphthyloxy, etc.

The "C₇₋₁₄ aralkyloxy group" means a C₆₋₁₀ aryl-C₁₋₄ alkyloxy group. The "C₇₋₁₄ aralkyl moiety" is the same as the above-mentioned C₇₋₁₄ aralkyl. Preferable one is a C₆ aryl-C₁₋₄ alkyloxy group (phenyl-C₁₋₄ alkyloxy group). Examples of the C₇₋₁₄ aralkyloxy group are benzyloxy, phenethyloxy, naphthylmethyloxy, etc.

The "C₁₋₄ alkoxycarbonyl group" means a group where the above-mentioned "C₁₋₄ alkoxy group" is attached to a carbonyl group. Examples thereof are methoxycarbonyl, ethoxycarbonyl, propoxycarbonyl, 2-propoxycarbonyl or tert-butoxycarbonyl, etc.

The "C₃₋₆ cycloalkoxycarbonyl group" is a group where the above-mentioned "C₃₋₆ cycloalkoxy group" is attached to a carbonyl group. Examples of the C₃₋₆ cycloalkoxy moiety are ones as exemplified as the above-mentioned cycloalkoxy group.

The "C₁₋₄ alkylcarbonyl group" means a group where the above-mentioned "C₁₋₄ alkyl group" is attached to a carbonyl group. Examples thereof are acetyl, propionyl or butyryl, etc.

The "C₃₋₁₀ cycloalkylcarbonyl group" means a group where the above-mentioned "C₃₋₁₀ cycloalkyl group" is attached to a carbonyl group. Preferable one includes a C₃₋₆ cycloalkylcarbonyl group. Examples thereof are cyclopropylcarbonyl, cyclobutylcarbonyl, cyclopentylcarbonyl, cyclohexylcarbonyl, adamantylcarbonyl or norbomylcarbonyl, etc.

The "C₆₋₁₀ arylcarbonyl group" means a group where the above-mentioned "C₆₋₁₀ aryl group" is attached to a carbonyl group. The "C₆₋₁₀ aryl moiety" is the same as the above-mentioned "C₆₋₁₀ aryl group". Preferable one is a "C₆ arylcarbonyl group" (phenylcarbonyl group). Examples of the "C₆₋₁₀ arylcarbonyl group" are benzoyl, 1-naphthoyl or 2-naphthoyl, etc.

The "heteroarylcarbonyl group" means a group where the above-mentioned heteroaryl is attached to a carbonyl group. The "heteroaryl moiety" is the same as ones exemplified as the above-mentioned heteroaryl group. Examples thereof are 2-pyridylcarbonyl, etc.

The "C₁₋₄ alkyl moiety" of the "C₁₋₄ alkylcarbonyloxy group" is the same as the above-mentioned C₁₋₄ alkyl group. Examples thereof are methylcarbonyloxy, ethylcarbonyloxy, isopropylcarbonyloxy, etc.

The "C₃₋₆ cycloalkyl moiety" of the "C₃₋₆ cycloalkylcarbonyloxy group" is the same as ones exemplified as the above-mentioned C₃₋₆ cycloalkyl group. Examples thereof are cyclopropylcarbonyloxy, cyclobutylcarbonyloxy, cyclopentylcarbonyloxy, etc.

The "C₁₋₄ alkylcarbonylamino group" means a group where one of the above-mentioned "C₁₋₄ alkylcarbonyl group" is attached to an amino group. Examples thereof are methylcarbonylamino, ethylcarbonylamino, etc.

The "C₁₋₄ alkoxycarbonylamino group" means a group where one of the above-mentioned "C₁₋4 alkoxycarbonyl group" is attached to an amino group. Examples thereof are methoxycarbonylamino, ethoxycarbonylamino, etc.

The "C₁₋₄ alkylsulfonylamino group" means a group where one of the above-mentioned "C₁₋₄ alkylsulfonyl group" is attached to an amino group. Examples thereof are methylsulfonylamino, ethylsulfonylamino, etc.

The "saturated heterocyclic group" includes a 5- or 6-membered saturated heterocyclic group containing the same or different 1 to 3 heteroatoms selected from nitrogen atom, oxygen atom and sulfur atom. Said nitrogen atom, oxygen atom and sulfur atom are ring-forming atoms. Examples thereof are pyranyl, tetrahydrofuryl, pyrrolidinyl, imidazolidinyl, piperidinyl, morpholinyl, thiomorpholinyl, dioxothiomorpholinyl, hexamethyleneiminyl, oxazolidinyl, thiazolidinyl, imidazolidinyl, oxoimidazolidinyl, dioxoimidazolidinyl, oxooxazolidinyl, dioxooxazolidinyl, dioxothiazolidinyl, tetrahydrofuranyl, tetrahydropyridinyl, or 1,4-dihydropyrrolidine, etc. Said group does not bind at the ring-forming nitrogen atom. Namely, said group does not include a concept such as pyrrolidino group.

The above-mentioned "saturated heterocyclic group" may form a condensed ring with a 6-membered aromatic hydrocarbon or a 6-membered unsaturated heterocyclic group. For example, said "saturated heterocyclic group" includes a bicyclic 11- or 12-membered "saturated heterocyclic group" wherein the above mentioned 5- or 6-membere saturated heterocyclic group is condensed with a 6-membered aromatic hydrocarbon group or a 6-membered unsaturated heterocyclic group. The 6-membered aromatic hydrocarbon group includes benzene, etc. The 6-membered unsaturated heterocyclic group includes pyridine, pyrimidine, or pyridazine, etc. Examples thereof are dihydroindolyl, dihydroisoindolyl, dihydropurinyl, dihydrothiazolopyrimidinyl, dihydrobenzodioxanyl, isoindolinyl, indazolyl, pyrrolidinyl, tetrahydroquinolinyl, decahydroquinolinyl, tetrahydroisoquinolinyl, decahydroisoquinolinyl, tetrahydronaphthyridinyl or tetrahydropyridoazepinyl, etc.

The "optionally substituted amino group" includes an amino group, a mono-substituted amino group, a di-substituted amino group, or a 5- or 6-membered cyclic amino group.

The "mono-substituted amino group" includes an amino group substituted by one group selected from C₁₋₆ alkyl, C₃₋₆ cycloalkyl, C₃₋₆ cycloalkyl-C₁₋₄ alkyl and benzyl. The "C₁₋₆ alkyl", the "C₃₋₆ cycloalkyl" and the "C₃₋₆ cycloalkyl-C₁₋₄ alkyl" are the same as defined above.

Examples of the "amino group substituted by a mono-(C₁₋₆ alkyl)" are methylamino, ethylamino, etc.

Examples of the "amino group substituted by a mono-(C₃₋₆ cycloalkyl)" are cyclopropylamino, cyclobutylamino, cyclopentylamino, etc.

Examples of the "amino group substituted by a mono-(C₃₋₆ cycloalkyl-C₁₋₄ alkyl)" are cyclopropylmethylamino, cyclobutylmethylamino, cyclopentylmethylamino, etc.

The "di-substituted amino group" means an amino group substituted by the same or different 2 groups selected from C₁₋₆ alkyl, C₃₋₆ cycloalkyl, C₃₋₆ cycloalkyl-C₁₋₄ alkyl and benzyl. The C₁₋₆ alkyl, the C₃₋₆ cycloalkyl, the C₁₋₄ alkylcarbonyl and the C₃₋₆ cycloalkylcarbonyl are the same as defined above.

Examples of the "amino group substituted by di-C₁₋₆ alkyl" are dimethylamino, diethylamino, etc.

Examples of the "amino group substituted by di-(C₃₋₆ cycloalkyl)" are dicyclopropylamino, dicyclobutylamino, cyclodipentylamino, etc.

Examples of the "amino group substituted by a C₁₋₆ alkyl and a benzyl" are N-methyl-N-benzylamino, N-ethyl-N-benzylamino, etc.

Examples of the "amino group substituted by a C₃₋₆ cycloalkyl and a benzyl" are N-cyclopropyl-N-benzylamino, N-cyclopentyl-N-benzylamino, N-cyclohexyl-N-benzylamino, etc.

The "5- or 6-membered cyclic amino group" means a cyclic amino group consisting of 5 members or 6 members. Said group binds at the ring-forming nitrogen atom. Examples thereof are pyrrolidino, piperidino, morpholino, thiomorpholino, thiomorpholino oxide, thimorpholino dioxide, piperazino, etc. Said ring may be optionally substituted by a C₁₋₄ alkyl or hydroxy group.

The substituent of the "optionally substituted C₁₋₆ alkyl group" includes, for example,
(a) a halogen atom,
(b) a cyano group,
(c) a C₃₋₆ cycloalkyl group (said group being optionally substituted by halogen, hydroxy or C₁₋₄ alkoxy),
(d) a hydroxy group,
(e) a C₁₋₄ alkoxy group (being otionally substituted by fluorine),
(f) a C₃₋₆ cycloalkyloxy group,
(g) a C₆ aryloxy group (said group being optionally substituted by the same or different groups selected from halogen, cyano and C₁₋₄ alkoxy),
(h) a benzyloxy group,
(i) a formyl group,
(j) a C₁₋₄ alkylcarbonyl group,
(k) a C₃₋₆ cycloalkylcarbonyl group,
(l) a phenylcarbonyl group,
(m) a benzylcarbonyl group,
(n) a formylcarbonyloxy group,
(o) a C₁₋₄ alkylcarbonyloxy group,
(p) a C₃₋₆ cycloalkylcarbonyloxy group,
(q) a carboxyl group,
(r) a C₁₋₄ alkoxycarbonyl group,
(s) a C₃₋₆ cycloalkoxycarbonyl group,
(t) an amino group,
(u) a mono-substituted amino group (said group being substituted by (ul) C₁₋₆ alkyl, (u2) C₃₋₆ cycloalkyl, (u3) C₃₋₆ cycloalkyl-C₁₋₄ alkyl, or (u4) benzyl),
(v) a di-substituted amino group (said group being substituted by the same or different 2 groups selected from the above-mentioned (ul)∼(u4)),
(w) a 5- or 6-membered cyclic amino group,
(x) an optionally substituted aminocarbonyl group, or
(y) an optionally substituted aminocarbonyloxy group.
In addition, the amino for the above-mentioned (x) and (y) is the same as the above-mentioned (u), (v) and (w).

The above-mentioned "optionally substituted aminocarbonyl group" means a group where the "optionally substituted amino" is attached to a carbonyl group. The "substituted amino" includes a mono-substituted amino group, a di-substituted amino group or a 5- or 6-membered cyclic amino. The substituent of the mono- or di-substituted amino includes, for example, a C₁₋₆ alkyl, a C₃₋₆ cycloalkyl, a C₃₋₆ cycloalkyl-C₁₋₄ alkyl, or benzyl.

The "mono-substituted aminocarbonyl group" includes, for example, an aminocarbonyl group substituted by the above-mentioned mono-(C₁₋₆ alkyl), or an aminocarbonyl group substituted by mono-(C₃₋₆ cycloalkyl).

Examples of the aminocarbonyl group substituted by mono-(C₁₋₆ alkyl) are methylaminocarbonyl, ethylaminocarbonyl, etc.

Examples of the aminocarbonyl group substituted by mono-(C₃₋₆ cycloalkyl) are cyclopropylaminocarbonyl, cyclobutylaminocarbonyl, cyclopentylaminocarbonyl, etc.

The "di-substituted aminocarbonyl group" includes, for example, an aminocarbonyl group substituted by a C₁₋₆ alkyl and a C₁₋₆ alkyl, an aminocarbonyl group substituted by a C₁₋₆ alkyl and a C₃₋₆ cycloalkyl, or an aminocarbonyl group substituted by a C₁₋₆ alkyl and a benzyl.

Examples of the "aminocarbonyl group substituted by a C₁₋₆ alkyl and a C₁₋₆ alkyl" are N-methyl-N-methylaminocarbonyl, N-methyl-N-ethylaminocarbonyl, etc.

Examples of the "aminocarbonyl group substituted by a C₁₋₆ alkyl and a C₃₋₆ cycloalkyl" are N-methyl-N-cyclopropylcarbonylamino, N-methyl-N-cyclobutylcarbonylamino, N-methyl-N-cyclopentylcarbonylamino, etc.

Examples of the "aminocarbonyl group substituted by a C₁₋₆ alkyl and a benzyl" are N-methyl-N-benzylamino, N-ethyl-N-benzylamino, etc.

The "5- or 6-membered cyclic aminocarbonyl group" may be optionally substituted by a C₆₋₁₀ aryloxy. Examples thereof are 3-phenyloxypyrrolidinocarbonyl, etc.

The "optionally substituted aminocarbonyl moiety" of the above-mentioned optionally substituted aminocarbonyloxy group is the same as the above-mentioned optionally substituted aminocarbonyl group. Examples thereof are aminocarbonyloxy, etc.

The substituent of the optionally substituted C₁₋₆ alkyl group for R^{1a} is preferably a halogen atom.

The substituent of the optionally substituted C₁₋₆ alkyl group for R^{1c} is preferably a C₁₋₄ alkoxycarbonyl.

The substituent of the optionally substituted C₁₋₆ alkyl group for R² is preferably a halogen atom or a C₃₋₆ cycloalkyl group.

The substituent of the optionally substituted C₁₋₆ alkyl group for B is preferably the following groups:
(a2) a halogen atom,
(b2) a C₃₋₆ cycloalkyl (being optionally substituted by halogen, hydroxy or C₁₋₄ alkoxy),
(c2) a hydroxy group,
(d2) a C₁₋₄ alkoxy,
(e2) a C₃₋₆ cycloalkoxy,
(f2) a C₆ aryloxy (being optionally substituted by C₁₋₄ alkyl),
(g2) a carboxy,
(h2) a C₁₋₄ alkoxycarbonyl,
(i2) an amino (being optionally substituted by C₁₋₆ alkyl or benzyl), and
(j2) an aminocarbonyl (the amino moiety being optionally substituted by C₁₋₆ alkyl, C₃₋₆ cycloalkyl, or C₃₋₆ cycloalkyl-C₁₋₄ alkyl).

When B is a C₁₋₆ alkyl group substituted by mono-(C₁₋₆ alkyl)-substituted amino, the C₁₋₆ alkyl moiety of the amino moiety may optionally be substituted by a carbamoyl group, a mono- or di-(C₁₋₆ alkyl)aminocarbonyl, or a 5- or 6-membered cyclic aminocarbonyl (provided that A should be a single bond, and B should be an optionally substituted C₁₋₆ alkyl group (in which (C₁₋₆ alkyl)amino and the cyclic amino moiety are the same as defined above)). Examples of the C₁₋₆ alkyl of the above-mentioned amino moiety are the following compounds.

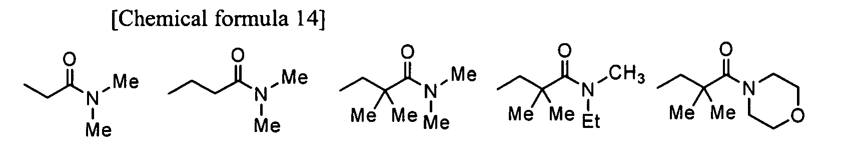

The substituents of the "optionally substituted C₁₋₆ alkyl group" for B may be optionally substituted by the same or different 1 to 3 groups selected from the above-mentioned group. For example, when A is a single bond, then said group may be optionally substituted by two groups of the above-mentioned (b2) and (j2). When A is a group other than a single bond, then said groups may be optionally substituted by two groups of the above-mentioned (b2) and (d2).

Examples thereof are the following groups.

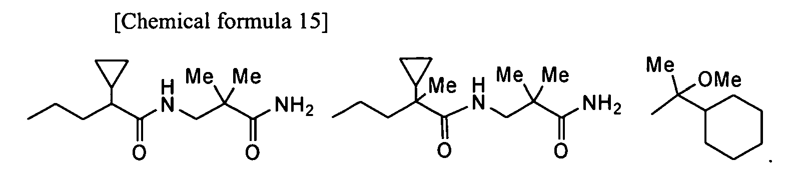

The substituents of the "optionally substituted C₁₋₆ alkoxy group" are, for example, a group selected from the above-mentioned (a) to (y) (in which the substituent of the mono-substituted amino group in (u) is restricted to a C₁₋₆ alkyl group and a C₃₋₆ cycloalkyl group, and the same as for (v), (x) and (y)).

The substituent of the "optionally substituted C₁₋₆ alkoxy group" for R^{1a} is preferably, for example, a halogen atom, a C₁₋₄ alkoxy or a C₃₋₆ cycloalkyl.

The substituent of the "substituted C₁₋₆ alkoxy group" for R^{1b} is preferably hydroxy, a C₁₋₄ alkoxy, trifluoromethyl, trifluoromethoxy, difluoromethoxy, carboxy, a mono- C₁₋₆ alkylcarbonylamino, or mono-C₁₋₆ alkoxycarbonylamino.

The substituent of the "optionally substituted C₁₋₆ alkoxy group" for R^{1c} is preferably a halogen atom, a C₃₋₆ cycloalkyl, a C₁₋₄ alkoxycarbonyl, a mono- or di-(C₁₋₆ alkyl)-substituted aminocarbonyl, or a 5- or 6-membered saturated heterocyclic group.

The substituent of the "optionally substituted C₂₋₆ alkenyl group" and the "optionally substituted C₂₋₆ alkynyl group" is, for example, a group selected from the above-mentioned (a) to (s).

The substituent of the "optionally substituted C₃₋₁₀ cycloalkyl group" and the "optionally substituted C₃₋₁₀ cycloalkyloxy group" is, for example, a group selected from the above-mentioned (x), a halogen atom, a C₁₋₄ alkyl and a C₆₋₁₀ aryl group (said aryl group being optionally substituted by a halogen atom, a C₁₋₄ alkyl, a hydroxy group, or a C₁₋₄ alkoxy).

The substituent of the "optionally substituted C₅₋₆ cycloalkenyl group" is, for example, a group selected from the above-mentioned (a) to (s) and a nitro group.

The substituent of the "optionally substituted C₁₋₄ alkylcarbonyl group" and the "optionally substituted C₃₋₁₀ cycloalkylcarbonyl group" is, for example, a group selected from the above-mentioned (a) to (h) and nitro group.

The substituent of the "optionally substituted C₁₋₆ alkylthio group", the "optionally substituted C₁₋₆ alkylsulfinyl group", the "optionally substituted C₁₋₆ alkylsulfonyl group" and the "optionally substituted C₁₋₄ alkoxycarbonyl group" is, for example, a group selected from a halogen atom, a hydroxy group, a nitro group, a cyano group, and the above-mentioned (d) to (h).

The substituent of the "optionally substituted C₆₋₁₀ aryl group" and the "optionally substituted 5- to 10-membered monocyclic or polycyclic heteroaryl group" is, for example, the same or different 1 to 3 groups selected from the following groups:
(a3) a halogen atom,
(b3) a cyano group,
(c3) a C₁₋₄ alkyl group,
(d3) a C₁₋₄ alkoxy group,
(e3) a C₆₋₁₀ aryl group (said group being optionally substituted by (e31) cyano, (e32) carboxy, (e33) C₁₋₄ alky optionally substituted by carboxy, or (e34) C₁₋₄ alkoxy optionally substituted by fluorine, hydroxy, or carboxy),
(f3) a C₆ aryloxy,
(g3) a C₇₋₁₀ aralkyloxy (being optionally substituted by C₁₋₄ alkoxy),
(h3) an aminocarbonyl (the amino moiety being optionally substituted by C₁₋₆ alkyl), and
(i3) a C₁₋₄ alkylsulfonylamino.

The substituent of the "optionally substituted C₆₋₁₀ aryl group" for B is preferably a group selected from the above-mentioned (a3), (b3), (c3), (d3), (e3), (f3), and (g3)

When A is a single bond, then B is preferably a C₆ aryl group substituted by a C₆ aryl (having optionally the same substituents as defined for the above-mentioned (e3)).

The substituent of the "optionally substituted C₆₋₁₀ aryloxy group" and the "optionally substituted 5- to 10-membered monocyclic or polycyclic heteroaryloxy group" includes, for example, the above-mentioned (a3) to (i3), etc.

The substituent of the aryl moiety of the "optionally substituted C₇₋₁₄ aralkyl group" is a group selected from the following groups:
(a4) a halogen atom,
(b4) a cyano group,
(c4) a C₁₋₄ alkyl group (being optionally substituted by 1 to 3 halogen atoms),
(d4) a hydroxy group,
(e4) a C₁₋₄ alkoxy group (being optionally substituted by 1 o 3 halogen atoms),
(f4) a carboxyl group,
(g4) a C₁₋₄ alkoxycarbonyl group,
(h4) a C₆ aryl group (being optionally substituted by C₁₋₄ alkoxy)
(i4) a C₆ aryloxy group,
(j4) a C₇₋₁₀ aralkyloxy group,
(k4) an aminocarbonyl group (the amino moiety being optionally substituted by C₁₋₆ alkyl),
(l4) a C₁₋₄ alkylsulfonylamino group, and
(m4) a C₁₋₄ alkylsulfonyl group.

The substituent of the above-mentioned (a4) to (m4) may substitute on the C₁₋₄ alkyl moiety of the C₇₋₁₄ aralkyl group (C₆₋₁₀ aryl-C₁₋₄ alkyl group).

The substituent of the "optionally substituted C₇₋₁₄ aralkyl group" for R^{1a} and R^{1c} is preferably (a4).

The substituent of the "optionally substituted C₇₋₁₄ aralkyl group" for B is preferably the same or different 1 to 3 groups selected from (a4), (b4), (c4), (d4), (e4), (f4), (g4), (k4) and (l4).

The substituent of the "optionally substituted C₇₋₁₄ aralkyloxy group" includes the above-mentioned (a4) to (m4), etc.

The substituent of the "optionally substituted C₇₋₁₄ aralkyloxy group" for R^{1a} includes (a4) and (k4), etc.

The "optionally substituted C₇₋₁₄ aralkyloxy group" for R^{1c} is preferably a C₇₋₁₀ aralkyloxy group.

The substituent of the heteroaryl moiety of the "optionally substituted 5- to 10-membered monocyclic or polycyclic heteroaryl-C₁₋₄ alkyl group" includes the same ones as exemplified as a substituent of the "optionally substituted 5- to 10-membered monocyclic or polycyclic heteroaryl group".

The "optionally substituted 5- to 10-membered monocyclic or polycyclic heteroaryl-C₁₋₄ alkyl group" for B is preferably a 5- or 6-membered monocyclic heteroaryl-C₁₋₄ alkyl group.

The substituent of the "saturated heterocyclic group" and the "saturated heterocycle" includes, for example, the following groups:
(a5) a halogen atom,
(b5) a hydroxy group,
(c5) a nitro group,
(d5) a cyano group,
(e5) a C₁₋₄ alkyl group (being optionally substituted by 1 to 3 halogen atoms),
(f5) a C₁₋₄ alkoxy group (being optionally substituted by 1 to 3 halogen atoms),
(g5) a carboxyl group,
(h5) a C₁₋₄ alkoxycarbonyl group,
(i5) a C₃₋₆ cycloalkoxycarbonyl group,
(j5) an amino group (being optionally substituted by C₁₋₄ alkyl),
(k5) a C₆ aryl group,
(l5) an aminocarbonyl group,
(m5) an oxo group, or
(n5) a thioxo group.
Said "saturated heterocyclic group" or "saturated heterocycle" may be optionally substituted by the same or different 2 substituents selected from the above substituents.

The "optionally substituted saturated heterocyclic group" for R^{1c} is preferably a 5- or 6-membered saturated heterocyclic group.

The "optionally substituted saturated heterocyclic group" for B is preferably, for example, a 5- or 6-membered saturated heterocyclic group containing the same or different 1 to 3 heteroatoms selected from nitrogen atom, oxygen atom and sulfur atom.

The phrase that R^{1a} is a hydrogen atom; R^{1b} and R^{1c} may combine each other to form a condensed ring with the benzene ring containing at least one heteroatom means that two of the above-mentioned definitions form a bicyclic condensed ring containing at least one heteroatom with the benzene ring to which said definitions attach. Herein, the "bicyclic condensed ring" includes a condensed ring of a 5-membered ring and a benzene ring, or a 6-membered ring and a benzene ring. The 5-membered ring and the 6-membered ring in the above-mentioned condensed ring are saturated ones, respectively. The heteroatom in said definitions is a nitrogen atom, an oxygen atom or a sulfur atom, and preferable one is a nitrogen atom or an oxygen atom, and may occasionally be the same or different two atoms.

The compounds wherein R^{1a} is a hydrogen atom; R^{1b} and R^{1c} may combine each other to form a condensed ring containing at least one heteroatom with the benzene ring includes compounds having a partial structure of the following formula:

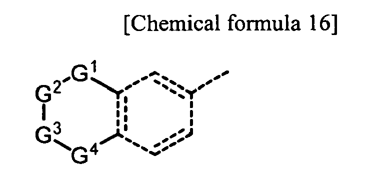

[wherein, (i) G¹ is -N(R^{1d})-, G² is -CO-, G³ is -C(R^{1c})(R^{1f})-, and G⁴ is -CH₂-, an oxygen atom, a sulfur atom, or does not exist at all,
(ii) G¹ is -N(R^{1d})-, G² is -CO-, G³ is -N(R^{1d})-, and G⁴ does not exist at all (R^{1d}s for G¹ and G³ are independent each other),
(iii) G¹ is an oxygen atom, G² is -CH₂-, G³ is an oxygen atom, and G⁴ does not exist at all, or
(iv) G¹ is an oxygen atom, G² is -CH₂-, G³ is -CH₂-, and G⁴ is an oxygen atom;
R^{1d} is a hydrogen atom, a C₆₋₁₀ aryl group, or a C₁₋₆ alkyl group optionally substituted by a group selected from hydroxy, C₃₋₆ cycloalkyl, C₁₋₆ alkoxy, C₃₋₆ cycloalkoxy, trifluoromethyl, trifluoromethoxy and mono-C₁₋₆ alkoxycarbonylamino;
R^{1e} and R^{1f} are independently the same or different and each is a hydrogen atom, a halogen atom, a hydroxy group, a carboxyl group, a cyano group, an optionally substituted C₁₋₆ alkyl group, an optionally substituted C₂₋₆ alkenyl group, an optionally substituted C₂₋₆ alkynyl group, an optionally substituted C₃₋₆ cycloalkyl group, an optionally substituted C₁₋₆ alkoxy group, an optionally substituted C₃₋₆ cycloalkoxy group, an optionally substituted amino group, an optionally substituted aminocarbonyl group, an optionally substituted C₁₋₄ alkoxycarbonyl group, an optionally substituted C₁₋₄ alkylcarbonyl group, an optionally substituted C₆₋₁₀ arylcarbonyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted C₆₋₁₀ aryloxy group, an optionally substituted 5- to 10-membered monocyclic or polycyclic heteroaryloxy group, or an optionally substituted C₇₋₁₄ aralkyloxy group, provided that the ring expressed with dashed line(s) in said chemical structure means a benzene ring]

Examples of R^{1e} and R^{1f} are, for example, one group selected from the following groups:
(a6) a hydrogen atom,
(b6) a halogen atom,
(c6) a hydroxy group,
(d6) a cyano group,
(e6) a C₃₋₆ cycloalkyl group,
(f6) a C₁₋₆ alkyl group (said group being optionally substituted by
   (f61) C₁₋₆ alkoxy,
   (f62) C₃₋₆ cycloalkyl,
   (f63) C₃₋₆ cycloalkyloxy,
   (f64) C₇₋₁₀ aralkyloxy, or
   (f65) aminocarbonyl (being optionally substituted by C₁₋₆ alkyl)),
(g6) a C₆₋₁₀ aryl group (being optionally substituted by halogen),
(h6) a C₇₋₁₄ aralkyl group (being optionally substituted by halogen),
(i6) an aminocarbonyl group (being optionally substituted by C₁₋₆ alkyl), and
(j6) a 5- to 10-membered monocyclic or polycyclic heteroaryl group (being optionally substituted by C₁₋₄ alkyl).
In addition, R^{1e} and R^{1f} are independently the above-mentioned groups.

The binding position of A in the "group of the formula: -A-B" is explained below. The "group of the formula: -A-B" means, for example, a group of the formula:
-(CH₂)ₛO-H when A is -(CH₂)ₛO-, and B is a hydrogen atom.

When A is -(CH₂)ₛN(R⁴)-, -(CH₂)ₛOCON(R⁴)-, -(CH₂)ₛCON(R⁴)-, -(CH₂)ₛN(R⁴)CON(R⁴)- or -(CH₂)ₛSO₂N(R⁴), the phrase that R⁴ and B combine each other to form a ring means that the N(R⁴)- moiety forms a 5- or 6-membered cyclic amino group. For example, A includes a group of the following formulae:

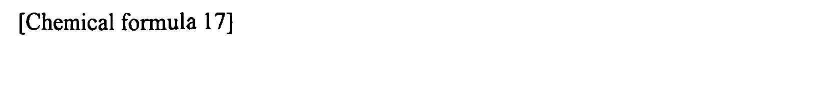

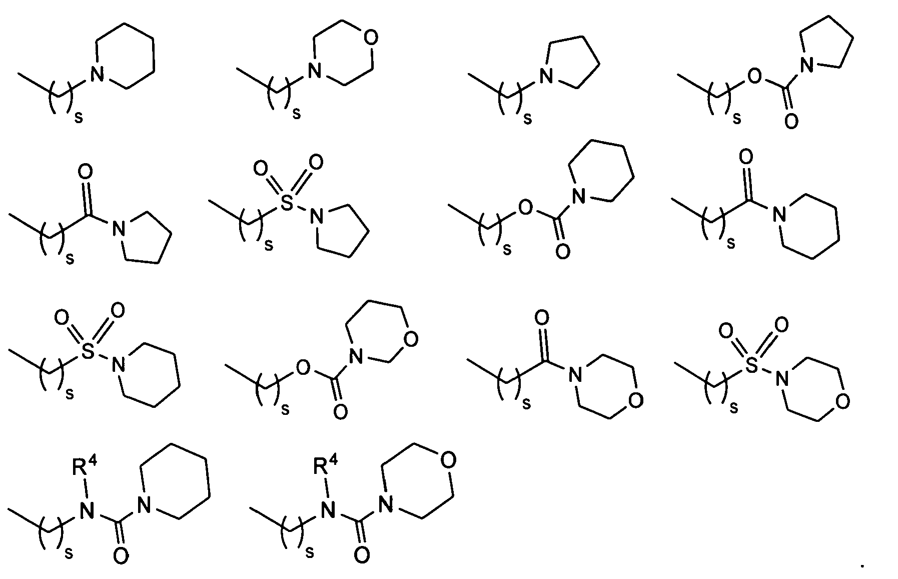

The above-mentioned cyclic amino groups are optionally substituted by the same substituents for the above-mentioned saturated heterocyclic group. Examples thereof are 4-hydroxypiperidino; 2-methoxymorpholino; 4-formylpiperidino; 4-methoxycarbonylpiperidino; 4-aminocarbonylpiperidino; 4-N-methylaminopiperidino; 3-phenylpyrrolidino; 4-dimethylaminopiperidino, etc.

The phrase that "two of R^{3a}, R^{3b}, R^{3c} and R^{3d} are a hydrogen atom, and the remaining two groups combine each other to form a fused ring with a hetero ring" means that the remaining two groups of the above-mentioned definitions form a fused ring with a hetero ring (pyrrolidine ring, piperidine ring, etc.) to which they attach. Said fused ring includes, for example, a bicyclic ring or a tricyclic ring containing one nitrogen atom and 5 to 10 carbon atoms, and preferably a bicyclic ring. Examples thereof are groups of the following structures:

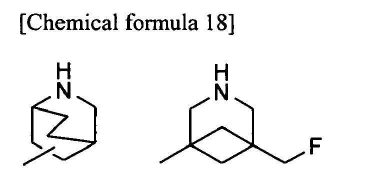

With respect to each substituent of the compound of the formula (I), more preferable other embodiments are illustrated hereinbelow.

Preferable groups for R^{1a}, R^{1b}, and R^{1c} for the following partial structure are as follows.

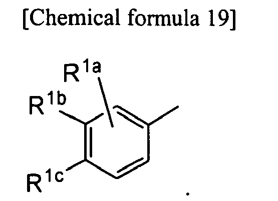

R^{1a} is a group selected from the following groups:
(a7) a hydrogen atom,
(b7) a halogen atom,
(c7) a hydroxy group,
(d7) a cyano group,
(e7) a C₁₋₆ alkyl group (being optionally substituted by halogen),
(f7) a C₂₋₆ alkenyl group,
(g7) a C₃₋₆ cycloalkyl group,
(h7) a C₁₋₆ alkoxy group (said group being optionally substituted by
   (h71) halogen,
   (h72) C₁₋₄ alkoxy, or
   (h73) C₃₋₆ cycloalkyl),
(i7) a C₃₋₆ cycloalkoxy group,
(j7) a C₆₋₁₀ aryl group,
(k7) a C₆ aryloxy group, and
(l7) a C₇₋₁₀ aralkyloxy group (said group being optionally substituted by
   (l71) halogen, or
   (l72) mono- or di-(C₁₋₆ alkyl)-substituted aminocarbonyl).

More preferable R^{1a} is a group selected from a hydrogen atom; a halogen atom; a hydroxy group; a cyano group; a C₁₋₆ alkyl group; a C₁₋₆ alkoxy group having optionally substituted by halogen, C₁₋₄ alkoxy or C₃₋₆ cycloalkyl; a C₃₋₆ cycloalkoxy group; a C₆ aryl group; and a C₇₋₁₀ aralkyloxy group optionally substituted by halogen or aminocarbonyl having a mono- or di-(C₁₋₆ alkyl) substituent.

Further preferable R^{1a} is a group selected from a hydrogen atom, a halogen atom, a hydroxy group, a cyano group and a C₁₋₆ alkyl group.

Especially preferable R^{1a} is a group selected from a hydrogen atom, a halogen atom, a cyano group and a C₁₋₆ alkyl group.

R^{1b} is a group selected from the following groups:
(a8) a C₁₋₆ alkyl group (said group being substituted by mono- C₁₋₆ alkoxycarbonylamino),
(b8) an amino group (said group being optionally substituted by C₁₋₆ alkyl having optionally a C₁₋₄ alkoxy substituent), and
(c8) a C₁₋₆ alkoxy group (said group being substituted by
   (c81) hydroxy,
   (c82) C₁₋₄ alkoxy,
   (c83) C₃₋₆ cycloalkoxy,
   (c84) trifluoromethyl,
   (c85) trifluoromethoxy,
   (c86) difluoromethoxy,
   (c87) carboxyl,
   (c88) C₁₋₄ alkoxycarbonylamino, or
   (c89) C₁₋₄ alkylcarbonylamino).

R^{1b} is preferably a C₁₋₆ alkoxy group being substituted by one group selected from C₁₋₄ alkoxy, carboxy, C₁₋₄ alkoxycarbonylamino, and C₁₋₄ alkylcarbonylamino.

More preferable R^{1b} is a C₁₋₆ alkoxy group being substituted by C₁₋₄ alkoxy or C₁₋₄ alkoxycarbonylamino.

Especially preferable R^{1b} is a C₁₋₄ alkoxy-substituted C₁₋₆ alkoxy group. Further, 3-methoxypropyloxy group is especially preferable.

R^{1c} is a group selected from the following groups:
(a9) a hydrogen atom,
(b9) a halogen atom,
(c9) a hydroxy group,
(d9) a cyano group,
(e9) a C₁₋₆ alkyl group (being optionally substituted by C₁₋₄ alkoxycarbonyl),
(f9) a C₇₋₁₀ aralkyl group,
(g9) a C₁₋₆ alkoxy group (said group being optionally substituted by
   (g 91) halogen,
   (g92) C₃₋₆ cycloalkyl,
   (g93) mono- or di-(C₁₋₆ alkyl)-substituted aminocarbonyl,
   (g94) 5- or 6-membered saturated heterocycle, or
   (g95) C₁₋₄ alkoxycarbonyl),
(h9) a C₃₋₆ cycloalkoxy group,
(i9) a C₆ aryloxy group,
(j9) a C₇₋₁₀ aralkyloxy group (being optionally substituted by mono- or di-(C₁₋₆ alkyl)aminocarbonyl), and
(k9) a 5- or 6-membered saturated heterocyclic group.

Further preferable R^{1c} is a hydrogen atom; a C₁₋₆ alkoxy group optionally substituted by halogen, C₃₋₆ cycloalkyl, mono- or di-(C₁₋₆ alkyl)-substituted aminocarbonyl, or 5- or 6-membered saturated heterocycle; a C₃₋₆ cycloalkoxy group; a C₆ aryloxy group; a C₇₋₁₀ aralkyloxy group; or a 5- or 6-membered saturated heterocyclic group.

The above-mentioned partial structure is preferably a partial structure selected from the following groups.

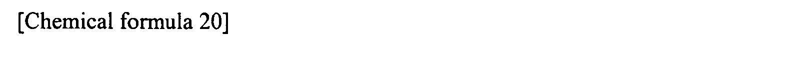

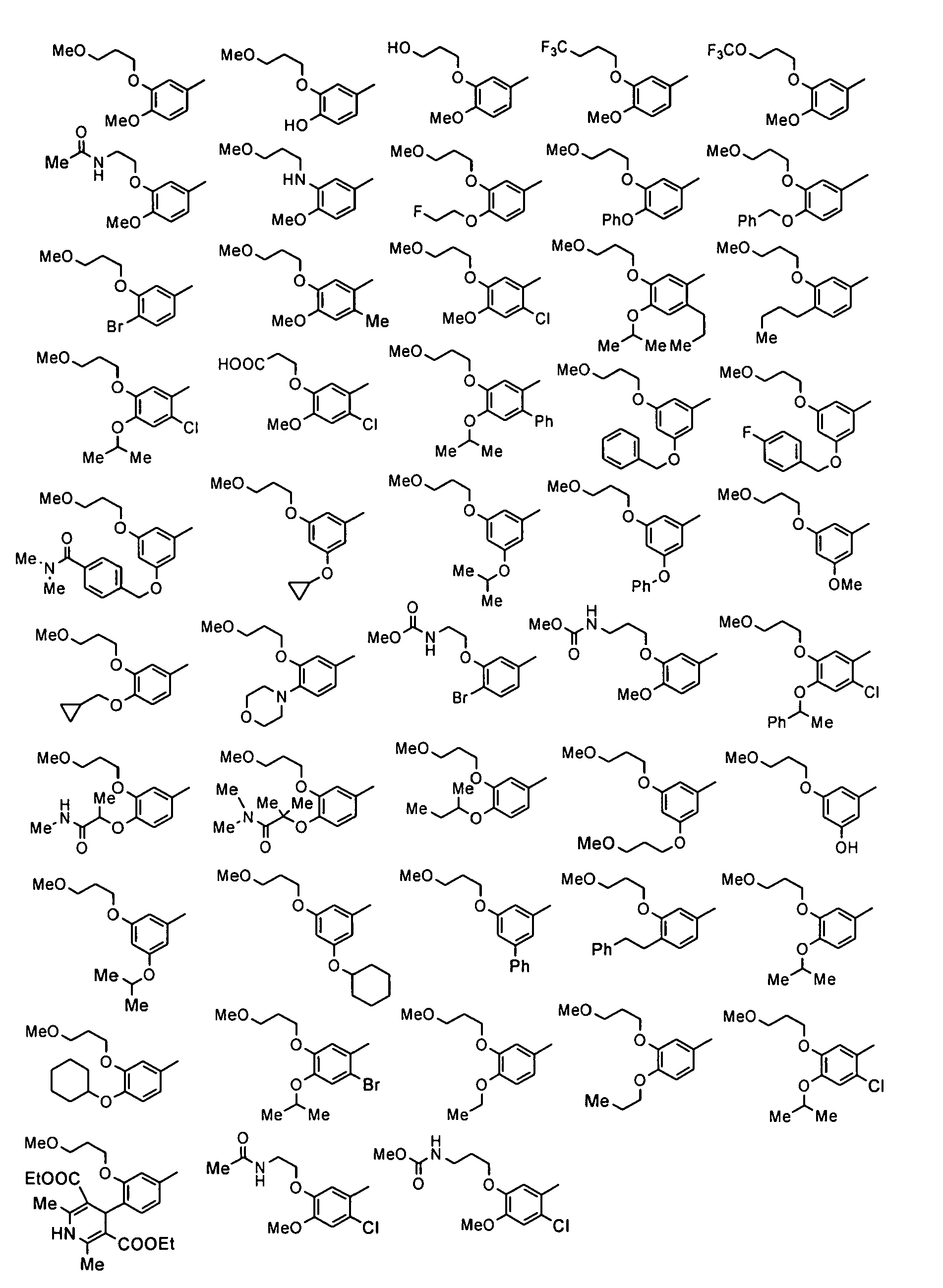

Especially preferable R^{1c} is a C₁₋₆ alkoxy group.

Preferable embodiment for the following partial structure is as follows.

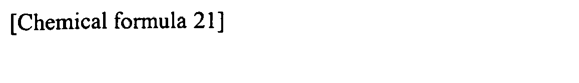

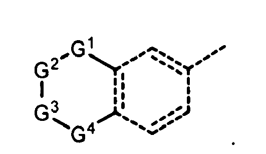

[wherein (i) G¹ is -N(R^{1d})-, G² is -CO-, G³ is -C(R^{1e})(R^{1f})-, and G⁴ is -CH₂-, oxygen atom, sulfur atom, or does not exist at all,
(ii) G¹ is -N(R^{1d})-, G² is -CO-, G³ is -N(R^{1d})-, and G⁴ does not exist at all (R^{1d} for G¹ and G³ are independent to each other),
(iii) G¹ is an oxygen atom, G² is -CH₂-, G³ is an oxygen atom, and G⁴ does not exist at all, or
(iv) G¹ is an oxygen atom, G² is -CH₂-, G³ is -CH₂-, and G⁴ is an oxygen atom;
R^{1d} is a hydrogen atom, a C₆₋₁₀ aryl group, or a C₁₋₆ alkyl group optionally substituted by a group selected from hydroxy, C₃₋₆ cycloalkyl, C₁₋₆ alkoxy, C₃₋₆ cycloalkoxy, trifluoromethyl, trifluoromethoxy and mono-C₁₋₆ alkoxycarbonylamino;
R^{1e} and R^{1f} are independently the same or different and each is a hydrogen atom; a halogen atom; a hydroxy group; a cyano group; a C₃₋₆ cycloalkyl group; a C₁₋₆ alkyl group optionally substituted by C₁₋₄ alkoxy, C₃₋₆ cycloalkyl, or C₇₋₁₀ aralkyloxy; a C₆₋₁₀ aryl group having optionally a halogen substituent; a C₇₋₁₄ aralkyl group having optionally a halogen substituent; an aminocarbonyl group optionally substituted by mono- or di-(C₁₋₆ alkyl); or a 5- to 10-membered monocyclic or polycyclic heteroaryl group optionally substituted by C₁₋₄ alkyl, provided that the ring expressed with dashed line(s) in said chemical structure means a benzene ring).

Examples of said definitions are groups as indicated by the following chemical structures, provided that the ring expressed with dashed line(s) in said chemical structure means a benzene ring, and the ring expressed with solid line(s) means a condensed ring formed by combining R^{1b} and R^{1c} with the above-mentioned benzene ring.

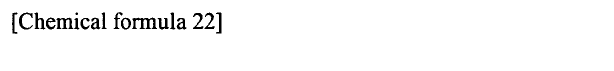

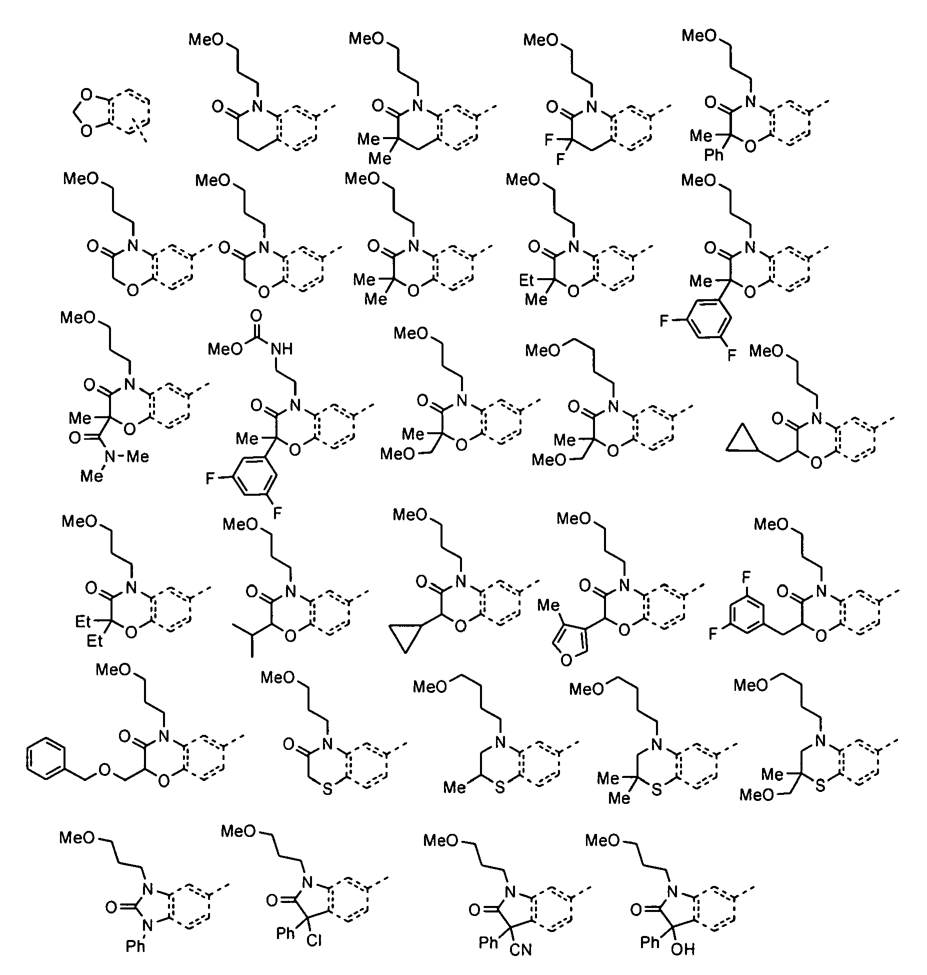

In the above-mentioned partial structure, R^{1d} is preferably a C₁₋₆ alkyl group optionally substituted by C₁₋₆ alkoxy or mono-C₁₋₆ alkoxycarbonylamino.

R^{1d} is preferably a C₁₋₆ alkyl group optionally substituted by C₁₋₆ alkoxy, and 4-methoxybutyl group is more preferable.

R^{1d} is preferably a C₁₋₆ alkyl group optionally substituted by mono-C₁₋₆ alkoxycarbonylamino, and methoxycarbonylaminoethyl group is more preferable.

The compounds having the above-mentioned partial structure wherein G¹ is -N(R^{1d})-; G²is -CO-; G³ is -C(R^{1e})(R^{1f})-; G⁴ is an oxygen atom or a sulfur atom; R^{1d} is a C₁₋₆ alkyl group substituted by C₁₋₆ alkoxy or mono-C₁₋₆ alkoxycarbonylamino; R^{1e} and R^{1f} are independently the same or different and each is a hydrogen atom, a C₁₋₆ alkyl group optionally substituted by C₁₋₆ alkoxy, a C₆₋₁₀ aryl group having optionally a halogen substituent, or an aminocarbonyl group being substituted by mono- or di-(C₁₋₆ alkyl) are preferable.

Preferable R² in the formula (I) is a group selected from the following groups:
(a10) a C₁₋₆ alkyl group (being optionally substituted by C₃₋₆ cycloalkyl),
(b10) a C₃₋₆ cycloalkyl group,
(c10) a C₆ aryl group (being optionally substituted by halogen), and
(d10) a C₇₋₁₀ aralkyl group (being optionally substituted by halogen).

R² is preferably a C₁₋₆ alkyl group, and isopropyl group is especially preferable.

Preferable B of the group of the formula: -A-B is a group selected from the following groups:
(a11) a hydrogen atom,
(b11) a C₁₋₆ alkyl group (being optionally substituted by a group selected from the above-mentioned (a2) to (j2)),
(c11) a C₂₋₆ alkenyl group,
(d11) a C₃₋₆ cycloalkyl group,
(e11) a C₆ aryl group (said group being optionally substituted by the same or different 1 to 3 groups selected from the above-mentioned (a3) to (i3)),
(f11) a C₇₋₁₄ aralkyl group (said group being optionally substituted by the same or different 1 to 3 groups selected from the above-mentioned (a4) to (m4)),
(g11) a 5- or 6-membered monocyclic heteroaryl group (being optionally substituted by halogen or C₆ aryl),
(h11) a 5- or 6-membered monocyclic heteroaryl-C₁₋₄ alkyl group (said group being optionally substituted by C₁₋₄ alkyl), and
(i11) a 5- or 6-membered saturated heterocyclic group.

More preferable B is a group selected from a hydrogen atom; a C₁₋₆ alkyl group (said group being optionally substituted by C₃₋₆ cycloalkyl, hydroxy, C₁₋₄ alkoxy, carboxy, C₁₋₄ alkoxycarbonyl, di-(C₁₋₆ alkyl)-substituted amino, or aminocarbonyl optionally substituted by mono- or di-(C₁₋₆ alkyl)); a C₂₋₆ alkenyl group; a C₆₋₁₀ aryl group (said group being optionally substituted by the same or different 1 to 3 groups selected from halogen, C₆ aryl, and C₆ aryloxy); a C₇₋₁₄ aralkyl group (said group being optionally substituted by the same or different 1 to 3 groups selected from halogen, cyano, C₁₋₄ alkyl having optionally 1 to 3 halogen substituents, hydroxy, C₁₋₄ alkoxy, carboxy, C₁₋₄ alkoxycarbonyl, aminocarbonyl, and C₁₋₄ alkylsulfonylamino); and 5- or 6-membered monocyclic heteroaryl-C₁₋₄ alkyl group.

Preferable R⁴ in the formula (I) is a group selected from a hydrogen atom, a C₁₋₆ alkyl group, a C₃₋₆ cycloalkyl group and a C₇ aralkyl group.

More preferable R⁴ is a group selected from a hydrogen atom and a C₃₋₆ cycloalkyl group, and especially preferable one is a C₃₋₆ cycloalkyl group.

The present compounds of the more preferable embodiments of the present invention include the compounds of the following formula.

(1) The compound of the following formula (Ia) or a pharmaceutically acceptable salt thereof.

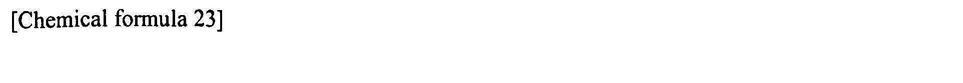

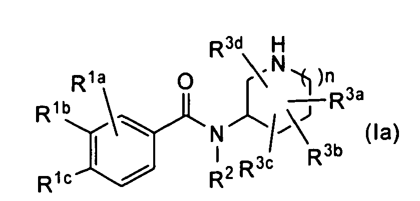

[wherein R^{1a}, R^{1b}, R^{1c}, R², R^{3a}, R^{3b}, R^{3e}, R^{3a} and n are as defined in Item 1, provided that in the formula (Ia), the compound of the following formula:

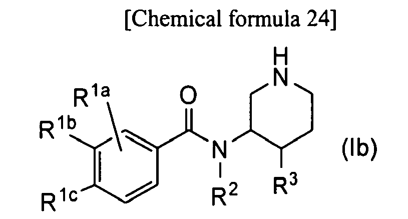

is limited to those wherein R^{1a}, R^{1b}, R^{1c} and R² are as defined in Item 1;
R³ is one of the above-mentioned R^{3a}, R^{3b}, R^{3c}, and R^{3d} (in which R^{3a}, R^{3b}, R^{3c} and R^{3d} are as defined in Item 1, provided that when R³ is a group of the formula: -A-B, then A is a single bond, and B is an optionally substituted C₆ aryl group, or an optionally substituted 5- to 10-membered monocyclic or polycyclic heteroaryl group. Said C₆ aryl group is substituted at the 3-position thereof by cyano, carboxy, C₁₋₄ alkyl having optionally a carboxy substituent, or a C₆ aryl group optionally substituted by a C₁₄ alkoxy optionally substituted by fluorine, hydroxy, or carboxy)]

The compounds of any one of Items 2 to 57 wherein the formula (I) is replaced by the formula (Ia) and a pharmaceutically acceptable salt thereof are also included in the present invention.

(2) The compound of the following formula (IIa) or a pharmaceutically acceptable salt thereof.

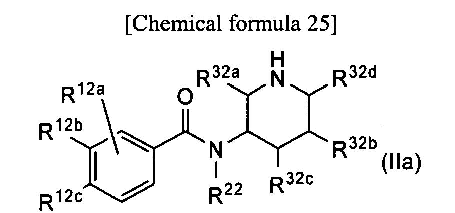

[wherein R^{12a}, R^{12b}, R^{12c}, R²², R^{32a}, R^{32b}, R^{32c} and R^{32d} are as defined in Item 58, provided that in the formula (IIa), the compound of the following formula:

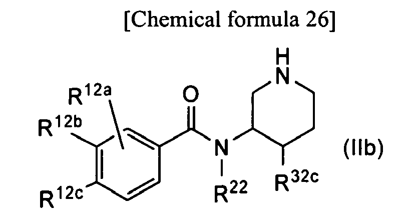

is limited to those wherein R^{12a}, R^{12b}, R^{12c} and R²² are as defined in Item 58, and R^{32c} is the same as R³ of the formula (Ib)].

The compounds of any one of Items 59 to 88 wherein the formula (II) is replaced by the formula (IIa) and a pharmaceutically acceptable salt thereof are also included in the present invention.

(3) The compound of the following formula (Ic) or a pharmaceutically acceptable salt thereof.

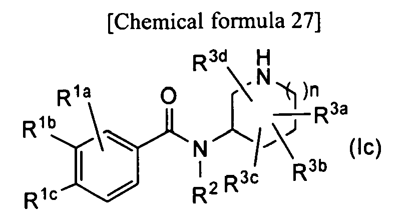

[wherein R^{1a}, R^{1b}, R^{1c}, R², R^{3a}, R^{3b}, R^{3c}, R^{3d} and n are as defined in Item 1, provided that in the formula (Ic), the compound of the following formula:

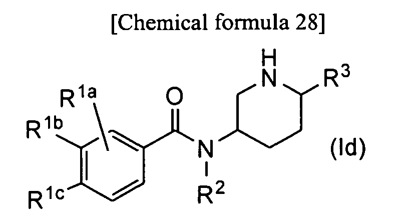

is limited to those wherein R^{1a}, R^{1b}, R^{1c} and R² are as defined in Item 1; R³ is one of the above-mentioned R^{3a}, R^{3b}, R^{3c}, and R^{3d} (in which R^{3a}, R^{3b}, R^{3c} and R^{3d} are as defined in the formula (I)),
provided that when R³ is a group of the formula: -A-B, then (i) A is -(CH₂)ₛN(R⁴)CO-, or -(CH₂)ₛN(R⁴)COO-; B is as defined in Item 1; R⁴ is an optionally substituted C₃₋₁₀ cycloalkyl group, an optionally substituted C₆₋₁₀ aryl group, or an optionally substituted 5- to 10-membered monocyclic or polycyclic heteroaryl group,
(ii) A is -(CH₂)ₛN(R⁴)-; B is as defined in Item 1; R⁴ is an optionally substituted C₃₋₁₀ cycloalkyl group, or an optionally substituted 5- to 10-membered monocyclic or polycyclic heteroaryl group,
(iii) A is -(CH₂)ₛN(R⁴)SO₂-, -(CH₂)ₛOCON(R⁴)- or -(CH₂)ₛSO₂N(R⁴)-; B is as defined in Item 1; R⁴ is an optionally substituted C₃₋₁₀ cycloalkyl group, or an optionally substituted 5- to 10-membered monocyclic or polycyclic heteroaryl group,
(iv) A is -(CH₂)ₛN(R⁴)CON(H)-; B is as defined in Item 1; R⁴ is an optionally substituted C₃₋₁₀ cycloalkyl group, or an optionally substituted 5- to 10-membered monocyclic or polycyclic heteroaryl group,
(v) A is -(CH₂)ₛCON(R⁴)-; B is as defined in Item 1; R⁴ is a C₁₋₆ alkyl group, an optionally substituted C₆₋₁₀ aryl group, or an optionally substituted C₇₋₁₄ aralkyl group,
(vi) A is -(CH₂)ₛSO₂-, -(CH₂)ₛCO-, or -(CH₂)ₛCOO-; B is a hydrogen atom, an optionally substituted C₃₋₁₀ cycloalkyl group, an optionally substituted C₅₋₆ cycloalkenyl group, or an optionally substituted C₆₋₁₀ aryl group, or
(vii) A is a single bond; B is a hydrogen atom, an optionally substituted C₃₋₁₀ cycloalkyl group, an optionally substituted C₅₋₆ cycloalkenyl group, an optionally substituted C₆₋₁₀ aryl group, an optionally substituted 5- to 10-membered monocyclic or polycyclic heteroaryl group, or an optionally substituted saturated heterocyclic group].

The compounds of any one of Item 2 to 57 wherein the formula (I) is replaced by the formula (Ic) or a pharmaceutically acceptable salt thereof are also included in the present invention.

(4) The compound of the following formula (IIc) or a pharmaceutically acceptable salt thereof.

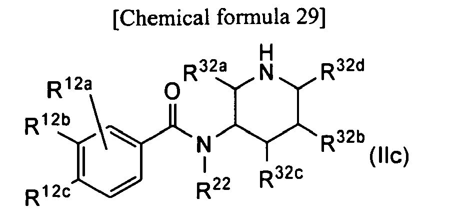

[0retinopathy]
[wherein R^{12a}, R^{12b}, R^{12c}, R²², R^{32a}, R^{32b}, R^{32c} and R^{32d} are as defined in Item 58, provided that in the formula (IIc), the compound of the following formula:

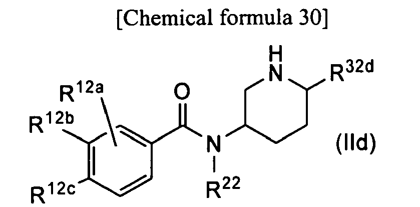

is limited to those wherein R^{12a}, R^{12b}, R^{12c} and R²² are as defined in Item 58; R^{32d} is as defined in the formula (II), provided that when R^{32d} is a group of the formula: -A²-B², then (i) A² is - (CH₂)ₛ₂N(R⁴²)CO-, or -(CH₂)ₛ₂N(R⁴²)COO-; B² is as defined in Item 58; R⁴² is an optionally substituted C₃₋₁₀ cycloalkyl group, an optionally substituted C₆₋₁₀ aryl group, or an optionally substituted 5- to 10-membered monocyclic or polycyclic heteroaryl group,
(ii) A² is -(CH₂)ₛ₂N(R⁴²)-; B² is as defined in Item 58; R⁴² is an optionally substituted C₃₋₁₀ cycloalkyl group, or an optionally substituted 5- to 10-membered monocyclic or polycyclic heteroaryl group,
(iii) A² is -(CH₂)ₛ₂N(R⁴²)SO₂-, -(CH₂)ₛ₂OCON(R⁴²)-, or -(CH₂)ₛ₂SO₂N(R⁴²)-; B² is as defined in Item 58; R⁴² is an optionally substituted C₃₋₁₀ cycloalkyl group, or an optionally substituted 5- to 10-membered monocyclic or polycyclic heteroaryl group,
(iv) A² is -(CH₂)ₛN(R⁴²)CON(H)-; B² is as defined in Item 58; R⁴² is an optionally substituted C₃₋₁₀ cycloalkyl group, or an optionally substituted 5- to 10-membered monocyclic or polycyclic heteroaryl group,
(v) A² is -(CH₂)ₛ₂CON(R⁴²)-; B² is as defined in Item 58; R⁴² is a C₁₋₆ alkyl group, an optionally substituted C₆₋₁₀ aryl group, or an optionally substituted C₇₋₁₄ aralkyl group,
(vi) A² is -(CH₂)ₛ₂SO₂-, -(CH₂)ₛ₂CO-, or -(CH₂)ₛ₂COO-; B² is a hydrogen atom, an optionally substituted C₃₋₁₀ cycloalkyl group, an optionally substituted C₅₋₆ cycloalkenyl group, or an optionally substituted C₆₋₁₀ aryl group, or
(vii) A² is a single bond; B² is a hydrogen atom, an optionally substituted C₃₋₁₀ cycloalkyl group, an optionally substituted C₅₋₆ cycloalkenyl group, an optionally substituted C₆₋₁₀ aryl group, an optionally substituted 5- to 10-membered monocyclic or polycyclic heteroaryl group, or an optionally substituted saturated heterocyclic group].

The present invention also includes the compounds disclosed in any one of Items 59 to Item 88, wherein the compound of the formula (II) is replaced by the compound of the formula (IIc) or a pharmaceutically acceptable salt thereof.

(5) The compound of the following formula (Ie), or a pharmaceutically acceptable salt thereof.

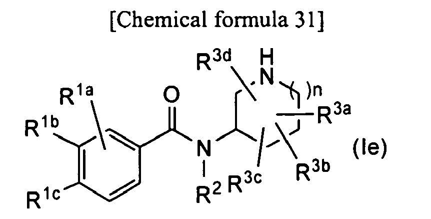

[wherein R^{1a}, R^{1b}, R^{1c}, R², R^{3a}, R^{3b}, R^{3c}, R^{3d} and n are defined in Item 1, provided that in the formula (Ie), the compounds of the formulae (Ib) and (Id) are limited to ones as disclosed above]

The present invention also includes the compounds as listed in Items 2 to Items 57, wherein the compound of the formula (I) is replaced by the compound of the formula (Ie).

(6) The compound of the following formula (IIe) or a pharmaceutically acceptable salt thereof.

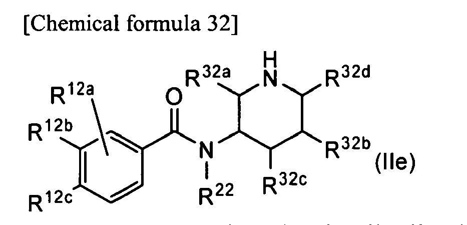

[wherein R^{12a}, R^{12b}, R^{12c}, R²², R^{32a}, R^{32b}, R^{32c} and R^{32d} are as defined in Item 58, provided that in the formula (IIe), the compounds of the formulae (IIb) and (IId) are limited to ones as disclosed above]

The present invention also includes the compound as disclosed in any one of Items 59 to 88, where the compound of the formula (II) is replaced by the compound of the formula (IIe), or a pharmaceutically acceptable salt thereof.

(7) The compound of the following formula (III), or a pharmaceutically acceptable salt thereof. 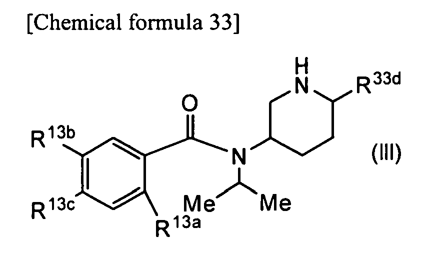 [wherein R^{13a}, R^{13b}, R^{13c} and R^{33d} are as defined in Item 89]

In the compound of the above-mentioned formula (III), the condensed ring is preferably a condensed ring having a partial structure of the following formula: 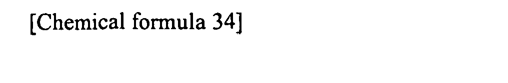 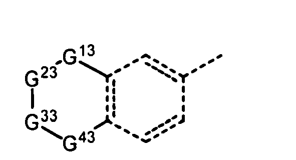 [wherein G¹³, G²³, G³³, and G⁴³ are as defined in Item 96].

The compound of the following formula (IIIa), or a pharmaceutically acceptable salt thereof. 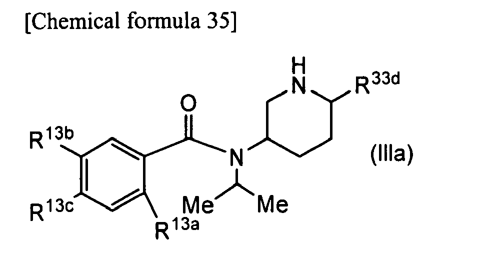 [wherein R^{13a} is a hydrogen atom, a halogen atom, a cyano group, a C₁₋₆ alkyl group, a C₃₋₆ cycloalkyl group, or a C₃₋₆ cycloalkoxy group; R^{13b} is a C₁₋₆ alkoxy group which is substituted by one group selected from C₁₋₄ alkoxy and mono-C₁₋₆ alkoxycarbonylamino; R^{13c} is a C₁₋₆ alkoxy group; R^{33d} is a group of the formula: -A³-B³ (A³ and B³ are as defined in Item 89)]

In the compound of the above-mentioned formula (IIIa), the compounds having a stereostructure of the following formula are preferable.

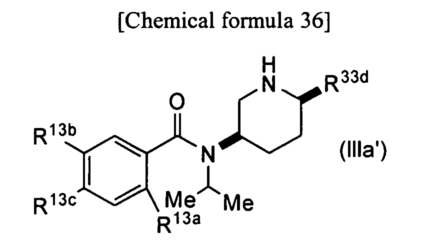

In the compounds of the above-mentioned formula (IIIa'), the compounds having a stereo structure of the following formula are preferable.

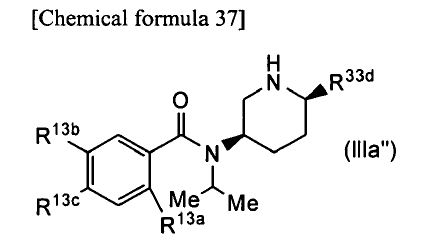

In the compounds of the formulae (IIIa), (IIIa') and (IIIa"), the preferable compounds are ones wherein R^{13a} is a hydrogen atom; R^{13b} is a C₁₋₆ alkoxy group substituted by C₁₋₄ alkoxy; R^{33d} is a group of the formula: -A³-B³ (in which A³ is -(CH₂)ₛ₃N(R⁴³)CO-, or -(CH₂)ₛ₃N(R⁴³)CON(H)-, B³ is an optionally substituted C₁₋₆ alkyl group, an optionally substituted C₂₋₆ alkenyl group, an optionally substituted C₆₋₁₀ aryl group, an optionally substituted C₇₋₁₄ aralkyl group, or an optionally substituted 5- or 6-membered monocyclic heteroaryl-C₁₋₄ alkyl group); R⁴³ is a C₃₋₆ cycloalkyl group; s3 is 1, or 2.

In the compounds of the formulae (IIIa) and (IIIa'), the preferable compounds are ones wherein R^{13a} is a hydrogen atom; R^{13b} is a C₁₋₆ alkoxy group substituted by C₁₋₄ alkoxy; when A³ for R^{33d} is - (CH₂)₂N(R⁴³)CO-, then B³ is an optionally substituted C₁₋₆ alkyl group, an optionally substituted C₂₋₆ alkenyl group, an optionally substituted C₇₋₁₄ aralkyl group, or an optionally substituted 5- or 6-membered monocyclic heteroaryl-C₁₋₄ alkyl group, or when A³ for R^{33d} is -(CH₂)₂N(R⁴³)CONH-, then B³ is an optionally substituted C₆₋₁₀ aryl group; R⁴³ is a C₃₋₆ cycloalkyl group; s3 is 1 or 2.

(8) The compound of the following formula (IIIb), or a pharmaceutically acceptable salt thereof. 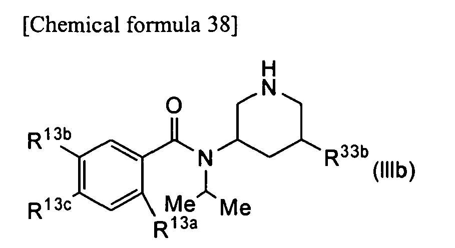 [wherein R^{13a}, R^{13b} and R^{13c} are as defined in Item 89; R^{33b} is a group of the formula:
-A²-B² (in which A² and B² are as defined in Item 58)]

In the compound of the above-mentioned formula (IIIb), the preferable compounds are ones having a stereo structure of the following formula:

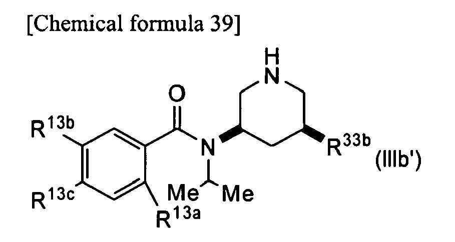

In the compounds of the above-mentioned formula (IIIb'), the preferable compounds are ones having a stereo structure of the following formula:

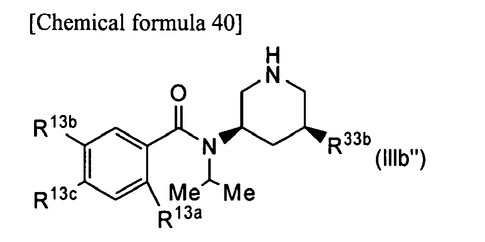

(9) The compound of the following formula (IIIc), or a pharmaceutically acceptable salt thereof. 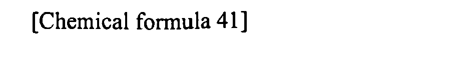 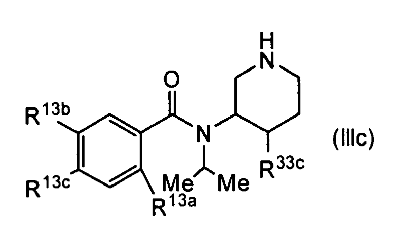 [wherein R^{13a}, R^{13b} and R^{13c} are as defined in Item 89; R^{33c} is a group of the formula:
-A²-B² (in which A² and B² are as defined in Item 58)]

In the compounds of the above-mentioned formula (IIIc), the preferable compounds are ones having a stereostructure of the following formula:

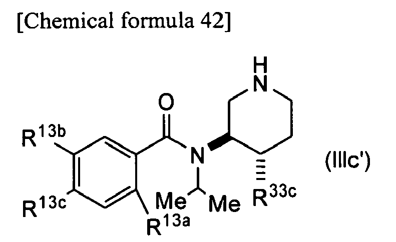

In the compounds of the above-mentioned formula (IIIc'), the preferable compounds are ones having a stereostructure of the following formula:

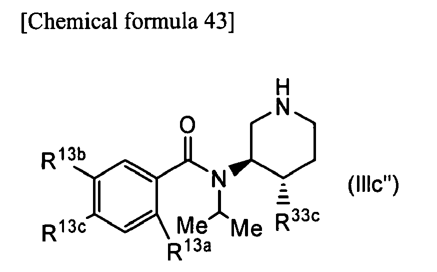

(10) The compounds of the following formula (IIId), or a pharmaceutically acceptable salt thereof. 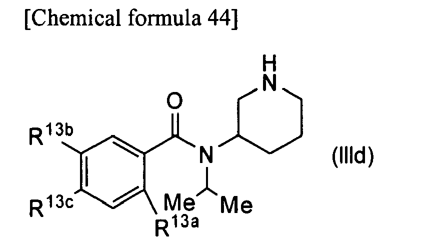 [wherein R^{13a}, R^{13b} and R^{13c} are as defined in Item 89]

(11) The compound of the following formula (IVa), or a pharmaceutically acceptable salt thereof. 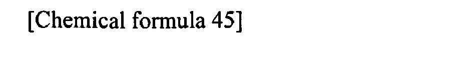 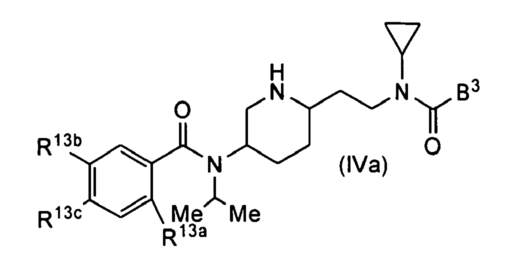 The definitions in the above formula are as defined in Item 89, and preferable embodiment for each definition is the same ones as defined in the compounds of the formulae (IIIa) and formula (IIIa').

(12) The compound of the following formula (IVb), or a pharmaceutically acceptable salt thereof. 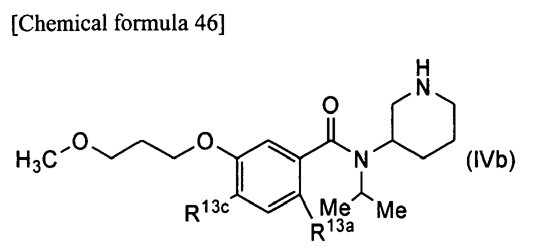 [wherein R^{13a} and R^{13c} are as defined in Item 89]

(13) The compound of the following formula (Va), or a pharmaceutically acceptable salt thereof. 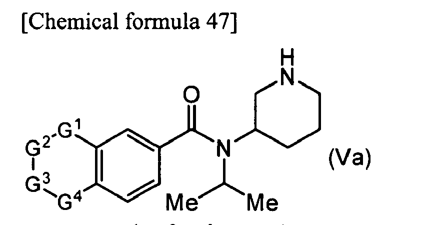 [wherein, G¹, G², G³ and G⁴ are as defined in Item 23]

(14) The compound of the following formula (Vb), or a pharmaceutically acceptable salt thereof. 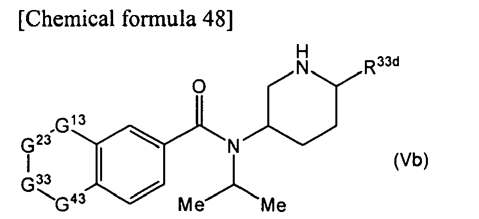 [wherein G¹³, G²³, G³³, G⁴³ are as defined in Item 96, and R^{33d} is the same as defined in Item 89]

(15) The compound of the following formula (Vc), or a pharmaceutically acceptable salt thereof. 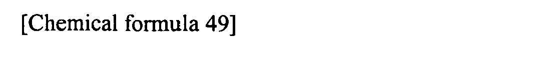 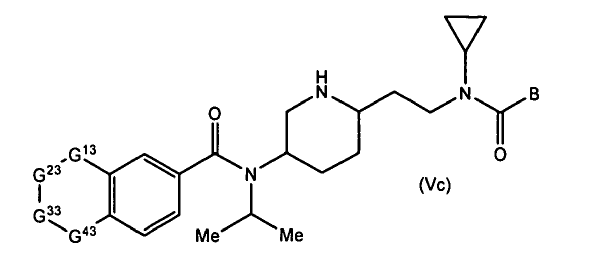 [wherein G¹³, G²³, G³³, and G⁴³ are as defined in Item 96, and B is as defined in Item 35]

In the compounds of the formulae (Vb) and (Vc), the preferable compounds are ones wherein G¹³, G²³, G³³, and G⁴³ are as defined above; R^{33d} is a group of the formula: -A³-B³ (in which A³ is - (CH₂)ₛ₃N(R⁴³)CO-, or -(CH₂)ₛ₃N(R⁴³)CON(H)-; B³ is an optionally substituted C₁₋₆ alkyl group, an optionally substituted C₂₋₆ alkenyl group, an optionally substituted C₆₋₁₀ aryl group, an optionally substituted C₇₋₁₄ aralkyl group, or an optionally substituted 5- or 6-membered monocyclic heteroaryl-C₁₋₄ alkyl group); R⁴³ is a C₃₋₆ cycloalkyl group; and s3 is 1, or 2.

(18) The compound of the following formula (Vd), or a pharmaceutically acceptable salt thereof. 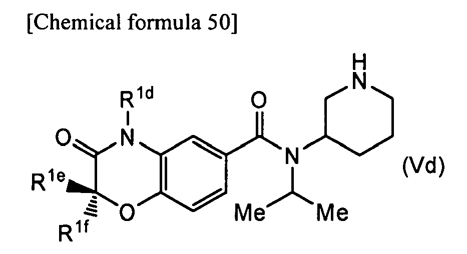 [wherein R^{1d}, R^{1e} and R^{1f} are as defined in Item 23]

Preferable embodiment for each definition of the above formula (Vd) is the same as one of the embodiments of Items 24 to 30.

(19) The compound of the following formula (Ve), or a pharmaceutically acceptable salt thereof.

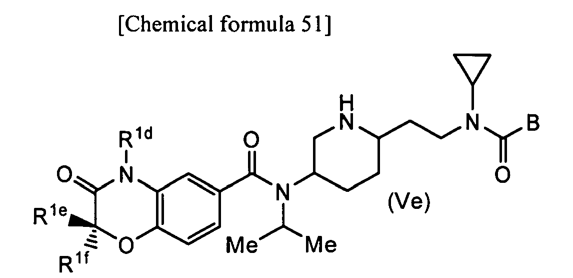

[wherein R^{1d}, R^{1e}, and R^{1f} are as defined in Item 23, and B is the same as defined in Item 35]

In the compounds of the formulae (Vd) and (Ve), the preferable compounds are ones wherein R^{1d} is a C₁₋₆ alkyl group substituted by C₁₋₆ alkoxy; R^{1e} and R^{1f} are independently the same or different and each is selected from a hydrogen atom and a C₁₋₆ alkyl group optionally substituted by C₁₋₆ alkoxy.

In the compounds as defined in the above-mentioned (1) to (19), the preferable embodiment for each substituents for "B", and the preferable embodiment for each substituent for "B²" and "B³" are the same as defined for the preferable embodiment of each substituent of the above "B".

The preferable compound of the intermediate of the above-mentioned formula (VII) are the following compounds. In addition, in those formulae, the concrete example of R^{37a}, R^{37b}, R^{37c} and R^{37d} are the same ones as the concrete examples of R^{3a}, R^{3b}, R^{3c} and R^{3d} in the formula (I). The preferable embodiment for each definition for "B⁷" is the same as the preferable embodiment of each definition for "B" and the preferable embodiment for each definition of "B²" and "B³".

R^{37f} of the formula (VII) may be a hydrogen atom, a protecting group being able to be removed by hydrogenation, or a protecting group being able to be removed under acidic conditions. The above-mentioned protecting group includes, for example, protecting groups disclosed in "PROTECTIVE GROUPS IN ORGANIC SYNTHESIS, John Willey and Sons, 4th edition", as far as said protecting group does not affect any harmful effects on the desired compound.

Concrete examples of said protecting groups are, for example, carbamate protecting groups (e.g., C₁₋₄ alkoxycarbonyl group such as t-butoxycarbonyl, etc., benzyloxycarbonyl group, allyloxycarbonyl group, etc.), amide protecting groups (e.g., trifluoroacetyl group, etc.), imide protecting groups (e.g., phthaloyl group, etc.), or sulfonamide protecting groups (e.g., p-toluenesulfonyl group, or 2-nitrobenzenesulfonyl group).

The protecting groups being able to be removed by hydrogenation or under acidic conditions are, for example, in addition to C₁₋₄ alkoxycarbonyl group, benzyloxycarbonyl group, allyloxycarbonyl group, p-toluenesulfonyl group, or nitrobenzenesulfonyl group, a group selected from the following compounds. In the following compounds, R^{5a} and R^{5b} are independently the same or different, and each is a hydrogen atom, a nitro group, a C₁₋₄ alkoxy group, or a C₁₋₄ alkylsulfinyl group.

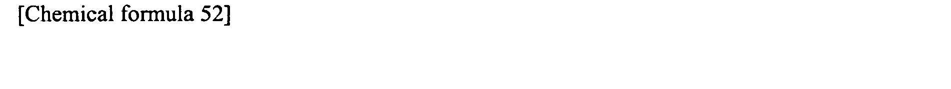

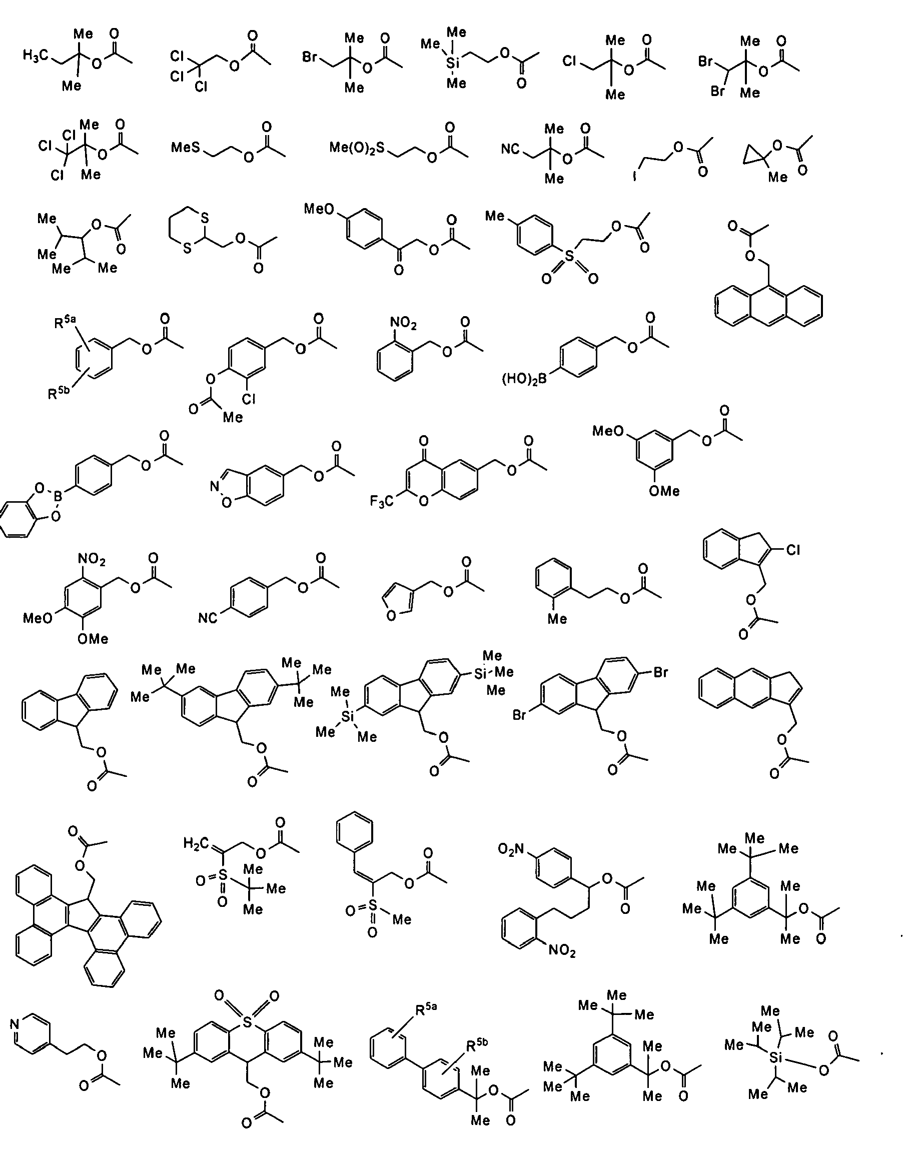

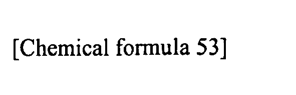

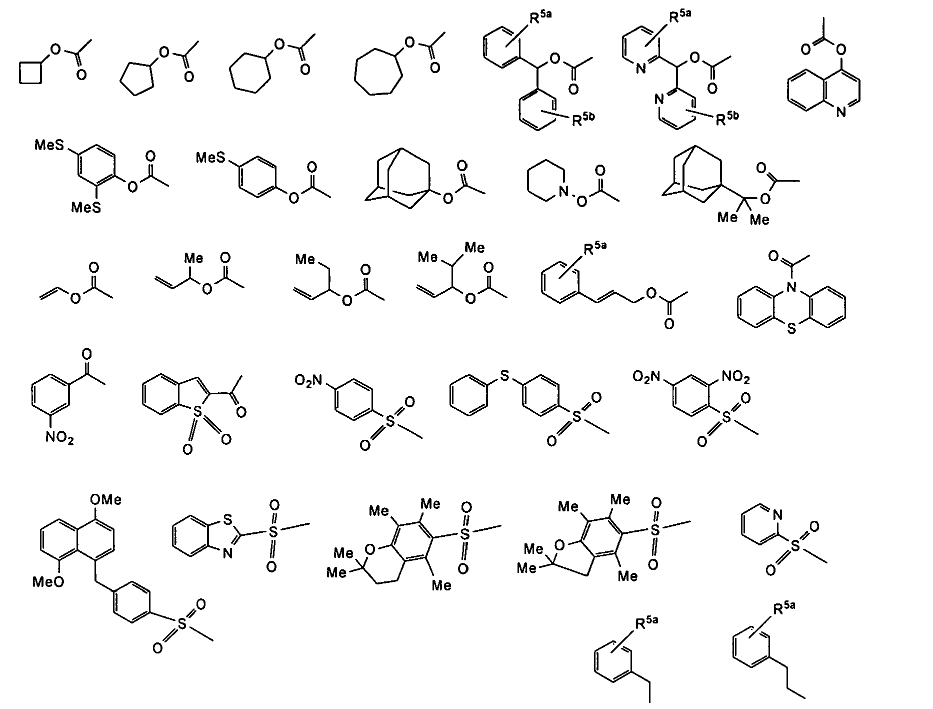

The preferable compound of the intermediate compound (VII) are listed below.
(1) The compound of the following formula (VIIa): 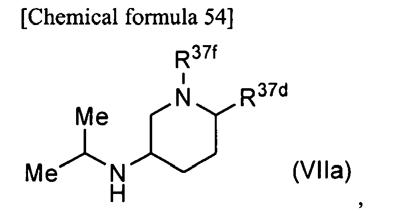 or a pharmaceutically acceptable salt thereof.

(2) The compound of the following formula (VIIb): 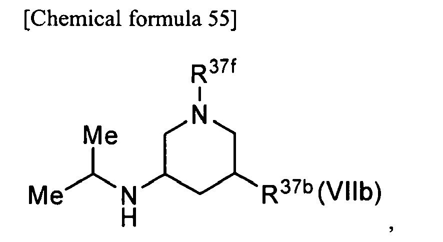 or a pharmaceutically acceptable salt thereof.

(3) The compound of the following formula (VIIc): 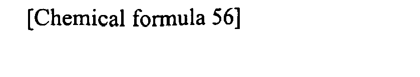 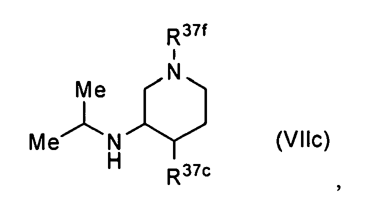 or a pharmaceutically acceptable salt thereof.

(4) The compound of the following formula (VIId): 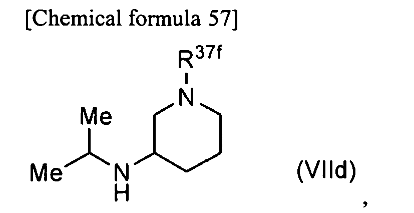 or a pharmaceutically acceptable salt thereof.

In addition, the compound of the above-mentioned formula (VII) is preferably ones having the following stereostructure (wherein the symbols are as defined above).

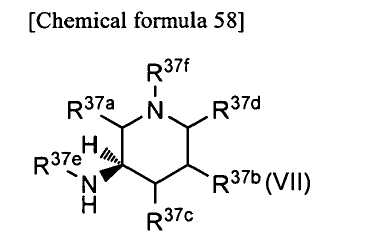

When the bond is expressed by a wedged solid or wedged broken line in the following disclosure, such as the formulae (F2), (F4), (F6), (F7) and (F8), it indicates the absolution configuration of a substituent, when the bond is expressed by a heavy line such as the formulae (F1), (F3), and (F5), then it indicates the relative configuration of a substituent (e.g., the formula (F1) indicates (±)-cis form).

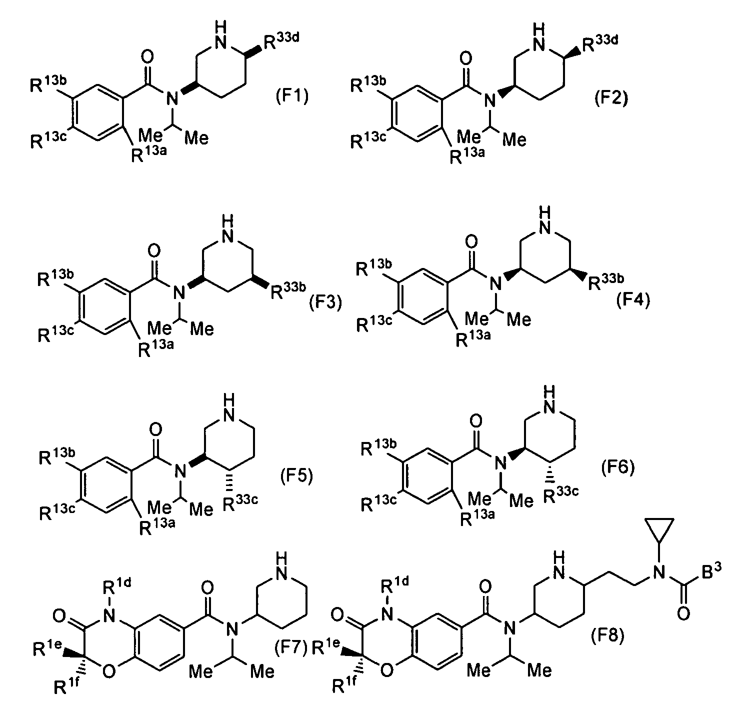

The pharmaceutically acceptable salt includes a salt with an inorganic acid such as hydrochloride, hydrobromide, sulfate, phosphate, nitrate, or a salt with an organic acid such as acetate, propionate, oxalate, succinate, lactate, malate, tartrate, citrate, maleate, fumarate, methanesulfonate, benzenesulfonate, p-toluenesulfonate, or ascorbate, etc.

In addition, the present invention includes the compound of the formula (I) or a prodrug thereof, or a pharmaceutically acceptable salt thereof. Further, the present invention also includes a hydrate thereof or a solvate thereof such as ethanolate. Further, the present invention includes a crystalline form of every embodiment.

The term "prodrug of the compound of the formula (I)" used in the present specification means a compound which is converted into the compound of the formula (I) in the living body by reaction by an enzyme or gastric acid under physiological conditions, namely, a compound which is converted into the compound of the formula (I) by oxidization, reduction, hydrolysis, etc. enzymatically, or by hydrolysis by gastric acid.

The compound of the formula (I) may exist in the form of a tautomer. Accordingly, the present invention also includes a tautomer of the compound of the formula (I).

The compound of the present invention may contain at least one asymmetric carbon atom. Accordingly, the present invention includes not only the racemic mixture of the present compound but also optically active compounds of the present compound. When the compound of the present invention has two or more asymmetric carbon atoms, then steric isomerism may occasionally occur. Accordingly, the present invention also includes these stereoisomers and a mixture thereof.

The compound of the present invention may have an axial isomerism due to rotational hindrance at a phenyl group and a carbonyl group. Because of this axial isomerism, the present invention includes the stereoisomers of the following formulae.

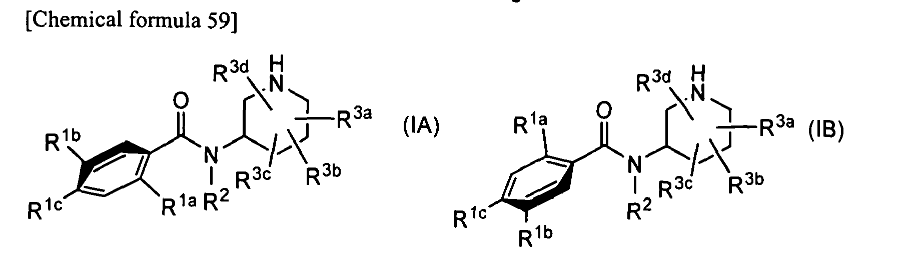

The present compound can be exemplified by the following compounds. In the following Tables, the compounds as expressed, for example, by No.1 (T1:H;T2:H;T3:H;T4:H;T5:H;T6:Q65;T7:Q211;T8:H;T9:H;T10:Q64) indicates the following compound (free base of Example 1).

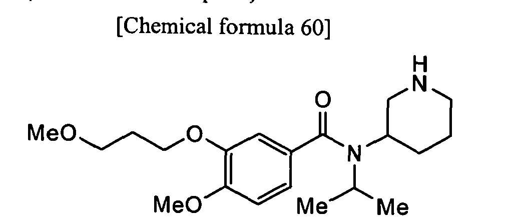

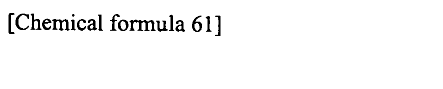

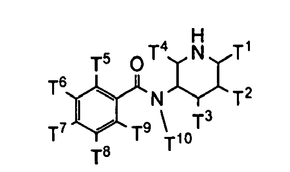

| No. | T¹ | T² | T³ | T⁴ | T⁵ | T⁶ | T⁷ | T⁸ | T⁹ | T¹⁰ |
|---|---|---|---|---|---|---|---|---|---|---|
| 1 | H | H | H | H | H | Q65 | Q211 | H | H | Q64 |
| 2 | H | H | Q301 | H | H | Q65 | Q211 | H | H | Q64 |
| 3 | H | H | Q71 | H | H | Q65 | Q211 | H | H | Q64 |
| 4 | H | H | Q72 | H | H | Q65 | Q211 | H | H | Q64 |
| 5 | H | H | Q73 | H | H | Q65 | Q211 | H | H | Q64 |
| 6 | H | H | Q74 | H | H | Q65 | Q211 | H | H | Q64 |
| 7 | H | H | H | H | H | Q65 | Q211 | H | H | Q108 |
| 8 | Q75 | H | H | H | H | Q65 | Q211 | H | Cl | Q64 |
| 9 | Q76 | H | H | H | H | Q65 | Q211 | H | Cl | Q64 |
| 10 | Q77 | H | H | H | H | Q65 | Q211 | H | H | Q64 |
| 11 | Q78 | H | H | H | H | Q65 | Q211 | H | H | Q64 |
| 12 | Q79 | H | H | H | H | Q65 | Q211 | H | H | Q64 |
| 13 | Q80 | H | H | H | H | Q65 | Q211 | H | H | Q64 |
| 14 | Q81 | H | H | H | H | Q65 | Q211 | H | H | Q64 |
| 15 | H | H | H | H | H | Q189 | Q211 | H | H | Q64 |
| 16 | Q83 | H | H | H | H | Q65 | Q211 | H | H | Q64 |
| 17 | H | Q73 | H | H | H | Q65 | Q211 | H | H | Q64 |
| 18 | H | H | H | H | H | Q65 | Q211 | H | H | Q62 |
| 19 | H | H | H | H | H | Q65 | Q211 | H | Q109 | Q64 |
| 20 | H | Q85 | H | H | H | Q65 | Q211 | H | H | Q64 |
| 21 | H | Q86 | H | H | H | Q65 | Q211 | H | H | Q64 |
| 22 | H | Q84 | H | H | H | Q65 | Q211 | H | H | Q64 |
| 23 | H | H | H | H | H | Q65 | Q211 | H | H | Q109 |
| 24 | H | H | H | H | H | Q65 | Q211 | H | H | Q110 |
| 25 | H | H | H | H | H | Q65 | Q211 | H | H | Q111 |
| 26 | H | H | H | H | H | Q65 | Q211 | H | H | Q112 |
| 27 | H | H | H | H | H | Q65 | Q211 | H | H | Q63 |
| 28 | H | H | H | H | H | Q65 | Q211 | H | H | Q113 |

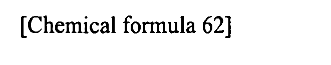

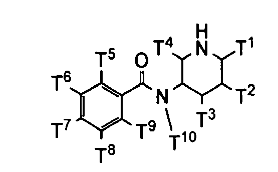

| No. | T¹ | T² | T³ | T⁴ | T⁵ | T⁶ | T⁷ | T⁸ | T⁹ | T¹⁰ |
|---|---|---|---|---|---|---|---|---|---|---|
| 29 | H | H | H | H | H | Q65 | Q211 | H | H | Q114 |
| 30 | H | Q88 | H | H | H | Q65 | Q211 | H | H | Q64 |
| 31 | H | Q190 | H | H | H | Q65 | Q211 | H | H | Q64 |
| 32 | H | Q90 | H | H | H | Q65 | Q211 | H | H | Q64 |
| 33 | H | Q91 | H | H | H | Q65 | Q211 | H | H | Q64 |
| 34 | H | H | Q92 | H | H | Q65 | Q211 | H | H | Q64 |
| 35 | H | Q93 | H | H | H | Q65 | Q211 | H | H | Q64 |
| 36 | H | Q94 | H | H | H | Q65 | Q211 | H | H | Q64 |
| 37 | H | Q95 | H | H | H | Q65 | Q211 | H | H | Q64 |
| 38 | H | Q96 | H | H | H | Q65 | Q211 | H | H | Q64 |
| 39 | H | Q97 | H | H | H | Q65 | Q211 | H | H | Q64 |
| 40 | Q98 | H | H | H | H | Q65 | Q211 | H | H | Q64 |
| 41 | Q99 | H | H | H | H | Q65 | Q211 | H | H | Q64 |
| 42 | H | H | H | H | H | Q65 | Q211 | H | Cl | Q64 |
| 43 | Q100 | H | H | H | H | Q65 | Q211 | H | H | Q64 |
| 44 | Q101 | H | H | H | H | Q65 | Q211 | H | H | Q64 |
| 45 | Q102 | H | H | H | H | Q65 | Q211 | H | H | Q64 |
| 46 | H | Q103 | H | H | H | Q65 | Q211 | H | H | Q64 |
| 47 | H | Q104 | H | H | H | Q65 | Q211 | H | H | Q64 |
| 48 | H | Q105 | H | H | H | Q65 | Q211 | H | H | Q64 |
| 49 | H | H | H | H | H | Q65 | Q211 | H | H | Q191 |
| 50 | H | Q106 | H | H | H | Q65 | Q211 | H | H | Q64 |
| 51 | H | Q107 | H | H | H | Q65 | Q211 | H | H | Q64 |

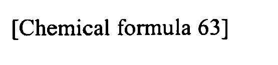

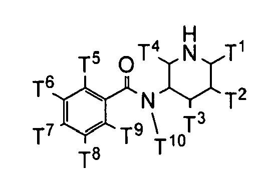

| No. | T¹ | T² | T³ | T⁴ | T⁵ | T⁶ | T⁷ | T⁸ | T⁹ | T¹⁰ |
|---|---|---|---|---|---|---|---|---|---|---|
| 52 | Q157 | H | H | H | H | Q65 | Q211 | H | H | Q64 |
| 53 | Q240 | H | H | H | H | Q65 | Q211 | H | H | Q64 |
| 54 | Q241 | H | H | H | H | Q65 | Q211 | H | H | Q64 |
| 55 | H | H | Q242 | H | H | Q65 | Q211 | H | H | Q109 |
| 56 | H | H | H | H | H | Q65 | Q213 | H | H | Q64 |
| 57 | H | H | H | H | H | Q65 | Q212 | H | H | Q64 |
| 58 | H | H | H | H | H | Q65 | Q214 | H | H | Q64 |
| 59 | H | H | H | H | H | Q65 | Q213 | H | Br | Q64 |
| 60 | H | H | H | H | H | Q65 | Q217 | H | H | Q64 |
| 61 | H | H | H | H | H | Q65 | Q218 | H | H | Q64 |
| 62 | H | H | H | H | H | Q65 | Q219 | H | H | Q64 |
| 63 | H | H | H | H | H | Q175 | Q211 | H | Cl | Q64 |
| 64 | Q82 | H | H | H | H | Q65 | Q211 | H | Cl | Q64 |
| 65 | H | H | H | H | H | Q65 | Q213 | H | Q113 | Q64 |
| 66 | H | H | H | H | H | Q65 | Q220 | H | H | Q64 |
| 67 | Q247 | H | H | H | H | Q65 | Q211 | H | H | Q64 |
| 68 | Q248 | H | H | H | H | Q65 | Q211 | H | H | Q64 |
| 69 | Q249 | H | H | H | H | Q65 | Q211 | H | H | Q64 |
| 70 | Q250 | H | H | H | H | Q65 | Q211 | H | H | Q64 |
| 71 | H | Q105 | H | H | H | Q65 | Q211 | H | Cl | Q64 |
| 72 | H | H | H | H | H | Q65 | Q211 | H | H | Q191 |
| 73 | H | H | H | H | H | Q57 | Q211 | H | Cl | Q64 |
| 74 | Q251 | H | H | H | H | Q65 | Q211 | H | H | Q64 |
| 75 | Q252 | H | H | H | H | Q65 | Q211 | H | H | Q64 |
| 76 | H | H | H | H | H | Q57 | Br | H | H | Q64 |
| 77 | H | H | H | H | H | Q222 | Q211 | Q65 | Cl | Q64 |
| 78 | H | H | H | H | H | Q65 | Q223 | H | Cl | Q64 |

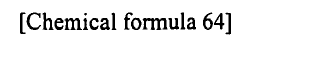

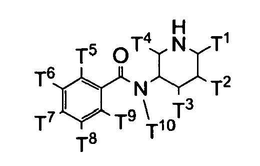

| No. | T¹ | T² | T³ | T⁴ | T⁵ | T⁶ | T⁷ | T⁸ | T⁹ | T¹⁰ |
|---|---|---|---|---|---|---|---|---|---|---|
| 79 | Q156 | H | H | H | H | Q65 | Q211 | H | Cl | Q64 |
| 80 | Q116 | H | H | H | H | Q65 | Q211 | H | Me | Q64 |
| 81 | H | H | H | H | H | Q65 | Q213 | H | CN | Q64 |
| 82 | H | H | H | H | H | Q65 | H | Q224 | H | Q64 |
| 83 | H | H | H | H | H | Q65 | H | Q174 | H | Q64 |
| 84 | H | H | H | H | H | Q65 | H | Q227 | H | Q64 |
| 85 | H | H | H | H | H | Q65 | H | Q225 | H | Q64 |
| 86 | H | H | H | H | H | Q65 | H | Q226 | H | Q64 |
| 87 | H | H | H | H | H | Q65 | Q228 | H | H | Q64 |
| 88 | H | H | H | H | H | Q65 | Q228 | H | H | Q64 |
| 89 | Q118 | H | H | H | H | Q65 | Q211 | H | H | Q64 |
| 90 | Q254 | H | H | H | H | Q65 | Q211 | H | H | Q64 |
| 91 | Q255 | H | H | H | H | Q65 | Q211 | H | H | Q64 |
| 92 | Q256 | H | H | H | H | Q65 | Q211 | H | H | Q64 |
| 93 | Q257 | H | H | H | H | Q65 | Q211 | H | H | Q64 |
| 94 | Q119 | H | H | H | H | Q65 | Q211 | H | H | Q64 |
| 95 | H | H | H | H | H | Q65 | Q229 | H | H | Q64 |
| 96 | H | H | H | H | H | Q65 | Q230 | H | H | Q64 |
| 97 | H | H | H | H | H | Q65 | Q231 | H | H | Q64 |
| 98 | Q259 | H | H | H | H | Q65 | Q211 | H | H | Q64 |
| 99 | Q260 | H | H | H | H | Q65 | Q211 | H | H | Q64 |
| 100 | Q261 | H | H | H | H | Q65 | Q211 | H | H | Q64 |
| 101 | Q262 | H | H | H | H | Q65 | Q211 | H | H | Q64 |
| 102 | Q263 | H | H | H | H | Q65 | Q211 | H | H | Q64 |
| 103 | H | H | H | H | H | Q65 | Q232 | H | H | Q64 |
| 104 | H | H | H | H | H | Q65 | Q233 | H | H | Q64 |
| 105 | Q264 | H | H | H | H | Q65 | Q211 | H | H | Q64 |
| 106 | Q265 | H | H | H | H | Q65 | Q211 | H | H | Q64 |

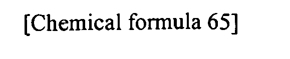

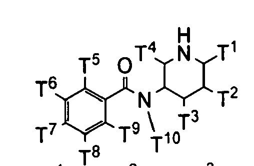

| No. | T¹ | T² | T³ | T⁴ | T⁵ | T⁶ | T⁷ | T⁸ | T⁹ | T¹⁰ |
|---|---|---|---|---|---|---|---|---|---|---|
| 107 | Q266 | H | H | H | H | Q65 | Q211 | H | H | Q64 |
| 108 | Q267 | H | H | H | H | Q65 | Q211 | H | H | Q64 |
| 109 | Q268 | H | H | H | H | Q65 | Q211 | H | H | Q64 |
| 110 | Q269 | H | H | H | H | Q65 | Q211 | H | H | Q64 |
| 111 | H | H | H | H | H | Q65 | Q234 | H | H | Q64 |
| 112 | H | H | H | H | H | Q65 | Q234 | H | H | Q64 |
| 113 | Q120 | H | H | H | H | Q65 | Q211 | H | H | Q64 |
| 114 | Q270 | H | H | H | H | Q65 | Q211 | H | H | Q64 |
| 115 | Q271 | H | H | H | H | Q65 | Q211 | H | H | Q64 |
| 116 | Q272 | H | H | H | H | Q65 | Q211 | H | H | Q64 |
| 117 | Q273 | H | H | H | H | Q65 | Q211 | H | H | Q64 |
| 118 | Q274 | H | H | H | H | Q65 | Q211 | H | H | Q64 |
| 119 | Q275 | H | H | H | H | Q65 | Q211 | H | H | Q64 |
| 120 | Q276 | H | H | H | H | Q65 | Q211 | H | H | Q64 |
| 121 | Q121 | H | H | H | H | Q65 | Q211 | H | H | Q64 |
| 122 | Q122 | H | H | H | H | Q65 | Q211 | H | H | Q64 |
| 123 | Q277 | H | H | H | H | Q65 | Q211 | H | H | Q64 |
| 124 | Q278 | H | H | H | H | Q65 | Q211 | H | H | Q64 |
| 125 | Q279 | H | H | H | H | Q65 | Q211 | H | H | Q64 |
| 126 | Q199 | H | H | H | H | Q65 | Q211 | H | Cl | Q64 |
| 127 | Q197 | H | H | H | H | Q65 | Q211 | H | H | Q64 |
| 128 | Q123 | H | H | H | H | Q65 | Q211 | H | H | Q64 |
| 129 | Q124 | H | H | H | H | Q65 | Q211 | H | H | Q64 |
| 130 | Q125 | H | H | H | H | Q65 | Q211 | H | H | Q64 |
| 131 | Q126 | H | H | H | H | Q65 | Q211 | H | H | Q64 |
| 132 | Q127 | H | H | H | H | Q65 | Q211 | H | H | Q64 |
| 133 | H | H | H | H | H | Q65 | H | Q132 | H | Q64 |
| 134 | H | H | H | H | H | Q65 | H | Q214 | H | Q64 |

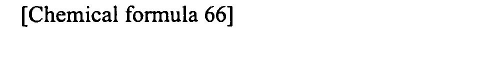

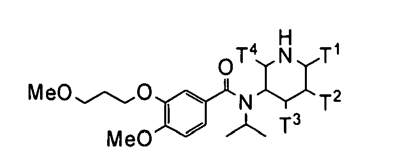

| No. | T¹ | T² | T³ | T⁴ | | No. T¹ | T² | T³ | T⁴ | No. | T¹ | T² | T³ | T⁴ |
|---|---|---|---|---|---|---|---|---|---|---|---|---|---|---|
| 135 | H | Q40 | H | H | 155 | Q66 | H | H | H | 175 | Q84 | H | H | H |
| 136 | H | Q235 | H | H | 156 | Q67 | H | H | H | 176 | Q117 | H | H | H |
| 137 | H | Q192 | H | H | 157 | Q140 | H | H | H | 177 | Q280 | H | H | H |
| 138 | H | Q236 | H | H | 158 | Q142 | H | H | H | 178 | Q85 | H | H | H |
| 139 | H | Q237 | H | H | 159 | Q143 | H | H | H | 179 | Q86 | H | H | H |
| 140 | H | Q159 | H | H | 160 | Q144 | H | H | H | 180 | Q75 | H | H | H |
| 141 | H | Q239 | H | H | 161 | Q145 | H | H | H | 181 | Q76 | H | H | H |
| 142 | H | Q238 | H | H | 162 | Q146 | H | H | H | 182 | Q55 | H | H | H |
| 143 | H | H | Q242 | H | 163 | Q156 H | | H | H | 183 | Q53 | H | H | H |
| 144 | H | H | Q243 | H | 164 | Q160 | H | H | H | 184 | Q196 | H | H | H |
| 145 | H | H | Q244 | H | 165 | Q161 | H | H | H | 185 186 | Q52 Q51 | H H | H H | H H |
| 146 | H | H | Q245 | H | 166 | Q162 | H | H | H | 187 | Q54 | H | H | H |
| 147 | Q128 | H | H | H | 167 | Q163 | H | H | H | 188 | Q40 | H | H | H |
| 148 | Q129 | H | H | H | 168 | Q164 | H | H | H | 189 | Q45 | H | H | H |
| 149 | Q130 | H | H | H | 169 | Q166 | H | H | H | 190 | Q82 | H | H | H |
| 150 | Q165 H | | H | H | 170 | Q167 | H | H | H | 191 | H | Q89 | H | H |
| 151 | Q136 | H | H | H | 171 | Q168 | H | H | H | 192 | H | Q241 | H | H |
| 152 | Q137 | H | H | H | 172 | Q169 | H | H | H | 193 | Q246 | H | H | H |
| 153 | Q138 | H | H | H | 173 | Q155 | H | H | H | 194 | Q253 | H | H | H |
| 154 | Q139 | H | H | H | 174 | Q156 | H | H | H | | | | | |

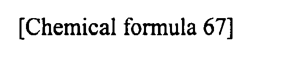

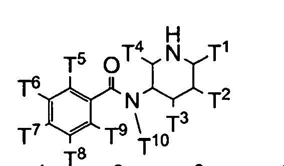

| No. | T¹ | T² | T³ | T⁴ | T⁵ | T⁶ | T⁷ | T⁸ | T⁹ | T¹⁰ |
|---|---|---|---|---|---|---|---|---|---|---|
| 195 | H | H | H | H | H | Q65 | Q209 | H | H | Q64 |
| 196 | H | H | H | H | H | Q65 | Q132 | H | H | Q64 |
| 197 | Q82 | H | H | H | H | Q60 | Q211 | H | H | Q64 |
| 198 | Q173 | H | H | H | H | Q208 | Q211 | H | H | Q64 |
| 199 | H | H | H | H | H | Q65 | Q215 | H | H | Q64 |
| 200 | H | H | H | H | H | Q65 | Q210 | H | H | Q64 |
| 201 | H | H | H | H | H | Q65 | Q216 | H | H | Q64 |
| 202 | Q162 | H | H | H | H | Q59 | Q211 | H | H | Q64 |
| 203 | Q161 | H | H | H | H | Q60 | Q211 | H | H | Q64 |
| 204 | Q160 | H | H | H | H | Q208 | Q211 | H | H | Q64 |
| 205 | Q164 | H | H | H | H | Q59 | Q211 | H | H | Q64 |
| 206 | H | H | H | H | H | Q65 | Q213 | H | Q92 | Q64 |
| 207 | Q142 | H | H | H | H | Q60 | Q211 | H | H | Q64 |
| 208 | Q143 | H | H | H | H | Q59 | Q211 | H | H | Q64 |
| 209 | Q137 | H | H | H | H | Q60 | Q211 | H | H | Q64 |
| 210 | Q138 | H | H | H | H | Q208 | Q211 | H | H | Q64 |
| 211 | Q139 | H | H | H | H | Q60 | Q211 | H | H | Q64 |
| 212 | H | H | H | H | H | Q65 | Q213 | H | Cl | Q64 |
| 213 | Q143 | H | H | H | H | Q60 | Q211 | H | H | Q64 |
| 214 | H | H | H | H | H | Q65 | Q213 | H | CN | Q64 |
| 215 | H | H | H | H | H | Q65 | Q228 | H | H | Q64 |
| 216 | H | H | H | H | H | Q65 | Q229 | H | H | Q64 |
| 217 | H | H | H | H | H | Q65 | Q230 | H | H | Q64 |
| 218 | H | H | H | H | H | Q65 | Q231 | H | H | Q64 |
| 219 | Q146 | H | H | H | H | Q59 | Q211 | H | H | Q64 |
| 220 | Q116 | H | H | H | H | Q60 | Q211 | H | H | Q64 |
| 221 | Q118 | H | H | H | H | Q208 | Q211 | H | H | Q64 |
| 222 | Q119 | H | H | H | H | Q60 | Q211 | H | H | Q64 |
| 223 | Q120 | H | H | H | H | Q60 | Q211 | H | H | Q64 |
| 224 | Q121 | H | H | H | H | Q208 | Q211 | H | H | Q64 |
| 225 | Q122 | H | H | H | H | Q59 | Q211 | H | H | Q64 |
| 226 | H | H | H | H | H | Q65 | H | Q65 | H | Q64 |

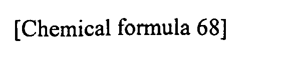

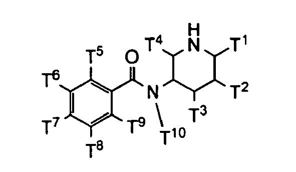

| No. | T¹ | T² | T³ | T⁴ | T⁵ | T⁶ | T⁷ | T⁸ | T⁹ | T¹⁰ |
|---|---|---|---|---|---|---|---|---|---|---|
| 227 | H | H | H | H | H | Q65 | H | Q209 | H | Q64 |
| 228 | Q123 | H | H | H | H | Q60 | H | H | H | Q64 |
| 229 | H | H | H | H | H | Q65 | H | Q210 | H | Q64 |
| 230 | H | H | H | H | H | Q65 | H | Q211 | H | Q64 |
| 231 | H | H | H | H | H | Q65 | H | Q212 | H | Q64 |
| 232 | H | H | H | H | H | Q65 | H | Q213 | H | Q64 |
| 233 | Q124 | H | H | H | H | Q59 | H | H | H | Q64 |
| 234 | Q125 | H | H | H | H | Q60 | H | H | H | Q64 |
| 235 | Q126 | H | H | H | H | Q208 | H | H | H | Q64 |
| 236 | Q127 | H | H | H | H | Q59 | H | H | H | Q64 |
| 237 | Q128 | H | H | H | H | Q60 | H | H | H | Q64 |
| 238 | Q129 | H | H | H | H | Q208 | H | H | H | Q64 |
| 239 | H | H | Q242 | H | H | Q65 | Q211 | H | H | H |
| 240 | Q130 | H | H | H | H | Q59 | H | H | H | Q64 |
| 241 | H | H | Q242 | H | H | Q65 | Q211 | H | H | Q63 |
| 242 | H | H | Q242 | H | H | Q65 | Q211 | H | H | Q113 |
| 243 | G1154 | H | H | H | H | Q65 | Q213 | H | H | Q64 |
| 244 | Q199 | H | H | H | H | Q65 | Q213 | H | H | Q64 |
| 245 | Q200 | H | H | H | H | Q65 | Q213 | H | H | Q64 |
| 246 | H | H | H | H | H | Q65 | H | H | H | Q64 |
| 247 | H | H | H | H | H | Q65 | Br | H | H | Q64 |
| 248 | H | H | H | H | H | Q221 | Q213 | H | Cl | Q64 |
| 249 | H | H | H | H | H | Q221 | Q213 | H | Br | Q64 |
| 250 | H | H | H | H | H | Q222 | Q213 | H | Cl | Q64 |

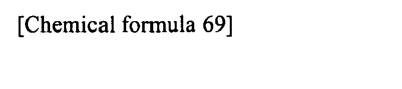

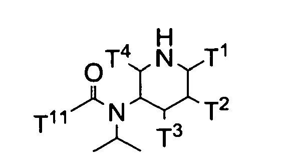

| No. | T¹ | T² | T³ | T⁴ | T¹¹ | No. | T¹ | T² | T³ | T⁴ | T¹¹ |
|---|---|---|---|---|---|---|---|---|---|---|---|
| 251 | H | H | H | H | Q181 | 263 | H | H | H | H | Q183 |
| 252 | H | H | H | H | Q198 | 264 | H | H | H | H | Q176 |
| 253 | H | H | H | H | Q178 | 265 | H | H | H | H | Q201 |
| 254 | Q83 | H | H | H | Q180 | 266 | Q120 | H | H | H | Q202 |
| 255 | H | H | H | H | Q194 | 267 | Q127 | H | H | H | Q203 |
| 256 | H | H | H | H | Q186 | 268 | H | H | H | H | Q204 |
| 257 | H | H | H | H | Q195 | 269 | H | H | H | H | Q203 |
| 258 | H | H | H | H | Q182 | 270 | H | H | H | H | Q205 |
| 259 | H | H | H | H | Q184 | 271 | Q120 | H | H | H | Q206 |
| 260 | H | H | H | H | Q177 | 272 | Q128 | H | H | H | Q207 |
| 261 | H | H | H | H | Q180 | 273 | Q102 | H | H | H | Q180 |
| 262 | H | H | H | H | Q185 | | | | | | |

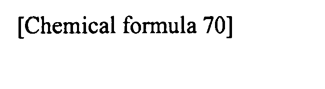

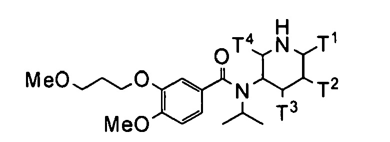

| No. | T¹ | T² | T³ | T⁴ | No. | T¹ | T² | T³ | T⁴ | No. | T¹ | T² | T³ | T⁴ |
|---|---|---|---|---|---|---|---|---|---|---|---|---|---|---|
| 274 | H | Q1 | H | H | 299 | H | Q26 | H | H | 324 | H | Q50 | H | H |
| 275 | H | Q2 | H | H | 300 | H | Q35 | H | H | 325 | H | Q51 | H | H |
| 276 | H | Q3 | H | H | 301 | H | Q36 | H | H | 326 | Q38 | H | H | H |
| 277 | H | Q4 | H | H | 302 | H | Q37 | H | H | 327 | Q174 | H | H | H |
| 278 | H | Q5 | H | H | 303 | H | H | Q27 | H | 328 | Q53 | H | H | H |
| 279 | H | Q6 | H | H | 304 | H | H | Q28 | H | 329 | Q128 | Q109 | H | H |
| 280 | H | Q7 | H | H | 305 | H | H | Q29 | H | 330 | Q128 | Q113 | H | H |
| 281 | H | Q8 | H | H | 306 | H | H | Q30 | H | 331 | Q45 | H | H | H |
| 282 | H | Q9 | H | H | 307 | H | H | Q31 | H | 332 | Q46 | H | H | H |
| 283 | H | Q10 | H | H | 308 | H | H | Q32 | H | 333 | Q47 | H | H | H |
| 284 | H | Q11 | H | H | 309 | H | H | Q33 | H | 334 | Q48 | H | H | H |
| 285 | H | Q12 | H | H | 310 | H | H | Q34 | H | 335 | Q35 | H | H | H |
| 286 | H | Q13 | H | H | 311 | H | Q39 | H | H | 336 | Q17 | H | H | H |
| 287 | H | Q14 | H | H | 312 | H | Q38 | H | H | 337 | Q15 | H | H | H |
| 288 | H | Q15 | H | H | 313 | H | Q41 | H | H | 338 | Q2 | H | H | H |
| 289 | H | Q16 | H | H | 314 | H | H | H | Q40 | 339 | Q43 | H | H | H |
| 290 | H | Q17 | H | H | 315 | H | H | H | Q41 | 340 | Q44 | H | H | H |
| 291 | H | Q18 | H | H | 316 | H | Q42 | H | H | 341 | Q35 | Q2 | H | H |
| 292 | H | Q19 | H | H | 317 | H | Q43 | H | H | 342 | Q35 | Q6 | H | H |
| 293 | H | Q20 | H | H | 318 | H | Q44 | H | H | 343 | Q35 | Q15 | H | H |
| 294 | H | Q21 | H | H | 319 | H | Q45 | H | H | 344 | Q15 | Q35 | H | H |
| 295 | H | Q22 | H | H | 320 | H | Q46 | H | H | 345 | Q35 | H | Q34 | H |
| 296 | H | Q23 | H | H | 321 | H | Q47 | H | H | 346 | Q15 | H | Q33 | H |
| 297 | H | Q24 | H | H | 322 | H | Q48 | H | H | 347 | Q54 | H | Q33 | H |
| 298 | H | Q25 | H | H | 323 | H | Q49 | H | H | 348 | Q43 | H | Q34 | H |

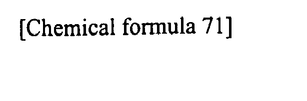

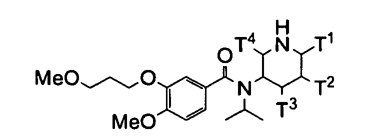

| No. | T¹ | T² | T³ | T⁴ | No. | T¹ | T² | T³ | T⁴ | No. | T¹ | T² | T³ | T⁴ |
|---|---|---|---|---|---|---|---|---|---|---|---|---|---|---|
| 349 | H | H | H | Q40 | 356 | H | Q37 | H | H | 363 | Q52 | H | H | H |
| 350 | H | H | H | Q41 | 357 | H | H | Q27 | H | 364 | Q53 | H | H | H |
| 351 | H | H | H | Q45 | 358 | H | H | Q28 | H | 365 | Q54 | H | H | H |
| 352 | H | H | H | Q13 | 359 | H | H | Q29 | H | 366 | Q42 | H | H | H |
| 353 | H | H | H | Q51 | 360 | H | Q50 | H | H | | | | | |
| 354 | H | H | H | Q46 | 361 | H | Q51 | H | H | | | | | |
| 355 | H | H | H | Q35 | 362 | Q51 | H | H | H | | | | | |

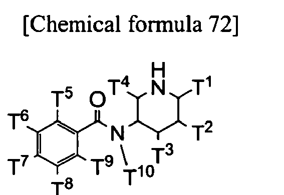

| No. | T¹ | T² | T³ | T⁴ | T⁵ | T⁶ | T⁷ | T⁸ | T⁹ | T¹⁰ |
|---|---|---|---|---|---|---|---|---|---|---|
| 367 | H | Q1 | H | H | H | Q56 | Q211 | H | H | Q64 |
| 368 | H | Q15 | H | H | H | Q57 | Q211 | H | H | Q62 |
| 369 | H | Q35 | H | H | H | Q58 | Q211 | H | H | Q62 |
| 370 | H | Q45 | H | H | H | Q59 | Q211 | H | H | Q62 |
| 371 | H | Q35 | H | H | H | Q60 | Q211 | H | H | Q62 |
| 372 | H | Q1 | H | H | H | Q61 | Q211 | H | H | Q62 |
| 373 | H | Q15 | H | H | H | Q65 | Q193 | H | H | Q63 |
| 374 | Q35 | Q1 | H | H | H | Q57 | Q211 | H | H | Q62 |
| 375 | Q15 | H | H | H | H | Q57 | Q211 | H | H | Q62 |
| 376 | H | H | Q34 | H | H | Q57 | Q211 | H | H | Q62 |
| 377 | H | H | Q27 | H | H | Q65 | Q193 | H | H | Q64 |
| 378 | H | H | H | H | H | Q57 | Q211 | H | Cl | Q64 |
| 379 | H | Q35 | H | H | H | Q65 | Q211 | H | Cl | Q64 |

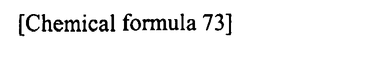

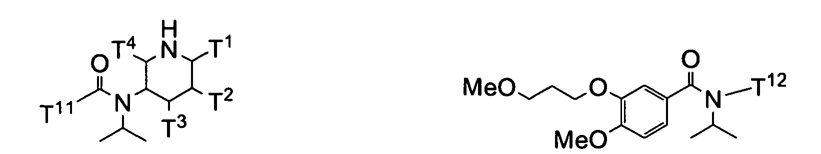

| No. | T¹ | T² | T³ | T⁴ | T¹¹ | No. | T¹² |
|---|---|---|---|---|---|---|---|
| 380 | H | Q35 | H | H | Q68 | 389 | Q187 |
| 381 | H | Q1 | H | H | Q68 | 390 | Q188 |
| 382 | Q35 | H | H | H | Q68 | 391 | Q115 |
| 383 | Q15 | H | H | H | Q68 | 392 | Q70 |
| 384 | H | Q35 | H | H | Q69 | | |
| 385 | Q45 | H | H | H | Q69 | | |
| 386 | H | Q15 | H | H | Q69 | | |
| 387 | H | H | Q34 | H | Q68 | | |
| 388 | H | H | H | Q40 | Q68 | | |

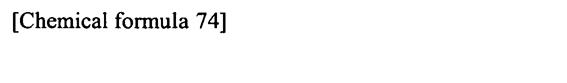

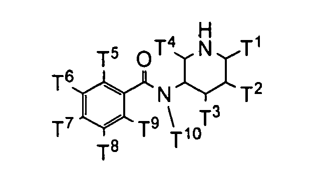

| No. | T¹ | T² | T³ | T⁴ | T⁵ | T⁶ | T⁷ | T⁸ | T⁹ | T¹⁰ |
|---|---|---|---|---|---|---|---|---|---|---|
| 393 | Q104 | H | H | H | H | Q65 | Q211 | H | H | Q64 |
| 394 | Q105 | H | H | H | H | Q65 | Q211 | H | H | Q64 |
| 395 | H | H | H | H | H | Q65 | Q211 | H | H | Q191 |
| 396 | Q106 | H | H | H | H | Q65 | Q211 | H | H | Q64 |
| 397 | Q107 | H | H | H | H | Q65 | Q211 | H | H | Q64 |
| 398 | Q264 | H | H | H | H | Q65 | Q211 | H | Q113 | Q64 |
| 399 | Q265 | H | H | H | H | Q65 | Q211 | H | Q109 | Q64 |
| 400 | Q266 | H | H | H | H | Q65 | Q211 | H | Q131 | Q64 |
| 401 | Q267 | H | H | H | H | Q65 | Q211 | H | Q132 | Q64 |
| 402 | Q269 | H | H | H | H | Q65 | Q211 | H | Q134 | Q64 |
| 403 | Q272 | H | H | H | H | Q65 | Q211 | H | Q218 | Q64 |
| 404 | Q274 | H | H | H | H | Q65 | Q211 | H | Q62 | Q64 |
| 405 | Q279 | H | H | H | H | Q65 | Q211 | H | Q133 | Q64 |
| 406 | Q199 | H | H | H | H | Q65 | Q211 | H | Cl | Q64 |
| 407 | Q196 | H | H | H | H | Q65 | Q211 | H | H | Q64 |
| 408 | Q280 | H | H | H | H | Q65 | Q211 | H | Cl | Q64 |
| 409 | Q129 | H | H | H | H | Q65 | Q211 | H | Cl | Q64 |
| 410 | Q246 | H | H | H | H | Q65 | Q211 | H | H | Q64 |
| 411 | Q173 | H | H | H | H | Q65 | Q211 | H | H | Q64 |
| 412 | Q254 | H | H | H | H | Q65 | Q211 | H | Cl | Q64 |
| 413 | Q255 | H | H | H | H | Q65 | Q211 | H | Cl | Q64 |
| 414 | Q256 | H | H | H | H | Q65 | Q211 | H | Cl | Q64 |
| 415 | Q257 | H | H | H | H | Q65 | Q211 | H | Cl | Q64 |
| 416 | Q259 | H | H | H | H | Q65 | Q211 | H | Cl | Q64 |
| 417 | Q260 | H | H | H | H | Q65 | Q211 | H | Cl | Q64 |
| 418 | Q261 | H | H | H | H | Q65 | Q211 | H | Cl | Q64 |
| 419 | Q262 | H | H | H | H | Q65 | Q211 | H | Cl | Q64 |
| 420 | Q268 | H | H | H | H | Q65 | Q211 | H | H | Q64 |

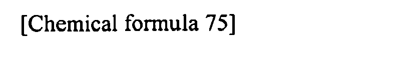

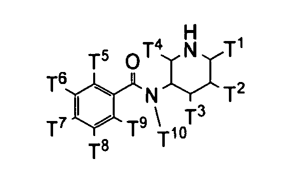

| No. | T¹ | T² | T³ | T⁴ | T⁵ | T⁶ | T⁷ | T⁸ | T⁹ | T¹⁰ |
|---|---|---|---|---|---|---|---|---|---|---|
| 421 | Q153 | H | H | H | H | Q65 | Q211 | H | H | Q170 |
| 422 | Q153 | H | H | H | H | Q65 | Q211 | H | H | Q171 |
| 423 | Q258 | H | H | H | H | Q65 | Q211 | H | H | Q64 |
| 424 | Q263 | H | H | H | H | Q65 | Q211 | H | Q134 | Q64 |
| 425 | Q128 | H | H | H | H | Q65 | Q211 | H | H | Q172 |
| 426 | Q265 | H | H | H | H | Q65 | Q213 | H | H | Q64 |
| 427 | Q266 | H | H | H | H | Q65 | Q212 | H | H | Q64 |
| 428 | Q271 | H | H | H | H | Q65 | Q213 | H | H | Q64 |
| 429 | Q274 | H | H | H | H | Q65 | Q213 | H | H | Q64 |
| 430 | Q154 | H | H | H | H | Q65 | Q211 | H | H | Q64 |
| 431 | Q141 | H | H | H | H | Q65 | Q211 | H | H | Q64 |
| 432 | Q200 | H | H | H | H | Q65 | Q211 | H | H | Q64 |
| 433 | Q281 | H | H | H | H | Q65 | Q211 | H | H | Q64 |
| 434 | Q282 | H | H | H | H | Q65 | Q211 | H | H | Q64 |
| 435 | Q38 | H | H | H | H | Q65 | Q213 | H | H | Q64 |
| 436 | Q283 | H | H | H | H | Q65 | Q211 | H | H | Q64 |
| 437 | Q284 | H | H | H | H | Q65 | Q211 | H | H | Q64 |
| 438 | Q285 | H | H | H | H | Q65 | Q211 | H | H | Q64 |
| 439 | H | H | H | H | H | Q65 | H | H | H | Q64 |
| 440 | H | H | H | Q109 | H | Q65 | Q211 | H | H | Q64 |
| 441 | H | H | H | H | H | Q65 | Q211 | Q65 | H | Q64 |
| 442 | H | H | H | H | H | Q65 | Br | H | H | Q64 |
| 443 | H | H | H | H | H | Q65 | H | Q209 | H | Q64 |
| 444 | H | H | H | H | H | Q65 | H | Q135 | H | Q64 |
| 445 | H | H | H | H | H | Q65 | H | Q228 | H | Q64 |
| 446 | H | H | H | H | H | Q65 | H | Q229 | H | Q64 |
| 447 | H | H | H | H | H | Q65 | H | Q230 | H | Q64 |
| 448 | H | H | H | H | H | Q65 | H | Q231 | H | Q64 |

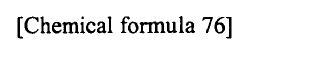

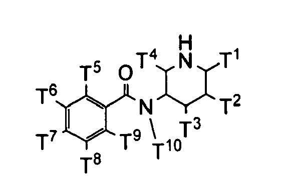

| No. | T¹ | T² | T³ | T⁴ | T⁵ | T⁶ | T⁷ | T⁸ | T⁹ | T¹⁰ |
|---|---|---|---|---|---|---|---|---|---|---|
| 449 | H | H | H | H | H | Q65 | H | Q214 | H | Q64 |
| 450 | H | H | H | H | H | Q65 | H | Q92 | H | Q64 |
| 451 | H | H | H | H | H | Q65 | Q209 | H | H | Q64 |
| 452 | H | H | H | H | H | Q65 | Q135 | H | H | Q64 |
| 453 | H | H | H | H | H | Q65 | Q215 | H | H | Q64 |
| 454 | H | H | H | H | H | Q65 | Q210 | H | H | Q64 |
| 455 | H | H | H | H | H | Q65 | Q216 | H | H | Q64 |
| 456 | H | H | H | H | H | Q65 | Q213 | H | H | Q64 |
| 457 | H | H | H | H | H | Q65 | Q212 | H | H | Q64 |
| 458 | H | H | H | H | H | Q65 | Q214 | H | H | Q64 |
| 459 | H | H | H | H | H | Q65 | Q213 | H | Br | Q64 |
| 460 | H | H | H | H | H | Q65 | Q213 | H | Q65 | Q64 |
| 461 | H | H | H | H | H | Q65 | Q218 | H | H | Q64 |
| 462 | H | H | H | H | H | Q65 | Q219 | H | H | Q64 |
| 463 | H | H | H | H | H | Q175 | Q211 | H | Cl | Q64 |
| 464 | H | H | H | H | H | Q175 | Q213 | H | Q113 | Q64 |
| 465 | H | H | H | H | H | Q65 | Q220 | H | H | Q64 |
| 466 | H | H | H | H | H | Q65 | Q286 | H | H | Q64 |
| 467 | H | H | H | H | H | Q65 | Q213 | H | Cl | Q64 |
| 468 | H | H | H | H | H | Q221 | Q211 | H | Cl | Q64 |
| 469 | H | H | H | H | H | Q221 | Br | H | H | Q64 |
| 470 | H | H | H | H | H | Q222 | Q211 | H | Cl | Q64 |
| 471 | H | H | H | H | H | Q65 | Q223 | H | H | Q64 |
| 472 | H | H | H | H | H | Q65 | Q223 | H | H | Q64 |
| 473 | H | Q147 | H | H | H | Q65 | Q211 | H | H | Q64 |
| 474 | H | Q148 | H | H | H | Q65 | Q211 | H | H | Q64 |
| 475 | H | Q149 | H | H | H | Q65 | Q211 | H | H | Q64 |
| 476 | H | Q150 | H | H | H | Q65 | Q211 | H | H | Q64 |
| 477 | H | Q151 | H | H | H | Q65 | Q211 | H | H | Q64 |
| 478 | H | Q152 | H | H | H | Q65 | Q211 | H | H | Q64 |
| 479 | H | Q153 | H | H | H | Q65 | Q211 | H | H | Q64 |
| 480 | H | Q87 | H | H | H | Q65 | Q211 | H | H | Q64 |
| 481 | H | Q158 | H | H | H | Q65 | Q211 | H | H | Q64 |

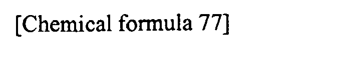

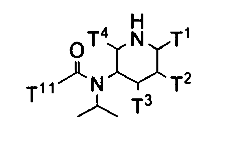

| No. | T¹ | T² | T³ | T⁴ | T¹¹ | No. | T¹ | T² | T³ | T⁴ | T¹¹ |
|---|---|---|---|---|---|---|---|---|---|---|---|
| 482 | H | H | H | H | Q287 | 495 | H | H | H | H | Q179 |
| 483 | H | H | H | H | Q288 | 496 | Q253 | H | H | H | Q287 |
| 484 | Q265 | H | H | H | Q185 | 497 | Q278 | H | H | H | Q287 |
| 485 | Q83 | H | H | H | Q183 | 498 | Q265 | H | H | H | Q287 |
| 486 | Q271 | H | H | H | Q177 | 499 | Q265 | H | H | H | Q185 |
| 487 | Q266 | H | H | H | Q177 | 500 | Q278 | H | H | H | Q294 |
| 488 | Q66 | H | H | H | Q201 | 501 | Q265 | H | H | H | Q295 |
| 489 | H | H | H | H | Q289 | 502 | Q278 | H | H | H | Q296 |
| 490 | H | H | H | H | Q180 | 503 | Q265 | H | H | H | Q297 |
| 491 | Q266 | H | H | H | Q289 | 504 | Q265 | H | H | H | Q298 |
| 492 | H | H | H | H | Q290 | 505 | Q265 | H | H | H | Q299 |
| 493 | H | H | H | H | Q291 | 506 | Q265 | H | H | H | Q300 |
| 494 | H | H | H | H | Q292 | | | | | | |

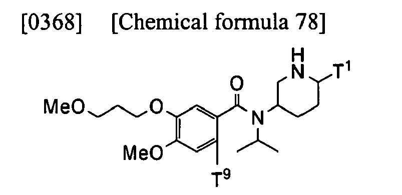

| No. | T¹ | T⁹ |
|---|---|---|
| 507 | Q264 | Q293 |
| 508 | Q266 | Q193 |
| 509 | Q133 | Q293 |

Each abbreviation used in the above-mentioned Tables indicates a partial structure selected from the following partial structures.

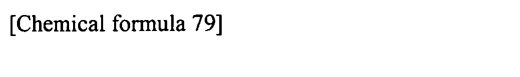

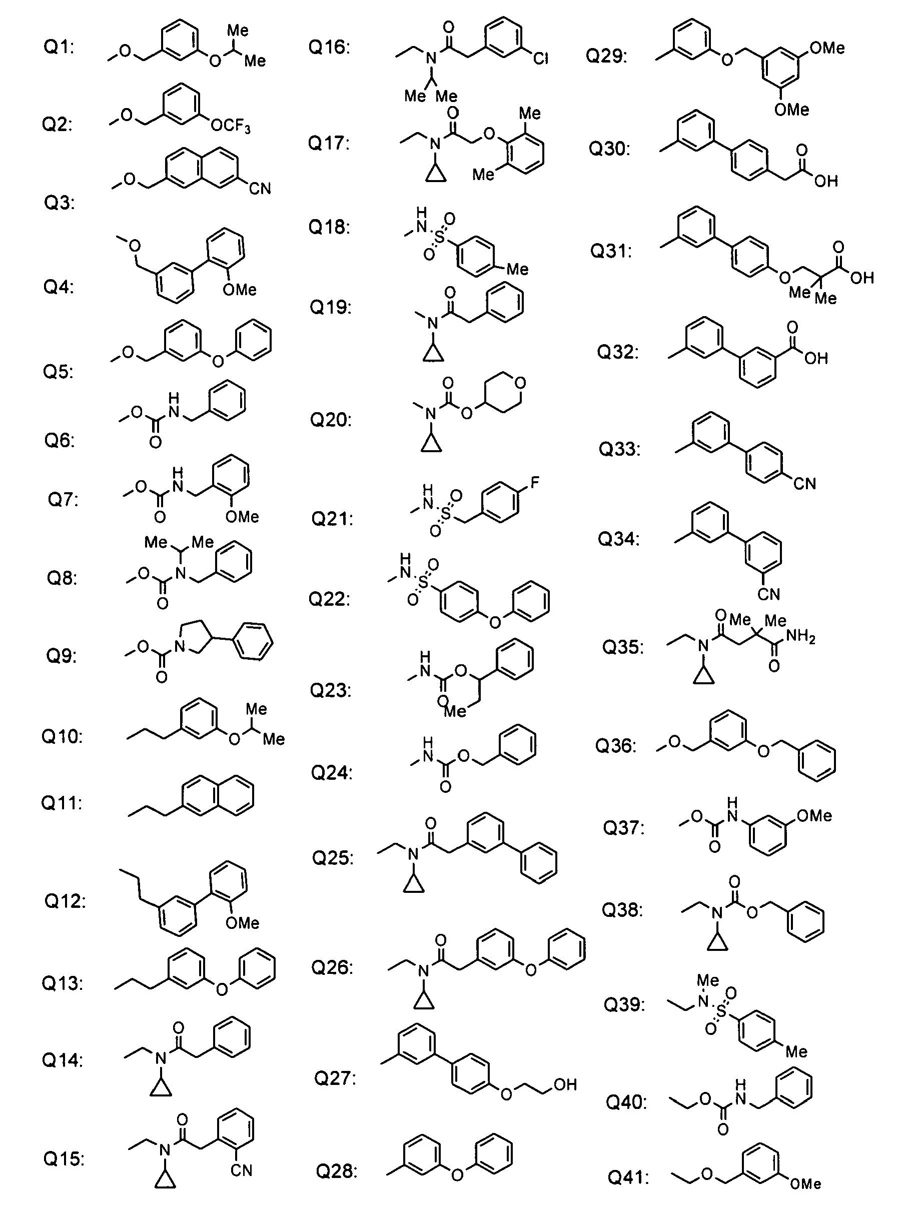

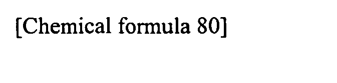

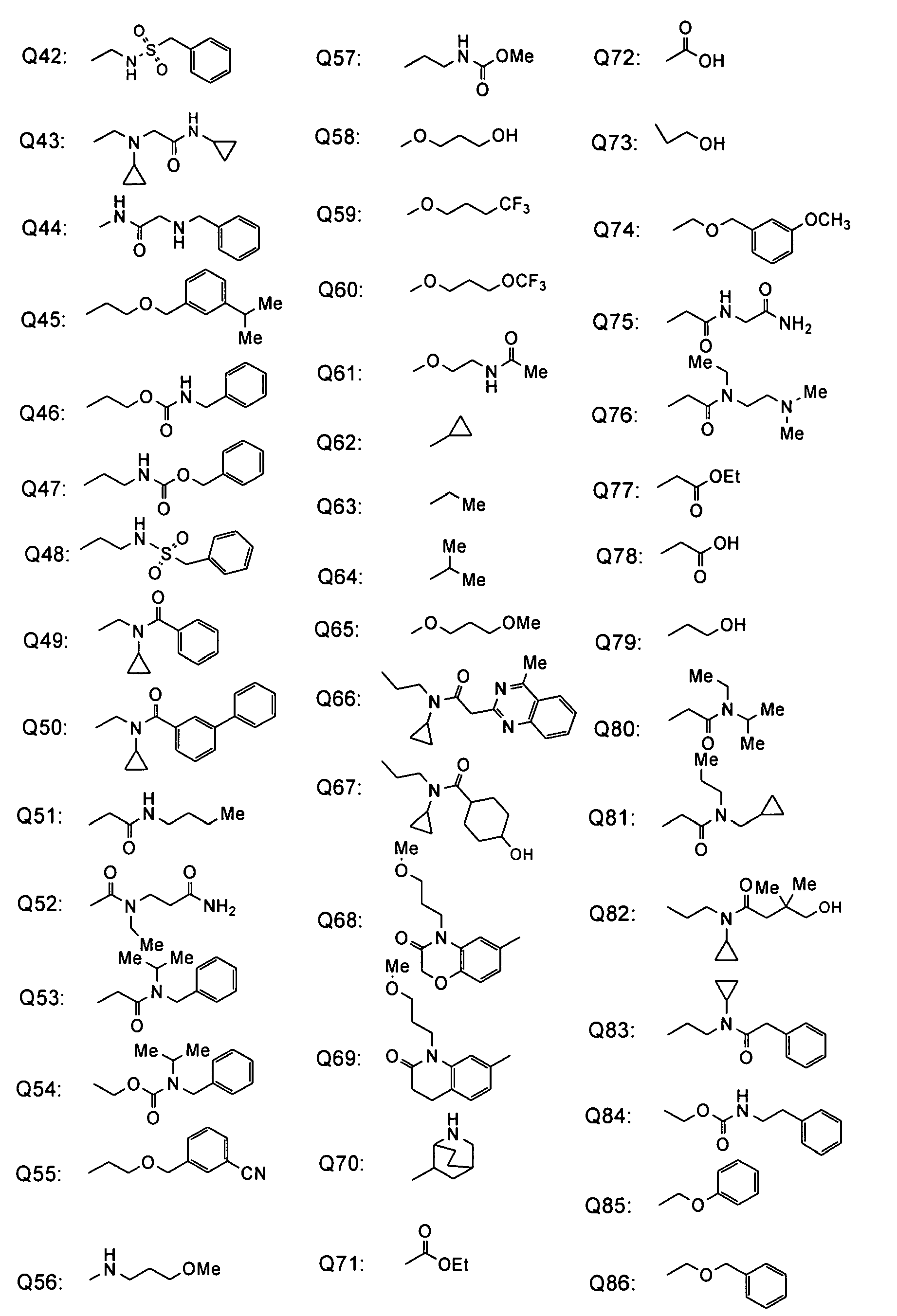

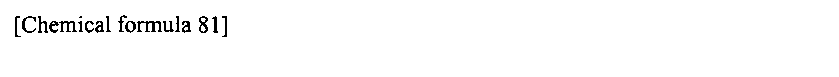

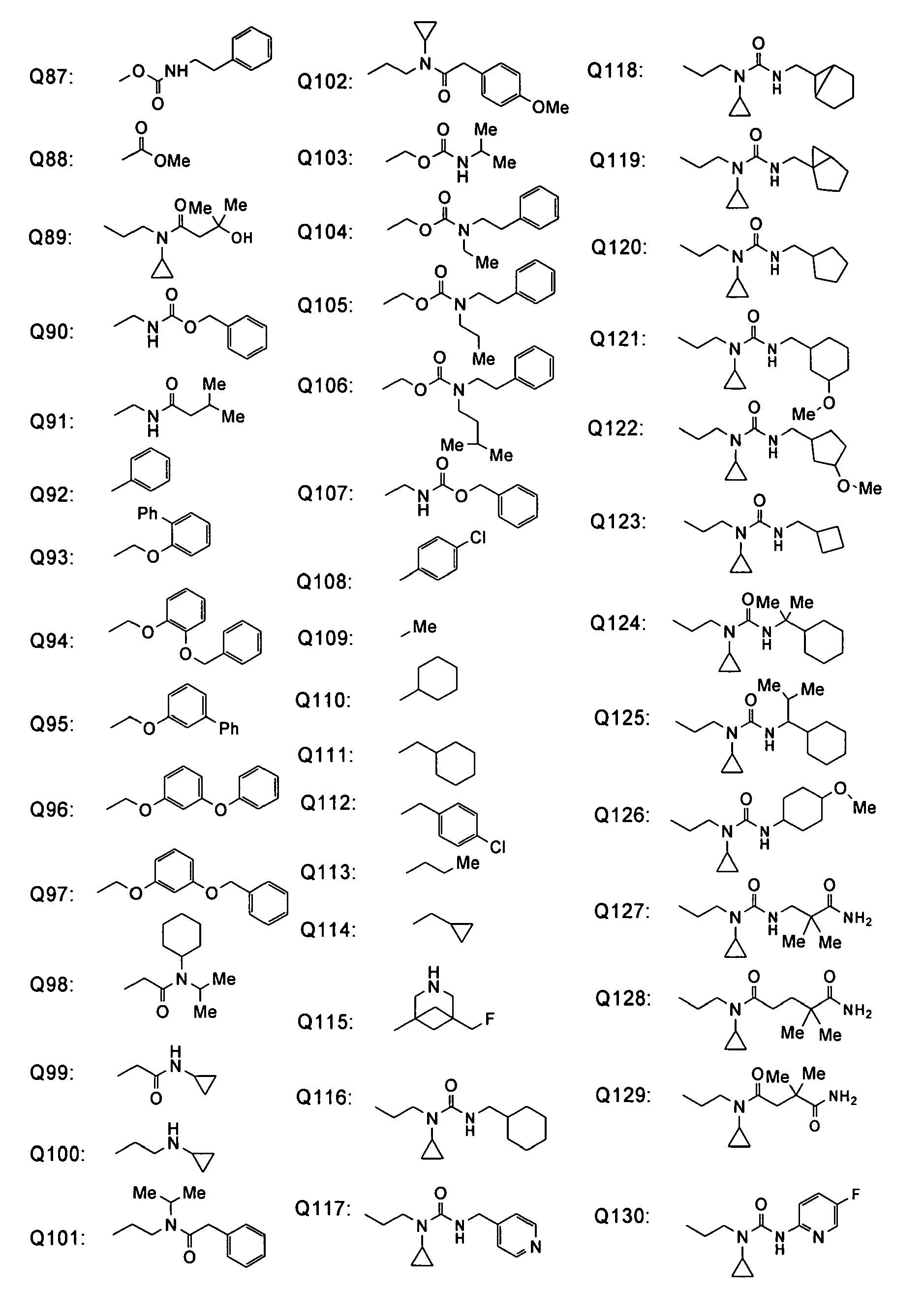

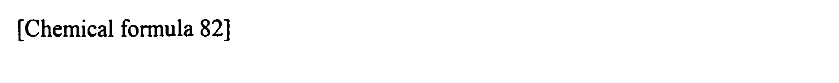

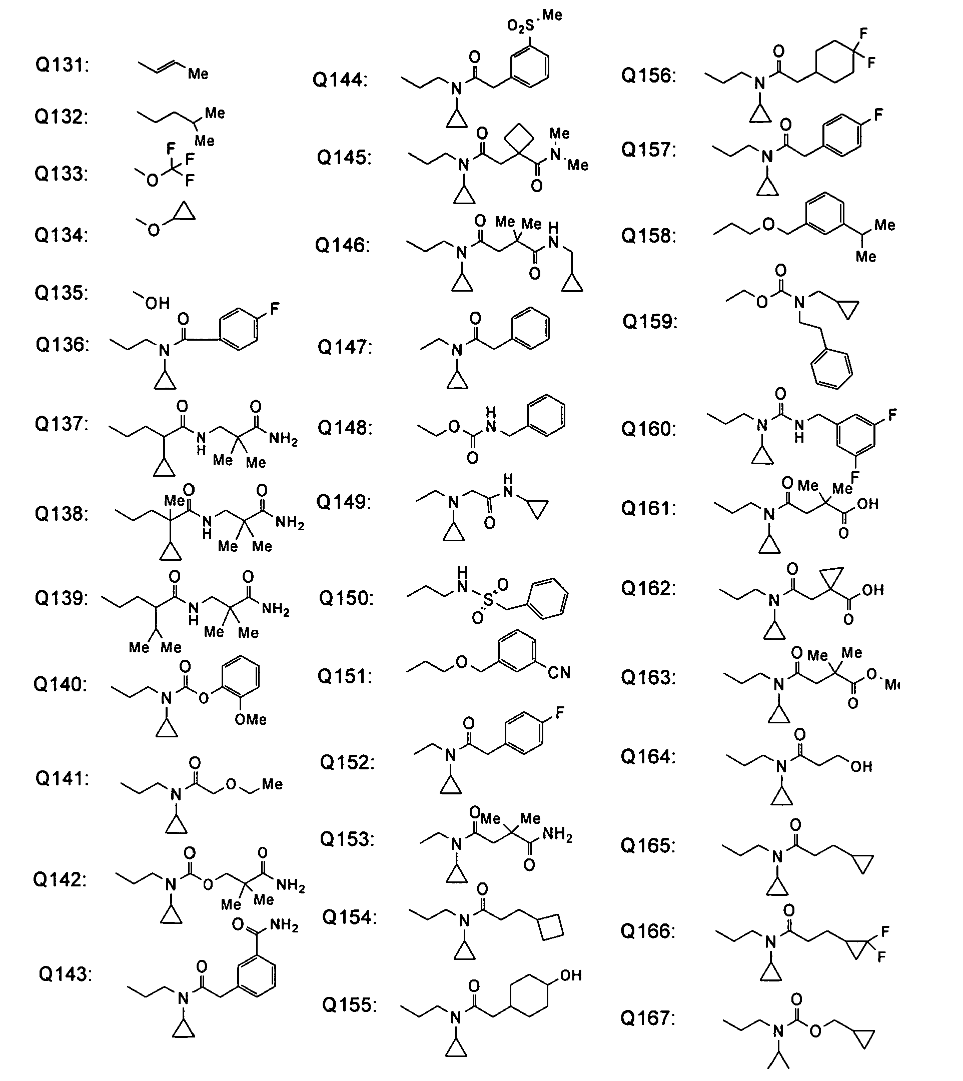

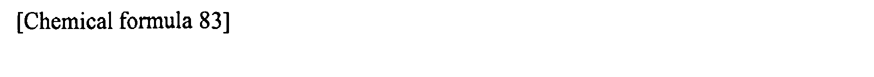

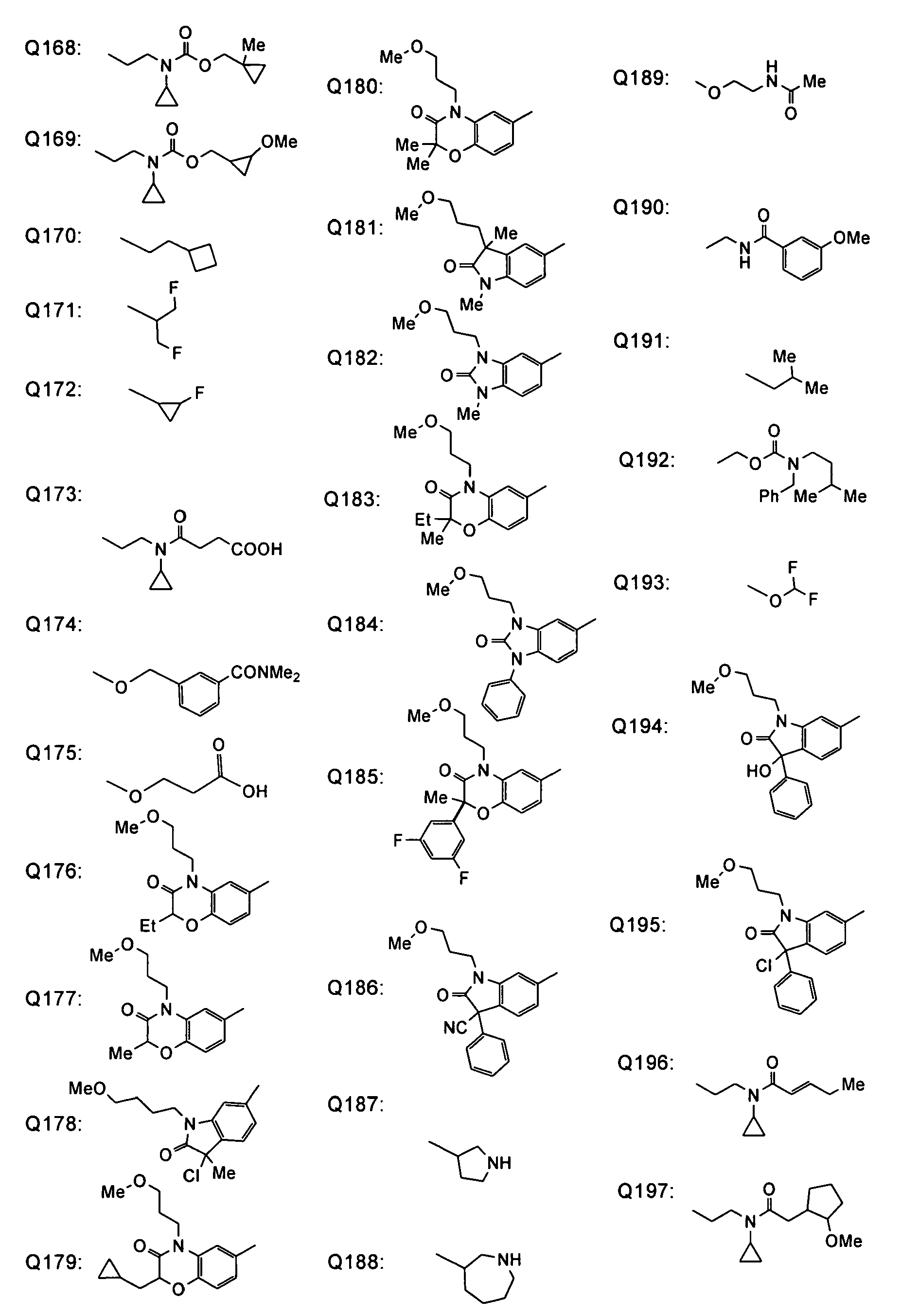

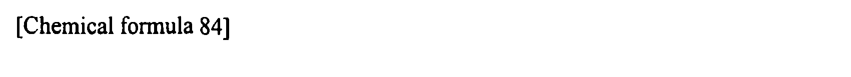

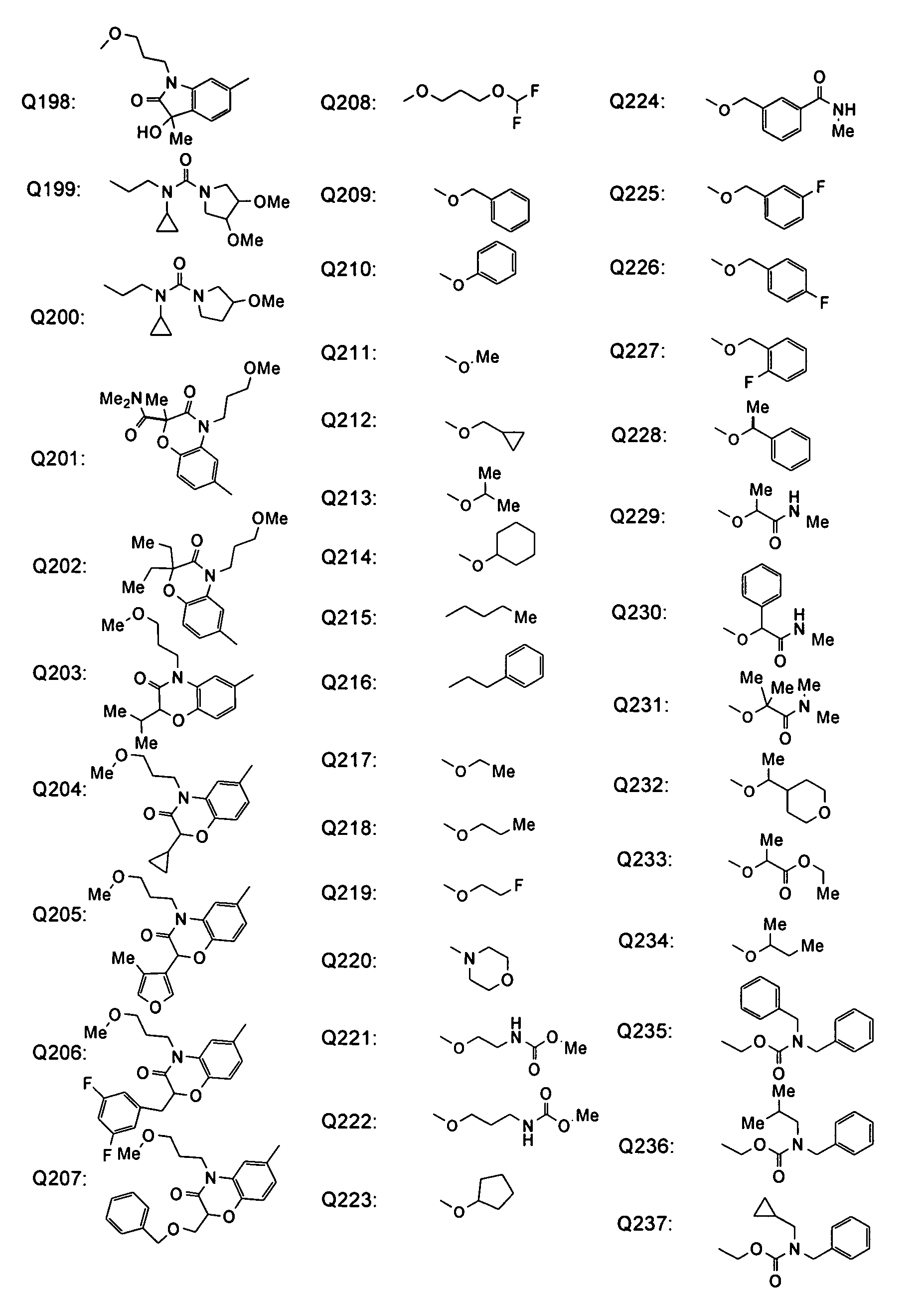

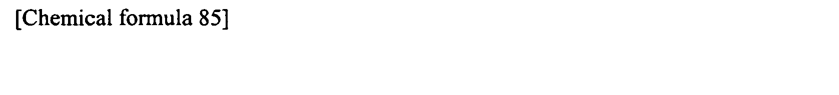

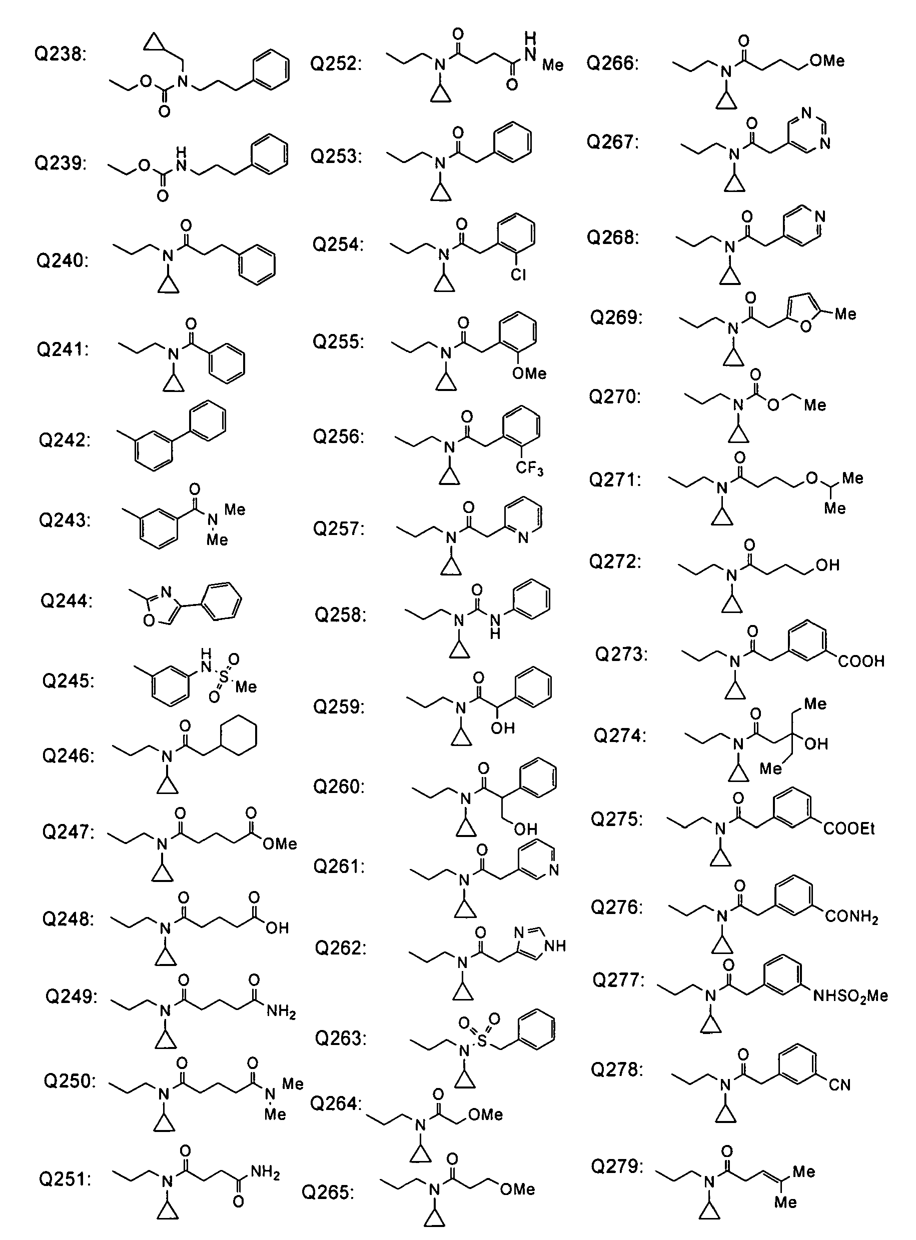

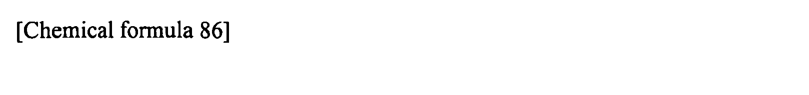

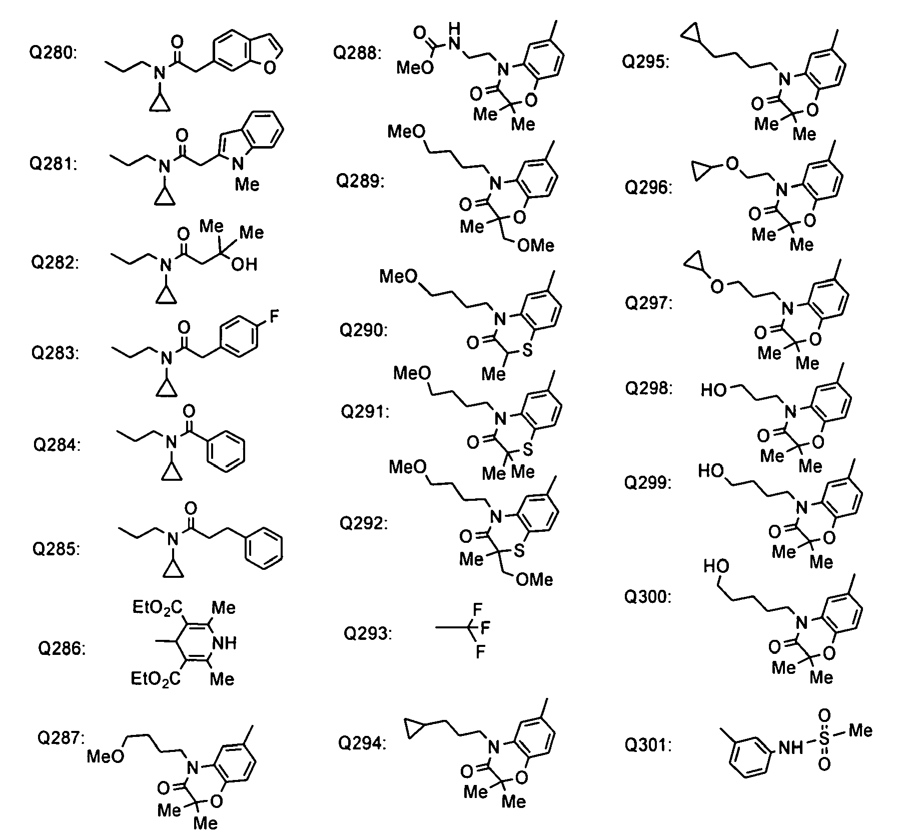

The process for preparing the compound of the formula (I) of the present invention will be illustrated hereinbelow by Examples, but the present invention should not be construed to be limited thereto. In addition, in order to simplify the description of the present invention, the following abbreviations may be used in the present specification.
- Boc:: tert-butoxycarbonyl group
- Cbz:: benzyloxycarbonyl group
- TMS:: trimethylsilyl group
- TBS:: tert-butyldimethylsilyl group
- SEM:: 2-[(trimethylsilyl)ethoxy]methyl group
- Ac:: acetyl group
- Me:: methyl group
- Et:: ethyl group
- Pr:: propyl group
- i-Pr:: isopropyl group
- Bu:: butyl group
- i-Bu:: isobutyl group
- t-Bu:: tert-butyl group
- Ph:: phenyl group
- Bn:: benzyl group
- Ms:: methanesulfonyl group
- TFA:: trifluoroacetic acid
- Alloc:: allyloxycarbonyl group
- Tf:: trifluoromethanesulfonate

The compound of the formula (I) may be synthesized from the well-known compound by a combination of the well-known methods, for example, by the following Methods. In addition, the compound of the formula (I) may be synthesized by suitably combining the following Methods due to the kinds of the starting compounds.

### Method 1

The compound of the formula (I) or a pharmaceutically acceptable salt thereof may be prepared, for example, by the following method.

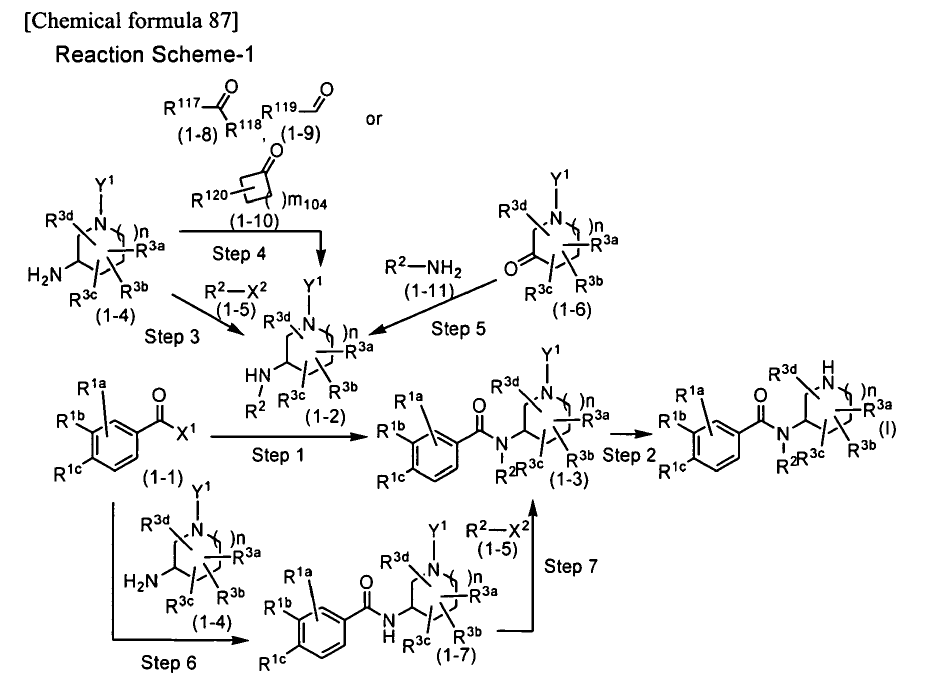

[wherein R^{1a}, R^{1b}, R^{1c}, R^{3a}, R^{3b}, R^{3c}, R^{3d}, R², and n are as defined in the above-mentioned Item 1; R¹¹⁷ is a C₁₋₃ alkyl group optionally substituted by fluorine, C₃₋₆ cycloalkyl or C₁₋₃ alkoxy, or a C₃₋₆ cycloalkyl group optionally substituted by fluorine or C₁₋₃ alkoxy; R¹¹⁸ is a C₁₋₂ alkyl group; R¹¹⁹ is a C₁₋₅ alkyl group optionally substituted by fluorine, C₃₋₆ cycloalkyl or C₁₋₃ alkoxy, or a C₃₋₆ cycloalkyl group optionally substituted by fluorine or C₁₋₃ alkoxy; R¹²⁰ is a fluorine atom or a C₁₋₃ alkoxy group; m₁₀₄ is an integer of 0, 1, 2, or 3; X¹ is a hydroxy group or a chlorine atom; X² is iodine atom, bromine atom, chlorine atom, methanesulfonyloxy group, trifluoromethanesulfonyloxy group, or p-toluenesulfonyloxy group; Y¹ is Cbz, Boc or Alloc]

### 1) Step 1

When X¹ is a hydroxy group, the compound of the formula (1-3) may be synthesized by reacting the compound of the formula (1-1) with the compound of the formula (1-2) in an inert solvent using a condensing agent, and if necessary, in the presence of a base. A phase-transfer catalyst may be used occasionally.
The base may be any conventional ones which are usually used as a base in conventional reactions and is not necessarily defined, and includes, for example, organic bases such as N-methylmorpholine, triethylamine, diisopropylethylamine, tributylamine, 1,8-diazabicyclo[5.4.0]undec-7-ene, 1,5-diazabicyclo[4.3.0]nona-5-ene, 1,4-diazabicyclo[5.4.0]undec-7-ene, pyridine, dimethylaminopyridine, or picoline, etc., or inorganic bases such as sodium hydrogen carbonate, potassium hydrogen carbonate, sodium carbonate, potassium carbonate, sodium hydroxide, or sodium hydride, etc. The phase-transfer catalyst includes, for example, quaternary ammonium salts such as tetrabutylammonium bromide or benzyltriethylammonium bromide, etc. or crown ethers such as 18-crown-6-ether, etc. The condensing agent may be ones as described in Jikken-Kagaku-Koza (edited by Chemical Society of Japan, Maruzen), vol. 22. For example, phosphoric esters such as diethyl cyanophosphate or diphenylphosphorylazide, etc., carbodiimides such as 1-ethyl-3-(3-dimethylaminopropyl)-carbodiimide·hydrochloride, or dicyclohexylcarbodiimide, a combination of a disulfide such as 2,2'-dipyridylsulfide, etc. and a phospine such as triphenylphosphine, a phosphorus halide such as N,N'-bis(2-oxo-3-oxazolidinyl)phosphinic chloride, etc., a combination of an azodicarboxylic diester such as diethylazodicarboxylate, etc., and a phosphine such as triphenylphosphine, etc. or 2-halo-1-lower alkylpyridinium halide such as 2-chloro-1-methylpyridinium iodide, etc. The inert solvent includes, for example, ethers such as tetrahydrofuran, diethyl ether, 1,4-dioxane, or 1,2-dimethoxyethane, etc., hydrocarbons such as hexane, heptane, toluene, benzene, or xylene, etc., halogenated hydrocarbons such as dichloromethane, chloroform, or 1,2-dichloroethane, etc., ketones such as acetone, etc., aprotic solvents such as acetonitrile, N,N'-dimethylformamide, dimethylsulfoxide, or hexamethylenephosphamide, etc., or a mixture of these solvents. The reaction temperature should be suitably selected from the range of about -70°C to about 80°C.

When X¹ is a chlorine atom, the compound of the formula (1-3) may be synthesized by reacting the compound of the formula (1-2) with the compound of the formula (1-1) in an inert solvent, and if necessary, in the presence of a base. The base includes, for example, organic bases such as N-methylmorpholine, triethylamine, diisopropylethylamine, tributylamine, 1,8-diazabicyclo[5.4.0]undec-7-ene, 1,5-diazabicyclo[4.3.0]non-5-ene, 1,4-diazabicyclo[5.4.0]undec-7-ene, pyridine, dimethylaminopyridine, or picoline, etc. The base is usually used in an amount of 1 to 20 equivalents to 1 equivalent of the compound of the formula (1-1) wherein X¹ is a chlorine atom. The inert solvent includes, for example, halogenated hydrocarbons such as dichloromethane, chloroform, or 1,2-dichloroethane. The reaction temperature should be suitably selected from the range of about -10°C to about 50°C.

The compound of the formula (1-1) wherein X¹ is a chlorine atom may be synthesized by reacting the compound of the formula (1-1) wherein X¹ is a hydroxy group with oxalyl chloride or thionyl chloride in an inert solvent in the presence or absence of an additive. The additive includes, for example, dimethylformamide, diethylformamide, etc. The inert solvent includes, for example, halogenated hydrocarbons such as dichloromethane, dichloroethane, or chloroform, etc. The reaction temperature should be suitably selected from the range of about -10°C to about 50°C. After the reaction is completed, the reaction solution is concentrated under reduced pressure in the presence of a hydrocarbon solvent such as benzene or toluene to give the compound of the formula (1-1) wherein X¹ is a hydroxy group.

The compound (1-1) wherein X¹ is a hydroxy group may be prepared by using a commercially available reagent or from a well-known compound by a conventional method. For example, the following compounds (1-1a) to (1-1m) may be synthesized by a similar method to the method disclosed in the literature (e.g., WO 06/100036, etc).

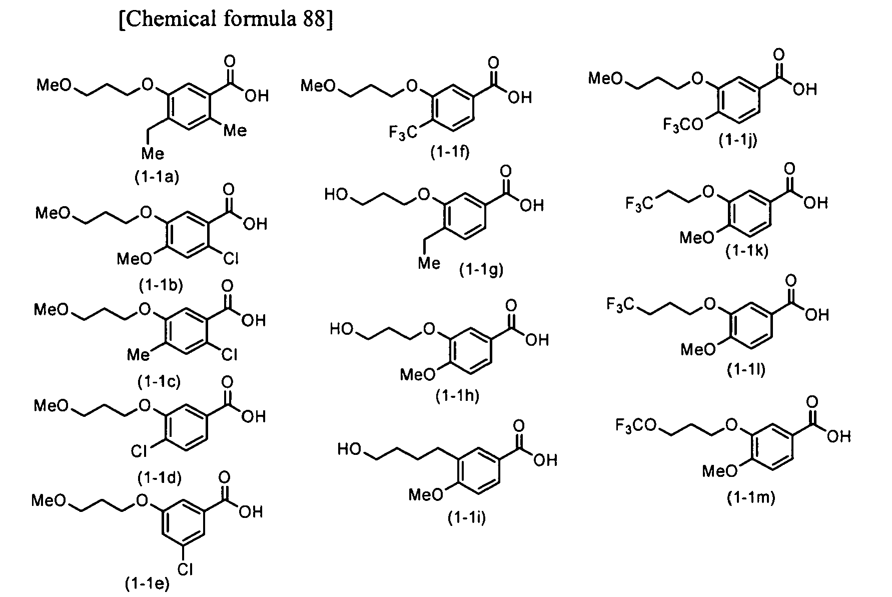

Among the compounds (1-1), the compound as defined as "bicyclic condensed ring" may be prepared, for example, by a similar method to the method disclosed in the literatures (e.g., WO 04/028467, Chemical & Pharmaceutical Bulletin 46, 1716 (1998), WO 04/007491, J. Combinatorial Chemistry 8, 469 (2006), etc.). R^{3a}, R^{3b}, R^{3c} and R^{3d}, that are substituents of the compound (1-3), may be converted into the substituents disclosed in Methods 2 to 18 by a method disclosed in Methods 2 to 18.

### 2) Step 2

The compound (I) may be synthesized from the compound (1-3) by a similar method to the method disclosed in the literature (e.g., Protective Groups in Organic Synthesis 2nd Edition (John Wiley & Sons, Inc.), etc.).

### 3) Step 3

The compound (1-2) may be synthesized from the compound (1-4) by a similar method to the method disclosed in Method 1, Step 7. In addition, when R² of the compound (1-5) is an optionally substituted aryl group or an optionally substituted heteroaryl group, the compound (1-2) may be prepared from the compound (1-4) by a similar method to the method disclosed in the literature (J. Or g. Chem. 71, 6522 (2006), etc.).

### 4) Step 4

The compound (1-2) may be prepared from the compound (1-4) by a similar method to the method disclosed in the literatures (e.g., J. Org. Chem. 61, 3849 (1996), J. Org. Chem. 68, 4120 (2003), J. Org. Chem. 63, 370 (1998), J. Org. Chem. 70, 2195 (2005), etc.). The following procedures are exemplified.
The compound (1-2) can be prepared by reacting a compound selected from the compound (1-8), the compound (1-9), and the compound (1-10) with the compound (1-4) in an inert solvent in the presence or absence of acetic acid by a reductive amination reaction using a boron hydride compound such as sodium triacetoxyborohydride, sodium cyanoborohydride, etc. The inert solvent includes, for example, halogenated hydrocarbons such as dichloromethane, or dichloroethane, etc.; alcohols such as methanol or ethanol, etc.; ether solvents such as tetrahydrofuran, or 1,4-dioxane, or 1,2-dimethoxyethane, etc. The boron hydride compound may be usually used in an amount of 1 to 3 equivalents to 1 equivalent of the compound (1-4). The reaction temperature should be suitably selected from the range of about - 10°C to about 40°C.

### 5) Step 5

The compound (1-2) may be prepared from the compound (1-6) by a similar method to Step 4 of Method 1.

### 6) Step 6

By a similar method to Step 1 of Method 1, the compound (1-7) may be prepared from the compound (1-1).

### 7) Step 7

The compound (1-3) is prepared by reacting the compound (1-7) with the compound (1-5) in an inert solvent in the presence of a base. The base includes, for example, alkali metal salts such as sodium hydrogen carbonate, potassium carbonate, or sodium hydroxide, etc.; organic bases such as triethylamine or 1,8-diazabicyclo[5.4.0]undec-7-ene (DBU), etc.; alkali metal hydrides such as sodium hydride or potassium hydride, etc.; alkali metal alkoxides such as potassium t-butoxide, etc.. When X² is a chlorine atom or a bromine atom, an additive such as sodium iodide or potassium iodide, etc. may be used. The inert solvent includes, for example, ether solvents such as tetrahydrofuran or 1,4-dioxane, etc.; aprotic solvents such as dimethylformamide or dimethylsulfoxide, etc.; halogenated hydrocarbons such as dichloromethane or dichloroethane, etc., or a mixture of these solvents. The reaction temperature should be suitably selected from the range of about 0°C to about 150°C.

### Method 2

Among the compounds of the formula (1-4), the compound of the formula (2-3) or a pharmaceutically acceptable salt thereof may be prepared, for example, by the following method. 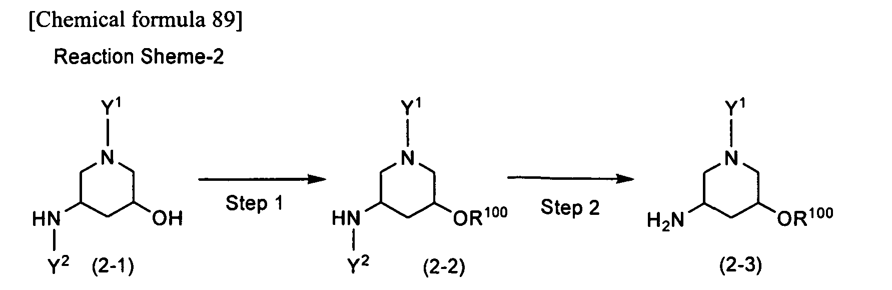 [wherein Y¹ is as defined in the above; Y² is Cbz, Boc or Alloc; R¹⁰⁰ is the same as defined for B of the above-mentioned Item 1]

### 1) Step 1

The compound (2-2) may be prepared fro the compound (2-1) by a similar method to ones disclosed in the literatures (e.g., Comprehensive Organic transformation, R. C. Laroque, VCH publisher Inc., 1989, etc.). The compound (2-1) may be prepared by a similar method to ones disclosed in the literatures (e.g., WO 05/028467, etc.).

### 2) Step 2

The compound (2-3) may be prepared from the compound (2-2) in a similar method to ones disclosed in the literatures (e.g., Protective Groups in Organic Synthesis 2nd Edition (John Wiley & Sons, Inc.), etc.).

### Method 3

Among the compounds of the formula (1-4), the compound of the formula (3-6) or a pharmaceutically acceptable salt thereof may be prepared, for example, by the following method. 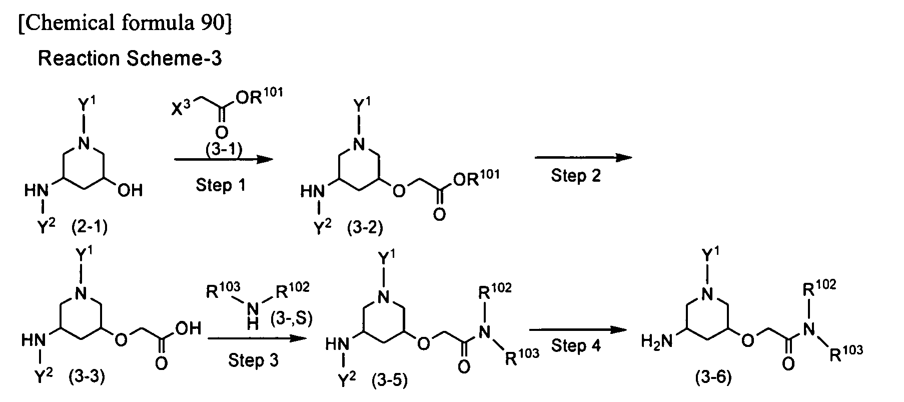 [wherein Y¹ and Y² are as defined in the above; X³ is a chlorine atom or a bromine atom; R¹⁰¹ is a C₁₋₄ alkyl group; R¹⁰² is the same as defined for B of the above-mentioned Item 1; R¹⁰³ is as defined in R⁴ of the above-mentioned Item 1; or R¹⁰² and R¹⁰³ may combine each other to form a ring]

### 1) Step 1

The compound (3-2) may be prepared from the compound (2-1) in a similar method to ones disclosed in the literatures (e.g., Comprehensive Organic transformation, R. C. Laroque, VCH publisher Inc., 1989, etc.). The compound (2-1) may be prepared by a similar method to ones disclosed in the literatures (e.g., WO 05/028467, etc.).

### 2) Step 2 - Step 3

The compound (3-5) may be prepared from the compound (3-2) by a similar method to ones disclosed in the literatures (e.g., Comprehensive Organic transformation, R. C. Laroque, VCH publisher Inc., 1989, etc.).

### 3) Step 4

The compound (3-6) may be prepared from the compound (3-5) by a similar method disclosed in the literatures (e.g., Protective Groups in Organic Synthesis 2nd Edition (John Wiley & Sons, Inc.), etc.).

### Method 4

Among the compounds of the formula (1-4), the compound of the formula (4-3) and the compound of the formula (4-6), or a pharmaceutically acceptable salt thereof may be prepared, for example, by the following method. 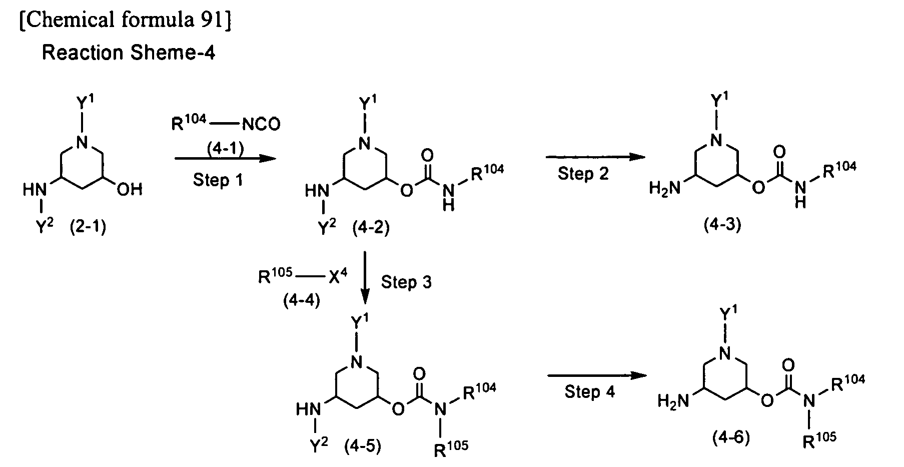 [wherein Y¹ and Y² are as defined in the above; R¹⁰⁴ is the same as defined for B of the above-mentioned Item 1; R¹⁰⁵ is the same as defined for R⁴ of the above-mentioned Item 1; or R¹⁰⁴ and R¹⁰⁵ may combine each other to form a ring; X⁴ is iodine atom, bromine atom, chlorine atom, methanesulfonyloxy group, trifluoromethanesulfonyloxy group, or p-toluenesulfonyloxy group]

### 1) Step 1

The compound (4-2) may be prepared from the compound (2-1) by a similar method to ones disclosed in the literatures (e.g., Comprehensive Organic transformation, R. C. Laroque, VCH publisher Inc., 1989, etc.). The compound (2-1) may be prepared by a similar method to ones disclosed in the literatures (e.g., WO 05/028467, etc.).

### 2) Step 2

The compound (4-3) may be prepared from the compound (4-2) by a similar method to ones disclosed in the literatures (e.g. Protective Groups in Organic Synthesis 2nd Edition (John Wiley & Sons, Inc.), etc.).

### 3) Step 3

The compound (4-5) may be prepared from the compound (4-2) by a similar method to ones disclosed in the literatures (e.g., Comprehensive Organic transformation, R. C. Laroque, VCH publisher Inc., 1989, etc.).

### 4) Step 4

The compound (4-6) may be prepared from the compound (4-5) by a similar method to ones disclosed in the literatures (e.g., Protective Groups in Organic Synthesis 2nd Edition (John Wiley & Sons, Inc.), etc.).

### Method 5

Among the compounds of the formula (1-4), the compound of the formula (5-4) or a pharmaceutically acceptable salt thereof may be prepared, for example, by the following method. 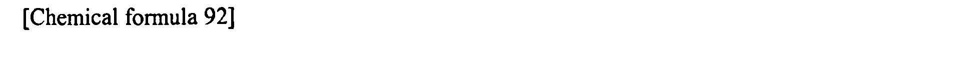 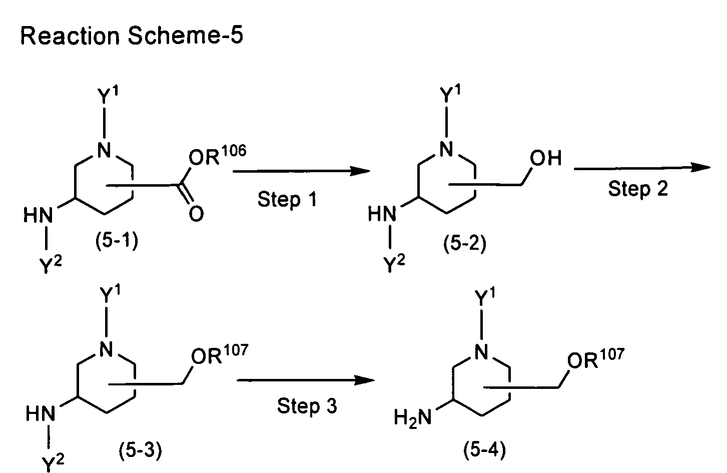 [wherein Y¹ and Y² are as defined in the above; R¹⁰⁶ is a C₁₋₄ alkyl group; R¹⁰⁷ is the same as defined for B of the above-mentioned Item 1. In addition, the substituent crossing the bond indicates that such a substituent bonds to one of the positions of R^{3a}, R^{3b}, R^{3c}, R^{3d} in Item 36. The same for the following formulae]

### 1) Step 1

The compound (5-2) may be prepared from the compound (5-1) by a similar method to ones disclosed in the literatures (e.g., Comprehensive Organic transformation, R. C. Laroque, VCH publisher Inc., 1989, etc.). The compound (5-1) may be prepared by a similar method to ones disclosed in the literatures (e.g., WO 97/18813, WO 02/10172, Tetrahedron Letters 46, 7495 (2005), WO 02/02525, etc.).

### 2) Step 2

The compound (5-3) may be prepared from the compound (5-2) by a similar method to ones disclosed in the literatures (e.g. Comprehensive Organic transformation, R. C. Laroque, VCH publisher Inc., 1989, etc.).

### 3) Step 3

The compound (5-4) may be prepared from the compound (5-3) by a similar method to ones disclosed in the literatures (e.g., Protective groups in Organic Synthesis 2nd Edition (John Wiley & Sons, Inc.), etc.).

### Method 6

Among the compounds of the formula (1-4), the compound of the formula (6-5) or a pharmaceutically acceptable salt thereof may be prepared, for example, by the following method. 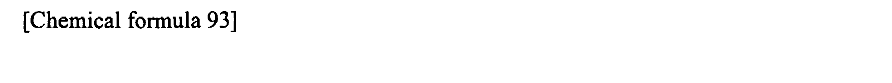 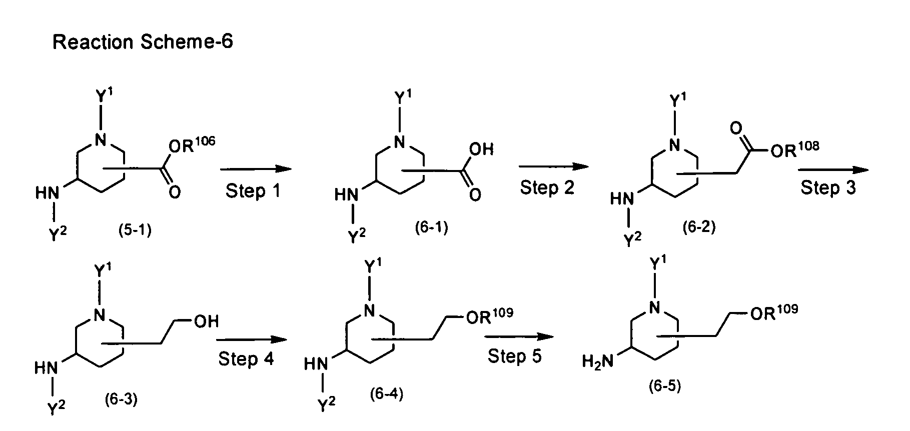 [wherein Y¹, Y² and R¹⁰⁶ are as defined in the above; R¹⁰⁸ is a C₁₋₄ alkyl group; R¹⁰⁹ is the same as defined for B of the above-mentioned Item 1]

### 1) Step 1

The compound (6-1) may be prepared from the compound (5-1) by a similar method to ones disclosed in the literatures (e.g., Comprehensive Organic transformation, R. C. Laroque, VCH publisher Inc., 1989, etc.). The compound (5-1) may be prepared by a similar method to ones disclosed in the literatures (e.g., WO 97/18813, WO 02/10172, Tetrahedron Letters 46, 7495 (2005), WO02/02525, J. Or g. Chem. 70, 6956 (2005), etc.).

### 2) Step 2

The compound (6-2) may be prepared from the compound (6-1) by a similar method to ones disclosed in the literatures (e.g., Comprehensive Organic transformation, R. C. Laroque, VCH publisher Inc., 1989, J. Or g. Chem. 57, 7194 (1992), etc.).

### 3) Step 3 - Step 4

The compound (6-4) may be prepared from the compound (6-2) by a similar method to ones disclosed in the literatures (e.g., Comprehensive Organic transformation, R. C. Laroque, VCH publisher Inc., 1989, etc.).

### 4) Step 5

The compound (6-5) may be prepared from the compound (6-4) by a similar method to ones disclosed in the literatures (e.g., Protective Groups in Organic Synthesis 2nd Edition (John Wiley & Sons, Inc.), etc.).

### Method 7

Among the compounds of the formula (1-4), the compound of the formula (7-4), the compound of the formula (7-6), and the compound of the formula (7-8) or a pharmaceutically acceptable salt thereof may be prepared, for example, by the following method. 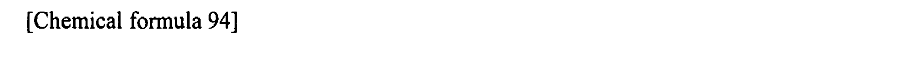 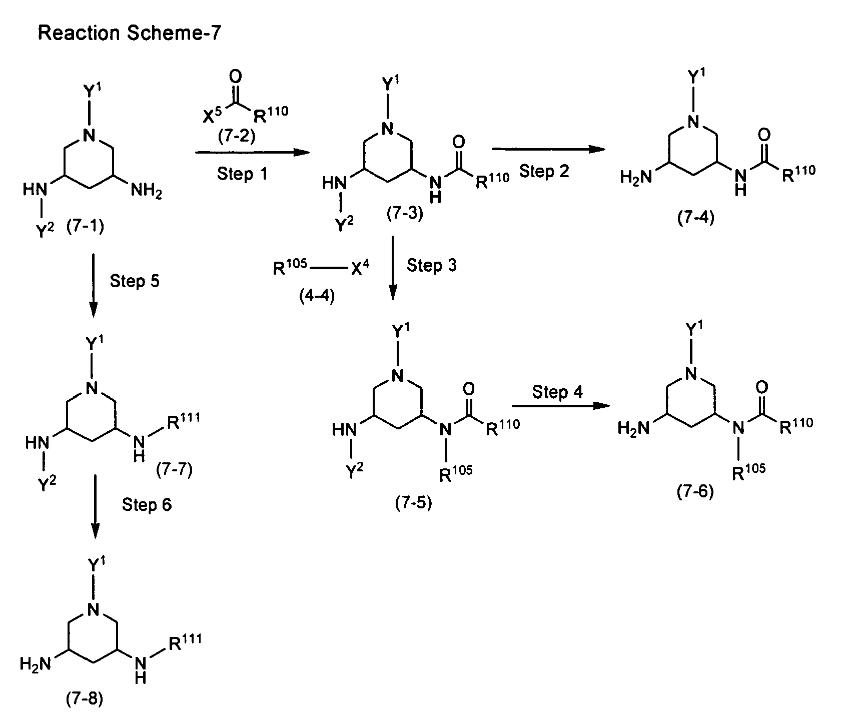 [wherein R¹⁰⁵, X⁴, Y¹ and Y² are as defined in the above; X⁵ is a hydroxy group or a chlorine atom; and R¹¹⁰ and R¹¹¹ are independently the same as B of the above-mentioned Item 1]

### 1) Step 1

The compound (7-3) may be prepared from the compound (7-1) by a similar method to ones disclosed in the literatures (e.g., Comprehensive Organic transformation, R. C. Laroque, VCH publisher Inc., 1989; Chem. Pharm. Bull. 40, 102 (1992); J. Med. Chem. 26, 507 (1983), etc.). The compound (7-1) may be prepared by a similar method to ones disclosed in the literatures (e.g., WO 05/028467, etc.).

### 2) Step 2

The compound (7-4) may be prepared from the compound (7-3) by a similar method to ones disclosed in the literatures (e.g., Protective Groups in Organic Synthesis 2nd Edition (John Wiley & Sons, Inc.), etc.).

### 3) Step 3

The compound (7-5) may be prepared from the compound (7-3) by a similar method to ones disclosed in the literatures (e.g., Comprehensive Organic transformation, R. C. Laroque, VCH publisher Inc., 1989, etc.).

### 4) Step 4

The compound (7-6) may be prepared from the compound (7-5) by a similar method to ones disclosed in the literatures (e.g., Protective Groups in Organic Synthesis 2nd Edition (John Wiley & Sons, Inc.), etc.).

### 5) Step 5

The compound (7-7) may be prepared from the compound (7-1) by a similar method to ones disclosed in the literatures (e.g., Comprehensive Organic transformation, R. C. Laroque, VCH publisher Inc., 1989; J. Org. Chem. 61, 3849 (1996); J. Org. Chem. 68, 4120 (2003); J. Org. Chem. 63, 370 (1998); J. Org. Chem. 70, 2195 (2005), etc.).

### 6) Step 6

The compound (7-8) may be prepared from the compound (7-7) by a similar method to ones disclosed in the literatures (e.g., Protective Groups in Organic Synthesis 2nd Edition (John Wiley & Sons, Inc.), etc.).

### Method 8

Among the compounds of the formula (1-4), the compound of the formula (8-4) or a pharmaceutically acceptable salt thereof may be prepared, for example, by the following method. 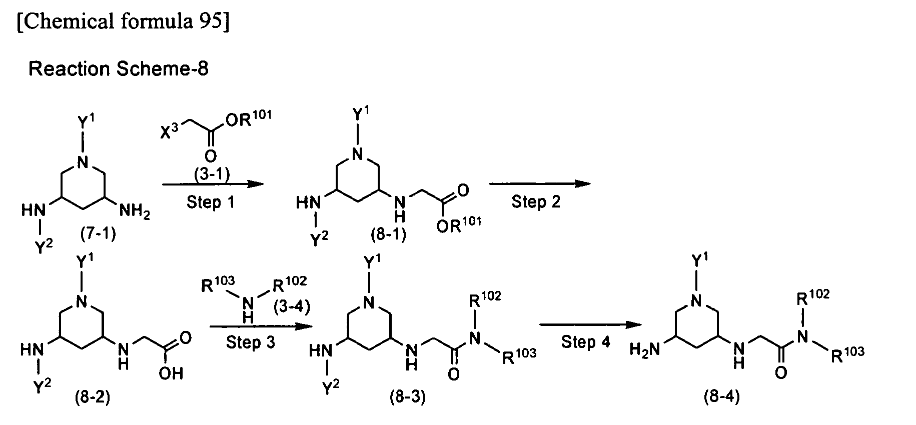 [wherein R¹⁰¹, R¹⁰², R¹⁰³, X³, Y¹ and Y² are as defined in the above]

### 1) Step 1

The compound (8-1) may be prepared from the compound (7-1) by a similar method to ones disclosed in the literatures (e.g., Comprehensive Organic transformation, R. C. Laroque, VCH publisher Inc., 1989; Synthetic Communications 34, 219 (2004); etc.). The compound (7-1) may be prepared by a similar method to ones disclosed in the literatures (e.g., WO 05/028467, etc.).

### 2) Step 2 - Step 3

The compound (8-3) may be prepared from the compound (8-1) by a similar method to ones disclosed in the literatures (e.g., Comprehensive Organic transformation, R. C. Laroque, VCH publisher Inc., 1989, etc.).

### 3) Step 4

The compound (8-4) may be prepared from the compound (8-3) by a similar method to ones disclosed in the literatures (e.g., Protective Groups in Organic Synthesis 2nd Edition (John Wiley & Sons, Inc.), etc.).

### Method 9

Among the compounds of the formula (1-4), the compound of the formula (9-4) or a pharmaceutically acceptable salt thereof may be prepared, for example by the following method. 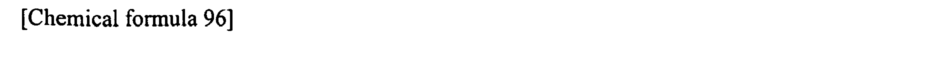 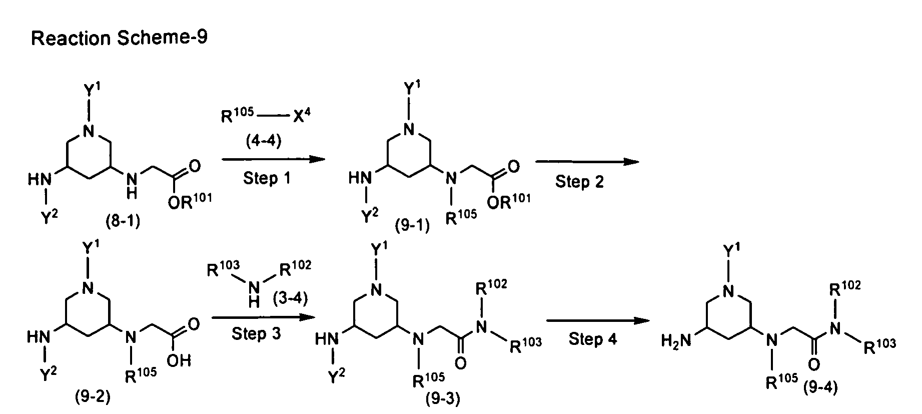 [wherein R¹⁰¹, R¹⁰², R¹⁰³, R¹⁰⁵, X⁴, Y¹ and Y² are as defined in the above]

### 1) Step 1 - Step 3

The compound (9-3) may be prepared from the compound (8-1) by a similar method to ones disclosed in the literatures (e.g., Comprehensive Organic transformation, R. C. Laroque, VCH publisher Inc., 1989, etc.).

### 2) Step 4

The compound (9-4) may be prepared from the compound (9-3) by a similar method to ones disclosed in the literatures (e.g., Protective Groups in Organic Synthesis 2nd Edition (John Wiley & Sons, Inc.), etc.).

### Method 10

Among the compounds of the formula (1-4), the compound of the formula (10-4) and the compound of the formula (10-6) or a pharmaceutically acceptable salt thereof may be prepared, for example, by the following method. 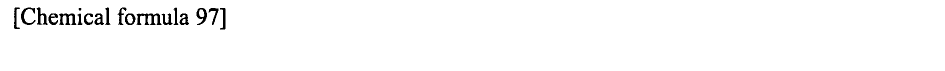 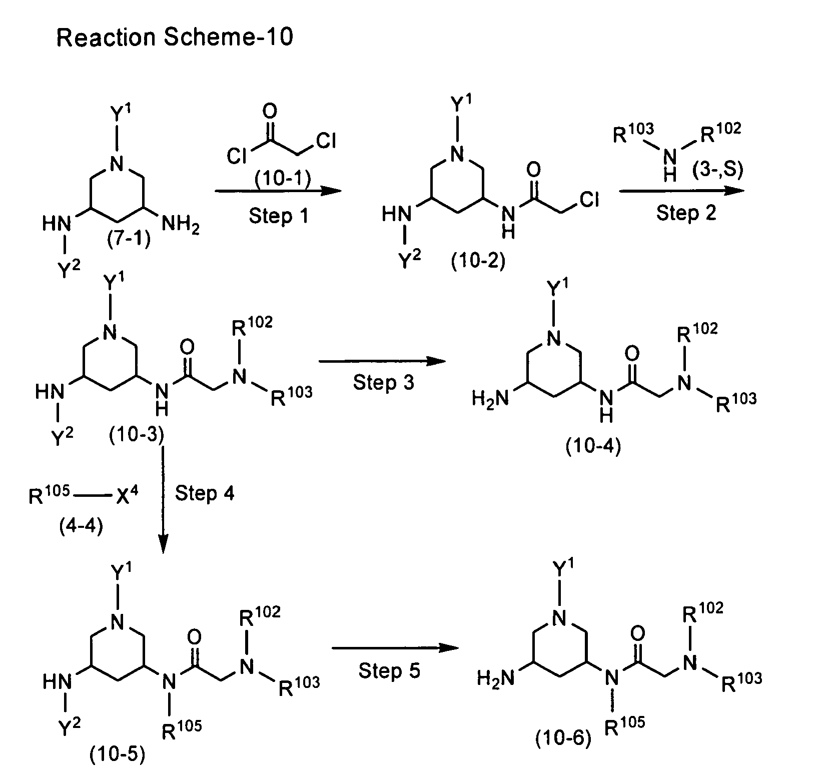 [wherein R¹⁰², R¹⁰³, R¹⁰⁵, X⁴, Y¹ and Y² are as defined in the above]

### 1) Step 1

The compound (10-2) may be prepared from the compound (7-1) by a similar method to ones disclosed in the literatures (e.g., Tetrahedron: Asymmetry 16, 2599 (2005), etc.). The compound (7-1) may be prepared by a similar method to ones disclosed in the literatures (e.g., WO 05/028467, etc.).

### 2) Step 2

The compound (10-3) may be prepared from the compound (10-2) by a similar method to ones disclosed in the literatures (e.g., Tetrahedron: Asymmetry 16, 2599 (2005), etc.).

### 3) Step 3

The compound (10-4) may be prepared from the compound (10-3) by a similar method to ones disclosed in the literatures (e.g., Protective Groups in Organic Synthesis 2nd Edition (John Wiley & Sons, Inc.), etc.).

### 4) Step 4

The compound (10-5) may be prepared from the compound (10-3) by a similar method to ones disclosed in the literatures (e.g., Comprehensive Organic transformation, R. C. Laroque, VCH publisher Inc., 1989, etc.).

### 5) Step 5

The compound (10-6) may be prepared from the compound (10-5) by a similar method to ones disclosed in the literatures (e.g., Protective Groups in Organic Synthesis 2nd Edition (John Wiley & Sons, Inc.), etc.).

### Method 11

Among the compounds of the formula (1-4), the compound of the formula (11-3) and the compound of the formula (11-5) or a pharmaceutically acceptable salt thereof may be prepared, for example, by the following method. 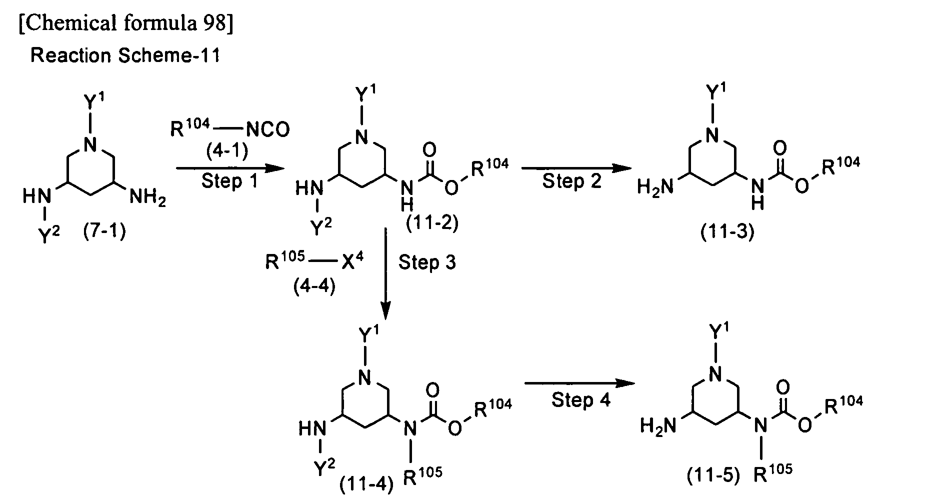 [wherein R¹⁰⁴, R¹⁰⁵, X⁴, Y¹ and Y² are as defined in the above]

### 1) Step 1

The compound (11-2) may be prepared from the compound (7-1) by a similar method to ones disclosed in the literatures (e.g., Comprehensive Organic transformation, R. C. Laroque, VCH publisher Inc., 1989; WO 05/095357; WO 05/085275; etc.). The compound (7-1) may be prepared by a similar method to ones disclosed in the literatures (e.g., WO 05/028467, etc.).

### 2) Step 2

The compound (11-3) may be prepared from the compound (11-2) by a similar method to ones disclosed in the literatures (e.g., Protective Groups in Organic Synthesis 2nd Edition (John Wiley & Sons, Inc.), etc.).

### 3) Step 3

The compound (11-4) may be prepared from the compound (11-2) by a similar method to ones disclosed in the literatures (e.g., Comprehensive Organic transformation, R. C. Laroque, VCH publisher Inc., 1989, etc.).

### 4) Step 4

The compound (11-5) may be prepared from the compound (11-4) by a similar method to ones disclosed in the literatures (e.g., Protective Groups in Organic Synthesis 2nd Edition (John Wiley & Sons, Inc.), etc.).

### Method 12

Among the compounds of the formula (1-4), the compound of the formula (12-3) or a pharmaceutically acceptable salt thereof may be prepared, for example, by the following method. 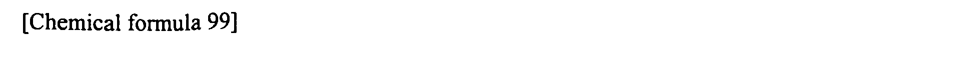 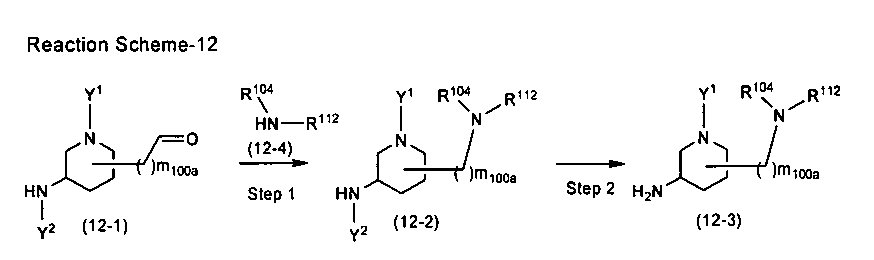 [wherein R¹⁰⁴, Y¹ and Y² are as defined in the above; R¹¹² is the same as defined for R⁴ of the above-mentioned Item 1, R¹⁰⁴ and R¹¹² may combine each other to form a ring and m₁₀₀ₐ is 1 or 2.]

### 1) Step 1

The compound (12-2) may be prepared from the compound (12-1) by a similar method to ones disclosed in the literatures (e.g., Comprehensive Organic transformation, R. C. Laroque, VCH publisher Inc., 1989, etc.)..

### 2) Step 2

The compound (12-3) may be prepared from the compound (12-2) by a similar method to ones disclosed in the literatures (e.g., Protective Groups in Organic Synthesis 2nd Edition (John Wiley & Sons, Inc.), etc.).

### Method 13

Among the compounds of the formula (1-4), the compound of the formula (13-4) and the compound of the formula (13-6) or a pharmaceutically acceptable salt thereof may be prepared, for example, by the following method. 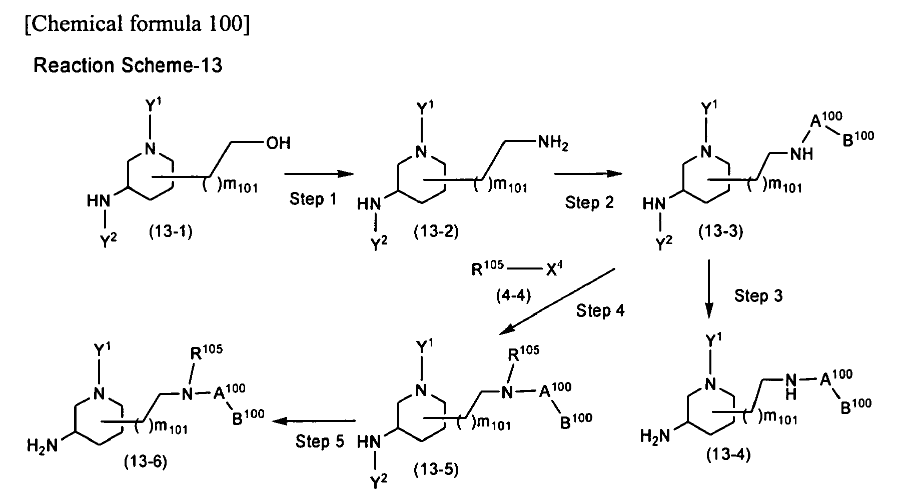 [wherein R¹⁰⁵, X⁴, Y¹ and Y² are as defined in the above; A¹⁰⁰ is -SO₂-, or -CO-; B¹⁰⁰ is the same as defined in B of the above-mentioned Item 1; m₁₀₁ is an integer of 0 or 1]

### 1) Step 1 - Step 2

The compound (13-3) may be prepared from the compound (13-1) by a similar method to ones disclosed in the literatures (e.g., Comprehensive Organic transformation, R. C. Laroque, VCH publisher Inc., 1989, etc.).

### 2) Step 3

The compound (13-4) may be prepared from the compound (13-3) by a similar method to ones disclosed in the literatures (e.g., Protective Groups in Organic Synthesis 2nd Edition (John Wiley & Sons, Inc.), etc.).

### 3) Step 4

The compound (13-5) may be prepared from the compound (13-3) by a similar method to ones disclosed in the literatures (e.g., Comprehensive Organic transformation, R. C. Laroque, VCH publisher Inc., 1989, etc.).

### 4) Step 5

The compound (13-6) may be prepared from the compound (13-5) by a similar method to ones disclosed in the literatures (e.g., Protective Groups in Organic Synthesis 2nd Edition (John Wiley & Sons, Inc.), etc.).

### Method 14

Among the compounds of the formula (1-4), the compound of the formula (14-2) or a pharmaceutically acceptable salt thereof may be prepared, for example, by the following method. 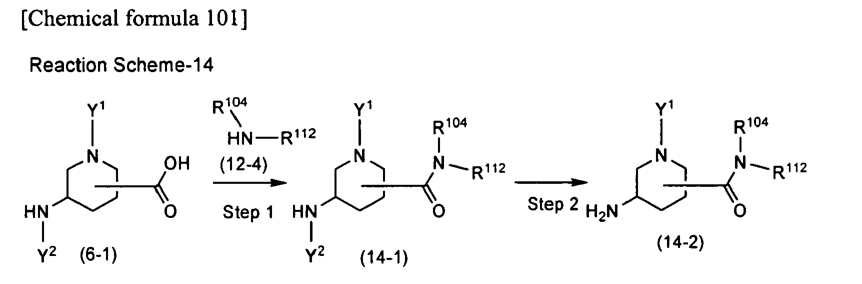 [wherein R¹⁰⁴, R¹¹², Y¹ and Y² are as defined in the above]

### 1) Step 1

The compound (14-1) may be prepared from the compound (6-1) by a similar method to ones disclosed in the literatures (e.g., Comprehensive Organic transformation, R. C. Laroque, VCH publisher Inc., 1989, etc.).

### 2) Step 2

The compound (14-2) may be prepared from the compound (14-1) by a similar method to ones disclosed in the literatures (e.g., Protective Groups in Organic Synthesis 2nd Edition (John Wiley & Sons, Inc.), etc.).

### Method 15

Among the compounds of the formula (1-4), the compound of the formula (15-3) or a pharmaceutically acceptable salt thereof may be prepared, for example, by the following method. 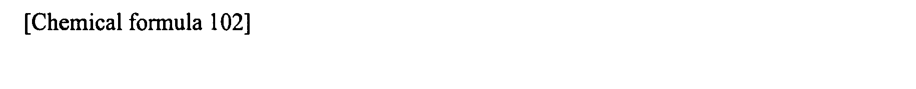 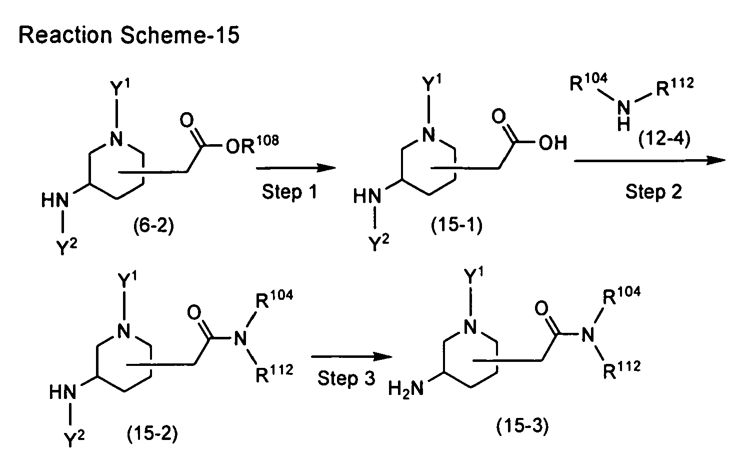 [wherein R¹⁰⁴, R¹¹², R¹⁰⁸, Y¹ and Y² are as defined in the above]

### 1) Step 1 - Step 2

The compound (15-2) may be prepared from the compound (6-2) by a similar method to ones disclosed in the literatures (e.g., Comprehensive Organic transformation, R. C. Laroque, VCH publisher Inc., 1989, etc.).

### 2) Step 3

The compound (15-3) may be prepared from the compound (15-2) by a similar method to ones disclosed in the literatures (e.g., Protective Groups in Organic Synthesis 2nd Edition (John Wiley & Sons, Inc.), etc.).

### Method 16

Among the compounds of the formula (1-4), the compound of the formula (16-4) and the compound of the formula (16-6) or a pharmaceutically acceptable salt thereof may be prepared, for example, by the following method. 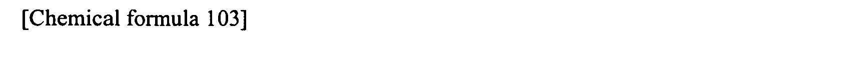 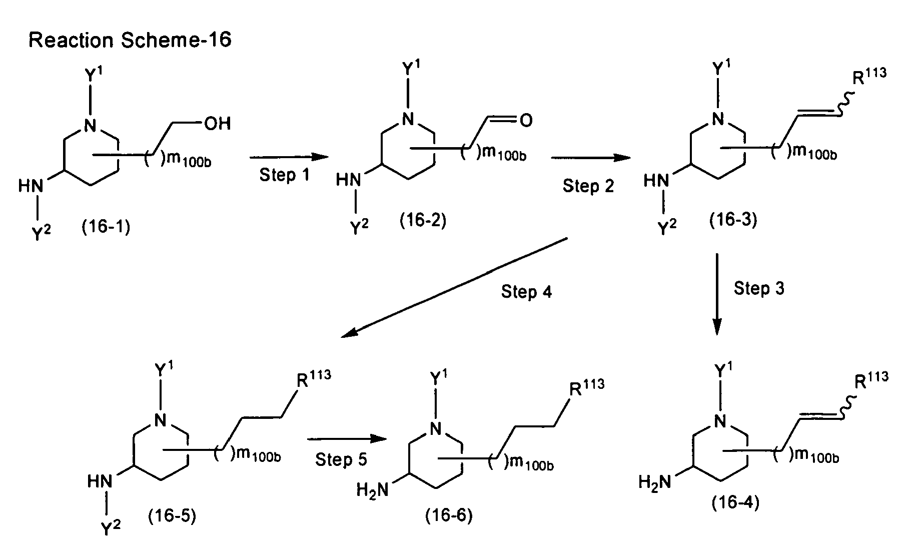 [wherein Y¹ and Y² are as defined in the above; m_{100b} is an integer of 0 to 4; R¹¹³ is one of the above-mentioned (a) to (y), provided that in the compound (16-4), R¹¹³ is one of the above-mentioned (a) to (s)]

### 1) Step 1

The compound (16-2) may be prepared from the compound (16-1) by a similar method to ones disclosed in the literatures (e.g., Tetrahedron: Asymmetry 17, 993 (2006), Comprehensive Organic transformation, R. C. Laroque, VCH publisher Inc., 1989, etc.).

### 2) Step 2

The compound (16-3) may be prepared from the compound (16-2) by a similar method to ones disclosed in the literatures (e.g., Tetrahedron: Asymmetry 8, 3685 (1997); J. Or g. Chem. 61, 6033 (1996); JP-A-8-12605; Comprehensive Organic transformation, R. C. Laroque, VCH publisher Inc., 1989, etc.).

### 3) Step 3

The compound (16-4) may be prepared from the compound (16-3) by a similar method to ones disclosed in the literatures (e.g., Protective Groups in Organic Synthesis 2nd Edition (John Wiley & Sons, Inc.), etc.).

### 4) Step 4

The compound (16-5) may be prepared from the compound (16-3) by a similar method to ones disclosed in the literatures (e.g., Comprehensive Organic transformation, R. C. Laroque, VCH publisher Inc., 1989, etc.).

### 5) Step 5

The compound (16-6) may be prepared from the compound (16-5) by a similar method to ones disclosed in the literatures (e.g., Protective Groups in Organic Synthesis 2nd Edition (John Wiley & Sons, Inc.), etc.).

### Method 17

Among the compounds of the formula (1-4), the compound of the formula (17-3) and the compound of the formula (17-5) or a pharmaceutically acceptable salt thereof may be prepared, for example, by the following method. 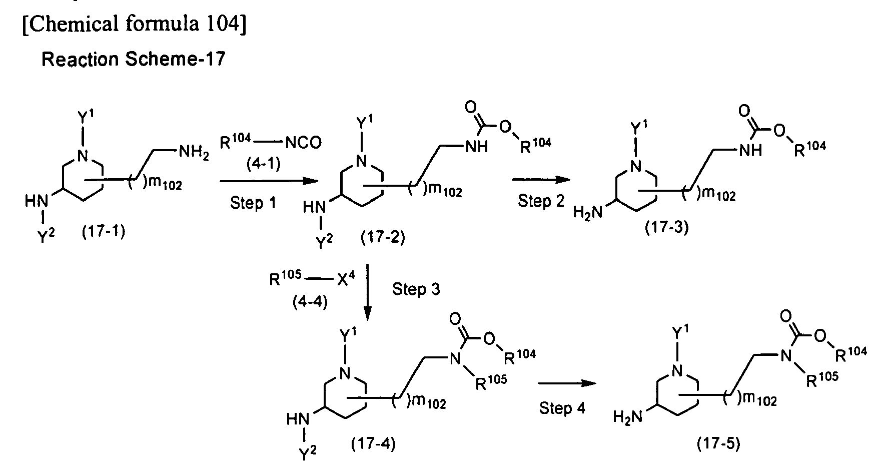 [wherein R¹⁰⁴, R¹⁰⁵, X⁴, Y¹ and Y² are as defined in the above; m₁₀₂ is an integer of 0 or 1]

### 1) Step 1

The compound (17-2) may be prepared from the compound (17-1) by a similar method to ones disclosed in the literatures (e.g., Comprehensive Organic transformation, R. C. Laroque, VCH publisher Inc., 1989; WO05/085275, etc.).

### 2) Step 2

The compound (17-3) may be prepared from the compound (17-2) by a similar method to ones disclosed in the literatures (e.g., Protective Groups in Organic Synthesis 2nd Edition (John Wiley & Sons, Inc.), etc.).

### 3) Step 3

The compound (17-4) may be prepared from the compound (17-2) by a similar method to ones disclosed in the literatures (e.g., Comprehensive Organic transformation, R. C. Laroque, VCH publisher Inc., 1989, etc.).

### 4) Step 4

The compound (17-5) may be prepared from the compound (17-4) by a similar method to ones disclosed in the literatures (e.g., Protective Groups in Organic Synthesis 2nd Edition (John Wiley & Sons, Inc.), etc.).

### Method 18

Among the compounds of the formula (1-4), the compound of the formula (18-5) and the compound of the formula (18-7) or a pharmaceutically acceptable salt thereof may be prepared, for example, by the following method. 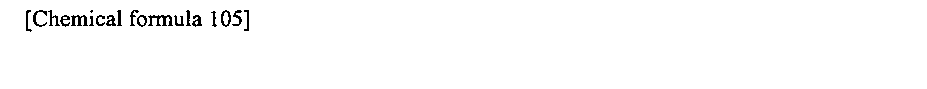 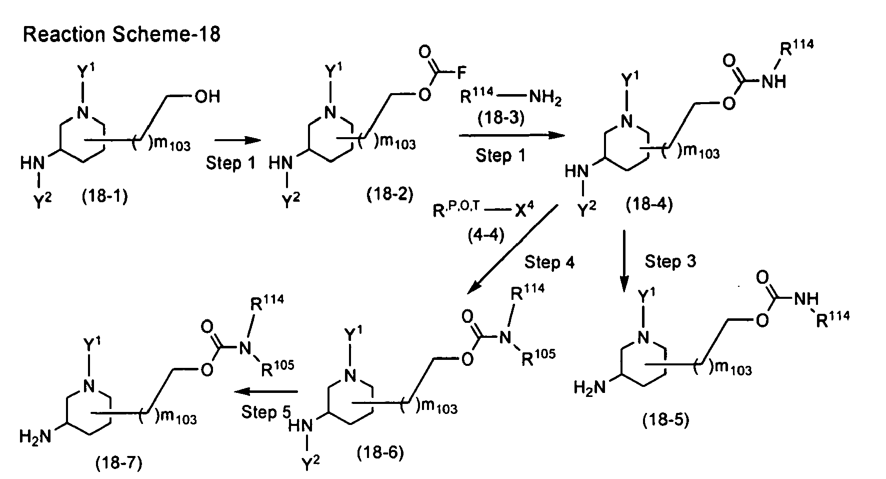 [wherein R¹⁰⁵, X⁴, Y¹ and Y² are as defined in the above; m₁₀₃ is an integer of 0 or 1; R¹¹⁴ is the same as defined for B of the above-mentioned Item 1]

### 1) Step 1

The compound (18-2) may be prepared from the compound (18-1) by a similar method to ones disclosed in the literatures (e.g., Tetrahedron Letters 43, 4275 (2002), etc.).

### 2) Step 2

The compound (18-4) may be prepared from the compound (18-2) by a similar method to ones disclosed in the literatures (e.g., Comprehensive Organic transformation, R. C. Laroque, VCH publisher Inc., 1989, etc.).

### 3) Step 3

The compound (18-5) may be prepared from the compound (18-4) by a similar method to ones disclosed in the literatures (e.g., Protective Groups in Organic Synthesis 2nd Edition (John Wiley & Sons, Inc.), etc.).

### 4) Step 4

The compound (18-6) may be prepared from the compound (18-4) by a similar method to ones disclosed in the literatures (e.g., Comprehensive Organic transformation, R. C. Laroque, VCH publisher Inc., 1989, etc.).

### 5) Step 5

The compound (18-7) may be prepared from the compound (18-6) by a similar method to ones disclosed in the literatures (e.g., Protective Groups in Organic Synthesis 2nd Edition (John Wiley & Sons, Inc.), etc.).

### Method 19

Among the compounds of the formula (1-4), the compound of the formula (19-13) or a pharmaceutically acceptable salt thereof may be prepared, for example, by the following method. 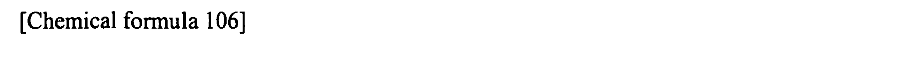 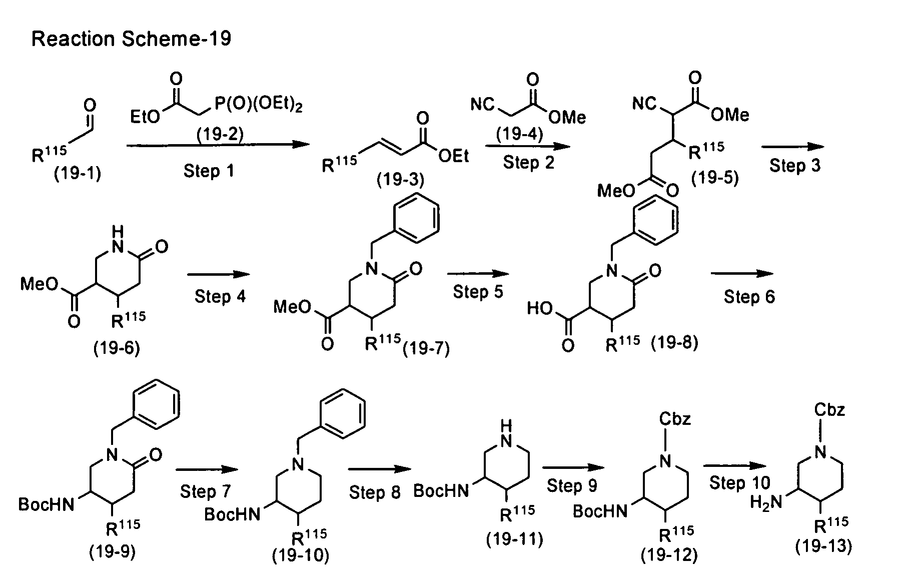 [wherein R¹¹⁵ is the same as defined for B of the above-mentioned Item 1]

### 1) Step 1 - Step 8

The compound (19-11) may be prepared from the compound (19-1) by a similar method to ones disclosed in the literatures (e.g., WO 06/039325, etc.).

### 2) Step 9 - Step 10

The compound (19-13) may be prepared from the compound (19-11) by a similar method to ones disclosed in the literatures (e.g., Protective Groups in Organic Synthesis 2nd Edition (John Wiley & Sons, Inc.), etc.).

### Method 20

Among the compounds of the formula (1-4), the compound of the formula (20-8) a pharmaceutically acceptable salt thereof may be prepared, for example, by the following method. 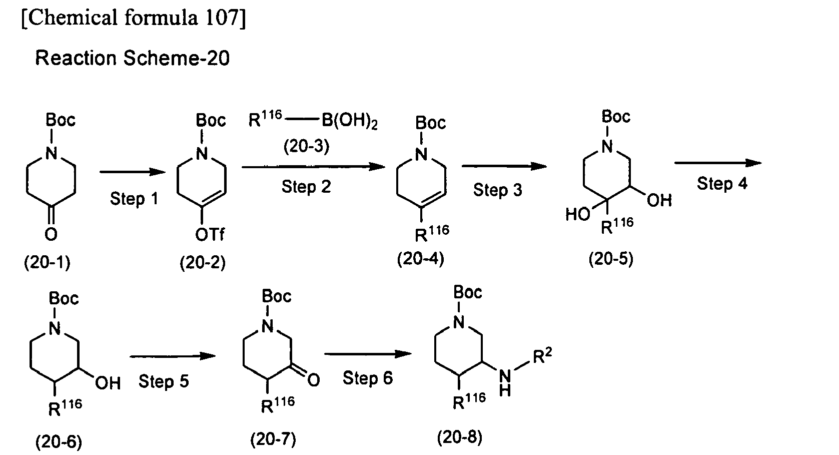 [wherein R² is as defined in the above Item 1; R¹¹⁶ is an optionally substituted C₆₋₁₀ aryl group, or an optionally substituted 5- to 10-membered monocyclic or polycyclic heteroaryl group.]

### 1) Step 1 - Step 5

The compound (20-7) may be prepared from the compound (20-1) by a similar method to ones disclosed in the literatures (e.g., Bioorganic & Medicinal Chemistry 13, 59 (2005), etc.).

### 2) Step 6

The compound (20-8) may be prepared from the compound (20-7) by a similar method to ones disclosed in the literatures (e.g., Protective Groups in Organic Synthesis 2nd Edition (John Wiley & Sons, Inc.), etc.).

### Method 21

Among the compounds of the formula (1-1), the compound of the formula (21-5) or a pharmaceutically acceptable salt thereof may be prepared, for example, by the following method.

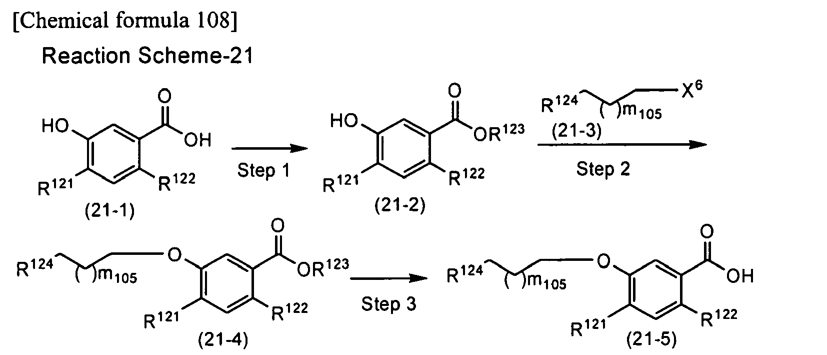

[wherein R¹²¹ and R¹²² are independently an optionally substituted C₁₋₆ alkyl group, an optionally substituted C₃₋₁₀ cycloalkyl group, an optionally substituted C₁₋₆ alkoxy group, an optionally substituted C₃₋₁₀ cycloalkyloxy group, or a halogen atom; R¹²³ is a C₁₋₄ alkyl group; R¹²⁴ is a hydroxy group, a C₁₋₄ alkoxy group, a C₃₋₆ cycloalkoxy group, a trifluoromethyl group, a trifluoromethoxy group, a carboxyl group, a mono-C₁₋₆ alkylcarbonylamino group, or a mono-C₁-₆ alkoxycarbonylamino group; m₁₀₅ is an integer of 0 to 4; X⁶ is a iodine atom, a bromine atom, a chlorine atom, a methanesulfonyloxy group, a trifluoromethanesulfonyloxy group, or a p-toluenesulfonyloxy group]

### 1) Step 1

The compound (21-2) may be prepared from the compound (21-1) by a similar method to ones disclosed in the literatures (e.g., Protective Groups in Organic Synthesis 2nd Edition (John Wiley & Sons, Inc.), etc.). The compound (21-1) may be a commercially available reagent or may be prepared by a conventional method from well-known compounds, and the following compounds and the following methods can be exemplified.

### [Chemical formula 109]

| Literatures disclosing methods | Literatures disclosing methods |
|---|---|
| 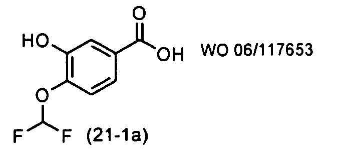 | 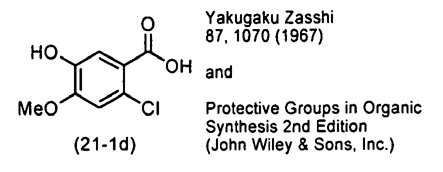 |
| 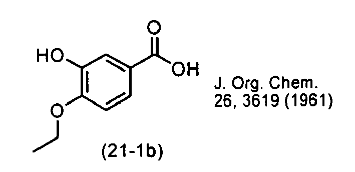 | 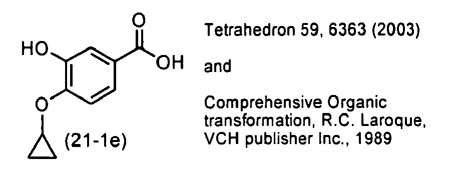 |
| 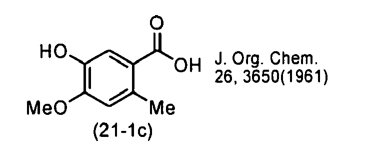 | |

### 2) Step 2

The compound (21-4) may be prepared from the compound (21-2) by a similar method to Step 7 of Method 1.

### 3) Step 3

The compound (21-5) may be prepared from the compound (21-4) by a similar method to ones disclosed in the literatures (e.g., Protective Groups in Organic Synthesis 2nd Edition (John Wiley & Sons, Inc.), etc.).

### Method 22

Among the compounds of the formula (1-4), the compound of formula (22-3) or a pharmaceutically acceptable salt thereof may be prepared, for example, by the following method. 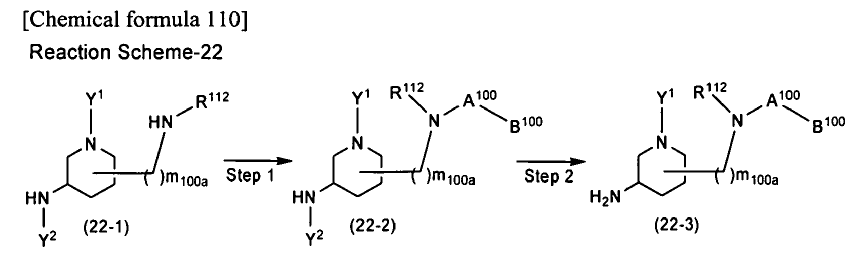 [wherein m₁₀₀ₐ, R¹¹², A¹⁰⁰, B¹⁰⁰, Y¹ and Y² are as defined in the above]

### 1) Step 1

The compound (22-2) may be prepared from the compound (22-1) by a similar method to Step 2 of Method 13. The compound (22-1) may be prepared by a similar method to ones disclosed in Method 12.

### 2) Step 2

The compound (22-3) may be prepared from the compound (22-2) by a similar method to ones disclosed in the literatures (e.g., Protective Groups in Organic Synthesis 2nd Edition (John Wiley & Sons, Inc.), etc.).

### Method 23

Among the compounds of the formula (1-1), the compound of the formula (23-7) or a pharmaceutically acceptable salt thereof may be prepared, for example, by the following method.

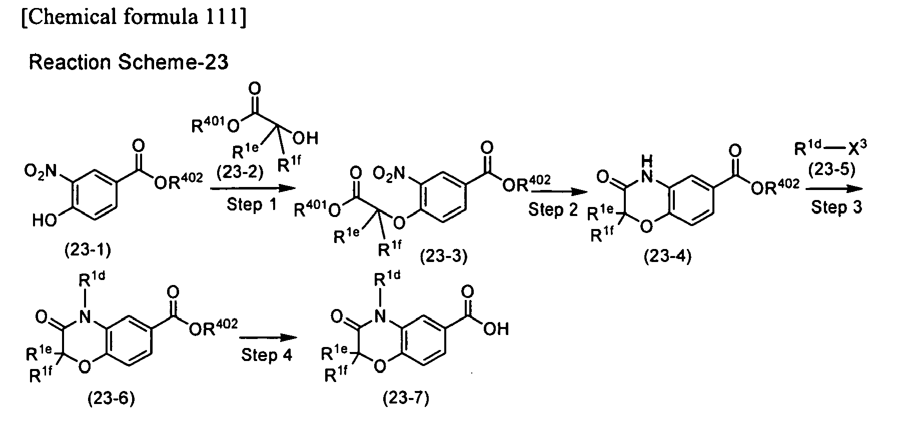

[wherein X³, R^{1d}, R^{1e} and R^{1f} are as defined in the above; R⁴⁰¹ is a C₁₋₆ alkyl group; R⁴⁰² is a C₁₋₂ alkyl group]

### 1) Step 1

The compound (23-3) may be prepared by reacting the compound (23-1) with the compound (23-2) in the presence of a phosphine and a condensing agent in an inert solvent. The phosphine includes, for example, triphenylphosphine, etc., and the inert solvent includes, for example, ethers such as tetrahydrofuran, diethyl ether, 1,4-dioxane, 1,2-dimethoxyethane, etc. The condensing agent includes, for example, azodicarboxylic acid diisopropyl ester, etc. The reaction temperature should be selected from the range of about 0°C to about 80°C.

### 2) Step 2

Step 2 may by carried out, for example, by the following method (i. or ii.).
i. The compound (23-4) may be prepared by reacting iron and the compound (23-3) in an inert solvent. The inert solvent includes, for example, water, acetic acid, or alcohols such as methanol, ethanol, 2-propanol, etc., or a mixture of these solvents. The reaction temperature should be selected from the range of about 30°C to about 100°C.
ii. The compound (23-4) may be prepared by subjecting the compound (23-3) to hydrogenation in the presence of palladium carbon or palladium hydroxide in an inert solvent. The inert solvent includes, for example, alcohols such as methanol, ethanol, 2-propanol, etc. or ethers such as tetrahydrofuran, diethyl ether, 1,4-dioxane, 1,2-dimethoxyethane, etc. The reaction temperature should be selected from the range of about 0°C to about 50°C.

### 3) Step 3

The compound (23-6) may be prepared from the compound (23-4) by a similar method to Step 3 of Method 24.

### 4) Step 4

The compound (23-7) may be prepared from the compound (23-6) by a similar method to Step 2 of Method 3.

### Method 24

Among the compounds of the formula (1-1), the compound of the formula (24-6) or a pharmaceutically acceptable salt thereof may be prepared, for example, by the following method.

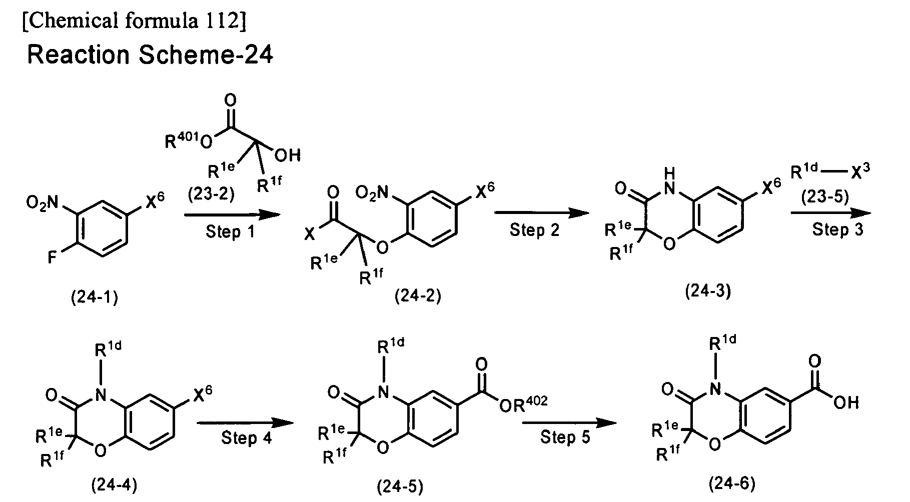

[wherein X³, R^{1d}, R^{1e}, R^{1f}, R⁴⁰¹ and R⁴⁰² are as defined in the above; X⁶ is a bromine atom or a iodine atom]

### 1) Step 1

The compound (24-2) may be prepared by reacting the compound (24-1) with the compound (23-2) in the presence or absence of a crown ether and in the presence of sodium hydride in an inert solvent. The crown ether includes, for example, 15-crown, etc. The inert solvent includes, for example, ethers such as tetrahydrofuran, diethyl ether, 1,4-dioxane, 1,2-dimethoxyethane, etc. The reaction temperature should be selected from the range from about 0°C to about 50°C.

### 2) Step 2

The compound (24-3) may be prepared from the compound (24-2) by a similar method to Step 2 of Method 23.

### 3) Step 3

The compound (24-4) may be prepared by reacting the compound (24-3) with the compound (24-5) in the presence of an inorganic base in an inert solvent. The inorganic base includes, for example, potassium carbonate, sodium hydride, etc. The inert solvent includes, for example, ethers such as tetrahydrofuran, diethyl ether, 1,4-dioxane, 1,2-dimethoxyethane, etc., nitrile solvents such as acetonitrile, propionitrile, etc. The reaction temperature should be selected from the range from about 30°C to about 100°C.

### 4) Step 4

The compound (24-5) may be prepared by reacting the compound (24-4) under carbon oxide atmosphere in the presence of methanol or ethanol, an organic base, an auxiliary ligand and palladium acetate in an inert solvent. The auxiliary ligand includes diphenylphosphinopropane, etc. The organic base includes N,N-diisopropylethylamine, etc. The inert solvent includes, for example, acetamide solvents such as dimethylacetamide, etc. The reaction temperature should be selected from the range from about 70°C to about 150°C.

### 5) Step 5

The compound (24-6) may be prepared from the compound (24-5) by a similar method to Step 2 of Method 3.

### Method 25

Among the compounds of the formula (1-3), the compound of the formula (25-8) or a pharmaceutically acceptable salt thereof may be prepared, for example, by the following method.

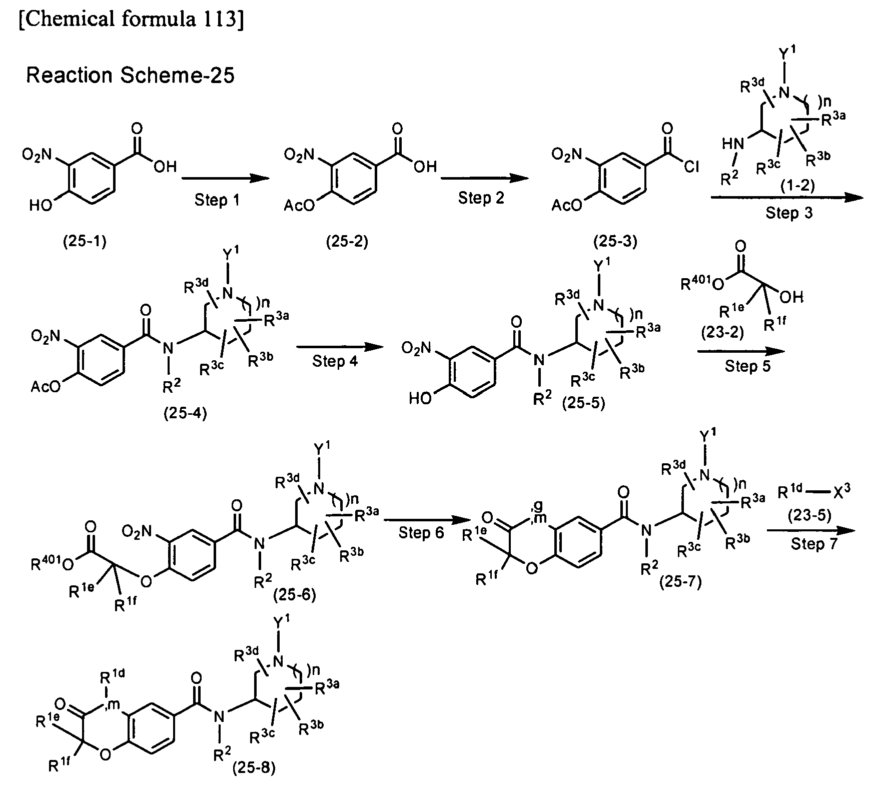

[wherein X³, R^{1d}, n, R⁴⁰¹, Y¹, R^{1e}, R^{1f}, R^{3a}, R^{3b}, R^{3c}, R^{3d} and R² are as defined in the above]

### 1) Step 1

The compound (25-2) may be prepared, for example, by reacting the compound (25-1) with acetic anhydride in a pyridine solvent. The reaction temperature should be selected from the range from about 10°C to about 40°C.

### 2) Step 2

The compound (25-3) may be prepared from the compound (25-2) in a similar manner to Step 1 of Method 1.

### 3) Step 3

The compound (25-4) may be prepared from the compound (25-3) in a similar manner to Step 1 of Method 1.

### 4) Step 4

The compound (25-5) may be prepared by reacting the compound (25-4) with a base in the presence or absence of Amberlite (registered trade name) in an inert solvent. The base includes inorganic bases such as sodium hydrogen carbonate, potassium hydrogen carbonate, sodium carbonate, potassium carbonate, sodium hydroxide, sodium hydride, etc. The inert solvent includes, for example, alcohols such as methanol, ethanol, 2-propanol, etc. The reaction temperature should be selected from the range from about 10°C to about 40°C.

### 5) Step 5

The compound (25-6) may be prepared from the compound (25-5) in a similar manner to Step 1 of Method 1.

### 6) Step 6

The compound (25-7) may be prepared from the compound (25-6) in a similar manner to Step 6 of Method 1.

### 7) Step 7

The compound (25-8) may be prepared from the compound (25-7) in a similar manner to Step 3 of Method 24.

### Method 26

The compound of the formula (24-6) or a pharmaceutically acceptable salt thereof may be prepared, for example, by the following method.

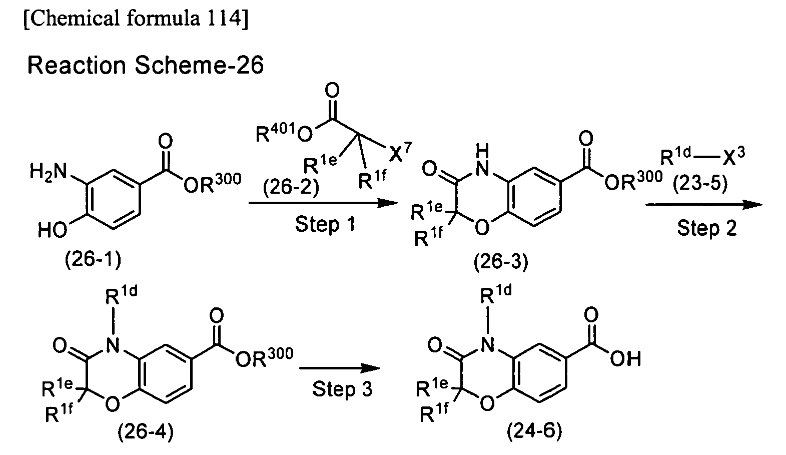

[wherein X³, R^{1d}, R^{1e}, R^{1f}, R⁴⁰¹ and R³⁰⁰ are as defined above; X⁷ is a bromine atom or a chlorine atom]

### 1) Step 1

The compound (26-3) may be prepared from the compound (26-1) by a similar method to ones disclosed in the literatures (e.g., Chem. Pharm. Bull. 46, 1716 (1998), etc.).

### 2) Step 2

The compound (26-4) may be prepared from the compound (26-3) in a similar manner to Step 3 of Method 24.

### 3) Step 3

The compound (26-6) may be prepared from the compound (26-4) in a similar manner to Step 2 of Method 3.

### Method 27

Among the compounds of the formula (1-1), the compound of the formula (27-6) or a pharmaceutically acceptable salt thereof may be prepared, for example, by the following method.

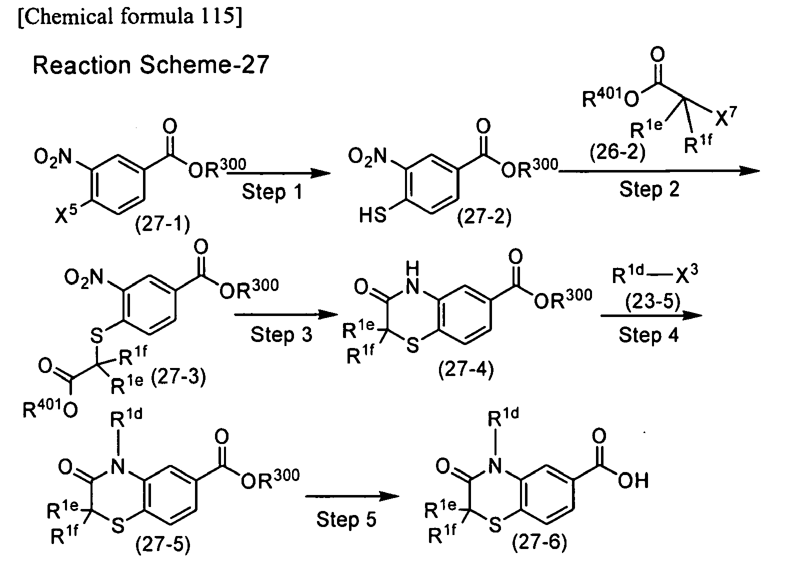

[wherein X³, R^{1d}, X⁷, R^{1e}, R^{1f}, R⁴⁰¹ and R³⁰⁰ are as defined in the above]

### 1) Step 1

The compound (27-2) may be prepared from the compound (27-1) by a similar method to ones disclosed in the literatures (e.g., Synth. Commun. 27, 2943 (1997), J. Chem. Soc. Perkin Trans 2, 691 (1988), etc.).

### 2) Step 2 and Step 3

The compound (27-4) may be prepared from the compound (27-2) by a similar method to ones disclosed in the literatures (e.g., WO 2005/082872, etc.).

### 3) Step 4

The compound (27-5) may be prepared from the compound (27-4) by a similar method to Step 3 of Method 24.

### 4) Step 5

The compound (27-6) may be prepared from the compound (27-5) by a similar method to Step 2 of Method 3.

### Method 28

Among the compounds of the formula (1-1), the formula (28-6) or a pharmaceutically acceptable salt thereof may be prepared, for example, by the following method.

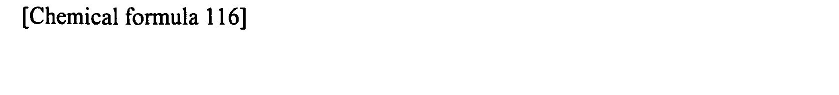

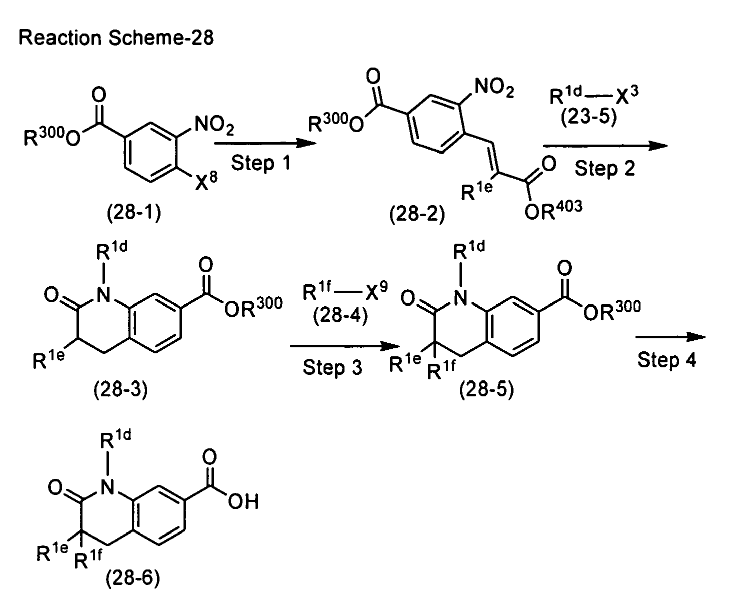

[wherein X³, R^{1d}, R^{1e}, R^{1f} and R³⁰⁰ are as defined in the above; R⁴⁰³ is a C₁₋₆ alkyl group; X⁹ is a iodine atom, a bromine atom, a chlorine atom, a methanesulfonyloxy group, a trifluoromethanesulfonyloxy group or a p-toluenesulfonyloxy group]

### 1) Step 1

The compound (28-2) may be prepared from the compound (28-1) by a similar method to ones disclosed in the literatures (e.g., J. Am. Chem. Soc. 123, 6989 (2001), J. Or g. Chem. 70, 4360 (2005), Synth. Commun. 29, 591 (1999), etc.).

### 2) Step 2

The compound (28-3) may be prepared from the compound (28-2) by a similar method to Step 8 of Method 1.

### 3) Step 3

The compound (28-5) may be prepared from the compound (28-3) by a similar method to ones disclosed in the literatures (e.g., WO 2004/096773, etc.).

### 4) Step 4

The compound (28-6) may be prepared from the compound (28-5) by a similar method to Step 2 of Method 3.

### Method 29

Among the compounds of the formula (1-2), the compound of the formula (29-3) or a salt thereof may be prepared, for example, by the following method.

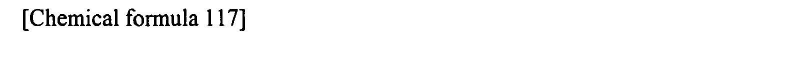

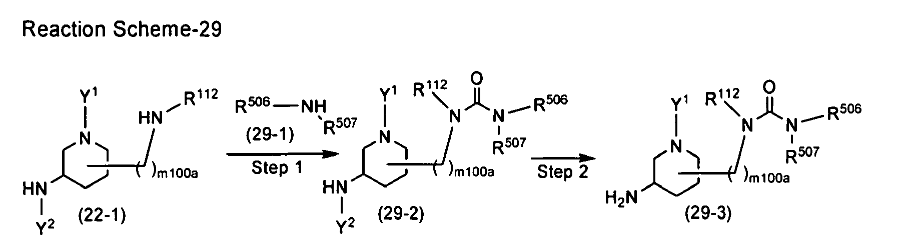

[wherein R¹¹², m₁₀₀ₐ and Y² are as defined in the above; R⁵⁰⁶ is the same as B of the above-mentioned Item 1; R⁵⁰⁷ is the same as R⁴ of the above-mentioned Item 1]

### 1) Step 1

The compound (29-2) may be prepared from the compound (22-1) by a similar method to ones disclosed in the literatures (e.g., Bioorganic & Medicinal Chemistry Letters 1621, 16 (2006), WO 99/054321, etc.).

### 2) Step 2

The compound (29-3) may be prepared from the compound (29-2) by a similar method to ones disclosed in the literatures (e.g., Protective Groups in Organic Synthesis 2nd Edition (John Wiley & Sons, Inc.), etc.).

### Method 30

Among the compounds of the formula (I), the compound of the formula (30-17) or a salt thereof may be prepared, for example, by the following method. 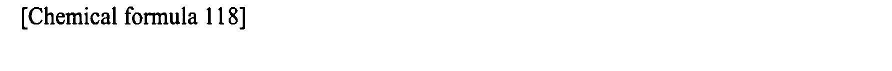 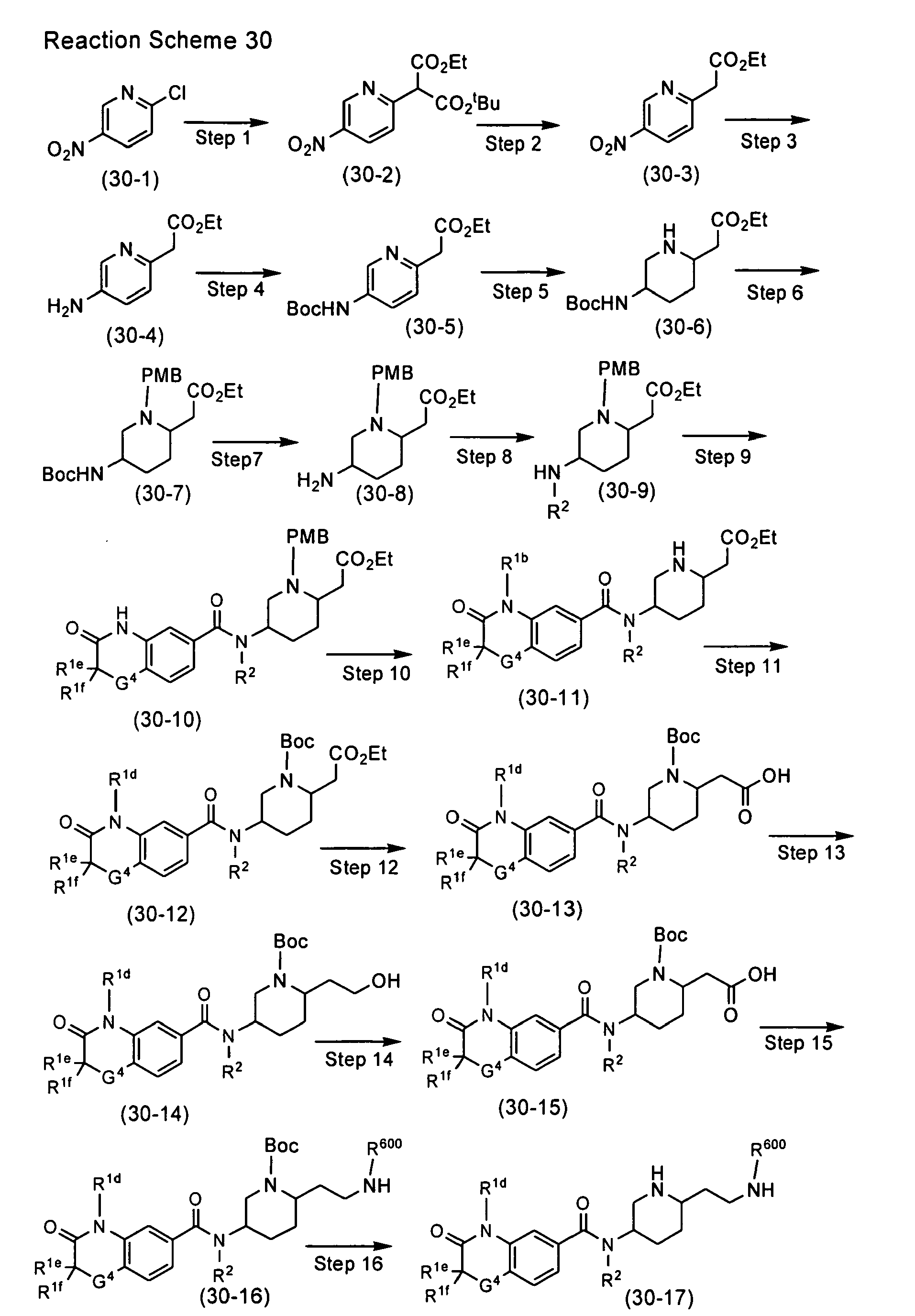 [wherein R^{1d}, R^{1e}, R^{1f} and R² are as defined in the above; R⁶⁰⁰ is the same as B of the above-mentioned Item 1; G⁴ is CH₂, an oxygen atom, or a sulfur atom]

### 1) Step 1

The compound (30-2) may be prepared by reacting the compound (30-1) with a base in an inert solvent, followed by reacting the resultant with tert-butyl ethyl malonate. The base includes inorganic bases such as potassium hydride, sodium hydride, etc. The inert solvent includes, for example, aprotic solvents such as N,N'-dimethylformamide, dimethylsulfoxide, hexamethylenephosphoamide, etc. The reaction temperature should be selected from the range from about 10°C to about 40°C.

### 2) Step 2

The compound (30-3) may be prepared by reacting the compound (30-2) with trifluoroacetic acid in an inert solvent. The inert solvent includes, for example, halogenated hydrocarbon solvents such as dichloromethane, chloroform, 1,2-dichloroethane, etc. The reaction temperature should be selected from the range from about 10°C to about 40°C.

### 3) Step 3

The compound (30-4) may be prepared by subjecting the compound (30-3) to reduction under hydrogen atmosphere in the presence of palladium carbon in an inert solvent. The inert solvent includes, for example, alcohols such as methanol, ethanol, etc. The reaction temperature should be selected from the range from about 10°C to about 40°C.

### 4) Step 4

The compound (30-5) may be prepared by reacting the compound (30-4) with di-tert-butyl dicarbonate in an inert solvent. The inert solvent includes, for example, ethers such as dioxane, tetrahydrofuran, etc. The reaction temperature should be selected from the range from about 10°C to about 40°C.

### 5) Step 5

The compound (30-6) may be prepared by subjecting the compound (30-5) to reduction under hydrogen atmosphere in the presence of platinum oxide in an inert solvent. The inert solvent includes alcohols such as methanol, ethanol, etc. The reaction temperature should be selected from the range from about 10°C to about 40°C.

### 6) Step 6

The compound (30-7) may be prepared by reacting the compound (30-6) with p-methoxybenzyl chloride in an inert solvent in the presence of an inorganic base. The inorganic base includes potassium carbonate, cesium carbonate, etc. The inert solvent includes, for example, aprotic solvents such as N,N'-dimethylformamide, dimethylsulfoxide, hexamethylenephosphoamide, etc. The reaction temperature should be selected from the range from about 10°C to about 40°C. In addition, in this Step, sodium iodide or potassium iodide may be added.

### 7) Step 7

The compound (30-8) may be prepared from the compound (30-7) by a similar method to Step 2 of Method 1.

### 8) Step 8

The compound (30-9) may be prepared from the compound (30-8) by a similar method to Step 4 of Method 1.

### 9) Step 9

The compound (30-10) may be prepared from the compound (30-9) by a similar method to Step 1 of Method 1.

### 10) Step 10

The compound (30-11) may be prepared by subjecting the compound (30-10) under hydrogen atmosphere in the presence of palladium carbon in an inert solvent to reduction. The inert solvent includes, for example, alcohols such as methanol, ethanol, etc. The reaction temperature should be selected from the range from about 10°C to about 40°C.

### 11) Step 11

The compound (30-12) may be prepared from the compound (30-11) by a similar method to Step 4 of Method 30.

### 12) Step 12

The compound (30-13) may be prepared from the compound (30-12) by a similar method to Step 2 of Method 3.

### 13) Step 13

The compound (30-14) may be prepared from the compound (30-13) by carrying out the following reactions (i. to ii.) continuously in an inert solvent. The inert solvent may be, for example, ethers such as dioxane, tetrahydrofuran, etc.
i. the compound (30-13) is reacted with ethyl chloroformate in the presence of triethylamine. The reaction temperature should be selected from the range from about -10°C to about 20°C.
ii. sodium borohydride is added to the reaction mixture of the above i., and the mixture is reacted. The reaction temperature should be selected from the range from about 0°C to about 20°C.

### 14) Step 14

The compound (30-15) may be prepared by subjecting the compound (30-14) to Swem oxidation. For instance, dimethylsulfoxide was added to oxalyl chloride in a halogenated hydrocarbon solvents such as dichloromethane, chloroform, 1,2-dichloroethane, etc., and then, the compound (30-14) is added thereto, and further, diisopropylethylamine is added. The reaction temperature should be selected from the range from about -80°C to about -30°C.

### 15) Step 15

The compound (30-16) may be prepared from the compound (30-15) by a similar method to Step 4 of Method 1.

### 16) Step 16

The compound (30-17) may be prepared from the compound (30-16) by a similar method to Step 2 of Method 1.

### Method 31

Among the compounds of the formula (I), the compound of the formula (31-2) or a salt thereof may be prepared, for example, by the following method.

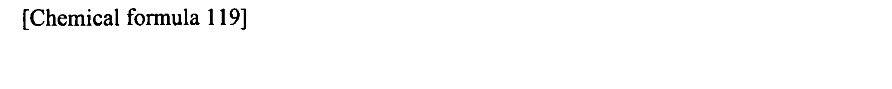

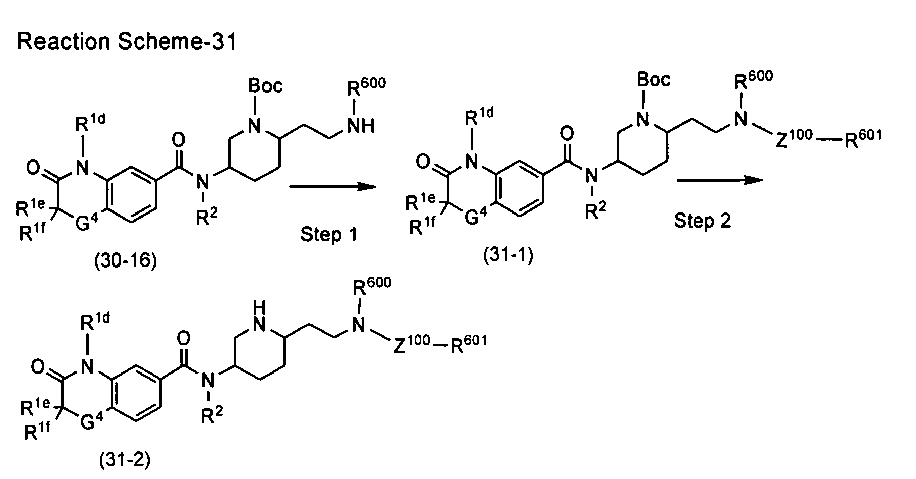

[wherein R⁶⁰⁰, R^{1d}, R^{1e}, R^{1f} and R² are as defined in the above; Z¹⁰⁰ is C(O), S(O)₂, C(O)O, or C(O)N(R⁶⁰²); R⁶⁰¹ is the same as B of the above-mentioned Item 1; R⁶⁰² is a C₁₋₆ alkyl group; G⁴ is CH₂, an oxygen atom, or a sulfur atom, provided that R⁶⁰⁰ is the same as R⁴ of Item 1]

### 1) Step 1

The compound (31-1) may be prepared from the compound (30-16) by a similar method to ones disclosed in the literatures (e.g., Comprehensive Organic transformation, R. C. Laroque,VCH publisher Inc., 1989 , etc.).

### 2) Step 2

The compound (31-2) may be prepared from the compound (31-1) by a similar method of Step 2 of Method 1.

### Method 32

Among the compounds of the formula (I), the formula (32-9) or a salt thereof may be prepared, for example, by the following method. 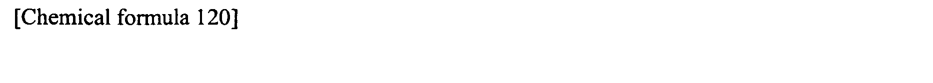 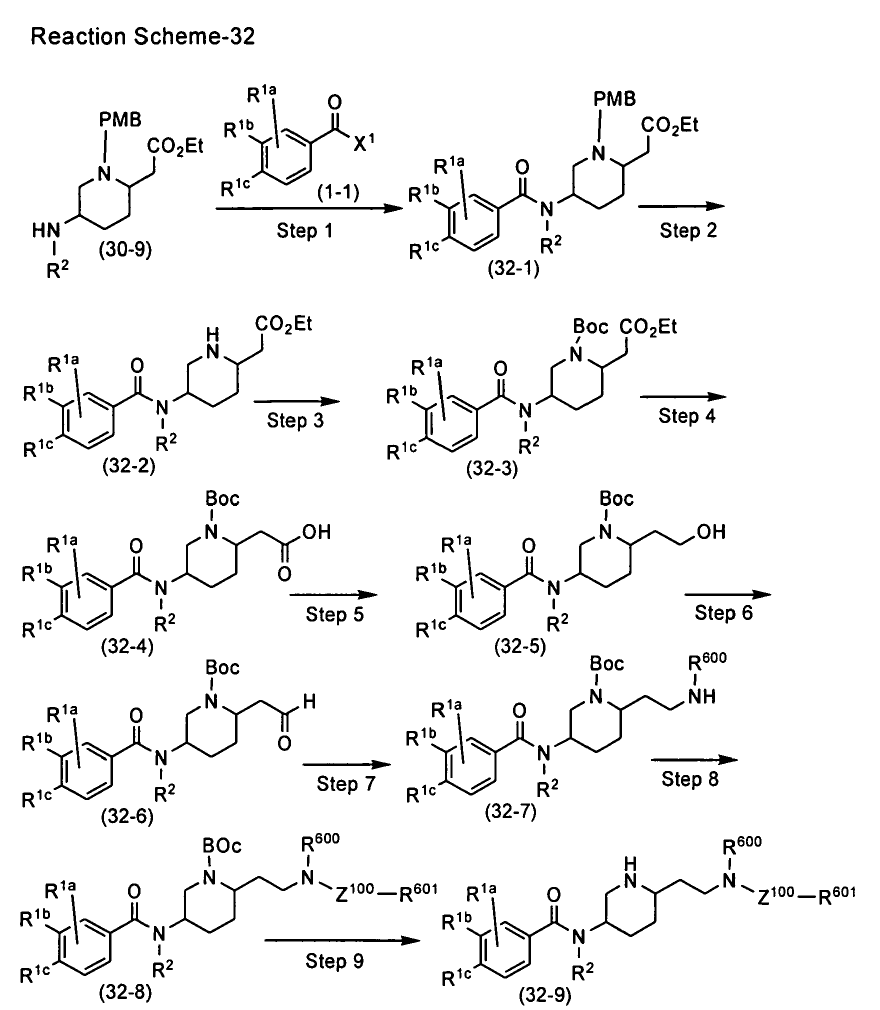 [wherein, X¹, Z¹⁰⁰, R⁶⁰⁰, R⁶⁰¹, R^{1a}, R^{1b}, R^{1c} and R² are as defined in the above]

### 1) Step 1

The compound (32-1) may be prepared from the compound (30-9) by a similar method to Step 1 of Method 1.

### 2) Step 2

The compound (32-2) may be prepared from the compound (32-1) by a similar method to Step 10 of Method 30.

### 3) Step 3

The compound (32-3) may be prepared from the compound (32-2) by a similar method to Step 4 of Method 30.

### 4) Step 4

The compound (32-4) may be prepared from the compound (32-3) by a similar method to Step 2 of Method 3.

### 5) Step 5

The compound (32-5) may be prepared from the compound (32-4) by a similar method to Step 13 of Method 30.

### 6) Step 6

The compound (32-6) may be prepared from the compound (32-5) by a similar method to Step 14 of Method 30.

### 7) Step 7

The compound (32-7) may be prepared from the compound (32-6) by a similar method to Step 4 of Method 1.

### 8) Step 8

The compound (32-8) may be prepared from the compound (32-7) by a similar method to Step 1 of Method 31.

### 9) Step 9

The compound (32-9) may be prepared from the compound (32-8) by a similar method to Step 2 of Method 1.

In the above-mentioned each step, when the starting compounds used in each reaction has a reactive group such as a hydroxy group, an amino group or a carboxyl group, then these groups at sites other than the sites to be reacted can be protected by a suitable protecting group prior to the reaction, if necessary, and such protecting groups can be removed after each reaction is complete or after some reactions are done, to give the desired compound. The protecting groups protecting a hydroxy group, an amino group, a carboxyl group, etc. may be any conventional protecting groups usually used in the organic chemistry field, and the introduction and the removal of these protecting groups may be carried out by a conventional method (e.g., the methods disclosed in Protective Groups in Organic Synthesis, T. W. Greene, co-written by G. M. Wuts, 2nd edition, John Wiley & Sons, Inc. (1991)).

For instance, a protecting group for a hydroxy group may be a tert-butyldimethylsilyl group, a methoxymethyl group, a tetrahydropyranyl group, etc. A protecting group for an amino group may be a tert-butyloxycarbonyl group, a benzyloxycarbonyl group, etc. A protecting group for a hydroxy group may be removed by reacting in the presence of an acid such as hydrochloric acid, sulfuric acid, acetic acid, etc. in a solvent such as aqueous methanol, aqueous ethanol, aqueous tetrahydrofuran, etc. In addition, a tert-butyldimethylsilyl group may be removed, for example, in the presence of tetrabutylammonium fluoride in a solvent such as tetrahydrofuran. As for the protecting group for an amino group, tert-butyloxycarbonyl group may be removed, for example, by reacting in the presence of an acid such as hydrochloric acid, trifluoroacetic acid, etc. in a solvent such as aqueous tetrahydrofuran, methylene chloride, chloroform, aqueous methanol, etc. A benzyloxycarbonyl group may be removed, for example, by reacting in the presence of an acid such as hydrobromic acid in a solvent such as acetic acid.

tert-Butyl ester, ortho ester, acid amide, etc. may be exemplified as a protecting group for a carboxyl group. These protecting groups may be removed, for example, when such a protecting group is a tert-butyl ester, then it can be removed by reacting in the presence of hydrochloric acid in an aqueous solvent. When an ortho ester is used, it can be removed, for example, by treating with an acid in a solvent such as aqueous methanol, aqueous tetrahydrofuran, aqueous 1,2-dimethoxyethane, followed by treatment with an alkali such as sodium hydroxide, etc. When an acid amide is used, then it may be removed, for example, by reacting in the presence of an acid such as hydrochloric acid, sulfuric acid, etc. in a solvent such as water, aqueous methanol, aqueous tetrahydrofuran, etc.

The compound of the formula (I) includes compounds having an optically-active center, and such compounds may be obtained in a mixture of racemic compounds, or in the form of an optically active compound when an optically active starting compound is used. If necessary, the obtained racemic mixture may be resolved physically or chemically by a conventional method into optical enantiomers thereof, or preferably resolved into diastereomers thereof by a reaction using an optically active resolving agent. Diastereomers in a different form may be resolved by a conventional method such as fractional crystallization.

The compound of the present invention may be converted into a salt thereof, for example, by mixing with a pharmaceutically acceptable acid in a solvent such as water, methanol, ethanol, acetone, etc. The pharmaceutically acceptable acid includes, for example, inorganic acids such as hydrochloric acid, hydrobromic acid, sulfuric acid, phosphoric acid, nitric acid, etc. or organic acids such as acetic acid, propionic acid, oxalic acid, succinic acid, lactic acid, malic acid, tartaric acid, citric acid, maleic acid, fumaric acid, methanesulfonic acid, p-toluenesulfonic acid, ascorbic acid, etc.

The compound of the present invention may possibly be applied to the treatment of various diseases because of its renin inhibitory activity. The compounds disclosed in the present specification are useful as a therapeutic agent for hypertension. These compounds are also useful in the control of acute and chronic congestive heart failure. These compounds can be expected to be useful in the treatment of primary or secondary pulmonary hypertension, secondary hyperaldosteronism, primary and secondary pulmonary hyperaldosteronism, kidney failure (such as glomerulonephritis, diabetic nephropathy, glomerulosclerosis, primary renal disease, end-stage renal disease, renovascular hypertension), left ventricular failure, diabetic retinopathy, and for the minimization of vascular diseases such as migraine, Raynaud's disease and atherosclerosis process. In addition, these compounds are also useful in the treatment of diseases relating to elevated intraocular pressure such as glaucoma.

When the present compound is used in the treatment, it may be administered orally or parenterally (e.g., intravenously, subcutaneously or intramuscularly, locally, rectally, precutaneously, or transnasally) in the form of a pharmaceutical composition. The composition for oral administration may be, for example, tablets, capsules, pills, granules, powders, solutions, suspensions, etc. The composition for parenteral administration may be, for example, aqueous solutions for injection, or oils, ointments, creams, lotions, aerosols, suppositories, adhesive preparations, etc. These preparations may be prepared by a conventional method, and may additionally contain a nontoxic and nonactive carrier or excipient, that is usually used in the pharmaceutical field.

The dosage may vary depending on each compound, or diseases, ages, body weights, sexes, conditions of each patient, or administration route, etc., and the present compound or a pharmaceutically acceptable salt thereof may usually be administered at a dose of 0.1 to 1000 mg/day, preferably at a dose of 1 to 300 mg/day in an adult (body weight: 50 kg), which is administered once a day or divided into 2 or 3 dosage forms. In addition, the present compound can be administered once in several days to once in several weeks.

Aiming at the enhancement of the pharmacological activity, the present compound may be used in a combination with a medicament such as an antidiabetic agent, a remedy for antidiabetic complications, an antilipedemic agent, a hypotensive agent, an antiobesity agent, a diuretic agent (hereinafter referred to as combined medicine). The administration timing of the present compound and a combined medicine is not necessarily defined, and they can be administered to a subject simultaneously or administered with time-interval.
In addition, the present compound and a combined medicine may be used in the form of a combination drug. The dosage of a combined medicine may be suitably selected based on the dosage thereof which is clinical used. In addition, the mixing ratio of the present compound and a combined medicine may suitably be determined depending on the subject to be administered, administration route, the disease to be treated, the conditions of a patient, and a kind of combination. For example, when the subject to be administered is human, then a combined medicine is used in an amount of 0.01 to 100 parts by weight to one part by weight of the present compound.

The antidiabetic agent includes insulin preparations (e.g., animal insulin preparations extracted from the bovine pancreas or swine pancreas; genetically-engineered human insulin preparations using Escherichia coli or yeast, etc.), insulin sensitizers (e.g., pioglitazone or hydrochloride thereof, troglitazon, rosiglitazone or a maleate thereof, GI-262570, JTT-501, MCC-555, YM-440, KRP-297, CS-011, etc.), α-glucosidase inhibitors (e.g., voglibose, acarbose, miglitol, emiglitate, etc.), biguanide preparations (e.g., metformine, etc.), insulin secretagogues (e.g., sulfonylurea preparations such as tolbutamide, glibenclamide, gliclazide, chlorpropamide, tolazamide, acetohexamide, glyclopyramide, glimepiride, etc.; repaglinide, senaglinide, nateglinide, mitiglinide, etc.), GLP-1, GLP-1 analogues (exenatide, liraglutide, SUN-E7001, AVE010, BIM-51077, CJC1131, etc.), protein tyrosine phosphatase inhibitors (e.g., vanadic acid, etc.), β3 agonists (e.g., GW-427353B, N-5984, etc.), DPPIV inhibitors (e.g., sitagliptin, vildagliptin, saxagliptin, SYR-322, etc.).

The therapeutic agents for diabetic complications include aldose reductase inhibitors (e.g., tolrestat, epalrestat, zenarestat, zopolrestat, minarestat, fidarestat, SK-860, CT-112, etc.), neurotrophic factors (e.g., NGF, NT-3, BDNF, etc.), PKC inhibitors (e.g., LY-333531, etc.), AGE inhibitors (e.g., ALT946, pimagedine, pyratoxatin, N-phenacylthiazolium bromide(ALT766), etc.), active oxygen erasers (e.g., thioctic acid, etc.), cerebral vasodilators (e.g., tiapride, mexiletine, etc.). A antilipidemic agent includes HMG-CoA reductase inhibitors (e.g., pravastatin, simvastatin, lovastatin, atorvastatin, fluvastatin, itavastatin or sodium salt thereof, etc.), squalene synthetase inhibitors, ACAT inhibitors, etc. The hypotensive agent includes angiotensin-converting enzyme inhibitors (e.g., captopril, enalapril, alacepril, delapril, lisinopril, imidapril, benazepril, cilazapril, temocapril, trandolapril, etc.), angiotensin II antagonists (e.g., olmesartan medoxomil, candesartan cilexetil, losartan, eprosartan, valsartan, telmisartan, irbesartan, tasosartan, etc.), calcium antagonists (e.g., nicardipine hydrochloride, manidipine hydrochloride, nisoldipine, nitrendipine, nilvadipine, amlodipine, etc.), ACE/NEP inhibitors (e.g., omapatrilat, fasidotril, etc.), beta blockers (e.g., atenolol, bisoprolol, betaxolol, metoprolol, etc.), alpha blockers (e.g., urapidil, terazosin, doxazosin, bunazosin, etc.), alpha beta blockers (e.g., amosulalol, arotinolol, labetalol, carvedilol, etc.).

The antiobesity agent includes, for example, central anti-obesity agents (e.g., phentermine, sibutramine, amfepramone, dexamphetamine, Mazindol, SR-141716A, etc.), pancreatic lipase inhibitors (e.g., Orlistat, etc.), peptidic appetite suppressors (e.g., leptin, CNTF (ciliary neurotrophic factor), etc.), cholecystokinin antagonists (e.g., lintitript, FPL-15849, etc.). The diuretic agent includes, for example, xanthine derivatives (e.g., theobromine sodium salicylate, theobromine calcium salicylate. etc.), thiazide preparations (e.g., Ethiazide, cyclopenthiazide, trichloromethiazide, cyclopenthiazide, trichlormethiazide, hydrochlorotiazide, hydroflumethiazide, bentylhydrochlorothiazide, penflutizide, polythiazide, methychlothiazide, etc.), antialdosterone preparations (e.g., spirolactone, triamterene, etc.), carbonate dehydratase inhibitors (e.g., acetazolamide, etc.), chlorbenzenesulfonamide preparations (e.g., chlorthalidone, mefruside, indapamide, etc.), azosemide, isosorbide, ethacrynic acid, Piretanide, bumetanide, furosemide, etc.

The above mentioned combined medicines may be used in a mixture of two or more of these drugs.

When the present compound is used in a combination of a combined medicine, the dosage of these drugs can be lessened within the safe range, in view of the side effects of the drugs. Accordingly, any possible side effect caused by these drugs may safely be inhibited.

### [EXAMPLES]

The present invention is illustrated in more detail by Reference Examples, Examples and Experiments, but the present invention should not be construed to be limited thereto. In addition, the compound names used in the following Reference Examples and Examples are not necessarily based on IUPAC nomenclature. Further, in order to simplify the description, some abbreviations may be used, and these abbreviations are as defined in the above-mentioned definitions.

### Reference Example 1

### Methyl 3-hydroxy-4-methoxybenzoate

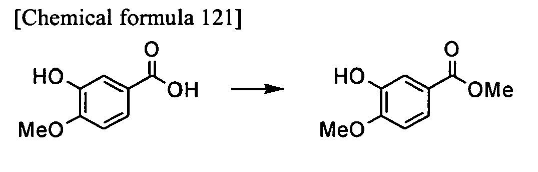

3-Hydroxy-4-methoxybenzoic acid (28.8 g) was dissolved in methanol (300 ml), and thereto was added conc. sulfuric acid (1.0 ml), and the mixture was heated under reflux for 18 hours. The reaction mixture was allowed to cool, and concentrated under reduced pressure. To the residue was added a saturated aqueous sodium hydrogen carbonate solution, and the mixture was extracted with ethyl acetate. The organic layer was dried over sodium sulfate, filtered, and concentrated under reduced pressure to give the title compound (30.9 g) as a white solid.

### Reference Example 2

### Methyl 4-methoxy-3-(3-methoxypropoxy)benzoate

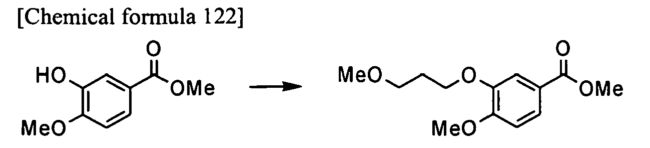

The compound of Reference Example 1 (19.7 g) was dissolved in acetonitrile (250 ml), and thereto were added potassium carbonate (22.4 g) and 1-bromo-3-methoxypropane (18.5 g), and the mixture was heated under reflux for 6 hours. The reaction mixture was allowed to cool, and concentrated under reduced pressure. To the residue was added water, and the mixture was extracted with ethyl acetate. The organic layer was dried over sodium sulfate, filtered, and concentrated under reduced pressure to give the title compound (29.9 g).

### Reference Example 3

### 4-Methoxy-3-(3-methoxypropoxy)carboxylic acid

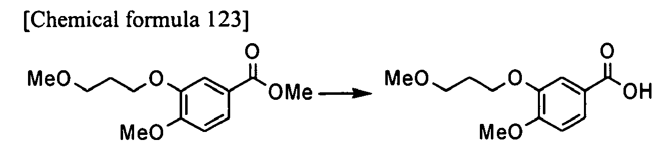

The compound of Reference Example 2 (29.9 g) was dissolved in methanol, and thereto was added 1N aqueous sodium hydroxide solution (160 ml), and the mixture was stirred at room temperature for 5 hours. The reaction mixture was concentrated under reduced pressure, and thereto was added water, and the mixture was extracted with ethyl acetate. To the aqueous layer was added 2N hydrochloric acid, and the mixture was extracted twice with ethyl acetate. The combined organic layers were washed with a saturated aqueous sodium chloride solution, dried over sodium sulfate, filtered and concentrated under reduced pressure. The obtained residue was crystallized from ethyl acetate to give the title compound (24.6 g) as a white solid.
¹H-NMR (400MHz, CDCl₃) 7.76(1H, dd, J = 2.0, 8.4 Hz, 1H), 7.63(d, J = 2.0 Hz, 1H), 6.91(d, J = 8.4 Hz, 1H), 4.18(t, J = 6.5 Hz, 2H), 3.94(s, 3H). 3.59(t, J = 6.2 Hz, 2H), 3.37(s, 3H), 2.17-2.11(m, 2H).

### Reference Example 4

### 4-Methoxy-3-(3-methoxypropoxy)benzoyl chloride

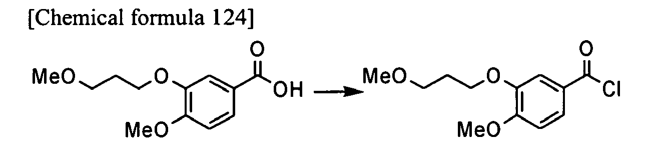

The compound of Reference Example 3 (2.45 g) was dissolved in dichloromethane (10 ml), and thereto was added oxalyl chloride (1.36 ml), and the mixture was stirred at room temperature for 6 hours. The solvent was removed by evaporation, and toluene was added thereto, and the mixture was concentrated under reduced pressure, and this procedure was repeated twice to give the title compound (2.62 g) as a white solid.

### Reference Example 5

### Benzyl (3R)-3-[(tert-butoxycarbonyl)amino]piperidine-1-carboxylate

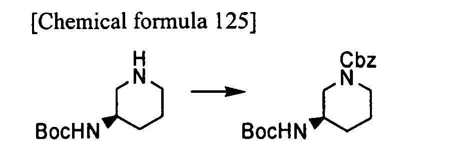

(R)-tert-3-Butyl piperidin-3-ylcarbamate (2.00 g) was dissolved in tetrahydrofuran (10 ml), and thereto were added a saturated aqueous sodium hydrogen carbonate solution (10 ml) and benzyl chloroformate (2.21 g), and the mixture was stirred at room temperature for 4 hours. The solvent was removed by evaporation, and water was added to the residue. The mixture was extracted with ethyl acetate, and the organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 5/1 to 5/2) to give the title compound (3.43 g) as a white solid.
¹H-NMR (400MHz, CDCl₃) 7.38-7.28(m, 5H), 5.15(d, J = 12.4 Hz, 1H), 5.11(d, J = 12.4 Hz, 1H), 4.59(brs, 1H), 3.69(m, 2H), 3.48(m, 1H), 3.37-3.27(m, 2H), 1.85(m, 1H), 1.73-1.67(m, 1H), 1.54(m, 2H), 1.43(s, 9H).
MS (ESI+) 335(M⁺+1, 100%)

### Reference Example 6

### Benzyl (3R)-3-aminopiperidine-1-carboxylate

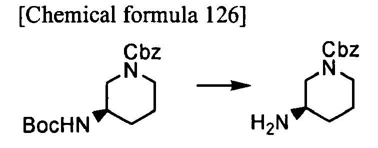

The compound of Reference Example 5 (508 mg) was dissolved in dioxane (5 ml), and thereto was added 4N hydrochloric acid/dioxane (5 ml), and the mixture was stirred at room temperature for 3 hours. The solvent was removed by evaporation, and thereto was added a saturated aqueous sodium hydrogen carbonate solution, and the mixture was extracted with chloroform. The organic layer was dried over sodium sulfate, filtered and concentrated under reduced pressure to give the title compound (373 mg) as a colorless oily compound.
MS (ESI+) 235 (M⁺+1, 100 %)

### Reference Example 7

### Benzyl (3R)-3-(isopropylamino)piperidine-1-carboxylate

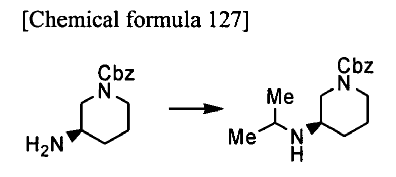

The compound of Reference Example 6 (373 mg) was dissolved in dimethylformamide (4 ml), and thereto were added potassium carbonate (315 mg) and 2-iodopropane (167 µl), and the mixture was stirred at 90°C for 4 hours. The reaction mixture was allowed to cool, and thereto was added a saturated aqueous sodium hydrogen carbonate solution, and the mixture was extracted with chloroform. The organic layer was washed with a saturated aqueous sodium hydrogen carbonate solution and a saturated aqueous sodium chloride solution, dried over sodium sulfate, filtered and concentrated under reduced pressure. The obtained residue was purified by silica gel column chromatography (developing solvent: chloroform/methanol = 20/1) to give the title compound (144 mg) as a colorless oily compound. ¹H-NMR (400MHz, CDCl₃) 7.38-7.29(m, 5H), 5.13(s, 2H), 4.13-4.01(m, 1H), 3.91-3.88(m, 1H), 2.95-2.92(m, 2H), 2.68(m, 2H), 1.95-1.92(m, 1H), 1.71-1.51(m, 3H), 1.32-1.26(m, 1H), 1.06(brs, 6H).
MS (ESI+) 277 (M+1, 100 %).

### Reference Example 8

### Benzyl (3R)-3-{isopropyl[4-methoxy-3-(3-methoxypropoxy)benzoyl]amino}piperidine-1-carboxylate

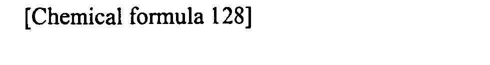

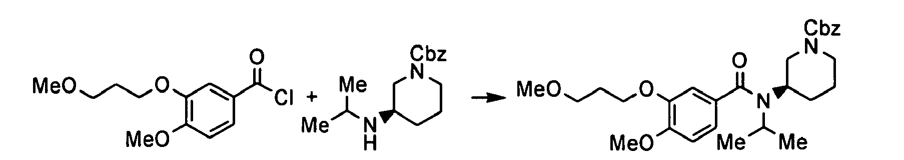

The compound of Reference Example 4 (50.9 mg) and the compound of Reference Example 7 (49.5 mg) were dissolved in dichloromethane (2 ml), and thereto was added triethylamine (32.4 µl), and the mixture was stirred at room temperature. To the reaction mixture was added a saturated aqueous ammonium chloride solution, and the mixture was extracted with ethyl acetate twice. The combined organic layers were washed with a saturated aqueous sodium chloride solution, dried over sodium sulfate, filtered and concentrated under reduced pressure. The obtained residue was purified by silica gel column chromatography (developing solvent: hexane/ethyl acetate = 1/1) to give the title compound (76.9 mg) as a colorless oily compound.
¹H-NMR (400MHz, CDCl₃) 7.37-7.31(m, 5H), 6.91-6.85(m, 3H), 5.18(d, J = 12.4 Hz, 1H), 5.11(d, J = 12.4 Hz, 1H), 4.20-4.12(m, 4H), 3.96-3.87(m, 1H), 3.87(s, 3H), 3.56(t, J = 6.5 Hz, 2H), 3.35(s, 3H), 3.10(brs, 1H), 2.78(brs, 2H), 2.13-2.05(m, 2H), 1.77-1.74(m, 3H), 1.53(m, 1H), 1.26-1.18(m, 6H).
MS (ESI+) 499(M+1, 100%).

### Reference Example 9

### Benzyl (3S)-3-[(tert-butoxycarbonyl)amino]piperidine-1-carboxylate

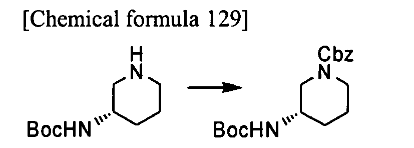

The title compound (936 mg) was synthesized in a similar manner to Reference Example 5. MS (ESI+) 335 (M+1, 100%).

### Reference Example 10

### Benzyl (3S)-3-aminopiperidine-1-carboxylate hydrochloride

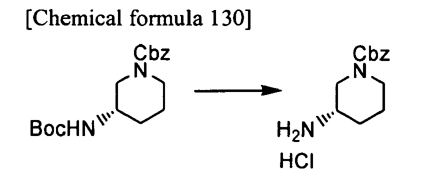

The compound of Reference Example 9 (936 mg) was dissolved in dioxane (3 ml), and thereto was added 4N hydrochloric acid/dioxane (3 ml), and the mixture was stirred at room temperature for 14 hours. The solvent was removed by evaporation, and to the residue was added toluene, and the mixture was concentrated under reduced pressure to give the title compound (750 mg) as a white solid.
MS (ESI+) 235 (M+1, 100%).

### Reference Example 11

### Benzyl (3S)-3-(isopropylamino)piperidine-1-carboxylate

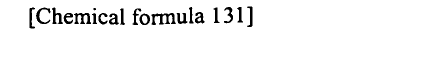

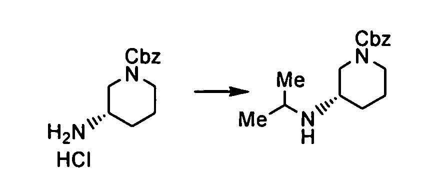

The title compound (15.1 mg) was synthesized in a similar manner to Reference Example 7 except that the compound of Reference Example 10 was used instead of the compound of Reference Example 6 in Reference Example 7.
MS (ESI+) 277 (M+1, 100%).

### Reference Example 12

### Benzyl (3S)-3-{isopropyl[4-methoxy-3-(3-methoxypropoxy)benzoyl]amino}piperidine-1-carboxylate

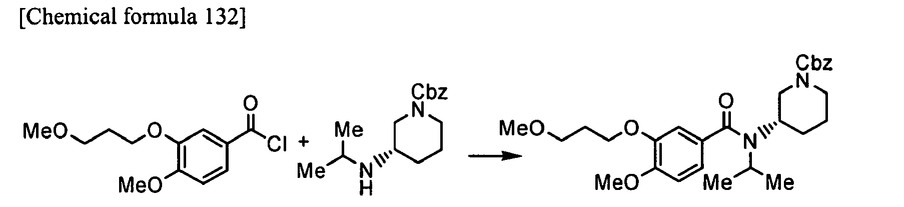

The title compound (10.8 mg) was synthesized in a similar manner to Reference Example 8 except that the compound of Reference Example 11 was used instead of the compound of Reference Example 7 in Reference Example 8.
MS (ESI+) 499 (M+1, 100%).

### Reference Example 13

### Benzyl 3-[(tert-butoxycarbonyl)amino]pyrrolidine-1-carboxylate

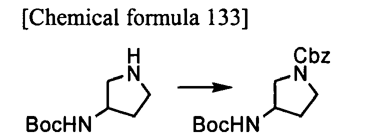

The title compound (1.92 g) was synthesized in a similar manner to Reference Example 5. MS (ESI+) 321 (M+1, 100%).

### Reference Example 14

### Benzyl 3-aminopyrrolidine-1-carboxylate

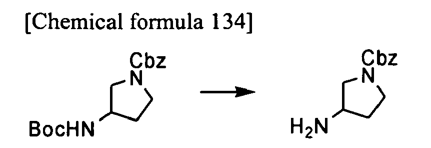

The title compound (982 mg) was synthesized in a similar manner to Reference Example 10 except that the compound of Reference Example 13 was used instead of the compound of Reference Example 9 in Reference Example 10.
MS (ESI+) 221 (M+1, 100 %).

### Reference Example 15

### Benzyl 3-(isopropylamino)pyrrolidine-1-carboxylate

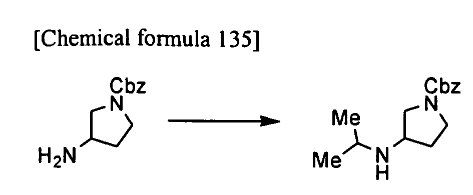

The compound of Reference Example 14 (121.5 mg) was dissolved in dichloromethane (2.8 ml), and thereto were added acetone (122 µl) and acetic acid (31.6 µl), and the mixture was stirred at room temperature. Two and half hours thereafter, to the mixture was added sodium triacetoxy brohydride (292 mg), and the mixture was stirred at room temperature for 3 hours. To the reaction mixture was added a saturated aqueous sodium hydrogen carbonate solution, and the mixture was extracted with chloroform. The organic layer was washed with a saturated aqueous sodium chloride solution, dried over sodium hydrogen sulfate, filtered and concentrated under reduced pressure. The obtained residue was purified by silica gel column chromatography (developing solvent: chloroform/methanol = 30/1 to 20/1) to give the title compound (87.0 mg) as a colorless oily compound. ¹H-NMR (300MHz, CDCl₃) 7.37-7.28(m, 5H), 5.13(s, 2H), 3.72-3.63(m, 1H), 3.58-3.50(m, 1H), 3.43-3.36(m, 2H), 3.13-3.04(m, 1H), 2.88-2.84(m, 1H), 2.14-2.04(m, 1H), 1.72-1.63(m, 1H), 1.18(m, 1H), 1.08-1.04(m, 6H).
MS (ESI+) 263 (M+1, 100%).

### Reference Example 16

### Benzyl 3-{isopropyl[4-methoxy-3-(3-methoxypropoxy)benzoyl]amino}pyrrolidine-1-carboxylate

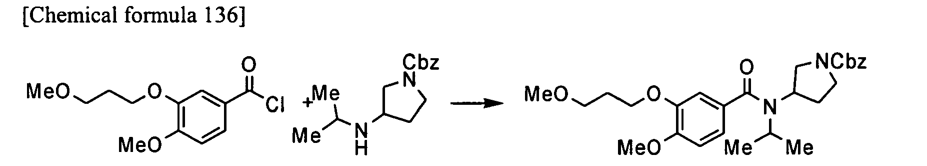

The title compound (125 mg) was synthesized in a similar manner to Reference Example 8 except that the compound of Reference Example 15 was used instead of the compound of Reference Example 7 in Reference Example 8.
¹H-NMR (300MHz, CDCl₃) 7.38-7.31(m, 5H), 6.92-6.83(m, 3H), 5.14(s, 2H), 4.12(t, J = 6.3 Hz, 2H), 3.96-3.88(m, 3H), 3.81-3.64(m, 2H), 3.56(t, J = 6.0 Hz, 2H), 3.47-3.38(m, 1H), 3.35(s, 3H), 2.15-2.06(m, 2H), 2.00(m, 1H), 1.70(m, 1H), 1.24(brs, 6H).
MS (ESI+) 485 (M+1, 100%).

### Reference Example 17

### Benzyl (2-oxoazepan-3-yl)carbamate

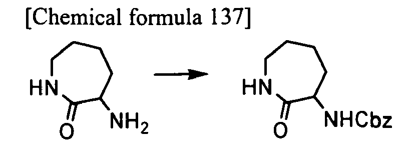

The title compound (3.76 g) was synthesized in a similar manner to Reference Example 5. ¹H-NMR (400MHz, CDCl₃) 7.36-7.30(m, 5H), 6.17(m, 2H), 5.12(d, J = 12.4 Hz, 1H), 5.09(d, J = 12.4 Hz, 1H), 4.36-4.32(m, 1H), 3.28-3.23(m, 2H), 2.13-2.10(m, 1H), 2.04-1.99(m, 1H), 1.86-1.74(m, 2H), 1.59-1.52(m, 1H), 1.44-1.37(m, 1H).
MS (ESI+) 263 (M+1, 100%).

### Reference Example 18

### Benzyl azepan-3-ylcarbamate

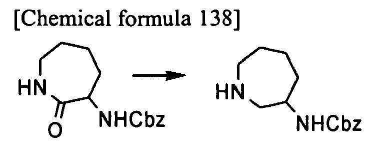

The compound of Reference Example 17 (3.76 g) was dissolved in tetrahydrofuran (28 ml), and thereto was added dropwise a 1.0M solution of boron-tetrahydrofuran complex in tetrahydrofuran (35.8 mL) at 0°C, and the mixture was stirred at room temperature for 4 hours. To the reaction mixture was added 3N hydrochloric acid, and the mixture was heated under reflux for 2 hours. The solvent was removed by evaporation, and thereto was added ethyl acetate, and the mixture was separated. To the organic layer was added 3N hydrochloric acid and the mixture was separated. To the combined aqueous layers were added 2N aqueous sodium hydroxide solution so that the pH value thereof was adjusted to around pH 11. This solution was extracted with chloroform three times, dried over sodium sulfate, filtered and concentrated under reduced pressure to give the title compound (2.44 g) as a white solid.
¹H-NMR (400MHz, CDCl₃) 7.37-7.30(m, 5H), 5.42(m, 1H), 5.09(s, 2H), 3.81(brs, 1H), 2.94-2.87(m, 2H), 2.84-2.81(m, 2H), 1.78-1.53(m, 7H).
MS (ESI+) 249 (M+1, 100%).

### Reference Example 19

### tert-Butyl 3-{[(benzyloxy)carbonyl]amino}azepane-1-carboxylate

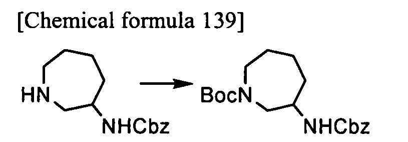

The compound of Reference Example 18 (2.44 g) was dissolved in tetrahydrofuran (20 ml) and a saturated aqueous sodium hydrogen carbonate solution (20 ml), and thereto was added di-tert-butyl dicarbonate (2.36 g). The mixture was stirred at room temperature for 15 hours. The solvent was removed by evaporation, and water was added to the residue, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 5 to 5/2) to give the title compound (2.54 g) as a colorless oily compound.
MS (ESI+) 349 (M+1, 100%).

### Reference Example 20

### tert-Butyl 3-aminoazepane-1-carboxylate

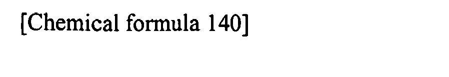

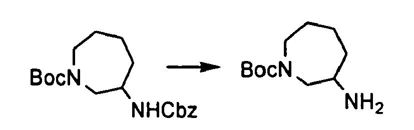

The compound of Reference Example 19 (2.54 g) was dissolved in methanol (20 ml), and thereto was added 10 % palladium carbon (508 mg), and the mixture was stirred at room temperature for 4 hours under hydrogen atmosphere. The mixture was filtered through celite, and the filtrate was concentrated under reduced pressure to give the title compound (1.55 g) as a colorless oil.
MS (ESI+) 215 (M+1, 100%).

### Reference Example 21

### tert-Butyl 3-(isopropylamino)azepane-1-carboxylate

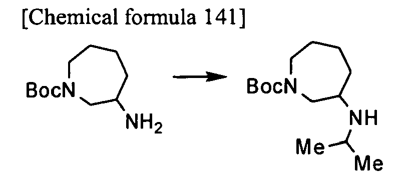

The title compound (384 mg) was synthesized in a similar manner to Reference Example 15 except that the compound of Reference Example 20 was used instead of the compound of Reference Example 14 in Reference Example 15.
MS (ESI+) 215 (M+1, 100%).

### Reference Example 22

### Benzyl 3-{isopropyl[4-methoxy-3-(3-methoxypropoxy)benzoyl]amino}azepane-1-carboxylate

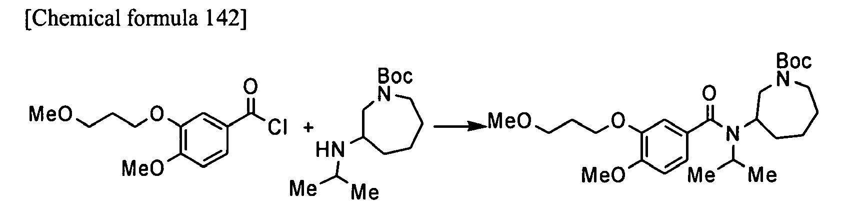

The title compound (636 mg) was synthesized in a similar manner to Reference Example 8 except that the compound of Reference Example 21 was used instead of the compound of Reference Example 7 in Reference Example 8.
¹H-NMR (400MHz, CDCl₃) 6.95-6.86(m, 3H), 4.14(t, J = 6.7 Hz, 2H), 3.93-3.92(m, 1H), 3.90(s, 3H), 3.87-3.76(m, 2H), 3.59-3.56(t, J = 6.2 Hz, 2H), 3.47-3.45(m, 1H), 3.36-3.35(m, 1H), 3.35(s, 3H), 3.02(brs, 1H), 2.31-2.25(m, 1H), 2.16 -2.09(m, 5H), 2.01-1.98(m, 1H), 1.85-1.82(m, 1H), 1.60-1.56(m, 1H), 1.25(d, J = 6.6 Hz, 3H), 1.16(d, J = 6.6 Hz, 3H).
MS (ESI+) 479 (M+1, 100%).

### Reference Example 23

### Ethyl 1-benzyl-3-(isopropylamino)piperidine-4-carboxylate

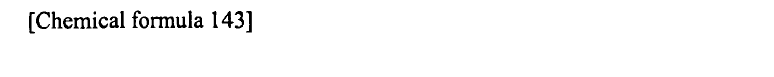

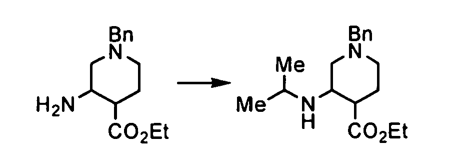

The title compound (3.39 g) was synthesized as a crude produt in a similar manner to Reference Example 15.
MS (ESI+) 305 (M+1, 100%).

### Reference Example 24

### Ethyl 1-benzyl-3-{isopropyl[4-methoxy-3-(3-methoxypropoxy)benzoyl]amino}piperidine-4-carboxylate

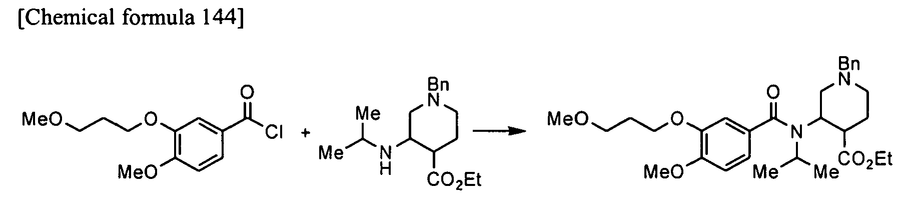

The title compound (1.10 g) was synthesized as a crude product in a similar manner to Reference Example 8 except that the compound of Reference Example 23 was used instead of the compound of Reference Example 4 in Reference Example 8.
MS (ESI+) 527 (M+1, 100%).

### Reference Example 25

### Ethyl 3-{isopropyl[4-methoxy-3-(3-methoxypropoxy)benzoyl]amino}piperidine-4-carboxylate

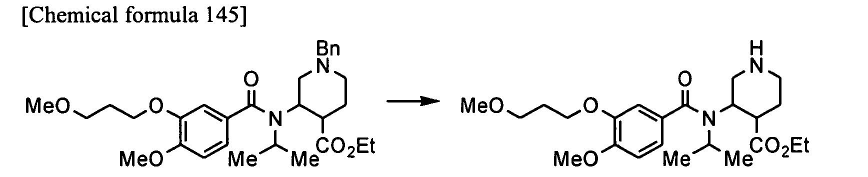

The title compound (1.0 g) was obtained as a crude product in a similar manner to Reference Example 20 except that the compound of Reference Example 24 was used instead of the compound of Reference Example 19 in Reference Example 20.
MS (ESI+) 437 (M+1, 100%).

### Reference Example 26

### 1-tert-Butyl 4-ethyl 3-{isopropyl[4-methoxy-3-(3-methoxypropoxy)benzoyl]amino}piperidine-1,4-dicarboxylate

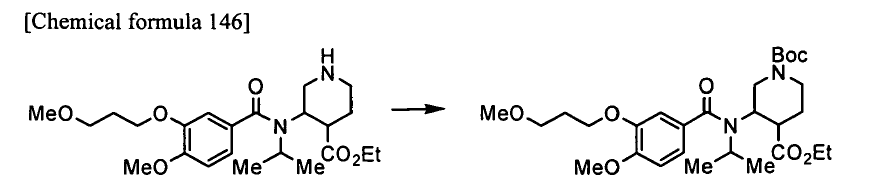

The title compound (254 mg) was synthesized in a similar manner to Reference Example 19 except that the compound of Reference Example 25 was used instead of the compound of Reference Example 18 in Reference Example 19.
¹H-NMR (400MHz, CDCl₃) 6.92-6.83(m, 3H), 4.17-3.84(m, 9H), 3.88(s, 3H), 3.56(t, J = 6.1 Hz, 2H), 3.34(s, 3H), 3.17(brs, 1H), 2.85-2.79(m, 1H), 2.14-2.07(m, 2H), 1.94-1.88(m, 1H), 1.67(brs, 1H), 1.47(s, 9H), 1.28-1.08(m, 9H).
MS (ESI+) 537 (M+1, 100%).

### Reference Example 27

### 1-(tert-Butoxycarbonyl)-3-{isopropyl[4-methoxy-3-(3-methoxypropoxy)benzoyl]amino}piperidine-4-carboxylic acid

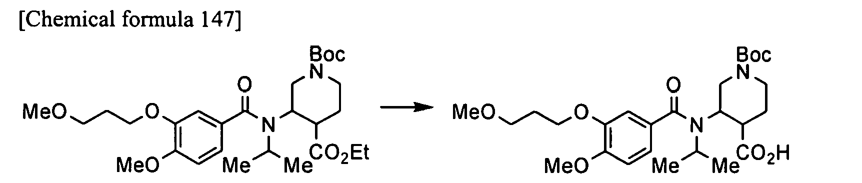

The compound of Reference Example 26 (241 mg) was dissolved in tetrahydrofuran (2 ml) and ethanol (1 ml), and thereto was added a 10 % aqueous sodium hydroxide solution (1 ml) at room temperature, and the mixture was stirred at 80°C for 5 hours. The reaction mixture was allowed to cool, and thereto was added a 5 % aqueous potassium hydrogen sulfate solution, and the mixture was extracted with ethyl acetate. The organic layer was washed with a saturated aqueous sodium chloride solution, dried over sodium sulfate, filtered and concentrated under reduced pressure to give the title compound (239 mg).
MS (ESI+) 509 (M+1, 100 %).

### Reference Example 28

### tert-Butyl 4-(hydroxymethyl)-3-{isopropyl[4-methoxy-3-(3-methoxypropoxy)benzoyl]amino}piperidine-1-carboxylate

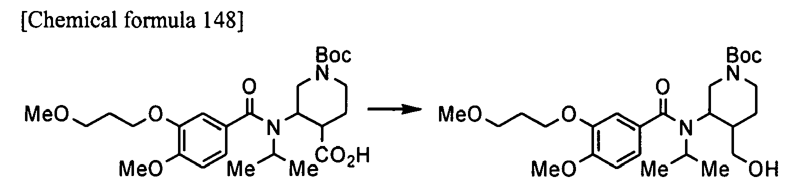

The compound of Reference Example 27 (211 mg) was dissolved in tetrahydrofuran (2 ml), and thereto were added at 0°C triethylamine (231 µl) and ethyl chloroformate (119 µl), and the mixture was stirred. One hour thereafter, water (1 ml) and sodium borohydride (82.4 mg) were added to the mixture, and the mixture was stirred at room temperature for 4 hours. To the reaction mixture was added a saturated aqueous ammonium chloride solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 1 to 1/3) to give the title compound (135 mg) as a colorless oily compound.
MS (ESI+) 495 (M+1, 100 %).

### Reference Example 29

### tert-Butyl 3-{isopropyl[4-methoxy-3-(3-methoxypropoxy)benzoyl]amino}-4-{[(3-methoxybenzyl)oxy]methyl}piperidine-1-carboxylate

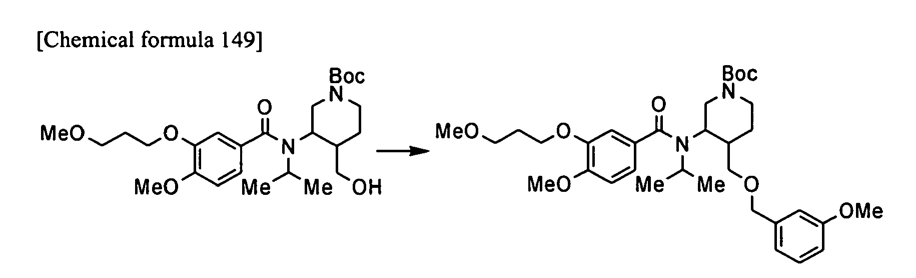

The compound of Reference Example 28 (40.3 mg) was dissolved in dimethylformamide (1.5 ml), and thereto was added sodium hydride (9.8 mg) at 0°C, and the mixture was stirred for 10 minutes. To the mixture were added tetrabutylammonium iodide (3.0 mg) and 3-methoxybenzyl chloride (35.5 µl), and the mixture was stirred at room temperature for 6 hours. To the reaction mixture was added a saturated aqueous ammonium chloride solution, and the mixture was extracted with ethyl acetate. The organic layer was washed with water and a saturated aqueous sodium chloride solution, dried over sodium sulfate, filtered and concentrated under reduced pressure. The obtained residue was purified by silica gel column chromatography (developing solvent: hexane/ethyl acetate = 1) to give the title compound (14.1 mg) as a colorless oily compound.
¹H-NMR (400MHz, CDCl₃) 7.25-7.22(m, 1H), 6.89-6.81(m, 6H), 4.49(d, J = 12.0 Hz, 1H), 4.42(d, J = 12.0 Hz, 1H), 4.13-4.04(m, 5H), 3.88(s, 3H), 3.77(s, 3H), 3.56(t, J = 6.1 Hz, 2H), 3.46-3.42(m, 2H), 3.35(s, 3H), 3.25(brs, 1H), 3.03-2.84(m, 2H), 2.13-2.05(m, 2H), 1.85(d, J = 13.3 Hz, 1H), 1.66-1.51(m, 2H), 1.46(s, 9H), 1.19-1.14(m, 6H).
MS (ESI+) 615 (M+1, 100 %).

### Reference Example 30

### Benzyl 1-[(tert-butoxycarbonyl)amino]-5-(fluoromethyl)-3-azabicyclo[3.1.1]heptan-3-carboxylate

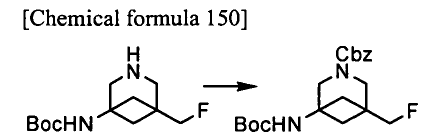

The title compound (479 mg) was synthesized in a similar manner to Reference Example 5. ¹H-NMR (400MHz, CDCl₃) 7.39-7.30(m, 5H), 5.17(s, 2H), 4.70(brs, 1H), 4.37(d, J = 2.8 Hz, 1H), 4.25(d, J = 2.8 Hz, 1H), 3.64(d, J = 5.9 Hz, 2H), 3.46(d, J = 2.0 Hz, 2H), 2.21-2.14(m, 2H), 1.87-1.81(m, 2H), 1.42(s, 9H).
MS (ESI+) 379(M+1, 100%).

### Reference Example 31

### Benzyl 1-amino-5-(fluoromethyl)-3-azabicyclo[3.1.1]heptane-3-carboxylate

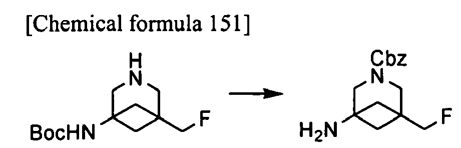

The title compound (370 mg) was synthesized in a similar manner to Reference Example 6 except that the compound of Reference Example 30 was used instead of the compound of Reference Example 6.
MS (ESI+) 279 (M+1, 100 %).

### Reference Example 32

### Benzyl 1-(fluoromethyl)-5-(isopropylamino)-3-azabicyclo[3.1.1]heptane-3-carboxylate

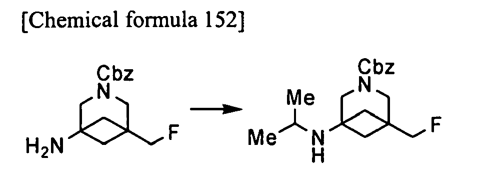

The title compound (351 mg) was synthesized in a similar manner to Reference Example 15 except that the compound of Reference Example 31 was used instead of the compound of Reference Example 14 in Reference Example 15.
¹H-NMR (400MHz, CDCl₃) 7.38-7.32(m, 5H), 5.17(s, 2H), 4.34(d, J = 6.1 Hz, 1H), 4.22(d, J = 6.1 Hz, 1H), 3.55(d, J = 10.2 Hz, 2H), 3.45(brs, 2H), 3.06-2.98(m, 1H), 1.85-1.68(m, 4H), 1.25-1.21(m, 1H), 1.05(d, J = 6.3 Hz, 3H), 1.04(d, J = 6.3 Hz, 3H).
MS (ESI+) 321 (M+1, 100 %).

### Reference Example 33

### Benzyl 1-(fluoromethyl)-5-{isopropyl[4-methoxy-3-(3-methoxypropoxy)benzoyl]amino}-3-azabicyclo[3.1.1]heptane-3-carboxylate

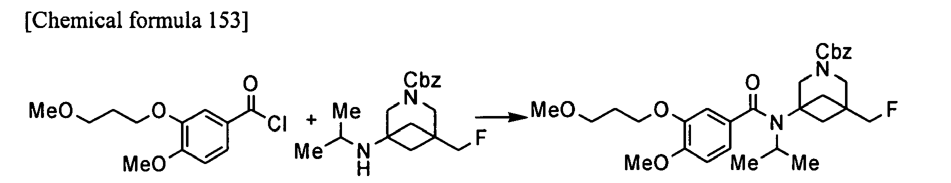

The title compound (43.0 mg) was synthesized in a similar manner to Reference Example 8 except that the compound of Reference Example 32 was used instead of the compound of Reference Example 7 in Reference Example 8.
¹H-NMR (400MHz, CDCl₃) 7.41-7.34(m, 5H), 6.91(m, 1H), 6.89-6.86(m, 1H), 6.82-6.80(m, 1H), 5.18(d, J = 15.2 Hz, 2H), 4.28(d, J = 14.6 Hz, 1H), 4.19-4.09(m, 3H), 3.93-3.86(m, 1H), 3.87(s 3H), 3.60-3.53(m, 2H), 3.38 -3.34(m, 2H), 3.36(s, 3H), 2.14-2.02(m, 4H), 1.83-1.74(m, 1H), 1.42(d, J = 6.8 Hz, 3H), 1.38(d, J = 6.8 Hz, 3H).
MS (ESI+) 543 (M+1, 100%).

### Reference Example 34

### tert-Butyl (3R)-3-{[(benzyloxy)carbonyl]amino}piperidine-1-carboxylate

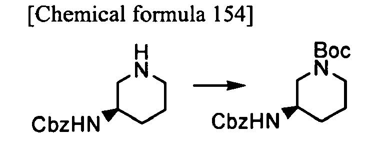

The title compound (4.69 g) was synthesized in a similar manner to Reference Example 19. MS (ESI+) 335 (M⁺+1, 100%).

### Reference Example 35

### tert-Butyl (3R)-3-aminopiperidine-1-carboxylate

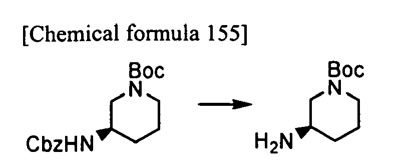

The title compound (2.62 g) was synthesized in a similar manner to Reference Example 20 except that the compound of Reference Example 34 was used instead of the compound of Reference Example 19 in Reference Example 20.
MS (ESI+) 201 (M⁺+1, 100 %).

### Reference Example 36

### tert-Butyl (3R)-3-[(4-chlorophenyl)amino]piperidine-1-carboxylate

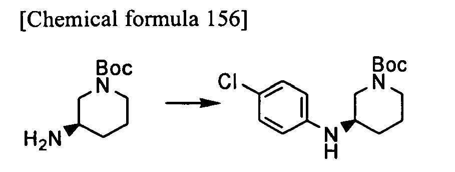

The compound of Reference Example 35 (198 mg) was dissolved in toluene (2 ml), and thereto were added 4-bromochlorobenzene (158 mg), tris(dibenzylideneacetone)dipalladium (90.4 mg), sodium tert-butoxide (133 mg) and 2-dicyclohexylphosphino-2'-(N,N-dimethylamino)biphenyl (48.5 mg), and the mixture was stirred at 100°C for 2 hours. The reaction mixture was allowed to cool, and thereto was added a saturated aqueous sodium hydrogen carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent:hexane/ethyl acetate = 6 to 5) to give the title compound (196 mg) as a brown solid.
¹H-NMR (400MHz, CDCl₃) 7.21-7.18(m, 2H), 6.87-6.84(m, 2H), 4.62(d, J = 14.4 Hz, 1H), 4.45(d, J = 14.4 Hz, 1H), 3.80(s, 3H), 3.66(s, 3H), 3.44-3.38(m, 2H), 2.83-2.73(m, 1H), 2.62-2.55(m, 1H), 2.50 - 2.41(m, 1H), 2.16-2.11(m, 1H), 2.04-1.95(m, 6H).
MS (ESI+) 311 (M⁺+1, 100%).

### Reference Example 37

### tert-Butyl (3R)-3-{(4-chlorophenyl)[4-methoxy-3-(3-methoxypropoxy)benzoyl]amino}piperidine-1-carboxylate

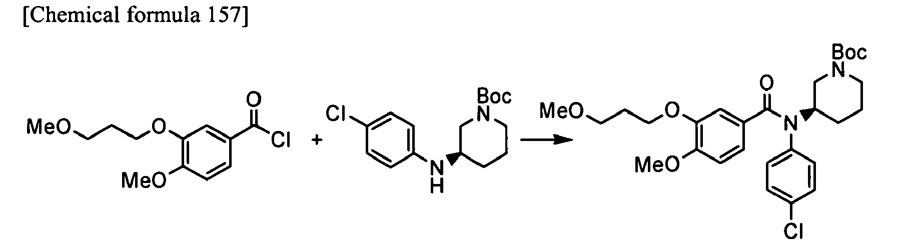

The title compound (17.0 mg) was synthesized in a similar manner to Reference Example 8 except that the compound of Reference Example 36 was used instead of the compound of Reference Example 7 in Reference Example 8.
¹H-NMR (400MHz, CDCl₃) 7.12-7.09(m, 2H), 6.57-6.53(m, 2H), 3.94(brs, 1H), 3.71-3.65(m, 2H), 3.36-3.31(m, 1H), 3.10(m, 1H), 2.90(brs, 1H), 1.99-1.96(m, 1H), 1.76-1.70(m, 1H), 1.60-1.49(m, 2H), 1.45(s, 9H).
MS (ESI+) 534(M+1, 100%).

### Reference Example 38

### Methyl 1-(4-methoxybenzyl)-6-oxo-1,4,5,6-tetrahydropyridine-3-carboxylate

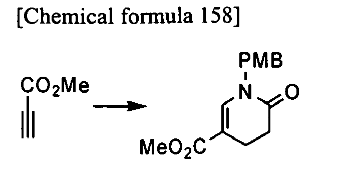

Methyl propiolate (18.6 g) was dissolved in diethyl ether (180 ml), and thereto was added dropwise a solution of 4-methoxybenzylamine (27.0 g) in diethyl ether (50 ml) at 0°C. The mixture was stirred at room temperature for 15.5 hours, and the solvent was removed by evaporation. To the obtained residue was added tetrahydrofuran (1.3L). To the mixture was added acrylic chloride (19.6 g) at room temperature, and the mixture was heated under refluxe for 12 hours. The solvent was removed by evaporation, and to the resultant was added a saturated aqueous sodium hydrogen carbonate solution, and the mixture was extracted with diethyl ether three times. The combined organic layers were dried over sodium sulfate, filtered and concentrated under reduced pressure. The obtained residue was purified by silica gel column chromatography (developing solvent: hexane/ethyl acetate = 3) to give the title compound (27.7 g) as yellow oily compound.
¹H-NMR (400MHz, CDCl₃) 7.28(s, 1H), 7.21-7.18(m, 2H), 6.88-6.85(m, 2H), 4.67(s, 2H), 3.79(s, 3H), 3.72(s, 3H), 2.62(s, 4H).
MS (ESI+) 276 (M+1, 100%).

### Reference Example 39

### Methyl 1-(4-methoxybenzyl)-6-oxopiperidine-3-carboxylate

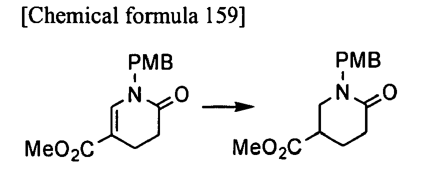

The compound of Reference Example 38 (27.7 g) was dissolved in ethanol (500 ml), and thereto were added sodium carbonate (32.0 g) and a 10 % palladium carbon (13.8 g), and the mixture was stirred at room temperature for 4 hours under hydrogen atmosphere. The reaction mixture was filtered through celite, and the filtrate was concentrated under reduced pressure. The obtained residue was purified by silica gel column chromatography (developing solvent: hexane/ethyl acetate = 2 to 1) to give the title compound (23.9 g) as a colorless oily compound.
¹H-NMR (400MHz, CDCl₃) 7.21-7.18(m, 2H), 6.87-6.84(m, 2H), 4.62(d, J = 14.4 Hz, 1H), 4.45(d, J = 14.4 Hz, 1H), 3.80(s, 3H), 3.66(s, 3H), 3.44-3.38(m, 2H), 2.83-2.73(m, 1H), 2.62-2.55(m, 1H), 2.50 - 2.41 (m, 1H), 2.16-2.11(m, 1H), 2.04-1.95(m, 6H).
MS (ESI+) 278 (M+1, 100%).

### Reference Example 40

### 1-(4-Methoxybenzyl)-6-oxopiperidine-3-carboxylic acid

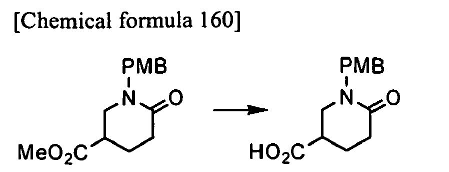

The compound of Reference Example 39 (23.9 g) was dissolved in tetrahydrofuran (100 ml) and water (400 ml), and thereto was added sodium hydroxide (6.90 g), and the mixture was stirred at room temperature for 8 hours. To the reaction mixture was added conc. hydrochloric acid, so that the pH value thereof was adjusted to pH 3 or below. The mixture was extracted with cycloform three times. The combined organic layers were dried over sodium sulfate, filtered and concentrated under reduced pressure to give the title compound (21.2 g) as a white solid.
MS (ESI+) 264 (M+1, 100%).

### Reference Example 41

### tert-Butyl [1-(4-methoxybenzyl)-6-oxopiperidin-3-yl]carbamate

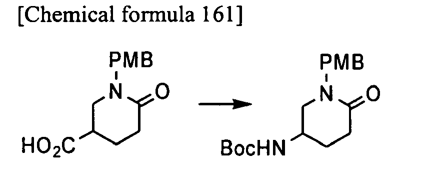

The compound of Reference Example 40 (23.9 g) was dissolved in toluene (100 ml), and thereto was added triethylamine (19.6 g), and the mixture was stirred at 80°C. To the mixture was added dropwise a solution of phenyl phosporylazide (26.6 g) in toluene (100 ml), and the mixture was stirred for 3 hours. To the mixture were added tert-butanol (180 ml) and pottasium tert-butoxide (13.6 g) at 50°C, and the mixture was stirred for 3 hours. The reaction mixture was allowed to cool, and water was added thereto. The mixture was extracted with ethyl acetate, and the organic layer was washed with water and a saturated aqueous sodium chloride solution, dried over sodium sulfate, filtered and concentrated under reduced pressure. The obtained residue was purified by silica gel column chromatography (developing solvent: hexane/ethyl acetate = 1 to 1/3) to give the title compound (15.4 g) as a white solid.
¹H-NMR (400MHz, CDCl₃) 6.97-6.94(m, 2H), 6.87-6.85(m, 1H), 4.15-4.10(m, 3H), 3.89(s, 3H), 3.59-3.47(m, 4H), 3.35(s, 3H), 3.35-3.27(m, 2H), 3.08(m, 1H), 2.74(m, 1H), 2.15-2.00(m, 3H), 1.69(m, 2H), 1.46(s, 9H), 1.26-1.17(m, 6H).
MS (ESI+) 335 (M+1, 100%).

### Reference Example 42

### tert-Butyl [1-(4-methoxybenzyl)-6-thioxopiperidin-3-yl]carbamate

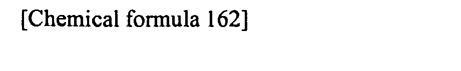

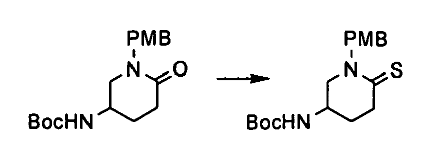

The compound of Reference Example 41 (7.35 g) was dissolved in tetrahydrofuran (55 ml), and thereto was added a Lawesson's reagent (4.45 g) at room temperature, and the mixture was stirred for 4 hours. To the reaction mixture was added a saturated aqueous sodium hydrogen carbonate solution, and the mixture was extracted with ethyl acetate twice. The combined organic layers were dried over sodium sulfate, filtered and concentrated under reduced pressure. The obtained residue was purified by silica gel column chromatography (developing solvent: hexane/ethyl acetate = 4 to 1) to give the title compound (7.25 g) as a white solid.
¹H-NMR (400MHz, CDCl₃) 7.30-7.28(m, 2H), 6.88-6.85(m, 2H), 5.45(d, J = 13.6 Hz, 1H), 4.99(d, J = 13.6 Hz, 1H), 4.46(brs, 1H), 3.99-3.97(m, 1H), 3.80(m, 3H), 3.55(dd, J = 4.8, 13.5 Hz, 1H), 3.25-3.18(m, 1H), 3.09-3.01(m, 1H), 2.06-1.99(m, 1H), 1.68-1.59(m, 1H), 1.39(s, 9H).
MS (ESI+) 351 (M+1, 100 %).

### Reference Example 43

### Ethyl (2E)-[5-[(tert-butoxycarbonyl)amino]-1-(4-methoxybenzyl)piperidin-2-ylidene]acetate

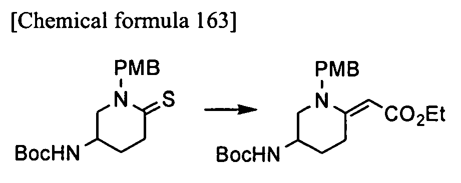

The compound of Reference Example 42 (6.40 g) was dissolved in acetonitrile (18 ml), and thereto was added ethyl bromoacetate (2.43ml) at room temperature and the mixture was stirred for 24 hours. To the reaction mixture were added dichloromethane (36 ml), triphenylphosphine (5.75 g) and triethylamine (3.82 ml), and the mixture was stirred for 24 hours. Water was added to the reaction mixture, and extracted with ethyl acetate three times. The combined organic layers were dried over sodium sulfate, filtered and concentrated under reduced pressure. The obtained residue was purified by silica gel column chromatography (developing solvent: hexane/ethyl acetate = 5 to 3/2) to give the title compound (3.07 g) as a white solid.
¹H-NMR (400MHz, CDCl₃) 7.10(d, J = 8.7 Hz, 1H), 6.88-6.86(m, 1H), 4.79(s, 1H), 4.54(m, 1H), 4.36(d, J = 15.8 Hz, 1H), 4.27(d, J = 15.8 Hz, 1H), 4.04(q, J = 7.1 Hz, 2H), 3.93(brs, 1H), 3.80(s, 3H), 3.58-3.50(m, 1H), 3.47-3.43(m, 1H), 3.08-2.94(m, 2H), 2.07-2.01(m, 1H), 1.58-1.51(m, 1H), 1.41(s, 9H), 1.21(t, J = 7.1 Hz, 3H).
MS (ESI+) 405 (M+1, 100 %).

### Reference Example 44

### Ethyl [(rac.)-(2S,5R)-5-[(tert-butoxycarbonyl)amino]-1-(4-methoxybenzyl)piperidin-2-yl]acetate and ethyl [(rac.)-(2R,5R)-5-[(tert-butoxycarbonyl)amino]-1-(4-methoxybenzyl)piperidin-2-yl]acetate

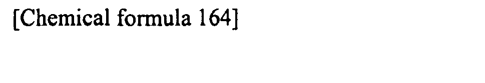

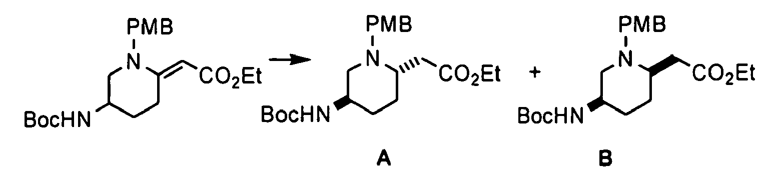

The compound of Reference Example 43 (3.07 g) was dissolved in dichloromethane (75 ml), and thereto were added trifluoroacetic acid (1.69 ml) and sodium cyanoborohydride (715 mg) at 0°C, and the mixture was stirred for 3 hours. To the reaction mixture was added a saturated aqueous sodium hydrogen carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 7/2 to 2) to give the title compound A (1.48 g) and B (1.51 g) as a colorless oily compound, respectively.

A:
   ¹H-NMR (400MHz, CDCl₃) 7.20(d, J = 8.5 Hz, 2H), 6.88-6.85(m, 2H), 4.85(brs, 1H), 4.12(q, J = 7.1 Hz, 2H), 3.80(s, 3H), 3.66(brs, 1H), 3.58(d, J = 13.1 Hz, 1H), 3.41(d, J = 13.1 Hz, 1H), 3.06(brs, 1H), 2.71 (brd, J = 10.7 Hz, 1H), 2.65(dd, J = 3.7, 14.6 Hz, 1H), 2.43(dd, J = 8.6, 14.6 Hz, 1H), 2.06(brs, 1H), 1.88-1.78(m, 1H), 1.55-1.48(m, 1H), 1.41(s, 9H), 1.24(t, J = 7.1 Hz, 3H).
   MS (ESI+) 407 (M+1, 100%).
B:
   ¹H-NMR (400MHz, CDCl₃) 7.18(d, J = 8.6 Hz, 2H), 6.86-6.84(m, 2H), 4.85(brs, 1H), 4.13(q, J = 7.1 Hz, 2H), 3.80(s, 3H), 3.77(d, J = 13.2 Hz, 1H), 3.68(brs, 1H), 3.26(d, J = 13.2 Hz, 1H), 2.89(brs, 1H), 2.68(dd, J = 4.9, 14.7 Hz, 1H), 2.50-2.34(m, 2H), 2.43(dd, J = 7.0, 14.7 Hz, 1H), 1.69-1.63(m, 4H), 1.41(s, 9H), 1.25(t, J = 7.1 Hz, 3H).
   MS (ESI+) 407 (M+1, 100%).

### Reference Example 45

### Ethyl [(rac.)-(2S,5R)-5-amino-1-(4-methoxybenzyl)piperidin-2-yl]acetate

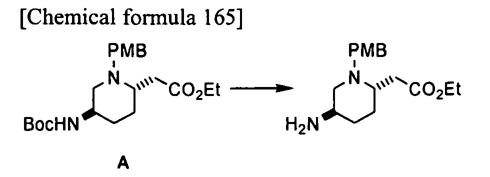

The title compound (1.23 g) was synthesized in a similar manner to Reference Example 6 except that the compound A of Reference Example 44 was used instead of the compound of Reference Example 5 in Reference Example 6.
MS (ESI+) 307 (M+1, 100%).

### Reference Example 46

### Ethyl [(rac.)-(2S,5R)-5-(isopropylamino)-1-(4-methoxybenzyl)piperidin-2-yl]acetate

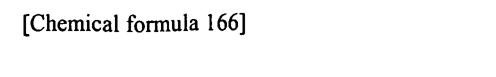

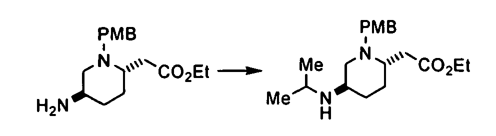

The compound of Reference Example 45 (1.23 g) was dissolved in ethanol (15 ml), and thereto were added acetone (802 µl) and acetic acid (219 µl), and the mixture was stirred at 60°C. Four hours thereafter, to the mixture was added sodium cyanoborohydride (1.14 g), and the mixture was further stirred at room temperature for 12 hours. To the reaction mixture was added a saturated aqueous sodium hydrogen carbonate solution, and the mixture was extracted with chloroform twice. The combined organic layers were washed with aqueous sodium hydrogen carbonate solution, dried over sodium sulfate, filtered and concentrated under reduced pressure. The obtained residue was purified by silica gel column chromatography (developing solvent: chloroform/methanol = 10/1 to 8/1) to give the title compound (966 mg) as a colorless oily compound.
¹H-NMR (400MHz, CDCl₃) 7.21-7.19(m, 2H), 6.86-6.83(m, 2H), 4.14(t, J = 7.2 Hz, 2H), 3.80(s, 3H), 3.78(d, J = 13.2 Hz, 1H), 3.26(d, J = 13.2 Hz, 1H), 2.84-2.71 (m, 5H), 2.41 (dd, J = 7.8, 14.7 Hz, 1H), 1.91-1.74(m, 3H), 1.49(m, 1H), 1.25(t, J = 7.2 Hz, 3H), 1.21-1.18(m, 1H), 0.99-0.95(m, 6H).
MS (ESI+) 349 (M+1, 100%).

### Reference Example 47

### Ethyl [(rac.)-(2S,5R)-5-{isopropyl[4-methoxy-3-(3-methoxypropoxy)benzoyl]amino}-1-(4-methoxybenzyl)piperidin-2-yl]acetate

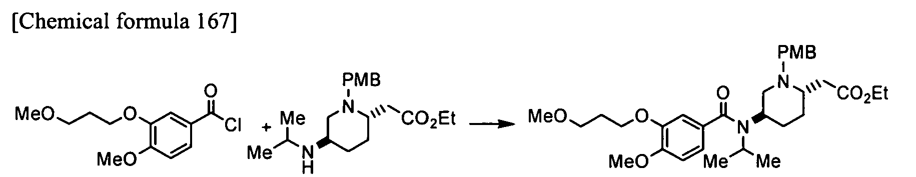

The title compound (921 mg) was synthesized in a similar manner to Reference Example 8 except that the compound of Reference Example 47 was used instead of the compound of Reference Example 7 in Reference Example 8.
MS (ESI+) 571 (M+1, 100%).

### Reference Example 48

### [(rac.)-(2S,5R)-5-{Isopropyl[4-methoxy-3-(3-methoxypropoxy)benzoyl]amino}-1-(4-methoxybenzyl)piperidin-2-yl]acetic acid

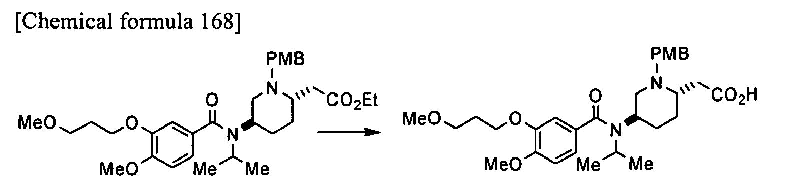

The title compound (253 mg) was synthesized in a similar manner to Reference Example 27 except that the compound of Reference Example 47 was used instead of the compound of Reference Example 26 in Reference Example 27.
MS (ESI+) 543 (M+1, 100%).

### Reference Example 49

### Ethyl [(rac.)-(2R,5R)-5-amino-1-(4-methoxybenzyl)piperidin-2-yl]acetate

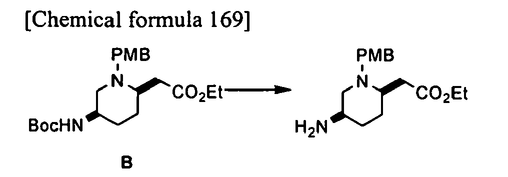

The title compound (144 g) was synthesized in a similar manner to Reference Example 6 except that the compound B of Reference Example 44 was used instead of the compound of Reference Example 5 in Reference Example 6.
MS (ESI+) 307 (M+1, 100 %).

### Reference Example 50

### tert-Butyl (3R)-3-{[4-methoxy-3-(3-methoxypropoxy)benzoyl]amino}piperidine-1-carboxylate

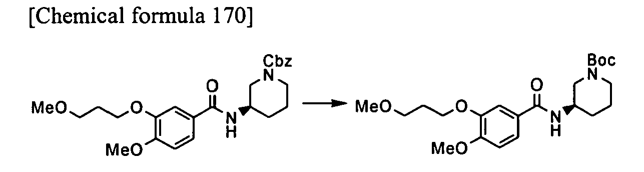

The compound of Reference Example 8 was subjected to reduction in a similar manner to Reference Example 20, and then, the resultant was subjected to Boc-zation in a similar manner to Reference Example 26 to give the title compound (370 mg).
MS (ESI+) 423 (M+1, 100%).

### Reference Example 51

### tert-Butyl (3R)-3-{[4-methoxy-3-(3-methoxypropoxy)benzoyl](methyl)amino}piperidine-1-carboxylate

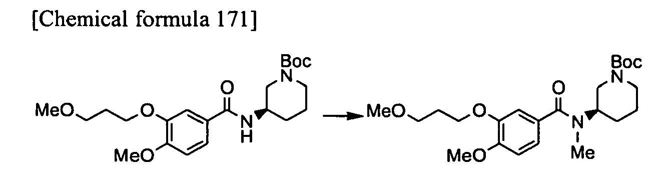

The compound of Reference Example 50 (99.7 mg) was dissolved in dimethylformamide (2 ml), and thereto was added sodium hydride (18.8 mg) at 0°C and the mixture was stirred. Ten minutes thereafter, to the mixture was added iodomethane (29.4 µl), and the mixture was stirred at room temperature for 4 hours. To the reaction mixture was added a saturated aqueous ammonium chloride solution, and the mixture was extracted with ethyl acetate. The organic layer was washed with water twice, and further washed with a saturated aqueous sodium chloride solution. The organic layer is dried over sodium sulfate, filtered and concentrated under reduced pressure. The obtained residue was purified by silica gel column chromatography (developing solvent: hexane/ethyl acetate = 1/2) to give the title compound (65.5 mg) as a colorless oily compound.
MS (ESI+) 437 (M+1, 100 %).

### Reference Example 52

### tert-Butyl (3R)-3-(cyclohexylamino)piperidine-1-carboxylate

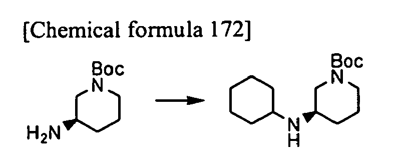

The title compound (232 mg) was synthesized in a similar manner to Reference Example 15 except that the compound of Reference Example 35 and cyclohexanone were used instead of the compound of Reference Example 14 and acetone in Reference Example 15, respectively.
MS (ESI+) 283 (M+1, 100 %).

### Reference Example 53

### tert-Butyl (3R)-3-{cyclohexyl[4-methoxy-3-(3-methoxypropoxy)benzoyl]amino}piperidine-1-carboxylate

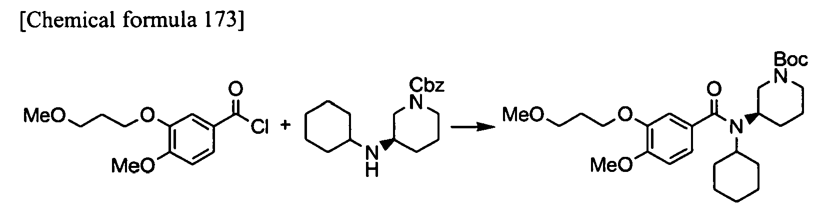

The title compound (85.3 mg) was synthesized in a similar manner to Reference Example 8 except that the compound of Reference Example 52 was used instead of the compound of Reference Example 7 in Reference Example 8.
MS (ESI+) 505 (M+1, 100%).

### Reference Example 54

### tert-Butyl (3R)-3-[(cyclohexylmethyl)amino]piperidine-1-carboxylate

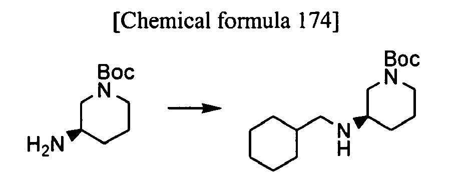

The compound of Reference Example 35 (191 mg) was dissolved in ethanol (3 ml), and thereto were added cyclohexanecarboxyaldehyde (121 µl) and acetic acid (54.5 µl), and the mixture was stirred at 60°C. Three hours thereafter, sodium triacetoxyborohydride (706 mg) was added to the mixture, and the mixture was stirred at room temperature for 3 hours. To the reaction mixture was added a saturated aqueous sodium hydrogen carbonate solution, and the mixture was extracted with chloroform. The organic layer was dried over sodium hydrogen sulfate, filtered and concentrated under reduced pressure. The obtained residue was purified by silica gel column chromatography (developing solvent: chloroform/methanol = 20/1) to give the title compound (246 mg) as a colorless oily compound.
MS (ESI+) 297(M+1, 100%).

### Reference Example 55

### tert-Butyl (3R)-3-{(cyclohexylmethyl)[4-methoxy-3-(3-methoxypropoxy)benzoyl]amino}piperidine-1-carboxylate

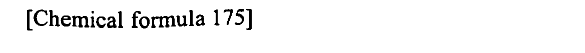

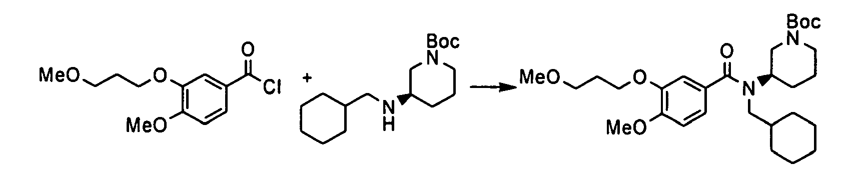

The title compound (155 mg) was synthesized in a similar manner to Reference Example 8 except that the compound of Reference Example 54 was used instead of the compound of Reference Example 7 in Reference Example 8.
MS (ESI+) 519 (M+1, 100 %).

### Reference Example 56

### tert-Butyl (3R)-3-[(4-chlorobenzyl)amino]piperidine-1-carboxylate

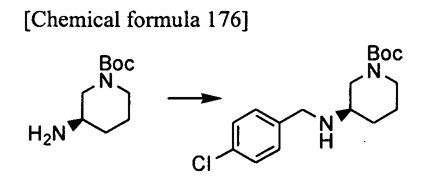

The title compound (182 mg) was synthesized in a similar manner to Reference Example 54. MS (ESI+) 325 (M+1, 100 %).

### Reference Example 57

### tert-Butyl (3R)-3-{(4-chlorobenzyl)[4-methoxy-3-(3-methoxypropoxy)benzoyl]amino}piperidine-1-carboxylate

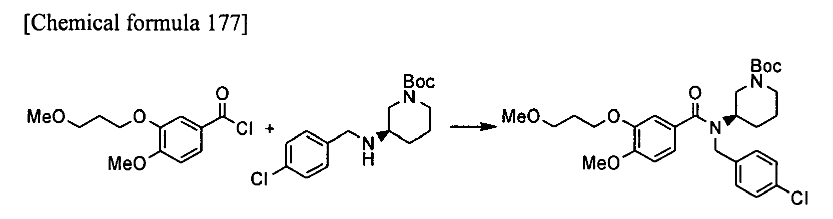

The title compound (279 mg) was synthesized in a similar manner to Reference Example 8 except that the compound of Reference Example 56 was used instead of the compound of Reference Example 7 in Reference Example 8.
MS (ESI+) 548 (M+1, 100%).

### Reference Example 58

### tert-Butyl (3R)-3-{ethyl[4-methoxy-3-(3-methoxypropoxy)benzoyl]amino}piperidine-1-carboxylate

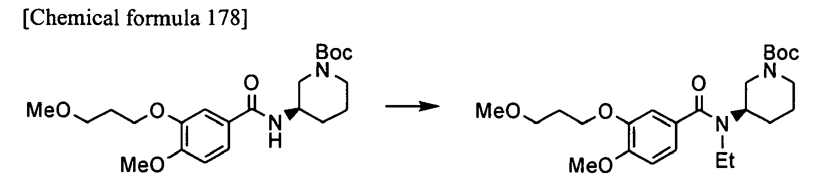

The title compound (45.7mg) was synthesized in a similar manner to Reference Example 51 except that iodoethane was used instead of iodomethane in Reference Example 51.
MS (ESI+) 451(M+1, 100%).

### Reference Examples 59 - 60

The compounds as listed in the following Table were synthesized in a similar manner to Reference Example 51.

**[Table 1]**

| Ref.Ex. | Structure | MS (ESI+) |
|---|---|---|
| 59 | 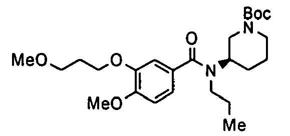 | 465 (M+1,100%) |
| 60 | 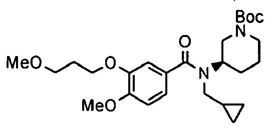 | 477 (M+1,100%) |

### Reference Example 61

### 1-{[(rac.)-(25,5R)-5-(Isopropyl[4-methoxy-3-(3-methoxypropoxy)benzoyl]amino}-1-(4-methoxybenzyl)piperidin-2-yl]acetyl}piperidine-4-carboxyamide

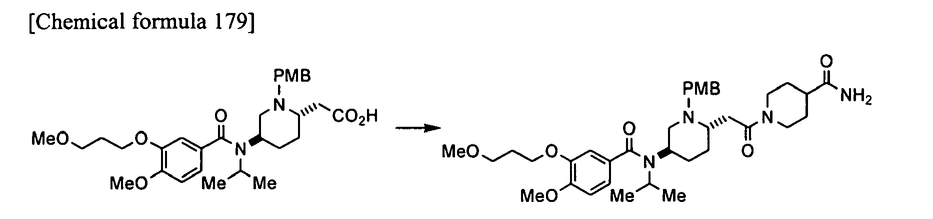

The compound of Reference Example 48 (51.0 mg) was dissolved in dimethylformamide (1 ml), and thereto were added 1-hydroxybenzotriazole (43.2 mg), 1-ethyl-3-(dimethylaminopropyl)-carbodiimide · hydrochloride (54.0 mg), triethylamine (39.3 µl) and isonipecotic amide (15.7 mg), and the mixture was stirred at room temperature for 21 hours. To the reaction mixture was added a saturated aqueous ammonium chloride solution, and the mixture was extracted with ethyl acetate. The organic layer was washed with a saturated aqueous sodium hydrogen carbonate solution, water and a saturated aqueous sodium chloride solution, dried over sodium sulfate, filtered and concentrated under reduced pressure. The obtained residue was purified by silica gel column chromatography (developing solvent: chloroform/methanol = 10) to give the title compound (47.2 mg) as a colorless oily compound. MS (ESI+) 653 (M+1, 100%).

### Reference Example 62

### N-[(rac.)-(2S,5R)-6-[2-(Butylamino)-2-oxoethyl]-1-(4-methoxybenzyl)piperidin-3-yl]-N-isopropyl-4-methoxy-3-(3-methoxypropoxy)benzamide

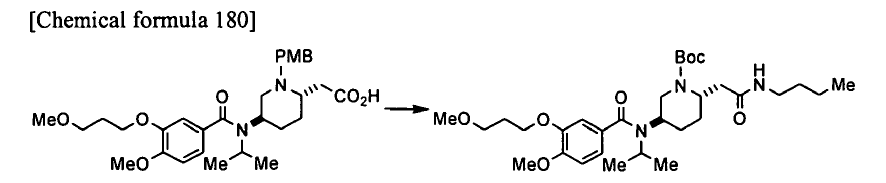

The title compound (14.0 mg) was synthesized in a similar manner to Reference Example 61 except that n-butylamine was used instead of isonipecotic amide in Reference Example 61.
MS (ESI+) 598 (M+1, 100%).

### Reference Example 63

### tert-Butyl (rac.)-(2S,5R)-2-(2-ethoxy-2-oxoethyl)-5-{isopropyl[4-methoxy-3-(3-methoxypropoxy)benzoyl]amino}piperidine-1-carboxylate

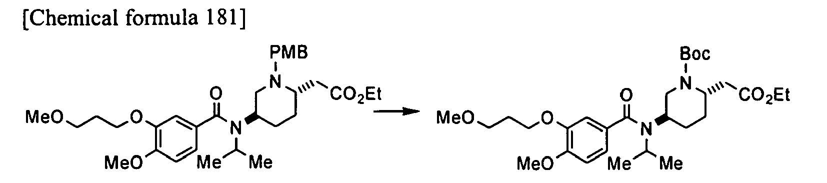

The title compound (442 mg) was synthesized in a similar manner to Reference Example 80 except that the compound of Reference Example 47 was used instead of the compound of Reference Example 79 in Reference Example 80.
MS (ESI+) 551 (M+1, 100 %).

### Reference Example 64

### 1-tert-Butyl 3-methyl(rac.)-(3R,5S)-5-(isopropylamino)piperidine-1,3-dicarboxylate

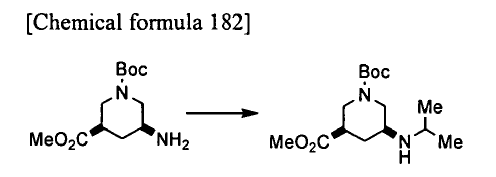

Using 1-tert-butyl 3-methyl(rac.)-(3R,5S)-5-aminopiperidine-1,3-dicarboxylate (US Patent 5,817,678; 475 mg) as a starting compound, the title compound (341 mg) was obtained as a colorless oily product in a similar manner to Reference Example 15.
¹H-NMR (400MHz, CDCl₃) δ 5.50-4.50(brs, 1H), 4.45-4.20(m, 2H), 3.69(s, 3H), 3.01-2.95(m, 1H), 2.78-2.60(m, 2H), 2.54-2.49(m, 1H), 2.30-2.23(m, 2H), 1.46(s, 9H), 1.33-1.24(m, 1H), 1.07-1.00(m, 6H).
MS (ESI+) 301 ([M+H]⁺, 100%).

### Reference Example 65

### 1-tert-Butyl 3-methyl(rac.)-(3S,5R)-5-{isopropyl[4-methoxy-3-(3-methoxypropoxy)benzoyl]-amino}piperidine-1,3-dicarboxylate

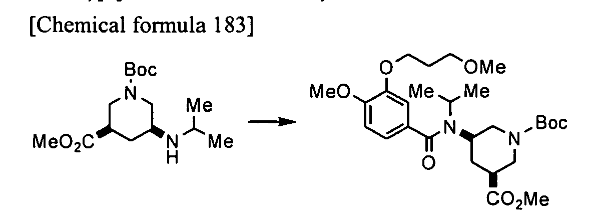

Using the compound of Reference Example 64 (259 mg) as a starting compound, the title compound (480 mg) was obtained as white amorphous in a similar manner to Reference Example 8. ¹H-NMR (400MHz, CDCl₃) δ 6.89-6.84(m, 3H), 4.20-4.17(m, 1H), 4.13-4.10(m, 2H), 4.01-3.95(m, 2H), 3.88(s, 3H), 3.70(s, 3H), 3.59-3.54(m, 2H), 3.35(s, 3H), 3.20-3.02(m, 1H), 2.85-2.80(m, 1H), 2.55-2.50(m, 1H) 2.14-2.04(m, 4H), 1.47(s, 9H), 1.27-1.21(m, 7H).
MS (ESI+) 523 ([M+H]⁺, 95 %).

### Reference Example 66

### tert-Butyl (rac.)-(3S,5R)-3-{[benryl(methyl)amino]carbonyl}-5-{isopropyl[4-methoxy-3-(3-methoxypropoxy)benzoyl]amino}piperidine-1-carboxylate

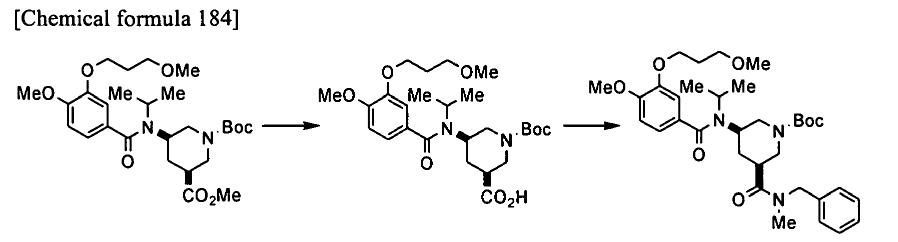

To a solution of the compound of Reference Example 65 (408 mg) in methanol (7.0 ml) was added 1N aqueous lithium hydroxide solution (7.8 ml), and the mixture was stirred at room temperature for one hour. To the reaction solution was added a 5% aqueous potassium hydrogen sulfate solution, and the mixture was extracted with ethyl acetate. The organic layer was washed with a saturated aqueous sodium chloride solution, dried over sodium sulfate, filtered and concentrated under reduced pressure to give a carboxylic acid intermediate (0.42 g). To a solution of the obtained carboxylic acid (50.8 mg) in N,N-dimethylformamide (0.5 ml) were added N-methylbenzylamine (0.0193 ml), 1-hydroxybenzotriazole (16.8 mg), 1-ethyl-3-(3-dimethylaminopropyl)-carbodiimide hydrochloride (23.0 mg), and triethylamine (0.0347 ml) at 0°C, and the mixture was stirred at room temperature for 18 hours. To the reaction mixture was added water, and the mixture was extracted with ethyl acetate. The organic layer was washed with a saturated aqueous sodium chloride solution, dried over sodium sulfate, filtered and concentrated under reduced pressure. The obtained residue was purified by preparative thin layer silica gel chromatography (hexane/ethyl acetate = 1/1 to 1/6) to give the title compound (39.8 mg) as white amorphous.
¹H-NMR (400MHz, CDCl₃) δ 7.32-7.21(m, 5H), 6.90-6.85(m, 3H), 4.70-4.50(m, 2H), 4.30-3.90(m, 6H), 3.87-3.85(m, 4H), 3.56(t, J=6.1 Hz, 2H), 3.35(s, 3H), 3.00-2.95(m, 4H), 2.80-2.70(m, 1H), 2.12-2.09(m, 2H), 1.90-1.82(m, 1H) 1.45(s, 9H), 1.38-1.24(m, 7H),.
MS (ESI+) 612 ([M+H]⁺, 31 %).

### Reference Example 67

### (rac.)-(3R,5R)-1-(tert-Butoxycarbonyl)-5-(methoxycarbonyl)piperidine-3-carboxylic acid

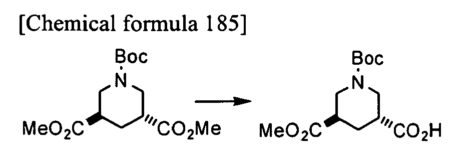

To a solution of 1-tert-butyl 3,5-dimethyl(rac.)-(3R,5R)-piperidine-1,3,5-tricarboxylate (US Patent 5,817,678; 1.14 g) in ethyleneglycol dimethyl ether (8.0 ml) were added water (8.0 ml) and aqueous lithium hydroxide solution (159 mg), and the mixture was stirred at room temperature for 18 hours. To the reacting solution was added water, and the mixture was washed with toluene. To the aqueous layer was added a 5% aqueous potassium hydrogen sulfate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (chloroform/methanol = 100/1 to 100/3) to give the title compound (798 mg) as a colorless oily product.
¹H-NMR (400MHz, CDCl₃) δ 3.75-3.51(m, 4H), 3.70(s, 3H), 2.88-2.78(m, 2H), 2.15-2.05(m, 2H), 1.44(s, 9H).
MS (ESI+) 288 ([M+H]⁺, 35 %).

### Reference Example 68

### 1-tert-Butyl 3-methyl(rac.)-(3R,5R)-5-{[(benzoyl)carbonyl]amino}piperidine-1,3-dicarboxylate

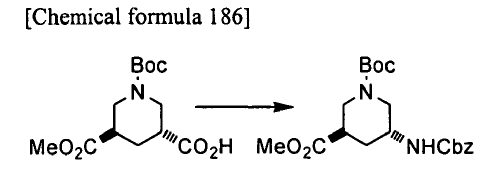

To a solution of the compound of Reference Example 67 (679 mg) and triethylamine (0.360 ml) in toluene (11 ml) was added dropwise a solution of diphenylphosphoryl azide (682 mg) in toluene (2.0 ml) at 80°C. One hour thereafter, the reaction solution was cooled to 50°C, and thereto was added benzyl alcohol (0.488 ml), and the mixture was stirred at 90°C for 3 hours. The reaction mixture was cooled to room temperature, and water was added to the reaction solution. The mixture was extracted with ethyl acetate, and the organic layer was washed with a saturated sodium hydrogen carbonate solution, water, a 5 % aqueous potassium hydrogen sulfate solution and a saturated aqueous sodium chloride solution, dried over sodium sulfate, filtered and concentrated under reduced pressure. The obtained residue was purified by silica gel column chromatography (hexane/ethyl acetate = 9/1 to 2/1) to give the title compound (703 mg) as a colorless oily product.
¹H-NMR (400MHz, CDCl₃) δ 7.39-7.32(m, 5H), 5.10(s, 2H), 4.98-4.95(m, 1H), 4.14-4.05(m, 1H), 3.98-3.94(m, 2H), 3.68(s, 3H), 3.04-2.97(m, 2H), 2.66-2.60(m, 1H), 2.22-2.20(m, 1H), 1.83-1.80(m, 1H), 1.44(s, 9H).
MS (ESI+) 393 ([M+H]⁺, 26%).

### Reference Example 69

### 1-tert-Butyl 3-methyl (rac.)-(3R,5R)-5-(isopropylamino)piperidine-1,3-dicarboxylate

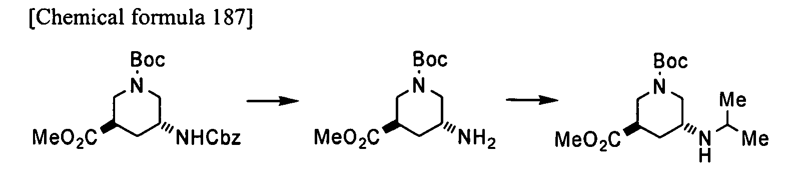

A suspension of the compound of Reference Example 68 (609 mg) and 10 % Pd/C (50 % wet, 207 mg) in methanol (9.0 ml) was stirred under hydrogen atmosphere at room temperature for 4 hours. After replacement with nitrogen gas, the reaction solution was filtered through celite, and concentrated under reduced pressure. To a solution of the obtained condensed residue in dichloromethane (10 ml) were added acetone (0.445 ml) and acetic acid (0.116 ml), and the mixture was stirred at room temperature for 2 hours. To the reaction solution was added triacetoxy borohydride (1.07 g), and the mixture was stirred at room temperature for 18 hours. To the reaction solution was added a 5 % aqueous potassium carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (chloroform/methanol = 100/1 to 100/3) to give the title compound (289 mg) as a colorless oily product.
¹H-NMR (400MHz, CDCl₃) δ 4.01-3.90(m, 1H), 3.68(s, 3H), 3.68-3.15(m, 4H), 2.98-2.89(m, 2H), 2.79-2.73(m, 1H), 1.95-1.91(m, 1H), 1.82-1.76(m, 1H), 1.45(s, 9H), 1.05-0.99(m, 6H).
MS (ESI+) 301 ([M+H]⁺, 30%).

### Reference Example 70

### 1-tert-Butyl 3-methyl(rac.)-(3R,5R)-5-{isopropyl[4-methoxy-3-(3-methoxypropoxy)-benzoyl]amino}piperidine-1,3-dicarboxylate

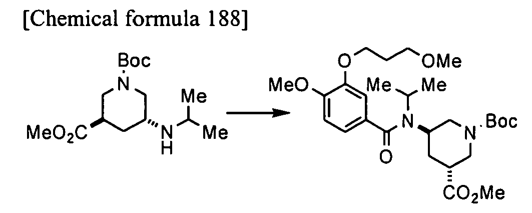

Using the compound of Reference Example 69 (219 mg) as a starting compound, the title compound (61.3 mg) was obtained as white amorphous in a similar manner to Reference Example 8.
¹H-NMR (400MHz, CDCl₃) δ 6.94-6.84(m, 3H), 4.52-4.49(m, 1H), 4.12(t, J=6.5 Hz, 2H), 4.05-4.00(m, 2H), 3.88(s, 3H), 3.72-3.68(m, 4H), 3.56(t, J=6.0 Hz, 2H), 3.35(s, 3H), 3.19-2.92(m, 2H), 2.83-2.79(m, 1H), 2.14-2.04(m, 4H), 1.45(s, 9H), 1.27-1.22(m, 6H).
MS (ESI+) 523 ([M+H]⁺, 63 %).

### Reference Example 71

### Ethyl (rac.)-(2R,5R)-[5-(isopropylamino)-1-(4-methoxybenzyl)piperidin-2-yl]acetate

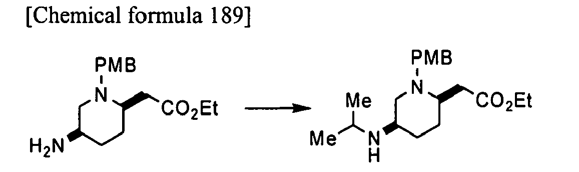

The title compound (1.17 g) was synthesized in a similar manner to Reference Example 54 except that acetone was used instead of cyclohexane carboxyaldehyde in Reference Example 54. MS (ESI+) 349 (M+1, 100 %).

### Reference Example 72

### ((rac.)-(2S,5R)-1-(tert-Butoxycarbonyl)-5-{isopropyl[4-methoxy-3-(3-methoxypropoxy)-benzoyl]amino}piperidin-2-yl)acetic acid

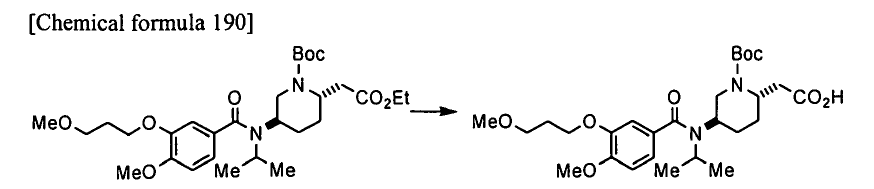

The title compound (225 mg) was synthesized in a similar manner to Reference Example 27 except that the compound of Reference Example 63 was used instead of the compound of Reference Example 26 in Reference Example 28.
MS (ESI+) 523 (M+1, 100%).

### Reference Example 73

### Methyl 3-(benzyloxy)-4-methoxybenzoate

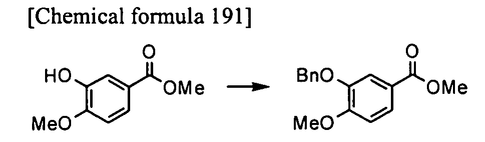

The title compound (2.13 g) was synthesized in a similar manner to Reference Example 29 except that the commercially available methyl 3-(hydroxy)-4-methoxybenzoate was used instead of the compound of Reference Example 28 in Reference Example 29.
¹H-NMR (400MHz, CDCl₃) δ 7.69(dd, J = 2.0, 8.5 Hz, 1H), 7.61(d, J = 2.0 Hz, 1H), 7.48-7.46(m, 2H), 7.40-7.36(m, 2H), 7.33-7.31(m, 1H), 6.90(d, J = 8.5 Hz, 1H), 3.93(s, 3H), 3.87(s, 3H).

### Reference Example 74

### 3-(Benzyloxy)-4-methoxybenzoic acid

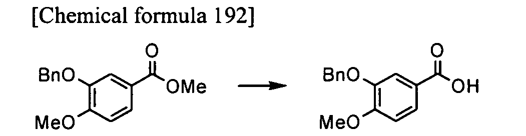

The title compound (1.91 g) was synthesized in a similar manner to Reference Example 3 except that the compound of Reference Example 73 was used instead of the compound of Reference Example 2 in Reference Example 3.
¹H-NMR (400MHz, CDCl₃) δ 7.77(dd, J = 2.0, 8.4 Hz, 1H), 7.65(d, J = 2.0 Hz, 1H), 7.49-7.47(m, 2H), 7.41-7.37(m, 2H), 7.34-7.32(m, 1H), 6.93(d, J = 8.4 Hz, 1H), 5.20(s, 2H), 3.95(s, 3H).

### Reference Example 75

### tert-Butyl (3R)-3-(isopropylamino)piperidine-1-carboxylate

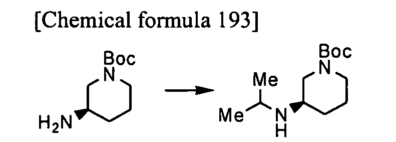

The title compound (1.49 g) was synthesized in a similar manner to Reference Example 15 except that the compound of Reference Example 35 was used instead of the compound of Reference Example 14 in Reference Example 15.
MS (ESI+) 243 (M+1, 100 %).

### Reference Example 76

### tert-Butyl (3R)-3-[[3-(benzyloxy)-4-methoxybenzoyl](isopropyl)amino]piperidine-1-carboxylate

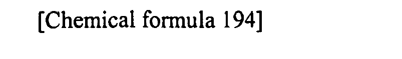

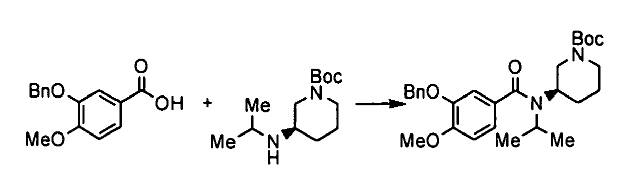

The compound of Reference Example 74 (716 mg) was dissolved in dichloromethane (10 ml), and thereto was added oxalyl chloride (369 µl), and the mixture was stirred at room temperature for 4 hours. To the reaction mixture were added triethylamine (1.16 ml) and the compound of Reference Example 73 (671 mg), and the mixture was stirred at room temperature for 3 hours. To the reaction mixture was added a saturated aqueous ammonium chloride solution, and the mixture was extracted with ethyl acetate. The organic layer was washed with a saturated aqueous sodium carbonate solution and a saturated aqueous sodium chloride solution, dried over sodium sulfate, filtered and concentrated under reduced pressure. The obtained residue was purified by silica gel column chromatography (developing solvent: hexane/ethyl acetate = 2) to give the title compound (326 mg) as a colorless oily compound.
MS (ESI+) 483 (M+1, 100%).

### Reference Example 77

### tert-Butyl (3R)-3-[(3-hydroxy-4-methoxybenzoyl)(isopropyl)amino]piperidine-1-carboxylate

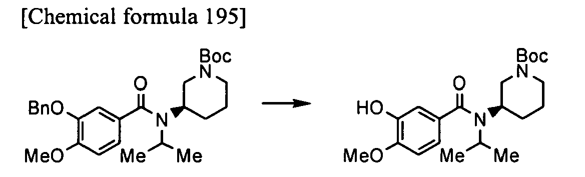

The title compound (211 mg) was synthesized in a similar manner to Reference Example 20 except that the compound of Reference Example 76 was used instead of Reference Example 19 in Reference Example 20.
MS (ESI+) 393 (M+1, 100 %).

### Reference Example 78

### tert-Butyl (3R)-3-[{3-[2-(acetylamino)ethoxy]-4-methoxybenzoyl}(isopropyl)amino]piperidine-1-carboxylate

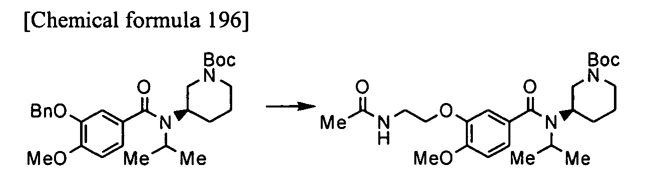

The compound of Reference Example 77 (60.1 mg) was dissolved in dichloromethane (2 ml), and thereto were added 2-acetamido ethanol (14.8 µl), triphenylphosphine (40.3 mg) and diisopropyl azodicarboxylate (77.3 mg), and the mixture was stirred at room temperature for 12 hours. The solvent was removed by evaporation, and the obtained residue was purified by silica gel column chromatography (developing solvent: chloroform/methanol = 10) to give the title compound (9.3 mg) as a colorless oily compound.
MS (ESI+) 478 (M+1, 100%).

### Reference Example 79

### Ethyl [(rac.)-(2R,5R)-5-{isopropyl[4-methoxy-3-(3-methoxypropoxy)benzoyl]amino}-1-(4-methoxybenzyl)piperidin-2-yl]acetate

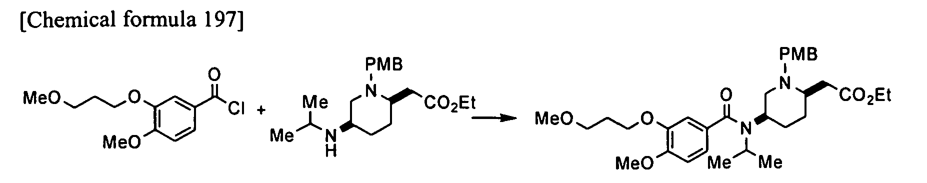

The title compound (262 mg) was synthesized in a similar manner to Reference Example 8 except that the compound of Reference Example 71 was used instead of the compound of Reference Example 7 in Reference Example 8.
MS (ESI+) 571 (M+1, 100 %).

### Reference Example 80

### tert-Butyl (rac.)-(2R,5R)-2-(2-ethoxy-2-oxoethyl)-5-{isopropyl[4-methoxy-3-(3-methoxypropoxy)benzoyl]amino}piperidine-1-carboxylate

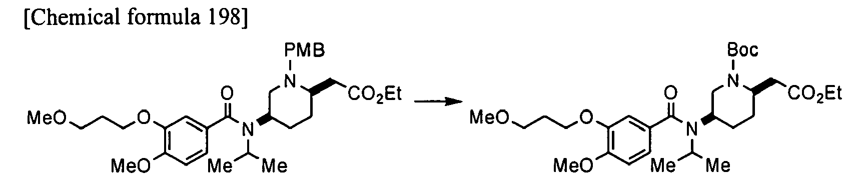

The compound of Reference Example 77 (245 mg) was dissolved in methanol (5 ml), and thereto was added a 10 % palladium carbon (304 mg), and the mixture was stirred at room temperature under hydrogen atmosphere for 3 hours. After filtration on celite, the filtrate was concentrated under reduced pressure. To the residue were added tetrahydrofuran (2 ml) and a saturated aqueous sodium hydrogen carbonate solution (2 ml), and further thereto was added di-tert-butyl dicarbonate (188 mg). The mixture was stirred at room temperature for 12 hours. Water was added to the reaction mixture, and the mixture was extracted with ethyl acetate. The organic layer was washed with a saturated aqueous sodium chloride solution, dried over anhydrous sodium sulfate, filtered, and the filtrate was concentrated under reduced pressure. The obtained residue was purified by silica gel column chromatography (developing solvent: hexane/ethyl acetate = 1) to give the title compound (206 mg) as a colorless oily compound.
MS (ESI+) 551 (M+1, 100 %).

### Reference Example 81

### ((rac.)-(2R,5R)-1-(tert-Butoxycarbonyl)-5-{isopropyl[4-methoxy-3-(3-methoxypropoxy)benzoyl]amino}piperidin-2-yl)acetic acid

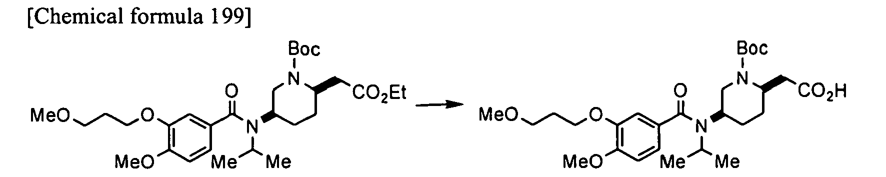

The title compound (145 mg) was synthesized in a similar manner to Reference Example 27 except that the compound of Reference Example 80 was used instead of the compound of Reference Example 26 in Reference Example 27.
MS (ESI+) 523 (M+1, 100 %).

### Reference Example 82

### tert-Butyl (rac.)-(2S,5R)-2-{2-[(2-amino-2-oxoethyl)amino]-2-oxoethyl}-5-{isopropyl[4-methoxy-3-(3-methoxypropoxy)benzoyl]amino}piperidine-1-carboxylate

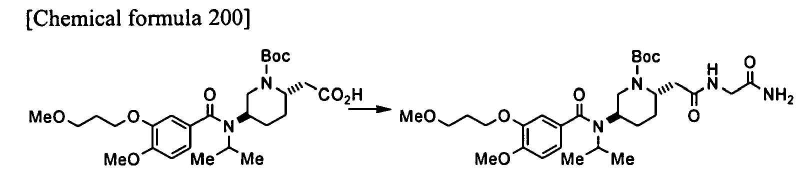

The title compound (21.7 mg) was synthesized in a similar manner to Reference Example 61.
MS (ESI+) 579 (M+1, 100 %).

### Reference Example 83

### tert-Butyl (rac.)-(2S,5R)-2-{2-[[2-(dimethylamino)ethyl](ethyl))amino]-2-oxoethyl}-5-{isopropyl[4-methoxy-3-(3-methoxypropoxy)benzoyl]amino}piperidine-1-carboxylate

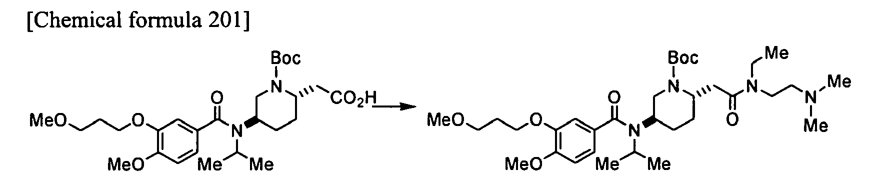

The title compound (16.0 mg) was synthesized in a similar manner to Reference Example 61.
MS (ESI+) 621 (M+1, 100 %).

### [0571] Reference Example 84

### tert-Butyl (rac.)-(2S,5R)-2-{2-[ethyl(isopropyl)amino]-2-oxoethyl}-5-{isopropyl[4-methoxy-3-(3-methoxypropoxy)benzoyl]amino}piperidine-1-carboxylate

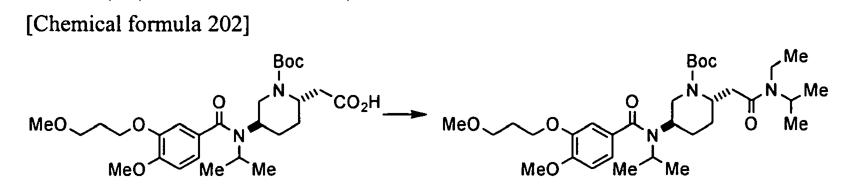

The title compound (17.0 mg) was synthesized in a similar manner to Reference Example 61.
MS (ESI+) 592 (M+1, 100 %).

### Reference Example 85

### tert-Butyl (rac.)-(2R,5R)-2-{2-[ethyl(isopropyl)amino]-2-oxoethyl}-5-{isopropyl[4-methoxy-3-(3-methoxypropoxy)benzoyl]amino}piperidine-1-carboxylate

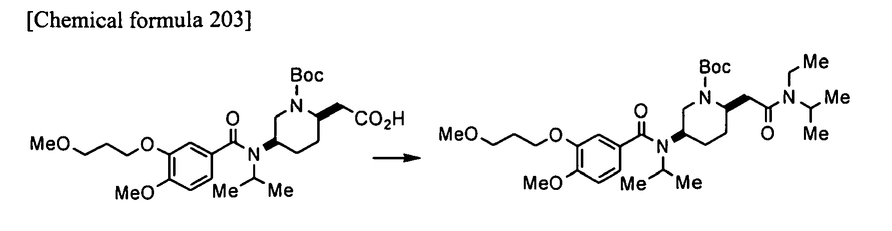

The title compound (2.3 mg) was synthesized in a similar manner to Reference Example 61.
MS (ESI+) 592 (M+1, 100 %).

### Reference Example 86

### tert-Butyl (rac.)-(2S,5R)-2-{2-[(cyclopropylmethyl)(propyl)amino]-2-oxoethyl}-5-{isopropyl[4-methoxy-3-(3-methoxypropoxy)benzoyl]amino}piperidine-1-carboxylate

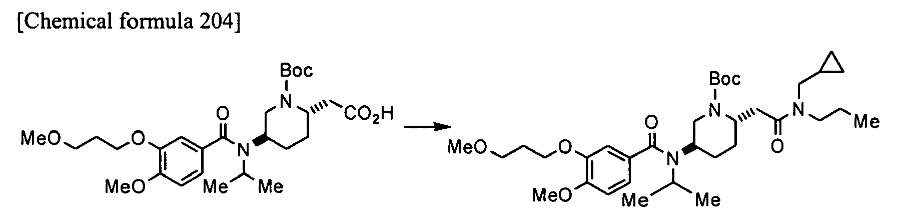

The title compound (6.8 mg) was synthesized in a similar manner to Reference Example 61.
MS (ESI+) 618 (M+1, 100 %).

### Reference Example 87

### tert-Butyl (rac.)-(2S,5R)-2-(2-hydroxyethyl)-5-{isopropyl[4-methoxy-3-(3-methoxypropoxy)benzoyl]amino}piperidine-1-carboxylate

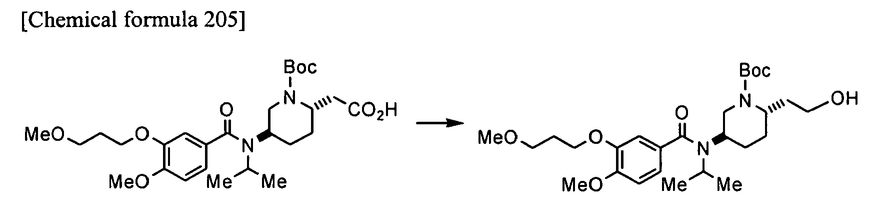

The title compound (120 mg) was synthesized in a similar manner to Reference Example 28 except that the compound of Reference Example 72 was used instead of the compound of Reference Example 27 in Reference Example 28.
MS (ESI+) 509 (M+1, 100 %).

### Reference Example 88

### tert-Butyl (rac.)-(2S,5R)-2-(2-hydroxyethyl)-5-{isopropyl[4-methoxy-3-(3-methoxypropoxy)benzoyl]amino}piperidine-1-carboxylate

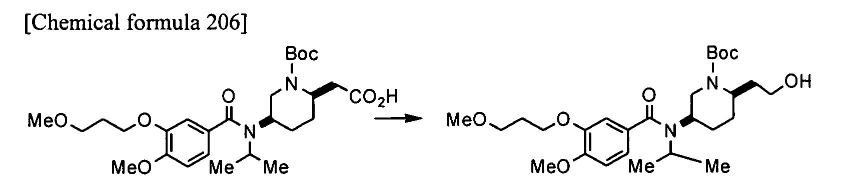

The title compound (91.5 mg) was synthesized in a similar manner to Reference Example 87 except that the compound of Reference Example 81 was used instead of the compound of Reference Example 72 in Reference Example 87.
MS (ESI+) 509 (M+1, 100 %).

### Reference Example 89

### tert-Butyl (rac.)-(2S,5R)-5-{isopropyl[4-methoxy-3-(3-methoxypropoxy)benzoyl]amino-2-(2-oxoethyl)piperidine-1-carboxylate

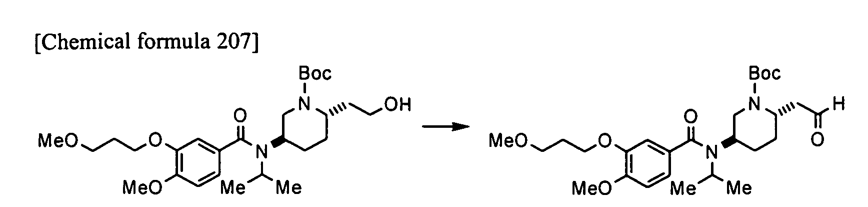

The compound of Reference Example 87 (56.2 mg) was dissolved in dichloromethane (2 ml), and thereto were added Molecular Sieves 4A (70 mg), N-methylmorpholine-N-oxide (19.4 mg) and tetra-n-propylammonium perruthenate (7.5 mg), and the mixture was stirred at room temperature for 3 hours. The reaction mixture was filtered through celite with chloroform, and the organic layer was washed with a saturated aqueous ammonium chloride solution, dried over anhydrous sodium sulfate, filtered, and the filtrate was concentrated under reduced pressure. The obtained residue was purified by silica gel column chromatography (developing solvent: chloroform/methanol = 20 to 10) to give the title compound (37.5 mg) as a colorless oily compound.
MS (ESI+) 507 (M+1, 100 %).

### Reference Example 90

### tert-Butyl (rac.)-(2R,5R)-5-{isopropyl[4-methoxy-3-(3-methoxypropoxy)benzoyl]amino}-2-(2-oxoethyl)piperidine-1-carboxylate

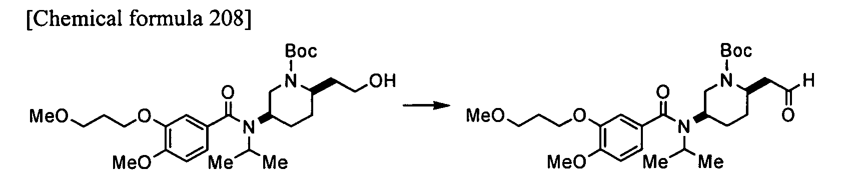

The title compound (43.8 mg) was obtained in a similar manner to Reference Example 89 except that the compound of Reference Example 88 was used instead of the compound of Reference Example 87 in Reference Example 89.
MS (ESI+) 507 (M+1, 100 %).

### Reference Example 91

### tert-Butyl (rac.)-(2S,5R)-2-[2-(cyclopropylamino)ethyl]-5-{isopropyl[4-methoxy-3-(3-methoxypropoxy)benzoyl]amino}piperidine-1-carboxylate

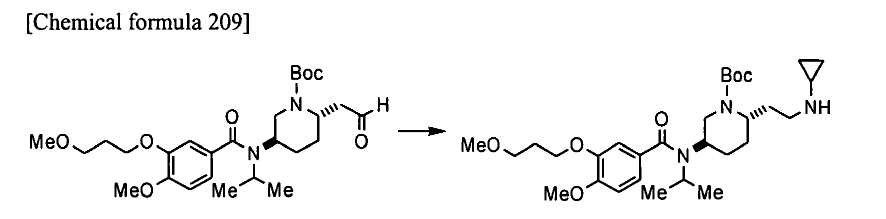

The title compound (13.2 mg) was synthesized in a similar manner to Reference Example 46 except that cyclopropylamine and the compound of Reference Example 89 were used instead of the compound of Reference Example 45 and acetone, respectively, in Reference Example 46.
MS (ESI+) 548 (M+1, 100 %).

### Reference Example 92

### tert-Butyl (rac.)-(2R,5R)-2-[2-(cyclopropylamino)ethyl]-5-{isopropyl[4-methoxy-3-(3-methoxypropoxy)benzoyl]amino}piperidine-1-carboxylate

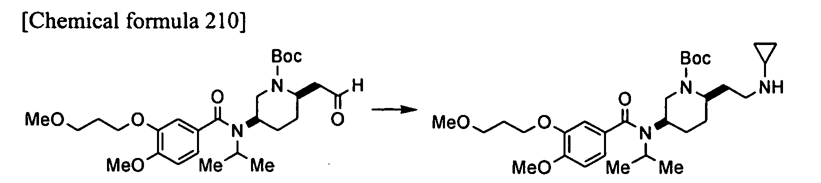

The title compound (25.9 mg) was synthesized in a similar manner to Reference Example 91 except that the compound of Reference Example 90 was used instead of the compound of Reference Example 89 in Reference Example 91.
MS (ESI+) 548 (M+1, 100 %).

### Reference Example 93

### tert-Butyl (rac.)-(2S,5R)-2-{2-[cyclopropyl(2-oxo-2-phenylethyl)amino]ethyl}-5-{isopropyl[4-methoxy-3-(3-methoxypropoxy)benzoyl]amino}piperidine-1-carboxylate

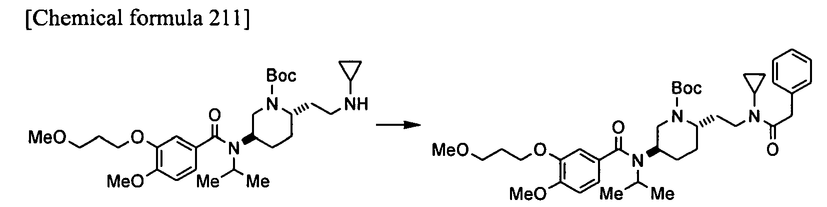

The compound of Reference Example 91 (13.2 mg) was dissolved in dichloromethane (2 ml), and thereto were added triethylamine (16.8 µl) and phenylacetyl chloride (9.6 µl) at room temperature, and the mixture was stirred for 2 hours. To the reaction mixture was added a saturated aqueous ammonium chloride solution, and the mixture was extracted with chloroform. The organic layer was dried over anhydrous sodium sulfate, filtered and concentrated under reduced pressure. The obtained residue was purified by silica gel column chromatography (developing solvent: chloroform/methanol = 10) to give the title compound (6.0 mg) as a colorless oily compound.
MS (ESI+) 666 (M+1, 100%).

### Reference Example 94

### tert-Butyl (rac.)-(2R,5R)-2-{2-[cyclopropyl(2-oxo-2-phenylethyl)amino]ethyl}-5-{isopropyl[4-methoxy-3-(3-methoxypropoxy)benzoyl]amino}piperidine-1-carboxylate

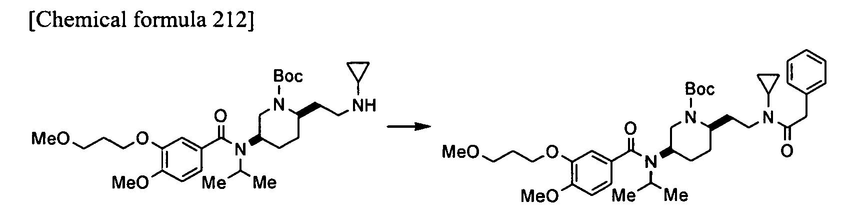

The compound of Reference Example 92 (25.9 mg) was dissolved in dichloromethane (1 ml), and thereto were added triethylamine (32.9 µl) and phenylacetyl chloride (18.7 µl) at room temperature, and the mixture was stirred for 2 hours. To the reaction mixture was added a saturated aqueous ammonium chloride solution, and the mixture was extracted with ethyl acetate. The organic layer was dried over anhydrous sodium sulfate, filtered and concentrated under reduced pressure. The obtained residue was purified by silica gel column chromatography (developing solvent: hexane/ethyl acetate = 1/3) to give the title compound (20.1 mg) as a colorless oily compound.
MS (ESI+) 666 (M+1, 100 %).

### Reference Example 95

### tert-Butyl (rac.)-(3S,5R)-3-(hydroxymethyl)-5-{isopropyl[4-methoxy-3-(3-methoxypropoxy)benzoyl]amino}piperidine-1-carboxylate

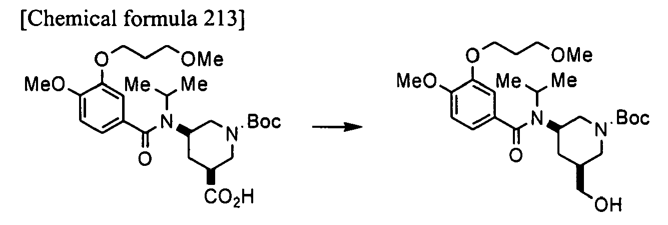

The title compound (23.5 mg) was synthesized in a similar manner to Reference Example 28. ¹H-NMR (400MHz, CDCl₃) δ 6.90-6.84(m, 3H), 4.25-4.18(m, 1H), 4.12(t, J=6.5 Hz, 2H), 4.05-3.90(m, 3H), 3.88(s, 3H), 3.58-3.52(m, 4H), 3.35(s, 3H), 2.55-2.40(m, 2H), 2.14-2.08(m, 2H), 1.82-1.80(m, 4H), 1.46(s, 9H), 1.28-1.21(m, 6H).
MS (ESI+) 495 ([M+H]⁺, 14%).

### Reference Example 96

### tert-Butyl 3-(cyclopropylamino)piperidine-1-carboxylate

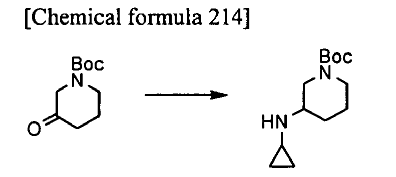

The title compound (234 mg) was synthesized in a similar manner to Reference Example 54.
MS (ESI+) 241 (M+1, 100%).

### Reference Example 97

### tert-Butyl 3-{cyclopropyl[4-methoxy-3-(3-methoxypropoxy)benzoyl]amino}piperidine-1-carboxylate

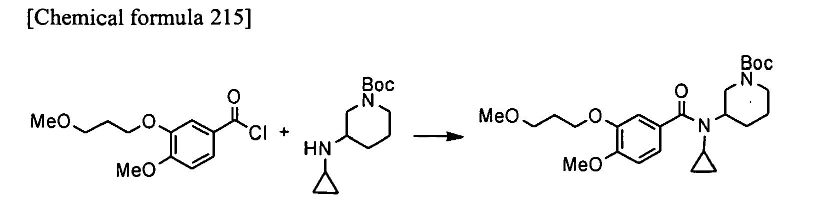

The title compound (112 mg) was synthesized in a similar manner to Reference Example 8 except that the compound of Reference Example 96 was used instead of the compound of Reference Example 7 in Reference Example 8.
MS (ESI+) 463 (M+1, 100 %).

### Reference Example 98

### 2-Methoxy-1-(3-methoxypropoxy)-4-methylbenzene

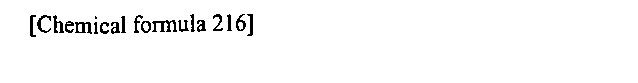

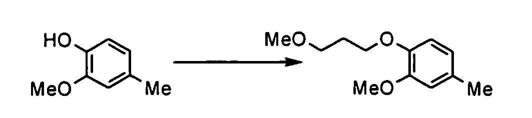

The title compound (2.69 g) was synthesized in a similar manner to Reference Example 2.
¹H-NMR (400MHz, CDCl₃) 6.80(d, J = 7.9 Hz, 1H), 6.70-6.67(m, 2H), 4.08(t. J = 6.5 Hz, 2H), 3.85(s, 3H), 3.57(t, J = 6.2 Hz, 2H), 3.35(s, 3H), 2.30(s, 3H), 2.12-2.05(m, 2H).

### Reference Example 99

### 1-Bromo-4-methoxy-5-(3-methoxypropoxy)-2-methylbenzene

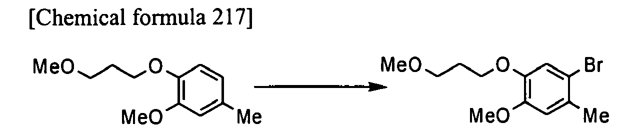

The compound of Reference Example 98 (2.69 g) was dissolved in acetonitrile (40 ml), and thereto was added N-bromosuccinimide (2.50 g), and the mixture was stirred at room temperature for 6 hours. The reaction mixture was concentrated, and the obtained residue was purified by silica gel column chromatography (developing solvent: hexane/ethyl acetate = 6) to give the title compound (3.08 g) as pale brown oily compound.
¹H-NMR (400MHz, CDCl₃) 7.05(s, 1H), 6.73(s, 1H), 4.07(t. J = 6.5 Hz, 2H), 3.83(s, 3H), 3.55(t, J = 6.1 Hz, 2H), 3.35(s, 3H), 2.32(s, 3H), 2.12-2.05(m, 2H).

### Reference Example 100

### 3-Methoxy-5-(3-methoxypropoxy)-2-methylbenzaldehyde

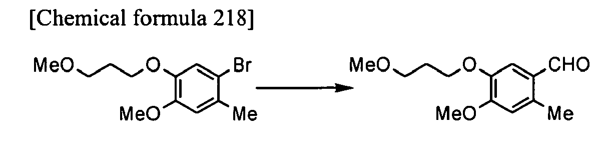

The compound of Reference Example 99 (3.08 g) was dissolved in tetrahydrofuran (30 ml), and thereto was added butyllithium (7.3 ml, 1.6M in Hexane) at -78°C. Thirty minutes thereafter, to the mixture was added N,N-dimethylformamide (1.24 ml), and warmed gradually to room temperature, and then stirred for 12 hours. To the reaction mixture was added 1M hydrochloric acid, and the mixture was extracted with diethyl ether. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 7/2) to give the title compound (1.91 g) as a colorless oily compound.
¹H-NMR (400MHz, CDCl₃) 10.19(s, 1H), 7.37(s, 1H), 6.69(s, 1H), 4.15(t. J = 6.6 Hz, 2H), 3.93(s, 3H), 3.57(t, J = 6.1 Hz, 2H), 3.36(s, 3H), 2.63(s, 3H), 2.13-2.08(m, 2H).

### Reference Example 101

### 4-Methoxy-5-(3-methoxypropoxy)-2-methylbenzoic acid

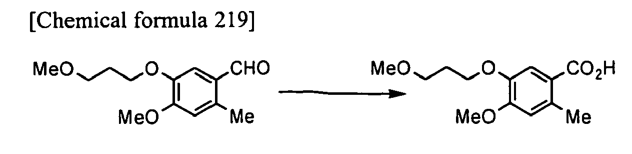

The compound of Reference Example 100 (1.62 g) was dissolved in t-butanol (60 ml), dichloromethane (20 ml) and water (60 ml), and thereto were added sodium dihydrogen phosphate dihydrate (6.80 g), 2-methyl-2-butene (8.66 ml) and sodium chlorite (6.04 g), and the mixture was stirred at room temperature for 5 hours. To the reaction mixture was added 1M hydrochloric acid, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: chloroform/methanol = 10/1) to give the title compound (801 mg) as a white solid.
¹H-NMR (400MHz, CDCl₃) 7.65(s, 1H), 6.71(s, 1H), 4.15(t. J = 6.5 Hz, 2H), 3.92(s, 3H), 3.58(t, J = 6.2 Hz, 2H), 3.37(s, 3H), 2.62(s, 3H), 2.15-2.09(m, 2H).

### Reference Example 102

### 4-Methoxy-5-(3-methoxypropoxy)-2-methylbenzoyl chloride

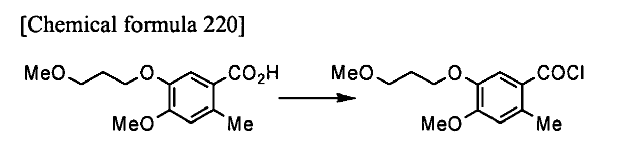

The title compound (320 mg) was synthesized in a similar manner to Reference Example 4.

### Reference Example 103

### tert-Butyl (rac.)-(3R,5S)-3-{isopropyl[4-methoxy-3-(3-methoxypropoxy)benzoyl]amino}-5-(phenoxymethyl)piperidine-1-carboxylate

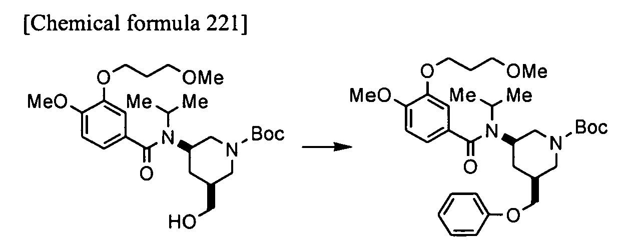

The compound of Reference Example 95 (49 mg) was dissolved in tetrahydrofuran (2 ml), and thereto were added phenol (19 mg), triphenylphosphine (52 mg) and a 1.9 M solution of diisopropyl azocarboxylate in toluene (105 ul), and the mixture was stirred at room temperature for 24 hours. To the reaction mixture was added water, and the mixture was extracted with ethyl acetate. The organic layer was washed with a saturated aqueous sodium carbonate solution and a saturated aqueous sodium chloride solution, dried over sodium sulfate, filtered and concentrated under reduced pressure. The obtained residue was purified by preparative thin layer silica gel column chromatography (developing solvent: hexane/ethyl acetate = 1/1) to give the title compound (47 mg) as a colorless oily compound.
MS (ESI+) 571 (M⁺+1, 45 %).

### Reference Example 104

### tert-Butyl (rac.)-(3S,5R)-3-[(benzyloxy)methyl]-5-{isopropyl[4-methoxy-3-(3-methoxypropoxy)benzoyl]amino}piperidine-1-carboxylate

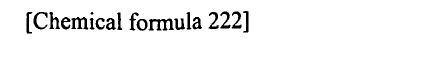

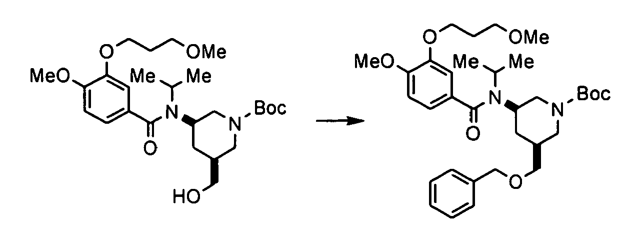

The compound of Reference Example 95 (49 mg) was dissolved in tetrahydrofuran (2 ml), and thereto was added sodium hydride (13 mg), and the mixture was heated with stirring at 50°C for 30 minutes. To the mixture was added benzyl chloride (43 mg), and the mixture was stirred at 50°C for 5 hours. To the reaction mixture was added a 5 % aqueous potassium hydrogen sulfate solution, and the mixture was extracted with ethyl acetate. The organic layer was washed with a saturated aqueous sodium chloride solution, dried over sodium sulfate, filtered and concentrated under reduced pressure. The obtained residue was purified by preparative thin layer silica gel column chromatography (developing solvent: hexane/ethyl acetate = 1/1) to give the title compound (38 mg) as a colorless oily compound.
MS (ESI+) 585 (M⁺+1, 41 %).

### Reference Example 105

### tert-Butyl (rac.)-(3R,5S)-3-{isopropyl[4-methoxy-3-(3-methoxypropoxy)benzoyl]amino}-5-[({[(2-phenylethyl)amino]carbonyl}oxy)methyl]piperidine-1-carboxylate

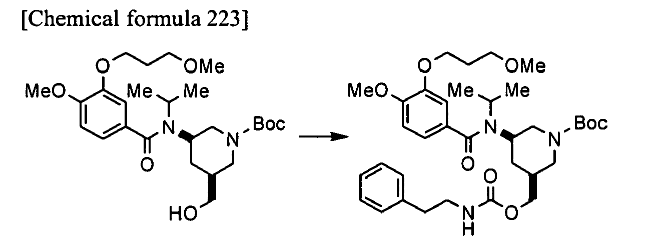

The compound of Reference Example 95 (49 mg) was dissolved in dioxane (0.5 ml), and thereto was added phenethyl isocyanate (145 mg), and the mixture was heated with stirring at 80°C for 15 hours. Water was added to the reaction mixture, and the mixture was extracted with ethyl acetate. The organic layer was washed with a saturated aqueous sodium chloride solution, dried over sodium sulfate, filtered and concentrated under reduced pressure. The obtained residue was purified by preparative thin layer silica gel column chromatography (developing solvent: hexane/ethyl acetate = 1/3) to give the title compound (41.3 mg) as a colorless oily compound.
MS (ESI+) 642 (M⁺+1, 33 %).

### Reference Example 106

### tert-Butyl (rac.)-(3R,5S)-3-{isopropyl[4-methoxy-3-(3-methoxypropoxy)-2-methylbenzoyl]amino}piperidine-1-carboxylate

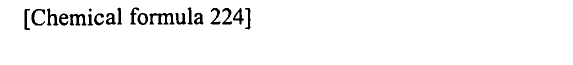

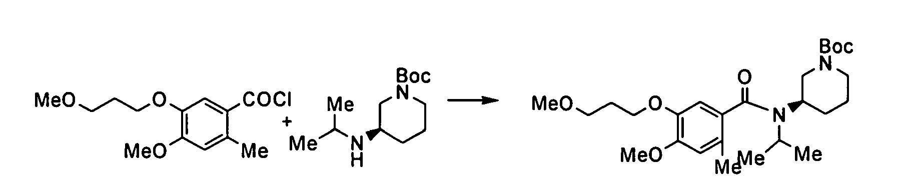

The title compound (483 mg) was synthesized in a similar manner to Reference Example 8 except that the compound of Reference Example 102 and the compound of Reference Example 35 were used instead of the compound of Reference Example 4 and the compound of Reference Example 7, respectively, in Reference Example 8.
MS (ESI+) 479 (M⁺+1, 100 %).

### Reference Examples 107-109

The compounds (20.7 mg) as listed in the following Table were synthesized in a similar manner to Reference Example 61.

**[Table 2]**

| Ref. Ex. | Structure | Yield (mg) | MS (ESI+) |
|---|---|---|---|
| 107 | 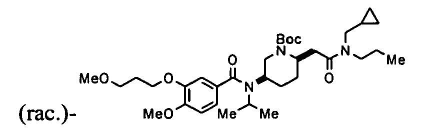 | 20.7 | 618 (M+1, 100%) |
| 108 | 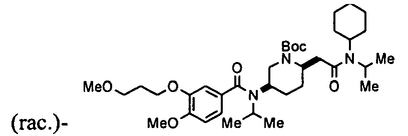 | 9.6 | 646 (M+1, 100%) |
| 109 | 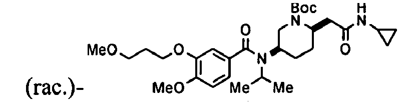 | 35.7 | 562 (M+1, 100%) |

### Reference Example 110

### tert-Butyl 3-(cyclopropylamino)piperidine-1-carboxylate

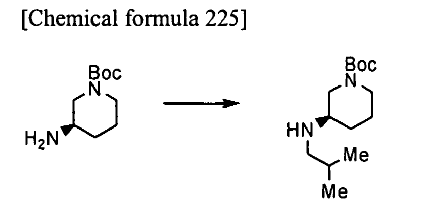

The title compound (73.8 mg) was synthesized in a similar manner to Reference Example 54.
MS (ESI+) 257 (M+1, 100 %).

### Reference Example 111

### tert-Butyl (3R)-3-{isobutyl[4-methoxy-3-(3-methoxypropoxy)benzoyl]amino}piperidine-1-carboxylate

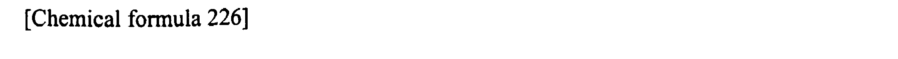

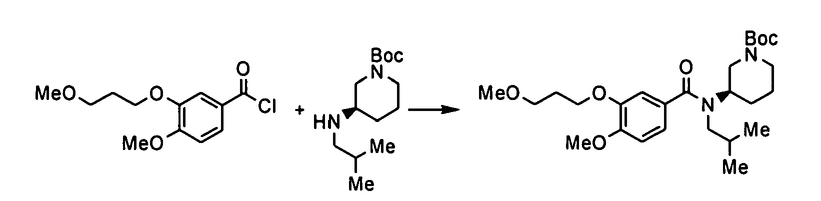

The title compound (108 mg) was synthesized in a similar manner to Reference Example 8.
MS (ESI+) 479 (M+1, 100%).

### Reference Example 112

### 2-Chloro-5-hydroxy-4-methoxybenzaldehyde

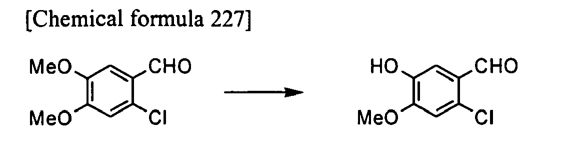

To 4,5-dimethoxy-2-chlorobenzaldehyde was added conc. sulfuric acid (23 ml), and the mixture was stirred at 65°C for 6 hours. The reaction mixture was added to an iced-water where sodium hydroxide (38 g) was dissolved, and the aqueous layer was washed twice with ethyl acetate. To the aqueous layer, which was cooled to 0°C, was added conc. hydrochloric acid so that the pH value thereof was adjusted to pH 1, and the mixture was extracted with ethyl acetate. The combined organic layers were dried over sodium sulfate, filtered and concentrated under reduced pressure. The obtained residue was purified by silica gel column chromatography (developing solvent: hexane/ethyl acetate = 2) to give the title compound (2.19 g) as a white solid.
¹H-NMR (400MHz, CDCl₃) 10.31(s, 1H), 7.47(s, 1H), 6.88(s, 1H), 5.67(brs, 1H), 3.99(s, 3H).

### Reference Example 113

### 2-Chloro-4-methoxy-5-(3-methoxypropoxy)benzaldehyde

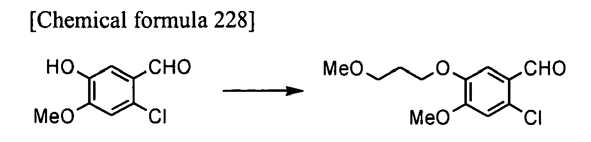

The title compound (2.73 g) was synthesized in a similar manner to Reference Example 2.
¹H-NMR (400MHz, CDCl₃) 10.31(s, 1H), 7.42(s, 1H), 6.87(s, 1H), 4.15(t, J= 6.6 Hz, 2H), 3.94(s, 3H), 3.55(t, J = 6.1 Hz, 2H), 3.35(s, 3H), 2.14-2.09(m, 2H).

### Reference Example 114

### 2-Chloro-4-methoxy-5-(3-methoxypropoxy)benzoic acid

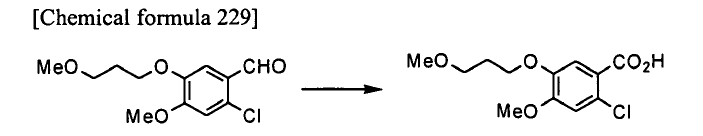

The title compound (495 mg) was synthesized in a similar manner to Reference Example 103.
¹H-NMR (400MHz, CDCl₃) 7.58(s, 1H), 6.90(s, 1H), 4.13(t, J= 6.4 Hz, 2H), 3.91(s, 3H), 3.57(t, J = 6.1 Hz, 2H), 3.36(s, 3H), 2.12-2.09(m, 2H).

### Reference Example 115

### 2-Chloro-4-methoxy-5-(3-methoxypropoxy)benzoyl chloride

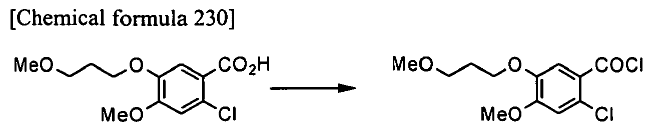

The title compound (190 mg) was synthesized in a similar manner to Reference Example 4.

### Reference Example 116

### tert-Butyl (3R)-3-[[2-chloro-4-methoxy-5-(3-methoxypropoxy)benzoyl](isopropyl)amino]piperidine-1-carboxylate

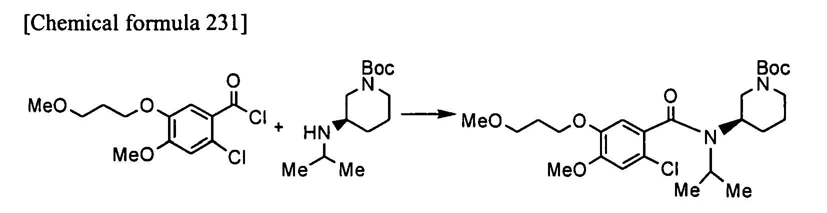

The title compound (317 mg) was synthesized in a similar manner to Reference Example 8.
MS (ESI+) 499 (M+1, 100 %).

### Reference Example 117

### tert-Butyl (rac.)-(2R,5R)-2-[2-(isopropylamino)ethyl]-5-{isopropyl[4-methoxy-3-(3-methoxypropoxy)benzoyl]amino}piperidine-1-carboxylate

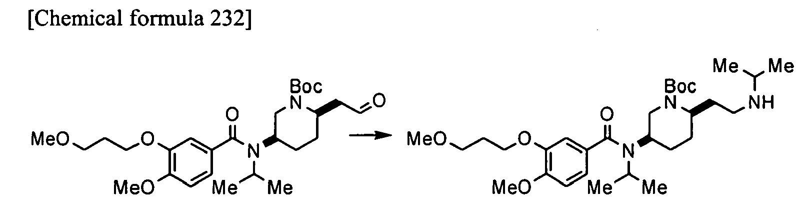

The title compound (11.7 mg) was synthesized in a similar manner to Reference Example 46.
MS (ESI+) 550 (M+1, 100 %).

### Reference Examples 118-119

The compounds (3.2 mg) as listed in the following Table were synthesized in a similar manner to Reference Example 88.

**[Table 3]**

| Ref. No. | Structure | Yield (mg) | MS (ESI) |
|---|---|---|---|
| 118 | 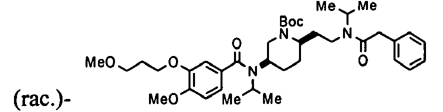 | 3.2 | 668 (M+1, 100%) |
| 119 | 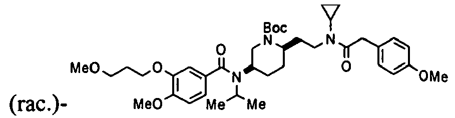 | 4.2 | 696 (M+1, 100%) |

### Reference Examples 120-124

The title compound (24.7 mg) was synthesized in a similar manner to Reference Example 103.

**[Table 4]**

| Ref. No. | Structure | Yield (mg) | MS (ESI+) |
|---|---|---|---|
| 120 | 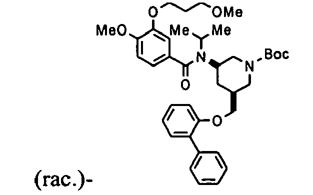 | 24.7 | 647(M⁺+1, 45%) |
| 121 | 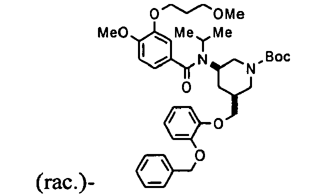 | 42.3 | 677(M⁺+1, 40%) |
| 122 | 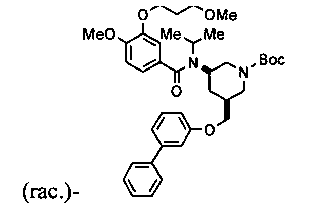 | 39.0 | 647(M⁺+1, 36%) |
| 123 | 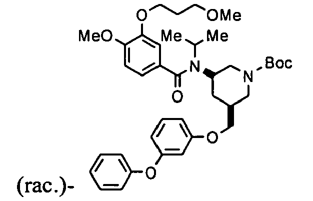 | 44.5 | 663(M⁺+1, 29%) |
| 124 | 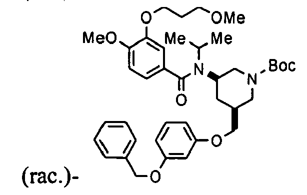 | 58.1 | 677(M⁺+1, 36%) |

### Reference Example 125

### tert-Butyl (rac.)-(3S,5R)-3-({[(isopropylamino)carbonyl]oxy}methyl)-5-{isopropyl[4-methoxy-3-(3-methoxypropoxy)benzoyl]ammo}piperidme-1-carboxylate

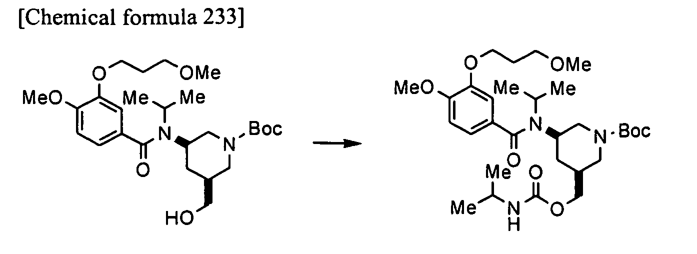

The title compound (176.6 mg) was synthesized in a similar manner to Reference Example 105.
MS (ESI+) 580 (M⁺+1, 100 %).

### Reference Example 126

### tert-Butyl (rac.)-(3S,5R)-3-[({[ethyl(2-phenylethyl)amino]carbonyl}oxy)methyl]-5-{isopropyl[4-methoxy-3-(3-methoxypropoxy)benzoyl]amino}piperidine-1-carboxylate

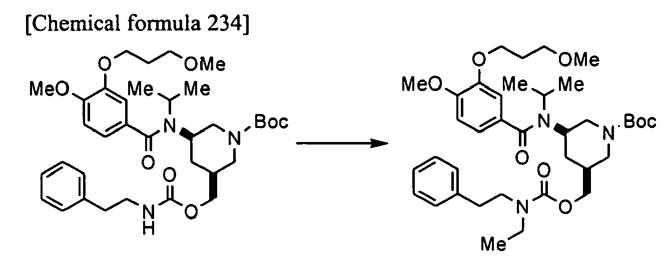

The compound of Reference Example 105 (54 mg) was dissolved in dimethylformamide (2 ml), and thereto was added sodium hydroxide (purity: 55 %, 11 mg), and the mixture was stirred at 40°C for 30 minutes. Then, to the mixture was added ethyl iodide (39 mg), and the mixture was stirred at room temperature overnight. To the reaction mixture was added a 5 % aqueous potassium hydrogen sulfate solution, and the mixture was extracted with ethyl acetate. The organic layer was dried over sodium sulfate, filtered and concentrated under reduced pressure. The obtained residue was purified by preparative thin layer silica gel column chromatography (developing solvent: hexane/ethyl acetate = 1/3) to give the title compound (15.1 mg) as a colorless oily compound.
MS (ESI+) 670 (M⁺+1, 25 %).

### Reference Examples 127-128

The compounds (12.6 mg) as listed in the following Table were synthesized in a similar manner to Reference Example 126.

**[Table 5]**

| Ref. No. | Structure | Yield (mg) | MS (ESI+) |
|---|---|---|---|
| 127 | 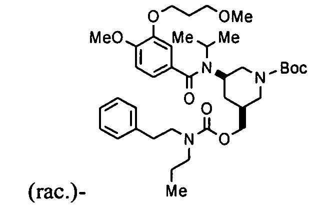 | 12.6 | 684(M⁺+1, 25%) |
| 128 | 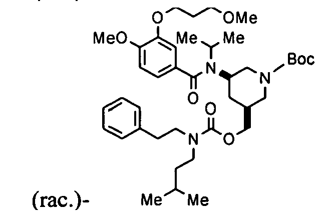 | 11.1 | 712(M⁺+1, 32%) |

### Reference Example 129

### tert-Butyl (rac.)-(3R,5R)-3-{isopropyl[4-methoxy-3-(3-methoxypropoxy)benzoyl]amino}-5-{[(3-methylbutanoyl)amino]methyl}piperidine-1-carboxylate

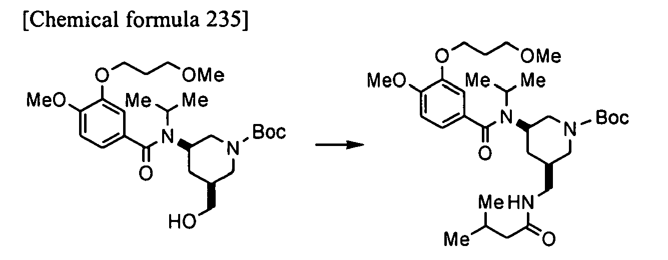

The compound of Reference Example 95 (1.042 g) was dissolved in tetrahydrofuran (20 ml), and thereto were added phthalimide (0.37 g), triphenylphosphine (0.66 g) and a 1.9M solution of diisopropyl azocarboxylate in toluene (1.33 ml), and the mixture was stirred at room temperature for 48 hours. To the reaction mixture was added water, and the mixture was extracted with ethyl acetate. The organic layer was washed with a saturated aqueous sodium carbonate solution and a saturated aqueous sodium chloride solution, dried over sodium sulfate, filtered and concentrated under reduced pressure. The obtained residue was purified by silica gel column chromatography (developing solvent: hexane/ethyl acetate = 1/2) to give a colorless oily compound (1.269 g).
Subsequently, said colorless oily compound (0.59 g) was dissolved in ethanol (10 ml), and thereto was added hydrazine monohydrate (459 µl), and the mixture was heated with stirring at 50°C for 3 hours. Ethanol was removed by evaporation, and diluted with diethyl ether. The insoluble white solid was removed by filtration, and the residue was concentrated. To the resultant was added a 10 % aqueous potassium carbonate solution, and the mixture was extracted with ethyl acetate. The organic layer was dried over sodium sulfate, filtered and concentrated under reduced pressure to give a crude amine product (657 mg).
Said crude amine product (90 mg) was dissolved in tetrahydrofuran (2 ml), and thereto was added triethylamine (51 µl), and further thereto was added dropwise isovaleric chloride (33 mg). The mixture was stirred at room temperature overnight. To the reaction mixture was added a 5 % aqueous potassium hydrogen sulfate solution, and the mixture was extracted with ethyl acetate. The organic layer was dried over sodium sulfate, filtered and concentrated under reduced pressure. The obtained residue was purified by preparative thin layer silica gel column chromatography (developing solvent: ethyl acetate) to give the title compound (54.8 mg) as a colorless oily compound.
MS (ESI+) 578 (M⁺+1, 20%).

### Reference Examples 130-131

The compounds (76.5 mg) as listed in the following Table were synthesized in a similar manner to Reference Example 129.

**[Table 6]**

| Ref. No. | Structure | Yield (mg) | MS (ESI⁺) |
|---|---|---|---|
| 130 | 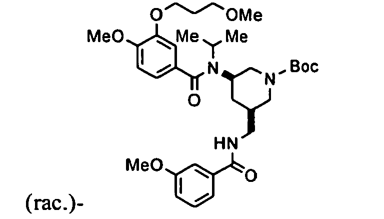 | 76.5 | 628(M⁺+1, 100%) |
| 131 | 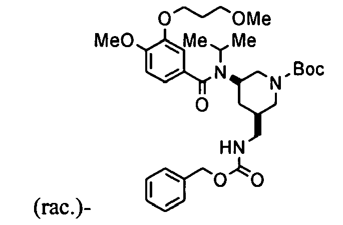 | 43.2 | 628 (M⁺+1, 19%) |

### Reference Example 132

### tert-Butyl [(rac.)-(3R,4R)-1-benzyl-4-phenylpiperidin-3-yl]carbonate

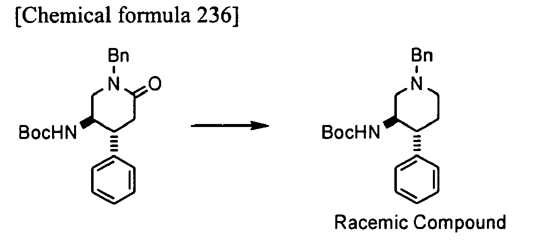

The title compound (170 mg) was synthesized in a similar manner to Reference Example 18.
MS (ESI+) 367 (M+1, 100%).

### Reference Example 133

### (rac.)-(3R,4R)-1-Benzyl-4-phenylpiperidine-3-amine dihydrochloride

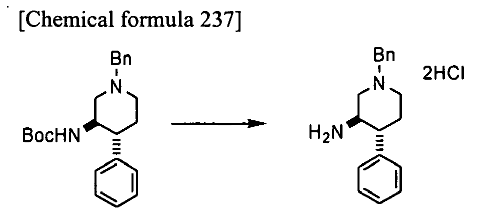

The title compound (156 mg) was synthesized in a similar manner to Reference Example 10.
MS (ESI+) 267 (M+1, 100 %).

### Reference Example 134

### (rac.)-(3R,4R)-1-Benzyl-N-isopropyl-4-phenylpiperidine-3-amine

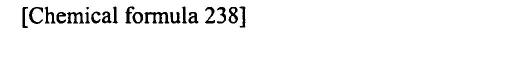

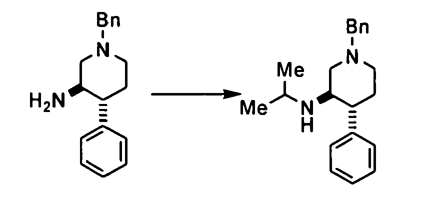

The title compound (137 mg) was synthesized in a similar manner to Reference Example 15.
MS (ESI+) 309 (M+1, 100 %).

### Reference Example 135

### N-[(rac.)-(3R,4R)-1-Benzyl-4-phenylpiperidin-3-yl]-N-isopropyl-4-methoxy-3-(3-methoxypropoxy)benzamide

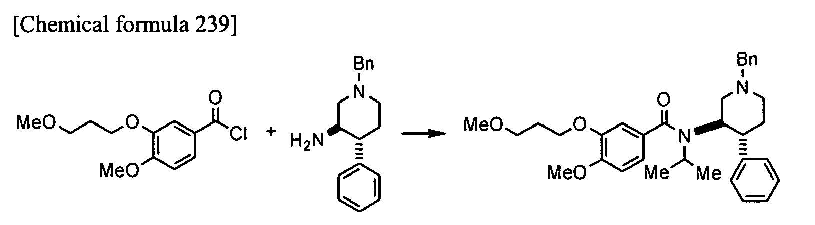

The title compound (174 mg) was synthesized in a similar manner to Reference Example 8.
MS (ESI+) 531 (M+1, 100 %).

### Reference Example 136

### tert-Butyl (2R,5R)-2-{2-[cyclopropyl(2-methoxyacetyl)amino]ethyl}-5-{isopropyl[4-methoxy-3-(3-methoxypropoxy)benzoyl]amino}piperidine-1-carboxylate

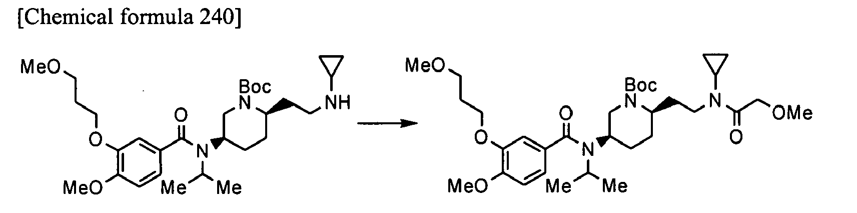

The title compound (60 mg) was synthesized in a similar manner to Reference Example 61 except that methoxyacetic acid and tert-butyl-(2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 620 (M+1, 100 %)

### Reference Example 137

### tert-Butyl (2R,5R)-2-{2-[cyclopropyl(3-methoxypropanoyl)amino]ethyl}-5-{isopropyl[4-methoxy-3-(3-methoxypropoxy)benzoyl]amino}piperidine-1-carboxylate

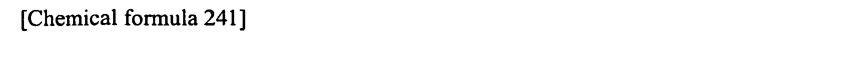

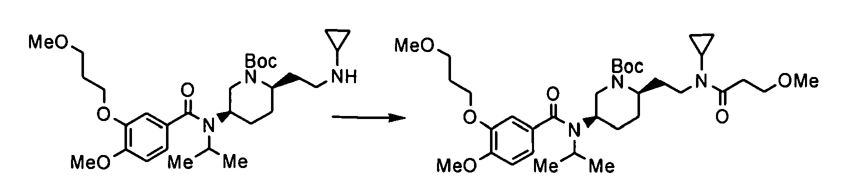

The title compound (16 mg) was synthesized in a similar manner to Reference Example 61 except that 3-methoxypropionic acid and tert-butyl (2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 634 (M+1, 100 %)

### Reference Example 138

### tert-Butyl (2R,5R)-2-{2-[cyclopropyl(4-methoxybutanoyl)amino]ethyl}-5-{isopropyl[4-methoxy-3-(3-methoxypropoxy)benzoyl]amino}piperidine-1-carboxylate

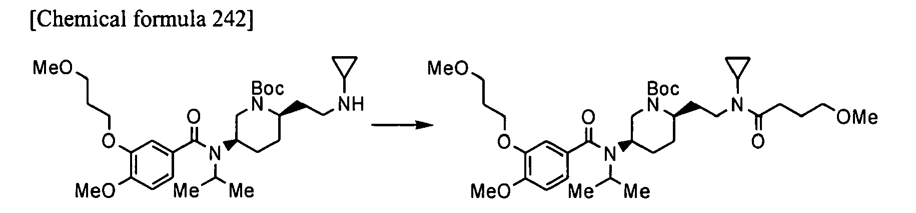

The title compound (35 mg) was synthesized in a similar manner to Reference Example 61 except that 3-methoxybutanoic acid and tert-butyl (2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 648 (M+1, 100 %)

### Reference Example 139

### tert-Butyl (2R,5R)-2-{2-[[4-(benzoyloxy)butanoyl](cyclopropyl)amino]ethyl}-5-{isopropyl[4-methoxy-3-(3-methoxypropoxy)benzoyl]amino}piperidine-1-carboxylate

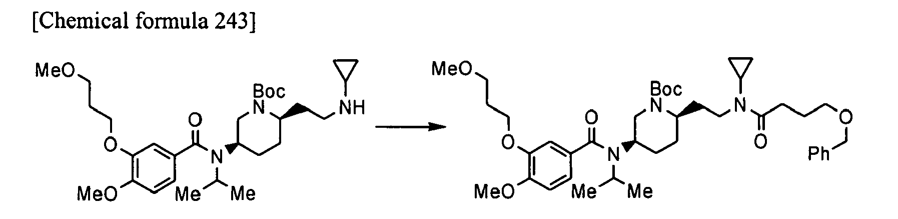

The title compound (73 mg) was synthesized in a similar manner to Reference Example 61 except that 4-benzyloxybutanoic acid and tert-butyl-(2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 724 (M+1, 100 %)

### Reference Example 140

### tert-Butyl (2R,5R)-2-{2-[cyclopropyl(4-hydroxybutanoyl)amino]ethyl}-5-{isopropyl[4-methoxy-3-(3-methoxypropoxy)benzoyl]amino}piperidine-1-carboxylate

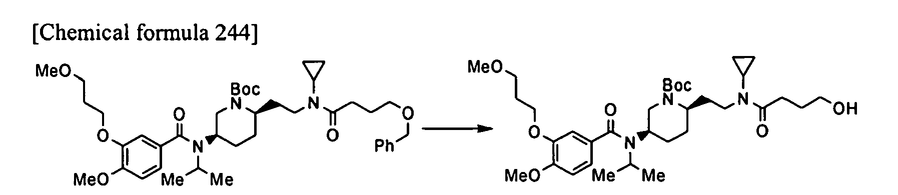

The compound obtained in Reference Example 139 (72 mg) was dissolved in methanol (1 ml), and thereto was added a 10% Pd/C (50 % wet, 100 mg), and the mixture was stirred at 25°C under hydrogen atmosphere for 3 hours. After replacement with nitrogen gas, the reaction solution was filtered through celite, and the filtrate was concentrated under reduced pressure to give the title compound (63 mg) as a colorless oily product.
MS (ESI+) 634 (M+1, 100 %)

### Reference Example 141

### tert-Butyl (2R,5R)-2-{2-[cyclopropyl(3-hydroxy-3-methylbutanoyl)amino]ethyl}-5-{isopropyl[4-methoxy-3-(3-methoxypropoxy)benzoyl]amino}piperidine-1-carboxylate

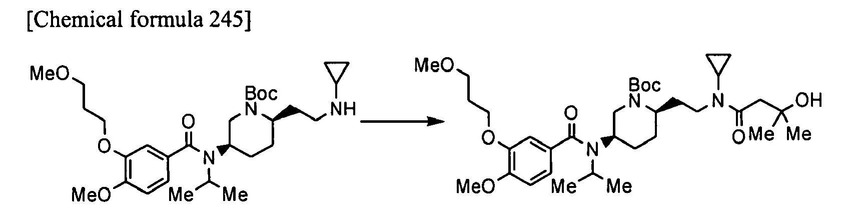

The title compound (40 mg) was synthesized in a similar manner to Reference Example 61 except that b-hydroxyisovaleric acid and tert-butyl-(2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 648 (M+1, 100%)

### Reference Example 142

### tert-Butyl (2R,5R)-2-{2-[cyclopropyl(3-methylbut-2-enoyl)amino]ethyl}-5-{isopropyl[4-methoxy-3-(3-methoxypropoxy)benzoyl]amino}piperidine-1-carboxylate

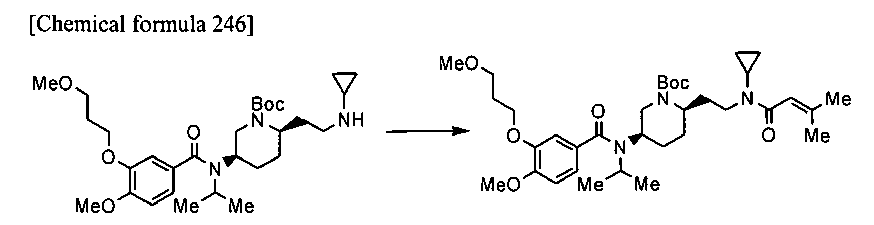

The title compound (18 mg) was synthesized in a similar manner to Reference Example 61 except that 3,3-dimethylacrylic acid and tert-butyl (2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 630 (M+1, 100 %)

### Reference Example 143

### (2R,5R)-2-{2-[(3-Cyano-3-methylbutanoyl)(cyclopropyl)amino]ethyl}-5-{isopropyl[4-methoxy-3-(3-methoxypropoxy)benzoyl]amino}piperidine-1-carboxylic acid tert-butyl ester

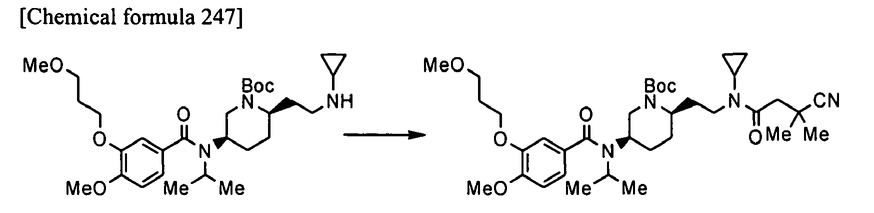

The title compound (50 mg) was synthesized in a similar manner to Reference Example 61 except that 3-cyano-3-methylbutanoic acid and tert-butyl (2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 657 (M+1, 100 %)

### Reference Example 144

### tert-Butyl (2R,5R)-2-{2-[(cyclohexylacetyl)(cyclopropyl)amino]ethyl}-5-{isopropyl[4-methoxy-3-(3-methoxypropoxy)benzoyl]amino}piperidine-1-carboxylate

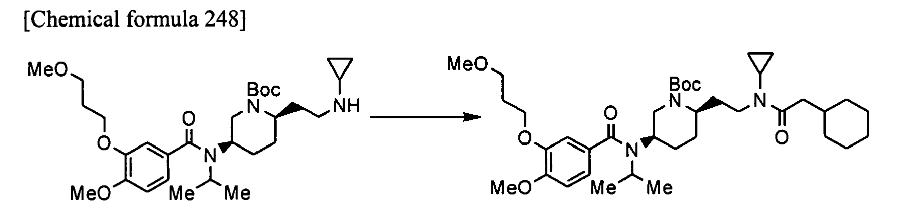

The title compound (30 mg) was synthesized in a similar manner to Reference Example 61 except that cyclohexylacetic acid and tert-butyl (2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic amide, respectively.
MS (ESI+) 672 (M+1, 100 %)

### Reference Example 145

### tert-Butyl (2R,5R)-2-{2-[cyclopropyl(4-ethoxy-4-oxobutanoyl)amino]ethyl}-5-{isopropyl[4-methoxy-3-(3-methoxypropoxy)benzoyl]amino}piperidine-1-carboxylate

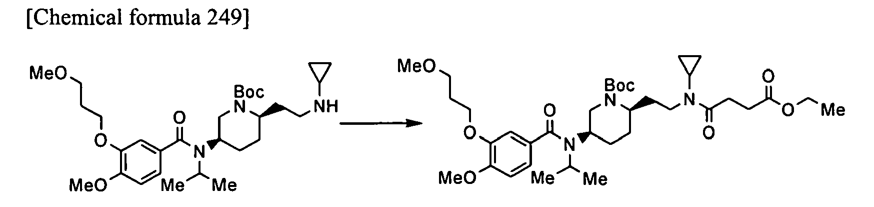

The title compound (25 mg) was synthesized in a similar manner to Reference Example 61 except that monoethylsuccinic acid and tert-butyl (2S,5R)-2-[2-(cyclopropylamino)ethyl]-5-{isopropyl[4-methoxy-3-(3-methoxypropoxy)benzoyl]amino}piperidine-1-carboxylate were used instead of the compound of Reference Example 48 and isopecotic acid, respectively.
MS (ESI+) 676 (M+1, 100 %)

### Reference Example 146

### 4-[[2-((2R,5R)-1-(tert-Butoxycarbonyl)-5-{isopropyl[4-methoxy-3-(3-methoxypropoxy)-benzoyl]amino}piperidin-2-yl)ethyl](cyclopropyl)amino]-4-oxobutanoic acid

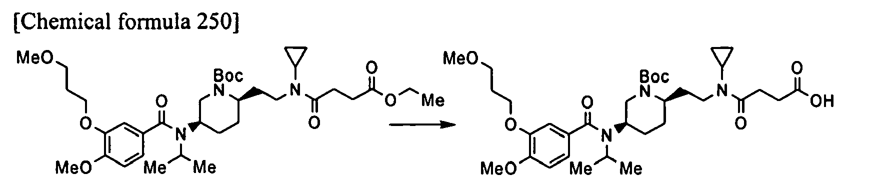

The compound obtained in Reference Example 145 (25 mg) was dissolved in ethanol (1 ml), and thereto was added sodium hydroxide (1 ml, 2M aqueous solution), and the mixture was stirred at 25°C for 10 hours. The reaction solution was washed with diethyl ether, and the aqueous layer was acidified with hydrochloric acid, and the mixture was extracted with diethyl ether. The organic layer was washed with a saturated aqueous sodium chloride solution, dried over sodium sulfate, filtered, and concentrated under reduced pressure to give the title compound (22 mg) as colorless liquid.
MS (ESI+) 648 (M+1, 100 %)

### Reference Example 147

### tert-Butyl (2R,5R)-2-{2-[(anilinocarbonyl)(cyclopropyl)amino]ethyl}-5-{isopropyl[4-methoxy-3-(3-methoxypropoxy)benzoyl]amino}piperidine-1-carboxylate

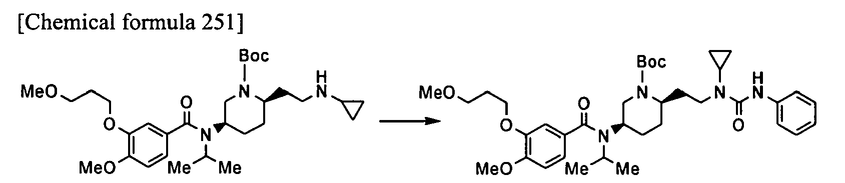

tert-Butyl-(2S,5R)-2-[2-(cyclopropylamino)ethyl]-5-{isopropyl[4-methoxy-3-(3-methoxypropoxy)benzoyl]amino}piperidine-1-carboxylate (68.0 mg) was dissolved in dichloromethane (0.6 ml), and thereto was added phenylisocyanate (0.027 ml). The reaction mixture was stirred at room temperature for 21 hours, and thereto was added aqueous ammonia (1.0 ml), and the mixture was stirred for 20 minutes. Water was added to the reaction solution, and the mixture was extracted with ethyl acetate. The organic layer was washed with a saturated aqueous sodium hydrogen carbonate solution, water and a saturated aqueous sodium chloride solution, dried over sodium sulfate, filtered and concentrated under reduced pressure. The obtained residue was purified by thin layer silica gel chromatography (developing solvent: hexane/ethyl acetate = 1/3) to give the title compound (62.7 mg) as a colorless oily compound.
¹H-NMR (400MHz, CDCl₃) 7.43-7.42(m, 2H), 7.29-7.27(m, 3H), 7.04-6.98(m, 1H), 6.90-6.85(m, 3H), 4.33-4.16(m, 1H), 4.13-4.09(m, 2H), 3.98-3.95(m, 2H), 3.87(s, 3H), 3.75-3.66(m, 1H), 3.56(t, J=6.1 Hz, 2H), 3.42-3.36(m, 1H), 3.34(s, 3H), 3.20-3.00(m, 2H), 2.77-2.66(m, 1H), 2.13-2.04(m, 2H), 1.74-1.55(m, 6H), 1.46(s, 9H), 1.27-1.17(m, 6H), 0.97-0.82(m, 4H).
MS (ESI+) 667 (M+1, 85%).

### Reference Example 148

### tert-Butyl (2R,5R)-2-{2-[(benzylsulfonyl)(cyclopropyl)amino]ethyl}-5-{isopropyl[4-methoxy-3-(3-methoxypropoxy)benzoyl]amino}piperidine-1-carboxylate

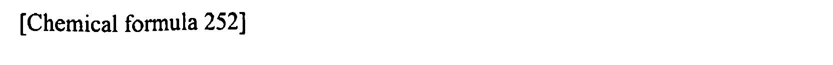

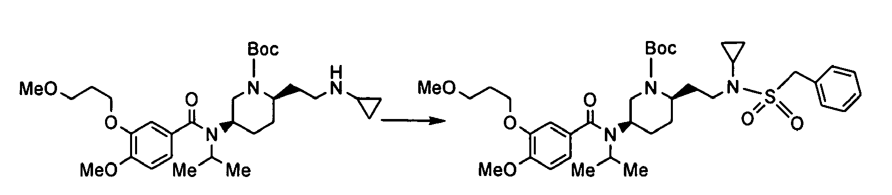

tert-Butyl (2S,5R)-2-[2-(cyclopropylamino)ethyl]-5-{isopropyl[4-methoxy-3-(3-methoxypropoxy)benzoyl]amino}piperidine-1-carboxylate (50.0 mg) was dissolved in dichloromethane (0.5 ml), and thereto were added α-toluenesulfonyl chloride (19.1 mg) and triethylamine (0.025 ml), and the mixture was stirred at room temperature for 3 hours. To the reaction solution was added methanol (0.1 ml), and the mixture was stirred for 20 minutes. To the reaction solution was added a saturated aqueous sodium hydrogen carbonate solution, and the mixture was extracted with ethyl acetate. The organic layer was washed with a saturated aqueous sodium hydrogen carbonate solution, water and a saturated aqueous sodium chloride solution, dried over sodium sulfate, filtered and concentrated under reduced pressure. The obtained residue was purified by thin layer silica gel chromatography (developing solvent: hexane/ethyl acetate = 2/3) to give the title compound (33.4 mg) as a colorless oily compound.
¹H-NMR (400MHz, CDCl₃) 7.38-7.33(m, 5H), 6.87-6.84(m, 3H), 4.25(s, 2H), 4.25-4.15(m, 1H), 4.10(t, J=6.5 Hz, 2H), 3.98-3.95(m, 1H), 3.87(s, 3H), 3.85-3.78(m, 1H), 3.56(t, J=6.1 Hz, 2H), 3.34(s, 3H), 3.20-3.00(m, 2H), 2.80-2.82(m, 1H), 2.49-2.40(m, 1H), 2.25-2.20(m, 1H), 2.11-2.04(m, 2H), 1.65-1.55(m, 6H), 1.46(s, 9H), 1.27-1.17(m, 6H), 0.73-0.70(m, 4H).
MS (ESI+) 702 (M+1, 50 %).

### Reference Example 149

### tert-Butyl (2R,5R)-2-{2-[cyclopropyl(ethoxycarbonyl)amino]ethyl}-5-{isopropyl[4-methoxy-3-(3-methoxypropoxy)benzoyl]amino}piperidine-1-carboxylate

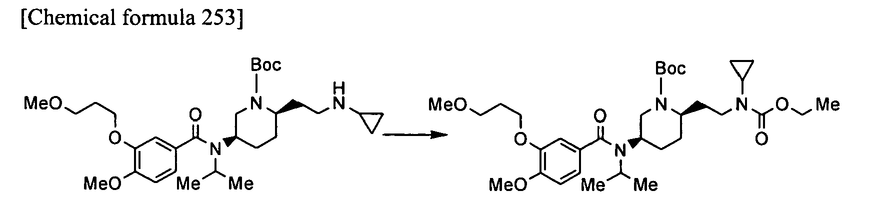

tert-Butyl-(2S,5R)-2-[2-(cyclopropylamino)ethyl]-5-{isopropyl[4-methoxy-3-(3-methoxypropoxy)benzoyl]amino}piperidine-1-carboxylate (50.0 mg) was dissolved in dichloromethane (0.5 ml), and thereto were added ethyl chloroformate (0.009 ml) and triethylamine(0.025 ml), and the mixture was stirred at room temperature for 5 hours. To the reaction solution was added methanol (0.1 ml), and the mixture was stirred for 20 minutes. To the reaction solution was added a saturated aqueous sodium hydrogen carbonate solution, and the mixture was extracted with ethyl acetate. The organic layer was washed with a saturated aqueous sodium hydrogen carbonate solution, water, and a saturated aqueous sodium chloride solution, dried over sodium sulfate, filtered and concentrated under reduced pressure. The obtained residue was purified by thin layer silica gel chromatography (developing solvent: hexane/ethyl acetate = 1/4) to give the title compound (33.7 mg) as a colorless oily compound.
¹H-NMR (400MHz, CDCl₃) 6.89-6.84(m, 3H), 4.25-4.22(m, 1H), 4.14-4.09(m, 4H), 4.00-3.90(m, 2H), 3.87(s, 3H), 3.85-3.78(m, 1H), 3.56(t, J=6.1 Hz, 2H), 3.34(s, 3H), 3.25-3.20(m, 1H), 3.20-3.05(m, 2H), 2.70-2.60(m, 1H), 2.13-2.08(m, 3H), 1.80-1.65(m, 4H), 1.65-1.50(m, 1H), 1.46(s, 9H), 1.27-1.17(m, 9H), 0.75-0.59(m, 4H).
MS (ESI+) 620 (M+1, 45 %).

### Reference Example 150

### tert-Butyl (2R,5R)-2-(2-[{[3-(butoxycarbonyl)phenyl]acetyl)(cyclopropyl)amino]ethyl}-5-{isopropyl[4-methoxy-3-(3-methoxypropoxy)benzoyl]amino}piperidine-1-carboxylate

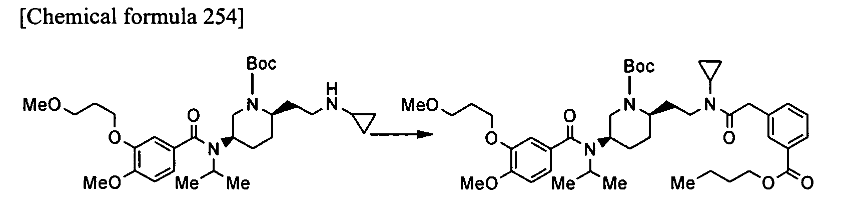

The title compound was obtained as a colorless oily compound in a similar manner to Reference Example 136 except that [3-(butoxycarbonyl)phenyl]acetic acid (WO 92/21672) was used.
¹H-NMR (400MHz, CDCl₃) 7.95-7.90(m, 2H), 7.51-7.47(m, 1H), 7.40-7.38(m, 1H), 6.89-6.85(m, 3H), 4.32-4.28(m, 2H), 4.11(t, J=6.5 Hz, 2H), 3.93-3.90(m, 4H), 3.87(s, 3H), 3.56(t, J=6.1 Hz, 2H), 3.34(s, 3H), 3.20-2.90(m, 3H), 2.75-2.65(m, 1H), 2.12-2.04(m, 4H), 1.75-1.65(m, 7H), 1.51-1.45(m, 12H), 1.25-1.15(m, 6H), 0.98-0.95(m, 7H).
MS (ESI+) 766 (M+1, 100 %).

### Reference Example 151

### tert-Butyl (2R,5R)-2-[2-(cyclopropyl{[3-(carboxyl)phenyl]acetyl}amino)ethyl]-5-{isopropyl[4-methoxy-3-(3-methoxypropoxy)benzoyl]amino}piperidine-1-carboxylate

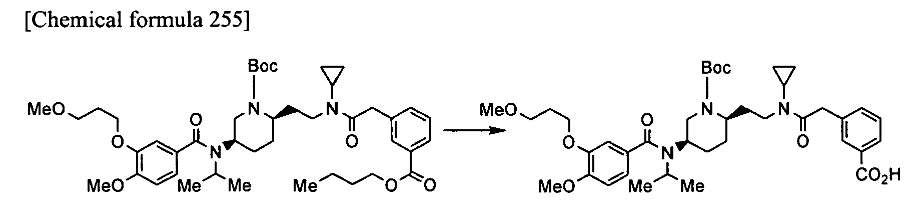

The compound of Reference Example 150 (265 mg) was dissolved in ethanol (150 ml), and thereto was added a 1N aqueous sodium hydroxide solution (132 ml), and the mixture was stirred at room temperature for 6 hours. Ethanol was removed by evaporation, and to the remaining residue was added a 5 % aqueous potassium hydrogen sulfate solution, and the mixture was extracted with ethyl acetate. The organic layer was washed with a saturated aqueous sodium chloride solution, dried over sodium sulfate, filtered and concentrated under reduced pressure to give the title compound (249 mg) as a colorless oily compound.
¹H-NMR (400MHz, CDCl₃) 7.93-7.90(m, 2H), 7.49-7.47(m, 1H), 7.40-7.36(m, 1H), 6.91-6.82(m, 3H), 4.14-4.08(m, 2H), 4.01-3.90(m, 4H), 3.87(s, 3H), 3.75-3.70(m, 2H), 3.56(t, J=6.1 Hz, 2H), 3.45-3.40(m, 1H), 3.34(s, 3H), 3.20-2.90(m, 3H), 2.75-2.65(m, 1H), 2.15-2.08(m, 3H), 1.85-1.65(m, 4H), 1.45(s, 9H), 1.25-1.15(m, 6H), 0.94-0.90(m, 4H).
MS (ESI+) 710 (M+1, 98 %).

### Reference Example 152

### tert-Butyl (2R,5R)-2-{2-[{[3-(aminocarbonyl)phenyl]acetyl}(cyclopropyl)amino]ethyl}-5-{isopropyl[4-methoxy-3-(3-methoxypropoxy)benzoyl]amino}piperidine-1-carboxylate

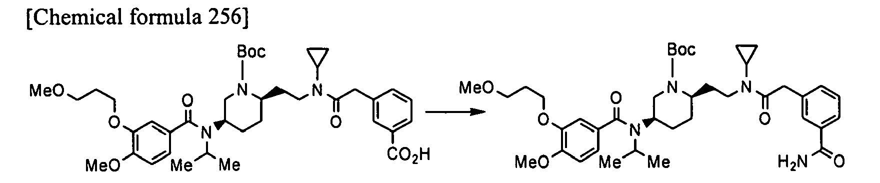

The title compound was obtained as a colorless oily compound in a similar manner to Reference Example 61.
¹H-NMR (400MHz, CDCl₃) 7.80-7.73(m, 2H), 7.54-7.51(m, 1H), 7.39-7.34(m, 2H), 6.87-6.84(m, 3H), 5.38-5.25(m, 1H), 4.19-3.80(m, 12H), 3.56(t, J=6.0 Hz, 2H), 3.45-3.40(m, 1H), 3.34(s, 3H), 3.20-2.80(m, 3H), 2.75-2.65(m, 1H), 2.15-2.08(m, 3H), 1.85-1.70(m, 3H), 1.45(s, 9H), 1.24-1.13(m, 6H), 0.87-0.79(m, 4H).
MS (ESI+) 709 (M+1, 100 %).

### Reference Example 153

### tert-Butyl (2R,5R)-2-[2-(cyclopropyl{[3-(ethoxycarbonyl)phenyl]acetyl}amino)ethyl]-5-{isopropyl[4-methoxy-3-(3-methoxypropoxy)benzoyl]amino}piperidine-1-carboxylate

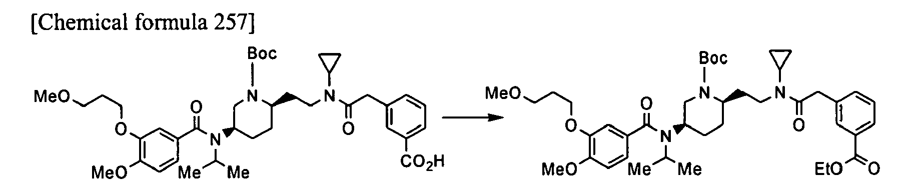

The title compound was obtained as a colorless oily compound in a similar manner to Reference Example 61.
¹H-NMR (400MHz, CDCl₃) 7.92-7.91 (m, 2H), 7.51-7.46(m, 1H), 7.39-7.36(m, 1H), 6.89-6.82(m, 3H), 4.35(q, J=7.1 Hz, 2H), 4.30-4.21(m, 1H), 4.11(t, J=6.4 Hz, 2H), 4.02-3.90(m, 4H), 3.87(s, 3H), 3.85-3.65(m, 2H), 3.55(t, J=6.1 Hz, 2H), 3.50-3.45(m, 1H), 3.34(s, 3H), 3.20-2.90(m, 2H), 2.75-2.65(m, 1H), 2.13-2.04(m, 3H), 1.75-1.65(m, 3H), 1.51-1.50(m, 1H), 1.44(s, 9H), 1.37(t, J=7.1 Hz, 3H), 1.22-1.15(m, 6H), 0.95-0.91(m, 4H).
MS (ESI+) 739 (M+1, 100 %).

### Reference Example 154

### tert-Butyl (rac.)-(2R,5R)-2-(2-{cyclopropyl[(4-fluorophenyl)acetyl]amino}ethyl)-5-{isopropyl[4-methoxy-3-(3-methoxypropoxy)benzoyl]amino}piperidine-1-carboxylate

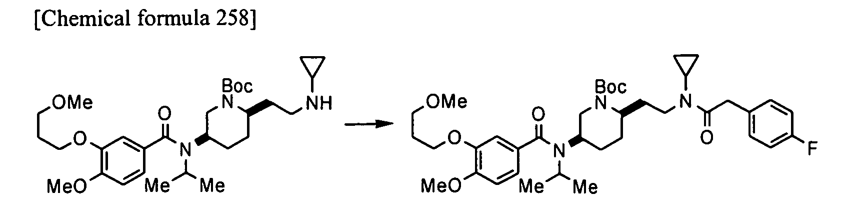

The title compound (15.7 mg) was synthesized in a similar manner to Reference Example 93.
MS (ESI+) 684 (M⁺+1, 100 %).

### Reference Example 155

### tert-Butyl (rac.)-(2R,5R)-2-{2-[benzoyl(cyclopropyl)amino]ethyl}-5-{isopropyl[4-methoxy-3-(3-methoxypropoxy)benzoyl]amino}piperidine-1-carboxylate

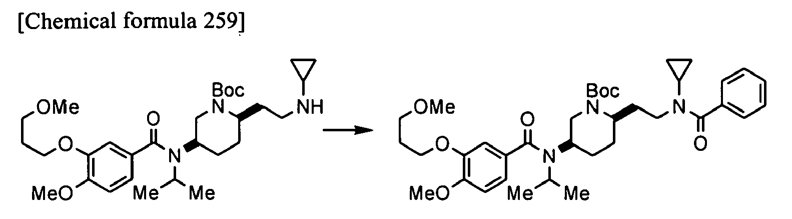

The title compound (3.8 mg) was synthesized in a similar manner to Reference Example 93.
MS (ESI+) 652 (M⁺+1, 100%).

### Reference Example 156

### tert-Butyl (rac.)-(2R,5R)-2-{2-[cyclopropyl(3-phenylpropanoyl)amino]ethyl}-5-{isopropyl[4-methoxy-3-(3-methoxypropoxy)benzoyl]amino}piperidine-1-carboxylate

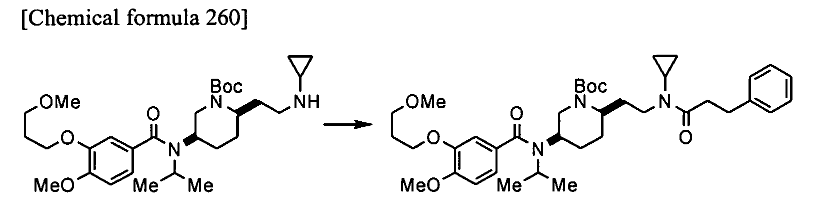

The title compound (7.7 mg) was synthesized in a similar manner to Reference Example 93.
MS (ESI+) 680 (M⁺+1, 100 %).

### Reference Example 157

### Methyl 3-hydroxybenzoate

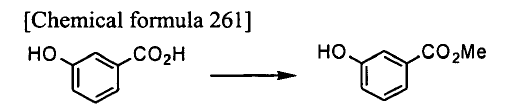

The title compound (525 mg) was synthesized in a similar manner to Reference Example 1.
¹H-NMR (400MHz, CDCl₃) 7.62-7.59(m, 2H), 7.34-7.29(m, 1H), 7.09-7.06(m, 1H), 5.92(brs, 2H), 3.92(s, 3H).

### Reference Example 158

### Methyl 3-(3-methoxypropoxy)benzoate

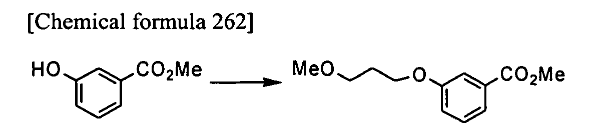

The title compound (738 mg) was synthesized in a similar manner to Reference Example 2.
¹H-NMR (400MHz, CDCl₃) 7.63-7.61(m, 1H), 7.57-7.56(m, 1H), 7.33(t, J = 8.0 Hz, 1H), 7.10(m, 1H), 4.10(t, J = 6.3 Hz, 2H), 3.91(s, 3H), 3.56(t, J = 6.2 Hz, 2H), 3.36(s, 3H), 2.06(m, 2H).

### Reference Example 159

### 3-(3-Methoxypropoxy)benzoic acid

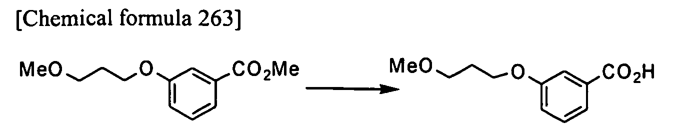

The title compound (618 mg) was synthesized in a similar manner to Reference Example 3.
¹H-NMR (400MHz, CDCl₃) 7.72-7.69(m, 1H), 7.64-7.63(m, 1H), 7.37(t, J = 8.0 Hz, 1H), 7.15(ddd, J = 0.9, 2.6, 8.2 Hz, 1H), 4.12(t, J = 6.3 Hz, 2H), 3.59(t, J = 6.2 Hz, 2H), 3.38(s, 3H), 2.14-2.05(m, 2H).

### Reference Example 160

### 3-(3-Methoxypropoxy)benzoyl chloride

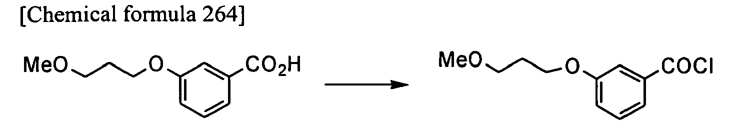

The title compound (85 mg) was synthesized in a similar manner to Reference Example 4.

### Reference Example 161

### tert-Butyl (3R)-3-{isopropyl[3-(3-methoxypropoxy)benzoyl]amino}piperidine-1-carboxylate

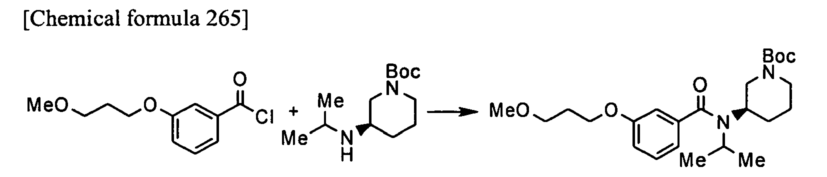

The title compound (218 mg) was synthesized in a similar manner to Reference Example 8.
MS (ESI+) 435 (M⁺+1, 100 %).

### Reference Example 162

### tert-Butyl ((1S)-1-acetyl-4-{[(benzyloxy)carbonyl]amino}butyl)carbamate

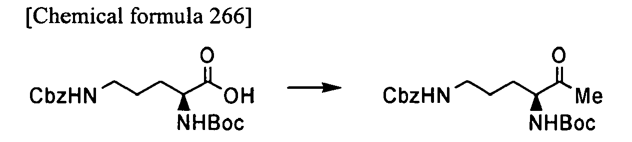

N⁵-[(Benzyloxy)carbonyl]-N²-(tert-butoxycarbonyl)-L-ornithine (366 mg) was dissolved in tetrahydrofuran (1.0 ml), and thereto was added methyl lithium (1.6M in ether) (2.7 ml) at 0°C. The mixture was stirred at room temperature for 5 hours. Water was added to the reaction mixture, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 1/1) to give the title compound (51 mg) as a colorless oily compound.
MS (ESI+) 365 (M⁺+1, 100 %).

### Reference Example 163

### tert-Butyl [(3R)-2-methylpiperidin-3-yl]carbamate

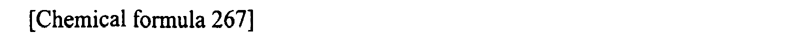

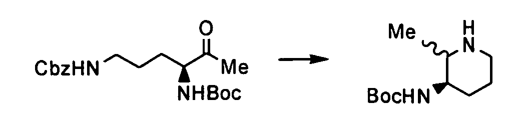

tert-Butyl ((1S)-1-acetyl-4-{[(benzyloxy)carbonyl]amino}butyl)carbamate (53 mg) was dissolved in methanol (1.0 ml), and thereto was added a 10 % palladium carbon (50 mg), and the mixture was stirred at room temperature for 6 hours under hydrogen atmosphere of 0.45MPa. The reaction solution was filtered through celite, and the obtained residue was concentrated under reduced pressure to give the title compound (30.0 mg) as a colorless oily diastereomers mixture.
MS (ESI+) 215 (M⁺+1, 100 %).

### Reference Example 164

### Benzyl (2R,3R)-3-[(tert-butoxycarbonyl)amino]-2-methylpiperidine-1-carboxylate

### Benzyl (2S,3R)-3-[(tert-butoxycarbonyl)amino]-2-methylpiperidine-1-carboxylate

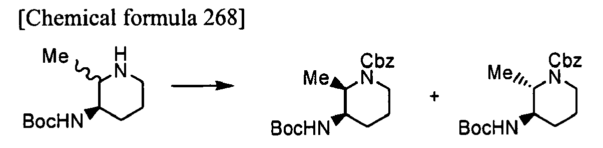

In a similar manner to Reference Example 5, benzyl (2R,3R)-3-[(tert-butoxycarbonyl)amino]-2-methylpiperidine-1-carboxylate(148 mg) and benzyl (2S,3R)-3-[(tert-butoxycarbonyl)amino]-2-methylpiperidine-1-carboxylate (343 mg) were synthesized.
MS (ESI+) 349 (M⁺+1, 100 %).

### Reference Example 165

### Benzyl (2R,3R)-3-amino-2-methylpiperidine-1-carboxylate hydrochloride

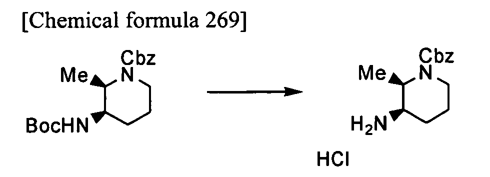

The title compound (330 mg) was synthesized in a similar manner to Reference Example 6.
MS (ESI+) 249 (M⁺+1, 100 %).

### Reference Example 166

### Benzyl (2R,3R)-3-(isopropylamino)-2-methylpiperidine-1-carboxylate

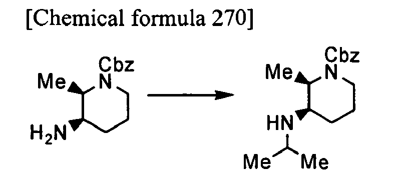

The title compound (107 mg) was synthesized in a similar manner to Reference Example 54.
MS (ESI+) 291 (M⁺+1, 100 %).

### Reference Example 167

### tert-Butyl (2R,3R)-3-{isopropyl[4-methoxy-3-(3-methoxypropoxy)benzoyl]amino}-2-methylpiperidine-1-carboxylate

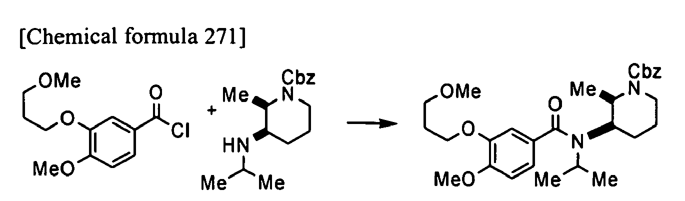

The title compound (110 mg) was synthesized in a similar manner to Reference Example 8.
MS (ESI+) 513 (M⁺+1, 100%).

Reference Example 168
(a) Methyl 3,5-bis(3-methoxypropoxy)benzoate
(b) Methyl 3-hydroxy-5-(3-methoxypropoxy)benzoate 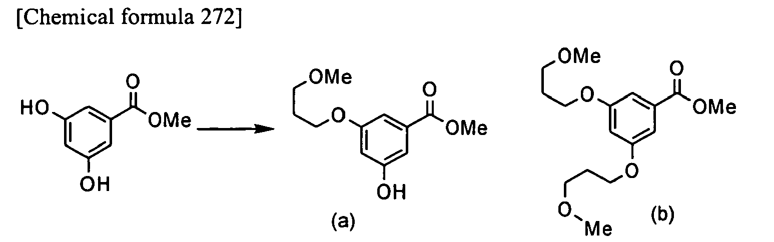
The title compounds (a) (2.06 g) and (b) (2.18 g) were synthesized in a similar manner to Reference Example 2.
(a) ¹H-NMR (400MHz, CDCl₃) 7.15-7.11(m, 2H), 6.79(brs, 1H), 6.62-6.61(m, 1H), 4.05(t, J = 6.3 Hz, 2H), 3.89(s, 3H), 3.59(t, J = 6.2 Hz, 2H), 3.38(s, 3H), 2.08-2.02(m, 2H).
(b) ¹H-NMR (400MHz, CDCl₃) 7.22-7.18(d, J = 2.3 Hz, 2H), 6.66(t, J = 2.3 Hz, 1H), 4.09(t, J = 7.1 Hz, 4H), 3.90(s, 3H), 3.54(t, J = 6.2 Hz, 4H), 3.36(s, 6H), 2.08-2.02(m, 4H).

### Reference Example 169

### 3,5-Bis(3-methoxypropoxy)benzoic acid

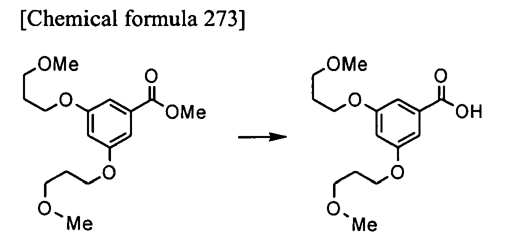

The title compound (2.20 g) was synthesized in a similar manner to Reference Example 3.
¹H-NMR (400MHz, CDCl₃) 7.24(d, J = 2.3 Hz, 2H), 6.70(t, J = 2.3 Hz, 1 H), 4.09(t, J = 6.3 Hz, 4H), 3.57(t, J = 6.2 Hz, 4H), 3.37(s, 6H), 2.09-2.03(m, 4H).

### Reference Example 170

### 3,5-Bis(3-methoxypropoxy)benzoyl chloride

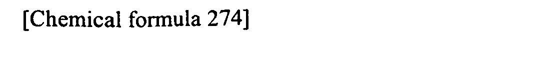

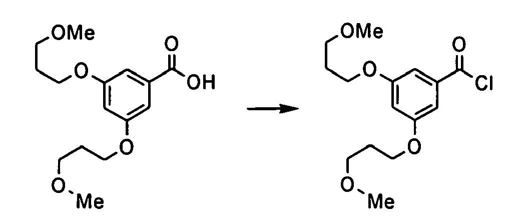

The title compound (2.20 g) was synthesized in a similar manner to Reference Example 4.

### Reference Example 171

### tert-Butyl (3R)-3-[[3,5-bis(3-methoxypropoxy)benzoyl](isopropyl)amino]piperidine-1-carboxylate

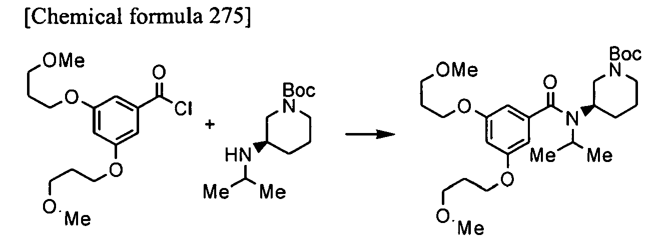

The title compound (513 mg) was synthesized in a similar manner to Reference Example 8.
MS (ESI+) 523 (M⁺+1, 100 %).

### Reference Example 172

### Methyl 4-bromo-3-(3-methoxypropoxy)benzoate

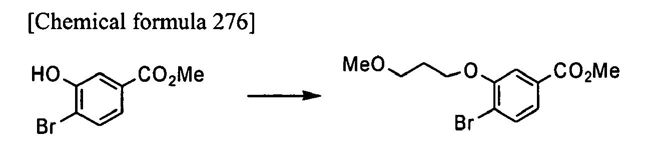

The title compound (3.49 g) was synthesized in a similar manner to Reference Example 2.
¹H-NMR (400MHz, CDCl₃) 7.59(d, J = 8.2 Hz, 1H), 7.55(d, J = 1.8 Hz, 1H), 7.49(dd, J = 1.8, 8.2 Hz, 1H), 4.18(t, J = 6.2 Hz, 2H), 3.91 (s, 3H), 3.61(t, J = 6.1 Hz, 2H), 3.36(s, 3H), 2.15-2.08(m, 2H).

### Reference Example 173

### 4-Bromo-3-(3-methoxypropoxy)benzoic acid

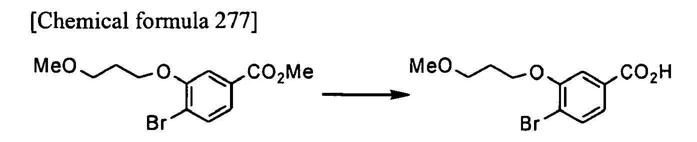

The title compound (3.10 g) was synthesized in a similar manner to Reference Example 3.
¹H-NMR (400MHz, CDCl₃) 7.63(d, J = 8.2 Hz, 1H), 7.60(d, J = 1.7 Hz, 1H), 7.56(dd, J = 1.7, 8.2 Hz, 1H), 4.21 (t, J = 6.2 Hz, 2H), 3.63(t, J = 6.1 Hz, 2H), 3.38(s, 3H), 2.17-2.10(m, 2H).

### Reference Example 174

### 4-Bromo-3-(3-methoxypropoxy)benzoyl chloride

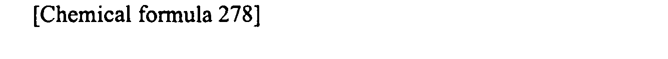

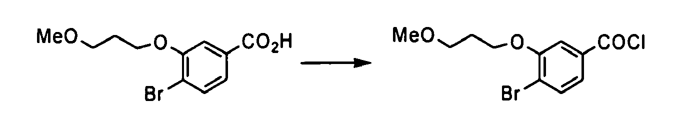

The title compound (3.57 g) was synthesized in a similar manner to Reference Example 4.

### Reference Example 175

### tert-Butyl (3R)-3-[[4-bromo-3-(3-methoxypropoxy)benzoyl](isopropyl)amino]piperidine-1-carboxylate

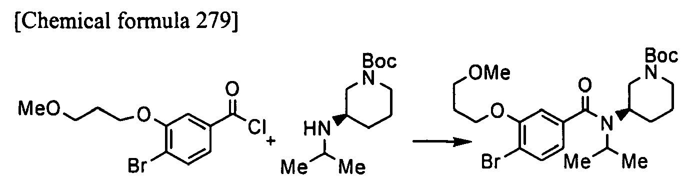

The title compound (5.37 g) was synthesized in a similar manner to Reference Example 8.
MS (ESI+) 513 (M⁺+1, 50 %), 515 (M⁺+1, 50 %).

### Reference Example 176

### Methyl 3-(benzyloxy)-5-(3-methoxypropoxy)benzoate

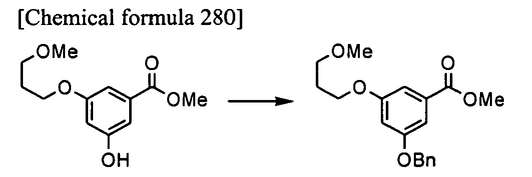

The title compound (2.44 g) was synthesized in a similar manner to Reference Example 29.
¹H-NMR (400MHz, CDCl₃) 7.43-7.32(m, 5H), 7.28-7.27(m, 1H), 7.22-7.21(m, 1H), 6.73(t, J = 2.3 Hz, 1H), 5.07(s, 2H), 4.07(t, J = 6.3 Hz, 2H), 3.90(s, 3H), 3.54(t, J = 6.2 Hz, 2H), 3.36(s, 3H), 2.07-2.01 (m, 2H).

### Reference Example 177

### 3-(Benzyloxy)-5-(3-methoxypropoxy)benzoic acid

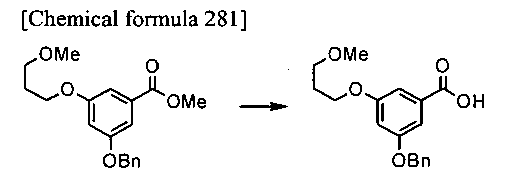

The title compound (2.34 g) was synthesized in a similar manner to Reference Example 3.

### Reference Example 178

### 3-(Benzyloxy)-5-(3-methoxypropoxy)benzoyl chloride

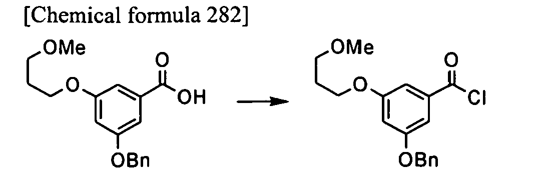

The title compound (2.40 g) was synthesized in a similar manner to Reference Example 4.

### Reference Example 179

### tert-Butyl (3R)-3-[[3-(benzyloxy)-5-(3-methoxypropoxy)benzoyl](isopropyl)amino]piperidine-1-carboxylate

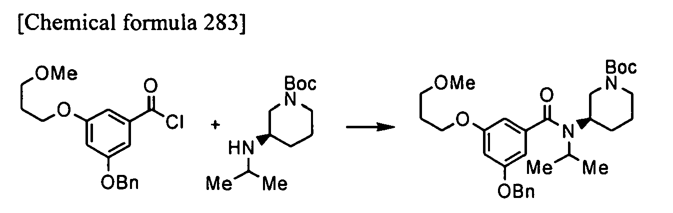

The title compound (3.58 g) was synthesized in a similar manner to Reference Example 8.
MS (ESI+) 541 (M⁺+1, 100 %).

### Reference Example 180

### tert-Butyl (3R)-3-[[3-hydroxy-5-(3-methoxypropoxy)benzoyl](isopropyl)amino]piperidine-1-carboxylate

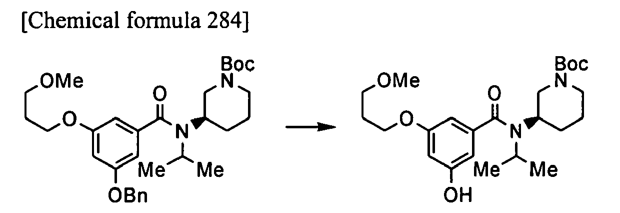

The title compound (2.46 g) was synthesized in a similar manner to Reference Example 20.
MS (ESI+) 451 (M⁺+1, 100 %).

### Reference Example 181

### tert-Butyl (3R)-3-{isopropyl[3-(3-methoxypropoxy)-5-phenoxybenzoyl]amino}piperidine-1-carboxylate

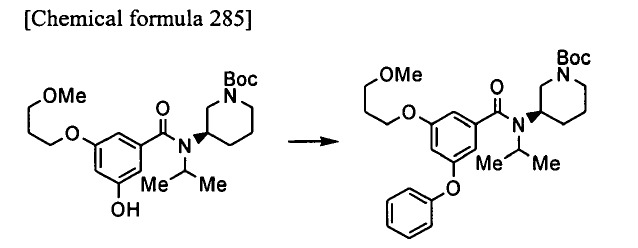

tert-Butyl (3R)-3-[[3-hydroxy-5-(3-methoxypropoxy)benzoyl](isopropyl)amino]piperidine-1-carboxylate(84.3 mg) was dissolved in dichloromethane (2 ml), and thereto were added triethylamine (52.1 µl), copper acetate (33.9 mg), and phenylboronic acid (45.6 mg). The mixture was stirred at room temperature for 15 hours, and thereto was added a saturated aqueous sodium hydrogen carbonate solution. The mixture was extracted with ethyl acetate, and the organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 3/1) to give the title compound (44.4 mg) as a colorless oily compound.
MS (ESI+) 527 (M⁺+1, 100 %).

### Reference Example 182

### tert-Butyl (3R)-3-{isopropyl[3-methoxy-5-(3-methoxypropoxy)benzoyl]amino}piperidine-1-carboxylate

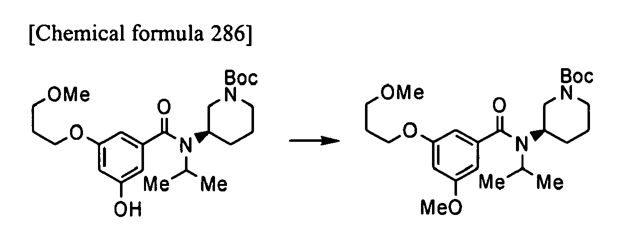

The title compound (65.6 mg) was synthesized in a similar manner to Reference Example 51.
MS (ESI+) 465 (M⁺+1, 100 %).

### Reference Example 183

### tert-Butyl (3R)-3-[[3-(cyclopropylmethyl)-5-(3-methoxypropoxy)benzoyl](isopropyl)amino]piperidine-1-carboxylate

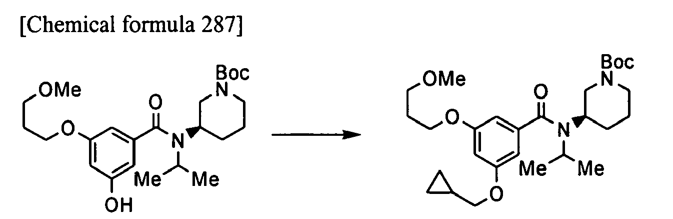

The title compound (86.1 mg) was synthesized in a similar manner to Reference Example 51.
MS (ESI+) 505 (M⁺+1, 100 %).

### Reference Example 184

### tert-Butyl (3R)-3-[[3-isopropoxy-5-(3-methoxypropoxy)benzoyl](isopropyl)amino]piperidine-1-carboxylate

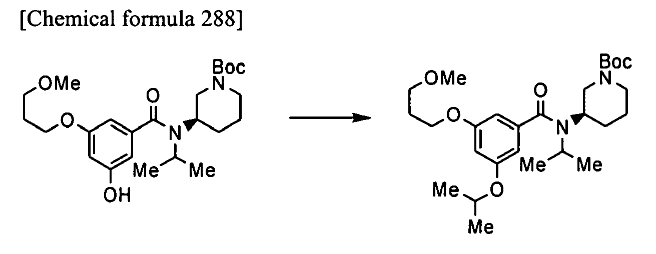

The title compound (73.5 mg) was synthesized in a similar manner to Reference Example 51.
MS (ESI+) 493 (M⁺+1, 100 %).

### Reference Example 185

### tert-Butyl (3R)-3-[[3-(cyclohexyloxy)-5-(3-methoxypropoxy)benzoyl](isopropyl)amino]piperidine-1-carboxylate

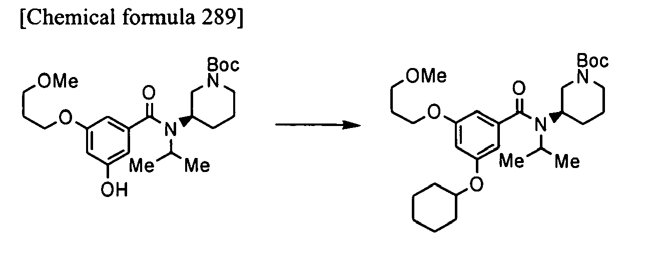

The title compound (12.1 mg) was synthesized in a similar manner to Reference Example 51.
MS (ESI+) 533 (M⁺+1, 100 %).

### Reference Example 186

### tert-Butyl (3R)-3-[isopropyl(3-(3-methoxypropoxy)-5-{[(trifluoromethyl)sulfonyl]oxy}-benzoyl)amino]piperidine-1-carboxylate

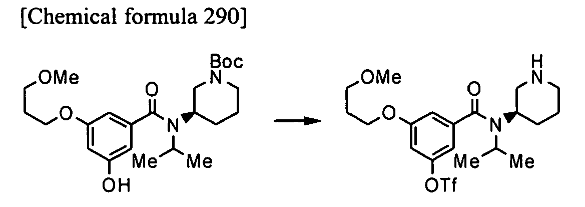

tert-Butyl (3R)-3-[[3-hydroxy-5-(3-methoxypropoxy)benzoyl](isopropyl)amino]piperidine-1-carboxylate (245 mg) was dissolved in dichloromethane (2 ml), and thereto were added triethylamine (151 µl) and trifluorosulfonic anhydride (110 µl) under ice-cooling. Then, the mixture was stirred at room temperature for 6 hours. To the reaction solution was added a saturated aqueous ammonium chloride solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 3/1) to give the title compound (266 mg) as a pale yellow oily compound.
MS (ESI+) 583 (M+1, 100 %).

### Reference Example 187

### tert-Butyl (3R)-3-(isopropyl{[5-(3-methoxypropoxy)biphenyl-3-yl]carbonyl}amino)piperidine-1-carboxylate

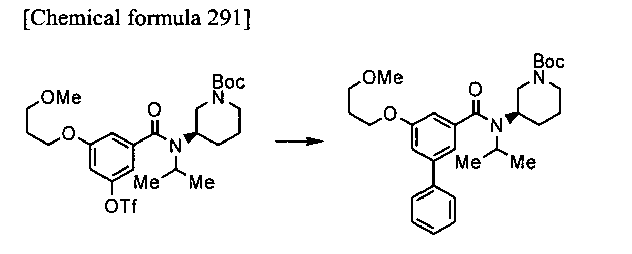

tert-Butyl (3R)-3-[isopropyl(3-(3-methoxypropoxy)-5-{[(trifluoromethyl)-sulfonyl]oxy}benzoyl)amino]piperidine-1-carboxylate (119 mg) was dissolved in 1,2-dimethoxyethane (2 ml) and water (1 ml), and thereto were added sodium carbonate (64.9 mg), phenylboronic acid (49.7 mg) and tetrakistriphenylphosphine palladium (46.3 mg), and the mixture was heated under reflux. Five hours thereafter, the reaction solution was cooled to 25°C, and thereto was added a saturated aqueous sodium hydrogen carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (hexane/ethyl acetate = 2/1) to give the title compound (59.9 mg) as a colorless oily compound.
MS (ESI+) 511 (M+1, 100 %).

### Reference Example 188

### Ethyl 3-hydroxy-4-(phenoxymethyl)benzoate

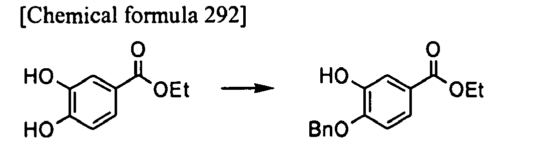

The title compound (9.88 g) was synthesized in a similar manner to Reference Example 29.

### Reference Example 189

### Ethyl 3-(3-methoxypropoxy)-4-(phenoxymethyl)benzoate

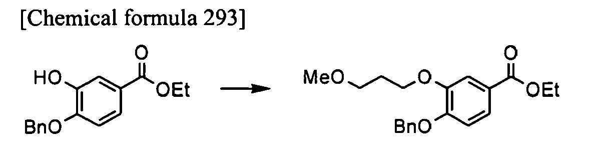

The title compound (12.06 g) was synthesized in a similar manner to Reference Example 2.

### Reference Example 190

### 3-(3-Methoxypropoxy)-4-(phenoxymethyl)benzoic acid

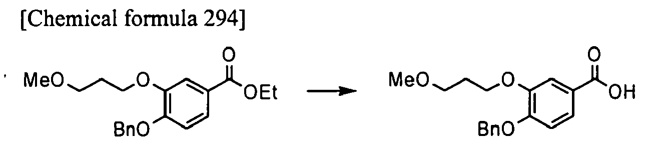

The title compound (10.38 g) was synthesized in a similar manner to Reference Example 3.

### Reference Example 191

### 3-(3-Methoxypropoxy)-4-(phenoxymethyl)benzoyl chloride

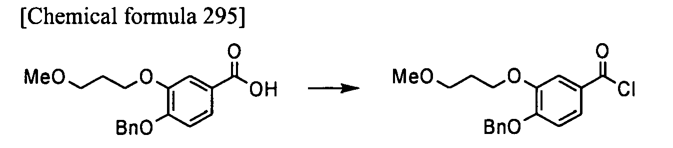

The title compound (10.5 g) was synthesized in a similar manner to Reference Example 4.

### Reference Example 192

### tert-Butyl (3R)-3-[[4-(benzyloxy)-3-(3-methoxypropoxy)benzoyl](isopropyl)amino]piperidine-1-carboxylate

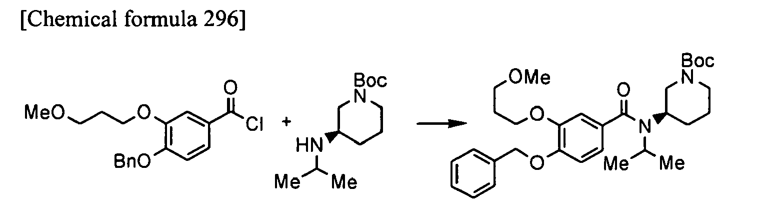

The title compound (15.41 g) was synthesized in a similar manner to Reference Example 8.
MS (ESI+) 541 (M⁺+1, 100 %).

### Reference Example 193

### tert-Butyl (3R)-3-[[4-hydroxy-3-(3-methoxypropoxy)benzoyl](isopropyl)amino]piperidine-1-carboxylate

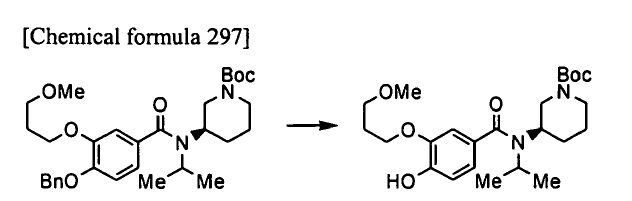

The title compound (3.35 g) was synthesized in a similar manner to Reference Example 20.
MS (ESI+) 451 (M⁺+1, 100 %).

### Reference Example 194

### tert-Butyl (3R)-3-[[4-butyl-3-(3-methoxypropoxy)benzoyl](isopropyl)amino]piperidine-1-carboxylate

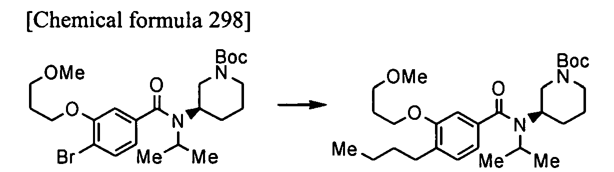

tert-Butyl (3R)-3-[[4-bromo-3-(3-methoxypropoxy)benzoyl](isopropyl)amino]piperidine-1-carboxylate (274 mg) was dissolved in 1,2-dimethoxyethane (2 ml) and water (1 ml), and thereto were added sodium carbonate (113 mg), butylboronic acid (81.5 mg), tetrakisphenylphosphine palladium (60.4 mg), and the mixture was heated under reflux. Five hours thereafter, the reaction solution was cooled to 25°C, and thereto was added a saturated aqueous sodium hydrogen carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (hexane/ethyl acetate = 2/1) to give the title compound (8.1 mg) as a colorless oily compound.
MS (ESI+) 491 (M+1, 100 %).

### Reference Example 195

### tert-Butyl (3R)-3-{isopropyl[3-(3-methoxypropoxy)-4-phenoxybenzoyl]amino}piperidine-1-carboxylate

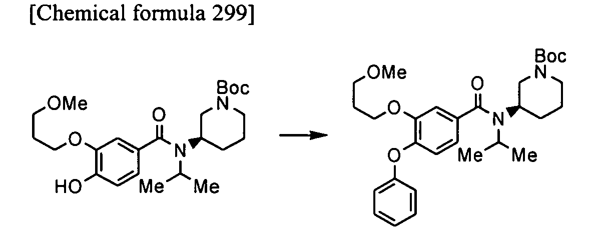

tert-Butyl (3R)-3-[[4-hydroxy-3-(3-methoxypropoxy)benzoyl](isopropyl)amino]piperidine-1-carboxylate (84.3 mg) was dissolved in dichloromethane (2 ml), and thereto were added triethylamine (116 µl), copper acetate (75.9 mg) and phenylboronic acid (102 mg). The mixture was stirred at room temperature for 15 hours, and thereto was added a saturated aqueous sodium hydrogen carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (developing solvent: hexane/ethyl acetate = 2/1) to give the title compound (50.7 mg) as a colorless oily compound.
MS (ESI+) 427 (M⁺+1, 100 %).

### Reference Example 196

### tert-Butyl (3R)-3-{isopropyl[3-(3-methoxypropoxy)-4-(2-phenylethyl)benzoyl]amino}piperidine-1-carboxylate

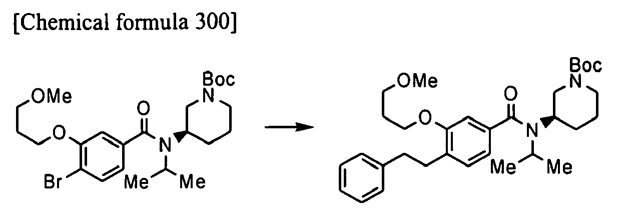

tert-Butyl (3R)-3-[[4-bromo-3-(3-methoxypropoxy)benzoyl](isopropyl)amino]piperidine-1-carboxylate (86.0 mg) was dissolved in 1,2-dimethoxyethane (2 ml) and water (1 ml), and thereto were added sodium carbonate (53.1 mg), phenethylboronic acid (50.1 mg) and tetrakistriphenylphosphine palladium (47.4 mg), and the mixture was heated under reflux. Five hours thereafter, the reaction solution was cooled to 25°C, and thereto was added a saturated aqueous sodium hydrogen carbonate solution, and the mixture was extracted with ethyl acetate. The organic layer was washed with a saturated aqueous sodium chloride solution, dried over sodium sulfate, filtered and concentrated under reduced pressure. The obtained residue was purified by silica gel column chromatography (hexane/ethyl acetate = 2/1) to give the title compound (4.8 mg) as a colorless oily compound.
MS (ESI+) 539 (M+1, 100 %).

### Reference Example 197

### tert-Butyl (3R)-3-[[4-isopropoxy-3-(3-methoxypropoxy)benzoyl](isopropyl)amino]piperidine-1-carboxylate

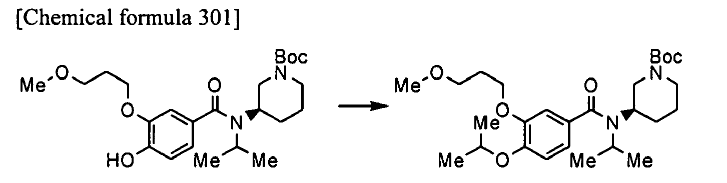

The title compound (49.2 mg) was synthesized in a similar manner to Reference Example 51.
MS (ESI+) 493 (M⁺+1, 100 %).

### Reference Example 198

### tert-Butyl (3R)-3-[[4-(cyclopropylmethoxy)-3-(3-methoxypropoxy)benzoyl](isopropyl)amino]piperidine-1-carboxylate

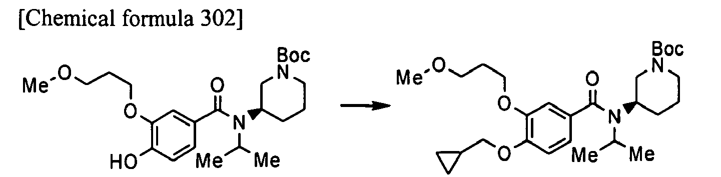

The title compound (49.6 mg) was synthesized in a similar manner to Reference Example 51.
MS (ESI+) 505 (M⁺+1, 100 %).

### Reference Example 199

### tert-Butyl (3R)-3-[[4-(cyclohexyloxy)-3-(3-methoxypropoxy)benzoyl](isopropyl)amino]piperidine-1-carboxylate

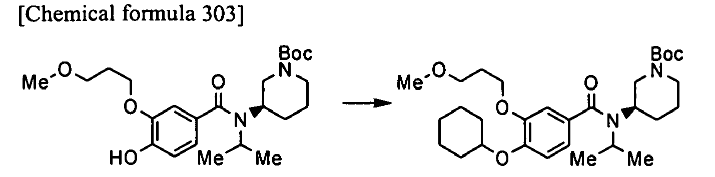

The title compound (10.2 mg) was synthesized in a similar manner to Reference Example 51.
MS (ESI+) 533 (M⁺+1, 100 %).

### Reference Example 200

### tert-Butyl (3R)-3-[[2-bromo-4-isopropoxy-5-(3-methoxypropoxy)benzoyl](isopropyl)amino]piperidine-1-carboxylate

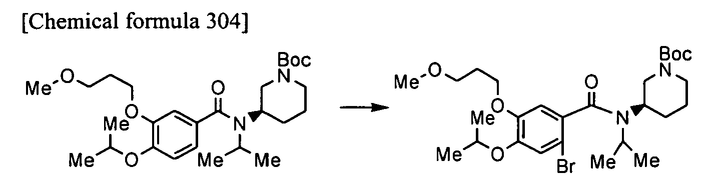

tert-Butyl (3R)-3-[[4-isopropoxy-3-(3-methoxypropoxy)benzoyl](isopropyl)amino]piperidine-1-carboxylate (56.4 mg) was dissolved in acetic acid (2 ml), and thereto were added sodium acetate (39.4 mg) and bromine (5.9 µl) at room temperature. Twelve hours thereafter, a saturated aqueous sodium thiosulfate solution was added to the reaction solution, and the mixture was extracted with ethyl acetate. The organic layer was washed with a 1M aqueous sodium hydroxide solution and a saturated aqueous sodium chloride solution, dried over sodium sulfate, filtered and concentrated under reduced pressure. The obtained residue was purified by silica gel column chromatography (hexane/ethyl acetate = 3/1) to give the title compound (60.6 mg) as a colorless oily compound.
MS (ESI+) 571 (M⁺+1, 50%), 573(M⁺+1, 50%).

### Reference Example 201

### tert-Butyl (3R)-3-[{[5-isopropoxy-4-(3-methoxypropoxy)biphenyl-2-yl]carbonyl}(isopropyl)-amino]piperidine-1-carboxylate

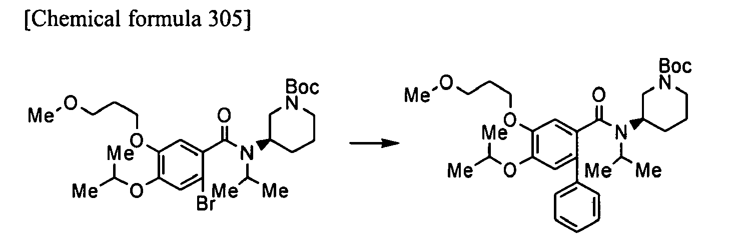

tert-Butyl (3R)-3-[[2-bromo-4-isopropoxy-5-(3-methoxypropoxy)benzoyl]-(isopropyl)amino]piperidine-1-carboxylate (33.1 mg) was dissolved in 1,2-dimethoxyethane (2 ml) and water (1 ml), and thereto were added sodium carbonate (12.1 mg), phenylboronic acid (14.1 mg), and tetrakistriphenylphosphine palladium (13.1 mg), and the mixture was heated under reflux. Five hours thereafter, the reaction solution was cooled to 25°C, and thereto was added a saturated aqueous sodium hydrogen carbonate solution. The mixture was extracted with ethyl acetate, and the organic layer was washed with a saturated aqueous sodium chloride solution, dried over sodium sulfate, filtered and concentrated under reduced pressure. The obtained residue was purified by silica gel column chromatography (hexane/ethyl acetate = 2/1) to give the title compound (5.8 mg) as a colorless oily compound.
MS (ESI+) 569 (M⁺+1, 100 %).

### Reference Example 202

### tert-Butyl (3R)-3-[[4-ethoxy-3-(3-methoxypropoxy)benzoyl](isopropyl)amino]piperidine-1-carboxylate

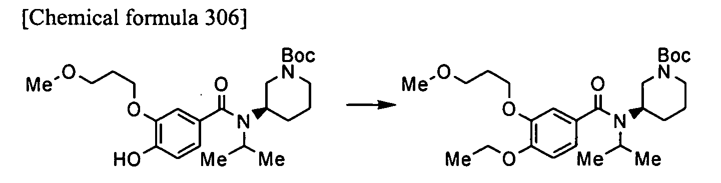

The title compound (57.3 mg) was synthesized in a similar manner to Reference Example 51.
MS (ESI+) 579 (M⁺+1, 100 %).

### Reference Example 203

### tert-Butyl (3R)-3-{isopropyl[3-(3-methoxypropoxy)-4-propoxybenzoyl]amino}piperidine-1-carboxylate

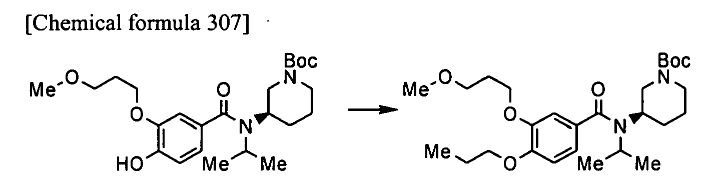

The title compound (58.6 mg) was synthesized in a similar manner to Reference Example 51.
MS (ESI+) 493 (M⁺+1, 100 %).

### Reference Example 204

### tert-Butyl (3R)-3-[[4-(2-fluoroethoxy)-3-(3-methoxypropoxy)benzoyl](isopropyl)amino]piperidine-1-carboxylate

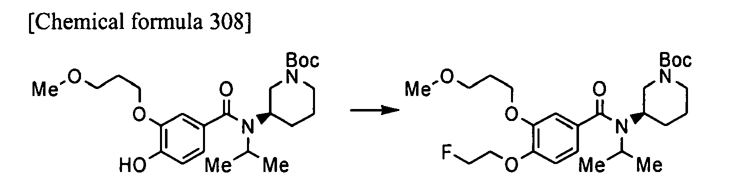

The title compound (58.0 mg) was synthesized in a similar manner to Reference Example 51.
MS (ESI+) 497 (M⁺+1, 100 %).

### Reference Example 205

### tert-Butyl (3R)-3-[[5-(3-tert-butoxy-3-oxopropoxy)-2-chloro-4-methoxybenzoyl]-(isopropyl)amino]piperidine-1-carboxylate

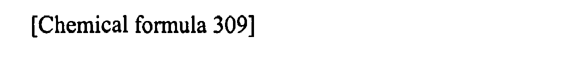

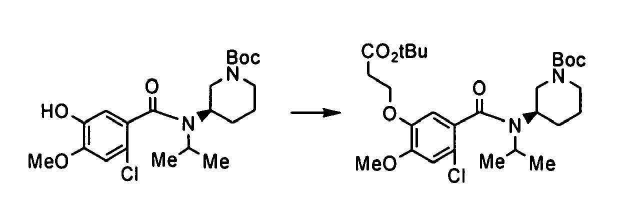

tert-Butyl (3R)-3-[(2-chloro-5-hydroxy-4-methoxybenzoyl)(isopropyl)amino]piperidine-1-carboxylate (108 mg) was dissolved in dichloromethane (2 ml), and thereto were added under ice-cooling t-butyl 3-hydroxypropiolate (56.0 µl), triphenylphosphine (113 mg) and diisopropyl azodicarboxylate (80.2 µl). The mixture was stirred at room temperature for 14 hours, and the reaction solution was concentrated under reduced pressure. The obtained residue was purified by silica gel column chromatography (hexane/ethyl acetate = 3/1) to give the title compound (11.0 mg) as a colorless oily compound.
MS (ESI+) 556 (M⁺+1, 100 %).

### Reference Example 206

### tert-Butyl (3R)-3-[{4-isopropoxy-5-(3-methoxypropoxy)-2-[(1E)-prop-1-en-1-yl]-benzoyl} (isopropyl)amino]piperidine-1-carboxylate

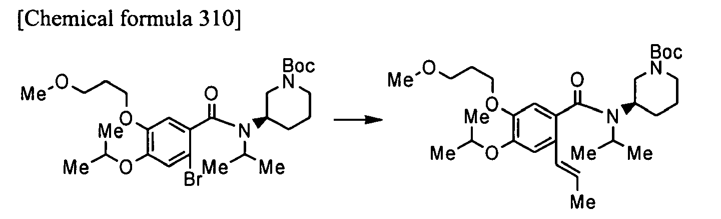

tert-Butyl (3R)-3-[[2-bromo-4-isopropoxy-5-(3-methoxypropoxy)benzoyl]-(isopropyl)amino]piperidine-1-carboxylate (294 mg) was dissolved in dimethoxyethane (2 ml) and water (1 ml), and thereto were added trans-propenylboronic acid (88.5 mg), sodium carbonate (109 mg), and palladium diphenylphosphine dichloride · dichloromethane complex (84.1 mg), and the mixture was heated under reflux for 3 hours. To the reaction mixture was added a saturated aqueous sodium hydrogen carbonate solution, and the mixture was extracted with ethyl acetate. The organic layer was washed with a saturated aqueous sodium chloride solution, dried over sodium sulfate, filtered, and concentrated under reduced pressure. The obtained residue was purified by silica gel column chromatography (hexane/ethyl acetate = 2/1) to give the title compound (195 mg) as a pale yellow oily compound.
MS (ESI+) 533 (M+1, 100 %).

### Reference Example 207

### tert-Butyl (3R)-3-[[4-isopropoxy-5-(3-methoxypropoxy)-2-propylbenzoyl](isopropyl)amino]piperidine-1-carboxylate

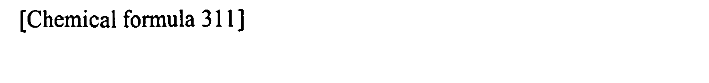

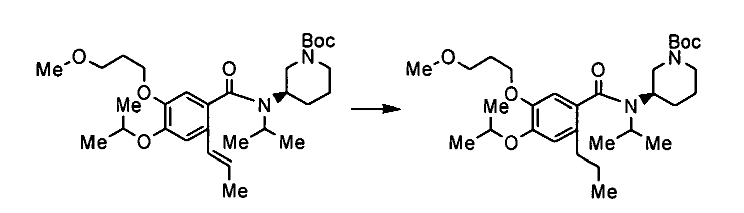

tert-Butyl (3R)-3-[{4-isopropoxy-5-(3-methoxypropoxy)-2-[(1E)-prop-1-en-1-yl]benzoyl}(isopropyl)amino]piperidine-1-carboxylate (195 mg) was dissolved in ethanol (4 ml), and thereto was added palladium carbon (190 mg), and the mixture was stirred under hydrogen atmosphere for 5 hours. The reaction solution was filtered through celite, and concentrated under reduced pressure. The obtained residue was purified by silica gel column chromatography (hexane/ethyl acetate = 2/1) to give the title compound (157 mg) as a pale yellow oily compound.
MS (ESI+) 535 (M+1, 100 %).

### Reference Example 208

### tert-Butyl (3R)-3-{isopropyl[3-(3-methoxypropoxy)-4-morpholin-4-ylbenzoyl]amino}piperidine-1-carboxylate

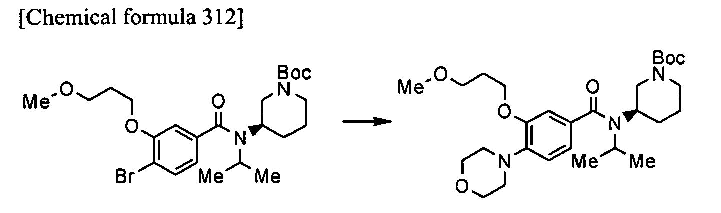

tert-Butyl (3R)-3-[[4-bromo-3-(3-methoxypropoxy)benzoyl](isopropyl)amino]piperidine-1-carboxylate (162 mg) was dissolved in toluene (2 ml), and thereto were added sodium t-butoxide (42.5 mg), tris(dibenzylideneacetone)dipalladium (28.9 mg), BINAP (29.5 mg) and morpholine (30.4 µl), and the mixture was heated under reflux. Ten hours thereafter, to the reaction mixture was added a saturated aqueous sodium hydrogen carbonate solution, and the mixture was extracted with chloroform. The organic layer was washed with a saturated aqueous sodium chloride solution, dried over sodium sulfate, filtered, and concentrated under reduced pressure. The obtained residue was purified by silica gel column chromatography (hexane/ethyl acetate = 1/3) to give the title compound (8.7 mg) as a colorless oily compound.
MS (ESI+) 520 (M+1, 100 %).

### Reference Example 209

### 3-Methoxypropoxy 4-formyl-3-(3-methoxypropoxy)benzoate

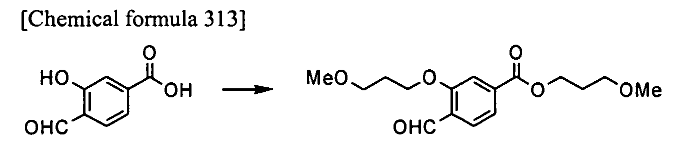

The title compound (191 mg) was synthesized in a similar manner to Reference Example 2.
¹H-NMR (400MHz, CDCl₃) 10.53(s, 1H), 7.88(d, J = 7.9 Hz, 1H), 7.69-7.66(m, 2H),4.45(t, J = 6.5 Hz, 2H), 4.27(t, J = 6.3 Hz, 2H), 3.60(t, J = 6.0 Hz, 2H), 3.54(t, J = 6.2 Hz, 2H), 3.38(s, 3H), 3.37(s, 3H), 2.19-2.12(m, 2H), 2.10-2.03(m, 2H).

### Reference Example 210

### 4-Formyl-3-(3-methoxypropoxy)benzoic acid

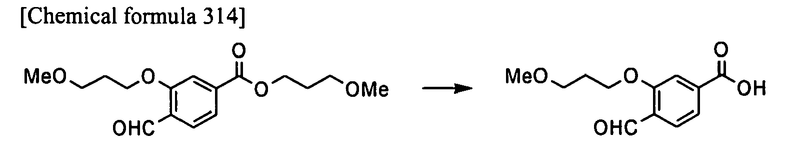

The title compound (147 mg) was synthesized in a similar manner to Reference Example 3.
¹H-NMR (400MHz, CDCl₃) 10.55 (s, 1H), 7.91 (d, J = 8.3 Hz, 1H), 7.75-7.73 (m, 2H), 4.28 (t, J = 6.2 Hz, 2H), 3.61 (t, J = 6.0 Hz, 2H), 3.38 (s, 3H), 2.19-2.13 (m, 2H).

### Reference Example 211

### 4-Formyl-3-(3-methoxypropoxy)benzoyl chloride

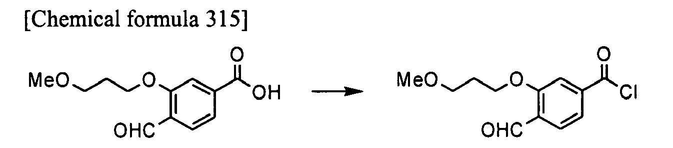

The title compound (150 mg) was synthesized in a similar manner to Reference Example 4.

### Reference Example 212

### tert-Butyl (3R)-3-[[4-formyl-3-(3-methoxypropoxy)benzoyl](isopropyl)amino]piperidine-1-carboxylate

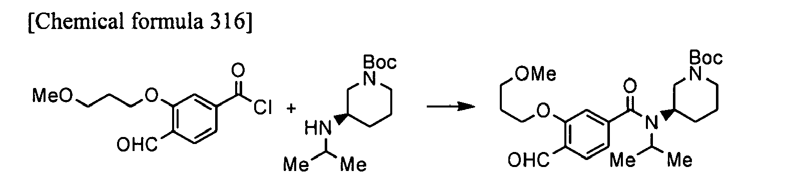

The title compound (111 mg) was synthesized in a similar manner to Reference Example 8
MS (ESI+) 463 (M⁺+1, 100 %).

### Reference Example 213

### Diethyl 4-[4- {[[(3R)-1-(tert-butoxycarbonyl)piperidin-3-yl](isopropyl)amino]carbonyl} -2-(3-methoxypropoxy)phenyl]-2,6-dimethyl-1,4-dihydropyridine-3,5-dicarboxylate

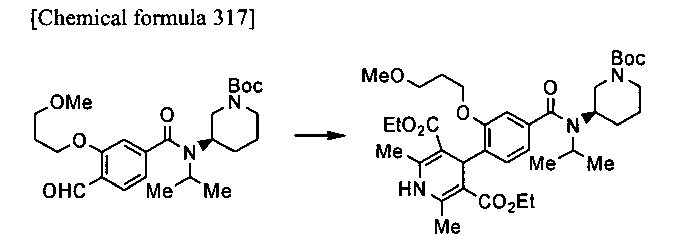

To a solution of thionyl chloride (21.1 µl) in dichloromethane (0.5 ml) was added dropwise pyridine (28.7 µl)/dichloromethane (0.5 ml) under ice-cooling. Then, a solution of tert-butyl (3R)-3-[[4-formyl-3-(3-methoxypropoxy)benzoyl](isopropyl)amino]piperidine-1-carboxylate (114 mg)/dichloromethane (2.0 ml) was added dropwise to the mixture, and the mixture was stirred at 0°C for 1 hour. To the mixture was added ethyl 3-aminochrotonate (91.4 µl), and the mixture was stirred at room temperature for 12 hours. To the reaction mixture was added a saturated aqueous ammonium chloride solution, and the mixture was extracted with ethyl acetate. The organic layer was washed with water and a saturated aqueous sodium chloride solution, dried over sodium sulfate, filtered, and concentrated under reduced pressure. he obtained residue was purified by silica gel column chromatography (hexane/ethyl acetate = 1/1) to give the title compound (58.5 mg) as a colorless oily compound.
MS (ESI+) 686 (M+1, 100 %).

### Reference Example 214

### tert-Butyl (3R)-3-[[2-chloro-4-isopropoxy-5-(3-methoxypropoxy)benzoyl](isopropyl)amino]piperidine-1-carboxylate

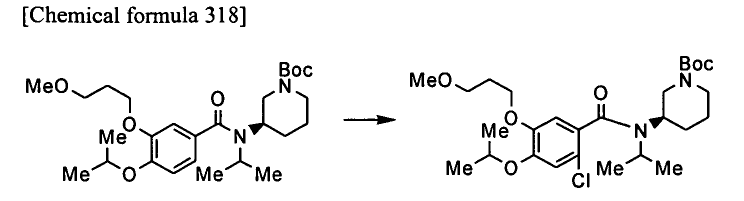

tert-Butyl (3R)-3-[[4-isopropoxy-3-(3-methoxypropoxy)benzoyl](isopropyl)amino]piperidine-1-carboxylate (86.1 mg) was dissolved in acetic acid (1ml), and thereto were added sodium acetate (60.3 mg) and N-chlorosuccinimide (25.7 mg), and the mixture was stirred at room temperature for 18 hours. To the reaction mixture was added a saturated aqueous sodium thiosulfate solution, and the mixture was extracted with ethyl acetate. The organic layer was washed with 1N aqueous sodium hydroxide solution twice, washed with a saturated aqueous sodium hydrogen carbonate solution, and further washed with a saturated aqueous sodium chloride solution, dried over sodium sulfate, filtered, and concentrated under reduced pressure. The obtained residue was purified by silica gel column chromatography (hexane/ethyl acetate = 1/1) to give the title compound (73.3 mg) as a colorless oily compound.
MS (ESI+) 527 (M+1, 100 %).

### Reference Example 215

### Methyl (2-bromoethyl)carbamate

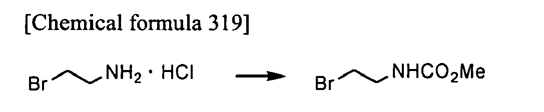

2-Bromoethylamine bromate (4.51 g) was dissolved in dichloromethane (15 ml), and thereto were added triethylamine (7.67 ml) and methyl chloroformate (1.87 ml) at 0°C. The mixture was stirred at room temperature for 12 hours, and thereto was added a saturated aqueous sodium hydrogen carbonate solution. The mixture was extracted with chloroform three times. The organic layer was dried over sodium sulfate, filtered, and concentrated under reduced pressure. The obtained residue was purified by silica gel column chromatography (hexane/ethyl acetate = 2/1) to give the title compound (478 mg) as a colorless oily compound.

H-NMR (400MHz, CDCl₃) 5.09(brs, 1H), 3.69(s, 3H), 3.63-3.57(m, 2H), 3.55-3.46(m, 2H).

### Reference Example 216

### tert-Butyl (3R)-3-[(2-chloro-4-methoxy-5-{2-[(methoxycarbonyl)amino]ethoxy}benzoyl)-(isopropyl)amino]piperidine-1-carboxylate

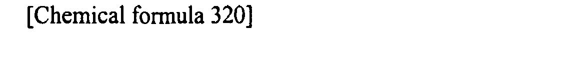

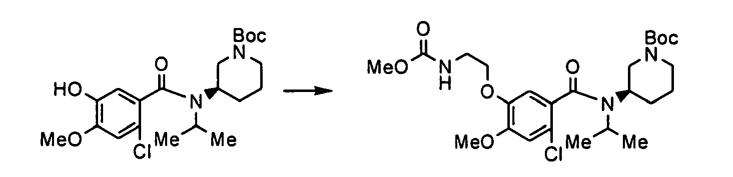

tert-Butyl (3R)-3-[(2-chloro-5-hydroxy-4-methoxybenzoyl)(isopropyl)amino]piperidine-1-carboxylate (55.0 mg) was dissolved in dimethylformamide (1 ml), and thereto was added sodium hydride (12.9 mg) at 0°C. The mixture was stirred for 15 minutes, and thereto was added methyl (2-bromoethyl)carbamate (70.4 mg), and the mixture was stirred for 12 hours. To the reaction solution was added a saturated aqueous ammonium chloride solution, and the mixture was extracted with ethyl acetate. The organic layer was washed with water and a saturated aqueous sodium chloride solution, dried over sodium sulfate, filtered, and concentrated under reduced pressure. The obtained residue was purified by silica gel column chromatography (hexane/ethyl acetate = 1/1) to give the title compound (54.4 mg) as a colorless oily compound.
MS (ESI+) 528 (M⁺+1, 100 %).

### Reference Example 217

### Methyl 4-bromo-3-{2-[(methoxycarbonyl)amino]ethoxy}benzoate

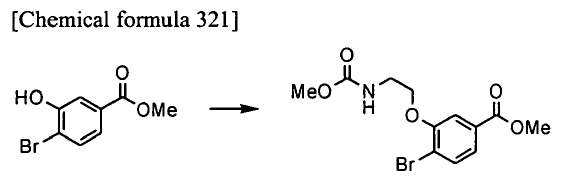

Methyl 4-bromo-3-hydroxybenzoate (600 mg) was dissolved in acetonitrile (7.0 ml), and thereto were added potassium carbonate (719 mg) and methyl (2-bromoethyl)carbamate (473 mg), and the mixture was heated under reflux for 6 hours. The reaction mixture was allowed to cool, and water was added thereto, and the mixture was extracted with ethyl acetate. The organic layer was washed with water and a saturated aqueous sodium chloride solution, dried over sodium sulfate, filtered and concentrated under reduced pressure. The obtained residue was purified by silica gel column chromatography (hexane/ethyl acetate = 2/1) to give the title compound (754 mg) as a white solid.
MS (ESI+) 332 (M⁺+1, 50%), 334 (M⁺+1, 50%).

### Reference Example 218

### 4-Bromo-3-{2-[(methoxycarbonyl)amino]ethoxy}benzoic acid

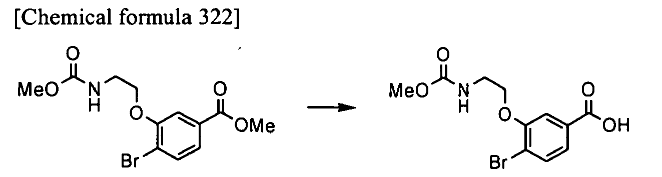

The title compound (658 mg) was synthesized in a similar manner to Reference Example 3.
MS (ESI+) 318 (M⁺+1, 50 %), 320 (M⁺+1, 50 %).

### Reference Example 219

### Methyl {2-[2-bromo-5-(chlorocarbonyl)phenoxy]ethyl}carbamate

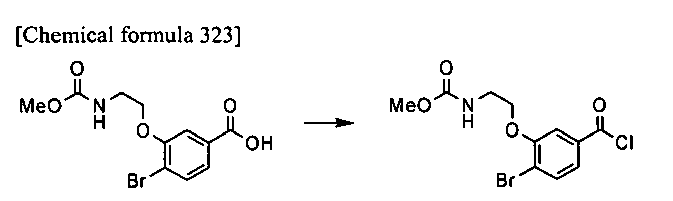

The title compound (764 mg) was synthesized in a similar manner to Reference Example 4.

### Reference Example 220

### tert-Butyl (3R)-3-[(4-bromo-3-{2-[(methoxycarbonyl)amino]ethoxy}benzoyl)(isopropyl)-amino]piperidine-1-carboxylate

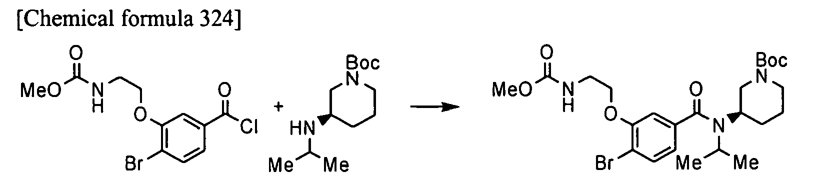

The title compound (542 mg) was synthesized in a similar manner to Reference Example 8.
MS (ESI+) 542 (M⁺+1 M⁺+1, 50 %), 544 (M⁺+1, 50%).

### Reference Example 221

### Methyl (3-bromopropyl)carbamate

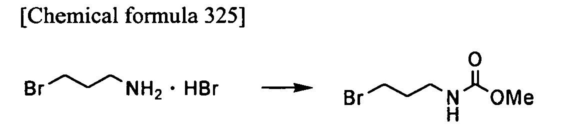

3-Bromopropylamine bromate (5.00 g) was dissolved in chloroform (80 ml), and thereto were added triethylamine (5.80 g) and methyl chloroformate (2.40 g) at 0°C. The mixture was stirred at room temperature for 12 hours, and to the reaction solution was added water, and the mixture was extracted with chloroform. The organic layer was dried over sodium sulfate, filtered and concentrated under reduced pressure. The obtained residue was purified by silica gel column chromatography (hexane/ethyl acetate = 4/1 to 3/1) to give the title compound (3.6 g) as a colorless oily compound.
MS (ESI+) 196 (M⁺+1, 50 %), 198 (M⁺+1, 50 %).

### Reference Example 222

### tert-Butyl (3R)-3-[(2-chloro-4-methoxy-5-{3-[(methoxycarbonyl)amino]propoxy}-benzoyl)(isopropyl)amino]piperidine-1-carboxylate

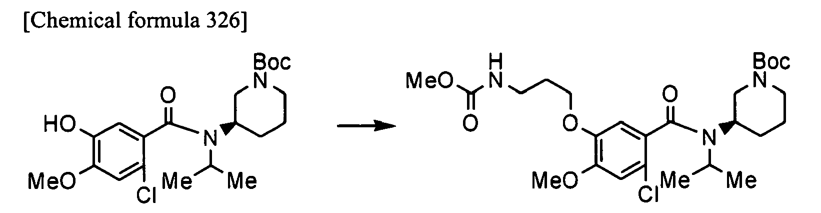

tert-Butyl (3R)-3-[(2-chloro-5-hydroxy-4-methoxybenzoyl)(isopropyl)amino]piperidine-1-carboxylate (134 mg) was dissolved in dimethylformamide (2 ml), and thereto was added sodium hydride (43.8 mg) at 0°C. The mixture was stirred for 10 minutes, and then, thereto was added methyl (3-bromopropyl)carbamate (307 mg), and the mixture was stirred for 6 hours. To the reaction solution was added a saturated aqueous ammonium chloride solution, and the mixture was extracted with ethyl acetate. The organic layer was washed with water twice, washed with a saturated aqueous sodium chloride solution, dried over sodium sulfate, filtered and concentrated under reduced pressure. The obtained residue was purified by silica gel column chromatography (hexane/ethyl acetate = 1/1) to give the title compound (124 mg) as a white solid.
MS (ESI+) 542 (M⁺+1, 100 %).

### Reference Example 223

### tert-Butyl (3R)-3-[[4-(cyclopentyloxy)-3-(3-methoxypropoxy)benzoyl](isopropyl)amino]piperidine-1-carboxylate

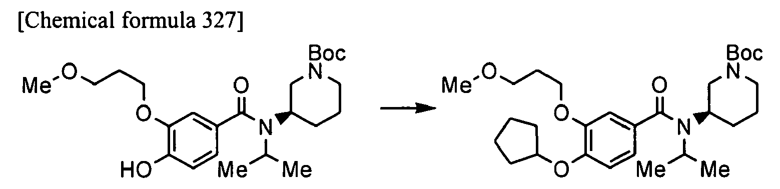

The title compound (201 mg) was synthesized in a similar manner to Reference Example 51.

MS (ESI+) 519 (M⁺+1, 100 %).

### Reference Example 224

### Methyl 4-(2-ethoxy-1,1-dimethyl-2-oxoethoxy)-3-nitrobenzoate

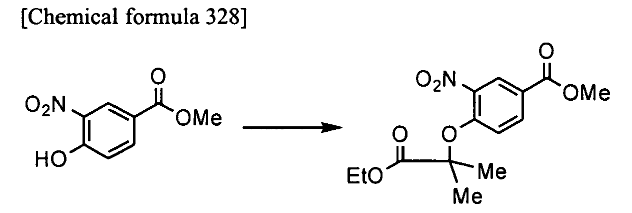

To a solution of methyl 4-hydroxy-3-nitrobenzoate (18.0 g) in N,N-dimethylformamide (70 ml) were added potassium carbonate (25.1 g) and ethyl 2-bromoisobutylate (20.0 g), and the mixture was heated with stirring at 80°C. Ten hours thereafter, the reaction solution was cooled to 25°C, and water was added to the reaction solution. The mixture was extracted with ethyl acetate, and the organic layer was washed with a saturated aqueous sodium chloride solution, dried over magnesium sulfate, filtered and concentrated under reduced pressure. The obtained residue was purified by silica gel column chromatography (hexane/ethyl acetate = 5/1) to give the title compound (11.8 g).
MS (ESI+) (M⁺+1, 100 %).

### Reference Example 225

### Methyl 2,2-dimethyl-3-oxo-3,4-dihydro-2H-1,4-benzoxazine-6-carboxylate

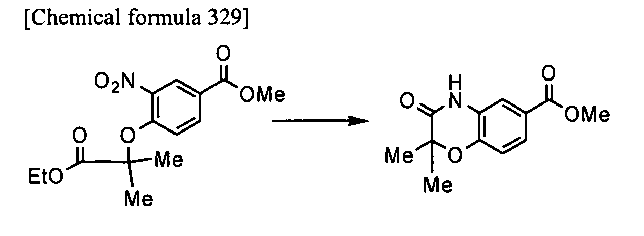

To a suspension of iron (12.0 g) in acetic acid (30 ml) was slowly added dropwise a solution of the compound of Reference Example 224 (11.8 g) in acetic acid (50 ml)/ethanol (50 ml) at 80°C, and after the addition, the mixture was heated with stirring at 80°C. Three hours thereafter, the reaction solution was cooled to 25°C, and the insoluble materials were removed by filtration through celite, and the filtrate was concentrated under reduced pressure. To the obtained residue was added water, and the mixture was extracted with ethyl acetate. The organic layer was washed with a saturated aqueous sodium chloride solution, dried over magnesium sulfate, filtered and concentrated under reduced pressure. The obtained residue was washed with ethyl acetate to give the title compound (6.8 g) as a white solid.
MS (ESI+) (M⁺+1, 100 %).

### Reference Example 226

### Methyl 4-(4-methoxybutyl)-2,2-dimethyl-3-oxo-3,4-dihydro-2H-1,4-benzoxazine-6-carboxylate

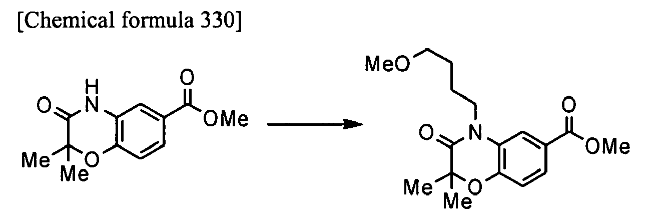

To a solution of the compound of Reference Example 225 (1.80 g) in N,N-dimethylformamide (20 ml) were added sodium hydride (55 % wg, 0.40 g) and 1-bromo-3-methoxypropane (1.40 g), and the mixture was heated with stirring at 80°C. Six hours thereafter, the reaction solution was cooled to 25°C, and thereto was added water. The mixture was extracted with ethyl acetate, and the organic layer was washed with a saturated aqueous sodium chloride solution, dried over magnesium sulfate, filtered and concentrated under reduced pressure. The obtained residue was purified by silica gel column chromatography (hexane/ethyl acetate = 5/1) to give the title compound (1.85 g).
MS (ESI+) (M⁺+1, 100 %).

### Reference Example 227

### 4-(4-Methoxybutyl)-2,2-dimethyl-3-oxo-3,4-dihydro-2H-1,4-benzoxazine-6-carboxylic acid

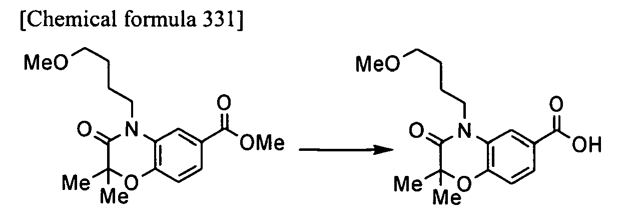

The compound of Reference Example 226 (2.0 g) was dissolved in tetrahydrofuran (5 ml) and methanol (5 ml), and thereto was added 2N aqueous sodium hydroxide solution (5 ml), and the mixture was stirred at 60°C for 5 hours. The reaction solution was concentrated under reduced pressure, and thereto was added a 5 % potassium hydrogen sulfate, and the mixture was extracted with ethyl acetate. The organic layer was washed with a saturated aqueous sodium chloride solution, dried over magnesium sulfate, filtered and concentrated under reduced pressure to give the title compound (1.81 g).
MS (ESI+) (M⁺+1, 100 %).

### Reference Example 228

### tert-Butyl (3R)-3-(isopropyl{[4-(4-methoxybutyl)-2,2-dimethyl-3-oxo-3,4-dihydro-2H-1,4-benzoxazin-6-yl]carbonyl}amino)piperidine-1-carboxylate

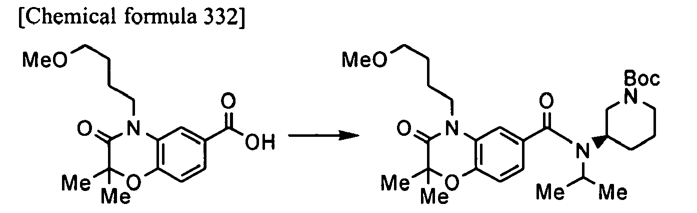

To a solution of the compound of Reference Example 227 (1.47 g) in dichloromethane (20 ml) were added oxalyl chloride (0.66 ml) and dimethylformamide (10 µl), and the mixture was stirred at room temperature for one hour. The solvent was removed by evaporation, and to the resultant was added toluene, and the mixture was concentrated under reduced pressure. The obtained residue was dissolved in dichloromethane (10 ml), and the mixture was added dropwise to a solution of tert-butyl (3R)-3-(isopropylamino)piperidine-1-carboxylate (1.51 g) and triethylamine (1.2 ml) in dichloromethane (20 ml), and the mixture was stirred at room temperature for 2 hours. Water was added to the reaction mixture, and the mixture was extracted with ethyl acetate. The organic layer was washed with a saturated aqueous sodium chloride solution, dried over magnesium sulfate, filtered, and concentrated under reduced pressure. The obtained residue was purified by silica gel column chromatography (developing solvent: hexane/ethyl acetate = 2/1) to give the title compound (2.0 g).
MS (ESI+) (M⁺+1, 100 %).

### Example 1

### N-Isopropyl-4-methoxy-3-(3-methoxypropoxy)-N-[(3R)-piperidin-3-yl]benzamide hydrochloride

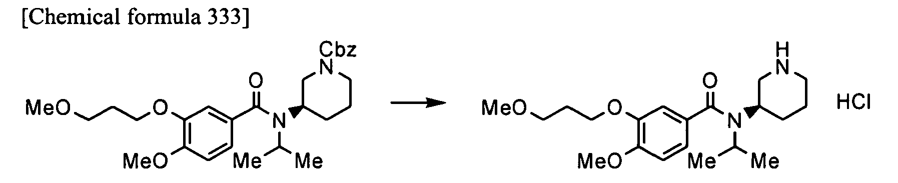

The compound of Reference Example 8 (79.6 mg) was dissolved in methanol (4 ml), and thereto was added a 10 % palladium carbon (80 mg), and the mixture was stirred at room temperature under hydrogen atmosphere for 3 hours. The mixture was filtered through celite, and chloroform was added thereto. The mixture was washed with a saturated aqueous sodium chloride solution, and the organic layer was dried over anhydrous sodium sulfate, and filtered. The filtrate was concentrated under reduced pressure, and the obtained residue was dissolved in THF (2 ml), and thereto was added a 4N hydrochloric acid-dioxane (2 ml). The mixture was concentrated to give the title compound (60.0 mg) as a colorless oily product.
¹H-NMR (400MHz, MeOD) 6.93(d, J = 8.3 Hz, 1H), 6.85-6.83(m, 2H), 3.99(t, J = 6.3 Hz, 2H), 3.92(brs, 1H), 3.77(s, 3H), 3.66-3.60(m, 1H), 3.58-3.55(m, 2H), 3.49-3.47(m, 3H), 3.29(m, 1H), 3.24(s, 3H), 2.87-2.81(m, 1H), 2.60(m, 1H), 2.03-1.91(m, 3H), 1.83-1.80(m, 2H), 1.16-1.15(m, 6H).
MS (ESI+) 365 (M⁺+1, 100 %)

### Example 2

### N-Isopropyl-4-methoxy-3-(3-methoxypropoxy)-N-[(3S)-piperidin-3-yl]benzamide

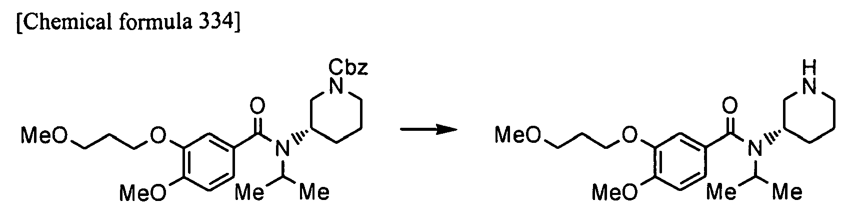

The compound of Reference Example 12 (10.8 mg) was dissolved in methanol (2 ml), and thereto was added a 10 % palladium carbon (8.4 mg), and the mixture was stirred at room temperature under hydrogen atmosphere for 2 hours. The mixture was filtered through celite, and the filtrate was concentrated under reduced pressure to give the title compound (9.3 mg) as a colorless oily product.
¹H-NMR (400MHz, CDCl₃) 6.90-6.83(m, 3H), 4.12(t, J = 6.5 Hz, 2H), 3.91-3.86(m, 1H), 3.87(s, 3H), 3.58-3.52(m, 3H), 3.35(s, 3H), 3.10-2.87(m, 3H), 2.40(brs, 2H), 2.13-2.07(m, 3H), 1.79-1.70(m, 3H), 1.26-1.19(m, 6H).
MS (ESI+) 365 (M⁺+1, 100 %)

### Example 3

### N-Isopropyl-4-methoxy-3-(3-methoxypropoxy)-N-pyrrolidin-3-ylbenzamide

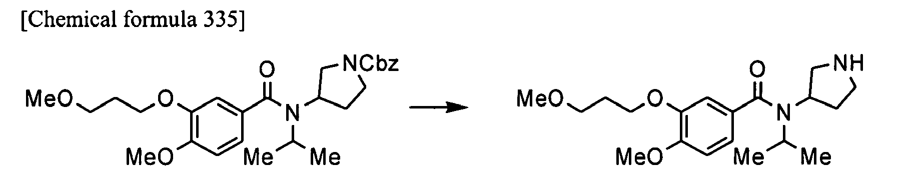

The compound of Reference Example 16 (124 mg) was dissolved in methanol (4 ml), and thereto was added a 10 % palladium carbon (18 mg), and the mixture was stirred at room temperature for 4 hours under hydrogen atmosphere. The mixture was filtered through celite, and the solvent was removed by evaporation. The residue was purified by preparative thin layer silica gel chromatography (developing solvent: chloroform/methanol = 10/1) to give the title compound (50.4 mg) as a colorless oily product.
¹H-NMR (300MHz, CDCl₃) 6.93-6.86(m, 3H), 4.27-4.11(m, 2H), 4.12(t, J = 6.6 Hz, 2H), 3.90(s, 3H), 3.81-3.75(m, 1H), 3.68-3.39(m, 3H), 3.57(t, J = 6.0 Hz, 2H), 3.35(s, 3H), 2.88(brs, 1H), 2.56-2.30(m, 2H), 2.16-2.07(m, 2H), 1.24-1.17(m, 6H).
MS (ESI+) 351 (M⁺+1, 100%).

### Example 4

### N-Azepan-3-yl-N-isopropyl-4-methoxy-3-(3-methoxypropoxy)benzamide hydrochloride

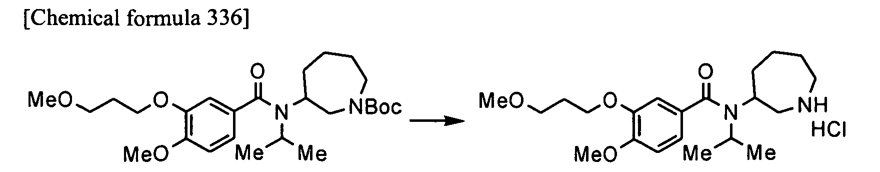

The compound of Reference Example 22 (378 mg) was dissolved in dioxane (4 ml), and thereto was added a 4N hydrochloric acid/dioxane (4 ml), and the mixture was stirred at 25°C for 2 hours. The reaction solution was concentrated under reduced pressure, and thereto was added toluene, and dioxane was completely removed by azeotropic distillation to give the title compound (164 mg) as a colorless oily product.
¹H-NMR (400MHz, CDCl₃) 6.95-6.86(m, 3H), 4.14(t, J = 6.7 Hz, 2H), 3.93-3.92(m, 1H), 3.90(s, 3H), 3.87-3.76(m, 2H), 3.59-3.56(t, J = 6.2 Hz, 2H), 3.47-3.45(m, 1H), 3.36-3.35(m, 1H), 3.35(s, 3H), 3.02(brs, 1H), 2.31-2.25(m, 1H), 2.16 -2.09(m, 5H), 2.01-1.98(m, 1H), 1.85-1.82(m, 1H), 1.60-1.56(m, 1H), 1.25(d, J = 6.6 Hz, 3H), 1.16(d, J = 6.6 Hz, 3H).
MS (ESI+) 379 (M⁺+1, 100 %).

### Example 5

### Ethyl 3-{isopropyl[4-methoxy-3-(3-methoxypropoxy)benzoyl]amino} piperidine-4-carboxylate hydrochloride

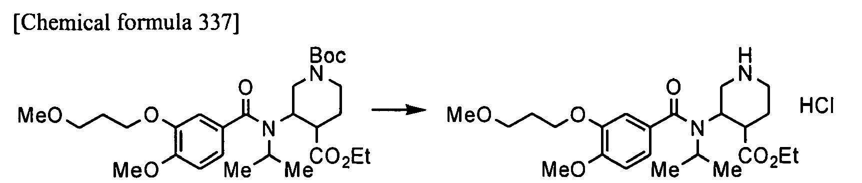

The title compound (13.3 mg) was synthesized in a similar manner to Example 4 except that the compound of Reference Example 26 was used instead of the compound of Reference Example 22 in Example 4.
¹H-NMR (400MHz, CDCl₃) 6.92-6.87(m, 3H), 4.19-4.12(m, 5H), 3.88(s, 3H), 3.79-3.76(m, 3H), 3.67-3.63(m, 3H), 3.58-3.52(m, 2H), 3.35(s, 3H), 3.00(m, 1H), 2.17-2.05(m, 2H), 1.69(m, 2H), 1.35-1.08(m, 9H).
MS (ESI+) 437 (M⁺+1, 100 %).

### Example 6

### 3-{Isopropyl[4-methoxy-3-(3-methoxypropoxy)benzoyl]amino piperidine-4-carboxylic acid hydrochloride

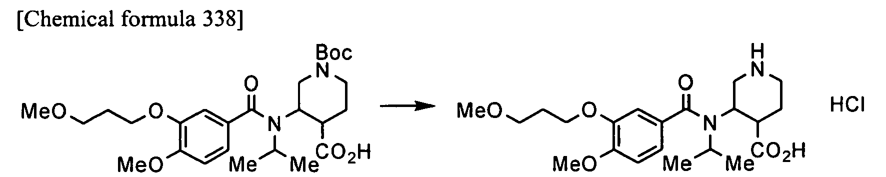

The title compound (19.5 mg) was synthesized in a similar manner to Example 4 except that the compound of Reference Example 27 was used instead of the compound of Reference Example 22 in Example 4.
¹H-NMR (400MHz, CDCl₃) 7.18-6.89(m, 3H), 4.42-4.15(m, 4H), 3.93-3.87(m, 2H), 3.87(s, 3H), 3.81-3.75(m, 2H), 3.71-3.62(m, 3H), 3.60-3.48(m, 1H), 3.48-3.36(m, 1H), 3.48(s, 3H), 2.38 -2.02(m, 5H), 1.03(s, 6H).
MS (ESI+) 409 (M⁺+1, 100 %).

### Example 7

### N-[5-(Fluoromethyl)-3-azabicyclo[3.1.1]hept-1-yl]-N-isopropyl-4-methoxy-3-(3-methoxypropoxy)benzamide

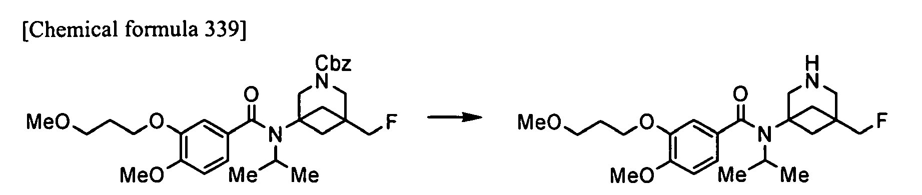

The title compound (9.1 mg) was synthesized in a similar manner to Example 3 except that the compound of Reference Example 33 was used instead of the compound of Reference Example 16 in Example 3.
¹H-NMR (400MHz, CDCl₃) 6.92(d, J = 1.8 Hz, 1H), 6.88(dd, J = 1.8, 8.1 Hz, 1H), 6.81(d, J = 8.1 Hz, 1H), 4.19(s, 1H), 4.12(t, J = 6.6 Hz, 1H), 4.07(s, 1H), 3.88(s, 3H), 3.68-3.65(m, 1H), 3.57(t, J = 6.1 Hz, 2H), 3.35(s, 3H), 3.28(s, 2H), 2.78(s, 2H), 2.14-2.02(m, 2H), 2.04-2.02 (m, 1H), 1.88(m, 3H), 1.39(d, J = 6.8 Hz, 6H).
MS (ESI+) 409 (M⁺+1, 100 %).

### Example 8

### N-[4-(Hydroxymethyl)piperidin-3-yl]-N-isopropyl-4-methoxy-3-(3-methoxypropoxy)benzamide hydrochloride

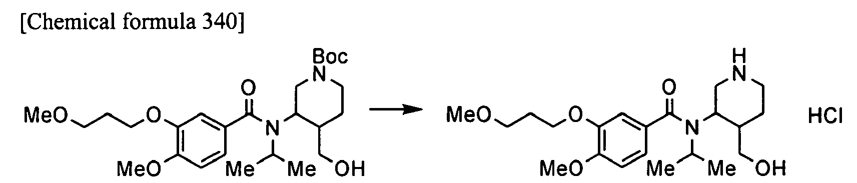

The title compound (21.5 mg) was synthesized in a similar manner to Example 4 except that the compound of Reference Example 28 was used instead of the compound of Reference Example 22 in Example 4.
¹H-NMR (400MHz, MeOD) 7.05-6.98(m, 3H), 4.12-3.98(m, 4H), 3.88(s, 3H), 3.76-3.65(m, 2H), 3.60-3.59(m, 2H), 3.49-3.40(m, 2H), 3.35(s, 3H), 3.06-3.03(m, 2H), 2.16(d, J = 14.6 Hz, 1H), 2.08-2.02(m, 2H), 1.76-1.72(m, 1H), 1.59-1.37(m, 1H), 1.29-1.19(m, 6H).
MS (ESI+) 395 (M⁺+1, 100 %).

### Example 9

### N-Isopropyl-4-methoxy-N-(4-{[(3-methoxybenzyl)oxy]methyl}piperidin-3-yl)-3-(3-methoxypropoxy)benzamide hydrochloride

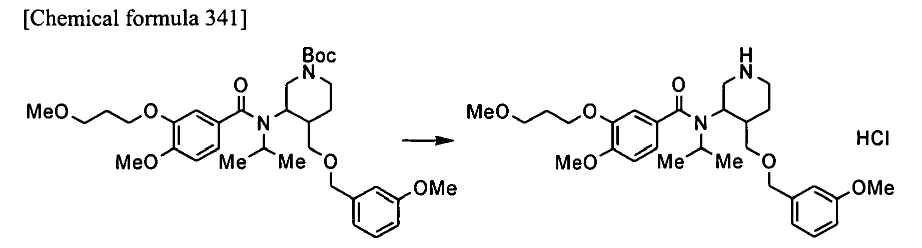

The title compound (13.7 mg) was synthesized in a similar manner to Example 4 except that the compound of Reference Example 29 was used instead of the compound of Reference Example 22 in Example 4.
¹H-NMR (400MHz, MeOD) 7.16-7.12(m, 1H), 6.87-6.76(m, 6H), 4.45(d, J = 11.8 Hz, 1H), 4.36(d, J = 11.8 Hz, 1H), 3.97-3.90(m, 4H), 3.77(s, 3H), 3.65(s, 3H), 3.58-3.55(m, 2H), 3.49-3.44(m, 2H), 3.41-3.35(m, 2H), 3.24(s, 3H), 3.11(m, 1H), 2.99-2.93(m, 1H), 2.06-2.03(m, 1H), 1.95-1.89(m, 2H), 1.76-1.74(m, 1H), 1.42-1.40(m, 1H), 1.12(d, J = 6.6 Hz, 3H), 1.07(d, J = 6.7 Hz, 3H).
MS (ESI+) 515 (M⁺+1, 100 %).

### Example 10

### N-(4-Chlorophenyl)-4-methoxy-3-(3-methoxypropoxy)-N-[3R-piperidin-3-yl]benzamide

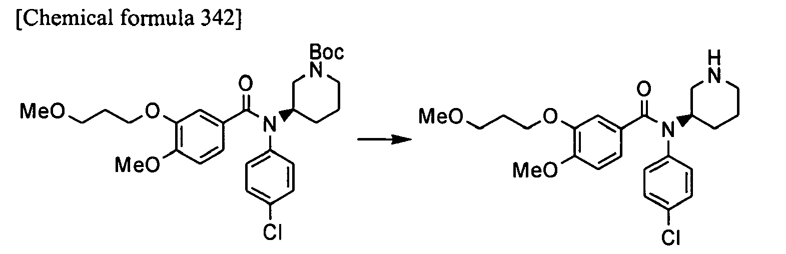

The compound of Reference Example 37 (17 mg) was dissolved in dioxane (2 ml), and thereto was added a 4N hydrochloric acid/dioxane (2 ml), and the mixture was stirred at 25°C for 2 hours. The reaction solution was concentrated under reduced pressure, and thereto was added a saturated aqueous sodium hydrogen carbonate solution. The mixture was extracted with chloroform, dried over sodium sulfate, filtered and concentrated under reduced pressure. The residue was purified by preparative thin layer silica gel chromatography (developing solvent: chloroform/ methanol = 10/1) to give the title compound (2.7 mg) as a colorless oily product.
¹H-NMR (300MHz, CDCl₃) 7.22(d, J = 8.7 Hz, 2H), 6.95(d, J = 8.7 Hz, 2H), 6.85-6.81(m, 2H), 6.62(d, J = 8.1 Hz, 1H), 4.65(m, 1H), 3.90(t, J = 6.3 Hz, 2H), 3.79(s, 3H), 3.51(t, J = 6.2 Hz, 2H), 3.36(m, 1H), 3.35(s, 3H), 3.03(brd, J = 12.9 Hz, 1H), 2.68-2.60(m, 1H), 2.45-2.37(m, 1H), 2.05-1.96(m, 3H), 1.96-1.41 (m, 4H).
MS (ESI+) 434 (M⁺+1, 100 %).

### Example 11

### N-((rac.)-(3R,6S)-6-{2-[(2-Amino-2-oxoethyl)amino]-2-oxoethyl}piperidin-3-yl)-N-isopropyl-4-methoxy-3-(3-methoxypropoxy)benzamide hydrochloride

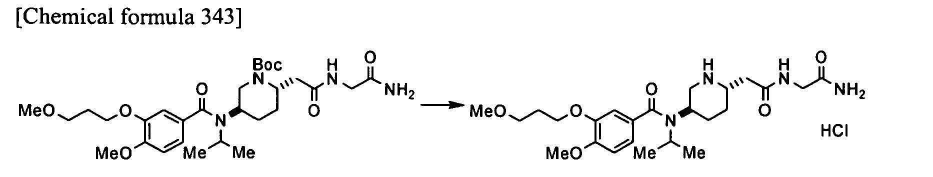

The title compound (18.9 mg) was synthesized in a similar manner to Example 4 except that the compound of Reference Example 82 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 479 (M⁺+1, 100 %).

### Example 12

### N-((rac.)-(3R,6S)-6-{2-[[2-(Dimethylamino)ethyl](ethyl)amino]-2-oxoethyl}piperidin-3-yl)-N-isopropyl-4-methoxy-3-(3-methoxypropoxy)benzamide dihydrochloride

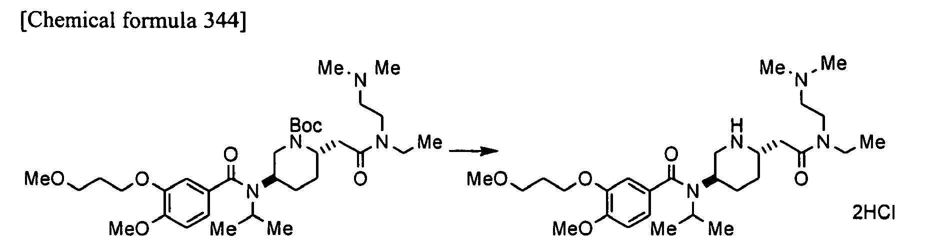

The title compound (16.7 mg) was synthesized in a similar manner to Example 4 except that the compound of Reference Example 83 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 521 (M⁺+1, 100 %).

### Example 13

### 3-Hydroxy-N-isopropyl-4-methoxy-N-[(3R)-piperidin-3-yl]benzamide hydrochloride

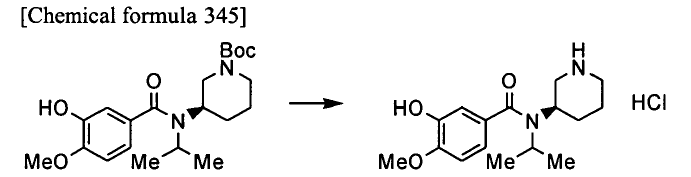

The title compound (15.0 mg) was synthesized in a similar manner to Example 4 except that the compound of Reference Example 77 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 293 (M⁺+1, 100%).

### Example 14

### Ethyl ((rac.)-(2R,SR)-5-{isopropyl[4-methoxy-3-(3-methoxypropoxy)benzoyl]amino}piperidin-2-yl)acetate

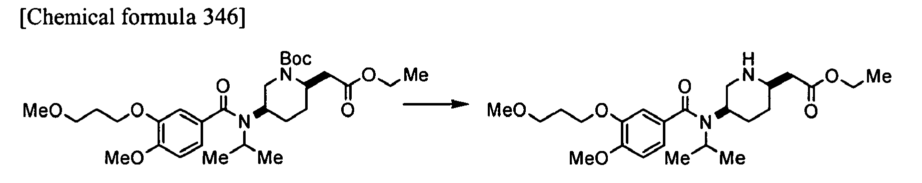

The title compound (7.5 mg) was synthesized in a similar manner to Example 10 except that the compound of Reference Example 80 was used instead of the compound of Reference Example 37 in Example 10.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 451(M⁺+1, 100 %).

### Example 15

### ((rac.)-(2R,5R)-5-{Isopropyl[4-methoxy-3-(3-methoxypropoxy)benzoyl]amino}piperidin-2-yl)acetic acid hydrochloride

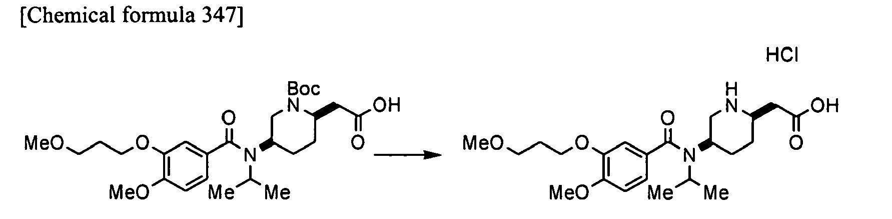

The title compound (5.0 mg) was synthesized in a similar manner to Example 4 except that the compound of Reference Example 81 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 423 (M⁺+1, 100%).

### Example 16

### ((rac.)-(2S,5R)-5-{Isopropyl[4-methoxy-3-(3-methoxypropoxy)benzoyl]amino}piperidin-2-yl)acetic acid hydrochloride

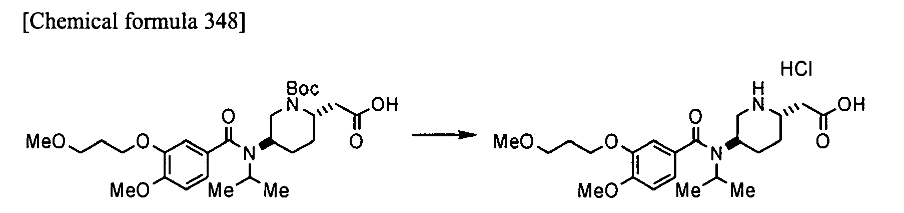

The title compound (5.0 mg) was synthesized in a similar manner to Example 4 except that the compound of Reference Example 72 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 423 (M⁺+1, 100%).

### Example 17

### N-[(rac.)-(3R,6S)-6-(2-Hydroxyethyl)piperidin-3-yl]-N-isopropyl-4-methoxy-3-(3-methoxypropoxy)benzamide hydrochloride

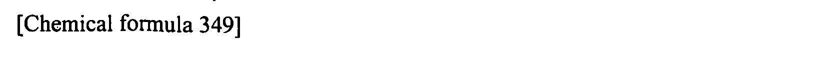

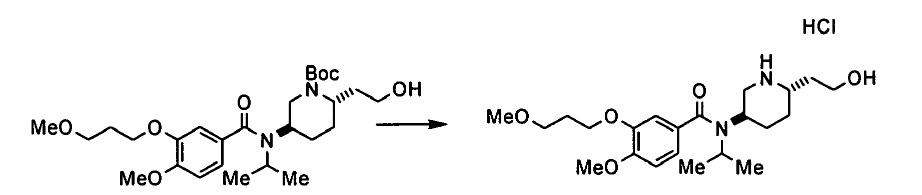

The title compound (2.5 mg) was synthesized in a similar manner to Example 4 except that the compound of Reference Example 87 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 409 (M⁺+1, 100 %).

### Example 18

### N-((rac.)-(3R,6S)-6-{2-[Ethyl(isopropyl)amino]-2-oxoethyl}piperidin-3-yl)-N-isopropyl-4-methoxy-3-(3-methoxypropoxy)benzamide hydrochloride

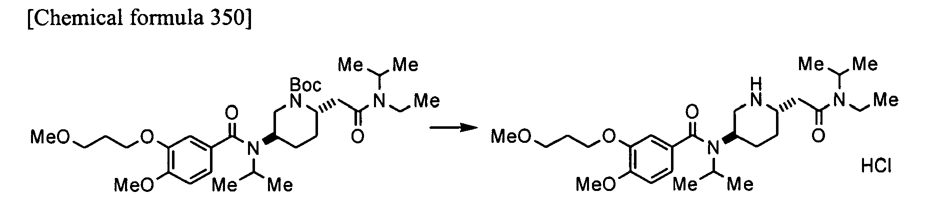

The title compound (14.8 mg) was synthesized in a similar manner to Example 4 except that the compound of Reference Example 84 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 492 (M⁺+1, 100 %).

### Example 19

### N-((rac.)-(3R,6S)-6-{2-[(Cyclopropylmethyl)(propyl)amino]-2-oxoethyl}piperidin-3-yl)-N-isopropyl-4-methoxy-3-(3-methoxypropoxy)benzamide hydrochloride

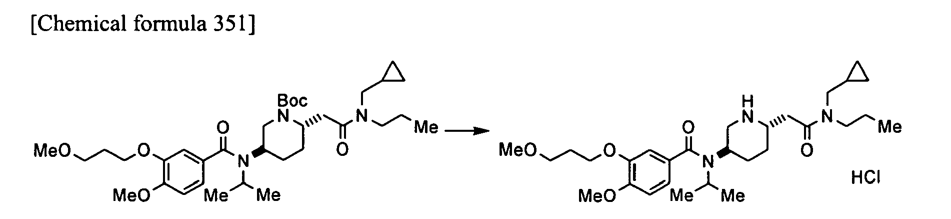

The title compound (4.5 mg) was synthesized in a similar manner to Example 4 except that the compound of Reference Example 86 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 518 (M⁺+1, 100%).

### Example 20

### N-((rac.)-(3R,6R)-6-{2-[(Cyclopropylmethyl)(propyl)amino]-2-oxoethyl}piperidin-3-yl)-N-isopropyl-4-methoxy-3-(3-methoxypropoxy)benzamide hydrochloride

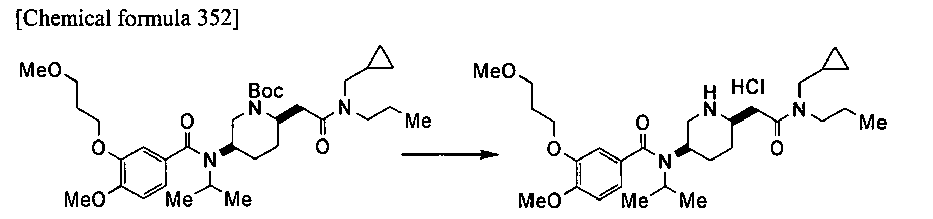

The title compound (15.7 mg) was synthesized in a similar manner to Example 4 except that the compound of Reference Example 107 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).

### Example 21

### 3-[2-(Acetylamino)ethoxy]-N-isopropyl-4-methoxy-N-[(3R)-piperidin-3-yl]benzamide hydrochloride

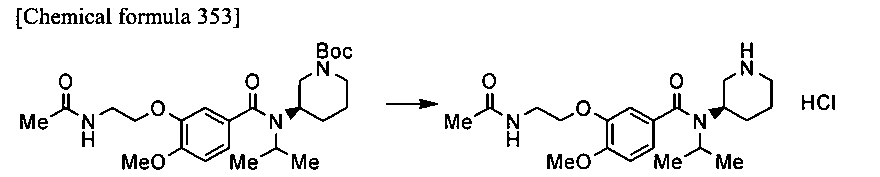

The title compound (11.7 mg) was synthesized in a similar manner to Example 4 except that the compound of Reference Example 78 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H). MS (ESI+) 378 (M⁺+1, 100%).

### Example 22

### N-((rac.)-(3R,6R)-6-{2-[Ethyl(isopropyl)amino]-2-oxoethyl}piperidin-3-yl)-N-isopropyl-4-methoxy-3-(3-methoxypropoxy)benzamide hydrochloride

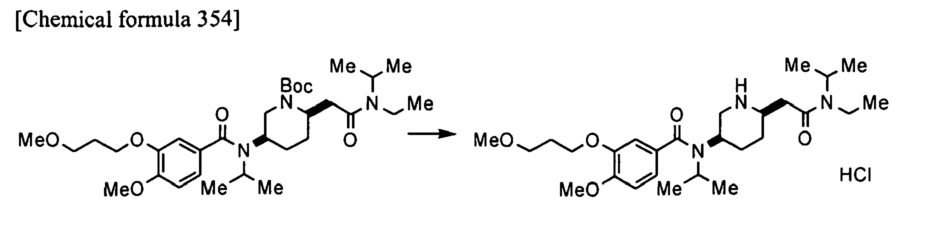

The title compound (3.7 mg) was synthesized in a similar manner to Example 4 except that the compound of Reference Example 85 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 492(M⁺+1, 100%).

### Example 23

### N-((rac.)-(3R,6S)-6-{2-[Cyclopropyl(phenylacetyl)amino]ethyl}piperidin-3-yl)-N-isopropyl-4-methoxy-3-(3-methoxypropoxy)benzamide hydrochloride

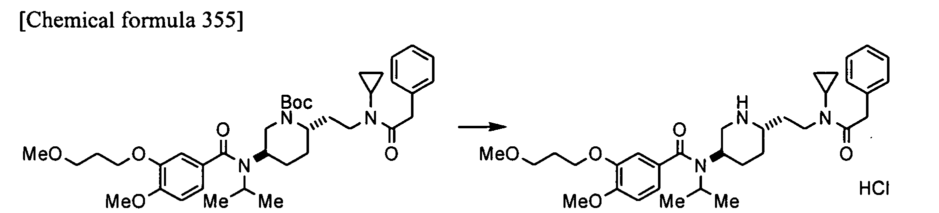

The title compound (3.7 mg) was synthesized in a similar manner to Example 4 except that the compound of Reference Example 93 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 492 (M⁺+1, 100 %).

### Example 24

### N-((rac.)-(3R,6R)-6-{2-[Cyclopropyl(phenylacetyl)amino]ethyl}piperidin-3-yl)-N-isopropyl-4-methoxy-3-(3-methoxypropoxy)benzamide hydrochloride

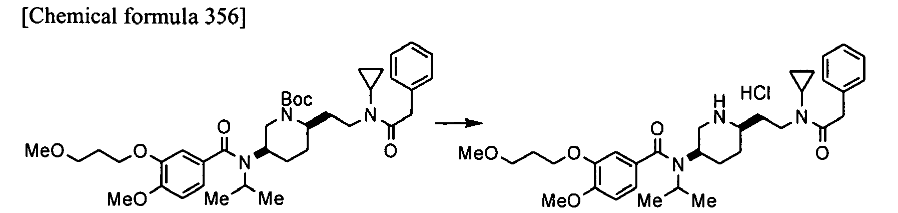

The title compound (19.4 mg) was synthesized in a similar manner to Example 4 except that the compound of Reference Example 94 was used instead of the compound of Reference Example 22 in Example 4.
¹H-NMR (400MHz, MeOD) 7.22-7.14(m, 5H), 6.94(d, J = 8.1 Hz, 1H), 6.88-6.86(m, 2H), 4.00(t, J = 6.3 Hz, 2H), 3.90(s, 2H), 3.78(s, 3H), 3.67-3.53(m, 5H), 3.51-3.40(m, 3H), 3.25(s, 3H), 3.09-3.04(m, 1H), 2.73-2.71 (m, 1H), 2.59(brs, 1H), 2.00-1.89(m, 6H), 1.72-1.69(m, 1H), 1.20-1.10(m, 6H), 0.93(d, J = 6.8 Hz, 2H), 0.83(brs, 2H).
MS (ESI+) 566 (M⁺+1, 100%).

### Example 25

### N-[(rac.)-(3R,5S)-5-(Hydroxymethyl)piperidin-3-yl]-N-isopropyl-4-methoxy-3-(3-methoxypropoxy)benzamide hydrochloride

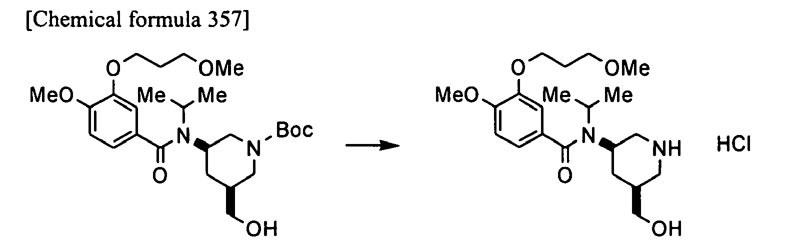

The title compound (3.7 mg) was synthesized in a similar manner to Example 4 except that that the compound of Reference Example 95 was used instead of the compound of Reference Example 22 in except that.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 492(M⁺+1, 100%).

### Example 26

### N-Cyclopropyl-4-methoxy-3-(3-methoxypropoxy)-N-[(3R)-piperidin-3-yl]benzamide hydrochloride

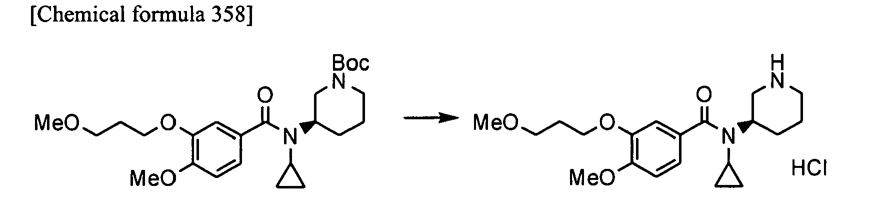

The title compound (101 mg) was synthesized in a similar manner to Example 4 except that the compound of Reference Example 97 was used instead of the compound of Reference Example 22 in Example 4.
¹H-NMR (400MHz, MeOD) 7.10-7.01 (m, 2H), 6.90(d, J = 8.3 Hz, 1H), 4.17-4.11(m, 1H), 3.99(t, J = 6.3 Hz, 2H), 3.78(s, 3H), 3.48(t, J = 6.2 Hz, 2H), 3.44-3.41(m, 1H), 3.37-3.24(m, 2H), 3.24(s, 3H), 2.92-2.88(m, 1H), 2.85-2.80(m, 1H), 2.26-2.19(m, 1H), 2.09-1.90(m, 4H), 1.78-1.75(m, 1H), 0.64-0.35(m, 2H), 0.45-0.35(m, 2H).
MS (ESI+) 363(M⁺+1, 100%).

### Example 27

### N-Isopropyl-4-methoxy-5-(3-methoxypropoxy)-2-methyl-N-[(3R)-piperidin-3-yl]benzamide

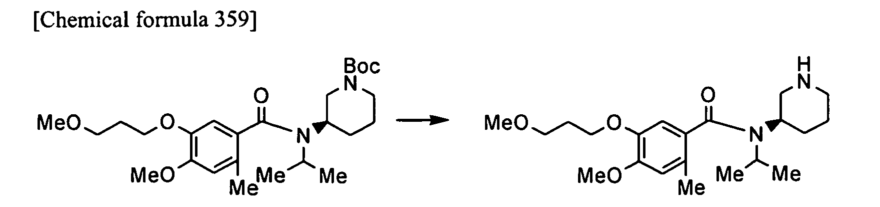

The title compound (254 mg) was synthesized in a similar manner to Example 10 except that the compound of Reference Example 106 was used instead of the compound of Reference Example 37 in Example 10.
¹H-NMR (400MHz, CDCl₃) 6.68(s, 1H), 6.63(s, 1H), 4.11-4.04(m, 2H), 3.93-383(m, 1H), 3.85(s, 3H), 3.75-3.70(m, 1H), 3.57-3.49(m, 2H), 3.34(s, 3H), 3.13(m, 1H), 2.99-2.85(m, 2H), 2.70-2.64(m, 1H), 2.23(s, 3H), 2.09-2.05(m, 2H), 1.80-1.54(m, 4H), 1.28-1.07(m, 6H).
MS (ESI+) 379(M⁺+1, 100%).

### Example 28

### N-[(rac.)-(3R,6R)-6-(2-Hydroxyethyl)piperidin-3-yl]-N-isopropyl-4-methoxy-3-(3-methoxypropoxy)benzamide hydrochloride

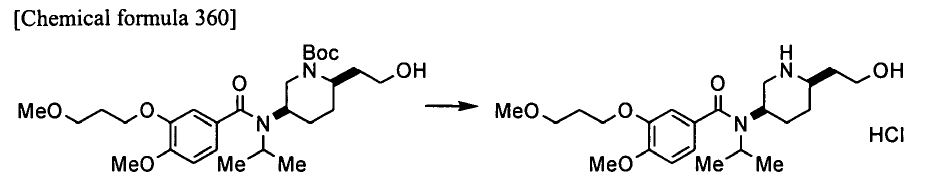

The title compound (17.3 mg) was synthesized in a similar manner to Example 4 except that the compound of Reference Example 88 was used instead of the compound of Reference Example 22 in Example 4.
¹H-NMR (400MHz, MeOD) 6.93(d, J = 8.0 Hz, 1H), 6.88-6.85(m, 2H), 4.01-3.95(m, 4H), 3.77(s, 3H), 3.75-3.71(m, 1H), 3.67-3.55(m 3H), 3.48(t, J = 6.1 Hz, 2H), 3.24(s, 3H), 3.13(dd, J = 5.2, 12.8 Hz, 1H), 2.60(brs, 1H), 2.08-2.02(m, 1H), 1.97-1.91(m, 4H), 1.87-1.73(m, 2H), 1.19-1.11(m, 6H).
MS (ESI+) 409(M⁺+1, 100%).

### Example 29

### N-Isopropyl-4-methoxy-3-(3-methoxypropoxy)-N-[(rac.)-(3R,5S)-5-(phenoxymethyl)piperidin-3-yl]benzamide hydrochloride

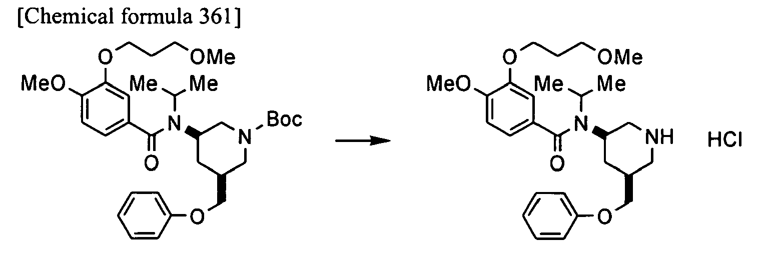

The title compound (41.8 mg) was synthesized in a similar manner to Example 4 except that the compound of Reference Example 103 was used instead of the compound of Reference Example 22 in Example 4.
¹H-NMR (300MHz, CDCl₃) 7.29-7.27(m, 3H), 6.98-6.84(m, 6H), 4.13-4.11(m, 2H), 4.04-3.43(m, 7H), 3.87(s, 3H), 3.58-3.54(m, 2H), 3.34(s, 3H), 2.88-2.84(m, 3H), 2.14-2.08(m, 2H), 1.93-1.90(m, 1H), 1.32-1.22(m, 6H).
MS (ESI+) 471(M⁺+1, 100%).

### Example 30

### N-{(rac.)-(3R,5S)-5-[(Benzyloxy)methyl]piperidin-3-yl}-N-isopropyl-4-methoxy-3-(3-methoxypropoxy)benzamide hydrochloride

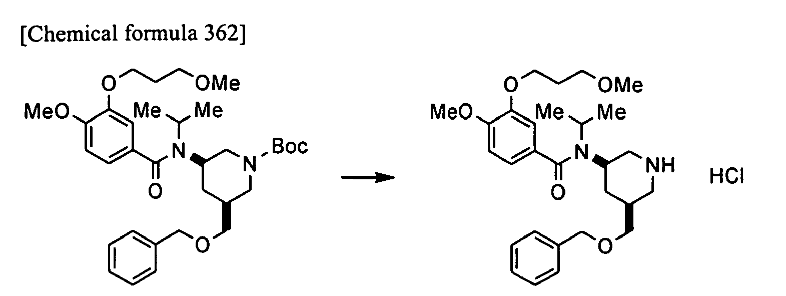

The title compound (34.5 mg) was synthesized in a similar manner to Example 4 except that the compound of Reference Example 104 was used instead of the compound of Reference Example 22 in Example 4.
¹H-NMR (300MHz, CDCl₃) 7.37-7.27(m, 5H), 6.88-6.85(m, 3H), 4.47(s, 2H), 4.13-4.09(m, 2H), 4.02-3.34(m, 7H), 3.87(s, 3H), 3.58-3.54(m, 2H), 3.34(s, 3H), 2.71-2.50(m, 3H), 2.14-2.06(m, 2H), 1.81-1.78(m, 1H), 1.27-1.17(m, 6H).
MS (ESI+) 485(M⁺+1, 100%).

### Example 31

### ((rac.)-(3S,5R)-5-{Isopropyl[4-methoxy-3-(3-methoxypropoxy)benzoyl]amino}piperidin-3-yl)methyl(2-phenylethyl)carbamate hydrochloride

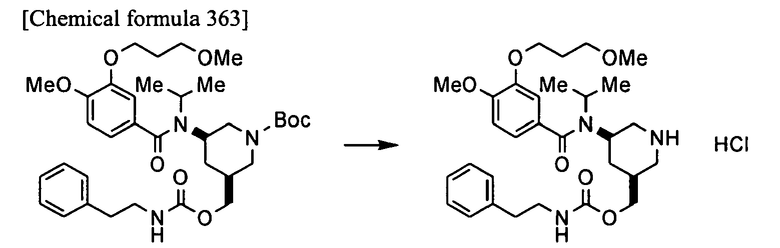

The title compound (41.0 mg) was synthesized in a similar manner to Example 4 except that the compound of Reference Example 105 was used instead of the compound of Reference Example 22 in Example 4.
¹H-NMR (300MHz, CDCl₃) 7.32-7.16(m, 5H), 6.89-6.86(m, 3H), 5.61 (bs, 1H), 4.13-4.08(m, 4H), 3.93-3.37(m, 7H), 3.87(s, 3H), 3.58-3.53(m, 2H), 3.34(s, 3H), 2.80-2.78(m, 2H), 2.70-2.60(m, 3H), 2.12-2.08(m, 2H), 1.78-1.76(m, 1H), 1.26-1.19(m, 6H).
MS (ESI+) 542(M⁺+1, 100%).

### Examples 32 - 38

The compounds as listed in the following Table were obtained in a similar manner to Example 4 except that the compounds of Reference Example 51, Reference Example 53, Reference Example 55, Reference Example 57, and Reference Examples 58 - 60 were used instead of the compound of Reference Example 22 in Example 4 respectively.

**[Table 7]**

| Ex. No. | Structure | 1H-NMR(400MHz, solvent)/MS(ESI+) |
|---|---|---|
| 32 | 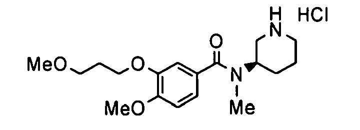 | ¹H-NMR(400MHz, MeOD) 7.05(m, 3H), 4.12-4.06(m, 2H), 3.89(s, 3H), 3.61-3.58(m, 2H), 3.40(m, 1H), 3.35-3.31 (m, 5H), 3.26-3.21(m, 1H), 2.99(m, 4H), 2.08-2.02(m, 6H). MS(ESI+) 337(M⁺+1, 100%). |
| 33 | 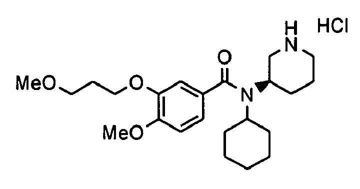 | ¹H-NMR(400MHz, CDCl₃) 6.90-6.83(m, 3H). 4.11(t, J = 6.5 Hz, 2H), 3.89(s,3H), 3.56(t, J = 6.1 Hz, 2H), 3.49-3.40(m, 2H), 3.35(s, 3H), 3.11-3.00(m, 3H), 2.66(m, 2H), 2.14-2.07(m, 2H), 1.84-1.56(m, 10H), 1.33-1.10(m, 2H), 0.90-0.84(m, 2H). MS(ESI+) 405(M⁺+1, 100%). |
| 34 | 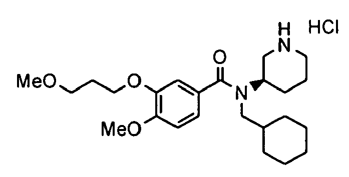 | ¹H-NMR(400MHz, CDCl₃) 6.95-6.92(m, 2H), 6.84(d, J = 8.1 Hz, 1H), 4.12(t, J = 6.5 Hz, 2H), 3.88(s, 3H), 3.81-3.74(m, 1H), 3.56(t, J = 6.1 Hz, 2H), 3.50(s, 3H), 3.21-2.71(m, 5H), 2.47(m, 2H), 2.13-2.07(m, 2H), 1.93-1.69(m, 9H), 1.41-1.16(m, 4H), 0.88(m, 2H). MS(ESI+) 419(M⁺+1, 100%). |
| 35 | 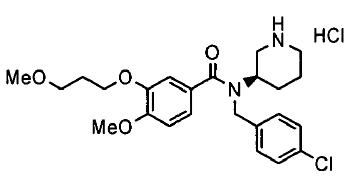 | ¹H-NMR(400MHz, DMSO-d₆) 9.34-9.32(m, 1H), 7.41-7.39(m, 2H), 7.33-7.31(m, 2H), 7.00(m, 3H), 4.59(brs, 2H), 4.18(brs, 1H), 3.95-3.83(m, 2H), 3.78(s, 3H), 3.49-3.44(m, 2H), 3.24(s, 3H), 3.22-3.16(m, 1H), 3.07-3.06(m, 2H), 2.71-2.68(m, 1H), 1.97-1.79(m, 5H), 1.53-1.52(m, 1H). MS(ESI+) 447(M⁺+1, 100%). |
| 36 | 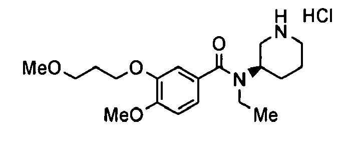 | ¹H-NMR(400MHz, DMSO-d₆) 7.01-6.98(m, 1H), 6.93-6.89(m, 2H), 4.05-3.99(m,3H), 3.79(s, 3H), 3.49-3.45(m, 2H), 3.33-3.25(m, 2H), 3.24(s, 3H), 3.19-3.16(m, 3H), 2.80-2.77(m, 1H), 2.12-1.90(m, 2H), 1.84(m, 3)H), 1.60-1.56(m, 1H), 1.07-1.05(m, 3H). MS(ESI+) 451(M⁺+1, 100%). |
| 37 | 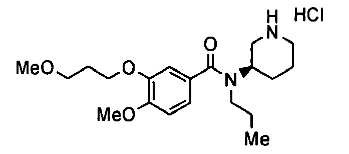 | ¹H-NMR(400MHz, CDCl₃) 7.04-7.02(m, 1H), 6.98-6.96(m, 2H), 4.09(t, J = 6.3 Hz, 2H), 3.87(s, 3H), 3.75-3.64(m, 5H), 3.58(t, J = 6.2 Hz, 2H), 3.40(brs, 1H), 3.34(s, 3H), 2.97-2.90(m, 1H), 2.19-2.01(m, 4H), 1.63-1.61(m, 3H), 1.33-1.29(m, 1H), 0.90-0.83(m, 3H). |
| 38 | 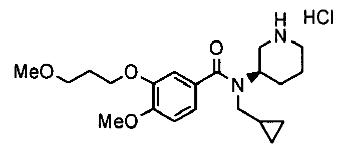 | ¹H-NMR(400MHz, CDCl₃) 6.95-6.88(m, 3H), 4.00-3.98(m, 3H), 3.77(s, 3H), 3.65-3.54(m, 3H), 3.48(t, J = 6.1 Hz, 2H), 3.39(brs, 1H), 3.24(s, 3H), 3.24(m, 1H), 1.99-1.91(m, 4H), 1.69(brs, 1H), 1.23-1.19(m, 1H), 0.93(brs, 1H), 0.45(d, J = 7.6 Hz, 2H), 0.08(brs, 2H). |

### Example 39

### N-((rac.)-(3R,6S)-6-{2-[Butylamino]-2-oxoethyl}piperidin-3-yl)-N-isopropyl-4-methoxy-3-(3-methoxypropoxy)benzamide hydrochloride

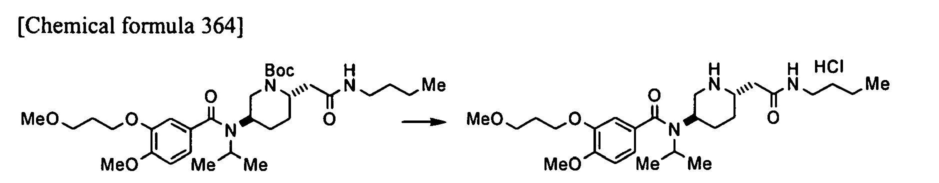

The title compound (14.8 mg) was synthesized in a similar manner to Example 4 except that the compound of Reference Example 62 was used instead of the compound of Reference Example 22 in Example 4.
¹H-NMR (400MHz, CDCl₃) 7.00-6.84(m, 3H), 4.13-4.06(m, 3H), 3.93-3.91(m, 1H), 3.88(s, 3H), 3.70-3.61 (m, 1H), 3.56(t, J = 6.1 Hz, 2H), 3.35(s, 3H), 3.29-3.12(m, 4H), 2.63-2.50(m, 4H), 2.14-2.08(m, 2H), 1.88-1.79(m, 2H), 1.51-1.44(m, 2H), 1.38-1.26(m, 8H), 0.94-0.84(m, 4H).
MS (ESI+) 478(M⁺+1, 100%).

### Example 40

### Methyl (rac.)-(3R,5R)-5-{isopropyl[4-methoxy-3-(methoxypropoxy)benzoyl]amino}piperidine-3-carboxylate hydrochloride

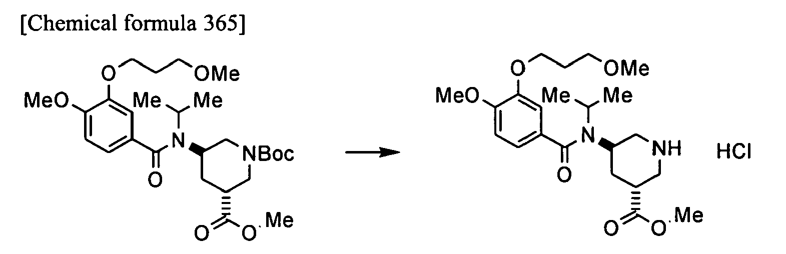

The title compound (17.9 mg) was synthesized in a similar manner to Example 4 except that the compound of Reference Example 70 was used instead of the compound of Reference Example 22 in Example 4.
¹H NMR (400 MHz, DMSO-d₆) δ 9.61-9.58(m, 1H), 8.93-8.90(m, 1H), 7.00-6.98(m, 1H), 6.92-6.86(m, 2H), 4.01(t, J=6.6 Hz, 2H), 3.84-3.73(m, 7H), 3.58-3.55(m, 1H), 3.46(t, J=6.2 Hz, 2H), 3.34(s, 3H), 3.24(s, 3H), 3.11-3.08(m, 2H), 2.90-2.80(m, 1H), 2.16-2.13(m, 1H), 1.97-1.93(m, 2H), 1.04-1.01(m, 6H).
MS (ESI+) 423([M+H]⁺, 69%).

### Example 41

### (rac.)-(3S,5R)-N-Benzyl-5-(isopropyl[4-methoxy-3-(3-methoxypropoxy)benzoyl]amino}-N-methylpiperidine-3-carboxamide hydrochloride

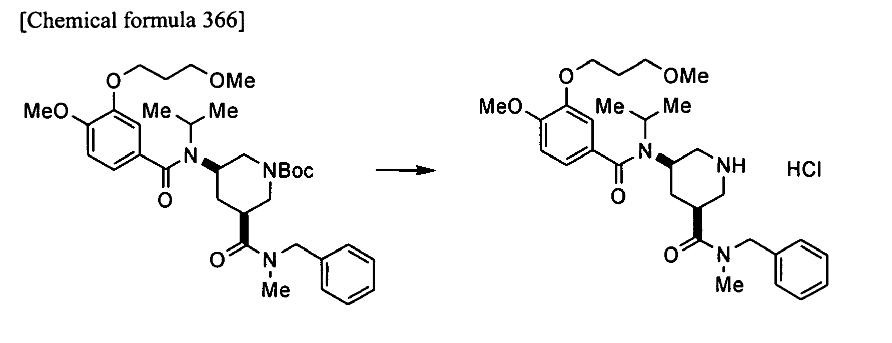

The title compound (30.0 mg) was synthesized in a similar manner to Example 4 except that the compound of Reference Example 66 was used instead of the compound of Reference Example 22 in Example 4.
¹H NMR (400 MHz, DMSO-d₆) δ 9.35-9.30(m, 2H), 7.42-7.22(m, 5H), 7.00-6.87(m, 3H), 4.67-4.63(m, 1H), 4.52-4.49(m, 1H), 4.02-3.98(m, 2H), 3.82-3.77(m, 6H), 3.46(t, J=6.3 Hz, 2H), 3.34(s, 3H), 3.23(s, 3H), 3.19-3.17(m, 1H), 3.05-2.95(m, 2H), 2.90-2.87(m, 2H), 2.80-2.75(m, 1H), 1.97-1.91(m, 2H), 1.23-0.93(m, 6H).
MS (ESI+) 512([M+H]⁺, 100%).

### Example 42

### N-Isopropyl-4-methoxy-N-((rac.)-(3R,5S)-5-{[(3-methoxybenzoyl)amino]methyl}piperidin-3-yl)-3-(methoxypropoxy)benzamide hydrochloride

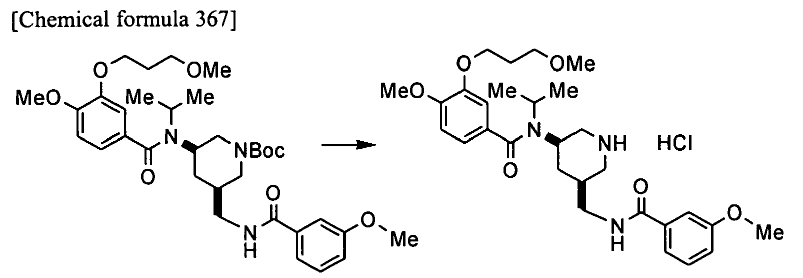

The title compound (61.5 mg) was synthesized in a similar manner to Example 4 except that the compound of Reference Example 130 was used instead of the compound of Reference Example 22 in Example 4.
¹H-NMR (300MHz, CDCl₃) δ 7.49-7.44(m, 3H), 6.99-6.96(m, 1H), 6.86-6.82(m, 3H), 4.07-3.97(m, 2H), 3.86(s, 3H), 3.81(s, 3H), 3.81-3.61(m, 6H), 3.57-3.52(m, 2H), 3.33(s, 3H), 2.60-2.54(m, 4H), 2.09-2.07(m, 2H), 1.83-1.81(m, 1H), 1.23-1.12(m, 6H).
MS (ESI+) 528(M⁺+1, 100%).

### Example 43

### Benzyl [((rac.)-(3S,5R)-5-{isopropyl[4-methoxy-3-(3-methoxypropoxy)benzoyl]amino}piperidin-3-yl)methyl]carbamate hydrochloride

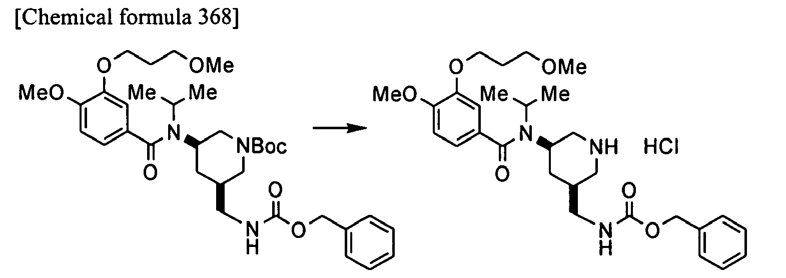

The title compound (40.8 mg) was synthesized in a similar manner to Example 4 except that the compound of Reference Example 131 was used instead of the compound of Reference Example 22 in Example 4.
¹H-NMR (300MHz, CDCl₃) δ 7.34-7.32(m, 5H), 6.89-6.85(m, 3H), 5.09(s, 2H), 4.10-4.02(m, 2H), 3.87(s, 3H), 3.79-3.62(m, 4H), 3.58-3.53(m, 2H), 3.34(s, 3H), 3.20-3.10(m, 2H), 2.58-2.17(m, 4H), 2.10-2.07(m, 2H), 1.78-1.76(m, 1H), 1.26-1.16(m, 6H).
MS (ESI+) 528(M⁺+1, 100%).

### Example 44

### N-Isopropyl-4-methoxy-3-(3-methoxypropoxy)-N-((rac.)-(3R,5S)-5-{[(3-methyl-butanoyl)amino]methyl}piperidin-3-yl)benzamide hydrochloride

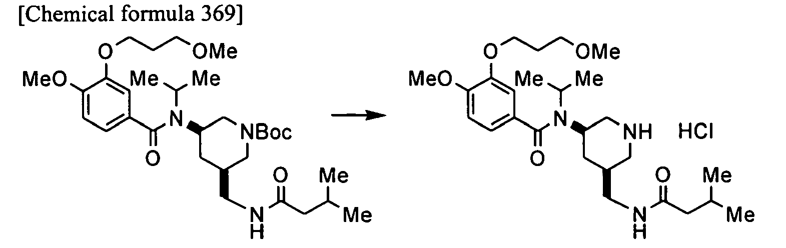

The title compound (28.3 mg) was synthesized in a similar manner to Example 4 except that the compound of Reference Example 129 was used instead of the compound of Reference Example 22 in Example 4.
¹H NMR (300 MHz, CDCl₃) δ 6.88-6.85(m, 3H), 4.11-4.02(m, 2H), 3.87(s, 3H), 3.80-3.62(m, 6H), 3.58-3.54(m, 2H), 3.34(s, 3H), 2.59-2.55(m, 3H), 2.20-2.10(m, 6H), 1.86-1.83(m, 1H), 1.26-1.18(m, 6H), 0.96-0.92(m, 6H).
MS (ESI+) 478(M⁺+1, 100%).

### Example 45

### N-Isopropyl-4-methoxy-3-(3-methoxypropoxy)-N-[(rac.)-(3R,4R)-4-phenylpiperidin-3-yl]benzamide hydrochloride

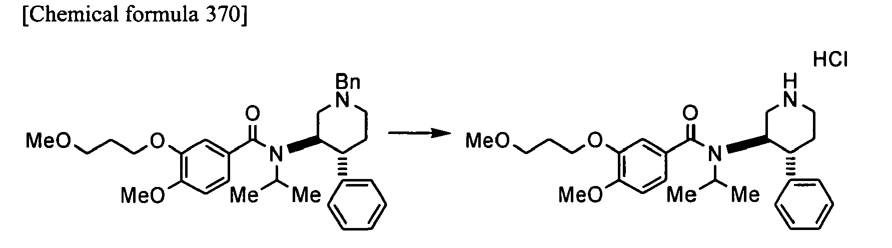

The title compound (108 mg) was synthesized in a similar manner to Example 1 except that the compound of Reference Example 129 was used instead of the compound of Reference Example 22 in Example 1.
MS (ESI+) 441(M⁺+1, 100%).

### Example 46

### N-{(rae.)-(3R,5S)-5-[(Biphenyl-2-yloxy)methyl]piperidin-3-yl}-N-isopropyl-4-methoxy-3-(3-methoxypropoxy)benzamide hydrochloride

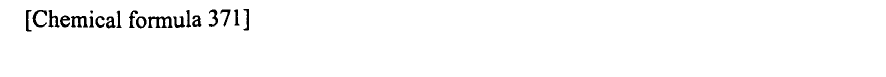

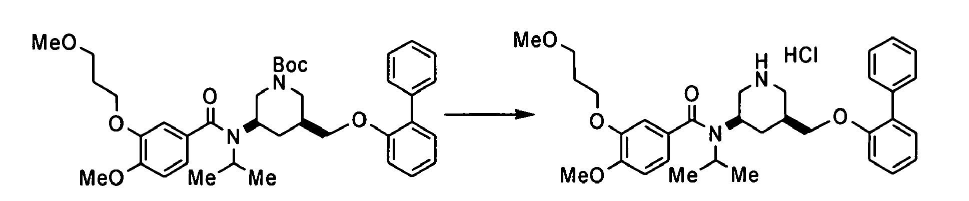

The title compound (28.3 mg) was synthesized in a similar manner to Example 4 except that the compound of Reference Example 120 was used instead of the compound of Reference Example 22 in Example 4.
¹H NMR (300 MHz, CDCl₃) δ 7.50-7.39(m, 4H), 7.35-7.26(m, 3H), 7.09-7.03(m, 1H), 6.91-6.85(m, 4H), 4.13-4.09(m, 2H), 4.01(s, 3H), 3.87-3.54(m, 8H), 3.34(s, 3H), 2.65-2.60(m, 3H), 2.15-2.08(m, 2H), 1.87-1.84(m, 2H), 1.24-1.12(m, 6H).
MS (ESI+) 547(M⁺+1, 100%).

### Example 47

### N-((rac.)-(3R,5S)-5-{[2-(Benzoyloxy)phenoxy]methyl}piperidin-3-yl)-N-isopropyl-4-methoxy-3-(3-methoxypropoxy)benzamide hydrochloride

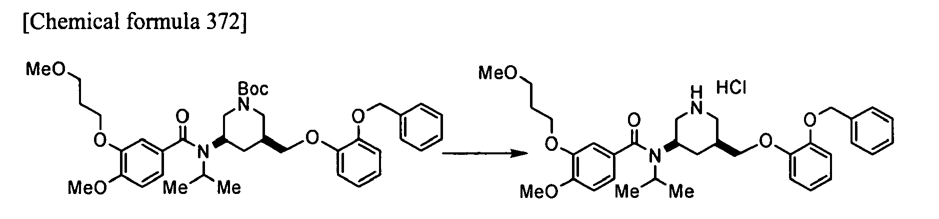

The title compound (42.4 mg) was synthesized in a similar manner to Example 4 except that the compound of Reference Example 121 was used instead of the compound of Reference Example 22 in Example 4.
¹H NMR (300 MHz, CDCl₃) δ 7.42-7.37(m, 3H), 7.31-7.29(m, 2H),6.91-6.84(m, 7H), 5.08(s, 2H), 4.10-4.08(m, 2H), 3.87(s, 3H), 3.79-3.62(m, 6H), 3.57-3.53(m, 2H), 3.34(s, 3H), 2.78-2.74(m, 3H), 2.12-2.07(m, 2H), 1.74-1.70(m, 2H), 1.28-1.16(m, 6H).
MS (ESI+) 577(M⁺+1, 100%).

### Example 48

### N-{(rac.)-(3R,5S)-5-[(Biphenyl-3-yloxy)methyl]piperidin-3-yl}-N-isopropyl-4-methoxy-3-(3-methoxypropoxy)benzamide hydrochloride

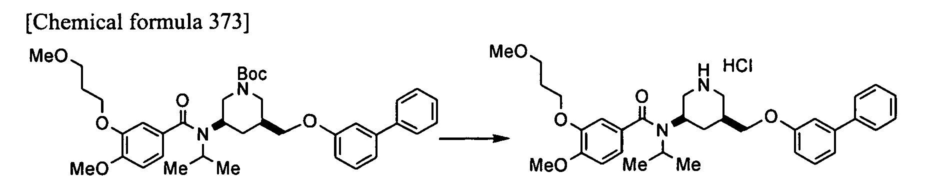

The title compound (34.8 mg) was synthesized in a similar manner to Example 4 except that the compound of Reference Example 122 was used instead of the compound of Reference Example 22 in Example 4.
¹H NMR (300 MHz, CDCl₃) δ 7.61-7.55(m, 2H), 7.45-7.26(m, 4H), 7.20-7.09(m, 2H), 6.89-6.84(m, 4H), 4.23-4.11(m, 2H), 3.91 (s, 3H), 3.86-3.62(m, 6H), 3.57-3.53(m, 2H), 3.33(s, 3H), 2.93-2.87(m, 2H), 2.11-2.09(m, 2H), 1.96-1.90(m, 2H), 1.32-1.22(m, 6H).
MS (ESI+) 547(M⁺+1, 100%).

### Example 49

### N-Isopropyl-4-methoxy-3-(3-methoxypropoxy)-N-{(rac.)-(3R,5S)-5-[(3-phenoxy-phenoxy)methyl]piperidin-3-yl}benzamide hydrochloride

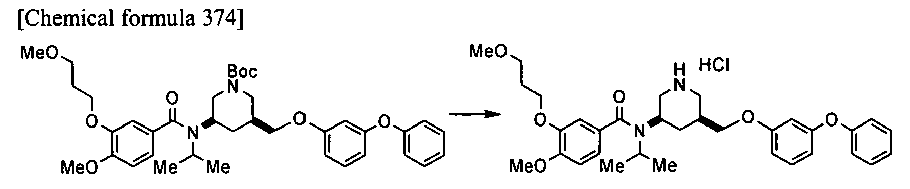

The title compound (44.5 mg) was synthesized in a similar manner to Example 4 except that the compound of Reference Example 123 was used instead of the compound of Reference Example 22 in Example 4.
¹H NMR (300 MHz, CDCl₃) δ 7.36-7.00(m, 6H), 6.89-6.85(m, 3H), 6.61-6.50(m, 3H), 4.13-4.09(m, 2H), 3.87(s, 3H), 3.80-3.62(m, 6H), 3.58-3.53(m, 2H), 3.34(s, 3H), 2.85-2.81(m, 3H), 2.12-2.08(m, 2H), 1.88-1.86(m, 2H), 1.30-1.20(m, 6H).
MS (ESI+) 563(M⁺+1, 100%).

### Example 50

### N-((rac.)-(3R,5S)-5-{[3-(Benzoyloxy)phenoxy]methyl}piperidin-3-yl)-N-isopropyl-4-methoxy-3-(3-methoxypropoxy)benzamide hydrochloride

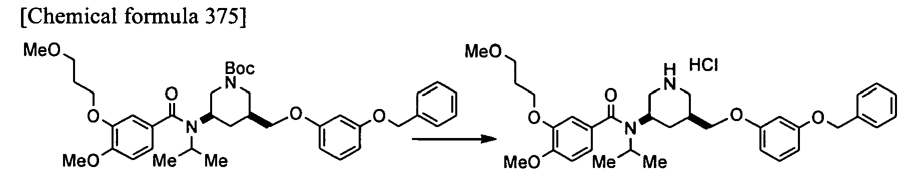

The title compound (54.3 mg) was synthesized in a similar manner to Example 4 except that the compound of Reference Example 124 was used instead of the compound of Reference Example 22 in Example 4.
¹H NMR (300 MHz, CDCl₃) δ 7.44-7.29(m, 5H), 7.18-7.12(m, 1H), 6.89-6.84(m, 3H), 6.59-6.45(m, 3H), 5.03(s, 2H), 4.13-4.09(m, 2H), 3.86(s, 3H), 3.79-3.61(m, 6H), 3.57-3.53(m, 2H), 3.34(s, 3H), 2.89-2.82(m, 3H), 2.12-2.08(m, 2H), 1.94-1.90(m, 2H), 1.31-1.21(m, 6H).
MS (ESI+) 577(M⁺+1, 100%).

### Example 51

### N-((rac.)-(3R,6R)-6-{2-[Cyclohexyl(isopropyl)amino]-2-oxoethyl}piperidin-3-yl)-N-isopropyl-4-methoxy-3-(3-methoxypropoxy)benzamide hydrochloride

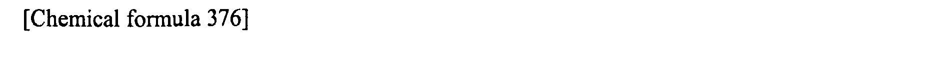

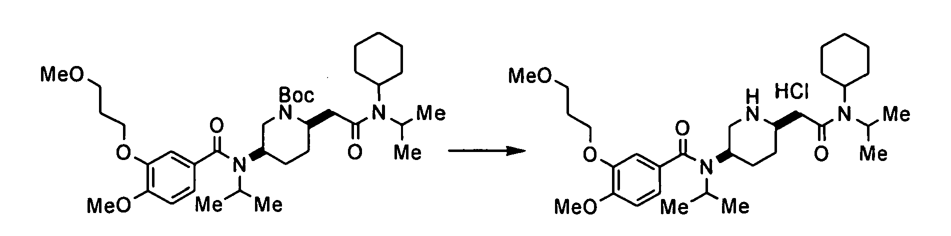

The title compound (9.0 mg) was synthesized in a similar manner to Example 4 except that the compound of Reference Example 108 was used instead of the compound of Reference Example 22 in Example 4.
¹H-NMR (400MHz, MeOD) 6.94(d, J = 8.2 Hz, 1H), 6.88-6.86(m, 2H), 4.00(m,2H), 3.88(m, 1H), 3.78(s, 3H), 3.74-3.71(m, 1H), 3.66-3.55(m, 4H), 3.49(d, J = 6.1 Hz, 2H), 3.25(s, 3H), 3.16-3.12(m, 1H), 2.96-2.87(m, 1H), 2.83-2.78(m, 1H), 2.60(brs, 1H), 2.32(brs, 1H), 1.98-1.92(m, 4H), 1.76-1.70(m, 4H), 1.54-1.36(m, 4H), 1.31-1.26(m, 4H), 1.23-1.05(m, 10H).
MS (ESI+) 546(M⁺+1, 100%).

### Example 52

### N-{(rac.)-(3R,6R)-6-[2-(Cyclopropylamino)-2-oxoethyl]piperidin-3-yl)-N-isopropyl-4-methoxy-3-(3-methoxypropoxy)benzamide hydrochloride

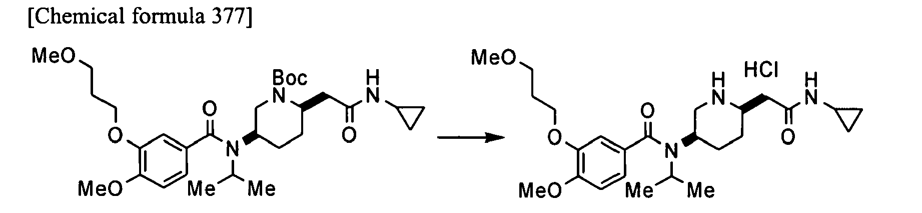

The title compound (30.8 mg) was synthesized in a similar manner to Example 4 except that the compound of Reference Example 109 was used instead of the compound of Reference Example 22 in Example 4.
¹H-NMR (400MHz, MeOD) 6.94(d, J = 7.9 Hz, 1H), 6.89-6.86(m, 2H), 4.00(d,J = 6.3 Hz, 2H), 3.96(m, 1H), 3.78(s, 3H), 3.66-3.55(m, 3H), 3.49(d, J = 6.1 Hz, 2H), 3.25(s, 3H), 3.16(dd, J = 5.2, 13.0 Hz, 1H), 2.78-2.72(m, 1H), 2.63-2.53(m, 3H), 1.93-1.75(m, 5H), 1.20-1.11(m, 6H), 0.66-0.62(m, 2H), 0.44-0.40(m, 2H).
MS (ESI+) 462(M⁺+1, 100%).

### Example 53

### 2-Chloro-N-isopropyl-methoxy-5-(3-methoxypropoxy)-N-[(3R)-piperidin-3-yl]benzamide hydrochloride

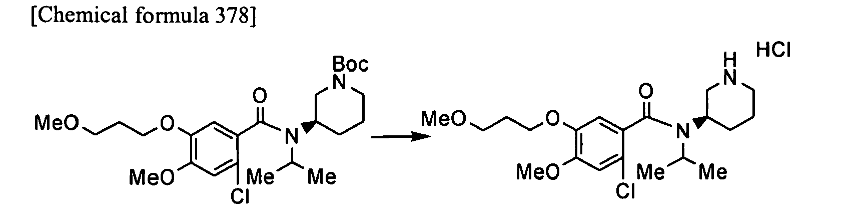

The title compound (274 mg) was synthesized in a similar manner to Example 4 except that the compound of Reference Example 116 was used instead of the compound of Reference Example 22 in Example 4.
¹H-NMR (400MHz, MeOD) 6.96-6.78(m, 2H), 4.03-3.96(m, 3H), 3.76(s, 3H), 3.68-3.57(m, 2H), 3.47(d, J = 6.0 Hz, 2H), 3.33-3.29(m, 1H), 3.24(s, 3H), 2.89-2.83(m, 1H), 2.78-2.68(m, 1H), 2.03-1.81(m, 5H), 1.48-1.43(m, 1H), 1.20-1.06(m, 6H).
MS (ESI+) 399(M⁺+1, 100%).

### Example 54

### N-{(rac.)-(3R,6R)-6-[2-(Cyclohexylamino)ethyl]piperidin-3-yl}-N-isopropyl-4-methoxy-3-(3-methoxypropoxy)benzamide dihydrochloride

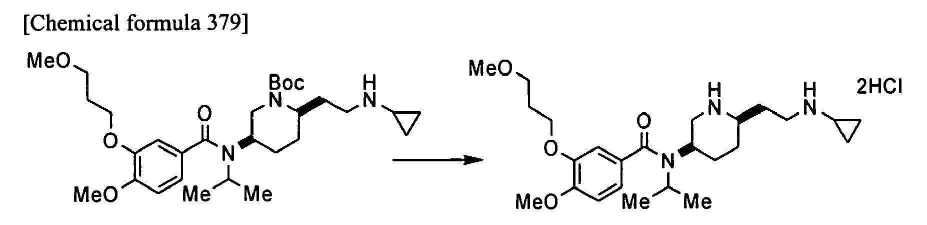

The title compound (6.8 mg) was synthesized in a similar manner to Example 4 except that the compound of Reference Example 92 was used instead of the compound of Reference Example 22 in Example 4.
¹H-NMR (400MHz, MeOD) 6.94(d, J = 8.6 Hz, 1H), 6.88-6.87(m, 2H), 4.01-3.95(m, 3H), 3.78(s, 3H), 3.70-3.54(m, 5H), 3.48(d, J = 6.1 Hz, 2H), 3.24(s, 3H), 2.73-2.69(m, 1H), 2.60(brs, 1H), 2.22-2.17(m, 1H), 1.97-1.91(m, 4H), 1.79-1.77(m, 1H), 1.19-1.11(m, 7H), 0.86-0.84(m, 4H).
MS (ESI+) 448(M⁺+1, 100%).

### Example 55

### N-Isopropyl-N-((rac.)-(3R,6R)-6-{2-[isopropyl(phenylacetyl)amino]ethyl}piperidin-3-yl)-4-methoxy-3-(3-methoxypropoxy)benzamide hydrochloride

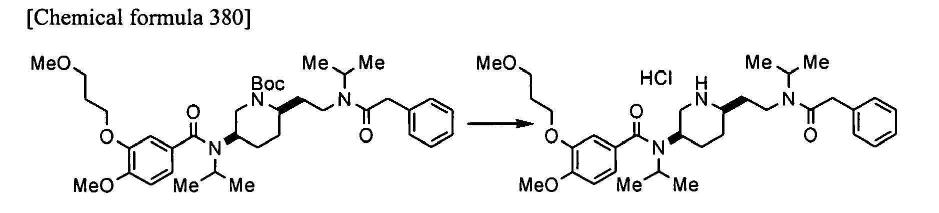

The title compound (3.5 mg) was synthesized in a similar manner to Example 4 except that the compound of Reference Example 118 was used instead of the compound of Reference Example 22 in Example 4.
¹H-NMR (400MHz, MeOD) 7.21-7.13(m, 5H), 6.93(d, J = 8.1 Hz, 1H), 6.87-6.85(m, 2H), 4.01-3.98(m, 3H), 3.77(s, 3H), 3.75(s, 2H), 3.65-3.54(m, 5H), 3.48(t, J = 6.0 Hz, 2H), 3.24(s, 3H), 3.12-3.07(m, 1H), 2.59(brs, 1H), 2.00-1.90(m, 6H), 1.75-1.71(m, 1H), 1.22-1.09(m, 7H), 1.05-1.01(m, 6H).
MS (ESI+) 568(M⁺+1, 100%).

### Example 56

### N-[(rac.)-(3R,6R)-6-(2-{cyclopropyl[(4-methoxyphenyl)acetyl]amino}ethyl)piperidin-3-yl)-N-isopropyl-4-methoxy-3-(3-methoxypropoxy)benzamide hydrochloride

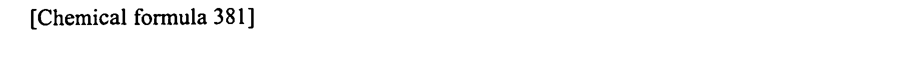

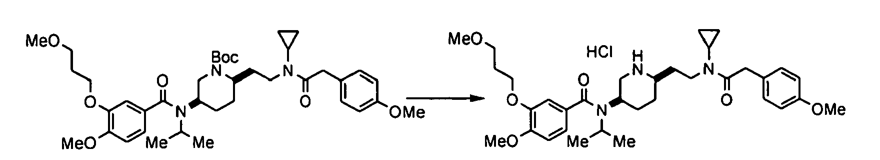

The title compound (4.7 mg) was synthesized in a similar manner to Example 4 except that the compound of Reference Example 119 was used instead of the compound of Reference Example 22 in Example 4.
¹H-NMR (400MHz, MeOD) 7.13-7.08(m, 2H), 6.93(d, J = 8.1 Hz, 1H), 6.87-6.85(m, 2H), 6.80-6.75(m, 2H), 4.01-3.98(m, 3H), 3.82(s, 2H), 3.77(s, 3H), 3.67(s, 3H), 3.64-3.43(m, 6H), 3.24(s, 3H), 3.06-3.02(m, 1H), 2.72-2.67(m, 1H), 2.56(brs, 1H), 1.98-1.86(m, 6H), 1.71-1.66(m, 1H), 1.21-1.09(m, 7H), 0.93-0.79(m, 4H).
MS (ESI+) 596(M⁺+1, 100%).

### Example 57

### ((rac.)-(3R,5R)-5-(Isopropyl[4-methoxy-3-(3-methoxypropoxy)benzoyl]amino)piperidin-3-yl)methyl isopropylcarbamate hydrochloride

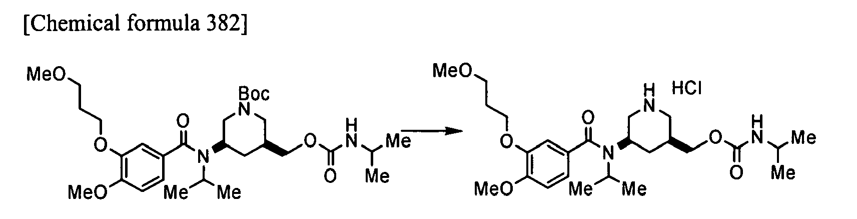

The title compound (32.1 mg) was synthesized in a similar manner to Example 4 except that the compound of Reference Example 125 was used instead of the compound of Reference Example 22.
¹H-NMR (300MHz, CDCl₃) 6.89-6.87(m, 3H), 5.50-5.48(m, 1H), 4.14-4.09(m, 2H), 3.83(s, 3H), 3.80-3.62(m, 6H), 3.59-3.54(m, 2H), 3.35(s, 3H), 2.70-2.63(m, 3H), 2.15-2.09(m, 3H), 1.78-1.60(m, 2H), 1.29-1.14(m, 12H).
MS (ESI+) 480(M⁺+1, 100%).

### Examples 58 - 59

The compounds as listed in the following Table were synthesized in a similar manner to Example 53 except that the compounds of Reference Example 126 and Reference Example 127 were used instead of the compound of Reference Example 119 in Example 53, respectively.

**[Table 8]**

| Ex. No. | Structure | MS(ESI⁺) |
|---|---|---|
| 58 | 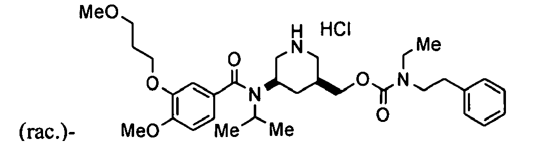 | 570(M⁺+1, 100%). |
| 59 | 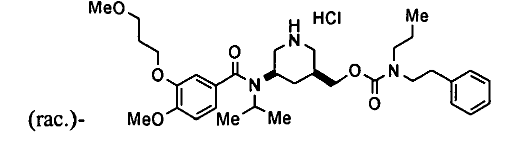 | 584(M⁺+1, 100%). |

### Example 60

### N-Isobutyl-4-methoxy-3-(3-methoxypropoxy)-N-[(3R)-piperidin-3-yl]benzamide hydrochloride

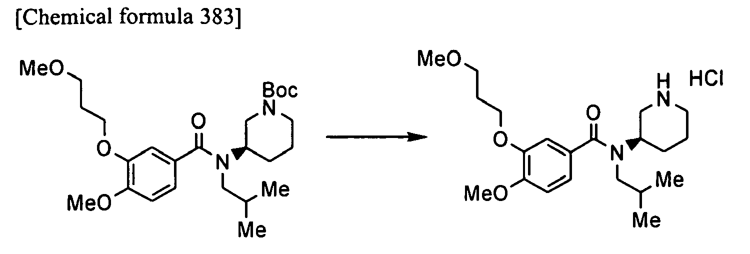

The title compound (92.0 mg) was synthesized in a similar manner to Example 4 except that the compound of Reference Example 111 was used instead of the compound of Reference Example 22 in Example 4.
¹H-NMR (400MHz, MeOD) 6.95-6.89(m, 3H), 3.99(d,J = 6.2 Hz, 2H), 3.90(brs, 1H), 3.78(s, 3H), 3.66-3.55(m, 2H), 3.49(d, J = 6.0 Hz, 2H), 3.35(brs, 1H), 3.25(s, 3H), 3.19-3.09(m, 2H), 2.88-2.82(m, 1H), 2.17(brs, 1H), 1.96-1.93(m, 5H), 1.65(brs, 1H), 0.76(brs, 1H).
MS (ESI+) 379(M⁺+1, 100%).

### Example 61

### ((rac.)-(3S,5R)-5-{Isopropyl[4-methoxy-3-(3-methoxypropoxy)benzoyl]amino}piperidin-3-yl)methyl(3-methylbutyl)(2-phenylethyl)carbamate hydrochloride

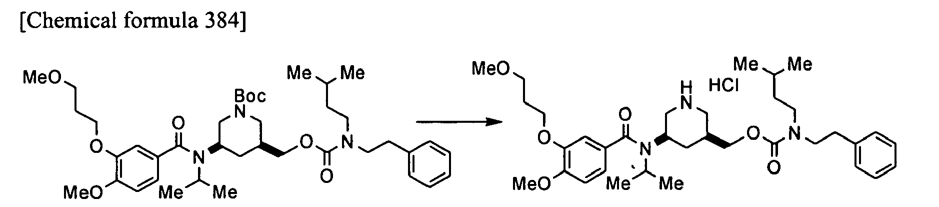

The title compound (13.6 mg) was synthesized in a similar manner to Example 4 except that the compound of Reference Example 128 was used instead of the compound of Reference Example 22 in Example 4.
MS (ESI⁺) 612(M⁺+1, 100%).

### Example 62

### Benzyl [((rac.)-(3S,5R)-5-{isopropyl[4-methoxy-3-(3-methoxypropoxy)benzoyl]amino}piperidin-3-yl)methyl]carbamate hydrochloride

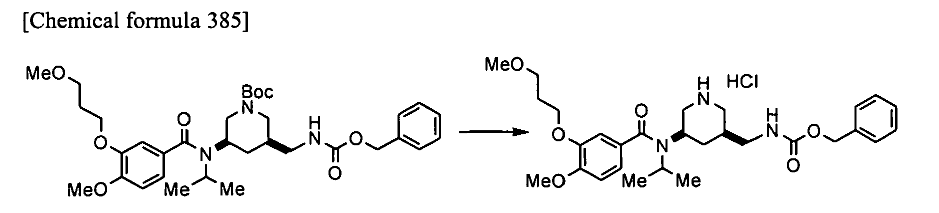

The title compound (40.8 mg) was synthesized in a similar manner to Example 4 except that the compound of Reference Example 131 was used instead of the compound of Reference Example 22 in Example 4.
¹H-NMR (300MHz, CDCl₃) 7.34-7.32(m, 5H), 6.89-6.85(m, 3H), 5.09(s, 2H), 4.10-4.02(m, 2H), 3.87(s, 3H), 3.79-3.62(m, 4H), 3.58-3.53(m, 2H), 3.34(s, 3H), 3.20-3.10(m, 2H), 2.58-2.17(m, 4H), 2.10-2.07(m, 2H), 1.78-1.76(m, 1H), 1.26-1.16(m, 6H).
MS (ESI+) 528(M⁺+1, 100%).

### Example 63

### N-((3R,6R)-6-{2-[Cyclopropyl(methoxyacetyl)amino]ethyl}piperidin-3-yl)-N-isopropyl-4-methoxy-3-(3-methoxypropoxy)benzamide hydrochloride

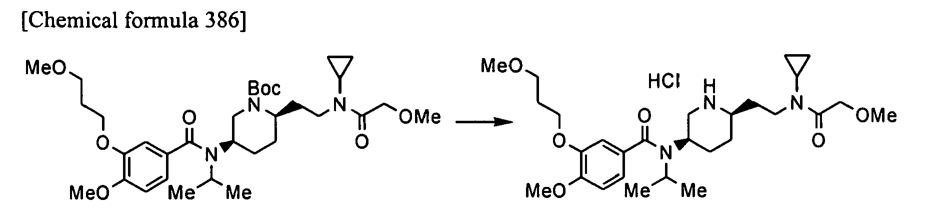

The title compound (60 mg) was synthesized in a similar manner to Example 4.
¹H NMR (400 MHz, DMSO-d₆) 9.76(m, 1H), 8.99-8.97(m, 1H), 6.99-6.97(m, 1H), 6.90-6.85(m, 2H), 4.30-4.22(m, 2H), 4.01(t, J = 6.7 Hz, 2H), 3.92-3.59(m, 3H), 3.77(s, 3H), 3.53-3.40(m, 5H), 3.28(s, 3H), 3.22(s, 3H), 2.94-2.91(m, 1H), 2.73-2.63(m, 2H), 2.00-1.84(m, 6H), 1.58-1.55(m, 1H), 1.15-1.10(m, 6H), 0.78(m, 4H)
MS (ESI+) 520(M⁺+1, 100%).

### Example 64

### N-((3R,6R)-6-{2-[Cyclopropyl(3-methoxypropanoyl)amino]ethyl}piperidin-3-yl)-N-isopropyl-4-methoxy-3-(3-methoxypropoxy)benzamide hydrochloride

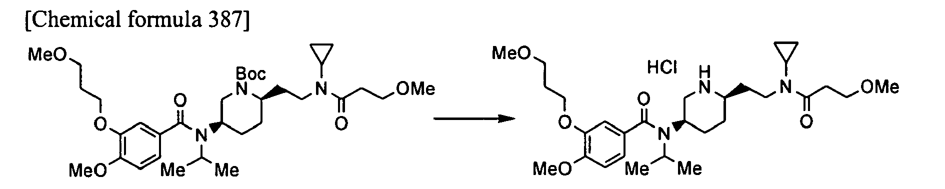

The title compound (14 mg) was synthesized in a similar manner to Example 4.
¹H NMR (400 MHz, DMSO-d₆) 9.2(m, 1H), 8.6(m, 1H), 7.00-6.97(m, 1H), 6.89-6.85(m, 2H), 4.01(t, J = 6.7 Hz, 2H), 3.83-3.80(m, 2H), 3.78(s, 3H), 3.58-3.33(m, 9H), 3.23(s, 3H), 3.22(s, 3H), 2.96-2.95(m, 1H), 2.82-2.66(m, 3H), 1.98-1.86(m, 6H), 1.58-1.56(m, 1H), 1.23-1.12(m, 6H), 0.88-0.76(m, 4H).
MS (ESI+) 534(M⁺+1, 100%)

### Example 65

### N-((3R,6R)-6-{2-[Cyclopropyl(4-methoxybutanoyl)amino]ethyl}piperidin-3-yl)-N-isopropyl-4-methoxy-3-(3-methoxypropoxy)benzamide hydrochloride

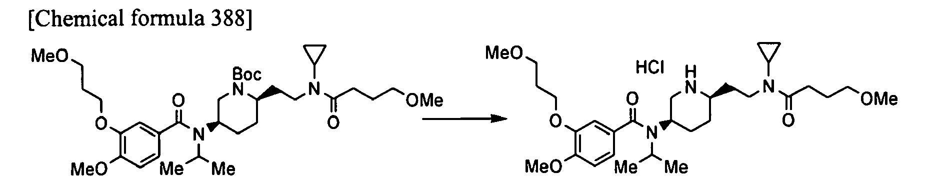

The title compound (34 mg) was synthesized in a similar manner to Example 4.
¹H NMR (400 MHz, DMSO-d₆) 9.5(m, 1H), 8.8(m, 1H), 6.99-6.97(m, 1H), 6.90-6.85(m, 2H), 4.00(t, J = 6.7 Hz, 2H), 3.98-3.48(m, 2H), 3.78(s, 3H), 3.48-3.24(m, 5H), 3.21(s, 3H), 3.19(s, 3H), 2.95-2.93(m, 1H), 2.76-2.66(m, 3H), 2.55(m, 2H), 1.98-1.82(m, 8H), 1.78-1.71(m, 2H), 1.58-1.55(m, 1H), 1.18-1.11(m, 6H), 0.86-0.74(m, 4H).
MS (ESI+) 548(M⁺+1, 100%)

### Example 66

### N-((3R,6R)-6-{2-[Cyclopropyl(4-hydroxybutanoyl)amino]ethyl}piperidin-3-yl)-N-isopropyl-4-methoxy-3-(3-methoxypropoxy)benzamide hydrochloride

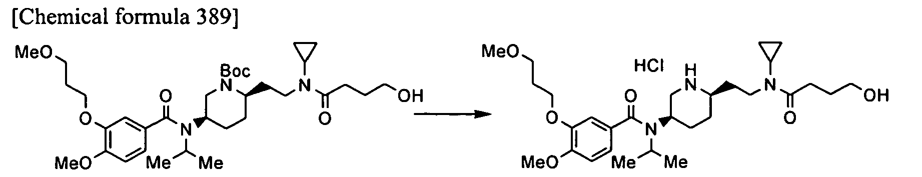

The title compound (63 mg) was synthesized in a similar manner to Example 4.
¹H NMR (400 MHz, DMSO-d₆) 6.99-6.97(m, 1H), 6.91-6.87(m, 2H), 4.46(m, 1H), 4.23-4.00(t, J = 6.7 Hz, 2H), 3.83-3.47(m, 3H), 3.78(s, 3H), 3.47-3.38(m, 6H), 3.23(s, 3H), 2.93(m, 1H), 2.74-2.52(m, 3H), 1.98-1.86(m, 7H), 1.68-1.55(m, 3H), 1.16-1.10(m, 6H), 0.85-0.83(m, 2H), 0.77-0.76(m, 2H).
MS (ESI+) 534(M⁺+1, 100%)

### Example 67

### N-((3R,6R)-6-{2-[Cyclopropyl(3-hydroxy-3-methylbutanoyl)amino]ethyl}piperidin-3-yl)-N-isopropyl-4-methoxy-3-(3-methoxypropoxy)benzamide hydrochloride

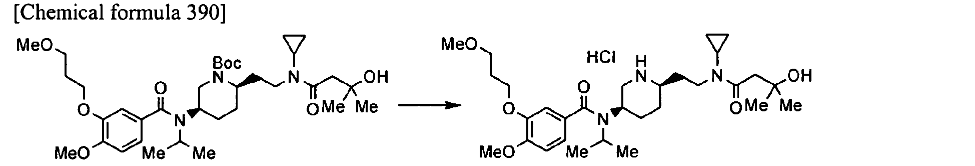

The title compound (30 mg) was synthesized in a similar manner to Example 4.
¹H-NMR (400MHz, CD₃OD) 7.05-7.03(m, 1H), 6.98-6.95(m, 2H), 4.11-4.06(m, 4H), 3.94-3.91(m, 2H), 3.88(s, 3H), 3.67-3.59(m, 4H), 3.57-3.20(m, 3H), 3.32(s, 3H), 2.85-2.82(m, 1H), 2.60(m, 2H), 2.13-2.01(m, 7H), 1.86-1.83(m, 1H), 1.71-1.68(m, 2H), 1.40-1.21(m, 8H), 1.00-0.98(m, 2H), 0.88-0.85(m, 2H).
MS (ESI+) 548(M⁺+1, 100%)

### Example 68

### N-((3R,6R-6-{2-[Cyclopropyl(3-methyl-2-butenoyl)amino]ethyl}piperidin-3-yl)-N-isopropyl-4-methoxy-3-(3-methoxypropoxy)benzamide hydrochloride

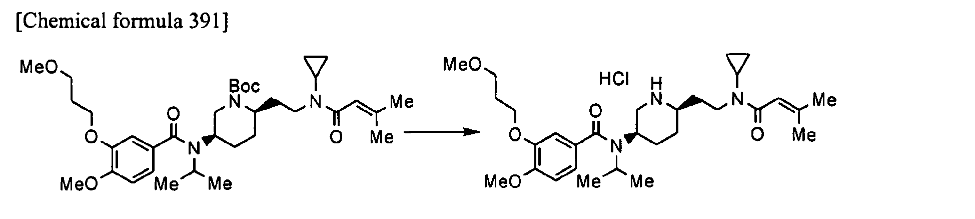

The title compound (15 mg) was synthesized in a similar manner to Example 4.
¹H-NMR (400MHz, CD₃OD) 7.05-7.03(m, 1H), 6.98-6.96(m, 2H), 6.35(s, 1H), 4.11-4.06(m, 4H), 3.88(s, 3H), 3.76-3.22(m, 8H), 3.32(s, 3H), 2.13-2.00(m, 8H), 1.93(s, 3H), 1.87-1.79(m, 2H), 1.32-1.13(m, 7H), 0.96-0.90(m, 3H), 0.80(m, 2H).
MS (ESI+) 530(M⁺+1, 100%)

### Example 69

### N⁴-Cyclopropyl-N⁴-[2-((2R,5R)-5-{isopropyl[4-methoxy-3-(3-methoxypropoxy)-benzoyl]amino}piperidin-2-yl)ethyl]-2,2-dimethylsuccinamide trifluoroacetate

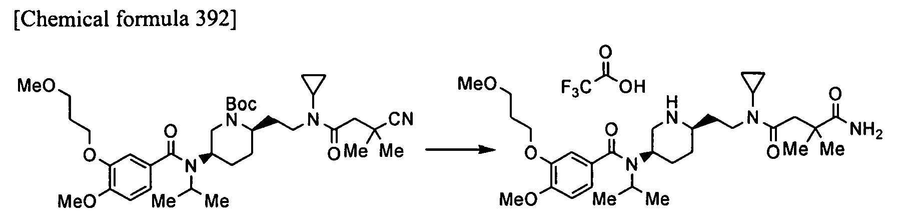

The compound obtained in Reference Example 143 (32 mg) was dissolved in trifluoroacetic acid (3 ml), and the mixture was stirred at 25°C for 5 hours. The reaction solution was concentrated under reduced pressure to give the title compound (30 mg) as a colorless oily compound.
¹H NMR (400 MHz, DMSO-d₆) 6.99-6.98(m, 1H), 6.90-6.85(m, 2H), 4.00(t, J = 6.7 Hz, 2H), 3.84(m, 1H), 3.78(s, 3H), 3.65-3.33(m, 6H), 3.23(s, 3H), 3.05-2.71(m, 5H), 1.96-1.85(m, 6H), 1.60(m, 1H), 1.38-1.06(m, 7H), 1.18(s, 6H), 0.86-0.84(m, 2H), 0.77-0.76(m, 2H).
MS (ESI+) 557(M⁺+1, 100%)

### Example 70

### N-((3R,6R)-6-{2-[(Cyclohexylacetyl)(cyclopropyl)amino]ethyl}piperidin-3-yl)-N-isopropyl-4-methoxy-3-(3-methoxypropoxy)benzamide hydrochloride

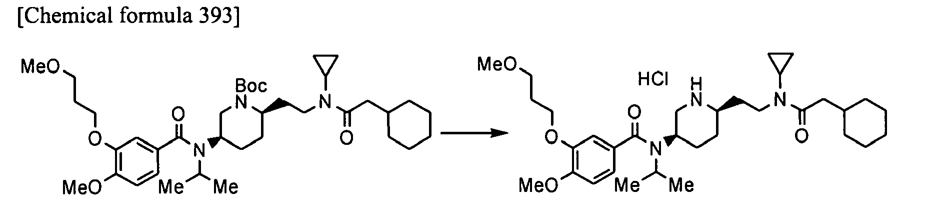

The title compound (13 mg) was synthesized in a similar manner to Example 4.
¹H-NMR (400MHz, CD₃OD) 7.03(m, 1H), 6.98-6.96(m, 2H), 4.11-4.06(m, 4H), 3.88(s, 3H), 3.70-3.40(m, 6H), 3.35(s, 3H), 3.27-3.22(m, 1H), 2.91-2.68(m, 4H), 2.16-2.00(m, 6H), 1.87-1.83(m, 1H), 1.33-1.20(m, 14H), 1.03-1.01(m, 2H), 0.90-0.89(m, 2H).
MS (ESI+) 572(M⁺+1, 100%)

### Example 71

### 4-{Cyclopropyl[2-((2R,5R)-5-{isopropyl[4-methoxy-3-(3-methoxypropoxy)benzoyl]amino}piperidin-2-yl)ethyl]amino}-4-oxobutanoic acid hydrochloride

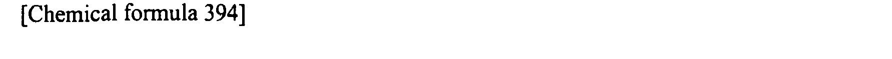

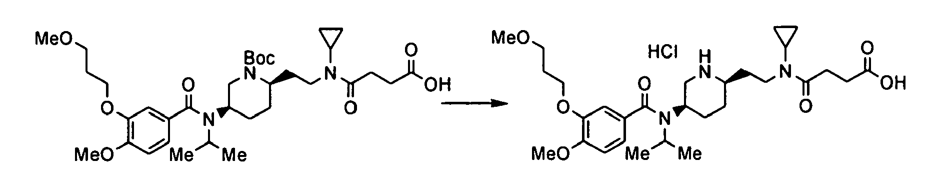

The title compound (15 mg) was synthesized in a similar manner to Example 4.
¹H NMR (400 MHz, DMSO-d₆) 9.2(m, 1H), 8.6(m, 1H), 7.00-6.97(m, 1H), 6.89-6.85(m, 2H), 4.01(t, J = 6.7 Hz, 2H), 3.83-3.80(m, 2H), 3.78(s, 3H), 3.58-3.33(m, 9H), 3.23(s, 3H), 2.96-2.95(m, 1H), 2.82-2.66(m, 3H), 1.98-1.86(m, 6H), 1.58-1.56(m, 1H), 1.23-1.12(m, 6H), 0.88-0.76(m, 4H).
MS (ESI+) 548(M⁺+1, 100%)

### Example 72

### N-((3R,6R)-6-{2-[[(2-chlorophenyl)acetyl](cyclopropyl)amino]ethyl}piperidin-3-yl)-N-isopropyl-4-methoxy-3-(3-methoxypropoxy)benzamide trifluoroacetate

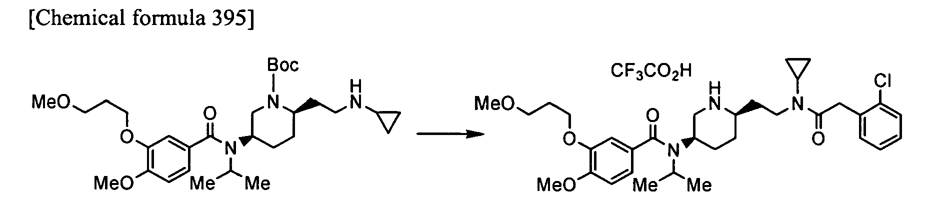

tert-Butyl-(2S,5R)-2-[2-(cyclopropylamino)ethyl]-5-{isopropyl[4-methoxy-3-(3-methoxypropoxy)benzoyl]amino}piperidine-1-carboxylate (50.0 mg) was dissolved in dimethylformamide (0.5 ml), and thereto were added o-chlorophenylacetic acid (23.4 mg), 1-hydroxybenzotriazole (19.7 mg), 1-ethyl-3-(dimethylaminopropyl)carbodiimide hydrochloride (29.8 mg) and triethylamine (37.9 µl), and the mixture was stirred at room temperature for 21 hours. To the reaction mixture was added a saturated aqueous sodium hydrogen carbonate solution, and the mixture was extracted with ethyl acetate. The organic layer was washed with a saturated aqueous sodium hydrogen carbonate solution, water, and a saturated aqueous sodium chloride solution, dried over sodium sulfate, filtered, and concentrated under reduced pressure. The obtained residue was dissolved in methanol (0.5 ml), and thereto was added a 4N solution of hydrochloric acid in 1,4-dioxane (0.5 ml), and the mixture was stirred at room temperature for 4 hours. The reaction solution was concentrated under reduced pressure, purified by reverse phase silica gel column chromatography (developing solvent: acetonitrile/aqueous trifluoroacetic acid solution) to give the title compound (58.3 mg) as a colorless oily compound.
¹H-NMR (400MHz, CD₃OD) 7.41-7.26(m, 4H), 7.04-6.95(m, 3H), 4.12-4.07(m, 5H), 3.98(s, 2H), 3.87(s, 3H), 3.59-3.55(m, 5H), 3.42-3.38(m, 2H), 3.34(s, 3H), 3.19-3.14(m, 1H), 3.01-2.96(m, 1H), 2.65-2.58(m, 1H), 2.12-1.98(m, 7H), 1.23-1.01(m, 6H), 1.01-0.98(m, 4H).
MS (ESI+) 600(M⁺+1, 100%)

### Example 73

### N-[(3R,6R)-6-(2-{Cyclopropyl[(2-methoxyphenyl)acetyl)amino}ethyl)piperidin-3-yl]-N-isopropyl-4-methoxy-3-(3-methoxypropoxy)benzamide trifluoroacetate

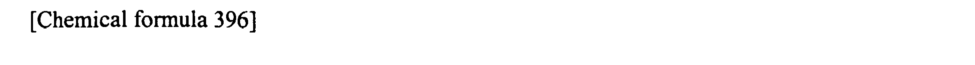

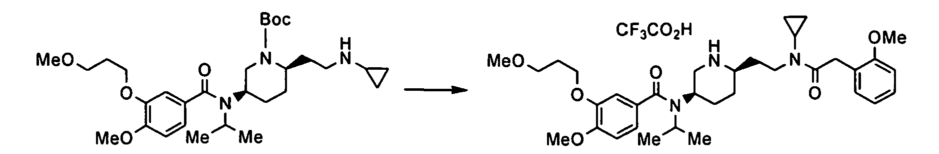

The title compound was synthesized in a similar manner to Example 72 as a colorless oily compound.
¹H-NMR (400MHz, CD₃OD) 7.26-7.23(m, 2H), 7.18-7.16(m, 5H), 4.09-4.02(m, 5H), 3.98-3.3.89(m, 5H), 3.50(s, 3H), 3.72-3.48(m, 5H), 3.35-3.39(m, 2H), 3.35(s, 3H), 3.15-3.10(m, 1H), 2.95-2.81(m, 1H), 2.75-2.62(m, 1H), 2.12-1.99(m, 7H), 1.23-1.12(m, 6H), 1.02-0.94(m, 4H).
MS (ESI+) 596(M+1, 100%).

### Example 74

### N-{(3R,6R)-6-[2-(Cyclopropyl {[2-(trifluoromethyl)phenyl]acetyl}amino)ethyl]piperidin-3-yl -N-isopropyl-4-methoxy-3-(3-methoxypropoxy)benzamide trifluoroacetate

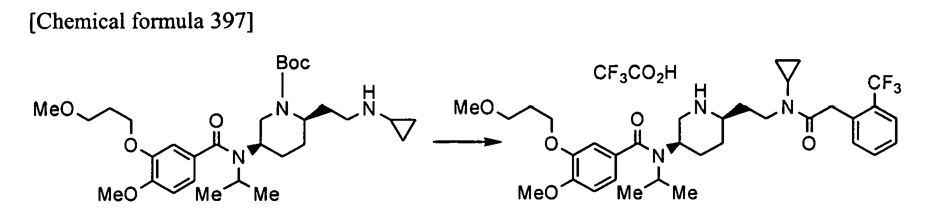

The title compound was synthesized in a similar manner to Example 72 as a colorless oily compound.
¹H-NMR (400MHz, CD₃OD) 7.69-7.67(m, 1H), 7.59-7.56(m, 1H), 7.46-7.39(m, 2H), 7.03-6.93(m, 3H), 4.20(s, 2H), 4.09-4.04(m, 5H), 3.97(s, 3H), 3.63-3.50(m, 5H), 3.42-3.38(m, 2H), 3.35(s, 3H), 3.19-3.11(m, 1H), 2.95-2.89(m, 1H), 2.70-2.62(m, 1H), 2.12-1.80(m, 7H), 1.28-1.17(m, 6H), 1.05-0.94(m, 4H).
MS (ESI+) 634(M+1, 100%).

### Example 75

### N-((3R,6R)-6-{2-[Cyclopropyl(pyridin-2-ylacetyl)amino]ethyl}piperidin-3-yl)-N-isopropyl-4-methoxy-3-(3-methoxypropoxy)benzamide trifluoroacetate

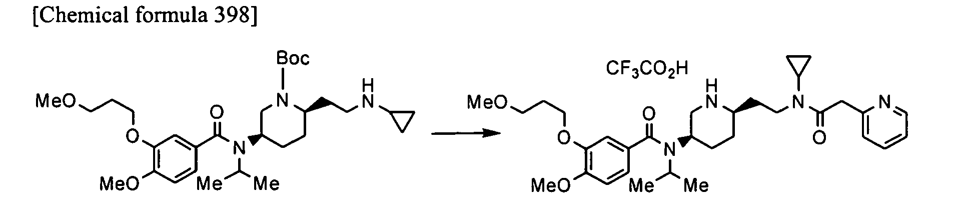

The title compound was synthesized in a similar manner to Example 72 as a colorless oily compound.
¹H-NMR (400MHz, CD₃OD) 8.77-8.72(m, 1H), 8.46-8.40(m, 1H), 7.92-7.86(m, 2H), 7.04-6.94(m, 3H), 4.11-4.01(m, 4H), 3.87(s, 3H), 3.70-3.49(m, 7H), 3.42-3.38(m, 3H), 3.35(s, 3H), 3.21-3.11(m, 1H), 2.99-2.89(m, 1H), 2.75-2.62(m, 1H), 2.18-1.80(m, 7H), 1.25-1.17(m, 6H), 1.10-0.98(m, 4H).
MS (ESI+) 567(M+1, 100%).

### Example 76

### N-[(3R,6R)-6-(2-{Cyclopropyl[(2R)-2-hydroxy-2-phenytacetyl]amino}ethyl)piperidin-3-yl]-N-isopropyl-4-methoxy-3-(3-methoxypropoxy)benzamide trifluoroacetate

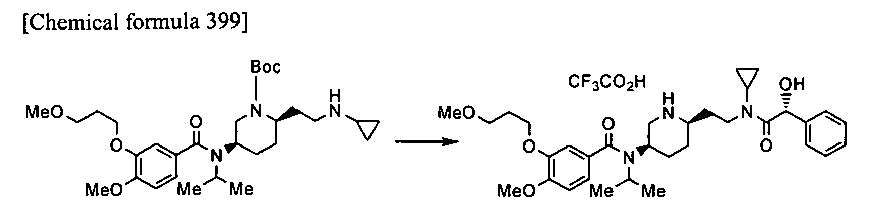

The title compound was synthesized in a similar manner to Example 72 as a colorless oily compound.
¹H-NMR (400MHz, CD₃OD) 7.49-7.47(m, 2H), 7.41-7.33(m, 3H), 7.03-6.94(m, 3H), 5.78(s, 1H), 4.10-3.97(m, 5H), 3.86(s, 3H), 3.85-3.76(m, 1H), 3.59-3.55(m, 4H), 3.42-3.38(m, 2H), 3.33(s, 3H), 3.19-3.15(m, 2H), 2.68-2.58(m, 1H), 2.44-2.37(m, 1H), 2.06-1.86(m, 7H), 1.23-1.11(m, 6H), 1.01-0.81(m, 4H).
MS (ESI+) 582(M+1, 100%).

### Example 77

### N-((3R,6R)-6-{2-[Cyclopropyl(3-hydroxy-2-phenylpropanoyl)amino]ethyl}piperidin-3-yl)-N-isopropyl-4-methoxy-3-(3-methoxypropoxy)benzamide trifluoroacetate

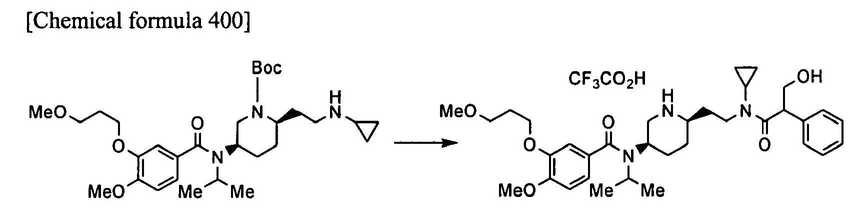

The title compound was synthesized in a similar manner to Example 72 as a colorless oily compound.
¹H-NMR (400MHz, CD₃OD 7.39-7.26(m, 5H), 7.03-6.94(m, 3H), 4.95-4.92(m,1H), 4.67-4.63(m, 1H), 4.14-4.06(m, 5H), 3.86(s, 3H), 3.67-3.55(m, 6H), 3.59-3.55(m, 2H), 3.33(s, 3H), 3.19-3.15(m, 2H), 2.69-2.64(m, 2H), 2.06-1.92(m, 7H), 1.23-1.18(m, 6H), 0.94-0.72(m, 4H).
MS (ESI+) 596(M+l, 100%).

### Example 78

### N-((3R,6R)-6-{2-[Cyclopropyl(pyridin-3-ylacetyl)amino]ethyl}piperidin-3-yl)-N-isopropyl-4-methoxy-3-(3-methoxypropoxy)benzamide trifluoroacetate

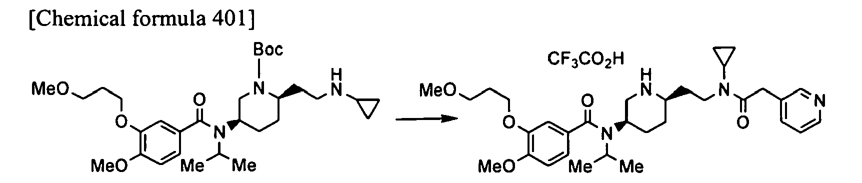

The title compound was synthesized in a similar manner to Example 72 as a colorless oily product.
¹H-NMR (400MHz, CD₃OD) 8.77-8.71(m, 2H), 8.46-8.40(m, 1H), 7.96-7.94(m, 1H), 7.03-6.93(m, 3H), 4.28-4.26(m, 2H), 4.09-4.07(m, 2H), 3.86(s, 3H), 3.70-3.49(m, 7H), 3.42-3.38(m, 3H), 3.35(s, 3H), 3.21-3.11(m, 1H), 2.99-2.89(m, 1H), 2.75-2.62(m, 1H), 2.18-1.80(m, 7H), 1.22-1.17(m, 6H), 1.10-0.99(m, 4H).
MS (ESI+) 567(M+1, 100%).

### Example 79

### N-((3R,6R)-6-{2-[Cyclopropyl(1H-imidazol-5-ylacetyl)amino]ethyl}piperidin-3-yl)-N-isopropyl-4-methoxy-3-(3-methoxypropoxy)benzamide trifluoroacetate

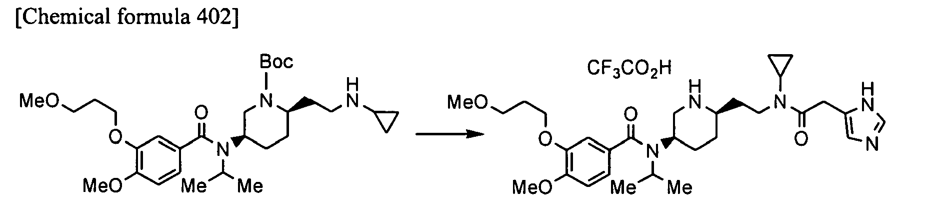

The title compound was synthesized in a similar manner to Example 72 as a colorless oily compound.
¹H-NMR (400MHz, CD₃OD) 8.79(s, 1H), 7.41(s, 1H), 7.04-6.93(m, 3H), 4.17(s, 2H), 4.09-4.06(m, 5H), 3.86(s, 3H), 3.70-3.49(m, 8H), 3.35(s, 3H), 3.12-3.12(m, 1H), 2.89-2.81(m, 1H), 2.85-2.74(m, 1H), 2.13-1.98(m, 7H), 1.23-1.17(m, 6H), 1.09-0.95(m, 4H).
MS (ESI+) 556(M+1, 100%).

### Example 80

### N-((3R,6R)-6-{2-[Cyclopropyl(pyridin-4-ylacetyl)amino]ethyl}piperidin-3-yl)-N-isopropyl-4-methoxy-3-(3-methoxypropoxy)benzamide trifluoroacetate

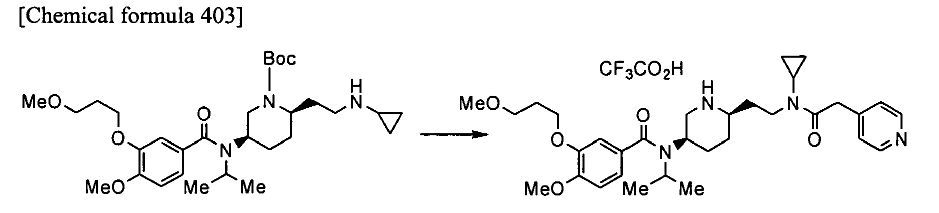

The title compound was synthesized in a similar manner to Example 72 as a colorless oily product.
1H-NMR (400MHz, CD₃OD) 8.75-8.73(m, 2H), 8.00-7.98(m, 2H), 7.04-6.94(m, 3H), 4.37-4.35(m, 2H), 4.09-3.97(m, 4H), 3.87(s, 3H), 3.62-3.55(m, 6H), 3.42-3.38(m, 2H), 3.35(s, 3H), 3.21-3.10(m, 1H), 2.95-2.80(m, 1H), 2.70-2.62(m, 1H), 2.12-1.98(m, 7H), 1.23-1.17(m, 6H), 1.06-0.98(m, 4H).
MS (ESI+) 567(M+1, 100%).

### Example 81

### N-((3R,6R)-6- {2-[Cyclopropyl({3-[(methylsulfonyl)amino]phenyl}acetyl)amino]ethyl}piperidin-3-yl)-N-isopropyl-4-methoxy-3-(3-methoxypropoxy)benzamide trifluoroacetate

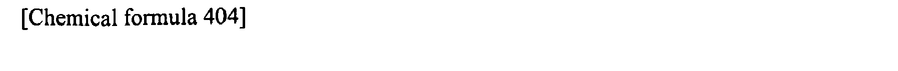

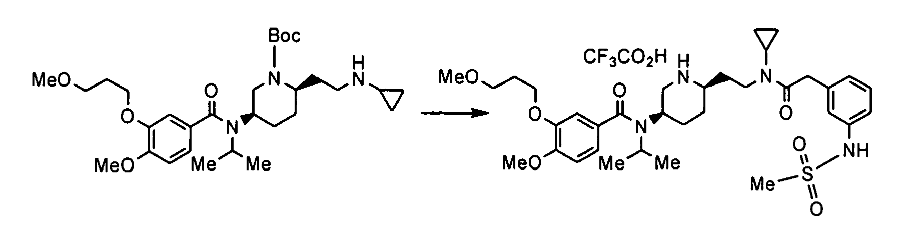

Using 3-(methanesulfonylamino)phenylacetic acid (WO 2005/105780), the title compound was synthesized in a similar manner to Example 72 as a colorless oily compound.
¹H-NMR (400MHz, CD₃OD) 7.32-7.26(m, 2H), 7.10-6.95(m, 5H), 4.10-3.97(m, 6H), 3.86(s, 3H), 3.64-3.57(m, 6H), 3.35(s, 3H), 3.28-3.15(m, 4H), 2.95(s, 3H), 2.82-2.76(m, 1H), 2.75-2.64(m, 1H), 2.06-2.02(m, 7H), 1.24-1.17(m, 6H), 1.02-0.90(m, 4H).
MS (ESI+) 659(M+1, 100%).

### Example 82

### N-((3R,6R)-6-{2-[[(3-Cyanophenyl)acetyl](cyclopropyl)amino]ethyl}piperidin-3-yl)-N-isopropyl-4-methoxy-3-(3-methoxypropoxy)benzamide trifluoroacetate

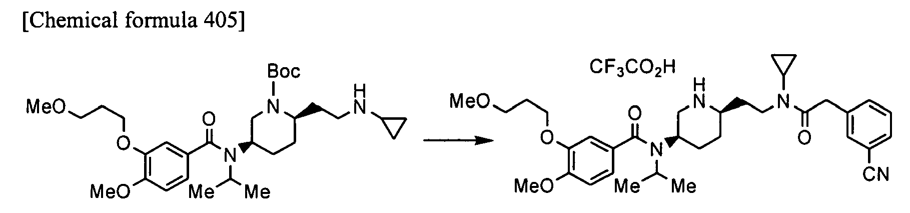

The title compound was synthesized in a similar manner to Example 72 as a colorless oily compound.
¹H-NMR (400MHz, CD₃OD) 7.66-7.60(m, 3H), 7.51-7.48(m, 1H), 7.04-6.94(m, 3H), 4.09-4.07(m, 6H), 3.86(s, 3H), 3.62-3.50(m, 6H), 3.42-3.38(m, 2H), 3.33(s, 3H), 3.21-3.11(m, 1H), 2.95-2.88(m, 1H), 2.75-2.67(m, 1H), 2.08-1.80(m, 7H), 1.25-1.17(m, 6H), 1.04-0.94(m, 4H).
MS (ESI+) 591(M+1, 100%).

### Example 83

### N-((3R,6R)-6-{2-[(Anilinocarbonyl)(cyclopropyl)amino]ethyl}piperidin-3-yl)-N-isopropyl-4-methoxy-3-(3-methoxypropoxy)benzamide hydrochloride

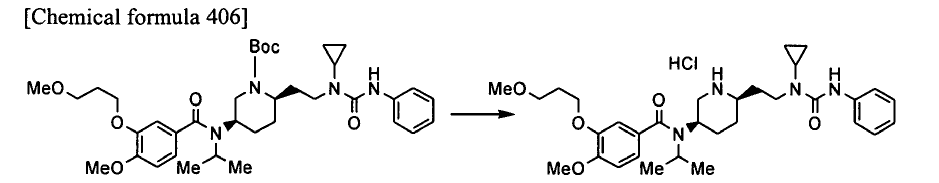

The compound of Reference Example 147 was dissolved in methanol (0.5 ml), and thereto was added a 4N solution of hydrochloric acid in 1,4-dioxane (0.5 ml), and the mixture was stirred at room temperature for 4 hours. The reaction solution was concentrated under reduced pressure to give the title compound (53.7 mg) as a colorless oily compound.
¹H-NMR (400MHz, CD₃OD) 7.46-7.42(m, 2H), 7.30-7.26(m, 2H), 7.08-6.94(m, 4H), 6.88-6.75(m, 2H), 4.10-4.07(m, 3H), 3.86(s, 3H), 3.68-3.65(m, 4H), 3.59-3.51(m, 4H), 3.33(s, 3H), 3.21-3.11(m, 1H), 2.95-2.88(m, 1H), 2.75-2.67(m, 1H), 2.08-1.80(m, 7H), 1.24-1.18(m, 6H), 1.05-0.89(m, 4H).
MS (ESI+) 567(M+1, 100%).

### Example 84

### N-((3R,6R)-6-{2-[(Benzylsulfonyl)(cyclopropyl)amino]ethyl}piperidin-3-yl)-N-isopropyl-4-methoxy-3-(3-methoxypropoxy)benzamide hydrochloride

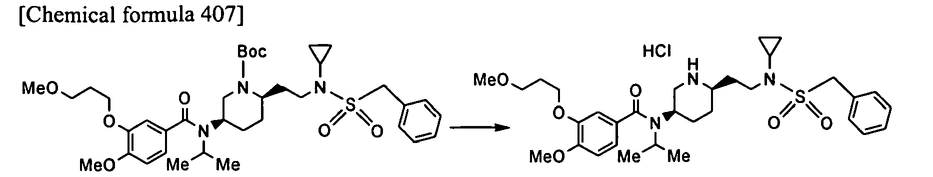

The title compound was synthesized in a similar manner to Example 83 as a colorless oily compound.
¹H-NMR (400MHz, CD₃OD) 7.43-7.36(m, 5H), 7.03-6.94(m, 3H), 4.88(m, 2H), 4.42(s, 2H), 4.09-3.93(m, 4H), 3.86(s, 3H), 3.68-3.64(m, 1H), 3.57(t, J=6.1 Hz, 2H), 3.51-3.47(m, 1H), 3.34(s, 3H), 3.27-3.18(m, 2H), 2.75-2.65(m, 1H), 2.66-2.55(m, 1H), 2.11-1.85(m, 7H), 1.23-1.18(m, 6H), 0.75-0.70(m, 4H).
MS (ESI+) 620(M+1, 50%).

### Example 85

### Ethyl cyclopropyl[2-((2R,5R)-5-{isopropyl[4-methoxy-3-(3-methoxypropoxy)benzoyl]amino}piperidin-2-yl)ethyl]carbamate hydrochloride

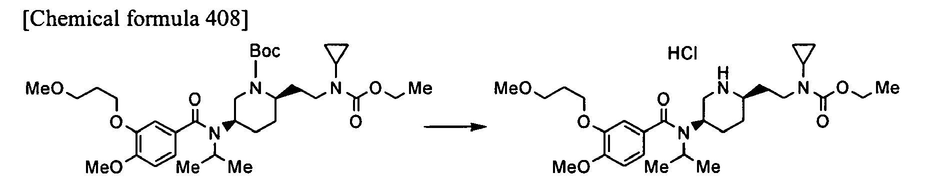

The title compound was synthesized in a similar manner to Example 83 as a colorless oily compound.
¹H-NMR (400MHz, CD₃OD) 7.04-7.01(m, 1H), 6.97-6.94(m, 2H), 4.88(m, 2H), 4.18-4.12(m, 2H), 4.10-4.07(m, 4H), 3.86(s, 3H), 3.71-3.64(m, 1H), 3.57(t, J=6.1 Hz, 2H), 3.54-3.48(m, 2H), 3.34(s, 3H), 3.27-3.20(m, 2H), 2.75-2.65(m, 2H), 2.10-2.00(m, 6H), 1.90-1.82(m, 1H), 1.28(t, J=7.1 Hz, 3H), 1.24-1.21(m, 6H), 0.82-0.68(m, 4H).
MS (ESI+) 520(M+1, 100%).

### Example 86

### 3-(2-{Cyclopropyl[2-((2R,5R)-5-{isopropyl[4-methoxy-3-(3-methoxypropoxy)benzoyl]amino}piperidin-2-yl)ethyl]amino}-2-oxoethyl)benzoic acid hydrochloride

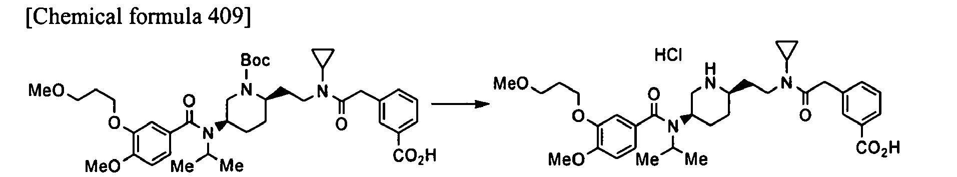

The title compound was synthesized in a similar manner to Example 83 as a colorless oily compound.
¹H-NMR (400MHz, CD₃OD) 7.96-7.91(m, 2H), 7.55-7.42(m, 2H), 7.03-6.91(m, 3H), 4.08-4.06(m, 4H), 4.01-3.98(m, 1H), 3.86(s, 3H), 3.75-3.51(m, 8H), 3.33(s, 3H), 3.29-3.18(m, 1H), 2.85-2.80(m, 1H), 2.66-2.54(m, 1H), 2.20-1.95(m, 4H), 1.87-1.80(m, 4H), 1.23-1.18(m, 6H), 1.10-1.02(m, 4H).
MS (ESI+) 610(M+1, 100%).

### Example 87

### N-((3R,6R)-6-{2-[{[3-(Aminocarbonyl)phenyl]acetyl}(cyclopropyl)amino]ethyl}piperidin-3-yl)-N-isopropyl-4-methoxy-3-(3-methoxypropoxy)benzamide hydrochloride

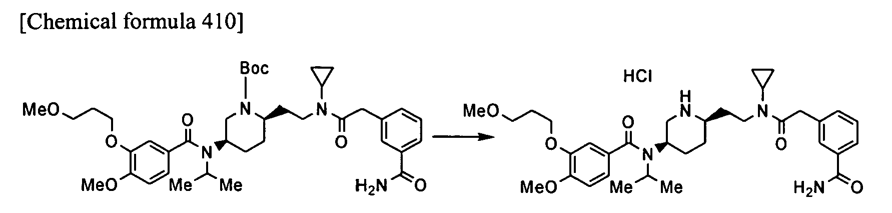

The title compound was synthesized in a similar manner to Example 83 as a colorless oily compound.
¹H-NMR (400MHz, CD₃OD) 7.81-7.75(m, 2H), 7.50-7.41(m, 2H), 7.03-6.91(m, 3H), 4.90-4.88(m, 4H), 4.09-3.91(m, 6H), 3.86(s, 3H), 3.70-3.55(m, 6H), 3.34(s, 3H), 3.27-3.18(m, 1H), 2.85-2.80(m, 1H), 2.70-2.60(m, 1H), 2.15-1.90(m, 6H), 1.87-1.80(m, 1H), 1.23-1.18(m, 6H), 1.04-0.93(m, 4H).
MS (ESI+) 609(M+1, 100%).

### Example 88

### Ethyl 3-(2-{cyclopropyl[2-((2R,5R)-5-{isopropyl[4-methoxy-3-(3-methoxypropoxy)-benzoyl]amino}piperidin-2-yl)ethyl]amino}-2-oxoethyl)benzoate hydrochloride

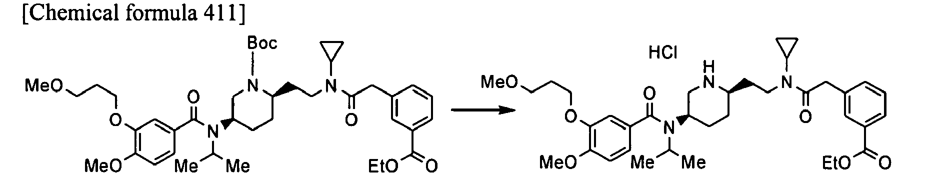

The title compound was synthesized in a similar manner to Example 83 as a colorless oily compound.
¹H-NMR (400MHz, CD₃OD) 7.94-7.90(m, 2H), 7.55-7.42(m, 2H), 7.03-6.91(m, 3H), 4.33(q, J=7.1 Hz, 2H), 4.15-3.90(m, 6H), 3.86(s, 3H), 3.75-3.55(m, 7H), 3.33(s, 3H), 3.20-3.15(m, 1H), 2.85-2.80(m, 1H), 2.70-2.60(m, 1H), 2.15-1.90(m, 6H), 1.87-1.75(m, 2H), 1.37(t, J=7.1 Hz, 3H), 1.23-1.17(m, 6H), 1.02-0.93(m, 4H).
MS (ESI+) 638(M+1, 100%).

### Example 89

### N-[(rac.)-(3R,6R)-6-(2-{Cyclopropyl[(4-fluorophenyl)acetyl]amino}ethyl)piperidin-3-yl]-N-isopropyl-4-methoxy-3-(3-methoxypropoxy)benzamide hydrochloride

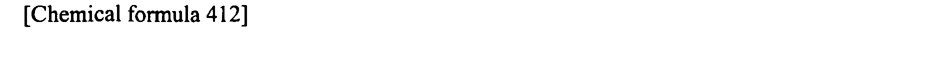

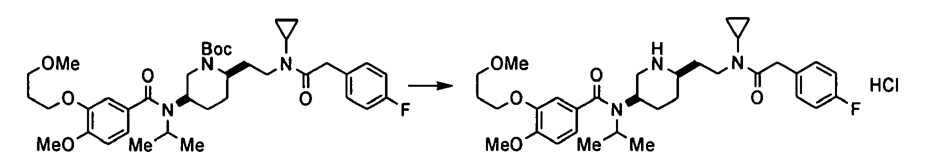

The title compound (3.8 mg) was synthesized in a similar manner to Example 4.
¹H-NMR (400MHz, MeOD) 7.22-7.19(m, 2H), 6.97-6.92(m, 3H), 6.87-6.85(m, 2H), 4.00-3.95(m, 3H), 3.88(s, 2H), 3.77(s, 3H), 3.68-3.41(m, 7H), 3.24(s, 3H), 3.08-3.06(m, 1H), 2.73(brs, 1H), 2.59(brs, 1H), 1.97-1.88(m, 6H), 1.71-1.69(m, 1H), 1.20-1.09(m, 6H), 0.92-0.83(m, 4H).
MS (ESI+) 584(M⁺+1, 100%).

### Example 90

### N-((rac.)-(3R,6R)-6-{2-[Benzoyl(cyclopropyl)amino]ethyl}piperidin-3-yl)-N-isopropyl-4-methoxy-3-(3-methoxypropoxy)benzamide hydrochloride

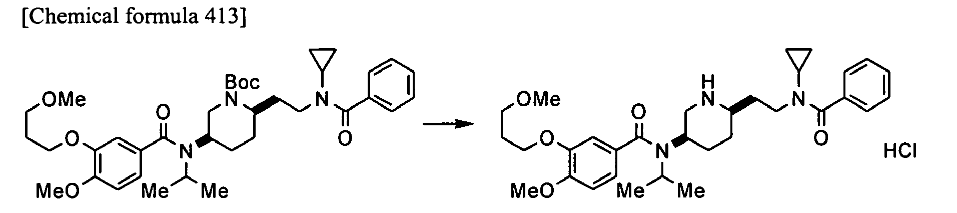

The title compound (3.8 mg) was synthesized in a similar manner to Example 4.
¹H-NMR (400MHz, MeOD) 7.46-7.42(m, 5H), 6.95-6.86(m, 3H), 4.01-3.95(m, 3H), 3.77(s, 3H), 3.67-3.54(m, 5H), 3.49-3.41(m, 3H), 3.24(s, 3H), 2.86(brs, 1H), 2.64(brs, 1H), 2.22-2.16(m, 1H), 1.97-1.91(m, 3H), 1.78(m, 1H), 1.51(m, 1H), 1.24-1.10(m, 7H), 0.54(brs, 2H), 0.42(brs, 2H).
MS (ESI+) 552(M⁺+1, 100%).

### Example 91

### N-((rac.)-(3R,6R)-6-{2-[Cyclopropyl(3-phenylpropanoyl)amino]ethyl}piperidin-3-yl)-N-isopropyl-4-methoxy-3-(3-methoxypropoxy)benzamide hydrochloride

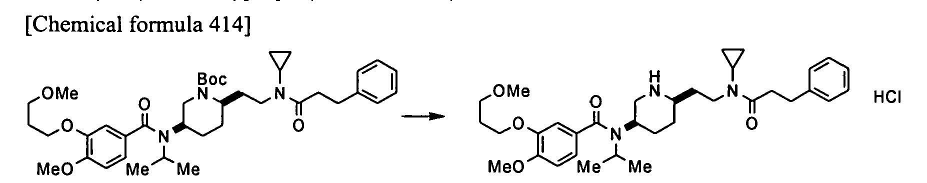

The title compound (9.7 mg) was synthesized in a similar manner to Example 4.
¹H-NMR (400MHz, MeOD) 7.20-7.10(m, 5H), 6.94(d, J = 8.4 Hz, 1H), 6.89-6.86(m, 2H), 4.01-3.98(m, 3H), 3.90-3.82(m, 1H), 3.78(s, 3H), 3.65-3.47(m, 7H), 3.37-3.30(m, 1H), 3.25(s, 3H), 3.07(m, 2H), 2.62-2.50(m, 3H), 1.98-1.85(m, 7H), 1.21-1.11(m, 6H), 0.87-0.79(m, 2H), 0.74-0.68(m, 2H).
MS (ESI+) 580(M⁺+1, 100%).

### Example 92

### N-Isopropyl-3-(3-methoxypropoxy)-N-[(3R)-piperidin-3-yl]benzamide hydrochloride

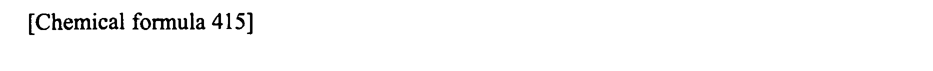

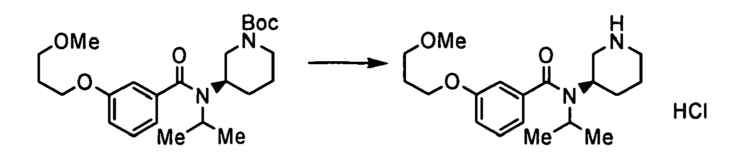

The title compound (193 mg) was synthesized in a similar manner to Example 4.
¹H-NMR (400MHz, MeOD) 7.33-7.29(m, 1H), 6.97-6.94(m, 1H), 6.84-6.83(m, 2H), 4.03-4.00(m, 3H), 3.84(brs, 1H), 3.68-3.59(m, 1H), 3.53-3.48(m, 2H), 3.27-3.24(m, 5H), 2.90-2.87(m, 1H), 2.69(brs, 1H), 2.06-1.83(m, 5H), 1.16(brs, 6H).
MS (ESI+) 335(M⁺+1, 100%).

### Example 93

### N-Isopropyl-4-methoxy-3-(3-methoxypropoxy)-N-[(2R,3R)-2-methylpiperidin-3-yl]benzamide

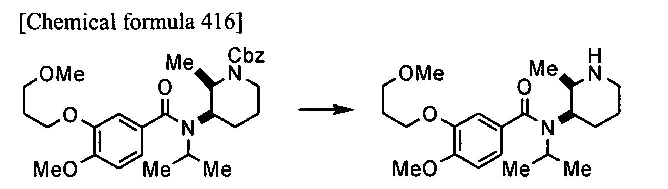

The title compound (1.6 mg) was synthesized in a similar manner to Example 2.
¹H-NMR (400MHz, CDCl₃) 6.91-6.82(m, 3H), 4.24-4.19(m, 1H), 4.15-4.10(m, 3H), 3.90(s, 3H), 3.65-3.55(m, 2H), 3.36-3.34(m, 5H), 3.14(brs, 1H), 3.03-2.95(m, 1H), 2.24(m, 1H), 2.15-2.00(m, 5H), 1.44-1.33(m, 3H), 1.27-1.18(m, 6H).
MS (ESI+) 379(M⁺+1, 100%).

### Example 94

### N-Isopropyl-3,5-bis(3-methoxypropoxy)-N-[(3R)-piperidin-3-yl]benzamide hydrochloride

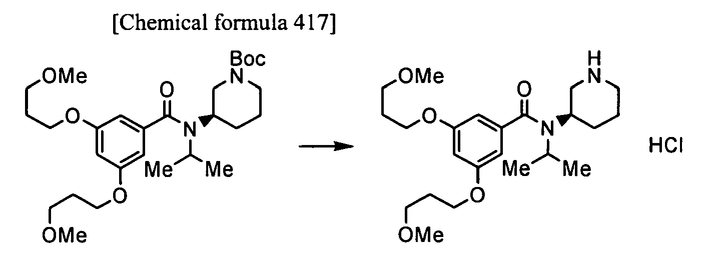

The title compound (338 mg) was synthesized in a similar manner to Example 4.
¹H NMR (400 MHz, DMSO-d₆) 9.27-9.16(m, 2H), 6.51(t, J = 2.2 Hz, 1H), 6.42(d, J = 2.2 Hz, 2H), 4.01 (t, J = 6.4 Hz, 4H), 3.73-3.59(m, 2H), 3.49-3.40(m, 7H), 3.24(s, 6H), 3.14-3.12(m, 1H), 2.76-2.73(m, 1H), 2.55-2.52(m, 1H), 2.02-1.71(m, 6H), 1.14-1.08(m, 6H).
MS (ESI+) 423(M⁺+1, 100%).

### Example 95

### 4-Bromo-N-isopropyl-3-(3-methoxypropoxy)-N-[(3R)-piperidin-3-yl]benzamide hydrochloride

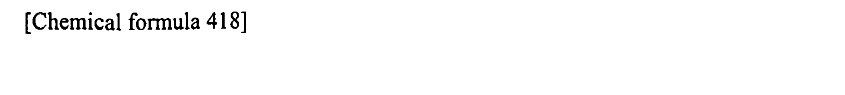

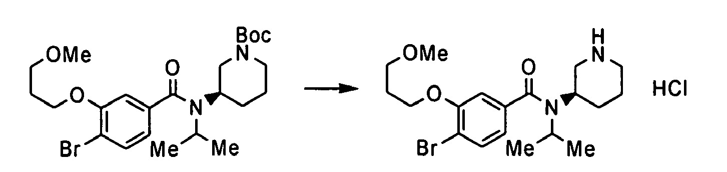

The title compound (83.5 mg) was synthesized in a similar manner to Example 4.
¹H-NMR (300MHz, MeOD) 7.63(d, J = 7.8 Hz, 1H), 7.02(d, J = 1.8 Hz, 1H), 6.83(dd, J = 1.8, 7.8 Hz, 2H), 4.16(t, J = 6.3 Hz, 2H), 4.04-3.90(m, 2H), 3.74-3.56(m, 4H), 3.35(m, 4H), 2.98-2.90(m, 1H), 2.75(brs, 1H), 2.11-2.03(m, 3H), 1.94-1.90(m, 2H), 1.24(brs, 6H).
MS (ESI+) 413(M⁺+1, 50%), 415(M⁺+1, 50%).

### Example 96

### 3-(Benzyloxy)-N-isopropyl-5-(3-methoxypropoxy)-N-[(3R)-piperidin-3-yl]benzamide hydrochloride

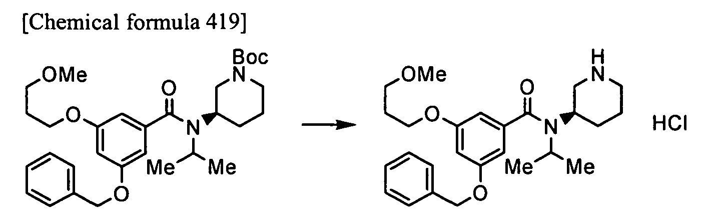

The title compound (50.6 mg) was synthesized in a similar manner to Example 4.
¹H-NMR (300MHz, MeOD) 7.43-7.32(m, 5H), 6.66-6.64(m, 1H), 6.52-6.47(m, 2H), 5.10(s, 2H), 4.05(m, 3H), 3.88(brs, 1H), 3.75-3.53(m, 6H), 2.96-2.87(m, 1H), 2.74(m, 1H), 2.04-1.96(m, 3H), 1.89-1.86(m, 2H), 1.17(brs, 6H).
MS (ESI+) 441(M⁺+1, 100%).

### Example 97

### 3-Hydroxy-N-isopropyl-5-(3-methoxypropoxy)-N-[(3R)-piperidin-3-yl]benzamide hydrochloride

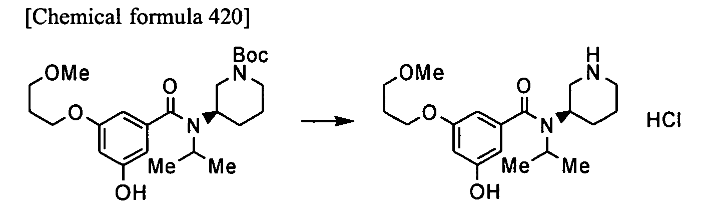

The title compound (30.2 mg) was synthesized in a similar manner to Example 4.
¹H-NMR(300MHz, MeOD) 6.44-6.42(m, 1H), 6.36-6.31(m, 2H), 4.04-4.00(m, 4H), 3.74-3.63(m, 2H), 3.59-3.53(m, 3H), 3.35(s, 3H), 2.97-2.90(m, 1H), 2.73(brs, 1H), 2.09-1.96(m, 3H), 1.91-1.87(m, 2H), 1.22(brs, 6H).
MS (ESI+) 351(M⁺+1, 100%).

### Example 98

### N-Isopropyl-3-(3-methoxypropoxy)-5-phenoxy-N-[(3R)-piperidin-3-yl]benzamide hydrochloride

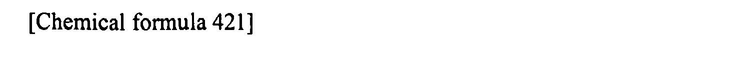

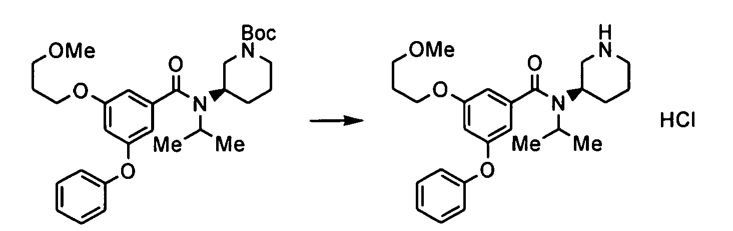

The title compound (42.7 mg) was synthesized in a similar manner to Example 4.
¹H-NMR (400MHz, MeOD) 7.41-7.37(m, 2H), 7.19-7.15(m, 1H), 7.05-7.03(m, 2H), 6.63-6.62(m, 2H), 6.43(m, 1H), 4.06-3.94(m, 4H), 3.75-3.66(m, 1H), 3.59-3.48(m, 3H), 3.37-3.32(m, 4H), 2.95-2.89(m, 1H), 2.70(brs, 1H), 2.07-1.97(m, 3H), 1.87-1.84(m, 2H), 1.19(brs, 6H).
MS (ESI+) 427(M⁺+1, 100%).

### Example 99

### N-Isopropyl-3-methoxy-5-(3-methoxypropoxy)-N-[(3R)-piperidin-3-yl]benzamide hydrochloride

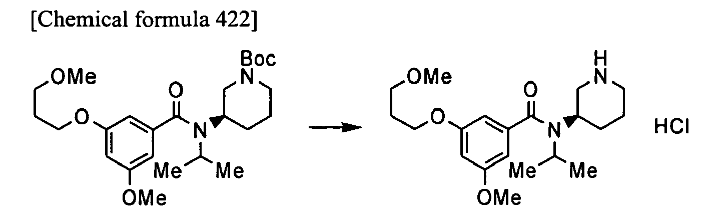

The title compound (63.0 mg) was synthesized in a similar manner to Example 4.
¹H-NMR (400MHz, MeOD) 6.57-6.56(m, 1H), 6.46(brs, 2H), 4.07-4.04(m, 3H), 3.93-3.85(m, 1H), 3.80(s, 3H), 3.75-3.66(m, 2H), 3.59-3.54(m, 2H), 3.39(m, 1H), 3.34(s, 3H), 2.96-2.93(m, 1H), 2.74(brs, 1H), 2.04-1.98(m, 3H), 1.91-1.89(m, 2H), 1.22(brs, 6H).
MS (ESI+) 365(M⁺+1, 100%).

### Example 100

### 3-(Cyclopropylmethoxy)-N-isopropyl-5-(3-methoxypropoxy)-N-[(3R)-piperidin-3-yl]benzamide hydrochloride

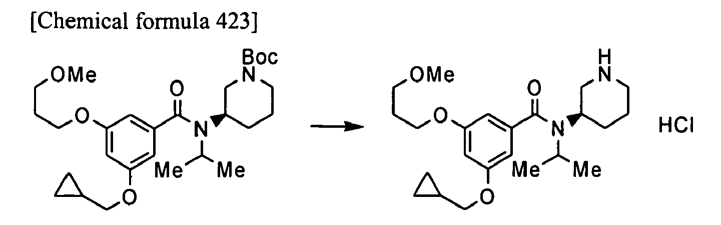

The title compound (77.0 mg) was synthesized in a similar manner to Example 4.
¹H-NMR (400MHz, MeOD) 6.55(m, 1H), 6.45(m, 2H), 4.07-4.04(m, 3H), 3.93(brs, 1H), 3.83-3.81(m, 2H), 3.74-3.66(m, 2H), 3.59-3.54(m, 2H), 3.39(m, 1H), 3.34(s, 3H), 2.93(m, 1H), 2.74(brs, 1H), 2.04-1.99(m, 3H), 1.91-1.89(m, 2H), 1.22(brs, 6H), 0.62-0.60(m, 2H), 0.35-0.34(m, 2H).
MS (ESI+) 405(M⁺+1, 100%).

### Example 101

### 3-Isopropyl-N-isopropyl-5-(3-methoxypropoxy)-N-[(3R)-piperidin-3-yl]benzamide hydrochloride

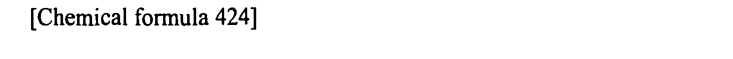

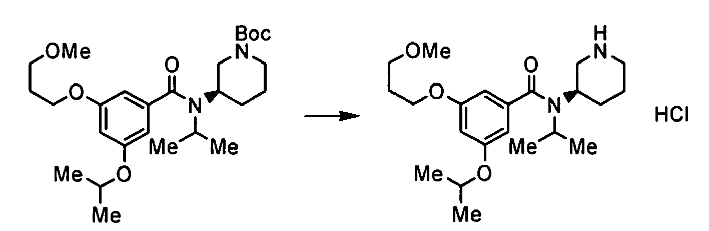

The title compound (68.0 mg) was synthesized in a similar manner to Example 4.
¹H-NMR (400MHz, MeOD) 6.54-6.53(m, 1H), 6.44-6.43(m, 2H), 4.64-4.57(m, 1H), 4.06-4.03(m, 3H), 3.95(brs, 1H), 3.75-3.66(m, 1H), 3.59-3.52(m, 3H), 3.39(m, 1H), 3.34(s, 3H), 2.97-2.91(m, 1H), 2.74(brs, 1H), 2.08-1.98(m, 3H), 1.92-1.89(m, 2H), 1.30(d, J = 6.0 Hz, 6H), 1.22(brs, 6H).
MS (ESI+) 393(M⁺+1, 100%).

### Example 102

### 3-(Cyclohexyloxy)-N-isopropyl-5-(3-methoxypropoxy)-N-[(3R)-piperidin-3-yl]benzamide hydrochloride

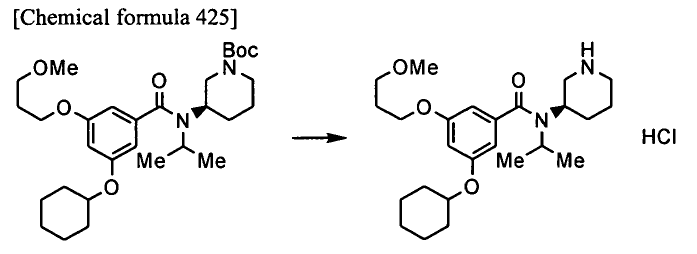

The title compound (68.0 mg) was synthesized in a similar manner to Example 4.
¹H-NMR (400MHz, MeOD) 6.54-6.53(m, 1H), 6.44(m, 2H), 4.32-4.30(m, 2H), 4.06-3.95(m, 4H), 3.57-3.54(m, 3H), 3.39(m, 1H), 3.34(s, 3H), 2.94(m, 1H), 2.74(brs, 1H), 2.05-1.89(m, 3H), 1.78(m, 2H), 1.59-1.22(m, 16H).
MS (ESI+) 433(M⁺+1, 100%).

### Example 103

### N-Isopropyl-5-(3-methoxypropoxy)-N-[(3R)-piperidin-3-yl]biphenyl-3-carboxyamide hydrochloride

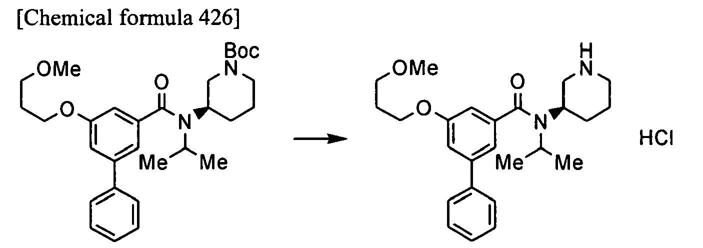

The title compound (59.0 mg) was synthesized in a similar manner to Example 4.
¹H-NMR(300MHz, MeOD) 7.63-7.60(m, 2H), 7.48-7.37(m, 3H), 7.26-7.24(m, 1H), 7.14-7.13(m, 1H), 6.89-6.88(m, 1H), 4.18-4.03(m, 4H), 3.74-3.57(m, 4H), 3.39(m, 1H), 3.35(s, 3H), 2.96(m, 1H), 2.79(m, 1H), 2.10-2.02(m, 3H), 1.93(m, 2H), 1.25(brs, 6H).
MS (ESI+) 411(M⁺+1, 100%).

### Example 104

### 4-(Benzyloxy)-N-isopropyl-3-(3-methoxypropoxy)-N-[(3R)-piperidin-3-yl]benzamide hydrochloride

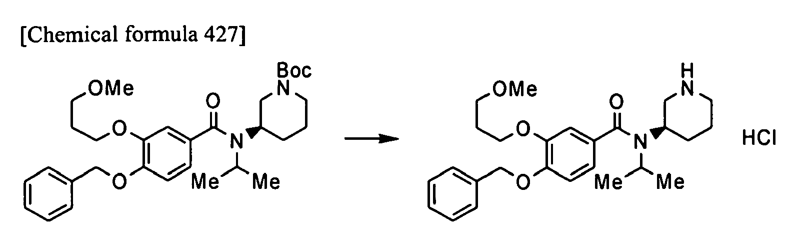

The title compound (38.0 mg) was synthesized in a similar manner to Example 4.
¹H-NMR (400MHz, MeOD) 7.46(m, 2H), 7.38-7.34(m, 2H), 7.32-7.28(m, 1H), 7.07(d, J = 8.2 Hz, 1H), 6.98(d, J = 1.8 Hz, 1H), 6.89(dd, J = 1.8, 8.2 Hz, 1H), 5.14(s, 2H),4.1 1(t, J = 6.2 Hz, 2H), 4.00(brs, 1H), 3.75-3.56(m, 5H), 3.39(m, 1H), 3.30(s, 3H), 2.97-2.90(m, 1H), 2.69(brs, 1H), 2.07-2.01(m, 3H), 1.91-1.88(m, 2H), 1.24(brs, 6H).
MS (ESI+) 441(M⁺+1, 100%).

### Example 105

### 4-Hydroxy-N-isopropyl-3-(3-methoxypropoxy)-N-[(3R)-piperidin-3-yl]benzamide hydrochloride

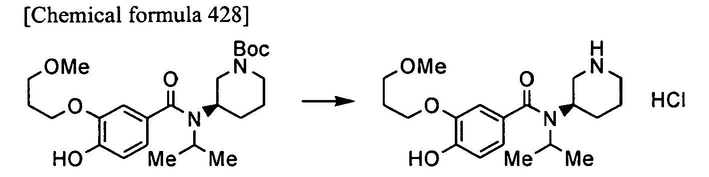

The title compound (65.0 mg) was synthesized in a similar manner to Example 4.
¹H-NMR (400MHz, MeOD) 6.93(d, J = 1.7 Hz, 1H), 6.86(d, J = 8.1 Hz, 1H), 6.82(dd, J = 1.7, 8.1 Hz, 1H), 4.14-4.11(m, 3H), 4.04(brs, 1H), 3.61-3.52(m, 3H), 3.39-3.35(m, 5H), 2.98-2.90(m, 1H), 2.78(brs, 1H), 2.09-2.03(m, 3H), 1.92-1.82(m, 2H), 1.26(brs, 6H).
MS (ESI+) 351(M⁺+1, 100%).

### Example 106

### 4-Butyl-N-isopropyl-3-(3-methoxypropoxy)-N-[(3R)-piperidin-3-yl]benzamide hydrochloride

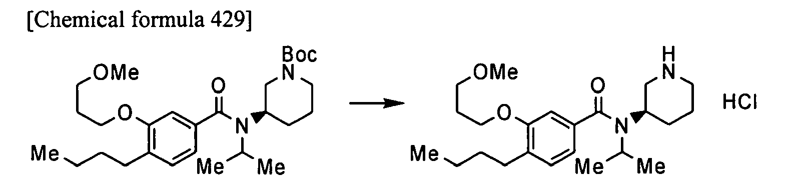

The title compound (8.0 mg) was synthesized in a similar manner to Example 4.
¹H-NMR (400MHz, MeOD) 7.21-7.19(m, 1H), 6.87-6.83(m, 2H), 4.35 -4.29(m, 2H), 4.22-4.16(m, 1H), 4.10-4.06(m, 2H), 3.74-3.65(m, 3H), 3.35-3.32(m, 4H), 2.95(m, 1H), 2.78-2.62(m, 3H), 2.08-2.01(m, 3H), 1.90(m, 2H), 1.59-1.54(m, 2H), 1.41-1.24(m, 8H), 0.94(t, J = 7.2 Hz, 3H).
MS (ESI+) 391(M⁺+1, 100%).

### Example 107

### N-Isopropyl-3-(3-methoxypropoxy)-4-phenoxyN-[(3R)-piperidin-3-yl]benzamide hydrochloride

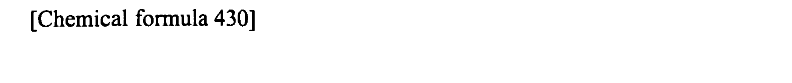

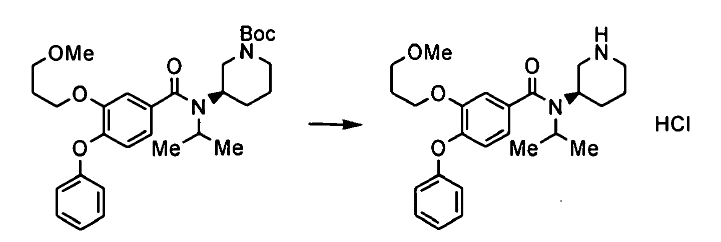

The title compound (48.8 mg) was synthesized in a similar manner to Example 4.
¹H-NMR (400MHz, MeOD) 7.32-7.28(m, 2H), 7.11-7.02(m, 3H), 6.97-6.95(m, 1H), 6.89-6.87(m, 2H), 4.06-4.03(m, 4H), 3.75-3.65(m, 1H), 3.40-3.35(m, 2H), 3.23(t, J = 6.2 Hz, 2H), 3.19(s, 3H), 2.98-2.92(m, 1H), 2.76(brs, 1H), 2.09-2.04(m, 1H), 1.94-1.81(m, 4H), 1.27(brs, 6H).
MS (ESI+) 427(M⁺+1, 100%).

### Example 108

### N-Isopropyl-3-(3-methoxypropoxy)-4-(2-phenylethyl)-N-[(3R)-piperidin-3-yl]benzamide hydrochloride

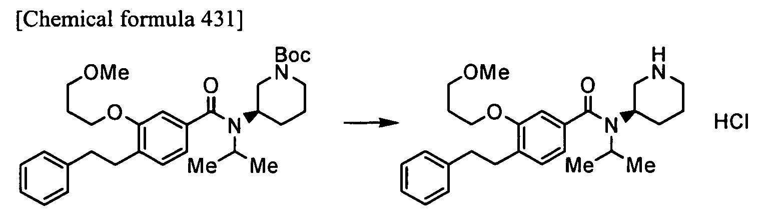

The title compound (9.4 mg) was synthesized in a similar manner to Example 4.
¹H-NMR (400MHz, MeOD) 7.24-7.20(m, 2H), 7.14-7.13(m, 4H), 6.89-6.88(m, 1H), 6.81-6.79(m, 1H), 4.32-4.30(m, 6H), 4.10-4.07(m, 3H), 3.79-3.61(m, 1H), 3.35(s, 3H), 2.95-2.87(m, 5H),2.11-2.05(m, 3H), 1.93-1.90(m, 2H), 1.23(brs, 6H).
MS (ESI+) 439(M⁺+1, 100%).

### Example 109

### 4-Isopropoxy-N-isopropyl-3-(3-methoxypropoxy)-N-[(3R)-piperidin-3-yl]benzamide hydrochloride

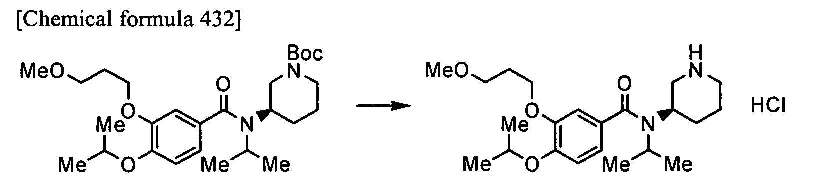

The title compound (49.8 mg) was synthesized in a similar manner to Example 4.
¹H-NMR (400MHz, MeOD) 7.02(d, J = 8.2 Hz, 1H), 6.96(d, J = 1.8 Hz, 1H), 6.90(dd, J = 1.8, 8.2 Hz, 1H),4.62-4.56(m, 1H), 4.17-4.02(m, 4H), 3.77-3.63(m, 4H), 3.39(m, 1H), 3.34(s, 3H), 2.97-2.91(m, 1H), 2.70(brs, 1H), 2.07-2.00(m, 3H), 1.92-1.89(m, 2H), 1.32(d, J = 6.1 Hz, 6H), 1.25(m, 6H).
MS (ESI+) 393(M⁺+1, 100%).

### Example 110

### 4-(Cyclopropylmethoxy)-N-isopropyl-3-(3-methoxypropoxy)-N-[(3R)-piperidin-3-yl]benzamide hydrochloride

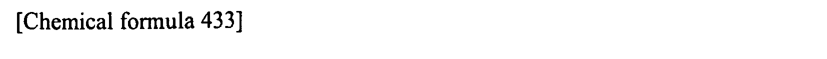

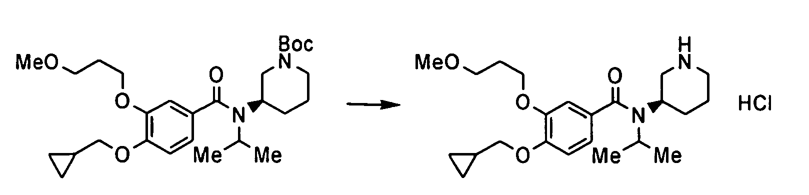

The title compound (48.2 mg) was synthesized in a similar manner to Example 4.
¹H-NMR (400MHz, MeOD) 6.99-6.90(m, 3H), 4.14-4.09(m, 2H), 4.00(m, 2H), 3.87(m, 2H), 3.72-3.59(m, 4H), 3.39(m, 1H), 3.34(s, 3H), 2.92(m, 1H), 2.69(brs, 1H), 2.04(m, 3H), 1.88(m, 2H), 1.24(m, 7H), 0.60(m, 2H), 0.35(m, 2H).
MS (ESI+) 405(M⁺+1, 100%).

### Example 111

### 4-(Cyclohexyloxy)-N-isopropyl-3-(3-methoxypropoxy)-N-[(3R)-piperidin-3-yl]benzamide hydrochloride

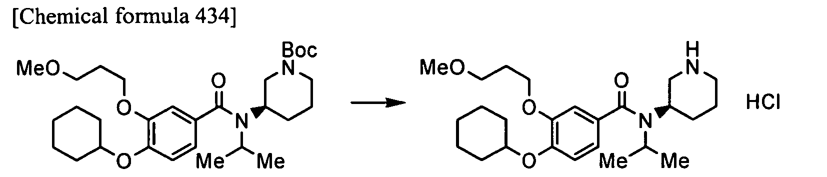

The title compound (10.0 mg) was synthesized in a similar manner to Example 4.

¹H-NMR (400MHz, MeOD) 7.03-7.01(m, 1H), 6.96-6.95(m, 1H), 6.89(m, 1H), 4.34-4.30(m, 2H), 4.11-3.98(m, 4H), 3.80-3.58(m, 3H), 3.39(m, 1H), 3.34(s, 3H), 2.92(m, 1H), 2.69(brs, 1H), 2.09-2.01(m, 3H), 1.92-1.80(m, 6H), 1.58-1.53(m, 3H), 1.44-1.35(m, 3H), 1.25(brs, 6H).
MS (ESI+) 433(M⁺+1, 100%).

### Example 112

### 2-Bromo-4-isopropoxy-N-isopropyl-5-(3-methoxypropoxy)-N-[(3R)-piperidin-3-yl]benzamide hydrochloride

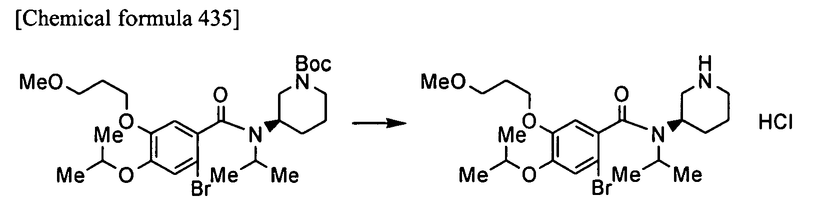

The title compound (27.5 mg) was synthesized in a similar manner to Example 4.
¹H-NMR (400MHz, MeOD) 7.18(m, 1H), 6.93-6.89(m, 1H), 4.59-4.56(m, 1H), 4.09-4.05(m, 3H), 3.79-3.56(m, 4H), 3.39-3.36(m, 2H), 3.34(s, 3H), 2.97(m, 1H), 2.80(m, 1H), 2.11-1.99(m, 3H), 1.95-1.86(m, 2H), 1.35-1.28(m, 9H), 1.19-1.16(m, 3H).
MS (ESI+) 471(M⁺+1, 50%), 473(M⁺+1, 50%).

### Example 113

### 5-Isopropoxy-N-isopropyl-4-(3-methoxypropoxy)-N-[(3R)-piperidin-3-yl]biphenyl-2-carboxamide hydrochloride

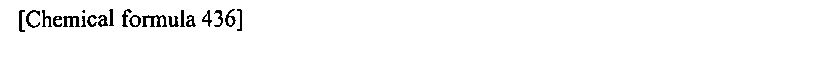

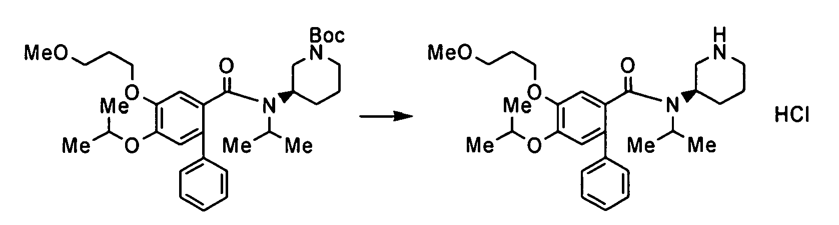

The title compound (6.0 mg) was synthesized in a similar manner to Example 4.
¹H-NMR (400MHz, MeOD) 7.48-7.35(m, 5H), 7.01(s, 1H), 6.93-6.89(m, 1H), 4.67-4.61(m, 1H), 4.31-4.30(m, 2H), 4.13(m, 2H), 3.78-3.36(m, 6H), 3.31(s, 3H), 2.99-2.85(m, 1H), 2.09-2.03(m, 3H), 1.82-1.73(m, 2H), 1.38-1.26(m, 9H), 1.00(d, J = 6.6 Hz, 3H).
MS (ESI+) 469(M⁺+1, 100%).

### Example 114

### 4-Ethoxy-N-isopropyl-3-(3-methoxypropoxy)-N-[(3R)-piperidin-3-yl]benzamide hydrochloride

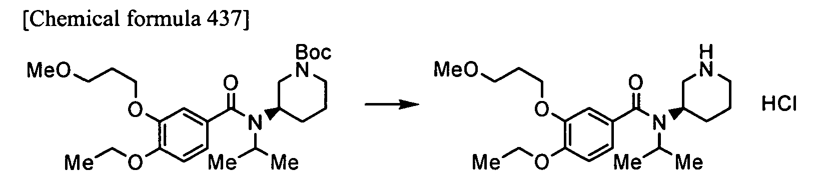

The title compound (59.5 mg) was synthesized in a similar manner to Example 4.
¹H-NMR (400MHz, MeOD) 7.00(d, J = 8.2 Hz, 1H), 6.95(d, J = 1.8 Hz, 1H), 6.91(dd, J = 1.8, 8.2 Hz, 1H), 4.12-4.02(m, 6H), 3.74-3.57(m, 4H), 3.39(m, 1H), 3.34(s, 3H), 2.97-2.91 (m, 1H), 2.70(brs, 1H), 2.08-2.00(m, 3H), 1.92-1.89(m, 2H), 1.41(t, J = 7.0 Hz, 3H), 1.25(brs, 6H).
MS (ESI+) 379(M⁺+1, 100%).

### Example 115

### N-Isopropyl-3-(3-methoxypropoxy)-N-[(3R)-piperidin-3-yl]-4-propoxybenzamide hydrochloride

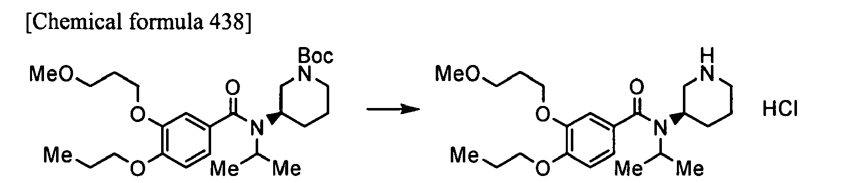

The title compound (59.1 mg) was synthesized in a similar manner to Example 4.
¹H-NMR (400MHz, MeOD) 7.00(d, J = 8.2 Hz, 1H), 6.95(d, J = 1.8 Hz, 1H), 6.91 (dd, J = 1.8, 8.2 Hz, 1H), 4.09(t, J = 6.3 Hz, 2H), 4.01-3.98(m, 3H), 3.75-3.58(m, 5H), 3.39(m, 1H), 3.34(s, 3H), 2.98-2.91(m, 1H), 2.70(brs, 1H), 2.05-2.00(m, 3H), 1.93-1.78(m, 4H), 1.26-1.24(m, 6H), 1.06(t, J = 7.4 Hz, 3H).
MS (ESI+) 393(M⁺+1, 100%).

### Example 116

### 4-(2-Fluoroethoxy)-N-isopropyl-3-(3-methoxypropoxy)-N-[(3R)-piperidin-3-yl]benzamide hydrochloride

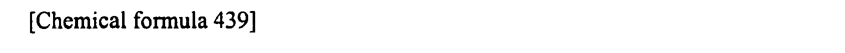

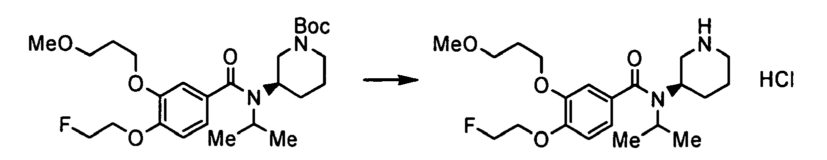

The title compound (60.0 mg) was synthesized in a similar manner to Example 4.
¹H-NMR (400MHz, MeOD) 7.05(d, J = 8.2 Hz, 1H), 6.98(d, J = 1.8 Hz, 1H), 6.92(dd, J = 1.8, 8.2 Hz, 1H), 4.83-4.78(m, 1H), 4.68-4.66(m, 1H), 4.35-4.30(m, 1H), 4.25-4.23(m, 1H), 4.11(t, J = 6.2 Hz, 2H), 4.00(m, 2H), 3.87-3.55(m, 4H), 3.39(m, 1H), 3.34(s, 3H), 2.97-2.91 (m, 1H), 2.69(brs, 1H), 2.09-2.0 1 (m, 3H), 1.93-1.90(m, 2H), 1.25(m, 6H).
MS (ESI+) 397(M⁺+1, 100%).

### Example 117

### 3-[4-Chloro-5-({isopropyl[(3R)-piperidin-3-yl]amino}carbonyl)-2-methoxyphenoxy]propanoic acid hydrochloride

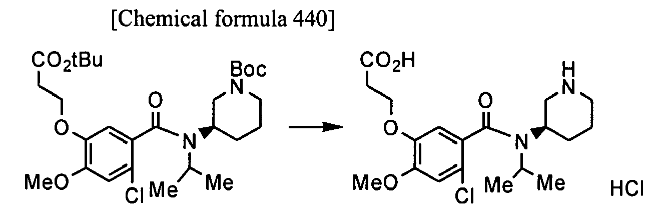

The title compound (7.0 mg) was synthesized in a similar manner to Example 4.
MS (ESI+) 399(M⁺+1, 100%).

### Example 118

### 4-Isopropoxy-N-isopropyl-5-(3-methoxypropoxy)-N-[(3R)-piperidin-3-yl]-2-propylbenzamide hydrochloride

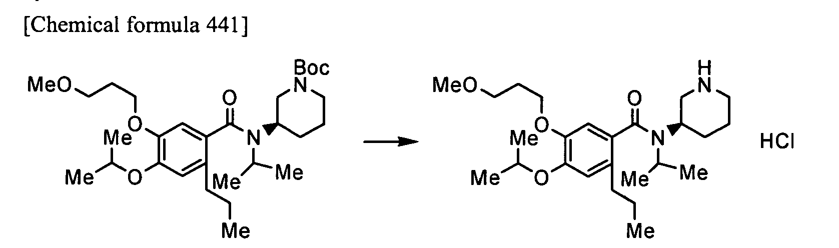

The title compound (12.7 mg) was synthesized in a similar manner to Example 4.
¹H-NMR (400MHz, MeOD) 6.88(m, 1H), 6.77-6.73(m, 1H), 4.57-4.54(m, 1H), 4.12-4.03(m, 3H), 3.84-3.56(m, 5H), 3.41-3.39(m, 1H), 3.34(s, 3H), 2.96(m, 1H), 2.80(m, 1H), 2.51-2.44(m, 2H), 2.10-1.98(m, 3H), 1.91-1.89(m, 2H), 1.67-1.56(m, 2H), 1.31(t, J = 6.0 Hz, 6H), 1.21-1.17(m, 6H), 0.95(t, J = 7.3 Hz, 3H).
MS (ESI+) 435(M⁺+1, 100%).

### Example 119

### N-Isopropyl-3-(3-methoxypropoxy)-4-morpholin-4-yl-N-[(3R)-piperidin-3-yl]benzamide hydrochloride

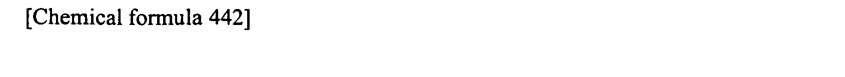

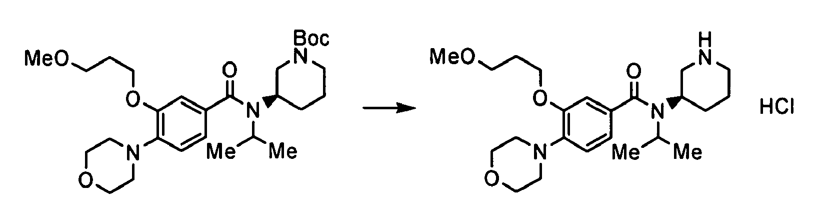

The title compound (12.7 mg) was synthesized in a similar manner to Example 4.
¹H-NMR (400MHz, MeOD) 7.62(d, J = 8.2 Hz, 1H), 7.24(s, 1H), 7.10(d, J = 8.2 Hz, 1H), 4.31(t, J = 6.2 Hz, 2H), 4.08-4.06(m, 5H), 3.89-3.87(m, 1H), 3.75-3.58(m, 7H), 3.39-3.36(m, 5H), 2.95(m, 1H), 2.78(brs, 1H), 2.19-2.13(m, 2H), 2.09-2.05(m, 1H), 1.95-1.89(m, 2H), 1.24(brs, 6H).
MS (ESI+) 420(M⁺+1, 100%).

### Example 120

### Diethyl 4-[4-({isopropyl[(3R)-piperidin-3-yl]amino}carbonyl)-2-(3-methoxypropoxy)phenyl-2,6-dimethyl-1,4-dihydropyridine-3,5-dicarboxylate

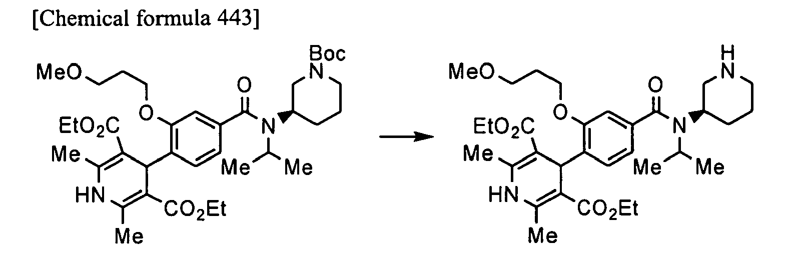

The title compound (17.3 mg) was synthesized in a similar manner to Example 10.
¹H-NMR (400MHz, MeOD) 7.21(d, J = 7.6 Hz, 1H), 6.72-6.69(m, 2H), 5.75(s,1H), 5.19(s, 1H), 4.05-3.95(m, 8H), 3.52(t, J = 6.6 Hz, 2H), 3.38-3.33(m, 1H), 3.36(s, 3H), 3.00-2.98(m, 2H), 2.70-2.56(m, 3H), 2.26(s, 6H), 2.06-1.99(m, 2H), 1.77(m, 2H), 1.34-1.13(m, 12H).
MS (ESI+) 586(M⁺+1, 100%).

### Example 121

### 2-Chloro-4-isopropoxy-N-isopropyl-5-(3-methoxypropoxy)-N-[(3R)-piperidin-3-yl]benzamide hydrochloride

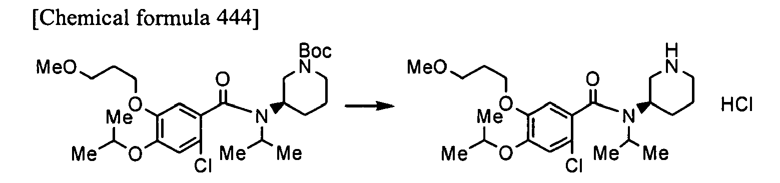

The title compound (69.7 mg) was synthesized in a similar manner to Example 4.

¹H-NMR (400MHz, MeOD) 7.05-7.04(m, 1H), 6.93-6.89(m, 1H), 4.62-4.56(m, 1H), 4.12-4.05(m, 3H), 3.79-3.72(m, 1H), 3.67-3.56(m, 4H), 3.41-3.38(m, 1H), 3.35(s, 3H), 2.97(m, 1H), 2.81(m, 1H), 2.11-1.97(m, 3H), 1.94-1.85(m, 2H), 1.38-1.16(m, 12H).
MS (ESI+) 427(M⁺+1, 100%).

### Example 122

### Methyl {2-[4-chloro-5-({isopropyl[(3R)-piperidin-3-yl]amino}carbonyl-2-methoxyphenoxy]ethyl}carbamate hydrochloride

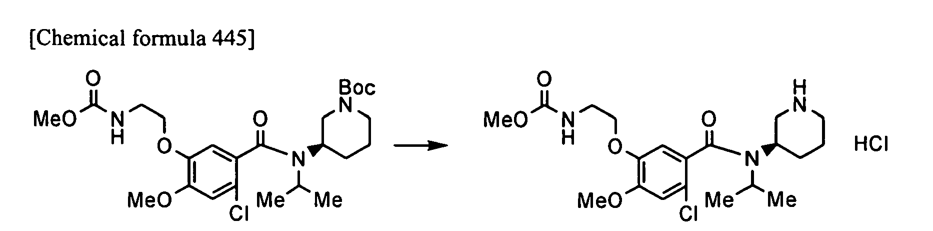

The title compound (50.0 mg) was synthesized in a similar manner to Example 4.
¹H-NMR (400MHz, MeOD) 7.08-7.07(m, 1H), 6.97-6.93(m, 1H), 4.12-4.02(m, 3H), 3.87(s, 3H), 3.77-3.72(m, 1H), 3.68-3.63(m, 4H), 3.59-3.52(m, 1H), 3.48-3.45(m, 2H), 3.40-3.37(m, 1H), 2.11-2.07(m, 1H), 1.94-1.82(m, 2H), 1.27-1.16(m, 6H).
MS (ESI+) 428(M⁺+1, 100%).

### Example 123

### Methyl {2-[2-bromo-5-({isopropyl[(3R)-piperidin-3-yl]amino}carbonyl)phenoxy]ethyl}carbamate hydrochloride

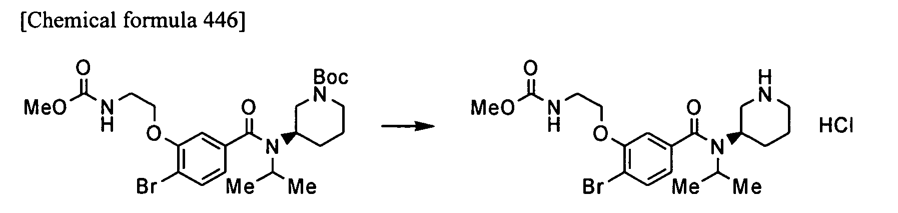

The title compound (24.9 mg) was synthesized in a similar manner to Example 4.

¹H-NMR (400MHz, MeOD) 7.63(d, J = 8.0 Hz, 1H), 7.05(d, J = 1.7 Hz, 1H), 6.86(dd, J = 1.7, 8.0 Hz, 1H), 4.12(t, J = 5.7 Hz, 1H), 4.05(brs, 1H), 3.90-3.88(m, 1H), 3.64(s, 3H), 3.59-3.48(m, 3H), 3.35(m, 2H), 2.94-2.91(m, 1H), 2.76(brs, 1H), 2.12-2.06(m, 1H), 1.97-1.84(m, 2H), 1.23(brs, 6H). MS (ESI+) 442(M⁺+1, 50%), 444(M⁺+1, 50%).

### Example 124

### Methyl {3-[5-({isopropyl[(3R)-piperidin-3-yl]amino}carbonyl)-2-methoxyphenoxy]propyl}carbamate hydrochloride

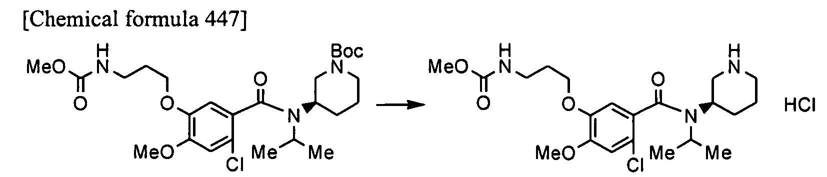

The title compound (114 mg) was synthesized in a similar manner to Example 4.
¹H-NMR (400MHz, MeOD) 7.06-7.05(m, 1H), 6.92-6.88(m, 1H), 4.13-4.03(m, 3H), 3.86(s, 3H), 3.77-3.72(m, 1H), 3.67-3.57(m, 5H), 3.41-3.28(m, 3H), 2.98-2.91 (m, 1H), 2.85-2.77(m, 1H), 2.11-2.07(m, 1H), 1.99-1.84(m, 4H), 1.28-1.16(m, 6H).
MS (ESI+) 442(M⁺+1, 100%).

### Example 125

### 4-(Cyclopentyloxy)-N-isopropyl-3-(3-methoxypropoxy)-N-[(3R)-piperidin-3-yl]benzamide hydrochloride

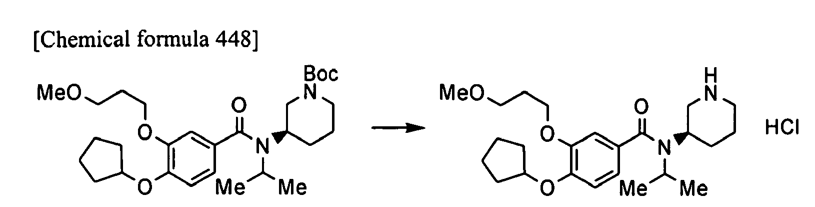

The title compound (144 mg) was synthesized in a similar manner to Example 4.
¹H-NMR (400MHz, MeOD) 7.00(d, J = 8.2 Hz, 1H), 6.94(d, J = 1.9 Hz, 1H), 6.91 (dd, J = 1.9, 8.2 Hz, 1H), 4.09-4.04(m, 4H), 3.60-3.57(m, 3H), 3.39-3.30(m, 6H), 2.97-2.90(m, 1H), 2.69(brs, 1H), 2.08-1.99(m, 3H), 1.94-1.79(m, 8H), 1.69-1.64(m, 2H), 1.26(brs, 6H).
MS (ESI+) 419(M⁺+1, 100%).

### Example 126

### N-Isopropyl-4-(4-methoxybutyl)-2,2-dimethyl-3-oxo-N-[(3R)-piperidin-3-yl]-3,4-dihydro-2H-1,4-benzoxazine-6-carboxyamide hydrochloride

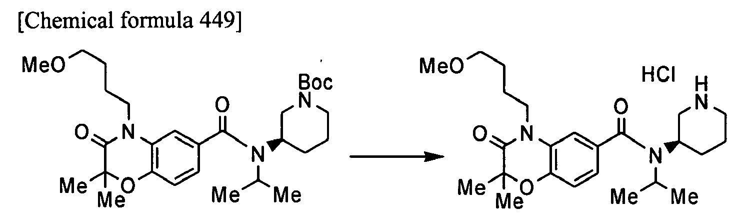

The title compound (20 mg) was synthesized in a similar manner to Example 4.
MS (ESI+) 432(M⁺+1, 100%).

### Examples 127 to 135

The compounds of Examples 127 - 135 are listed in the following Table, said compounds being synthesized as an analog compounds of Example 126.

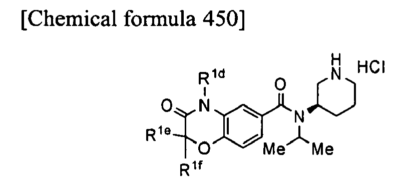

| **Ex. No.** | **R^{1d}** | **R^{1e}** | **R^{1f}** | **Ex. No.** | **R^{1d}** | **R^{1e}** | **R^{1f}** |
|---|---|---|---|---|---|---|---|
| **127** | MeO₂CNH(CH₂)₂ | Me | Me | **131** | MeO(CH₂)₃ | H | Et |
| **128** | MeO(CH₂)₃ | (S)-Ph(2,5-F) | (R)-Me | **132** | MeO(CH₂)₃ | CONMe2 | Me |
| **129** | MeO(CH₂)₃ | Me | Et | **133** | MeO₂CNH(CH₂)₂ | (S)-Ph(2,5-F) | (R)-Me |
| **130** | MeO(CH₂)₃ | H | (R)-Me | **134** | MeO(CH₂)₃ | Me | Me |
| | | | | **135** | MeO(CH₂)₄ | CH2OMe | Me |

The physiochemical properties of the above compounds are shown below.
The compound of Example 127: MS (ESI+) 447(M⁺+1, 100%).
The compound of Example 128: MS (ESI+) 516(M⁺+1, 100%).
The compound of Example 129: MS (ESI+) 432(M⁺+1, 100%).
The compound of Example 130: MS (ESI+) 404(M⁺+1, 100%).
The compound of Example 131: MS (ESI+) 418(M⁺+1, 100%).
The compound of Example 132: MS (ESI+) 475(M⁺+1, 100%).
The compound of Example 133: MS (ESI+) 545(M⁺+1, 100%).
The compound of Example 134: MS (ESI+) 418(M⁺+1, 100%).
The compound of Example 135: MS (ESI+) 448(M⁺+1, 100%).

### Examples 136-169

The compounds of Examples 136- 169 are listed in the following Tables, said compounds being synthesized as an analog compound of Example 1.

**[Table 9]**

| Ex. No. | Structure | MS(ESI+) |
|---|---|---|
| 136 | 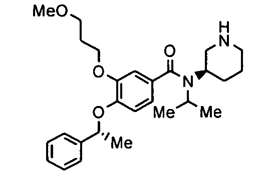 | 455(M⁺+1, 100%). |
| 137 | 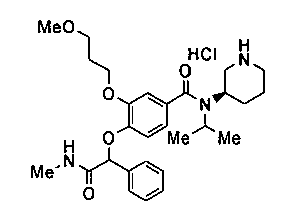 | 498(M⁺+1, 100%). |
| 138 | 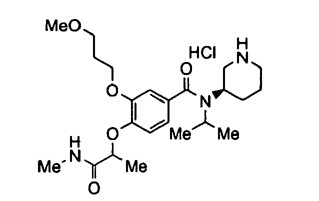 | 584(M⁺+1, 100%). |
| 139 | 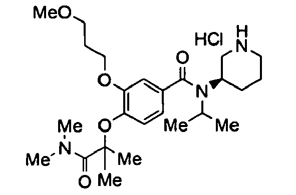 | 464(M⁺+1, 100%). |
| 140 | 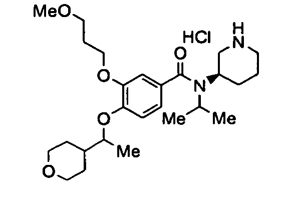 | 463(M⁺+1, 100%). |
| 141 | 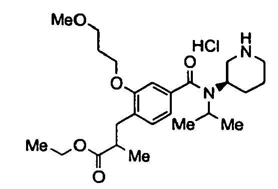 | 449(M⁺+1,100%). |
| 142 | 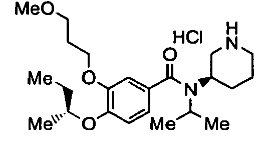 | 407(M⁺+1, 100%). |

| | | |
|---|---|---|
| 143 | 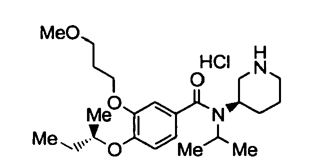 | 407(M⁺+1, 100%). |
| 144 | 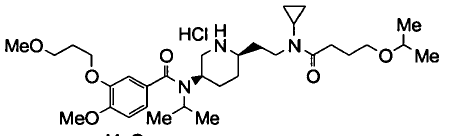 | 576(M⁺+1, 100%). |
| 145 | 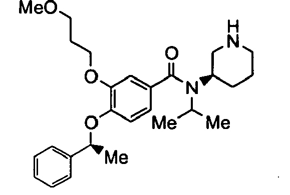 | 455(M⁺+1, 100%). |
| 146 | 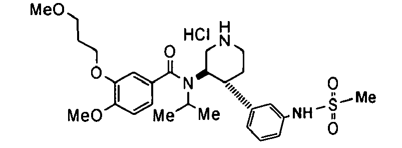 | 534(M⁺+1, 100%). |
| 147 | 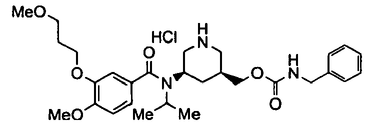 | 528(M⁺+1, 100%). |
| 148 | 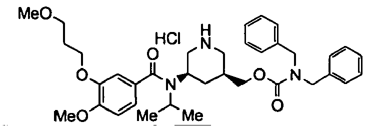 | 618(M⁺+1, 100%). |
| 149 | 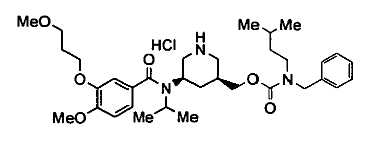 | 598(M⁺+1, 100%). |
| 150 | 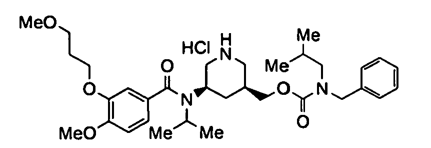 | 584(M⁺+1, 100%). |
| 151 | 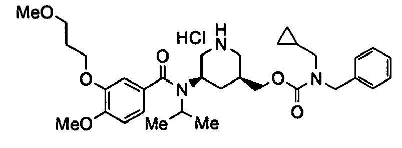 | 582(M⁺+1, 100%). |

| | | |
|---|---|---|
| 152 | 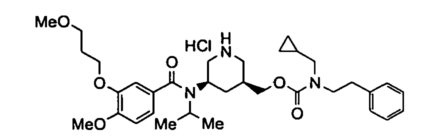 | 596(M⁺+1, 100%). |
| 153 | 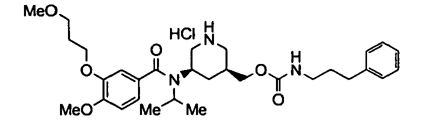 | 556(M⁺+1, 100%). |
| 154 | 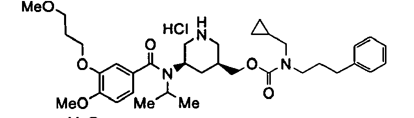 | 610(M⁺+1, 100%). |
| 155 | 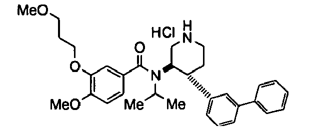 | 517(M⁺+1, 100%). |
| 156 | 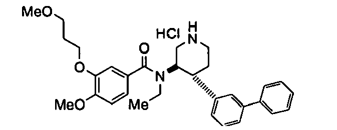 | 503(M⁺+1, 100%). |
| 157 | 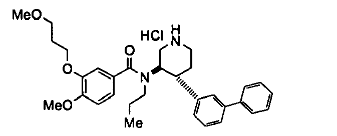 | 517(M⁺+1, 100%). |
| 158 | 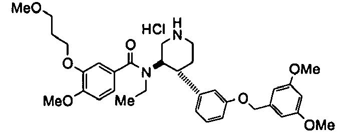 | 593(M⁺+1, 100%). |
| 159 | 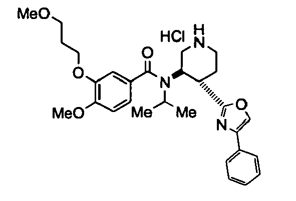 | 508(M⁺+1, 100%). |
| 160 | 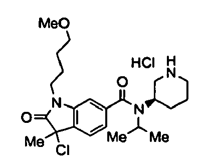 | 574(M⁺+1, 100%) |
| 161 | 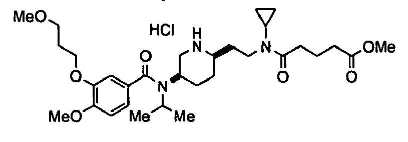 | 576(M⁺+1, 100%) |

| | | |
|---|---|---|
| 162 | 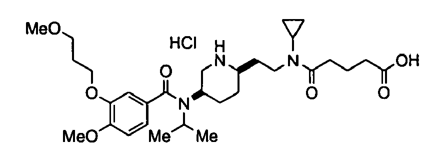 | 562(M⁺+1, 100%) |
| 163 | 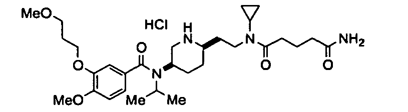 | 561(M⁺+1, 100%) |
| 164 | 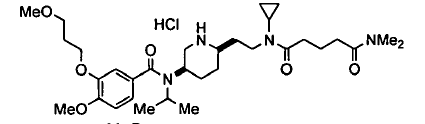 | 589(M+1, 100%). |
| 165 | 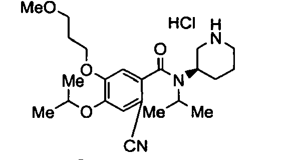 | 418(M⁺+1, 100%). |
| 166 | 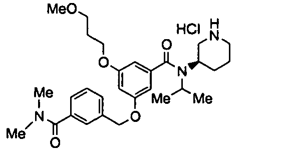 | 512(M⁺+1, 100%). |
| 167 | 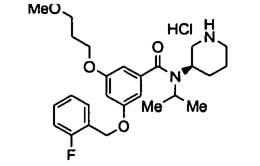 | 459(M⁺+1, 100%). |
| 168 | 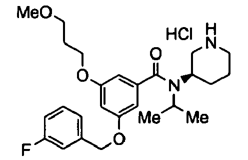 | 459(M⁺+1, 100%). |
| 169 | 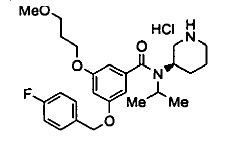 | 459(M⁺+1, 100%). |

### Examples 170-172

The compounds of Examples 170-172 are listed in the following Table, said comopund being synthesized as an analog compound of Example 126.

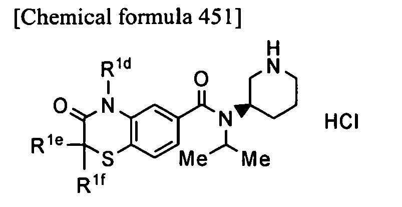

| Ex. No. | R^{1d} | R^{1e} | R^{1f} |
|---|---|---|---|
| 170 | MeO(CH₂)₄ | H | (*R*)-Me |
| 171 | MeO(CH₂)₄ | Me | Me |
| **172** | MeO(CH₂)₄ | (*S*)-CH₂OMe | (*R*)-Me |

(2R)-N-Isopropyl-4-(4-methoxybutyl)-2-methyl-3-oxo-N-[(3R)-piperidin-3-yl]-3,4-dihydro-2H-1,4-benzothiazine-6-carboxyamide hydrochloride, that is the compound of Example 170, was synthesized by the following method. 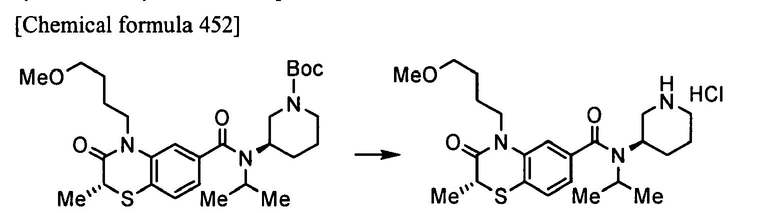 The compound of Example 170 (140 mg) was synthesized from tert-butyl (3R)-3-(isopropyl{[(2R)-4-(4-methoxybutyl)-2-methyl-3-oxo-3,4-dihydro-2H-1,4-benzothiazin-6-yl]carbonyl}amino)piperidine-1-carboxylate in a similar manner to Example 4.
¹H-NMR (400MHz, CDCl₃) 9.90-9.59 (brs, 2H), 7.30 (d, 1H, J = 7.6 Hz),7.01 (brs, 1H), 6.87 (d, 1H, J = 7.6 Hz), 4.16-3.70 (m, 4H), 3.48-3.26 (m, 4H), 3.23 (s, 3H), 2.90-2.65 (m, 2H), 2.12-1.45 (m, 8H), 1.38 (d, 3H, J = 7.0 Hz), 1.32-1.06 (m, 6H).
MS (ESI+) 434(M⁺+1, 100%).

tert-Butyl (3R)-3-(isopropyl{[(2R)-4-(4-methoxybutyl)-2-methyl-3-oxo-3,4-dihydro-2H-1,4-benzothiazin-6-yl]carbonyl}amino)piperidine-1-carboxylate was synthesized by the following Method. 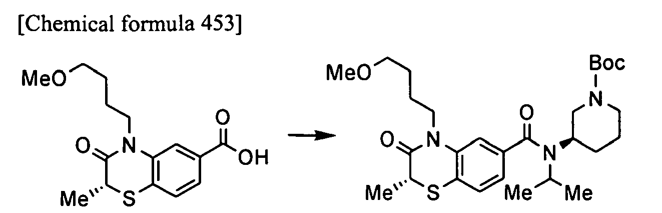 tert-Butyl (3R)-3-(isopropyl{[(2R)-4-(4-methoxybutyl)-2-methyl-3-oxo-3,4-dihydro-2H-1,4-benzothiazin-6-yl]carbonyl}amino)piperidine-1-carboxylate (149.7 mg) was synthesized from (2R)-4-(4-methoxybutyl)-2-methyl-3-oxo-3,4-dihydro-2H-1,4-benzothiazine-6-carboxylic acid in a similar manner to Reference Example 228.
¹H-NMR (400MHz, CDCl₃) 7.38-7.36(m, 1H), 7.09 (brs, 1H), 6.97-6.95 (m, 1H), 4.14-3.74 (m, 5H), 3.49-3.45 (m, 1H), 3.39 (t, 1H, J = 6.3 Hz), 3.31 (s, 3H), 3.15-2.96 (m, 1H), 2.88-2.65 (m, 2H), 1.82-1.55 (m, 7H), 1.54-1.39 (m, 12H), 1.34-1.08 (m, 6H).
MS (ESI+) 534(M⁺+1, 100%).

(2R)-4-(4-Methoxybutyl)-2-methyl-3-oxo-3,4-dihydro-2H-1,4-benzothiazine-6-carboxylic acid was synthesized by the following method. 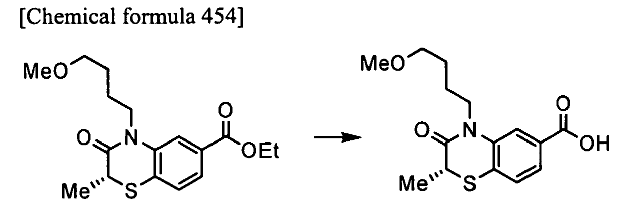 (2R)-4-(4-Methoxybutyl)-2-methyl-3-oxo-3,4-dihydro-2H-1,4-benzothiazine-6-carboxylic acid was synthesized from ethyl (2R)-4-(4-methoxybutyl)-2-methyl-3-oxo-3,4-dihydro-2H-1,4-benzothiazine-6-carboxylate (178.3 mg) in a similar manner to Reference Example 3.
MS (ESI+) 310(M⁺+1, 100%).

Ethyl (2R)-4-(4-methoxybutyl)-2-methyl-3-oxo-3,4-dihydro-2H-1,4-benzothiazine-6-carboxylate was synthesized by the following method. 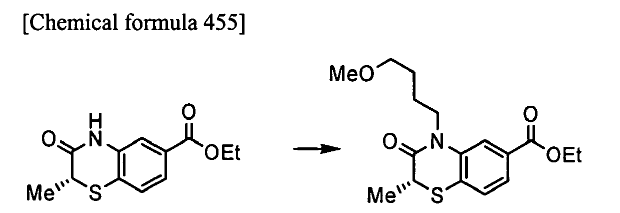 To a solution of ethyl (2R)-2-methyl-3-oxo-3,4-dihydro-2H-1,4-benzothiazine-6-carboxylate (164.7 mg) in N,N-dimethylformamide (5 ml) were added 1-chloro-4-methoxybutane (121 mg), potassium carbonate (181 mg), and a catalytic amount of potassium iodide, and the mixture was stirred at 90°C. After cooling, water was added to the mixture, and the mixture was extracted with ethyl acetate. The organic layer was washed with a saturated aqueous sodium chloride solution, dried over sodium sulfate, filtered and concentrated under reduced pressure. The obtained residue was purified by silica gel chromatography to give ethyl (2R)-4-(4-methoxybutyl)-2-methyl-3-oxo-3,4-dihydro-2H-1,4-benzothiazine-6-carboxylate (178.3 mg).
MS (ESI+) 338 (M⁺+1, 100%).

Ethyl (2R)-2-methyl-3-oxo-3,4-dihydro-2H-1,4-benzothiazine-6-carboxylate was synthesized by the following method. 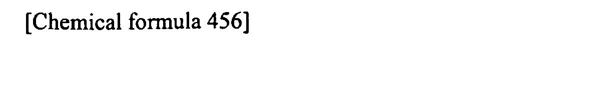 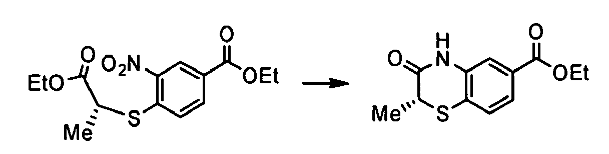 Ethyl (2R)-2-methyl-3-oxo-3,4-dihydro-2H-1,4-benzothiazine-6-carboxylate (164.7 mg) was synthesized from ethyl 4-{[(1R)-2-ethoxy-1-methyl-2-oxoethyl]thio}-3-nitrobenzoate (308.8 mg) in a similar manner to Reference Example 225.
MS (ESI+) 252 (M⁺+1, 100%).

### In vitro renin inhibitory activity assay

Recombinant human renin (50 ng) was reacted in 0.1M HEPES buffer (pH 7.4) containing 0.1M NaCl, 1mM EDTA and 0.1 mg/mL BSA together with a substrate and a test compound at 37°C for one hour. As the substrate, Arg-Glu(EDANS)-Ile-His-Pro-Phe-His-Leu-Val-Ile-His-Thr-Lys(DABCYL)-Arg or DABCYL-γ-Abu-Ile-His-Pro-Phe-His-Leu-Val-Ile-His-Thr-EDANS was added in such an amount so that the final concentration thereof became 4 µM. The elevated fluorescence intensity at the exciting wavelength 340 nm and the fluorescence wavelength 500 nm was measured by a fluorescence plate reader. The concentration of a test compound to be needed to inhibit an enzyme activity in the presence of a test compound in plural concentrations by 50 % was calculated as an IC₅₀ value.

**[Table 10]**

| Test Compound | Human renin inhibitory activity IC₅₀ (µM) | Test Compound | Human renin inhibitory activity IC₅₀ (µM) |
|---|---|---|---|
| Example 1 | 0.33 | Example 58 | 0.15 |
| Example 14 | 0.93 | Example 59 | 0.11 |
| Example 20 | 0.42 | Example 64 | 0.0168 |
| Example 22 | 0.48 | Example 66 | 0.061 |
| Example 23 | 0.33 | Example 67 | 0.0129 |
| Example 27 | 0.19 | Example 68 | 0.0067 |
| Example 31 | 0.24 | Example 69 | 0.0279 |
| Example 41 | 0.43 | Example 72 | 0.0037 |
| Example 43 | 0.77 | Example 73 | 0.0046 |
| Example 45 | 0.41 | Example 74 | 0.0051 |
| Example 46 | 0.79 | Example 75 | 0.0047 |
| Example 48 | 0.62 | Example 78 | 0.00079 |
| Example 49 | 0.74 | Example 81 | 0.0013 |
| Example 50 | 0.54 | Example 86 | 0.004 |
| Example 51 | 0.50 | Example 126 | 0.0217 |
| Example 52 | 0.59 | Example 135 | 0.0353 |
| Example 53 | 0.22 | Compound A | 1.6 |
| Example 55 | 0.69 | Compound B | 2.3 |
| Example 57 | 0.83 | | |

The chemical structures of Compound A and Compound B are shown as follows. Compound A and Compound B are disclosed in Examples of the literature WO 06/066896.

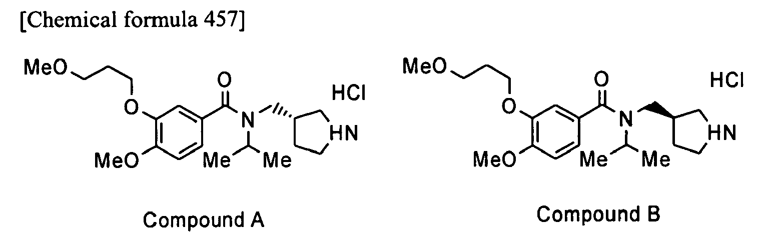

### [INDUSTRIAL APPLICABILITY]

The compounds of the present invention are useful as a therapeutic agent for treatment of hypertension. These compounds are also useful in the control of acute and chronic congestive heart failure. These compounds can also be expected to be useful in the treatment of primary and secondary pulmonary hypertension, secondary hyperaldosteronism, primary and secondary pulmonary hyperaldosteronism, kidney failure, such as glomerulonephritis, diabetic nephropathy, glomerulosclerosis, primary kidney disease, end stage kidney disease, renovascular hypertension, left ventricular failure, diabetic retinopathy, and also useful in inhibition of angiopathy such as migraine, Raynaud's disease, and atherosclerosis process as much as possible. In addition, these compounds are useful in the treatment of diseases relating to elevated intraocular pressure such as glaucoma.
[Sequence Listing Free Text]

The amino acid sequence disclosed in Seq ID: 1 is an amino acid sequence used in the renin inhibitory activity assay.

The amino acid sequence disclosed in Seq ID:2 is an amino acid sequence used in the renin inhibitory activity assay.

## Claims

1. A compound of the formula (I) or a pharmaceutically acceptable salt thereof. 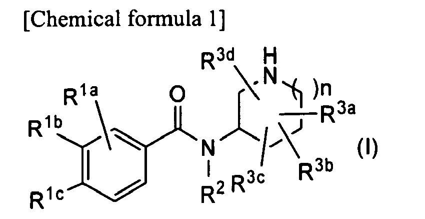
[wherein R^{1a} is a hydrogen atom, a halogen atom, a hydroxy group, a formyl group, a carboxyl group, a cyano group, an optionally substituted C₁₋₆ alkyl group, an optionally substituted C₂₋₆ alkenyl group, an optionally substituted C₂₋₆ alkynyl group, an optionally substituted C₃₋₆ cycloalkyl group, a C₁₋₆ alkylthio group, a C₁₋₆ alkylsulfonyl group, a C₁₋₆ alkoxy group optionally substituted by halogen, C₁₋₄ alkoxy or C₃₋₆ cycloalkyl, an optionally substituted C₃₋₆ cycloalkoxy group, an optionally substituted amino group, an aminocarbonyl group, a C₁₋₄ alkoxycarbonyl group, a C₁₋₄ alkylcarbonyl group, an optionally substituted C₆₋₁₀ aryl group, an optionally substituted C₆₋₁₀ aryloxy group, or an optionally substituted C₇₋₁₄ aralkyloxy group;
R^{1b} is a C₁₋₆ alkyl group substituted by mono-C₁₋₆ alkoxycarbonylamino, an optionally substituted C₁₋₆ alkylsulfinyl group, an optionally substituted C₁₋₆ alkylsulfonyl group, a substituted C₁₋₆ alkoxy group, an optionally substituted amino group, an optionally substituted aminocarbonyl group, an optionally substituted C₁₋₄ alkoxycarbonyl group, or an optionally substituted C₁₋₄ alkylcarbonyl group (where the substituted C₁₋₆ alkoxy group is substituted by one of the members selected from hydroxy, C₁₋₄ alkoxy, C₃₋₆ cycloalkoxy, trifluoromethyl, trifluoromethoxy, difluoroethoxy, carboxy, mono-C₁₋₆ alkylcarbonylamino and mono-C₁₋₆ alkoxycarbonylamino);
R^{1c} is a hydrogen atom, a halogen atom, a hydroxy group, a formyl group, a carboxyl group, a cyano group, an optionally substituted C₁₋₆ alkyl group, an optionally substituted C₃₋₆ cycloalkyl group, an optionally substituted C₅₋₆ cycloalkenyl group, an optionally substituted 5- or 6-membered saturated heterocyclic group, an optionally substituted C₁₋₆ alkylthio group, an optionally substituted C₁₋₆ alkylsulfinyl group, an optionally substituted C₁₋₆ alkylsulfonyl group, an optionally substituted C₆₋₁₀ arylthio group, an optionally substituted C₆₋₁₀ arylsulfinyl group, an optionally substituted C₆₋₁₀ arylsulfonyl group, an optionally substituted C₁₋₆ alkoxy group, an optionally substituted C₃₋₆ cycloalkyloxy group, an optionally substituted C₆₋₁₀ aryloxy group, an optionally substituted C₇₋₁₄ aralkyloxy group, an optionally substituted amino group, an optionally substituted aminocarbonyl group, an optionally substituted C₁₋₄ alkoxycarbonyl group, an optionally substituted C₃₋₆ cycloalkyloxycarbonyl group, an optionally substituted C₁₋₄ alkylcarbonyl group, an optionally substituted C₃₋₆ cycloalkylcarbonyl group, an optionally substituted C₆₋₁₀ arylcarbonyl group, an optionally substituted C₇₋₁₄ aralkyl group, or an optionally substituted 5- to 10-membered monocyclic or polycyclic heteroarylcarbonyl group, or
R^{1a} is a hydrogen atom; and R^{1b} and R^{1c} may combine each other to form a condensed ring with a benzene ring, containing at least one heteroatom;
R² is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, or an optionally substituted 5- to 10-membered monocyclic or polycyclic heteroaryl group (provided that when R^{1a} is a C₁₋₆ alkoxy group having a halogen substituent, then R² is not a hydrogen atom);
R^{3a}, R^{3b}, R^{3e} and R^{3d} are independently the same or different and each is a halogen atom, a cyano group, or a group of the formula: -A-B (in which A is a single bond, -(CH₂)ₛO-, -(CH₂)ₛN(R⁴)-, - (CH₂)ₛSO₂-, -(CH₂)ₛCO-, -(CH₂)ₛCOO-, -(CH₂)ₛN(R⁴)CO-, -(CH₂)ₛN(R⁴)SO₂-, -(CH₂)ₛN(R⁴)COO-, -(CH₂)ₛOCON(R⁴)-, -(CH₂)ₛO-CO-, -(CH₂)ₛCON(R⁴)-, -(CH₂)ₛN(R⁴)CON(R⁴)-, or -(CH₂)ₛSO₂N(R⁴)-,
B is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted 5- to 10-membered monocyclic or polycyclic heteroaryl-C₁₋₄ alkyl group, or an optionally substituted saturated heterocyclic group (provided that when A is -(CH₂)ₛN(R⁴)-, -(CH₂)ₛOCON(R⁴)-, -(CH₂)ₛCON(R⁴)-, -(CH₂)_{S}N(R⁴)CON(R⁴)-, or -(CH₂)ₛSO₂N(R⁴)-, then R⁴ and B may combine each other to form a ring)), or two of R^{3a}, R^{3b}, R^{3c} and R^{3d} are a hydrogen atom, and the remaining two groups may combine to form a fused ring with a heterocyclic ring;
R⁴ is a hydrogen atom, an optionally substituted C₁₋₆ alkyl group, an optionally substituted C₃₋₁₀ cycloalkyl group, an optionally substituted C₆₋₁₀ aryl group, an optionally substituted C₇₋₁₄ aralkyl group, or an optionally substituted 5- to 10-membered monocyclic or polycyclic heteroaryl group;
s is 0, 1 or 2 (provided that when A is -(CH₂)ₛN(R⁴)-, then s is 0 or 2, and when A is-(CH₂)ₛCON(R⁴)-, then s is 1 or 2);
n is 0, 1, or 2]

2. The compound according to claim 1 or a pharmaceutically acceptable salt thereof, wherein R^{1a} is a hydrogen atom, a halogen atom, a hydroxy group, a cyano group, a C₃₋₆ cycloalkyl group, a C₁₋₆ alkyl group, or a C₃₋₆ cycloalkoxy group;
R^{1b} is a C₁₋₆ alkyl group substituted by mono-C₁₋₆ alkoxycarbonylamino, a substituted C₁₋₆ alkoxy group, or an optionally substituted amino group (in which the substituted C₁₋₆ alkoxy group is substituted by one group selected from hydroxy, C₁₋₄ alkoxy, trifluoromethyl, trifluoromethoxy, difluoromethoxy, carboxy, mono-C₁₋₆ alkylcarbonylamino and mono-C₁₋₆ alkoxycarbonylamino),
R^{1c} is a hydrogen atom; a halogen atom; a C₁₋₆ alkoxy group optionally substituted by 1 to 3 halogens or by one group selected from C₃₋₆ cycloalkyl, mono- or di-(C₁₋₆ alkyl)-substituted aminocarbonyl or 5- or 6-membered saturated heterocycle, and C₁₋₄ alkoxycarbonyl; a C₃₋₆ cycloalkyloxy group; or a 5- or 6-membered saturated heterocyclic group, or
R^{1a} is a hydrogen atom; and R^{1b} and R^{1c} may combine each other to form a condensed ring with the benzene ring containing at least one heteroatom.

3. The compound according to claim 1 or a pharmaceutically acceptable salt thereof, wherein R^{1a} is a hydrogen atom, a halogen atom, a cyano group, a C₁₋₆ alkyl group having optionally a halogen substituent, a C₂₋₆ alkenyl group, a C₃₋₆ cycloalkyl group, a C₁₋₆ alkoxy group having optionally a halogen substituent, or a C₃₋₆ cycloalkoxy group.

4. The compound according to claim 3 or a pharmaceutically acceptable salt thereof, wherein R^{1a} is a hydrogen atom, a halogen atom, a cyano group, a C₁₋₆ alkyl group, a C₃₋₆ cycloalkyl group, or a C₃₋₆ cycloalkoxy group.

5. The compound according to any one of claims 1 to 4 or a pharmaceutically acceptable salt thereof, wherein R^{1b} is a substituted C₁₋₆ alkoxy group, which is substituted by one group selected from C₁₋₄ alkoxy, carboxy, mono-C₁₋₆ alkylcarbonylamino and mono-C₁₋₆ alkoxycarbonylamino.

6. The compound according to claim 5 or a pharmaceutically acceptable salt thereof, wherein R^{1b} is a 3-methoxypropyloxy group.

7. The compound according to any one of claims 1 to 6 or a pharmaceutically acceptable salt thereof, wherein R^{1c} is a C₁₋₆ alkoxy group.

8. The compound according to claim 1 or a pharmaceutically acceptable salt thereof, wherein R^{1a} is a hydrogen atom; and R^{1b} and R^{1c} combine each other to form a condensed ring with the benzene ring containing at least one heteroatom.

9. The compound according to claim 8 or a pharmaceutically acceptable salt thereof, wherein the condensed ring is a condensed ring consisting of a 5- or 6-membered saturated ring and a benzene ring.

10. The compound according to claim 9 or a pharmaceutically acceptable salt thereof, wherein the condensed ring has a partial structure of the following formula: 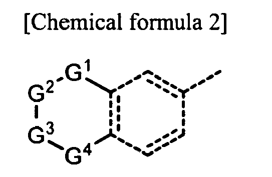
[wherein (i) G¹ is -N(R^{1d})-, G² is -CO-, G³ is -C(R^{1e})(R^{1f})-, and G⁴ is -CH₂-, an oxygen atom, a sulfur atom, or does not exist at all,
(ii) G¹ is -N(R^{1d})-, G² is -CO-, G³ is -N(R^{1d})-, and G⁴ does not exist at all (R^{1d} for G¹ and G³ is independent each other),
(iii) G¹ is an oxygen atom, G² is -CH₂-, G³ is an oxygen atom, and G⁴ does not exist at all, or
(iv) G¹ is an oxygen atom, G² is -CH₂-, G³ is -CH₂-, and G⁴ is an oxygen atom;
R^{1d} is a hydrogen atom, a C₆₋₁₀ aryl group, or a C₁₋₆ alkyl group optionally substituted by one of the members selected from hydroxy, C₃₋₆ cycloalkyl, C₁₋₆ alkoxy, C₃₋₆ cycloalkoxy, trifluoromethyl, trifluoromethoxy and mono-C₁₋₆ alkoxycarbonylamino;
R^{1e} and R^{1f} are independently the same or different and each is a hydrogen atom, a halogen atom, a hydroxy group, a carboxyl group, a cyano group, an optionally substituted C₁₋₆ alkyl group, an optionally substituted C₂₋₆ alkenyl group, an optionally substituted C₂₋₆ alkynyl group, an optionally substituted C₃₋₆ cycloalkyl group, an optionally substituted C₁₋₆ alkoxy group, an optionally substituted C₃₋₆ cycloalkoxy group, an optionally substituted amino group, an optionally substituted aminocarbonyl group, an optionally substituted C₁₋₄ alkoxycarbonyl group, an optionally substituted C₁₋₄ alkylcarbonyl group, an optionally substituted C₆₋₁₀ arylcarbonyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted C₆₋₁₀ aryloxy group, an optionally substituted 5- to 10-membered monocyclic or polycyclic heteroaryloxy group, or an optionally substituted C₇₋₁₄ aralkyloxy group, provided that the ring expressed with dashed line(s) in said chemical structure means a benzene ring].

11. The compound according to claim 10 or a pharmaceutically acceptable salt thereof, wherein R^{1e} and R^{1f} are independently the same or different and each is a hydrogen atom; a halogen atom; a hydroxy group; a cyano group; a C₃₋₆ cycloalkyl group; a C₁₋₆ alkyl group optionally substituted by C₁₋₄ alkoxy, C₃₋₆ cycloalkyl, C₃₋₆ cycloalkyloxy, C₇₋₁₀ aralkyloxy, or aminocarbonyl having optionally a mono- or di-(C₁₋₆ alkyl) substituent; a C₆₋₁₀ aryl group having optionally a halogen substituent; a C₇₋₁₄ aralkyl group having optionally a halogen substituent; an optionally substituted aminocarbonyl group; or a 5- to 10-membered monocyclic or polycyclic heteroaryl group optionally substituted by C₁₋₄ alkyl.

12. The compound according to any one of claims 1 to 11 or a pharmaceutically acceptable salt thereof, wherein R² is a C₁₋₆ alkyl group having optionally a C₃₋₆ cycloalkyl substituent, a C₃₋₆ cycloalkyl group, a C₆₋₁₀ aryl group having optionally a halogen substituent, or a C₇₋₁₄ aralkyl group having optionally a halogen substituent.

13. The compound according to claim 12 or a pharmaceutically acceptable salt thereof, wherein R² is a C₁₋₆ alkyl group or a C₃₋₆ cycloalkyl group.

14. The compound according to claim 13 or a pharmaceutically acceptable salt thereof, wherein R² is isopropyl group.

15. The compound according to any one of claims 1 to 14 or a pharmaceutically acceptable salt thereof, wherein R^{3a}, R^{3b}, R^{3c}, and R^{3d} are independently a group of the formula: -A-B (in which A is a single bond, -(CH₂)ₛO-, -(CH₂)ₛN(R⁴)-, -(CH₂)ₛSO₂-, -(CH₂)ₛCO-, -(CH₂)ₛCOO-, -(CH₂)ₛN(R⁴)CO-, -(CH₂)ₛN(R⁴)SO₂-, -(CH₂)ₛN(R⁴)COO-, -(CH₂)ₛOCON(R⁴)-, -(CH₂)ₛO-CO-, -(CH₂)ₛCON(R⁴)-, -(CH₂)ₛN(R⁴)CON(R⁴)-, or -(CH₂)ₛSO₂N(R⁴)-,
B is a hydrogen atom; an optionally substituted C₁₋₆ alkyl group; a C₂₋₆ alkenyl group; a C₃₋₁₀ cycloalkyl group; a C₆₋₁₀ aryl group optionally substituted by the same or different 1 to 3 groups selected from halogen, cyano, C₁₋₄ alkyl, C₁₋₄ alkoxy, cyano, carboxy, C₁₋₄ alkyl having optionally carboxy substituent, or C₆ aryl having optionally a C₁₋₄ alkoxy substituent (said C₁₋₄ alkoxy being optionally substituted by fluorine, hydroxy, or carboxy), C₆ aryloxy, C₇₋₁₀ aralkyloxy having optionally a C₁₋₄ alkoxy substituent, aminocarbonyl having optionally a mono- or di-(C₁₋₆ alkyl) substituent, and C₁₋₄ alkylsulfonylamino; a C₇₋₁₄ aralkyl group optionally substituted by the same or different 1 to 3 groups selected from halogen, cyano, C₁₋₄ alkyl having optionally a halogen substituent, hydroxy, C₁₋₄ alkoxy having optionally a halogen substituent, carboxy, C₁₋₄ alkoxycarbony, C₆ aryl having optionally a C₁₋₄ alkoxy substituent, a C₆ aryloxy, C₇₋₁₀ aralkyloxy, aminocarbonyl having optionally a mono- or di-(C₁₋₄ alkyl) substituent, C₁₋₄ alkylsulfonylamino and C₁₋₄ alkylsulfonyl; a 5- or 6-membered monocyclic heteroaryl group optionally substituted by halogen or C₆ aryl; a 5- or 6-membered monocyclic heteroaryl-C₁₋₄ alkyl group optionally substituted by C₁₋₄ alkyl; or a 5- or 6-membered saturated heterocyclic group (provided that when A is -(CH₂)ₛN(R⁴)-, -(CH₂)ₛOCON(R⁴)-, -(CH₂)ₛCON(R⁴)-, -(CH₂)ₛN(R⁴)CON(R⁴)-, or -(CH₂)ₛSO₂N(R⁴)-, then R⁴ and B combine each other to form a ring)).

16. The compound according to claim 15 or a pharmaceutically acceptable salt thereof, wherein R⁴ is a hydrogen atom, a C₁₋₆ alkyl group, a C₃₋₆ cycloalkyl group, a C₆₋₁₀ aryl group, a C₇₋₁₄ aralkyl group, or a 5- to 10-membered monocyclic or polycyclic heteroaryl group.

17. The compound according to any one of claims 1 to 16 or a pharmaceutically acceptable salt thereof, wherein R^{3a}, R^{3b}, R^{3c} and R^{3d} attach to the piperidine ring at the substitution positions as shown in the following formula:

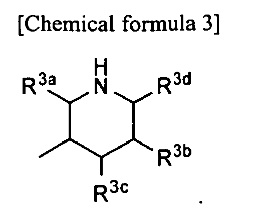

18. The compound according to claim 17 or a pharmaceutically acceptable salt thereof, wherein R^{3a}, R^{3b} and R^{3c} are a group of the formula: -A-B (in which A is a single bond, B is a hydrogen atom);
R^{3d} is a group of the formula: -A-B (in which A is a single bond, -(CH₂)ₛO-, -(CH₂)ₛN(R⁴)-, -(CH₂)ₛCOO-, -(CH₂)ₛN(R⁴)CO-, -(CH₂)ₛN(R⁴)SO₂-, -(CH₂)ₛN(R⁴)COO-, - (CH₂)ₛOCON(R⁴)-, or -(CH₂)ₛCON(R⁴)-, B is a hydrogen atom, an optionally substituted C₁₋₆ alkyl group, an optionally substituted C₂₋₆ alkenyl group, an optionally substituted C₆₋₁₀ aryl group, an optionally substituted C₇₋₁₄ aralkyl group, an optionally substituted 5- to 10-membered monocyclic or polycyclic heteroaryl-C₁₋₄ alkyl group, or an optionally substituted 5- or 6-membered saturated heterocyclic group).

19. The compound according to claim 18 or a pharmaceutically acceptable salt thereof, wherein A is -(CH₂)ₛN(R⁴)CO-.

20. The compound according to claim 18 or a pharmaceutically acceptable salt thereof, wherein A for R^{3d} is -(CH₂)ₛN(R⁴)CO-, -(CH₂)ₛN(R⁴)SO₂-, or -(CH₂)ₛN(R⁴)COO-; B for R^{3d} is an optionally substituted C₁₋₆ alkyl group, an optionally substituted C₂₋₆ alkenyl group, an optionally substituted C₆₋₁₀ aryl group, an optionally substituted C₇₋₁₄ aralkyl group, or an optionally substituted 5- to 10-membered monocyclic or polycyclic heteroaryl-C₁₋₄ alkyl group; and R⁴ is a C₃₋₆ cycloalkyl group.

21. The compound according to any one of claims 18 to 20 or a pharmaceutically acceptable salt thereof, wherein B is a C₁₋₆ alkyl group optionally substituted by one group selected from C₃₋₆ cycloalkyl, hydroxy, C₁₋₄ alkoxy, carboxy, C₁₋₄ alkoxycarbonyl, di-(C₁₋₆ alkyl)amino, and aminocarbonyl having optionally a mono- or di-(C₁₋₆ alkyl) substituent; a C₂₋₆ alkenyl group; a C₆₋₁₀ aryl group optionally substituted by the same or different 1 to 3 groups selected from halogen, C₆ aryl and C₆ aryloxy; a C₇₋₁₄ aralkyl group optionally substituted by the same or different 1 to 3 groups selected from halogen, cyano, C₁₋₄ alkyl having optionally 1 to 3 halogen substituents, hydroxy, C₁₋₄ alkoxy, carboxy, C₁₋₄ alkoxycarbonyl, aminocarbonyl, and C₁₋₄ alkylsulfonylamino; or a 5- to 10-membered monocyclic or polycyclic heteroaryl-C₁₋₄ alkyl group.

22. The compound according to any one of claims 18 to 21 or a pharmaceutically acceptable salt thereof, wherein s is 2.

23. The compound according to any one of claims 18 to 22 or a pharmaceutically acceptable salt thereof, wherein R⁴ is a C₃₋₆ cycloalkyl group.

24. The compound according to any one of claims 1 to 17 or a pharmaceutically acceptable salt thereof, wherein R^{3a}, R^{3b}, R^{3c} and R^{3d} are independently a group of the formula: -A-B (in which A is a single bond, B is a hydrogen atom).

25. The compound according to any one of claims 1 to 17 or a pharmaceutically acceptable salt thereof, wherein n is 1.

26. A compound of the following formula (III) or a pharmaceutically acceptable salt thereof. 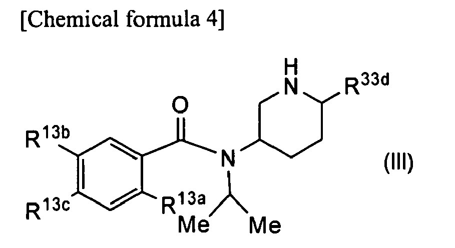
[wherein R^{13a} is a hydrogen atom, a halogen atom, a cyano group, a C₁₋₆ alkyl group having optionally a halogen substituent, a C₃₋₆ cycloalkyl group, or a C₃₋₆ cycloalkoxy group;
R^{13b} is a C₁₋₆ alkoxy group substituted by one group selected from C₁₋₄ alkoxy, carboxy, mono-C₁₋₆ alkylcarbonylamino and mono-C₁₋₆ alkoxycarbonylamino,
R^{13c} is a C₁₋₆ alkoxy group, or R^{13a} is a hydrogen atom; R^{13b} and R^{13c} combine each other to form a condensed ring with the benzene ring containing at least one heteroatom;
R^{33d} is a group of the formula: -A³-B³ (in which A³ is -(CH₂)ₛ₃N(R⁴³)-, -(CH₂)ₛ₃N(R⁴³)CO-, -(CH₂)ₛ₃N(R⁴³)SO₂-, -(CH₂)ₛ₃N(R⁴³)COO-, -(CH₂)ₛ₃OCON(R⁴³)-, -(CH₂)ₛ₃CON(R⁴³)-, -(CH₂)ₛ₃N(R⁴³)CON(H)-, or -(CH₂)ₛ₃SO₂N(R⁴³)-, B³ is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted 5- to 10-membered monocyclic or polycyclic heteroaryl-C₁₋₄ alkyl group, or an optionally substituted saturated heterocyclic group (provided that when A³ is -(CH₂)ₛ₃N(R⁴³)-, -(CH₂)ₛ₃OCON(R⁴³)-, -(CH₂)ₛ₃CON(R⁴³)-, or -(CH₂)ₛ₃SO₂N(R⁴³)-, then R⁴³ and B³ combine each other to form a ring));R⁴³ is a C₃₋₆ cycloalkyl group;
and s3 is 0, 1 or 2].

27. The compound according to claim 26 or a pharmaceutically acceptable salt thereof, wherein R^{13a} is a hydrogen atom.

28. The compound according to claim 26 or a pharmaceutically acceptable salt thereof, wherein R^{13a} is a halogen atom.

29. The compound according to claim 26 or a pharmaceutically acceptable salt thereof, wherein R^{13a} is a C₁₋₆ alkyl group.

30. The compound according to claim 26 or a pharmaceutically acceptable salt thereof, wherein R^{13a} is a cyano group.

31. The compound according to any one of claims 26 to 30 or a pharmaceutically acceptable salt thereof, wherein R^{13b} is a C₁₋₆ alkoxy group substituted by C₁₋₄ alkoxy.

32. The compound according to claim 31 or a pharmaceutically acceptable salt thereof, wherein R^{13b} is a 3-methoxypropyloxy group.

33. The compound according to claim 26 or a pharmaceutically acceptable salt thereof, wherein the condensed ring is a condensed ring having a partial structure of the following formula: 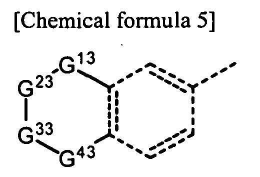
[wherein G¹³ is -N(R^{13d})-, G²³ is -CO-, G³³ is -C(R^{13e})(R^{13f})-, and G⁴³ is an oxygen atom or a sulfur atom; R^{13d} is a C₁₋₆ alkyl group optionally substituted by C₁₋₆ alkoxy or mono-C₁₋₆ alkoxycarbonylamino; R^{13e} and R^{13f} are independently the same or different and each is a hydrogen atom; a halogen atom; a hydroxy group; a cyano group; a C₃₋₆ cycloalkyl group; a C₁₋₆ alkyl group optionally substituted by C₁₋₄ alkoxy, C₃₋₆ cycloalkyl, or C₇₋₁₀ aralkyloxy; a C₆₋₁₀ aryl group having optionally a halogen substituent; a C₇₋₁₄ aralkyl group having optionally a halogen substituent; an aminocarbonyl group having optionally a mono- or di-(C₁₋₆ alkyl) substituent; or a 5- to 10-membered monocyclic or polycyclic heteroaryl group having optionally a C₁₋₄ alkyl substituent, provided that the ring expressed with dashed lines in said chemical structure means a benzene ring].

34. The compound according to claim 33 or a pharmaceutically acceptable salt thereof, wherein G⁴³ is an oxygen atom.

35. The compound according to claim 33 or a pharmaceutically acceptable salt thereof, wherein G⁴³ is a sulfur atom.

36. The compound according to any one of claims 33 to 35 or a pharmaceutically acceptable salt thereof, wherein R^{13d} is a 4-methoxybutyl group.

37. The compound according to any one of claims 26 to 36 or a pharmaceutically acceptable salt thereof, wherein A³ is -(CH₂)ₛ₃N(R⁴³)CO-.

38. The compound according to any one of claims 26 to 37 or a pharmaceutically acceptable salt thereof, wherein S3 is 2.

39. A renin inhibitor comprising as the active ingredient the compound as set forth in any one of claims 1 to 38 or a pharmaceutically acceptable salt thereof.

40. A therapeutic agent for hypertension comprising as the active ingredient the compound as set forth in any one of claims 1 to 38 or a pharmaceutically acceptable salt thereof.

41. A compound of the following formula (VII) or a pharmaceutically acceptable salt thereof. 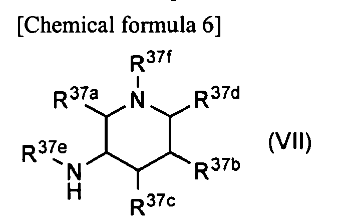
[wherein R^{37a}, R^{37b}, R^{37c} and R^{37d} are independently the same or different and each is a halogen atom, a cyano group, or a group of the formula: -A⁷-B⁷ (in which A⁷ is a single bond, -(CH₂)ₛ₇O-, -(CH₂)ₛ₇N(R⁴⁷)-, -(CH₂)ₛ₇SO₂-, -(CH₂)ₛ₇CO-, -(CH₂)ₛ₇COO-, -(CH₂)ₛ₇N(R⁴⁷)CO-, -(CH₂)ₛ₇N(R⁴⁷)SO₂-, -(CH₂)ₛ₇N(R⁴⁷)COO-, -(CH₂)ₛ₇OCON(R⁴⁷)-, -(CH₂)ₛ₇O-CO-, -(CH₂)ₛ₇CON(R⁴⁷)-, -(CH₂)ₛ₇N(R⁴⁷)CON(R⁴⁷)-, or -(CH₂)ₛ₇SO₂N(R⁴⁷)-, B⁷ is a hydrogen atom, an optionally substituted C₁₋₆ alkyl group, an optionally substituted C₂₋₆ alkenyl group, an optionally substituted C₂₋₆ alkynyl group, an optionally substituted C₃₋₁₀ cycloalkyl group, an optionally substituted C₅₋₆ cycloalkenyl group, an optionally substituted C₆₋₁₀ aryl group, an optionally substituted C₇₋₁₄ aralkyl group, an optionally substituted 5- to 10-membered monocyclic or polycyclic heteroaryl group, an optionally substituted 5- to 10-membered monocyclic or polycyclic heteroaryl-C₁₋₄ alkyl group, or an optionally substituted saturated heterocyclic group (provided that when A⁷ is -(CH₂)ₛ₇N(R⁴⁷)-, - (CH₂)ₛ₇OCON(R⁴⁷)-, -(CH₂)ₛ₇CON(R⁴⁷)-, or -(CH₂)ₛ₇SO₂N(R⁴⁷)-, then R⁴⁷ and B⁷ combine each other to form a ring));
R⁴⁷ is a hydrogen atom, an optionally substituted C₁₋₆ alkyl group, an optionally substituted C₃₋₁₀ cycloalkyl group, an optionally substituted C₆₋₁₀ aryl group, an optionally substituted C₇₋₁₄ aralkyl group, or an optionally substituted 5- to 10-membered monocyclic or polycyclic heteroaryl group;
s7 is 0, 1 or 2 (provided that when A⁷ is -(CH₂)ₛ₇N(R⁴⁷)-, the s7 is 0 or 2, and when A⁷ is - (CH₂)ₛ₇CON(R⁴⁷)-, then s7 is I or 2);
R^{37e} is an isopropyl group or a cyclopropyl group;
R^{37f} is a hydrogen atom, a C₁₋₄ alkoxycarbonyl group, a benzyloxycarbonyl group, an allyloxycarbonyl group, a p-toluenesulfonyl group, or a nitrobenzenesulfonyl group (provided that when R^{37e} is a cyclopropyl group, and R^{37f} is a t-butoxycarbonyl group, then (i) the compound where R^{37a}, R^{37b} and R^{37d} are a group of the formula: -A⁷-B⁷ (in which A⁷ is a single bond, B⁷ is a hydrogen atom); R^{37c} is a group of the formula: -A⁷-B⁷ (in which A⁷ is a single bond, and B⁷ is a C₆ aryl group), and (ii) the compound where R^{37a}, R^{37b}, R^{37c}, and R^{37d} are independently a group of the formula: -A⁷-B⁷ (in which A⁷ is a single bond, and B⁷ is a hydrogen atom) are excluded].

42. The compound according to claim 41 or a salt thereof, wherein R^{37e} is isopropyl group.

43. The compound according to claim 41 or a salt thereof, wherein R^{37e} is cyclopropyl group.

44. The compound according to any one of claims 41 to 43 or a salt thereof, wherein R^{37f} is a hydrogen atom.
